(19) Europäisches Patentamt
European Patent Office
Office européen des brevets

(11) EP 1 942 109 A1

(12) **EUROPEAN PATENT APPLICATION**
published in accordance with Art. 153(4) EPC

(43) Date of publication:
**09.07.2008 Bulletin 2008/28**

(21) Application number: **06812154.0**

(22) Date of filing: **24.10.2006**

(51) Int Cl.:
***C07D 493/08*** *(2006.01)* ***A61K 31/397*** *(2006.01)*
***A61K 31/422*** *(2006.01)* ***A61K 31/443*** *(2006.01)*
***A61P 35/00*** *(2006.01)*

(86) International application number:
**PCT/JP2006/321094**

(87) International publication number:
**WO 2007/049575 (03.05.2007 Gazette 2007/18)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.10.2005 JP 2005309474**

(71) Applicant: **Daiichi Sankyo Company, Limited**
**Chuo-ku**
**Tokyo 103-8426 (JP)**

(72) Inventors:
- **UOTO, Kouichi**
  **Edogawa-ku, Tokyo 134-8630 (JP)**
- **TAKEDA, Yasuyuki**
  **Edogawa-ku, Tokyo 134-8630 (JP)**
- **SAKAMOTO, Atsunobu**
  **Edogawa-ku, Tokyo 134-8630 (JP)**
- **TAKAYANAGI, Yoshihiro**
  **Edogawa-ku, Tokyo 134-8630 (JP)**

(74) Representative: **Polypatent**
**An den Gärten 7**
**51491 Overath (DE)**

# (54) TAXANE COMPOUND WITH AZETIDINE RING STRUCTURE

(57) A compound represented by the general formula (1) [$X^1$ and $X^2$ represent hydrogen atom, a halogen atom, hydroxyl group and the like, $R^1$ represents a phenyl group, $R^2$ represents an alkyl group, an alkenyl group, or an alkoxy group, $R^3$ represents hydrogen atom, a halogen atom, hydroxyl group, or an alkoxy group, $R^4$ represents hydrogen atom, or an alkyl group, $Z^1$ and $Z^2$ represent hydrogen atom, a halogen atom, hydroxyl group and the like, $Z^3$ represents cyano group, an alkyl group, an alkenyl group and the like, $Z^4$ represents an alkyl group, an alkenyl group, an alkynyl group and the like, and ---- represents a single bond or a double bond], which shows high antitumor effect against cancer cells including drug resistant cells.

EP 1 942 109 A1

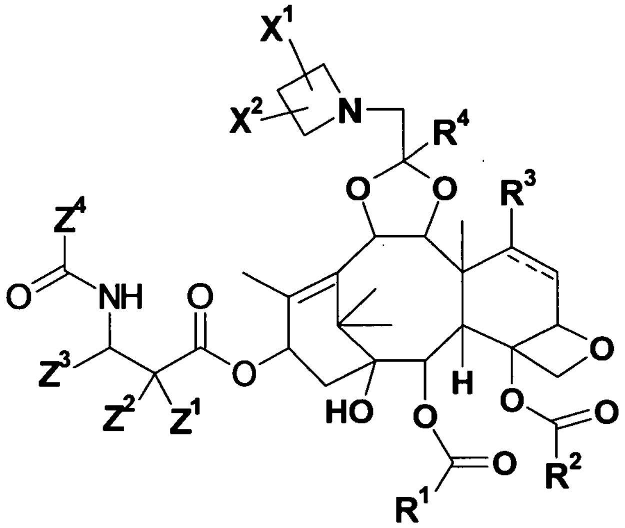

(1)

## Description

Field of the Invention

The present invention relates to a taxane compound having antitumor activity. Specifically, the present invention relates to a taxane compound having an azetidine ring structure.

Background Art

As compounds having antitumor activity, taxane compounds are available. A typical example of the taxane compounds includes paclitaxel represented by the following general formula (I) which is a naturally occurring substance.

[Formula 1]

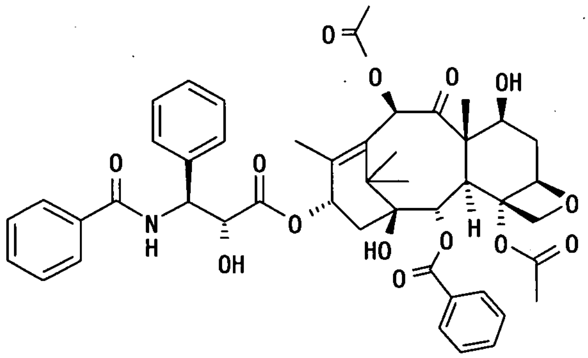

(I)

Paclitaxel is obtainable from the trunk of Taxus baccata and the like in a small amount. The mechanism of action of paclitaxel is considered to be based on inhibitory action on depolymerization of microtubules in cell division.

Several taxane compounds having an antitumor activity superior to that of paclitaxel have been reported in recent years (see, Patent documents 1 to 3).

[Patent document 1] Japanese Patent Unexamined Publication (KOKAI) No. 9-12578
[Patent document 2] International Patent Publication WO01/027115
[Patent document 3] Japanese Patent Unexamined Publication No. 2002-332287

Disclosure of the Invention

Object to be Achieved by the Invention

Since anticancer agents generally have an optimum dose very close to a maximum tolerance dose (MTD), problems of side effects such as bone marrow depression and digestive organ diseases arise in many clinical cases. Patients may sometimes die from these side effects when they are serious. Therefore, development of anticancer agents has been desired which have a wide effective dose range (therapeutic range) and an optimum dose wide apart from MTD. The present invention provides a novel taxane anticancer agent having high antitumor activity and a wider effective dose range.

Means for Achieving the Object

The inventor of the present invention conducted various researches, and as a result, they found that the compounds represented by the following general formula (1) had high antitumor effect against cancer cells including drug resistant cells, and a wide effective dose range to accomplish the present invention.

The present invention thus provides the followings:

1. A compound represented by the general formula (1) or a salt thereof, or a solvate thereof:

[Formula 2]

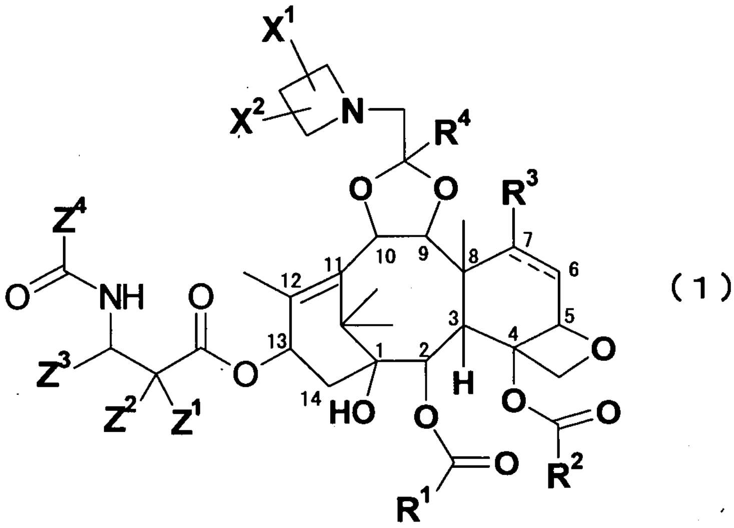

(1)

[wherein $X^1$ and $X^2$ independently represent hydrogen atom, a halogen atom, hydroxyl group, cyano group, carboxy group, an alkoxy group which may be substituted, an alkoxycarbonyl group, an alkyl group which may be substituted, an alkenyl group which may be substituted, an alkynyl group which may be substituted, a phenyl group which may be substituted, an alkanoyl group which may be substituted, a carbamoyl group which may be substituted, a carbamoyloxy group which may be substituted, an alkylsulfonyl group which may be substituted, an aminosulfonyl group which may be substituted, an amino group which may be substituted, or a 4- to 6-membered saturated heterocyclic group, or

$X^1$ and $X^2$ may together form oxo group or a =N-OY group (Y represents hydrogen atom, or an alkyl group which may be substituted), or $X^1$ and $X^2$ may form a 4- to 6-membered saturated hydrocarbon ring binding as a spiro ring system or a condensed ring system, or a 5- or 6-membered saturated heterocyclic ring binding as a spiro ring system or a condensed ring system together with the carbon atom or atoms to which $X^1$ and $X^2$ bind,

$R^1$ represents a phenyl group which may be substituted,

$R^2$ represents an alkyl group which may be substituted, an alkenyl group which may be substituted, or an alkoxy group which may be substituted,

$R^3$ represents hydrogen atom, a halogen atom, hydroxyl group, or an alkoxy group which may be substituted,

$R^4$ represents hydrogen atom, or an alkyl group which may be substituted,

$Z^1$ and $Z^2$ independently represent hydrogen atom, a halogen atom, hydroxyl group, an alkoxy group which may be substituted, or an alkyl group which may be substituted,

$Z^3$ represents cyano group, an alkyl group which may be substituted, an alkenyl group which may be substituted, an alkynyl group which may be substituted, a phenyl group which may be substituted, or a 4- to 6-membered saturated or unsaturated heterocyclic group which may be substituted,

$Z^4$ represents an alkyl group which may be substituted, an alkenyl group which may be substituted, an alkynyl group which may be substituted, a phenyl group which may be substituted, an aralkyl group which may be substituted, a 5- or 6-membered aromatic heterocyclic group which may be substituted, or an alkoxy group which may be substituted, and

the 6- and 7-positions of the partial structure represented by the following general formula (2)

[Formula 3]

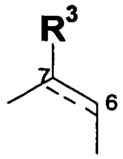

(2)

may be bonded with a double bond (when the bond is a double bond, $R^3$ is hydrogen atom);

2. A compound represented by the general formula (1) having the absolute configuration represented by the general formula (3) or a salt thereof, or a solvate thereof:

[Formula 4]

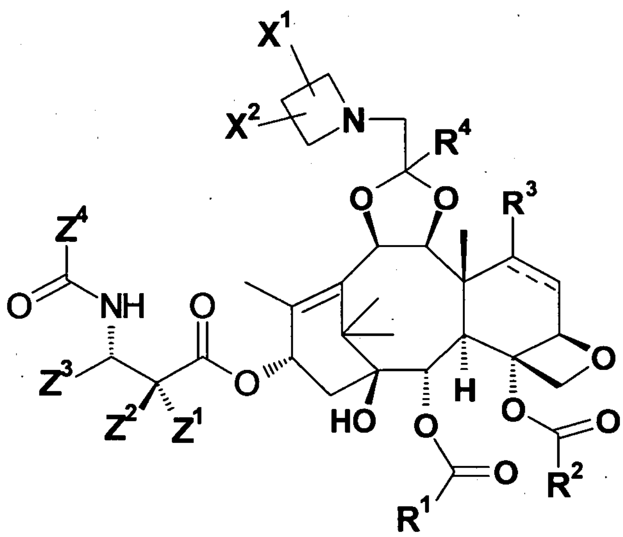

(3)

(wherein $X^1$, $X^2$, $R^1$ to $R^4$, and $Z^1$ to $Z^4$ have the same meanings as those defined above);

3. A medicament comprising a compound represented by the aforementioned general formula (1) or (3) or a salt thereof, or a solvate thereof as an active ingredient;
4. An anticancer agent comprising a compound represented by the aforementioned general formula (1) or (3) or a salt thereof, or a solvate thereof as an active ingredient;
5. A pharmaceutical composition comprising a compound represented by the aforementioned general formula (1) or (3) or a salt thereof, or a solvate thereof, and a pharmaceutically acceptable carrier;
6. Use of a compound represented by the aforementioned general formula (1) or (3) or a salt thereof, or a solvate thereof for the manufacture of the medicament;
7. Use of a compound represented by the aforementioned general formula (1) or (3) or a salt thereof, or a solvate thereof for the manufacture of the anticancer agent;
8. A method for therapeutic treatment of a cancer, which comprises administering a compound represented by the aforementioned general formula (1) or (3) or a salt thereof, or a solvate thereof.

Effect of the Invention

The compounds of the present invention have high antitumor effect against cancer cells including drug resistant cells. Accordingly, they can be used as potent anticancer agents. Furthermore, the compounds of the present invention have a wide range of effective dose, thereby an optimum dose widely apart from MTD is expected, and therefore they can be used as safe anticancer agents hardly causing side effects. Best Mode for Carrying out the Invention
The substituents in the general formula (1) are explained below.
Examples of the "halogen atom" include fluorine atom, chlorine atom, and bromine atom.
The "alkoxy group which may be substituted" means an alkoxy group having a linear, branched or cyclic alkyl group having 1 to 6 carbon atoms, which may have one or more substituents. Examples of the alkoxy group include, for example, methoxy group, ethoxy group, propoxy group, isopropoxy group, butoxy group, and cyclopentyloxy group. Examples of the substituent which can substitute on these alkoxy groups include, for example, a halogen atom.
The "alkoxycarbonyl group" means a group formed by carbonyl group (-CO-) bound with an alkoxy group having a linear, branched or cyclic alkyl group having 1 to 6 carbon atoms, and examples include, for example, methoxycarbonyl group,

ethoxycarbonyl group, and propylcarbonyl group.

The "alkyl group which may be substituted" means a linear, branched or cyclic alkyl group which may have one or more substituents, and examples include, for example, an alkyl group having 1 to 6 carbon atoms, which may have one or more substituents, and a cycloalkyl group having 3 to 8 carbon atoms, which may have one or more substituents. Examples of the alkyl group include, for example, methyl group, ethyl group, propyl group, isopropyl group, cyclopropyl group, cyclobutyl group, cyclopentyl group, and cyclohexyl group. Examples of the substituent which can substitute on this alkyl group include, for example, a halogen atom, hydroxyl group, an alkoxy group, carbamoyl group, amino group, an alkanoyl group, and cyano group, and the number of the substituent which can substitute on the alkyl group is preferably 1 to 3.

The "alkenyl group which may be substituted" means a linear or branched alkenyl group having 2 to 6 carbon atoms, which may have one or more substituents. Examples of the alkenyl group include, for example, vinyl group, allyl group, and butenyl group. Examples of the substituent which can substitute on the alkenyl group include, for example, a halogen atom, hydroxyl group, an alkoxy group, carbamoyl group, amino group, an alkanoyl group, and cyano group, and the number of the substituent which can substitute on the alkenyl group is preferably 1 to 3.

The "alkynyl group which may be substituted" means a linear or branched alkynyl group having 2 to 6 carbon atoms, which may have one or more substituents. Examples of the alkynyl group include, for example, ethynyl group, propynyl group, butynyl group, pentynyl group, and hexynyl group. Examples of the substituent which can substitute on this alkynyl group include, for example, a halogen atom, hydroxyl group, an alkoxy group, carbamoyl group, amino group, an alkanoyl group, and cyano group, and the number of the substituent which can substitute on this alkynyl group is preferably 1 to 3.

The "phenyl group which may be substituted" means unsubstituted phenyl group or a substituted phenyl group. In the case of the substituted phenyl group, examples of the substituent include, for example, a halogen atom, a hydroxyl group, an alkoxy group, an alkyl group, carbamoyl group, amino group, an alkanoyl group, and cyano group, and the number of the substituent which can substitute on the phenyl group is preferably 1 to 3, more preferably 1 or 2.

The "alkanoyl group which may be substituted" means an alkanoyl group which may have one or more substituents. Examples of the alkanoyl group include, for example, formyl group, acetyl group, methylpropionyl group, and cyclopentanecarbonyl group. Examples of the substituent which can substitute on the alkanoyl group include, for example, a halogen atom and amino group.

The "carbamoyl group which may be substituted" means a carbamoyl group which may have one or two substituents. Examples of the substituent which can substitute on this carbamoyl group include, for example, an alkyl group having 1 to 6 carbon atoms.

The "carbamoyloxy group which may be substituted" means a carbamoyloxy group which may have one or two substituents. Examples of the substituent which can substitute on this carbamoyloxy group include, for example, an alkyl group having 1 to 6 carbon atoms.

The "alkylsulfonyl group which may be substituted" means a group formed by sulfonyl group ($-SO_2-$) bound with an alkyl group having 1 to 6 carbon atoms, which may have one or more substituents. Examples of the alkylsulfonyl group include, for example, methanesulfonyl group and ethanesulfonyl group. Examples of the substituent which can substitute on the alkylsulfonyl group include, for example, carboxy group and an alkoxycarbonyl group.

The "aminosulfonyl group which may be substituted" means a group formed by sulfonyl group ($-SO_2-$) bound with an amino group which may have one or more substituents. Examples of the aminosulfonyl group include, for example, methylaminosulfonyl group, ethylaminosulfonyl group, dimethylaminosulfonyl group, and diethylaminosulfonyl group.

The "amino group which may be substituted" means amino group or an amino group substituted with one or two substituents. Examples of the substituent which can substitute on the amino group include, for example, an alkyl group, an alkanoyl group, an alkylsulfonyl group, an alkoxycarbonyl group, carbamoyl group, an alkylcarbamoyl group, and a dialkylcarbamoyl group, more specifically, methyl group, ethyl group, acetyl group, methanesulfonyl group, ethanesulfonyl group, methoxycarbonyl group, ethoxycarbonyl group, carbamoyl group, methylcarbamoyl group, ethylcarbamoyl group, dimethylcarbamoyl group, and diethylcarbamoyl group.

The "4- to 6-membered saturated heterocyclic group" means a substituent derived from a 4- to 6-membered saturated heterocyclic compound containing one or more oxygen atoms, nitrogen atoms or sulfur atoms as constituent atoms of a ring structure, and this heterocyclic group may bind at any position. Examples of the 4- to 6-membered saturated heterocyclic group include, for example, a substituent derived from a saturated heterocyclic compound such as pyrrolidine, tetrahydrofuran, tetrahydrothiophene, imidazolidine, tetrahydropyran, piperidine, piperazine, dioxane, pyrazolidine, morpholine, azetidine, and oxetane. When this saturated heterocyclic group has a substituent, examples of the substituent include, for example, one or more halogen atoms, alkyl groups, amino groups, and alkoxy groups.

"Y" in the "=N-OY group (Y represents hydrogen atom or an alkyl group which may be substituted)" is hydrogen atom, or a linear, branched, or cyclic alkyl group having 1 to 6 carbon atoms, which may have one or more substituents. Typical examples of the "=N-OY group" include, for example, =N-O-H group, =N-O-methyl group, =N-O-ethyl group, =N-O-propyl group, and =N-O-cyclopropyl group.

The "4- to 6-membered saturated hydrocarbon ring binding as a spiro ring system" formed by $X^1$ and $X^2$, together with the carbon atom of the ring to which $X^1$ and $X^2$ bind, means a ring formed by an alkylene group having 3 to 5 carbon atoms binding to the carbon atom of the azetidine ring in the general formula (1) as a spiro ring system. Specifically, the azetidine ring in the general formula (1) and the saturated hydrocarbon ring form a substituent derived from, for example,
2-aza-spiro[3.3]heptane, 1-aza-spiro[3.3]heptane, 2-aza-spiro[3.4]octane,
1-aza-spiro[3.4]octane, 2-aza-spiro[3.5]nonane, or 1-aza-spiro[3.5]nonane.

The "4- to 6-membered saturated hydrocarbon ring binding as a condensed ring system" formed by $X^1$ and $X^2$, together with the carbon atoms of the ring to which $X^1$ and $X^2$ bind, means a ring formed by an alkylene group having 2 to 4 carbon atoms binding to the carbon atoms of the azetidine ring in the general formula (1) as a condensed ring system. Specifically, the azetidine ring in the general formula (1) and the saturated hydrocarbon ring form a substituent derived from, for example, 2-aza-bicyclo[2.2.0]hexane, 6-aza-bicyclo[3.2.0]heptane, or 7-aza-bicyclo[4.2.0]octane.

The "5- or 6-membered saturated heterocyclic ring binding as a spiro ring system" formed by $X^1$ and $X^2$, together with the carbon atom of the ring to which $X^1$ and $X^2$ bind, means a ring formed by an alkyleneoxy group or alkyleneoxyalkylene group having 3 or 4 carbon atoms binding to the carbon atom of the azetidine ring in the general formula (1) as a spiro ring system. Specifically, the azetidine ring in the general formula (1) and the saturated heterocyclic ring form a substituent derived from, for example, 5-oxa-2-aza-spiro[3.4]octane, 6-oxa-2-aza-spiro[3.4]octane,
5-oxa-1-aza-spiro[3.4]octane, 6-oxa-1-aza-spiro[3.4]octane,
5-oxa-2-aza-spiro[3.5]nonane, 6-oxa-2-aza-spiro[3.5]nonane,
7-oxa-2-aza-spiro[3.5]nonane, 5-oxa-1-aza-spiro[3.5]nonane,
6-oxa-1-aza-spiro[3.5]nonane, or 7-oxa-1-aza-spiro[3.5]nonane.

The "5- or 6-membered saturated heterocyclic ring binding as a condensed ring system" formed by $X^1$ and $X^2$, together with the carbon atoms of the ring to which $X^1$ and $X^2$ bind, means a ring formed by an alkyleneoxy group or alkyleneoxyalkylene group having 2 or 3 carbon atoms binding to the carbon atoms of the azetidine ring in the general formula (1) as a condensed ring system. Specifically, the azetidine ring in the general formula (1) and the saturated heterocyclic ring form a substituent derived from, for example, 2-oxa-7-aza-bicyclo[3.2.0]heptane,
3-oxa-6-aza-bicyclo[3.2.0]heptane, 2-oxa-6-aza-bicyclo[3.2.0]heptane,
2-oxa-8-aza-bicyclo[4.2.0]octane, 3-oxa-8-aza-bicyclo[4.2.0]octane,
3-oxa-7-aza-bicyclo[4.2.0]octane, or 2-oxa-7-aza-bicyclo[4.2.0]octane.

The "4- to 6-membered saturated or unsaturated heterocyclic group which may be substituted" means a substituent derived from a 4- to 6-membered saturated or unsaturated heterocyclic compound containing one or more oxygen atoms, nitrogen atoms, or sulfur atoms as constituent atoms of the ring structure, which may have one or more substituents. This heterocyclic group may bind at any position. Examples of the 4- to 6-membered saturated or unsaturated heterocyclic group include, for example, a group derived from pyridine, pyrimidine, pyrrole, furan, thiophene, imidazole, pyrazole, oxazole, thiazole, isothiazole, oxadiazole, triazole, oxetane, tetrahydrofuran, or the like. When this heterocyclic group has a substituent, examples of the substituent include, for example, one or more halogen atoms, alkyl groups, amino groups, and alkoxy groups.

The "aralkyl group which may be substituted" means an aralkyl group which may have one or more substituents. Examples of the aralkyl group include, for example, benzyl group and phenethyl group. When the aralkyl group has a substituent, examples of the substituent include, for example, a halogen atom, hydroxyl group, an alkoxy group, amino group, and an alkyl group.

In an embodiment of the present invention, $X^1$ and $X^2$ preferably independently represent hydrogen atom, a halogen atom, hydroxyl group, cyano group, an alkoxy group which may be substituted, an alkyl group which may be substituted, a carbamoyl group which may be substituted, a carbamoyloxy group which may be substituted, an alkylsulfonyl group which may be substituted, or an amino group which may be substituted, or $X^1$ and $X^2$ together represent a =N-OY group (Y represents hydrogen atom or an alkyl group which may be substituted).

In another embodiment of the present invention, $X^1$ and $X^2$ more preferably independently represent hydrogen atom, a halogen atom, hydroxyl group, cyano group, a lower alkoxy group, a lower alkyl group, a hydroxy(lower alkyl) group, a (lower alkoxy)(lower alkyl) group, a halogenated lower alkyl group, a cycloalkyl group having 3 to 8 carbon atoms, a di(lower alkyl)carbamoyl group, carbamoyloxy group, a di(lower alkyl)carbamoyloxy group, a (lower alkyl) sulfonyl group, a N-(lower acyl)-N-(lower alkyl)amino group, a N-(lower alkyl)sulfonyl-N-(lower alkyl)amino group, a N-(lower alkoxy)carbonyl-N-(lower alkyl)amino group, or a N,N-di(lower alkyl)amino group, or $X^1$ and $X^2$ together represent a =N-O-(lower alkyl) group. The term "lower" used for the "lower alkoxy group", "lower alkyl group", "hydroxy (lower alkyl) group", and the like means that the number of carbon atoms of these groups is 1 to 6.

In another embodiment of the present invention, $X^1$ and $X^2$ still more preferably independently represent hydrogen atom, fluorine atom, hydroxyl group, cyano group, methoxy group, methyl group, ethyl group, hydroxymethyl group, methoxymethyl group, trifluoromethyl group, cyclopropyl group, dimethylcarbamoyl group, carbamoyloxy group, dimethylcarbamoyloxy group, methanesulfonyl group, N-acetyl-N-methylamino group, N-methanesulfonyl-N-methylamino group, N-methoxycarbonyl-N-methylamino group, or N,N-dimethylamino group, or $X^1$ and $X^2$ together represent =N-O-methyl group.

In an embodiment of the present invention, $R^1$ is preferably unsubstituted phenyl group: A phenyl group substituted with one or two fluorine atoms, chlorine atoms, methyl groups, or methoxy groups at the meta-position or meta-positions is also preferred.
In an embodiment of the present invention, $R^2$ is preferably an alkyl group which may be substituted, more preferably methyl group.

In an embodiment of the present invention, $R^3$ is preferably hydrogen atom, fluorine atom, or hydroxyl group, more preferably hydrogen atom.
In an embodiment of the present invention, $R^4$ is preferably hydrogen atom.
In an embodiment of the present invention, $Z^1$ is preferably a halogen atom or hydroxyl group, more preferably hydroxyl group.

In an embodiment of the present invention, $Z^2$ is preferably hydrogen atom, a halogen atom, or an alkyl group which may be substituted, more preferably hydrogen atom or methyl group.
In an embodiment of the present invention, examples of preferred combinations of $Z^1$ and $Z^2$ include, combination of hydroxyl group as $Z^1$ and hydrogen atom as $Z^2$, combination of hydroxyl group as $Z^1$ and methyl group as $Z^2$, and combination of fluorine atoms as $Z^1$ and $Z^2$.

In an embodiment of the present invention, $Z^3$ is preferably an alkyl group which may be substituted, or a 4- to 6-membered saturated or unsaturated heterocyclic group which may be substituted. $Z^3$ is more preferably an unsubstituted alkyl group, an alkyl group substituted with a halogen atom, unsubstituted pyrimidinyl group, unsubstituted pyridyl group, or a pyridyl group substituted with a halogen atom. $Z^3$ is still more preferably an alkyl group substituted with fluorine atom, unsubstituted pyrimidinyl group, or a pyridyl group substituted with fluorine atom.
In an embodiment of the present invention, $Z^4$ is preferably a phenyl group which may be substituted, or an alkoxy group which may be substituted.

The 6- and 7-positions of the partial structure represented by the following general formula (2)

[Formula 5]

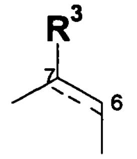

(2)

are meant to be optionally bound with a double bond (when the bond is a double bond, $R^3$ is hydrogen atom)
In an embodiment of the present invention, the dotted line moiety between the 6- and 7-positions is preferably a single bond.

In the present invention, the compound is preferably in the absolute configuration represented by the general formula (3).

[Formula 6]

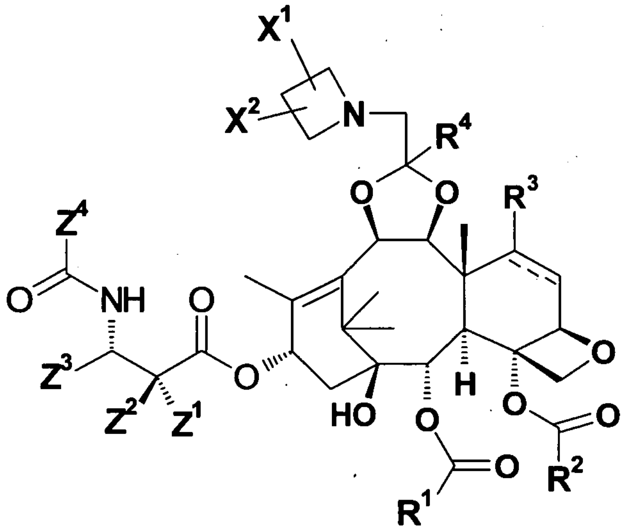

(3)

The configuration of the 3'-position to which the substituent $Z^3$ binds is more preferably the same configuration as that of natural paclitaxel. When the bond between the 6- and 7-positions is a single bond, the configuration of $R^3$ at the 7-position may be either of α - or β -configuration.

Stereoisomers or optical isomers based on asymmetric carbon atoms of the compounds of the present invention represented by the general formula (1) may exist, and any of these stereoisomers and optical isomers, and mixtures thereof fall within the scope of the present invention. Preferred examples of the compounds of the present invention represented by the general formula (1) include those in the absolute configuration represented by the general formula (3).

Salts of the compounds of the present invention represented by the general formula (1) are not particularly limited so long as they are medically acceptable salts, including acid addition salts or salts of carboxy group, for example, although the taxane derivatives of the present invention may be used in a free form.
Examples of the acid addition salts include inorganic acid salts such as hydrochlorides, sulfates, nitrates, hydrobromides, hydroiodides and phosphates, and organic acid salts such as acetates, methanesulfonates, benzenesulfonates, toluenesulfonates, citrates, maleates, fumarates, and lactates.

Examples of the salts of carboxy group include, for example, alkali metal salts such as lithium salts, sodium salts, and potassium salts, alkaline earth metal salts such as magnesium salts and calcium salts, ammonium salts, triethylamine salts, N-methylglucamine salts, tris(hydroxymethyl)aminomethane salts, and the like, and they may be inorganic salts or organic salts.

Methods for preparing the compounds represented by the general formula (3) will be explained below as typical examples of the compounds represented by the general formula (1). The compounds of the present invention can be prepared by various methods, and the preparation methods shown below are mere examples. Therefore, the present invention should not be construed to be limited to these examples.

When the reactions are performed, the reactions can be carried out with protecting substituents with appropriate protective groups as required, and types of the protective groups and the order of conversions of the substituents are not particularly limited.

[Preparation method 1]

[Formula 7]

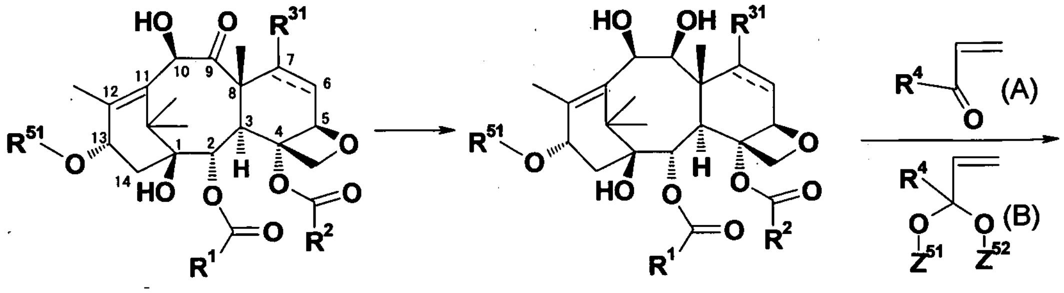


(4) (5)

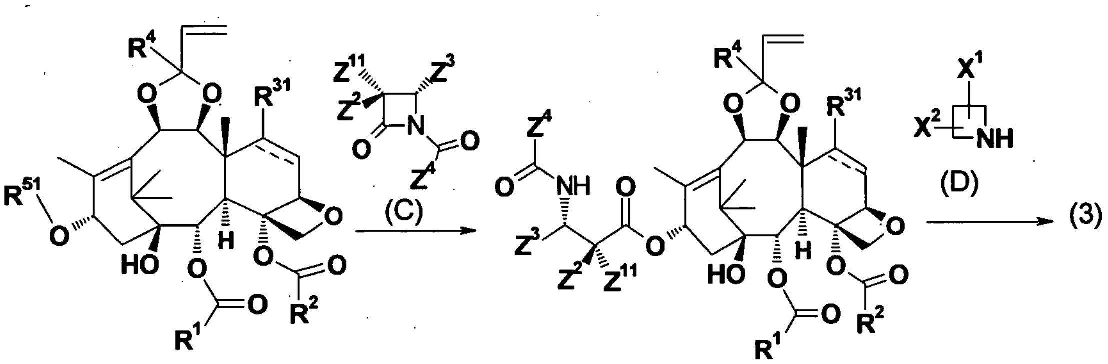


(6) (7)

[In the formulas, $R^{31}$ means $R^3$ or $R^3$ protected with a protective group (when $R^3$ is hydroxyl group). $R^{51}$ means hydrogen atom or a protective group of hydroxyl group. $Z^{51}$ and $Z^{52}$ mean an alkyl group, respectively. $Z^{11}$ means $Z^1$ or $Z^1$ protected with a protective group (when $Z^1$ is hydroxyl group). $X^1$, $X^2$, $R^1$ to $R^4$, and $Z^1$ to $Z^4$ have the same meanings as those defined above.]

Examples of the protective group of hydroxyl group include a silyl type protective group such as trimethylsilyl group, triethylsilyl group, triisopropylsilyl group and tert-butyldimethylsilyl group, 2,2,2-trichloroethoxycarbonyl group, benzyloxycarbonyl group, benzyl group, and the like.

First, the 9-oxo group of a compound represented by the formula (4) (henceforth referred to as the compound (4), and the compounds represented by the other formulas are also referred to in a similar manner) can be treated with diborane or a metal borohydride compound such as tetra-n-butylammonium borohydride in a solvent such as methylene chloride and tetrahydrofuran according to, for example, a method described in literature (Bioorg. Med. Chem. Lett., 2002, 12, 2815, or Japanese Patent Unexamined Publication No. 2003-342269) to obtain the compound (5) having 9 β-hydroxy group.

The compound (5) and the compound (A) or compound (B) can be reacted in a solvent such as methylene chloride, tetrahydrofuran, and 1,4-dioxane in the presence of an acidic catalyst such as (±)-camphor-10-sulfonic acid, p-toluenesulfonic acid or zinc chloride according to, for example, a method described in literature (Bioorg. Med. Chem. Lett., 2003, 13,185) to obtain the compound (6).

In order to obtain the compound (7) from the compound (6), when the substituent at the 13-position, $R^{51}$, is a protective group, deprotection can be first performed to obtain a compound having hydroxyl group at the 13-position, and then the compound can be condensed with the compound (C) as a racemate or optically active substance by using a base such as sodium hexamethyldisilazane, lithium hexamethyldisilazane and sodium hydride in a solvent such as ether and tetrahydrofuran according to, for example, a method described in literature (Tetrahedron, 1992, 48, 6985).

The compound (3) can be prepared from the compound (7) as follows. First, the double bond of the 9,10-propenylidenedioxy moiety of the compound (7) is treated with a catalytic amount of osmium tetroxide in a solvent (water, acetone, tetrahydrofuran, ether, pyridine, 1,4-dioxane, mixtures of these solvents, and the like) in the presence of an oxidizing agent such as 4-methylmorpholine N-oxide to obtain a diol compound, the diol compound is converted into a ketone or aldehyde by a treatment with sodium metaperiodate in a solvent (water, ether, tetrahydrofuran, methanol, ethanol, 1,4-dioxane, mixed solvent of these, and the like), and the ketone or aldehyde is treated together with an azetidine derivative (D) in a solvent (water, tetrahydrofuran, methanol, ethanol, 1,4-dioxane, methylene chloride, mixed solvent of these, and the like) in the presence of a reducing agent such as sodium borohydride, sodium cyanoborohydride, sodium triacetoxyborohydride, and lithium aluminum hydride to obtain the compound (3) of the present invention introduced with any one of various azetidino groups. By converting a substituent of the resulting compound (3) or performing deprotection for protective groups as required, another compound (3) of the present invention can be obtained.

The compounds of the present invention prepared as described above can be isolated and purified by known methods such as extraction, precipitation, fractionation chromatography, fractional crystallization, and recrystallization. Further, the compounds of the present invention can be derived into desired salts by subjecting the compounds to a usual salt formation reaction.

The compounds (3) of the present invention can also be prepared by the following method.

[Preparation method 2]

[Formula 8]

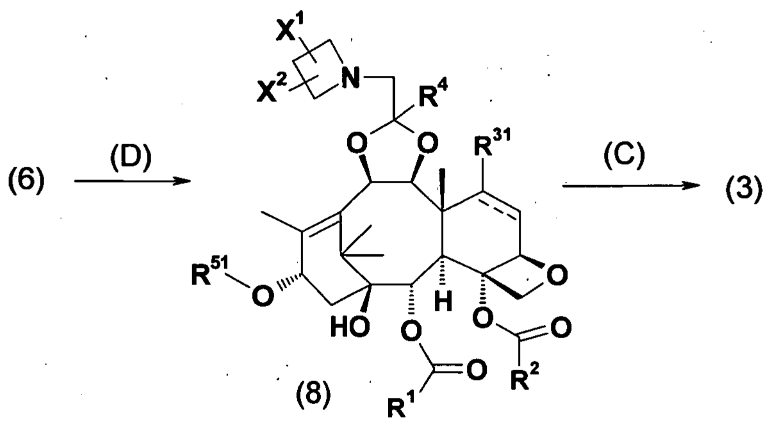

[In the formula, $X^1$, $X^2$, $R^1$, $R^2$, $R^{31}$, $R^4$, and $R^{51}$ have the same meanings as those defined above.]

The compound (8) can be obtained from the compound (6) of which substituent at the 13-position, i.e., $R^{51}$, is a protective group, by using reagents and conditions similar to those used for the method of converting the double bond of the 9,10-propenylidenedioxy moiety of the compound (7) into various azetidino groups of the compounds (3) described in Preparation method 1.

The compound (3) of the present invention can be obtained from the compound (8) by first performing deprotection when the substituent at the 13-position, $R^{51}$, is a protective group to obtain a compound having hydroxyl group at the 13-position, then converting the 13-position according to the method for preparing the compound (7) from the compound (6) described in Preparation method 1, and performing conversion of a substituent and deprotection for a protective group as required.

Conversion of $R^1$, $R^2$, or $R^{31}$ into any other substituents among those defined for the groups (for example, conversion of phenyl group into fluorophenyl group) can be performed by using a usual organic chemical method, and examples thereof are mentioned below.

The methods of converting $R^1$ include, for example, a method of selectively hydrolyzing the benzoyl group at the 2-position according to a method described in literature (Tetrahedron Lett., 1994, 35, 8931), and then acylating the resultant, and the compounds of which $R^1$ is a group other than phenyl can be obtained by this method.
The methods of converting $R^2$ include, for example, a method of selectively hydrolyzing the acetyl group at the 4-position according to a method described in literature (J. Med. Chem., 1995, 38, 2263), and then acylating the resultant, and the compounds of which $R^2$ is a group other than methyl can be obtained by this method.

The compound of which $R^{31}$ is hydrogen atom and the bond between the 6- and 7-positions is a double bond can be obtained by, for example, reacting the compound having hydroxyl group at the 7-position and a single bond between the 6- and 7-positions with trifluoromethanesulfonic acid anhydride in a solvent such as methylene chloride in the presence of a base such as N,N-dimethylaminopyridine, and then treating the resultant with a base such as 1,8-diazabicyclo[5.4.0]-7-undecene, according to the method described in WO2001/27115.

The compound of which $R^{31}$ is fluorine atom and the bond between the 6- and 7-positions is a single bond can be obtained by, for example, treating the compound having hydroxyl group at the 7-position and a single bond between the 6- and 7-positions with a fluorinating reagent such as (diethylamino)sulfur trifluoride in a solvent such as methylene chloride according to a method described in literature (Tetrahedron Lett., 1995, 36, 1609).

The compound of which $R^{31}$ is methoxy group and the bond between the 6- and 7-positions is a single bond

can be obtained by, for example, treating the compound having hydroxyl group at the 7-position and a single bond between the 6- and 7-positions with methyl trifluoromethanesulfonate in a basic solvent such as 2,6-di-tert-butylpyridine according to a method described in literature (Bioorg. Med. Chem. Lett., 2002, 12, 2815).

The compound of which $R^{31}$ is hydrogen atom and the bond between the 6- and 7-positions is a single bond can be obtained by, for example, treating the compound having hydroxyl group at the 7-position and a single bond between the 6- and 7-positions with 1,1'-thiocarbonyldiimidazole in a solvent such as tetrahydrofuran in the presence of a base such as 1,8-diazabicyclo[5.4.0]-7-undecene to derive the compound into the compound of which 7-hydroxyl group is (imidazolyl)thiocarbonylated, and treating the resultant with tributyltin hydride, tris(trimethylsilyl)silane hydride, or the like in an inert solvent such as tetrahydrofuran and 1,4-dioxane in the presence of a radical initiator such as 2',2'-azobis(isobutyronitrile) and benzoyl peroxide according to a method described in literature (Bioorg. Med. Chem. Lett., 2002, 12, 2815).

Further, the compound of which $R^{31}$ is hydrogen atom and the bond between the 6- and 7-positions is a single bond can also be obtained by, for example, hydrogenating the compound of which $R^{31}$ is hydrogen atom and bond between the 6- and 7-positions is a double bond by catalytic hydrogenation in a solvent such as tetrahydrofuran, 1,4-dioxane, methanol and ethanol in the presence of a catalyst such as rhodium/alumina, palladium hydroxide/carbon, palladium/carbon, and platinum oxide according to a method described in literature (Chem. Pharm. Bull., 2002, 50, 1398).

The compound (4) as the starting material for the preparation may be a marketed product, 10-deacetyl baccatin III, or can be synthesized from 10-deacetyl baccatin III. For example, the compound wherein $R^{31}$ is fluorine atom, and $R^{51}$ is triethylsilyl group, and the compound wherein $R^{31}$ and $R^{51}$ are hydrogen atoms are described in literature (Bioorg. Med. Chem. Lett., 2002, 12, 2815).

The compound (A) and the compound (B) as the starting materials for the preparation may be marketed products, or can be synthesized according to a method described in literature (J. Am. Chem. Soc., 1938, 60, 1160 or Tetrahedron Lett., 1976, 17, 2935).

As for the general synthetic method of racemic or optically active compound (C) as the starting material for the preparation, the compound can be synthesized according to a method described in literature (Tetrahedron Lett., 1993, 34, 4149).

The compound (D) as the starting material for the preparation may be a marketed product, or can be synthesized according to the method described in the reference examples.

The compounds of the present invention can be used for therapeutic treatment of various cancers such as lung cancer, gastrointestinal cancer, ovarian cancer, uterine cancer, breast cancer, hepatic cancer, head and neck cancer, blood cancer, renal cancer, and testicular tumor. In particular, the compounds of the present invention are expected to be effective against cancers of the digestive system, e.g., colon cancer, which are hardly treated therapeutically by using conventional anticancer agents.

As causes involved in acquisition of drug resistance, various causes have been elucidated. In the case of anticancer agents, an example of the causes includes expression of P-glycoprotein (P-gp) having an action of extracellularly discharging drugs. For example, it has been reported that anticancer agents such as paclitaxel and docetaxel are likely to be affected by P-gp.

Pharmaceutical compositions containing the compounds of the present invention can be administered as various injections such as intravenous injection, intramuscular injection and subcutaneous injection, or by various methods such as oral administration and dermal administration. Among these administration methods, intravenous administration of an aqueous preparation and oral administration are preferred. The aqueous preparations can be prepared by forming an acid addition product with a pharmacologically acceptable acid, or a salt with an alkali metal such as sodium. In the case of oral administration, they may be used in the form of a free compound or a salt.

As for methods for manufacturing the preparations, the preparations can be manufactured according to ordinarily applied methods for preparing various pharmaceutical preparations by choosing a suitable form depending on a method for administration. Among dosage forms of the antitumor agent of the present invention, examples of oral preparations include, for example, tablet, powder, granule, capsule, solution, syrup, elixir, oily or aqueous suspension, and the like. In the case of injections, stabilizers, preservatives, dissolving aids, and the like may be used in the preparations. A solution which optionally contains such auxiliary agents may be stored in a container and made into solid preparation by lyophilization or the like to obtain a formulation to be prepared just before use. A unit dosage for single administration may be packed in a single container or units for several administrations may be packed in a single container.

Examples of the solid preparations include, for example, tablets, capsules, granules, pills, troches, and powders. Such solid preparations may also contain pharmaceutically acceptable additives together with the compounds of the present invention. Examples of the additives include, for example, fillers, bulking agents, binders, disintegrating agents, dissolution enhancers, wetting agents, and lubricants, and these additives can be chosen and mixed as required to form the preparations.

Examples of liquid preparations include solutions, elixirs, syrups, suspensions, emulsions, and the like. Such liquid preparations may also contain pharmaceutically acceptable additives together with the compounds of the present invention. Examples of the additives include, for example, suspending agents and emulsifiers, and these additives can

be chosen and mixed as required to form the preparations.

The compounds of the present invention can be used for therapeutic treatment of cancers of mammals, especially humans. Doses and administration intervals may be suitably chosen by medical practitioners depending on a site of the disease, body height and weight, sexuality, or pathological history of a patient. When the compounds are administered to humans, they are preferably administered once or 2 to 4 times a day as divided portions, and the administration is preferably repeated with appropriate intervals. Doses are preferably in a range of about 0.5 to 500 mg, preferably about 1 to 300 mg, more preferably about 1 to 100 mg, per 1 $m^2$ of body surface area for a single administration. A daily dose may exceed the aforementioned doses, if necessary, as determined by medical practitioners.

Examples

The present invention will be specifically explained with reference to the following examples. However, the present invention is not limited to these examples, and these examples should not be construed in any limitative manner. Reagents, solvents and starting materials mentioned in the specification without particular explanations can be easily obtained from commercial supply sources.

[Reference Example 1]

3-Methoxyazetidine hydrochloride

[Formula 9]

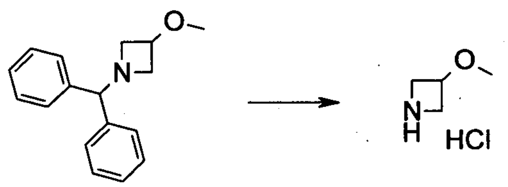

1-Benzhydryl-3-methoxyazetidine (J. Org. Chem., 1972, 37, 3953, 1.10 g) was dissolved in ethanol (40 ml), the solution was added with 20% palladium hydroxide/carbon (1.1 g), and catalytic reduction was performed for 17 hours under a hydrogen atmosphere. The catalyst was removed by filtration, and the reaction mixture was added with 1 N hydrochloric acid in ethanol (4.5 ml), concentrated, and then added with ether. The deposited solid was collected by filtration, and dried to obtain the title compound (480 mg) as colorless solid.

$^1$H-NMR (400MHz, $d_6$-DMSO) $\delta$ : 2.17 (3H, s), 3.75-3.79 (2H, m), 4.06-4.11 (2H, m), 4.21-4.27 (1H, m), 9.28 (2H, br).
ESI-MS; m/z: 88 $(M+H)^+$.

[Reference Example 2]

[Formula 10]

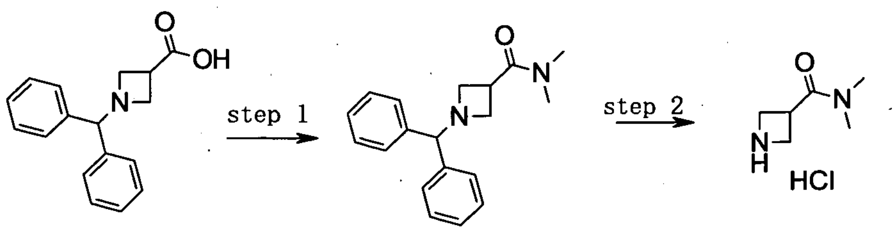


Step 1: 1-Benzhydryl-N,N-dimethyl-3-azetidinecarboxamide

1-Benzhydryl-3-azetidinecarboxylic acid (1.17 g), dimethylamine (2 mole solution in tetrahydrofuran, 2.6 ml),

triethylamine (0.67 ml) and
1-hydroxybenzotriazole (300 mg) were dissolved in dichloromethane (25 ml) and cooled on ice. Then, the solution was added with
1-ethyl-3-(3-dimethylaminopropyl)carbodiimide hydrochloride (925 mg), and the mixture was returned to room temperature, and stirred for 6 hours. The reaction mixture was added with saturated aqueous sodium hydrogencarbonate, and extracted with ethyl acetate. The organic layer was successively washed with water and saturated brine, and dried over anhydrous sodium sulfate, and the solvent was evaporated under reduced pressure. The resulting residue was purified by silica gel chromatography [developing solvent; ethyl acetate:n-hexane=2:1 (v/v)] to obtain the title compound (1.10 g) as colorless solid.
$^1$H-NMR (400MHz, $CDCl_3$) δ : 2.85 (3H, s), 2.92 (3H, s), 3.23-3.29 (2H, m), 3.46-3.48 (3H, m), 4.38 (1H, s), 7.16-7.41 (10H, m).

Step 2: N,N-Dimethyl-3-azetidinecarboxamide hydrochloride

The compound obtained in Step 1 mentioned above was used as a starting material to perform the same procedure as that-of Reference Example 1 and thereby to obtain the title compound as colorless solid.
$^1$H-NMR (400MHz, $d_6$-DMSO) δ : 2.82 (3H, s), 2.84 (3H, s), 3.85-3.94 (1H, m), 4.03-4.05 (4H, m), 9.10 (1H, br), 9.60 (1H, br).
ESI-MS; m/z: 129 $(M+H)^+$.

[Reference Example 3]

3-Methyl-3-azetidinol hydrochloride

[Formula 11]

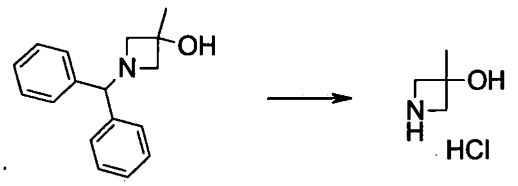

1-Benzhydryl-3-methyl-3-azetidinol (Tetrahedron Lett., 1996, 37, 1297) was used as a starting material to perform the same procedure as that of Reference Example 1 and thereby to obtain the title compound as colorless solid.
$^1$H-NMR (400MHz, $d_6$-DMSO) δ : 1.43 (3H, s), 3.71 (2H, d, J=11.2Hz), 3.83 (2H, d, J=11.2Hz), 6.11 (1H, s), 9.38 (2H, br).
ESI-MS; m/z: 88 $(M+H)^+$.

[Reference Example 4]

[Formula 12]

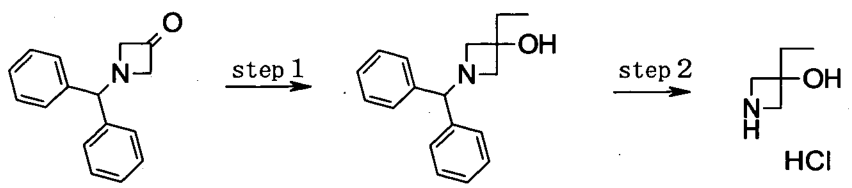


Step 1: 1-Benzhydryl-3-ethyl-3-azetidinol

A solution of ethyl lithium [0.5 mole solution in benzene:cyclohexane (90:10), 40 ml] was added dropwise with

an ether solution (6 ml) of 1-benzhydrylazetidin-3-one (J. Heterocycl. Chem., 1994, 31, 271, 2.37 g) at room temperature under a nitrogen atmosphere, and the mixture was refluxed for 0.5 hour by heating. The reaction mixture was cooled on ice, and then added with water, and the mixture was stirred for about 5 minutes. The reaction mixture was added with ether, the organic layer was dried over anhydrous sodium sulfate, and the solvent was evaporated under reduced pressure. The resulting residue was purified by silica gel chromatography [developing solvent; ethyl acetate:n-hexane=1: 4 (v/v)] to obtain the title compound (2.67 g) as colorless oil.
$^1$H-NMR (400MHz, $CDCl_3$) δ : 0.96 (3H, t, J=7.3Hz), 1.80 (2H, q , J=7.3Hz), 1.86 (1H, br), 2.94 (2H, d, J=8.0Hz), 3.21 (2H, d, J=8.0Hz), 4.12 (1H, d, J=7.0Hz), 7.15-7.42 (10H, m).

Step 2: 3-Ethyl-3-azetidinol hydrochloride

The compound obtained in Step 1 mentioned above was used as a starting material to perform the same procedure as that of Reference Example 1 and thereby to obtain the title compound as colorless solid.
$^1$ H-NMR (400MHz, $d_6$ -DMSO) δ : 0.84 (3H, t, J=7.3Hz), 1.75 (2H, q , J=7.3Hz), 3.72-3.74 (2H, m), 3.77-3.81 (2H, m), 6.04 (1H, brs), 9.22 (1H, br), 9.47 (1H, br).
ESI-MS; m/z: 102 $(M+H)^+$.

[Reference Example 5]

[Formula 13]

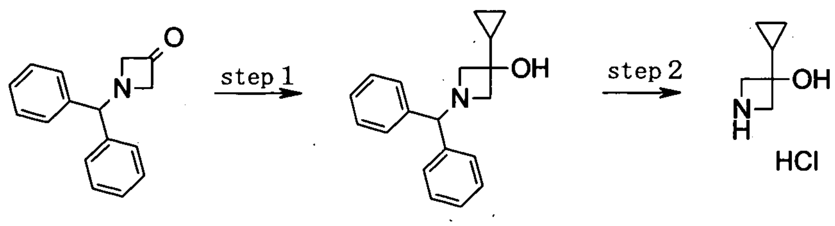


Step 1: 1-Benzhydryl-3-cyclopropyl-3-azetidinol

Metal magnesium (1.03 g) was suspended in tetrahydrofuran (30 ml), the suspension was added with a catalytic amount of iodine (20 mg), and the atmosphere was purged with nitrogen. Then, the reaction mixture was slowly added dropwise with bromocyclopropane (3.50 ml), and then further stirred for 30 minutes. The reaction mixture was cooled to -78°C, then added dropwise with a solution of 1-benzhydrylazetidin-3-one (2.00 g) in tetrahydrofuran (10 ml), and stirred for 1 hour. The reaction mixture was cooled on ice, then added with saturated aqueous sodium hydrogencarbonate, and extracted with ethyl acetate. The organic layer was successively washed with water and saturated brine, and then dried over anhydrous sodium sulfate, and the solvent was evaporated under reduced pressure. The resulting residue was purified by silica gel chromatography [developing solvent; ethyl acetate:n-hexane=1: 2 (v/v)] to obtain the title compound (2.20 g) as colorless oil.
$^1$H-NMR (400MHz, $CDCl_3$) δ : 0.45-0.51 (2H, m), 0.52-0.58 (2H, m), 1.17-1.27 (1H, m), 1.98 (1H, br), 2.92 (2H, d, J=8.8Hz), 3.09 (2H, d, J=8.8Hz), 4.34 (1H, s), 7.15-7.41 (10H, m).

Step 2: 3-Cyclopropyl-3-azetidinol hydrochloride

The compound obtained in Step 1 mentioned above was used as a starting material to perform the same procedure as that of Reference Example 1 and thereby to obtain the title compound as colorless solid.
$^1$H-NMR (400MHz, $d_6$-DMSO) δ : 0.41 (4H, d, J=6.8Hz), 1.25-1.32 (1H, m), 3.70-3.81 (4H, m), 6.04 (1H, br), 9.31 (2H, br).
ESI-MS; m/z: 114 $(M+H)^+$.

[Reference Example 6]

[Formulà 14]

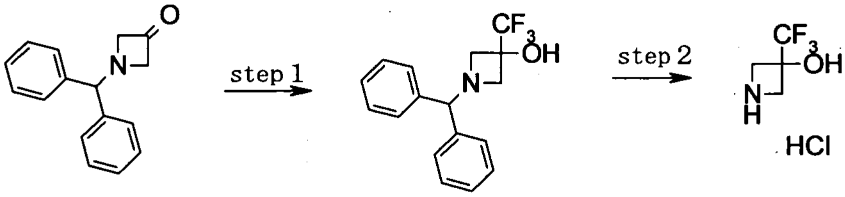


Step 1: 1-Benzhydryl-3-trifluoromethyl-3-azetidinol

A solution of 1-benzhydrylazetidin-3-one (2.00 g) in tetrahydrofuran (10 ml) was cooled on ice, added with (trifluoromethyl)trimethylsilane (1.50 ml) under a nitrogen atmosphere, and added with tetra-n-butylammonium fluoride (1 mole solution in tetrahydrofuran, 0.10 ml). After 10 minutes, the reaction mixture was added dropwise with tetra-n-butylammonium fluoride (1 mole solution in tetrahydrofuran, 8.50 ml), returned to room temperature, and stirred for 15 minutes. The reaction mixture was diluted with ethyl acetate, the organic layer was successively washed with water (2 times) and saturated brine, and then dried over anhydrous sodium sulfate, and the solvent was evaporated under reduced pressure. The resulting residue was purified by silica gel chromatography [developing solvent; ethyl acetate:n-hexane=1:4 (v/v)] to obtain the title compound (2.07 g) as pale yellow oil.
$^1$H-NMR (400MHz, $CDCl_3$) δ : 2.66 (1H, br), 3.19 (2H, d, J=9.3Hz), 3.53 (2H, d, J=9.3Hz), 4.45 (1H, s), 7.17-7.43 (10H, m).

Step 2: 3-Trifluoromethyl-3-azetidinol hydrochloride

The compound obtained in Step 1 mentioned above was used as a starting material to perform the same procedure as that of Reference Example 1 and thereby to obtain the title compound as colorless solid.
$^1$H-NMR (400MHz, $d_6$ -DMSO) δ : 4.05 (2H, d, J=1.5Hz), 4.08 (2H, d, J=1.5Hz), 7.99 (1H, s), 9.80 (2H, br).
ESI-MS; m/z: 142 $(M+H)^+$.

[Reference Example 7]

3-Azetidinecarbonitrile hydrochloride

[Formula 15]

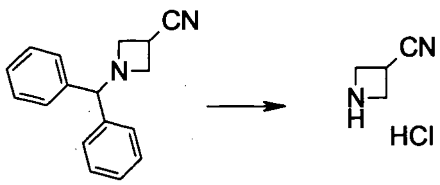

1,2-Dichloroethane (16 ml) was added with 1-benzhydrylazetidine-3-carbonitrile (1.24 g), and added with 1-chloroethyl chloroformate (0.82 ml) at room temperature, and the mixture was refluxed for 0.5 hour by heating. The reaction mixture was concentrated, then the resulting residue was added with methanol (16 ml), and the mixture was refluxed for 1 hour by heating, and left to cool. The mixture was concentrated again, and then the resulting residue was added with ethyl acetate, and the deposited solid was collected by filtration, and dried to obtain the title compound (360 mg) as colorless solid.
$^1$H-NMR (400MHz, $d_6$-DMSO) δ : 3.94-4.02 (1H, m), 4.14 (2H, s), 4.16 (2H, s), 9.61 (2H, br).
EI-MS; m/z: 82 $(M^+)$.

[Reference Example 8]

3-Azetidinemethanol hydrochloride

[Formula 16]

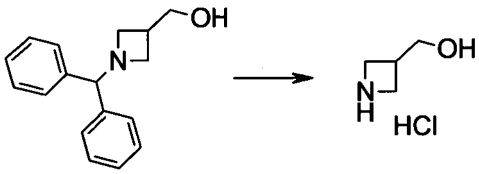

(1-Benzhydryl-3-azetidino)methanol was used as a starting material to perform the same procedure as that of Reference Example 1 and thereby to obtain the title compound as colorless solid.
$^1$H-NMR (400MHz, $d_6$ -DMSO) δ : 2.78-2.87 (1H, m), 3.51 (2H, t, J=5.4Hz), 3.70-3.74 (2H, m), 3.90 (2H, t, J=9.5Hz), 5.69 (1H, t, J=5:4Hz), 9.13 (2H, br).
ESI-MS; m/z: 88 $(M+H)^+$.

[Reference Example 9]

[Formula 17]

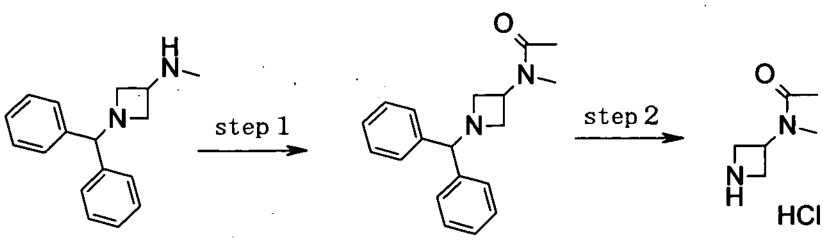


Step 1: N-(1-Benzhydryl-3-azetidino)-N-methylacetamide

1-Benzhydryl-3-azetidinomethylamine (J. Med. Chem., 1993, 36, 801, 1.50 g) and triethylamine (0.92 ml) were dissolved in dichloromethane (30 ml), and the solution was added with acetic anhydride (0.62 ml), and stirred at room temperature for 20 hours. The reaction mixture was concentrated, and then the resulting residue was diluted with ethyl acetate. The organic layer was successively washed with water and saturated brine, and then dried over anhydrous sodium sulfate, and the solvent was evaporated under reduced pressure. The resulting residue was added with ether, and the deposited solid was collected by filtration, and dried to obtain the title compound (1.42 g) as colorless solid.
$^1$H-NMR (400MHz, $CDCl_3$) δ : 2.03, 2.07 (total 3H, each s), 3.02, 3.11 (total 3H, each s), 3.05, 3.13 (total 2H, each t, J=7.3Hz), 3.47 (2H, t, J=7.0Hz), 4.34, 4.35 (total 1H, each s), 4.45-4.52, 4.87-4.93 (total1H each m), 7.16-7.41 (10H, m).

Step 2: N-(3-Azetidino)-N-methylacetamide hydrochloride

The compound obtained in Step 1 mentioned above was used as a starting material to perform the same procedure as that of Reference Example 1 and thereby to obtain the title compound as colorless solid.
$^1$ H-NMR (400MHz, $d_6$ -DMSO) δ : 2.01 (3H, s), 3.00 (3H, s), 3.97-4.15 (4H, m), 4.94-5.02 (1H, m), 9.21 (2H, br).
ESI-MS; m/z: 129 $(M+H)^+$.

[Reference Example 10]

[Formula 18]

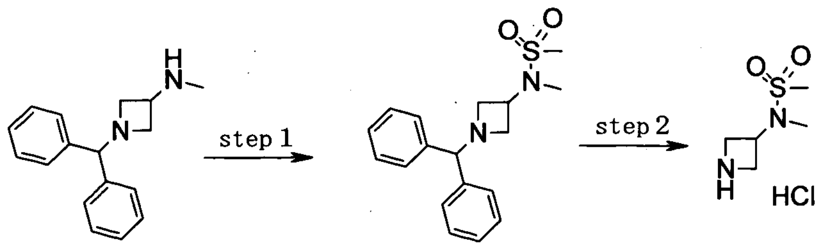


Step 1: N-(1-Benzhydryl-3-azetidino)-N-methylmethanesulfonamide

1-Benzhydryl-3-azetidinomethylamine (1.50 g) and triethylamine (0.92 ml) were dissolved in dichloromethane (30 ml), and the solution was cooled on ice. Then, the solution was added with methanesulfonyl chloride (0.51 ml), and stirred for 1 hour. The reaction mixture was concentrated, and then the resulting residue was diluted with ethyl acetate. The organic layer was successively washed with water and saturated brine, and then dried over anhydrous sodium sulfate, and the solvent was evaporated under reduced pressure. The resulting residue was added with ether, and the deposited solid was collected by filtration, and dried to obtain the title compound (1.55 g) as colorless solid.
$^1$ H-NMR (400MHz, $CDCl_3$) δ : 2.72 (3H, s), 2.90 (3H, s), 3.16 (2H, br), 3.49 (2H, br), 4.23-4.37 (1H, m), 4.37 (1H, br), 7.18-7.41 (10H, m).

Step 2: N-(3-Azetidino)-N-methylmethanesulfonamide hydrochloride

The compound obtained in Step 1 mentioned above was used as a starting material to perform the same procedure as that of Reference Example 7 and thereby to obtain the title compound as colorless solid.
$^1$H-NMR (400MHz, $d_6$-DMSO) δ : 2.87 (3H, s), 2.93 (3H, s), 4.04-4.12 (2H, m), 4.15-4.21 (2H, m), 4.60-4.69 (1H, m), 9.23 (2H, br).
ESI-MS; m/z: 165 $(M+H)^+$.

[Reference Example 11]

[Formula 19]

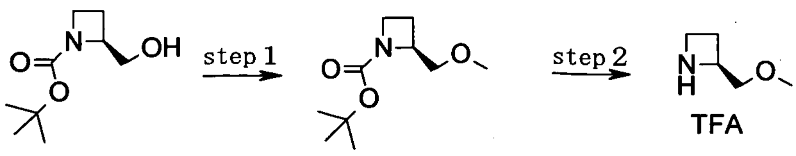


Step 1: (S)-2-(Methoxymethyl)-1-azetidinecarboxylic acid tert-butyl ester

60% Sodium hydride (240 mg) was suspended in N,N-dimethylformamide (12 ml), and the suspension was cooled on ice under a nitrogen atmosphere. The suspension was added dropwise with a solution of (2S)-2-(hydroxymethyl)-1-azetidinecarboxylic acid tert-butyl ester (936 mg) dissolved in N,N-dimethylformamide (6 ml), and then stirred for 20 minutes. Then, the reaction mixture was added with methyl iodide (0.40 ml), returned to room temperature, and stirred

overnight. The reaction mixture was added with cooled saturated aqueous ammonium chloride, and extracted with ethyl acetate. The organic layer was washed with saturated brine, and dried over anhydrous sodium sulfate, and then the solvent was evaporated under reduced pressure. The resulting residue was purified by silica gel chromatography [developing solvent; ethyl acetate:n-hexane=1:4 (v/v)] to obtain the title compound (910 mg) as colorless oil.
$^1$ H-NMR (400MHz, $CDCl_3$) δ : 1.44 (9H, s), 2.11-2.27 (2H, m), 3.41 (3H, s), 3.54 (1H, dd, J=10, 3.2Hz), 3.65 (1H, dd, J=10, 5.4Hz), 3.81 (2H, dd, J=8.2, 6.9Hz), 4.27-4.33 (1H, m).

Step 2: (S)-2-(Methoxymethyl)azetidine trifluoroacetate

The compound obtained in Step 1 mentioned above (905 mg) was dissolved in dichloromethane (4 ml), and the solution was added with trifluoroacetic acid (TFA, 4 ml) under ice cooling, returned to room temperature, and stirred for 1 hour. The reaction mixture was concentrated to obtain the title compound (970 mg) as colorless oil.
$^1$H-NMR (400MHz, $d_6$ -DMSO) δ : 2.21-2.30 (1H, m), 2.34-2.40 (1H, m), 3.34 (3H, s), 3.53 (1H, dd, J=11.3, 3.5Hz), 3.65 (1H, dd, J=11.3, 6.9Hz), 3.70-3.77 (1H, m), 3.84-3.93 (1H, m), 4.50 (1H, br), 8.72 (2H, br).
ESI-MS; m/z: 102 $(M+H)^+$.

[Reference Example 12]

[Formula 20]

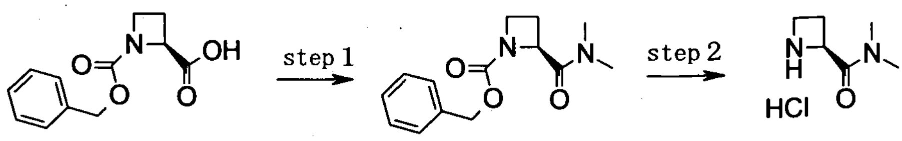


Step 1: (2S)-2-(Dimethylcarbamoyl)-1-azetidinecarboxylic acid benzyl ester

(2S)-1-[(Benzyloxy)carbonyl]-2-azetidinecarboxylic acid (Tetrahedron, 1993, 49, 8211) was used as a starting material to perform the same procedure as that of Reference Example 2, Step 1 and thereby to obtain the title compound as colorless oil. $^1$H-NMR (400MHz, $CDCl_3$) δ : 2.20-2.27 (1H, m), 2.45-2.54 (1H, m), 2.97 (6H, brs), 3.92-3.98 (1H, m), 4.11-4.17 (1H, m), 5.00-5.03 (1H, m), 5.09 (2H, brs), 7.27-7.35 (5H, m).

Step 2: (2S)-N,N-Dimethyl-2-azetidinecarboxamide hydrochloride

The compound obtained in Step 1 mentioned above was used as a starting material to perform the same procedure as that of Reference Example 1 and thereby to obtain the title compound as colorless solid.
$^1$ H-NMR (400MHz, $d_6$ -DMSO) δ : 2.39-2.50 (1H, m), 2.70-2.79 (1H, m), 2.83 (3H, s), 2.90 (3H, s), 3.66-3.72 (1H, m), 3.88-3.93 (1H, m), 5.27 (1H, t, J=8.5Hz), 8.68 (1H, br), 10.29 (1H, br).
ESI-MS; m/z: 129 $(M+H)^+$.

[Reference Example 13]

[Formula 21]

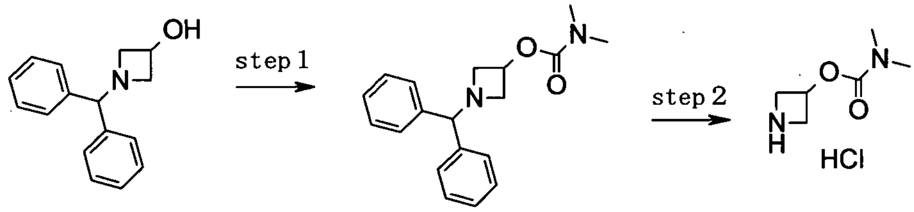


Step 1: 1-Benzhydryl-3-azetidino dimethylcarbamate

60% Sodium hydride (480 mg) was suspended in N,N-dimethylformamide (5 ml), and the suspension was cooled on ice. The suspension was added dropwise with a solution of 1-benzhydrylazetidin-3-ol (2.39 g) dissolved in tetrahydrofuran (20 ml), and then the mixture was stirred for 30 minutes. Then, the mixture was added with N,N-dimethylcarbamoyl chloride (1.1 ml), returned to room temperature, and stirred overnight. The reaction mixture was added with cooled saturated aqueous ammonium chloride, and extracted with ethyl acetate. The organic layer was washed with saturated aqueous sodium hydrogencarbonate and saturated brine, and dried over anhydrous sodium sulfate, and then the solvent was evaporated under reduced pressure. The resulting residue was added with ether/n-hexane, and the deposited solid was collected by filtration, and dried to obtain the title compound (2.40 g) as colorless solid.
$^1$H-NMR (400MHz, $CDCl_3$) δ : 2.88 (3H, brs), 2.90 (3H, brs), 2.98-3.02 (2H, m), 3.58-3.62 (2H, m), 4.37 (1H, s), 5.00-5.06 (1H, m), 7.16-7.29 (10H, m).

Step 2: 3-Azetidino dimethylcarbamate hydrochloride

The compound obtained in Step 1 mentioned above was used as a starting material to perform the same procedure as that of Reference Example 1 and thereby to obtain the title compound as colorless solid.
$^1$ H-NMR (400MHz, $d_6$ -DMSO) δ : 2.82 (3H, s), 2.90 (3H, s), 3.92-3.95 (2H, m), 4.17-4.22 (2H, m), 5.04-5.09 (1H, m), 9.37 (1H, br).
ESI-MS; m/z: 145 $(M+H)^+$.

[Reference Example 14]

[Formula 22]

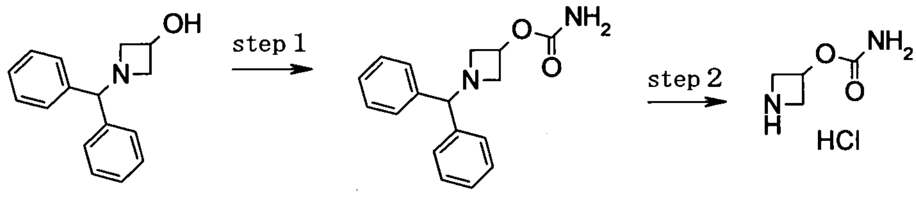


Step 1: 1-Benzhydryl-3-azetidino carbamate

1-Benzhydrylazetidin-3-ol (2.39 g) was dissolved in ethyl acetate (50 ml) and cooled on ice. Then, the solution was added with trichloroacetyl isocyanate (1.25 ml), and stirred at the same temperature for 1 hour, and then the reaction mixture was concentrated under reduced pressure. The resulting residue was dissolved in methanol (50 ml), and the solution was added with water (4 ml) and sodium formate (816 mg), and stirred for 24 hours at room temperature. The reaction mixture was concentrated, and then diluted with ethyl acetate. The reaction mixture was washed with saturated aqueous sodium hydrogencarbonate and saturated brine, and dried over anhydrous sodium sulfate, and then the solvent was evaporated under reduced pressure. The resulting residue was added with ether, and the deposited solid was collected by filtration, and dried to obtain the title compound (1.75 g) as colorless solid. $^1$H-NMR (400MHz, $CDCl_3$) δ : 3.05 (2H, t, J=8.0Hz), 3.57 (2H, t, J=8.0Hz), 4.37 (1H, s), 4.61 (2H, br), 5.01-5.08 (1H, m), 7.16-7.40 (10H, m).

Step 2: 3-Azetidino carbamate hydrochloride

The compound obtained in Step 1 mentioned above was used as a starting material to perform the same procedure as that of Reference Example 1 and thereby to obtain the title compound as colorless solid.
$^1$H-NMR (400MHz, $d_6$-DMSO) δ : 3.86-3.90 (2H, m), 4.16-4.21 (2H, m), 5.01-5.06 (1H, m), 6.77 (1H, br), 6.93 (1H, br), 9.23 (2H, br).
ESI-MS; m/z: 117 $(M+H)^+$.

[Reference Example 15]

[Formula 23]

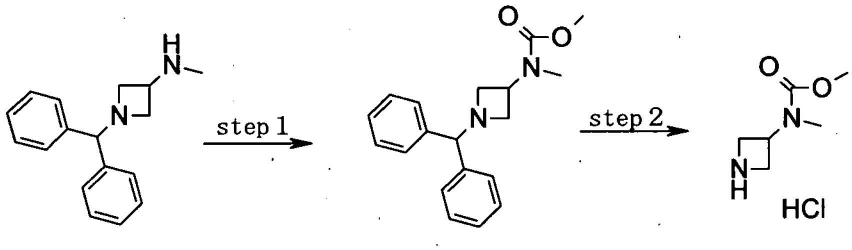


Step 1: Methyl 1-benzhydryl-3-azetidino(methyl)carbamate

1-Benzhydryl-3-azetidinomethylamine (1.50 g) was dissolved in dichloromethane (25 ml), and the solution was added with triethylamine (1.0 ml) and cooled on ice. Then, the reaction mixture was added dropwise with methyl chloroformate (0.53 ml), returned to room temperature, and stirred overnight. The reaction mixture was concentrated, and then the resulting residue was diluted with ethyl acetate. The organic layer was successively washed with water, saturated aqueous sodium hydrogencarbonate and saturated brine, and then dried over anhydrous sodium sulfate, and the solvent was evaporated under reduced pressure. The resulting residue was added with ether, and the deposited solid was collected by filtration, and dried to obtain the title compound (1.52 g) as colorless solid.
$^1$H-NMR (400MHz, $CDCl_3$) δ : 2.95 (3H, s), 3.02 (2H, brs), 3.42-3.46 (2H, m), 3.65 (3H, s), 4.32 (1H, s), 4.49 (1H, br), 7.16-7.41 (10H, m).

Step 2: Methyl 3-azetidino(methyl)carbamate hydrochloride

The compound obtained in Step 1 mentioned above was used as a starting material to perform the same procedure as that of Reference Example 1 and thereby to obtain the title compound as colorless solid.
$^1$H-NMR (400MHz, $d_6$-DMSO) δ : 2.90 (3H, s), 3.62 (3H, s), 4.01 (2H, brs), 4.13 (2H, brs), 4.81-4.87 (1H, m), 9.12 (2H, br).
ESI-MS; m/z: 145 $(M+H)^+$.

[Reference Example 16]

[Formula 24]

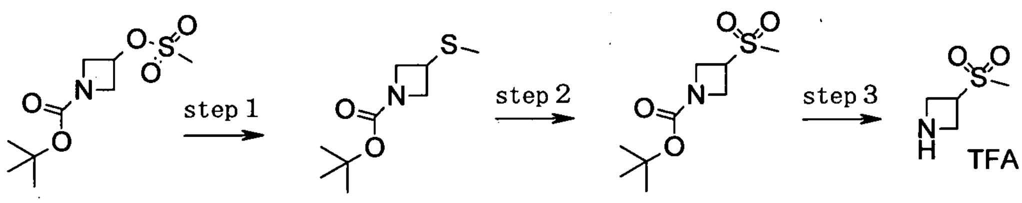

Step 1: 3-(Methylsulfanyl)-1-azetidinecarboxylic acid tert-butyl ester

3-[(Methanesulfonyl)oxy]-1-azetidinecarboxylic acid tert-butyl ester (J. Med. Chem., 2001, 44, 94, 2.60 g) was dissolved in N,N-dimethylformamide (16 ml), and the solution was added with sodium thiomethoxide (1.45 g), and stirred at 80°C for 20 hours under a nitrogen atmosphere. The reaction mixture was left to cool, then poured into ice water, and extracted with ethyl acetate. The organic layer was successively washed with water and saturated brine, and then dried over anhydrous sodium sulfate, and the solvent was evaporated under reduced pressure. The resulting residue was purified by silica gel chromatography [developing solvent; ethyl acetate:n-hexane=1:4 (v/v)] to obtain the title compound (2.04 g) as colorless oil.
$^1$H-NMR (400MHz, $CDCl_3$) δ : 1.44 (9H, s), 2.11 (3H, s), 3.53-3.60 (1H, m), 3.82 (2H, dd, J=9.0, 5.5Hz), 4.23 (2H, dd, J=9.0, 8.1Hz).

Step 2: 3-(Methanesulfonyl)-1-azetidinecarboxylic acid tert-butyl ester

The compound obtained in Step 1 mentioned above (2.04 g) was dissolved in methanol (20 ml), and the solution was cooled on ice. Then, the solution was slowly added with a solution of oxone (12.3 g) dissolved in water (12.4 ml), and stirred for 4 hours at the same temperature. The reaction mixture was diluted with water, and then extracted 4 times with ethyl acetate. The organic layer was dried over anhydrous sodium sulfate, and the solvent was evaporated under reduced pressure. The resulting residue was added with ethyl acetate/n-hexane, and the deposited solid was collected by filtration, and dried to obtain the title compound (1.85 g) as colorless solid.
$^1$H-NMR (400MHz, $CDCl_3$) δ : 1.45 (9H, s), 2.90 (3H, s), 3.86-3.93 (1H, m), 4.19-4.29 (4H, m).

Step 3: 3-(Methanesulfonyl)azetidine trifluoroacetate

The compound obtained in Step 1 mentioned above was used as a starting material to perform the same procedure as that of Reference Example 12, Step 2 and thereby to obtain the title compound as colorless solid.
$^1$H-NMR (400MHz, $d_6$-DMSO) δ : 3.09 (3H, s), 4.15-4.20 (2H, m), 4.27-4.33 (2H, m), 4.49-4.57 (1H, m), 9.41 (2H, br).
ESI-MS; m/z: 136 $(M+H)^+$.

[Reference Example 17]

[Formula 25]

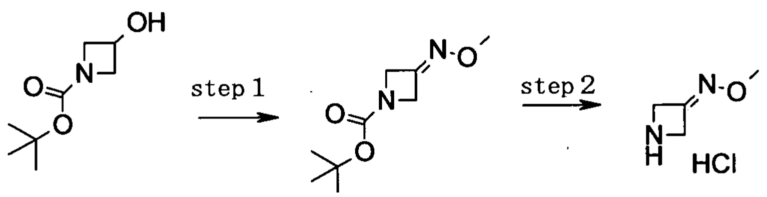


Step 1: 3-(Methoxyimino)-1-azetidinecarboxylic acid tert-butyl ester

3-Hydroxy-1-azetidinecarboxylic acid tert-butyl ester (1.50 g) was dissolved in dichloromethane (15 ml), and the solution was added dropwise with a solution of Dess-Martin reagent (5.51 g) in dichloromethane (60 ml), and then further stirred for 3 hours. The reaction mixture was added with saturated aqueous sodium hydrogencarbonate and sodium thiosulfate pentahydrate (10 g), and vigorously stirred for about 30 minutes. The organic layer was successively washed with water and saturated brine, dried over anhydrous sodium sulfate, and the solvent was evaporated under reduced pressure. The resulting residue was dissolved in a mixture of ethanol (30 ml) and water (15 ml), and the solution was added with methoxyamine hydrochloride (1.08 g) and sodium acetate (1.06 g), and refluxed for 1 hour by heating. The reaction mixture was left to cool, and then concentrated, and the resulting residue was added with water, and extracted with ethyl acetate. The organic layer was successively washed with saturated aqueous sodium hydrogencarbonate and saturated brine, and dried over anhydrous sodium sulfate, and the solvent was evaporated under reduced pressure. The resulting residue was purified by silica gel chromatography [developing solvent; ethyl acetate:n-hexane=1:4 (v/v)] to obtain the title compound (1.60 g) as colorless solid.
$^1$H-NMR (400MHz, $CDCl_3$) δ : 1.46 (9H, s), 3.87 (3H, s), 4.57-4.60 (4H, m).

Step 2: 3-Azetidine O-methyloxime hydrochloride

The compound obtained in Step 1 mentioned above (1.60 g) was dissolved in methanol (60 ml), and the solution was added with a 4 N hydrochloric acid solution in dioxane (10 ml), and stirred at room temperature for 15 hours. The reaction mixture was concentrated, and then added with tetrahydrofuran, and the deposited solid was collected by filtration, and dried to obtain the title compound (960 mg) as colorless solid (hygroscopic).
$^1$H-NMR (400MHz, $d_6$-DMSO) δ : 3.82 (3H, t, J=1.2Hz), 4.73 (2H, d, J=1.2Hz), 4.79 (2H, s), 9.84 (2H, br).
ESI-MS; m/z: 101 $(M+H)^+$.

[Reference Example 18]

[Formula 26]

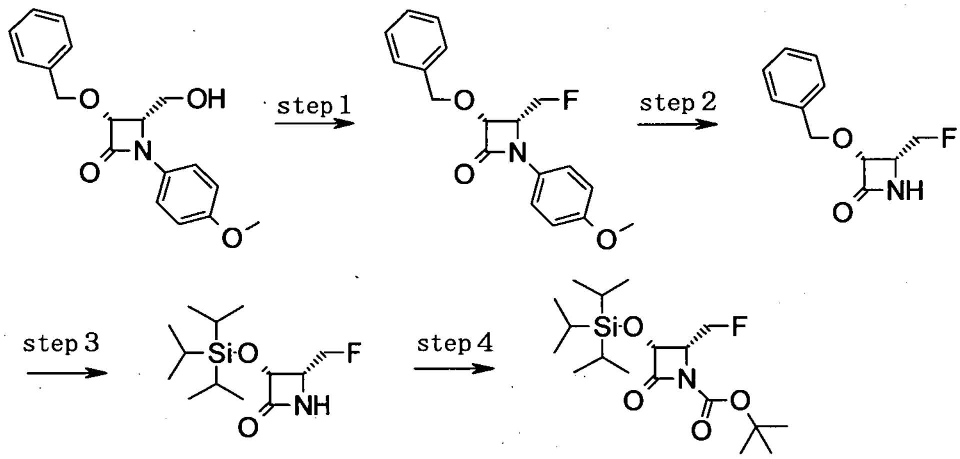


Step 1: (3R,4R)-3-(Benzyloxy)-4-(fluoromethyl)-1-(4-methoxyphenyl)-2-azetidinone

(3R,4S)-3-(Benzyloxy)-4-(hydroxymethyl)-1-(4-methoxyphenyl)-2-azetidinone (J. Med. Chem., 1994, 37, 2655, 1.00 g) was dissolved in dichloromethane (16 ml), and the solution was added with diethylaminosulfur trifluoride (0.843 ml) at -78°C, and stirred for 23 hours with warming to room temperature. The reaction mixture was cooled to 0°C, and then added with methanol. The solvent was evaporated under reduced pressure, and the resulting residue was purified by silica gel chromatography [developing solvent; ethyl acetate:n-hexane=1:9 (v/v)] to obtain the title compound (333 mg) as colorless solid.
$^1$H-NMR (400MHz, $CDCl_3$) δ : 3.79 (3H, s), 4.41-4.48 (1H, m), 4.66-4.91 (5H, m), 6.85-6.89 (2H, m), 7.31-7.46 (7H, m).
ESI-MS; m/z: 316 $(M+H)^+$.

Step 2: (3R,4R)-3-(Benzyloxy)-4-(fluoromethyl)-2-azetidinone

The compound obtained in Step 1 mentioned above (897 mg) was dissolved in acetonitrile (15 ml) and water (2 ml), and the solution was added with an aqueous solution (13 ml) of cerium(IV) diammonium nitrate (5.46 g) at 0°C, and stirred for 1 hour. The reaction mixture was added with ethyl acetate, and the organic layer was washed with water, saturated aqueous sodium hydrogencarbonate, and saturated brine, and dried over anhydrous sodium sulfate. Then, the solvent was evaporated under reduced pressure, and the resulting residue was purified by silica gel chromatography [developing solvent; ethyl acetate:n-hexane=1:3 to 1:1 (v/v)] to obtain the title compound (408 mg) as yellow solid.
$^1$ H-NMR (400MHz, $CDCl_3$) δ : 4.03 (1H, ddt, J=11.2, 7.6, 4.6Hz), 4.47-4.69 (3H, m), 4.80-4.84 (2H, m), 6.23 (1H, br s), 7.26-7.39 (5H, m).
ESI-MS; m/z: 210 $(M+H)^+$.

Step 3: (3R,4R)-4-(Fluoromethyl)-3-[(triisopropylsilyl)oxy]-2-azetidinone

The compound obtained in Step 2 mentioned above (408 mg) was dissolved in ethyl acetate (10 ml), and the

solution was added with 5% palladium/carbon (408 mg) at room temperature, and stirred for 3 days under a hydrogen atmosphere. The reaction mixture was filtered through Celite, and the filtrate was concentrated under reduced pressure. The residue was dissolved in N,N-dimethylformamide (4 ml), and the solution was added with imidazole (199 mg) and triisopropylsilyl chloride (0.645 ml) at 0°C, and stirred at room temperature for 18 hours. The reaction mixture was added with water, and extracted with ethyl acetate, and the organic layer was washed with water and saturated brine. The organic layer was dried over anhydrous sodium sulfate, and then the solvent was evaporated under reduced pressure. The resulting residue was purified by silica gel chromatography [developing solvent; ethyl acetate:n-hexane=1:3 (v/v)] to obtain the title compound (432 mg) as colorless oil.
$^1$H-NMR (400MHz, $CDCl_3$) δ : 1.07-1.28 (21H, m), 4.00-4.07 (1H, m), 4.57 (1H, ddd, J=48.1, 10.0, 8.1Hz), 4.73 (1H, ddd, J=46.4, 10.0, 3.9Hz), 5.09 (1H, dd, J=5.1, 2.4Hz), 6.11 (1H, br s).

Step 4: (3R,4R)-1-(tert-Butoxycarbonyl)-4-(fluoromethyl)-3-[(triisopropylsilyl)oxy]-2-azetidinone

The compound obtained in Step 3 mentioned above (432 mg) and di-tert-butyldicarbonate (529 mg) were dissolved in tetrahydrofuran (16 ml), and the solution was added with 4-dimethylaminopyridine (38.3 mg) at room temperature, and stirred for 16.5 hours. The reaction mixture was concentrated under reduced pressure, and the resulting residue was purified by silica gel chromatography [developing solvent; ethyl acetate:n-hexane=3:97 (v/v)] to obtain the title compound (555 mg) as colorless oil.
$^1$H-NMR (400MHz, $CDCl_3$) δ : 0:91-1.28 (21H, m), 1.52 (9H, s), 4.27 (1H, ddt, J=15.4, 5.6, 3.9Hz), 4.73 (1H, ddd, J=45.9, 9.8, 3.9Hz), 4.81 (1H, ddd, J=46.9, 9.8, 5.6Hz), 5.07 (1H, d, J=5.6Hz).

[Reference Example 19]

[Formula 27]

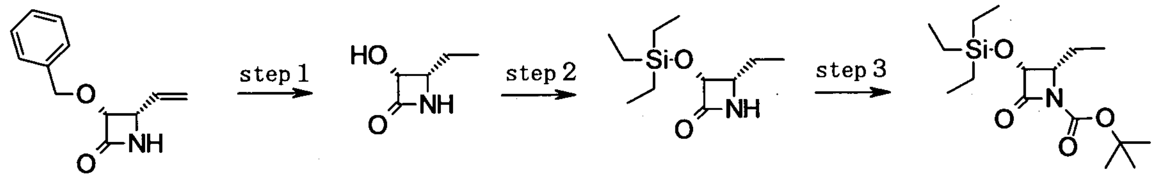


Step 1: (3R,4S)-4-Ethyl-3-hydroxy-2-azetidinone

(3R,4S)-3-(Benzyloxy)-4-vinyl-2-azetidinone (J. Med. Chem., 1994, 37, 2655, 680 mg) was dissolved in methanol (10 ml), and the solution was added with 5% palladium/carbon (680 mg × 2) at room temperature, and stirred for 44 hours under a hydrogen atmosphere. The catalyst was removed by filtration through Celite, and the filtrate was concentrated to obtain the title compound (450 mg) as red oil.
$^1$H-NMR (DMSO-$d_6$) δ : 0.87 (3H, t, J=7.4Hz), 1.34-1.57 (2H, m), 3.36-3.42 (1H, m), 4.65 (1H, d, J=4.6Hz), 5.86 (1H, brs), 8.17 (1H, brs).

Step 2: (3R,4S)-4-Ethyl-3-[(triethylsilyl)oxy]-2-azetidinone

The compound obtained in Step 1 mentioned above (580 mg) was dissolved in N,N-dimethylformamide (5 ml), and the solution was added with imidazole (1.04 g). The mixture was added dropwise with triethylsilyl chloride (1.28 ml) at 0°C, returned to room temperature, and stirred for 14 hours. The reaction mixture was added with water, and extracted with ethyl acetate, and the organic layer was washed with saturated aqueous sodium hydrogencarbonate and saturated brine, and dried over anhydrous sodium sulfate. Then, the solvent was evaporated under reduced pressure, and the resulting residue was purified by silica gel chromatography [developing solvent; ethyl acetate:n-hexane=1:6 (v/v)] to obtain the title compound (920 mg) as pale yellow amorphous solid.
$^1$H-NMR ($CDCl_3$) δ : 0.63-0.73 (6H, m), 0.93-1.01 (12H, m), 1.55-1.73 (2H, m), 3.57-3.63 (1H, m), 4.85 (1H, dd, J=4.6, 2.4Hz), 5.95 (1H, brs).

Step 3: (3R,4S)-1-(tert-Butoxycarbonyl)-4-ethyl-3-[(triethylsilyl)oxy]-2-azetidinone

The compound obtained in Step 2 mentioned above was used as a starting material to perform the same procedure as that of Reference Example 18, Step 4 and thereby to obtain the title compound as pale yellow amorphous solid.
$^1$ H-NMR ($CDCl_3$) δ : 0.63-0.73 (6H, m), 0.96-1.00 (12H, m), 1.51 (9H, s), 1.75-1.94 (2H, m), 3.92-3.97 (1H, m), 4.85 (1H, d, J=5.9Hz).

[Reference Example 20]

[Formula 28]

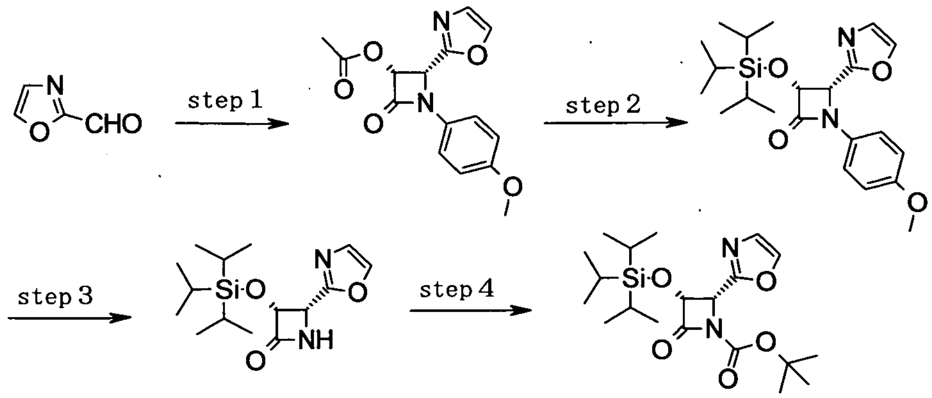


Step 1: cis-3-Acetoxy-1-(4-methoxyphenyl)-4-(2-oxazolyl)-2-azetidinone

Oxazole-2-carboxyaldehyde (J. Org. Chem., 1991, 56, 449, 1.85 g) and p-anisidine (1.80 g) were dissolved in benzene (30 ml), and the solution was added with anhydrous sodium sulfate (5 g), and stirred at room temperature for 2 hours. The insoluble matters were removed by filtration, and then the reaction mixture was concentrated under reduced pressure to obtain an imine as yellow oil. A solution of the resulting imine and triethylamine (3.05 ml) in dichloromethane (20 ml) was cooled to -78°C under a nitrogen atmosphere, then added dropwise with acetoxyacetyl chloride (1.57 ml), and warmed overnight to room temperature. The reaction mixture was concentrated, and then diluted with ethyl acetate. The reaction mixture was successively washed with saturated aqueous ammonium chloride, saturated aqueous sodium hydrogencarbonate and saturated brine, and then dried over anhydrous sodium sulfate, and the solvent was evaporated under reduced pressure. The resulting residue was purified by silica gel chromatography (developing solvent: ethyl acetate), and added with ether, and the deposited solid was collected by filtration, and dried to obtain the title compound (3.05 g) as colorless solid.
$^1$H-NMR (400MHz, $CDCl_3$) δ : 1.96 (3H, s), 3.77 (3H, s), 5.51 (1H, d, J=4.9Hz), 6.10 (1H, d, J=4.9Hz), 6.82-6.86 (2H, m), 7.18 (1H, d, J=0.5Hz), 7.25-7.28 (2H, m), 7.70 (1H, d, J=0.5Hz).
ESI-MS; m/z: 303 $(M+H)^+$.

Step 2: cis-1-(4-Methoxyphenyl)-4-(2-oxazolyl)-3-[(triisopropylsilyl)oxy]-2-azetidinone

The compound obtained in Step 1 mentioned above (2.80 g) was dissolved in a mixture of tetrahydrofuran (28 ml) and methanol (28 ml), and the solution was added with potassium carbonate (50 mg), and stirred at room temperature for 60 minutes. The mixture was added with prepared Dowex 50 (strongly acidic resin), stirred for about 5 minutes, then filtered, concentrated, and azeotroped 2 times with toluene. The resulting residue was dissolved in N,N-dimethylformamide (30 ml), and the solution was added with imidazole (770 mg) and triisopropylsilyl chloride (2.40 ml) at 0°C, and the mixture was stirred at room temperature for 24 hours. The reaction mixture was added with water, and extracted with ethyl acetate, and the organic layer was washed with water and saturated brine, and dried over anhydrous sodium sulfate. Then, the solvent was evaporated under reduced pressure, and the resulting residue was purified by silica gel chromatography [developing solvent; ethyl acetate:n-hexane=1:5 (v/v)] to obtain the title compound (3.30 g) as colorless solid.

$^1$H-NMR (400MHz, $CDCl_3$) δ : 0.97-1.01 (18H, m), 1.03-1.12 (3H, m), 3.76 (3H, s), 5.36 (2H, s), 6.80-6.84 (2H, m), 7.15 (1H, d, J=0.7Hz), 7.23-7.25 (2H, m), 7.06 (1H, d, J=0.7Hz).

Step 3: cis-4-(2-Oxazolyl)-3-[(triisopropylsilyl)oxy]-2-azetidinone

The compound obtained in Step 2 mentioned above was used as a starting material to perform the same procedure as that of Reference Example 18, Step 2 and thereby to obtain the title compound as colorless solid.
$^1$H-NMR (400MHz, $CDCl_3$) δ : 0.96-1.09 (21H, m), 4.98 (1H, d, J=4.9Hz), 5.29 (1H, dd, J=4.9, 2.0Hz), 6.34 (1H, br), 7.13 (1H, d, J=0.7Hz), 7.69 (1H, d, J=0.7Hz).

Step 4: cis-1-(tert-Butoxycarbonyl)-4-(2-oxazolyl)-3-[(triisopropylsilyl)oxy]-2-azetidinone

The compound obtained in Step 3 mentioned above was used as a starting material to perform the same procedure as that of Reference Example 18, Step 4 and thereby to obtain the title compound as pale yellow solid.
$^1$H-NMR ($CDCl_3$) δ : 0.95-0.97 (18H, m), 1.00-1.08 (3H, m), 1.45 (9H, s), 5.24 (2H, AB type d, J=5.5Hz), 7.15 (1H, s,), 7.68 (1H, s).
ESI-MS; m/z: 433 $(M+Na)^+$.

[Reference Example 21]

(3R,4R)-4-(Difluoromethyl)-1-(isopropoxycarbonylamino)-3-[(triisopropylsilyl)oxy]-2-azetidinone

[Formula 29]

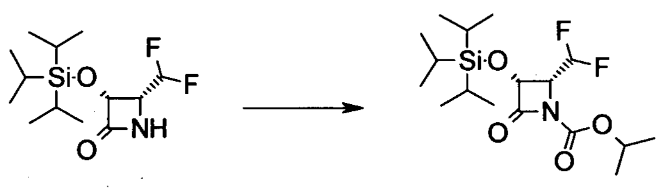

(3R,4R)-4-(Difluoromethyl)-3-[(triisopropylsilyl)oxy]-2-azetidinone (Bioorg. Med. Chem., 2000, 8, 1619, 400 mg) was dissolved in dichloromethane (8.0 ml), and the solution was cooled on ice. Then, the solution was added with N,N-diisopropylethylamine (0.28 ml), 4-dimethylaminopyridine (33.3 mg) and isopropyl chlorocarbonate (0.20 ml), and the mixture was stirred for 3 hours. The reaction mixture was added with ethyl acetate, and the organic layer was successively washed with 1 N aqueous hydrochloric acid, saturated aqueous sodium hydrogencarbonate, and saturated brine, and dried over anhydrous sodium sulfate. The solvent was evaporated under reduced pressure, and the resulting residue was purified by silica gel chromatography [developing solvent; ethyl acetate:n-hexane=10:90 (v/v)] to obtain the title compound (505 mg) as colorless oil.
$^1$H-NMR (400MHz, $CDCl_3$) δ : 1.07-1.22 (21H, m), 1.32 (3H, d, J=6.3Hz), 1.34 (3H, d, J=6.3Hz), 4.28-4.35 (1H, m), 5.03-5.09 (1H, m), 5.16 (1H, dd, J=6.1, 1.0Hz), 6.03 (1H, ddd, J=55.4, 54.0, 4.9Hz).
ESI-MS; m/z: 402 $(M+Na)^+$.

[Reference Example 22]

[Formula 30]

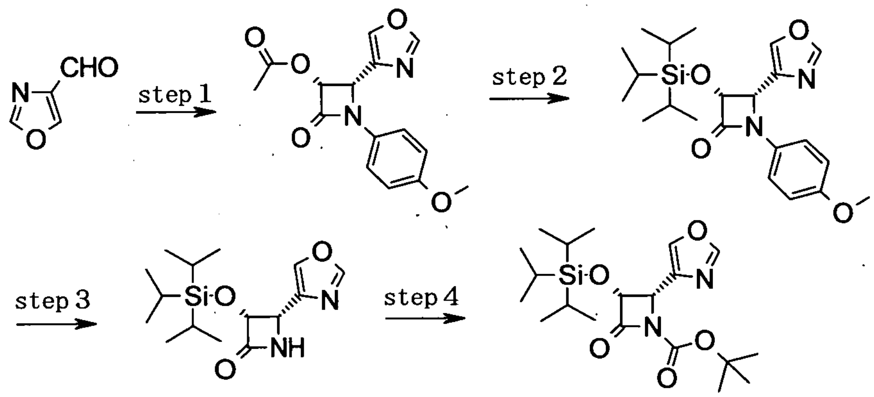


Step 1: cis-3-Acetoxy-1-(4-methoxyphenyl)-4-(4-oxazolyl)-2-azetidinone

Oxazole-4-carboxyaldehyde (J. Org. Chem., 1991, 56, 449) was used as a starting material to perform the same procedure as that of Reference Example 20, Step 1 and thereby to obtain the title compound as pale yellow solid.
$^1$ H-NMR (400MHz, $CDCl_3$) δ : 1.99 (3H, s), 3.77 (3H, s), 5.41 (1H, d, J=4.9Hz), 6.04 (1H, d, J=4.9Hz), 6.82-6.86 (2H, m), 7.31-7.35 (2H, m), 7.68 (1H, t, J=0.9Hz), 7.90 (1H, d, J=0.7Hz).

Step 2: cis-1-(4-Methoxyphenyl)-4-(4-oxazolyl)-3-[(triisopropylsilyl)oxy]-2-azetidinone

The compound obtained in Step 1 mentioned above was used as a starting material to perform the same procedure as that of Reference Example 20, Step 2 and thereby to obtain the title compound as pale yellow solid.
$^1$H-NMR (400MHz, $CDCl_3$) δ : 0.98-1.24 (21H, m), 3.76 (3H, s), 5.24 (1H, d, J=4.9Hz), 5.29 (1H, d, J=4.9Hz), 6.80-6.84 (2H, m), 7.33-7.37 (2H, m), 7.67 (1H, s), 7.89 (1H, s).
ESI-MS; m/z: 439 $(M+Na)^+$.

Step 3: cis-4-(4-Oxazolyl)-3-[(triisopropylsilyl)oxy]-2-azetidinone

The compound obtained in Step 2 mentioned above was used as a starting material to perform the same procedure as that of Reference Example 18, Step 2 and thereby to obtain the title compound as pale brown solid.
$^1$H-NMR (400MHz, $CDCl_3$) δ : 0.97-1.12 (21H, m), 4.87 (1H, d, J=4.2Hz), 5.22 (1H, dd, J=4.9, 2.7Hz), 6.06 (1H, brs), 7.74 (1H, s), 7.86 (1H, s).
ESI-MS; m/z: 333 $(M+Na)^+$.

Step 4: cis-1-(tert-Butoxycarbonyl)-4-(4-oxazolyl)-3-[(triisopropylsilyl)oxy]-2-azetidinone

The compound obtained in Step 3 mentioned above was used as a starting material to perform the same procedure as that of Reference Example 18, Step 4 and thereby to obtain the title compound as pale yellow solid.
$^1$H-NMR ($CDCl_3$) δ : 0.94-1.11 (21H, m), 1.47 (9H, s), 5.15 (1H, d, J=5.6Hz), 5.19 (1H, d, J=5.6Hz), 7.69 (1H, s), 7.87 (1H, s).
ESI-MS; m/z: 433 $(M+Na)^+$.

[Reference Example 23]

[Formula 31]

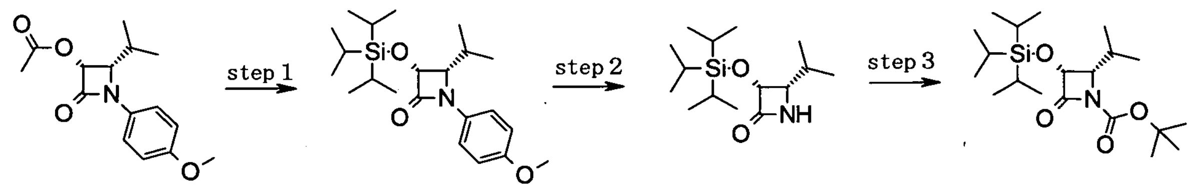


Step 1: cis-4-Isopropyl-1-(4-methoxyphenyl)-3-[(triisopropylsilyl)oxy]-2-azetidinone

cis-3-Acetoxy-4-isopropyl-1-(4-methoxyphenyl)-2-azetidinone (Bioorg. Med. Chem., 2003, 11, 4315) was used as a starting material to perform the same procedure as that of Reference Example 20, Step 2 and thereby to obtain the title compound as colorless solid.
$^1$H-NMR (400MHz, $CDCl_3$) δ : 1.02 (3H, d, J=6.8Hz), 1.09 (3H, d, J=6.8Hz), 1.11-1.14 (18H, m), 1.17-1.27 (3H, m), 2.28-2.35 (1H, m), 3.78 (3H, s), 4.07 (1H, t, J=5.3Hz), 5.05 (1H, t, J=5.3Hz), 6.83-6.87 (2H, m), 7.33-7.36 (2H, m).

Step 2: cis-4-Isopropyl-3-[(triisopropylsilyl)oxy]-2-azetidinone

The compound obtained in Step 1 mentioned above was used as a starting material to perform the same procedure as that of Reference Example 18, Step 2 and thereby to obtain the title compound as pale brown solid.
$^1$H-NMR (400MHz, $CDCl_3$) δ : 0.94-0.97 (6H, m), 1.08-1.11 (18H, m), 1.14-1.28 (3H, m), 1.90-1.99 (1H, m), 3:32 (1H, dd, J=8.6, 4.8Hz), 4.98 (1H, dd, J=4.8, 2.7Hz), 5.96 (1H, br).

Step 3: cis-1-(tert-Butoxycarbonyl)-4-isopropyl-3-[(triisopropylsilyl)oxy]-2-azetidinone

The compound obtained in Step 2 mentioned above was used as a starting material to perform the same procedure as that of Reference Example 18, Step 4 and thereby to obtain the title compound as pale yellow solid.
$^1$ H-NMR ($CDCl_3$) δ : 1.02 (6H, d, J=6.8Hz), 1.07-1.11 (18H, m), 1.14-1.23 (3H, m), 1.52 (9H, s), 2.15-2.20 (1H, m), 3.89-3.92 (1H, m), 4.98 (1H, d, J=6.3Hz).

[Reference Example 24]

[Formula 32]

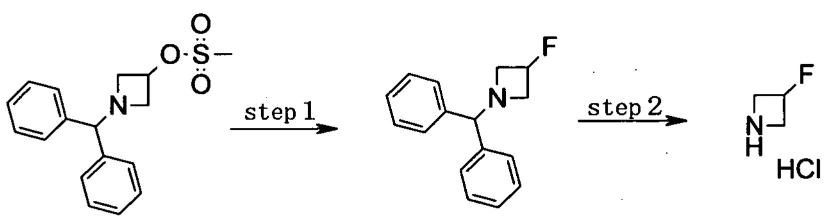


Step 1: 1-Benzhydryl-3-fluoroazetidine

1-Benzhydryl-3-azetidinyl methanesulfonate (2.50 g) was dissolved in acetonitrile (50 ml), and the solution was added with tetra-n-butylammonium fluoride (1 mole solution in tetrahydrofuran, 7.9 ml), and refluxed for 6 hours by heating. The reaction mixture was concentrated, and then the resulting residue was diluted with ethyl acetate. The reaction mixture was successively washed with water (2 times) and saturated brine, and then dried over anhydrous sodium sulfate, and the solvent was evaporated under reduced pressure. The resulting residue was purified by silica gel chromatography [developing solvent; ethyl acetate:n-hexane=1:10 (v/v)] to obtain the title compound (1.40 g) as colorless solid.

$^1$H-NMR (400MHz, $CDCl_3$) δ : 3.09-3.18 (2H, m), 3.50-3.58 (2H, m), 4.38 (1H, s), 5.06-5.24 (1H, m), 7.17-7.40 (10H, m).

Step 2: 3-Fluoroazetidine hydrochloride

The compound obtained in Step 1 mentioned above was used as a starting material to perform the same procedure as that of Reference Example 1 and thereby to obtain the title compound as colorless solid.
$^1$H-NMR (400MHz, $d_6$-DMSO) δ : 4.00-4.10 (2H, m), 4.22-4.32 (2H, m), 5.29-5.49 (1H, m), 9.66 (2H, br).
ESI-MS; m/z: 112 $(M+H)^+$.

[Reference Example 25]

(S)-1-Azetidin-2-ylmethanol hydrochloride

[Formula 33]

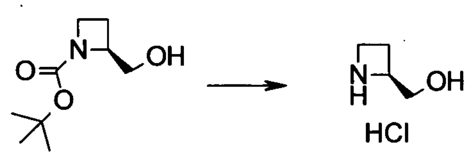

(S)-2-(Hydroxymethyl)-1-azetidinecarboxylic acid tert-butyl ester (4.20 g) was added with concentrated hydrochloric acid (14 ml), and the mixture was stirred at room temperature for 1 hour. The reaction mixture was concentrated under reduced pressure to obtain the title compound (2.68 g) as colorless oil.
$^1$H-NMR (400MHz, $d_6$-DMSO) δ : 2.22-2.38 (2H, m), 3.36 (1H, brs), 3.70-3.74 (1H, m), 3.78-3.85 (1H, m), 4.34-4.36 (1H, m), 5.41-5.43 (1H, m), 8.90 (1H, brs), 9.33 (1H, brs).

[Reference Example 26]

(R)-1-Azetidin-2-ylmethanol hydrochloride

[Formula 34]

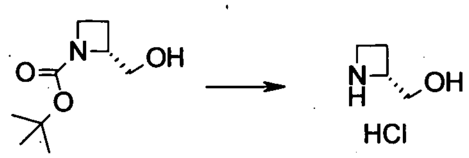

(R)-2-(Hydroxymethyl)-1-azetidinecarboxylic acid tert-butyl ester was used as a starting material to perform the same procedure as that of Reference Example 25 and thereby to obtain the title compound as colorless oil.
$^1$H-NMR (400MHz, $CDCl_3$) δ : 2.45-2.65 (2H, m), 3.83-4.05 (4H, m), 4.62 (1H, brs), 5.62 (1H, brs), 9.06 (1H, brs), 9.57 (1H, brs).

[Reference Example 27]

[Formula 35]

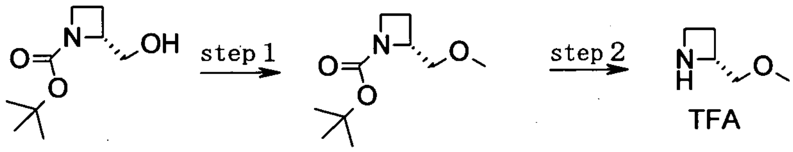


Step 1: (R)-2-(Methoxymethyl)-1-azetidinecarboxylic acid tert-butyl ester

(R)-2-(Hydroxymethyl)-1-azetidinecarboxylic acid tert-butyl ester was used as a starting material to perform the same procedure as that of Reference Example 11, Step 1 and thereby to obtain the title compound as colorless oil. $^{1}$H-NMR (400MHz, $CDCl_3$) δ : 1.44 (9H, s), 2.11-2.27 (2H, m), 3.41 (3H, s), 3.54 (1H, dd, J=10.1, 3.1Hz), 3.63-3.67 (1H, m), 3.82 (2H, dd, J=8.2, 6.7Hz), 4.27-4.33 (1H, m).

Step 2: (R)-2-(Methoxymethyl)azetidine trifluoroacetate

The compound obtained in Step 1 mentioned above was used as a starting material to perform the same procedure as that of Reference Example 11, Step 2 and thereby to obtain the title compound as colorless oil.
$^{1}$H-NMR (400MHz, $d_6$-DMSO) δ : 2.20-2.29 (1H, m), 2.33-2.42 (1H, m), 3.32 (3H, s), 3.53 (1H, dd, J=11.2, 3.4Hz), 3.64 (1H, dd, J=11.2, 7.1Hz), 3.69-3.79 (1H, m), 3.83-3.93 (1H, m), 4.50 (1H, brs), 8.81 (2H, brs).
ESI-MS; m/z: 102 $(M+H)^+$.

[Reference Example 28]

[Formula 36]

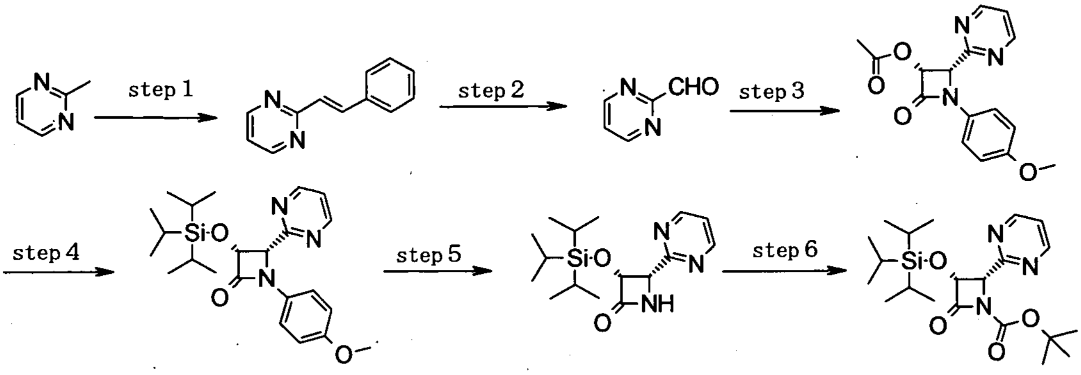


Step 1: 2-[(E)-Styryl]pyrimidine

A mixture of 2-methylpyrimidine (5.00 g) and benzaldehyde (8.10 ml) was added with zinc chloride (1 mole solution in ether, 7.97 ml) at room temperature, and refluxed at 160°C by heating for 23 hours. The reaction mixture was cooled to room temperature, and then purified by silica gel column chromatography [developing solvent; ethyl acetate: n-hexane=1:9 to 3:7 (v/v)] to obtain the title compound (6.85 g) as brown solid.
$^{1}$H-NMR (400MHz, $CDCl_3$) δ : 7.11 (1H, t, J=4.9Hz), 7.25 (1H, d, J=16.1Hz), 7.32-7.42 (3H, m), 7.62-7.64 (2H, m), 8.00 (1H, d, J=16.1Hz), 8.73 (2H, d, J=4.6Hz).
ESI-MS; m/z: 183 $(M+Na)^+$.

Step 2: Pyrimidine-2-carboxyaldehyde

The compound obtained in Step 1 mentioned above (6.62 g) was dissolved in 1,4-dioxane (90 ml) and water (30 ml), and the solution was added with osmium tetroxide (catalytic amount) and sodium metaperiodate (15.6 g) at room temperature. The reaction mixture was stirred for 2 days, and filtered through Celite, and then the solvent was evaporated under reduced pressure. The resulting residue was purified by silica gel column chromatography [developing solvent; ethyl acetate:n-hexane=2:1 to 100:0 (v/v)] to obtain the title compound (3.27 g) as brown solid.
$^1$H-NMR (400MHz, $CDCl_3$) δ : 7.52 (1H, t, J=4.9Hz), 9.01 (2H, d, J=4.9Hz), 10.14 (1H, s).
ESI-MS; m/z: 109 $(M+Na)^+$.

Step 3: cis-3-Acetoxy-1-(4-methoxyphenyl)-4-(2-pyrimidinyl)-2-azetidinone

The compound obtained in Step 2 mentioned above was used as a starting material to perform the same procedure as that of Reference Example 20, Step 1 and thereby to obtain the title compound as brown solid.
$^1$H-NMR (400MHz, $CDCl_3$) δ : 1.80 (3H, s), 3.76 (3H, s), 5.56 (1H, d, J=5.1Hz), 6.20 (1H, d, J=5.1Hz), 6.82 (2H, dt, J=9.8, 2.9Hz), 7.26-7.30 (3H, m), 8.77 (2H, d, J=4.9Hz). ESI-MS; m/z: 314 $(M+Na)^+$.

Step 4: cis-1-(4-Methoxyphenyl)-4-(2-pyrimidinyl)-3-[(triisopropylsilyl)oxy]-2-azetidinone

The compound obtained in Step 3 mentioned above was used as a starting material to perform the same procedure as that of Reference Example 20, Step 2 and thereby to obtain the title compound as colorless solid.
$^1$H-NMR (400MHz, $CDCl_3$) δ : 0.98-1.24 (21H, m), 3.76 (3H, s), 5.24 (1H, d, J=4.9Hz), 5.29 (1H, d, J=4.9Hz), 6.80-6.84 (2H, m), 7.33-7.37 (2H, m), 7.67 (1H, s), 7.89 (1H, s). ESI-MS; m/z: 428 $(M+Na)^+$.

Step 5: cis-4-(2-Pyrimidinyl)-3-[(triisopropylsilyl)oxy]-2-azetidinone

The compound obtained in Step 4 mentioned above was used as a starting material to perform the same procedure as that of Reference Example 18, Step 2 and thereby to obtain the title compound as pale yellow oil.
$^1$H-NMR (400MHz, $CDCl_3$) δ : 0.90-1.06 (21H, m), 5.05 (1H, d, J=4.9Hz), 5.36 (1H, dd, J=4.9, 1.5Hz), 6.28 (1H, brs), 7.22 (1H, t, J=4.9Hz), 8.74 (2H, d, J=4.9Hz).
ESI-MS; m/z: 322 $(M+Na)^+$.

Step 6: cis-1-(tert-Butoxycarbonyl)-4-(2-pyrimidinyl)-3-[(triisopropylsilyl)oxy]-2-azetidinone

The compound obtained in Step 5 mentioned above was used as a starting material to perform the same procedure as that of Reference Example 18, Step 4 and thereby to obtain the title compound as colorless solid.
$^1$H-NMR ($CDCl_3$) δ : 0.88-1.06 (21H, m), 1.45 (9H, s), 5.28 (1H, d, J=5.9Hz), 5.31 (1H, d, J=5.9Hz), 7.22 (1H, t, J=4.9Hz), 8.73 (2H, d, J=4.9Hz).

[Reference Example 29]

[Formula 37]

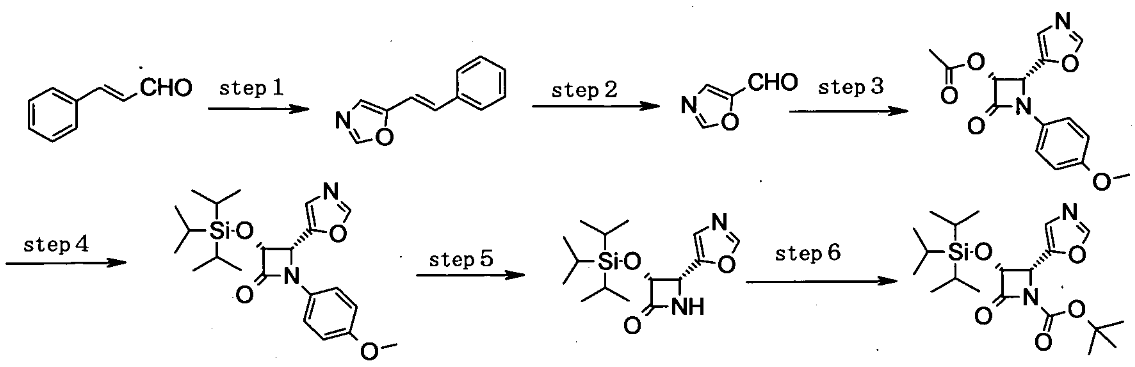

Step 1: 5-[(E)-Styryl]oxazole

A suspension of trans-cinnamaldehyde (4.0 g) and (p-toluenesulfonyl) methylisocyanide (5.9 g) in methanol (100 ml) was added with potassium carbonate (5.0 g), and the mixture was stirred at 80°C for 2 hours. The reaction mixture was concentrated under reduced pressure, and the resulting residue was added with water and ethyl acetate. Two of the layers were separated, and the aqueous layer was extracted with ethyl acetate. The organic layers were combined, washed with saturated brine, and then dried over anhydrous sodium sulfate, and the solvent was evaporated under reduced pressure. The resulting residue was purified by silica gel column chromatography [developing solvent; ethyl acetate:n-hexane=1:3 (v/v)] to obtain the title compound (4.4 g) as orange solid.
$^1$H-NMR-(400MHz, $CDCl_3$) $\delta$ : 6.92 (1H, d, J=16.4Hz), 7.08 (1H, s), 7.11 (1H, d, J=16.1Hz), 7.29 (1H, t, J=7.3Hz), 7.37 (2H, t, J=7.6Hz), 7.48 (2H, d, J=7.8Hz), 7.84 (1H, s).
ESI-MS; m/z: 172 $(M+H)^+$.

Step 2: Oxazole-5-carboxyaldehyde

The compound obtained in Step 1 mentioned above was used as a starting material to perform the same procedure as that of Reference Example 28, Step 2 and thereby to obtain the title compound as yellow oil.
$^1$H-NMR (400MHz, $CDCl_3$) $\delta$ : 7.89 (1H, s), 8.12 (1H, s), 9.87 (1H, d, J=0.5Hz).

Step 3: cis-3-Acetoxy-1-(4-methoxyphenyl)-4-(5-oxazolyl)-2-azetidine

The compound obtained in Step 2 mentioned above was used as a starting material to perform the same procedure as that of Reference Example 20, Step 1 and thereby to obtain the title compound as brown solid.
$^1$H-NMR (400MHz, $CDCl_3$) $\delta$ : 1.98 (3H, s), 3.77 (3H, s), 5.48 (1H, d, J=4.6Hz), 6.02 (1H, d, J=4.6Hz), 6.84 (2H, d, J=9.0Hz), 7.18 (1H, s), 7.28 (2H, d, J=8.8Hz), 7.91 (1H, s).
ESI-MS; m/z: 303 $(M+H)^+$.

Step 4: cis-1-(4-Methoxyphenyl)-4-(5-oxazolyl)-3-[(triisopropylsilyl)oxy]-2-azetidinone

The compound obtained in Step 3 mentioned above was used as a starting material to perform the same procedure as that of Reference Example 20, Step 2 and thereby to obtain the title compound as pale yellow solid.
$^1$H-NMR (400MHz, $CDCl_3$) $\delta$ : 0.96-1.16 (21H, m), 3.76 (3H, s), 5.30 (2H, s), 6.82 (2H, d, J=9.7Hz), 7.16 (1H, s), 7.29 (2H, d, J=9.0Hz), 7.88 (1H, s).
FAB-MS; m/z: 417 $(M+H)^+$.

Step 5: cis-4-(5-Oxazolyl)-3-[(triisopropylsilyl)oxy]-2-azetidinone

The compound obtained in Step 4 mentioned above was used as a starting material to perform the same procedure as that of Reference Example 18, Step 2 and thereby to obtain the title compound as brown solid.
$^1$H-NMR (400MHz, $CDCl_3$) $\delta$ : 0.95-1.12 (21H, m), 4.93 (1H, d, J=4.6Hz), 5.24 (1H, dd, J=4.6, 2.4Hz), 6.04 (1H, brs), 7.13 (1H, s), 7.88 (1H, s).
ESI-MS; m/z: 311 $(M+H)^+$.

Step 6: cis-1-(tert-Butoxycarbonyl)-4-(5-oxazolyl)-3-[(triisopropylsilyl)oxy]-2-azetidinone

The compound obtained in Step 5 mentioned above was used as a starting material to perform the same procedure as that of Reference Example 18, Step 4 and thereby to obtain the title compound as colorless solid.
$^1$H-NMR ($CDCl_3$) $\delta$ : 0.93-1.11 (21H, m), 1.46 (9H, s), 5.21 (2H, s), 7.15 (1H, s), 7.89 (1H, s).
ESI-MS; m/z: 411 $(M+H)^+$.

[Reference Example 30]

[Formula 38]

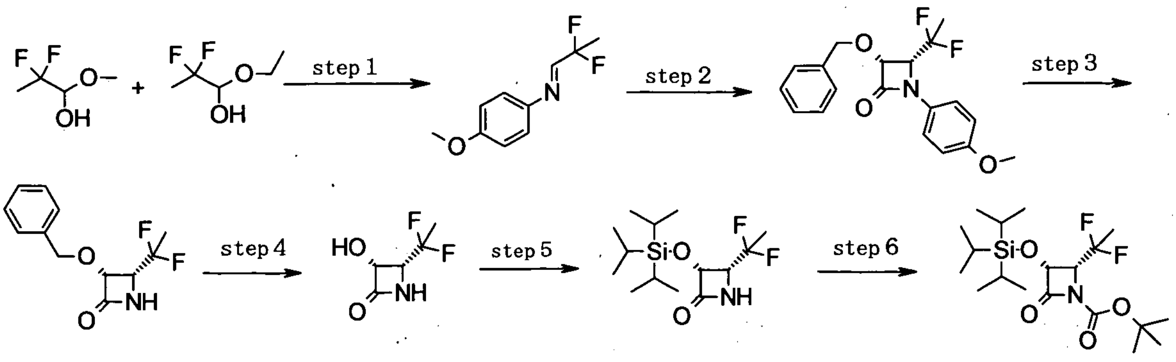


Step 1: N-(2,2-Difluoropropylidene)-4-methoxyaniline

A mixture of 1-ethoxy-2,2-difluoro-1-propanol and 1-methoxy-2,2-difluoro-1-propanol (J. Org. Chem., 1995, 60, 5174, 722 mg) was dissolved in toluene, and the solution was added with p-anisidine (0.52 g), and refluxed by heating for 3 hours. The reaction mixture was cooled to room temperature, then added with saturated aqueous sodium hydrogencarbonate, and extracted with toluene. The organic layer was washed with saturated brine, and dried over anhydrous magnesium sulfate, and then the solvent was evaporated under reduced pressure to obtain the title compound (989 mg) as yellow oil.
$^1$H-NMR (400MHz, $CDCl_3$) δ : 1.89 (3H, t, J=18.8Hz), 3.83 (3H, s), 6.89-6.94 (2H, m), 7.15-7.28 (2H, m), 7.87 (1H, t, J=3.4Hz).

Step 2: cis-1-(4-Methoxyphenyl)-3-(benzyloxy)-4-(1,1-difluoroethyl)-2-azetidinone

The compound obtained in Step 1 mentioned above (837 mg) was dissolved in dichloromethane (4.94 ml), and the solution was added with triethylamine (2.05 ml) at 0°C, then added dropwise with benzyloxyacetyl chloride (1.55 g), and stirred at 45°C for 84 hours. Then, the reaction mixture was added with water, and extracted with ethyl acetate. The organic layer was washed with saturated brine, and dried over anhydrous magnesium sulfate, and the solvent was evaporated under reduced pressure. The resulting residue was added with ether, and the deposited solid was collected by filtration, and dried to obtain the title compound (931 mg) as pale brown solid.
$^1$H-NMR (400MHz, $CDCl_3$) δ : 1.77 (3H, t, J=19.5Hz), 3.79 (3H, s), 4.48-4.52 (1H, m), 4.77-4.84 (1H, m), 4.91 (1H, d, J=5.1Hz), 6.85-6.90 (2H, m), 7.30-7.41 (5H, m), 7.43-7.48 (2H, m).

Step 3: cis-3-(Benzyloxy)-4-(1,1-difluoroethyl)-2-azetidinone

The compound obtained in Step 2 mentioned above was used as a starting material to perform the same procedure as that of Reference Example 18, Step 2 and thereby to obtain the title compound as pale yellow solid.
$^1$H-NMR (400MHz, $CDCl_3$) δ : 1.76 (3H, t, J=19.3Hz), 3.95-4.02 (1H, m), 4.72-4.84 (2H, m), 6.05 (1H, brs), 7.30-7.38 (5H, m).

Step 4: cis-4-(1,1-Difluoroethyl)-3-hydroxy-2-azetidinone

The compound obtained in Step 3 mentioned above was used as a starting material to perform the same procedure as that of Reference Example 19, Step 1 and thereby to obtain the title compound as pale yellow amorphous solid.
$^1$H-NMR (400MHz, $CDCl_3$) δ : 1.77 (3H, t, J=19.1Hz), 2.87 (1H, brs), 3.89-3.97 (1H, m), 5.01-5.05 (1H, m), 6.07 (1H, brs).

Step 5: cis-4-(1,1-Difluoroethyl)-3-[(triisopropylsilyl)oxy]-2-azetidinone

The compound obtained in Step 4 mentioned above was used as a starting material, and reacted with triisopropylsilyl chloride instead of triethylsilyl chloride to perform the same procedure as that of Reference Example 19, Step 2 and thereby to obtain the title compound as pale yellow oil.
$^1$H-NMR (400MHz, $CDCl_3$) δ : 0.97-1.28 (21H, m), 1.77 (3H, t, J=19.3Hz), 3.89-3.99 (1H, m), 5.09-5.14 (1H, m), 5.96 (1H, brs).

Step 6: cis-1-(tert-Butoxycarbonyl)-4-(1,1-difluoroethyl)-3-[(triisopropylsilyl)oxy]-2-azetidinone

The compound obtained in Step 5 mentioned above was used as a starting material to perform the same procedure as that of Reference Example 18, Step 4 and thereby to obtain the title compound as colorless solid.
$^1$H-NMR ($CDCl_3$) δ : 0.97-1.28 (21H, m), 1.52 (9H, s), 1.76 (3H, t, J=19.0Hz), 4.29-4.36 (1H, m), 5.13 (1H, d, J=6.3Hz).

[Reference Example 31]

[Formula 39]

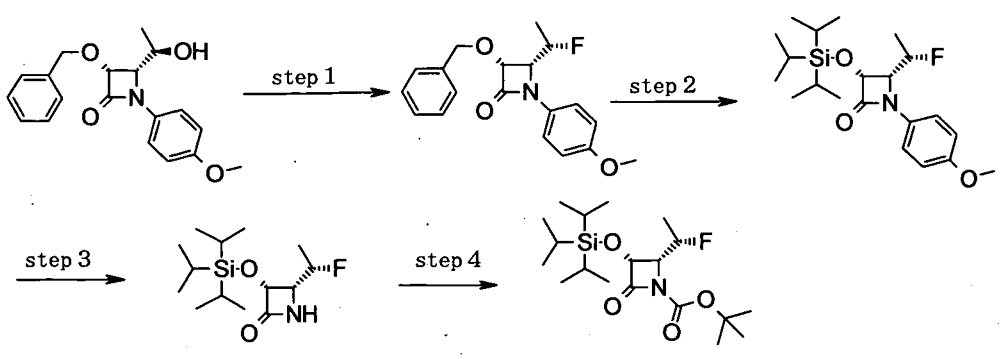


Step 1: (3R,4R)-3-(Benzyloxy)-4-[(1S)-1-fluoroethyl]-1-(4-methoxyphenyl)-2-azetidinone

(3R,4S)-3-(Benzyloxy)-4-[(1R)-hydroxyethyl]-1-(4-methoxyphenyl)-2-azetidinone (J. Org. Chem., 1994, 59, 3123) was used as a starting material to perform the same procedure as that of Reference Example 18, Step 1 and thereby to obtain the title compound yellow solid.
$^1$H-NMR (400MHz, $CDCl_3$) δ : 1.41 (3H, dd, J=25.1, 6.3Hz), 3.79 (3H, s), 4.47-4.54 (1H, m), 4.73 (1H, d, J=11.7Hz), 4.85 (1H, d, J=5.6Hz), 4.89-5.11 (2H, m), 6.88 (2H, d, J=9.0Hz), 7.28-7.52 (7H, m).

Step 2: (3R,4R)-4-[(1S)-1-Fluoroethyl]-1-(4-methoxyphenyl)-3-[(triisopropylsilyl)oxy]-2-azetidinone

The compound obtained in Step 1 mentioned above was used as a starting material to perform the same procedure as that of Reference Example 18, Step 3 and thereby to obtain the title compound as colorless oil.
$^1$H-NMR (400MHz, $CDCl_3$) δ : 1.08-1.27 (21H, m), 1.41 (3H, dd, J=25.1, 6.3Hz), 3.79 (3H, s), 4.52 (1H, dd, J=16.1, 5.6Hz), 4.97-5.16 (1H, m), 5.13 (1H, d, J=5.6Hz), 6.87 (2H, d, J=9.0Hz), 7.51 (2H, d, J=9.0Hz).
ESI-MS; m/z: 396 $(M+H)^+$.

Step 3: (3R,4R)-4-[(1S)-1-Fluoroethyl]-3-[(triisopropylsilyl)oxy]-2-azetidinone

The compound obtained in Step 2 mentioned above was used as a starting material to perform the same procedure as that of Reference Example 18, Step 2 and thereby to obtain the title compound as orange oil.
$^1$H-NMR (400MHz, $CDCl_3$) δ : 1.05-1.22 (21H, m), 1.43 (3H, dd, J=24.9, 6.3Hz), 3.94 (1H, dt, J=12.9, 4.9Hz), 4.81-5.01 (1H, m), 5.07 (1H, dd, J=5.1, 2.7Hz), 5.90 (1H, brs). ESI-MS; m/z: 290 $(M+H)^+$.

Step 4: (3R,4R)-1-(tert-Butoxycarbonyl)-4-[(1S)-1-fluoroethyl]-3-[(triisopropylsilyl)oxy]-2-azetidinone

The compound obtained in Step 3 mentioned above was used as a starting material to perform the same procedure as that of Reference Example 18, Step 4 and thereby to obtain the title compound as colorless oil.
$^1$H-NMR (400MHz, $CDCl_3$) δ : 1.04-1.22 (21H, m), 1.46 (3H, dd, J=24.4, 6.6Hz), 1.52 (9H, s), 4.24-4.32 (1H, m), 4.90-5.09 (1H, m), 5.08 (1H, d, J=6.1Hz).
ESI-MS; m/z: 412 $(M+Na)^+$.

[Reference Example 32]

[Formula 40]

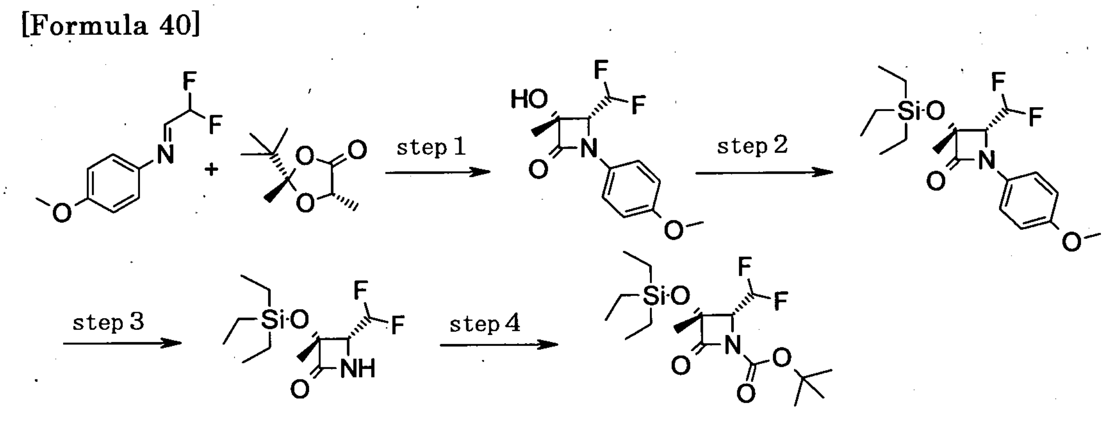


Step 1: (3R,4R)-4-Difluoromethyl-3-hydroxy-1-(4-methoxyphenyl)-3-methyl-2-azetidinone

A solution of (2S,5S)-2-(tert-butyl)-2,5-dimethyl-1,3-dioxolan-4-one (J. Am. Chem. Soc., 1995, 117, 6394, 1.55 g) in tetrahydrofuran (20 ml) was added with lithium bis(trimethylsilyl)amide (1 mole solution in tetrahydrofuran, 10.8 ml) at -78°C under an argon flow, and the mixture was stirred at the same temperature for 30 minutes. The reaction mixture was added with a solution of hexamethylphosphoramide (6.0 ml) and N-(2,2-difluoroethylidene)-4-methoxyaniline (J. Org. Chem., 1997, 62, 8826, 3.3 g) in tetrahydrofuran (3.2 ml), and stirred at the same temperature for 4 hours. The reaction mixture was added with saturated aqueous ammonium chloride, and then extracted with ethyl acetate. The extract was successively washed with 1 N aqueous hydrochloric acid and saturated,brine, and then dried over anhydrous sodium sulfate, and the solvent was evaporated under reduced pressure. The resulting residue was purified by silica gel column chromatography [developing solvent; ethyl acetate:n-hexane=1:4 (v/v)] to obtain the title compound (0.86 g) as colorless solid.
$^1$H-NMR (400MHz, $CDCl_3$) δ : 1.69 (3H, s), 3.02 (1H, brs), 3.80 (3H, s), 4.08-4.16 (1H, m), 6.11 (1H, ddd, J=57.2, 52.1, 3.6Hz), 6.89 (2H, d, J=9.0Hz), 7.44 (2H, d, J=8.8Hz). FAB-MS; m/z: 258 $(M+H)^+$.

Step 2: (3R,4R)-4-Difluoromethyl-1-(4-methoxyphenyl)-3-methyl-3-[(triethylsilyl)oxy]-2-azetidinone

The compound obtained in Step 1 mentioned above was used as a starting material to perform the same procedure as that of Reference Example 19, Step 2 and thereby to obtain the title compound as colorless oil.
$^1$H-NMR (400MHz, $CDCl_3$) δ : 0.68-0.80 (6H, m), 0.99 (9H, t, J=7.8Hz), 1.57 (3H, s), 3.79 (3H, s), 3.96-4.03 (1H, m); 5.97 (1H, ddd, J=57.7, 52.5, 3.8Hz), 6.85-6.90 (2H, m), 7.44 (2H, d, J=9.3Hz).

Step 3: (3R,4R)-4-Difluoromethyl-3-methyl-3-[(triethylsilyl)oxy]-2-azetidinone

The compound obtained in Step 2 mentioned above was used as a starting material to perform the same procedure as that of Reference Example 18, Step 2 and thereby to obtain the title compound as orange oil.
$^1$H-NMR (400MHz, $CDCl_3$) δ : 0.66-0.77 (6H, m), 0.97 (9H, t, J=8.1Hz), 1:59 (3H, s), 3.52-3.59 (1H, m), 5.81 (1H, ddd, J=57.6, 50.8, 3.4Hz), 5.90 (1H, brs).

Step 4: (3R,4R)-1-(tert-Butoxycarbonyl)-4-difluoromethyl-3-methyl-3-[(triethylsilyl)oxy]-2-azetidinone

The compound obtained in Step 3 mentioned above was used as a starting material to perform the same procedure as that of Reference Example 18, Step 4 and thereby to obtain the title compound as orange oil.
$^1$H-NMR (400MHz, $CDCl_3$) δ : 0.59-0.77 (6H, m), 0.96 (9H, t, J=7.8Hz), 1.52 (9H, s), 1.57 (3H, s), 3.86-3.94 (1H, m), 5.91 (1H, ddd, J=57.1, 52.7, 3.2Hz).

[Reference Example 33]

(3R,4R)-1-Benzoyl-4-difluoromethyl-3-methyl-3-[(triethylsilyl)oxy]-2-azetidinone

[Formula 41]

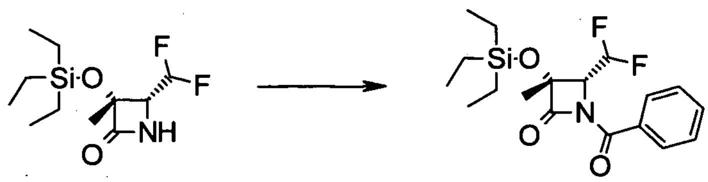

A solution of the compound obtained in Reference Example 32, Step 3 (0.17 g) in dichloromethane (5 ml) was added with triethylamine (134 μl), benzoyl chloride (112 μl) and 4-dimethylaminopyridine (16 mg), and the mixture was stirred at room temperature for 1 hour. The reaction mixture was added with saturated aqueous ammonium chloride, and then two of the layers were separated. The aqueous layer was extracted with chloroform, and the organic layers were combined, washed with saturated aqueous sodium hydrogencarbonate and saturated brine, and then dried over anhydrous sodium sulfate. The solvent was evaporated under reduced pressure, and the resulting residue was purified by silica gel column chromatography [developing solvent; ethyl acetate:n-hexane=1:8 (v/v)] to obtain the title compound (0.2 g) as yellow oil.
$^1$H-NMR (400MHz, $CDCl_3$) δ : 0.67-0.79 (6H, m), 1.00 (9H, t, J=7.8Hz), 1.63 (3H, s), 4.31-4.39 (1H, m), 6.06 (1H, dt, J=54.7,4.2Hz), 7.49 (2H, t, J=8.1Hz), 7.62 (1H, t, J=7.3Hz), 7.96 (2H, d, J=8.3Hz).

[Reference Example 34]

cis-4-(1,1-Difluoroethyl)-1-(isopropoxycarbonyl)-3-[(triisopropylsilyl)oxy]-2-azetidinone

[Formula 42]

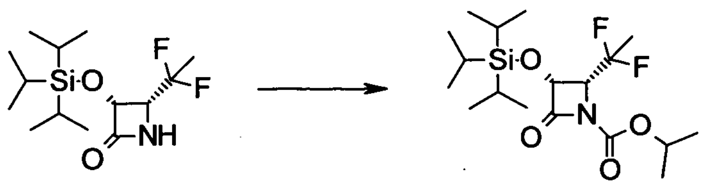

The compound obtained in Reference Example 30, Step 5 was used as a starting material to perform the same procedure as that of Reference Example 21 and thereby to obtain the title compound as colorless oil.
$^1$H-NMR (400MHz, $CDCl_3$) δ : 1.04-1.21 (21H, m), 1.25-1.38 (6H, m), 1.76 (3H, dt, J=19.0, 6.3Hz), 4.33-4.40 (1H, m), 5.01-5.10 (1H, m), 5.15 (1H, d, J=6.1Hz).

[Reference Example 35]

(3R,4S)-3-[(tert-Butyldimethylsilyl)oxy]-1-(isopropoxycarbonyl)-4-phenyl-2-azetidinone

[Formula 43]

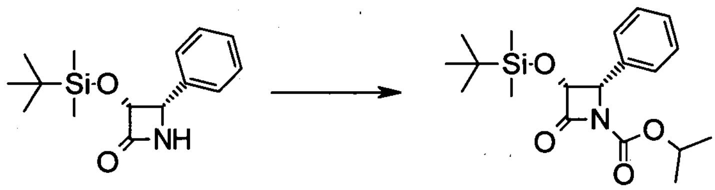

(3R,4S)-3-[(tert-Butyldimethylsilyl)oxy]-4-phenyl-2-azetidinone (Tetrahedron, 1992, 48, 6985) was used as a starting material to perform the same procedure as that of Reference Example 21 and thereby to obtain the title compound as colorless solid. $^1$H-NMR (400MHz, $CDCl_3$) δ : -0.16 (3H, s), 0.05 (3H, s), 0.64 (9H, s), 1.16 (3H, d, J=6.1Hz), 1.27 (3H, d, J=6.6Hz), 4.93-5.01 (1H, m), 5.06 (1H, d, J=5.6Hz), 5.09 (1H, d, J=5.6Hz), 7.25-7.37 (5H, m).

[Reference Example 36]

(3R,4R)-1-(Isopropoxycarbonyl)-4-(trifluoromethyl)-3-[(triisopropylsilyl)oxy]-2-azetidinone

[Formula 44]

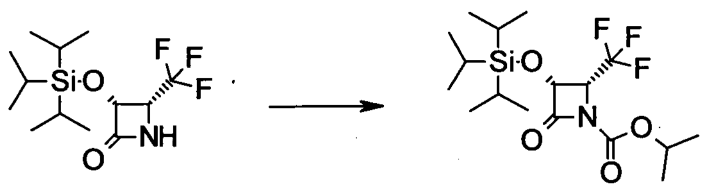

(3R,4R)-4-(Trifluoromethyl)-3-[(triisopropylsilyl)oxy]-2-azetidinone (J. Fluorine. Chem., 2004, 125, 487) was used as a starting material to perform the same procedure as that of Reference Example 21 and thereby to obtain the title compound as pale yellow oil.
$^1$H-NMR (400MHz, $CDCl_3$) δ : 1.03-1.22 (21H, m), 1.32 (3H, d, J=6.2Hz), 1.34 (3H, d, J=6.2Hz), 4.49-4.54 (1H, m), 5.03-5.09 (1H, m), 5.23 (1H, d, J=6.2Hz).
ESI-MS; m/z: 420 $(M+Na)^+$.

[Reference Example 37]

2-[(2S)-Azetidinyl]-2-propanol hydrochloride

[Formula 45]

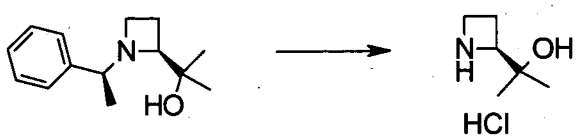

2-{(2S)-1-[(1S)-Phenylethyl]azetidinyl}-2-propanol (Tetrahedron, 1998, 54, 4991) was used as a starting material to perform the same procedure as that of Reference Example 1 and thereby to obtain the title compound as off-white solid.
ESI-MS; m/z: 116 $(M+Na)^+$.

[Reference Example 38]

(3R,4S)-4-(3-Fluoro-2-pyridyl)-1-(isopropoxycarbonyl)-3-[(triisopropylsilyl)oxy]-2-azetidinone

[Formula 46]

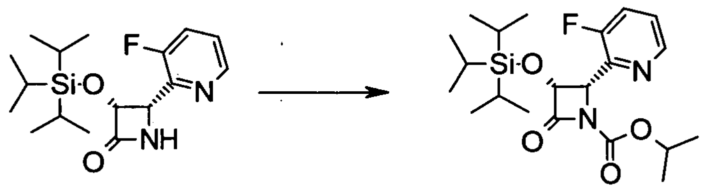

(3R,4S)-4-(3-Fluoro-2-pyridyl)-3-[(triisopropylsilyl)oxy]-2-azetidinone (Bioorg. Med. Chem. Lett., 2004, 14, 3209) was used as a starting material to perform the same procedure as that of Reference Example 21 and thereby to obtain the title compound as pale yellow oil.
$^1$H-NMR (400MHz, $CDCl_3$) δ : 0.87-1.04 (21H, m), 1.20 (3H, d, J=6.1Hz), 1.30 (3H, d, J=6.1Hz), 4.97-5.05 (1H, m), 5.30 (1H, d, J=5.9Hz), 5.50 (1H, d, J=5.6Hz), 7.22-7.28 (1H, m), 7.38 (1H, t, J=9.3Hz), 8.42 (1H, d, J=4.6Hz).

[Reference Example 39]

(3R,4S)-3-[(tert-Butyldimethylsilyl)oxy]-1-(isopropoxycarbonyl)-3-methyl-4-(2-pyridyl)-2-azetidinone

[Formula 47]

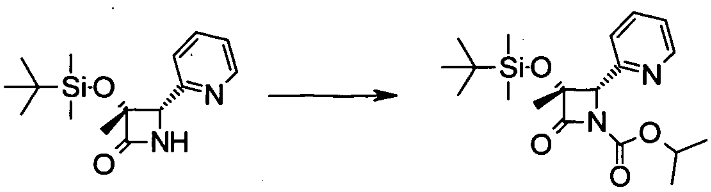

(3R,4S)-3-[(tert-Butyldimethylsilyl)oxy]-3-methyl-4-(2-pyridyl)-2-azetidinone (Bioorg. Med. Chem. Lett., 2003, 13, 185) was used as a starting material to perform the same procedure as that of Reference Example 21 and thereby to obtain the title compound as colorless oil.
$^1$H-NMR (400MHz, $CDCl_3$) δ : 0.02 (3H, s), 0.08 (3H, s), 0.49 (9H, s), 1.20 (3H, d, J=6.3Hz), 1.30 (3H, d, J=6.1Hz), 1.72 (3H, s), 4.95 (1H, s), 5.00-5.07 (1H, m), 7.19-7.22 (2H, m), 7.67 (1H, dt, J=7.7, 1.7Hz), 8.57-8.60 (1H, m).
ESI-MS; m/z: 379 $(M+H)^+$.

[Reference Example 40]

(3R,4R)-4-Difluoromethyl-1-(isopropoxycarbonyl)-3-methyl-3-[(triethylsilyl)oxy]-2-azetidinone

[Formula 48]

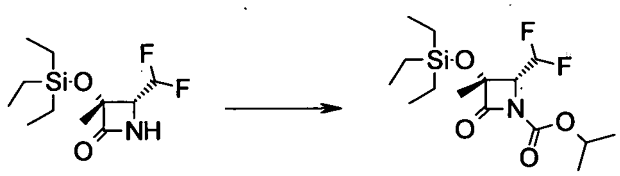

The compound obtained in Reference Example 32, Step 3 was used as a starting material to perform the same procedure as that of Reference Example 21 and thereby to obtain the title compound as colorless oil.
$^1$H-NMR (400MHz, $CDCl_3$) δ : 0.64-0.73 (6H, m), 0.96 (9H, t, J=7.1Hz), 1.31-1.36 (6H, m), 1.54 (3H, s), 3.90-3.99 (1H, m), 5.01-5.10 (1H, m), 5.93 (1H, ddd, J=57.2, 52.6, 3.2Hz).

[Reference Example 41]

[Formula 49]

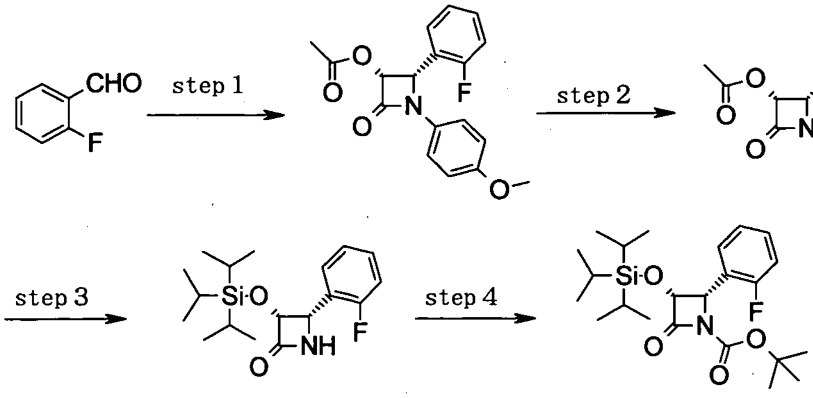


Step 1: cis-3-Acetoxy-4-(2-fluorophenyl)-1-(4-methoxyphenyl)-2-azetidinone

2-Fluorobenzaldehyde was used as a starting material to perform the same procedure as that of Reference Example 20, Step 1 and thereby to obtain the title compound as colorless solid.
$^1$H-NMR (400MHz, $CDCl_3$) δ : 1.76 (3H, s), 3.76 (3H, s), 5.67 (1H, d, J=4.9Hz), 6.02 (1H, d, J=4.9Hz), 6.80-6.84 (2H, m), 7.10-7.13 (2H, m), 7.26-7.37 (4H, m).

Step 2: cis-3-Acetoxy-4-(2-fluorophenyl)-2-azetidinone

The compound obtained in Step 1 mentioned above was used as a starting material to perform the same procedure as that of Reference Example 18, Step 2 and thereby to obtain the title compound as pale yellow oil.
$^1$H-NMR (400MHz, $CDCl_3$) δ : 1.76 (3H, s), 5.35 (1H, d, J=4.6Hz), 6.00 (1H, dd, J=4.6, 2.0Hz), 6.27 (1H, br), 7.03-7.44 (4H, m).

Step 3: cis-4-(2-Fluorophenyl)-3-[(triisopropylsilyl)oxy]-2-azetidinone

The compound obtained in Step 2 mentioned above was used as a starting material to perform the same procedure as that of Reference Example 20, Step 2 and thereby to obtain the title compound as colorless solid.
$^1$H-NMR (400MHz, $CDCl_3$) δ : 0.87-1.05 (21H, m), 5.18-5.25 (2H, m), 6.00 (1H, brs), 7.00-7.47 (4H, m).

Step 4: cis-1-(tert-Butoxycarbonyl)-4-(2-fluorophenyl)-3-[(triisopropylsilyl)oxy]-2-azetidinone

The compound obtained in Step 3 mentioned above was used as a starting material to perform the same procedure as that of Reference Example 18, Step 4 and thereby to obtain the title compound as colorless solid.
$^1$H-NMR ($CDCl_3$) δ : 0.79-1.02 (21H, m), 1.43 (9H, s), 5.21 (1H, d, J=5.8Hz), 5.42 (1H, d, J=5.8Hz), 7.02-7.32 (4H, m).
ESI-MS; m/z: 460 $(M+Na)^+$.

[Reference Example 42]

cis-4-(2-Fluorophenyl)-1-(isopropoxycarbonyl)-3-[(triisopropylsilyl)oxy]-2-azetidinone

[Formula 50]

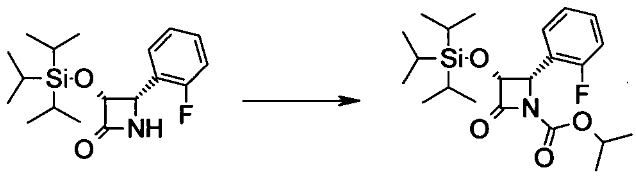

The compound obtained in Reference Example 41, Step 3 was used as a starting material to perform the same procedure as that of Reference Example 21 and thereby to obtain the title compound as colorless oil.
$^1$H-NMR ($CDCl_3$) δ : 0.88-1.03 (21H, m), 1.19 (3H, d, J=6.1Hz), 1.29 (3H, d, J=6.1Hz), 4.96-5.01 (1H, m), 5.24 (1H, d, J=5.9Hz), 5.46 (1H, d, J=5.9Hz), 7.02-7.31 (4H, m).
ESI-MS; m/z: 446 $(M+Na)^+$.

[Reference Example 43]

[Formula 51]

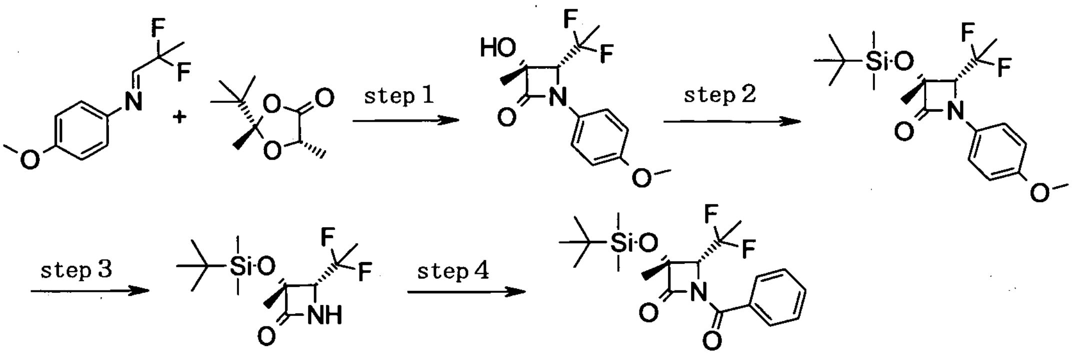


Step 1: (3R,4R)-4-(1,1-Difluoroethyl)-3-hydroxy-1-(4-methoxyphenyl)-3-methyl-2-azetidinone

The compound obtained in Reference Example 30, Step 1 was used as a starting material to perform the same procedure as that of Reference Example 32, Step 1 and thereby to obtain the title compound as brown oil.
$^1$H-NMR ($CDCl_3$) δ : 1.67 (3H, s), 1.79 (3H, t, J=19.1Hz), 3.06 (1H, brs), 3.80 (3H, s), 4.14-4.19 (1H, m), 6.87-6.90 (2H, m), 7.44-7.47 (2H, m).
FAB-MS; m/z: 272 $(M+H)^+$.

Step 2: (3R,4R)-3-[(tert-Butyldimethylsilyl)oxy]-4-(1,1-difluoroethyl)-1-(4-methoxyphenyl)-3-methyl-2-azetidinone

The compound obtained in Step 1 mentioned above (935 mg) was dissolved in dichloromethane (20 ml), and the solution was added with tert-butyldimethylsilyl trifluoromethanesulfonate (1.58 ml) and 2,6-lutidine (1.61 ml) at 0°C, stirred at room temperature for 3 days, further added with tert-butyldimethylsilyl trifluoromethanesulfonate (1.58 ml) and 2,6-lutidine (1.61 ml), and refluxed overnight by heating. The reaction mixture was added with water, and the layers were separated. The organic layer was successively washed with 10% aqueous citric acid and saturated brine, and dried over anhydrous sodium sulfate, and the solvent was evaporated under reduced pressure. The resulting residue was purified by silica gel chromatography [developing solvent; hexane: ethyl acetate=4:1 (v/v)] to obtain the title compound (703 mg) as colorless oil.
$^1$H-NMR ($CDCl_3$) δ : 0.21 (3H, s), 0.31 (3H, s), 0.91 (9H, s), 1.61 (3H, s), 1.74 (3H, t, J=19.1Hz), 3.79 (3H, s), 4.02-4.07 (1H, m), 6.85-6.90 (2H, m), 7.44-7.48 (2H, m).
EI-MS; m/z: 385 ($M^+$).

Step 3: (3R,4R)-3-[(tert-Butyldimethylsilyl)oxy]-4-(1,1-difluoroethyl)-3-methyl-2-azetidinone

The compound obtained in Step 2 mentioned above was used as a starting material to perform the same procedure as that of Reference Example 18, Step 2 and thereby to obtain the title compound as brown oil.
$^1$H-NMR ($CDCl_3$) δ : 0.15 (3H, s), 0.27 (3H, s), 0.89 (9H, s), 1.57 (3H, s), 1.71 (3H, dd, J=19.4, 18.1Hz), 3.54 (1H, dd, J=17.7, 4.4Hz), 5.99 (1H, brs).
FAB-MS; m/z: 280 $(M+H)^+$.

Step 4: (3R,4R)-1-Benzoyl-3-[(tert-butyldimethylsilyl)oxy]-4-(1,1-difluoroethyl)-3-methyl-2-azetidinone

The compound obtained in Step 3 mentioned above was used as a starting material to perform the same procedure as that of Reference Example 33 and thereby to obtain the title compound as colorless oil.
$^1$H-NMR ($CDCl_3$) δ : 0.23 (3H, s), 0.26 (3H, s), 0.93 (9H, s), 1.56 (3H, s), 1.79 (3H, dd, J=19.1, 18.4Hz), 4.37 (1H; dd, J=15.2, 6.1Hz), 7.47-7.56 (3H, m), 7.95-7.98 (2H, m).

[Reference Example 44]

(3R,4R)-4-[(1S)-Fluoroethyl]-1-(isopropoxycarbonyl)-3-[(triisopropylsilyl)oxy]-2-azetidinone

[Formula 52]

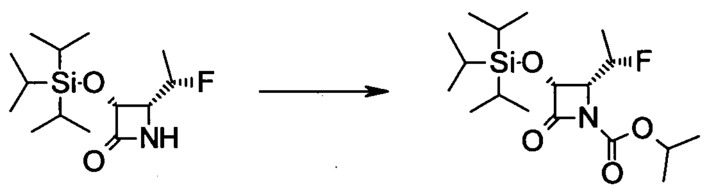

The compound obtained in Reference Example 31, Step 3 was used as a starting material to perform the same procedure as that of Reference Example 21 and thereby to obtain the title compound as colorless oil.
$^1$H-NMR ($CDCl_3$) δ : 1.05-1.23 (21H, m), 1.31-1.36 (6H, m), 1.46 (3H, dd, J=24.2, 6.6Hz), 4.25-4.34 (1H, m), 4.93-5.13 (3H, m).

[Reference Example 45]

[Formula 53]

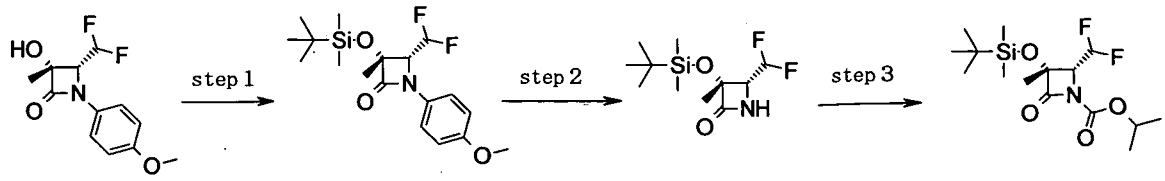


Step 1: (3R,4R)-3-[(tert-Butyldimethylsilyl)oxy]-4-difluoromethyl-1-(4-methoxyphenyl)-3-methyl-2-azetidinone

The compound obtained in Reference Example 32, Step 1 was used as a starting material to perform the same procedure as that of Reference Example 43, Step 2 and thereby to obtain the title compound as pale yellow amorphous solid.
$^1$H-NMR ($CDCl_3$) δ : 0.22 (3H, s), 0.29 (3H, s), 0.91 (9H, s), 1.61 (3H, s), 3.80 (3H, s), 3.97-4.03 (1H, m), 5.81-6.10 (1H, m), 6.86-6.90 (2H, m), 7.43-7.47 (2H, m).
EI-MS; m/z: 371 ($M^+$).

Step 2: (3R,4R)-3-[(tert-Butyldimethylsilyl)oxy]-4-difluoromethyl-3-methyl-2-azetidinone

The compound obtained in Step 1 mentioned above was used as a starting material to perform the same procedure as that of Reference Example 18, Step 2 and thereby to obtain the title compound as brown oil.
$^1$H-NMR ($CDCl_3$) δ : 0.18-0.24 (6H, m), 0.89 (9H, s), 1.58 (3H, s), 3.53-3.59 (1H, m), 5.62-5.93 (1H, m), 6.01 (1H, brs).

Step 3: (3R,4R)-3-[(tert-Butyldimethylsilyl)oxy]-4-difluoromethyl-1-(isopropoxycarbonyl)-3-methyl-2-azetidinone

The compound obtained in Step 2 mentioned above was used as a starting material to perform the same procedure as that of Reference Example 21 and thereby to obtain the title compound as colorless oil.
$^1$H-NMR ($CDCl_3$) δ : 0.18 (3H, s), 0.24 (3H, s), 0.88 (9H, s), 1.32-1.36 (6H, m), 1.61 (3H, s), 3.95 (1H, dt, J=8.6, 4.3Hz), 5.06 (1H, sep, J=6.3Hz), 5.93 (1H, ddd, J=55.4, 54.0,
4.4Hz).

[Reference Example 46]

[Formula 54]

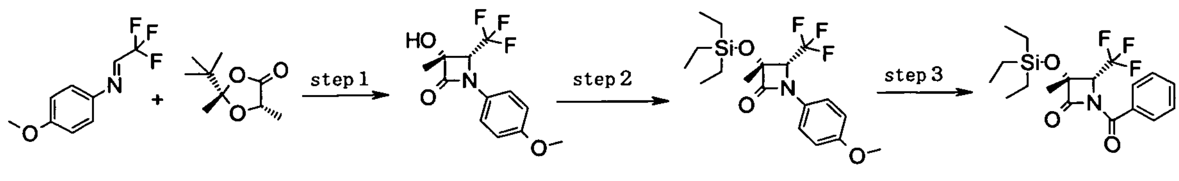


Step 1: (3R,4R)-3-Hydroxy-1-(4-methoxyphenyl)-3-methyl-4-trifluoromethyl-2-azetidinone

N-(2,2,2-Trifluoroethylidene)-4-methoxyaniline (J. Org. Chem., 1997, 62, 8826) was used as a starting material to perform the same procedure as that of Reference Example 32, Step 1 and thereby to obtain the title compound as pale red solid.
$^1$H-NMR ($CDCl_3$) δ : 1.72 (3H, s), 3.37 (1H, brs), 3.80 (3H, s), 4.34 (1H, q , J=5.8Hz), 6.88-6.92 (2H, m), 7.38-7.42 (2H, m).

Step 2: (3R,4R)-1-(4-Methoxyphenyl)-3-methyl-3-[(triethylsilyl)oxyl-4-trifluoromethyl-2-azetidinone

The compound obtained in Step 1 mentioned above was used as a starting material to perform the same procedure as that of Reference Example 19, Step 2 and thereby to obtain the title compound as pale yellow oil.
$^1$H-NMR ($CDCl_3$) δ : 0.68-0.79 (6H, m), 0.98 (9H, t, J=8.1Hz), 1.65 (3H, s), 3.79 (3H, s), 4.21 (1H, q , J=5.9Hz), 6.87-6.90

(2H, m), 7.38-7.41 (2H, m).

Step 3: (3R,4R)-1-Benzoyl-3-methyl-3-[(triethylsilyl)oxy]-4-trifluoromethyl-2-azetidinone

The compound obtained in Step 2 mentioned above was used as a starting material to perform the same procedure as that of Reference Example 18, Step 2, and then the same procedure as that of Reference Example 33 and thereby to obtain the title compound as colorless oil.
$^1$H-NMR ($CDCl_3$) δ : 0.67-0.79 (6H, m), 0.97-1.02 (9H, m), 1.64 (3H, s), 4.58 (1H, q , J=5.9Hz), 7.48-7.53 (2H, m), 7.61-7.66 (1H, m), 7.96-7.98 (2H, m).

[Reference Example 47]

3-Methylazetidine hydrochloride

[Formula 55]

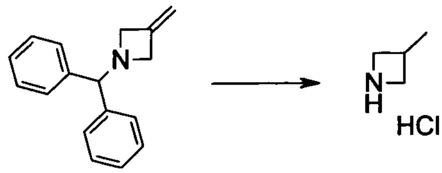

1-Benzhydryl-3-methyleneazetidine (Chem. Pharm. Bull., 1973, 21, 228, 3.45 g) was dissolved in ethanol (50 ml), the solution was added with 1 N hydrochloric acid in ethanol (30 ml) and 20% palladium hydroxide/carbon (1.50 g), and catalytic reduction was performed for 3 days under a hydrogen atmosphere. The catalyst was removed by filtration, and the reaction mixture was concentrated. Then, the resulting residue was added with 1 N aqueous hydrochloric acid and dichloromethane, and the mixture was stirred for 10 minutes. The aqueous layer was evaporated under reduced pressure to obtain the title compound (1.54 g) as pale yellow oil.
$^1$H-NMR (400MHz, $d_6$-DMSO) δ : 1.16 (3H, d, J=6.8Hz), 2.78-2.86 (1H, m), 3.43-3.58 (2H, m), 3.77-4.01 (2H, m), 9.26 (2H, brs).

[Reference Example 48]

[Formula 56]

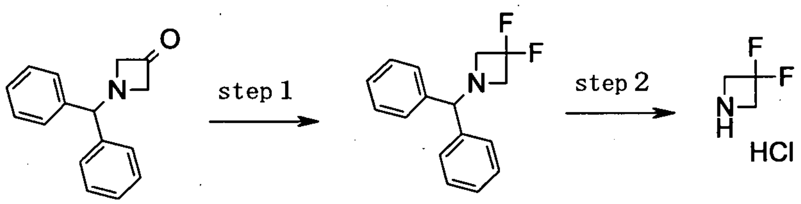


Step 1: 1-Benzhydryl-3,3-difluoroazetidine

1-Benzhydrylazetidin-3-one was used as a starting material to perform the same procedure as that of Reference Example 18, Step 1 using bis(methoxyethyl)aminosulfur trifluoride instead of diethylaminosulfur trifluoride and thereby to obtain the title compound as pale yellow oil.
$^1$H-NMR (400MHz, $CDCl_3$) δ : 3.51 (4H, t, J=12.2Hz), 4.44 (1H, s), 7.20 (2H, tt, J=7.3, 1.6Hz), 7.26-7.30 (4H, m), 7.41-7.44 (4H, m).
ESI-MS; m/z: 260 $(M+H)^+$.

Step 2: 3,3-Difluoroazetidine hydrochloride

The compound obtained in Step 1 mentioned above was used as a starting material to perform the same procedure as that of Reference Example 1 and thereby to obtain the title compound as colorless solid.
$^1$H-NMR (400MHz, $d_6$-DMSO) δ : 4.48 (4H, t, J=11.8Hz), 10.06 (2H, s).

[Reference Example 49]

[Formula 57]

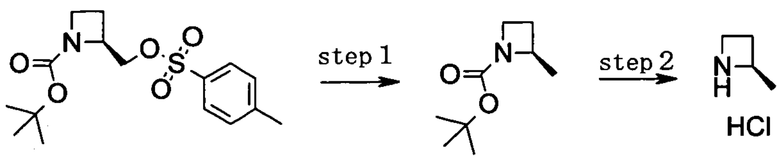


Step 1: (R)-2-Methylazetidinecarboxylic acid tert-butyl ester

(S)-2-(Toluene-4-sulfonyloxymethyl)azetidinecarboxylic acid tert-butyl ester (J. Med. Chem., 2005, 24, 7637, 6.50 g) was dissolved in tetrahydrofuran (16 ml), and the solution was added dropwise with lithium triethylborohydride (1 mole solution in tetrahydrofuran, 76.2 ml) at 0°C, and stirred at room temperature for 14 hours. The reaction mixture was added with water, and extracted with ethyl acetate. The organic layer was successively washed with 1 N aqueous hydrochloric acid, saturated aqueous sodium hydrogencarbonate and saturated brine, and then dried over anhydrous magnesium sulfate, and the solvent was evaporated under reduced pressure. The resulting residue was purified by silica gel column chromatography [developing solvent; ethyl acetate:n-hexane=1:19 to 1:1 (v/v)] to obtain the title compound (1.48 g) as colorless oil.
$^1$H-NMR (400MHz, $CDCl_3$) δ : 1.37 (3H, d, J=6.13Hz), 1.44 (9H, s), 1.72-1.81 (1H, m), 2.23-2.32 (1H, m), 3.81 (2H, t, J=7.6Hz), 4.23-4.32 (1H, m).

Step 2: (R)-2-Methylazetidine hydrochloride

The compound obtained in Step 1 mentioned above was used as a starting material to perform the same procedure as that of Reference Example 25 and thereby to obtain the title compound as pale yellow oil.
$^1$H-NMR (400MHz, $CDCl_3$) δ : 1.66 (3H, d, J=6.6Hz), 2.25-2.36 (1H, m), 2.53-2.64 (1H, m), 3.89-4.06 (2H, m), 4.56-4.67 (1H, m), 9.60 (2H, brs).

[Reference Example 50]

[Formula 58]

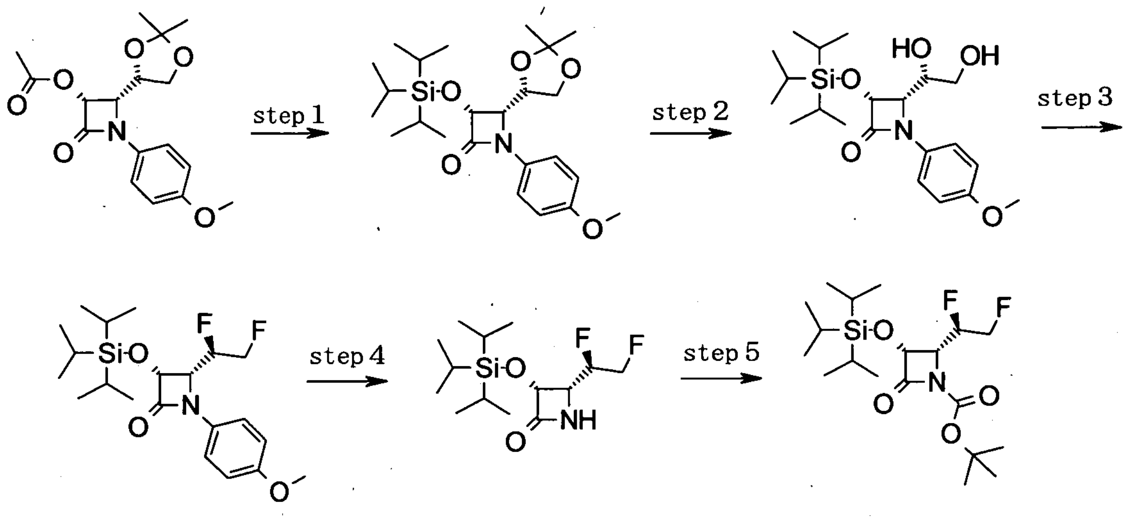


Step 1: (3R,4S)-4-[(4S)-2,2-Dimethyl-1,3-dioxolan-4-yl]-1-(4-methoxyphenyl)-3-[(triisopropylsilyl)oxy]-2-azetidinone

(3R,4S)-3-Acetoxy-4-[(4S)-2,2-dimethyl-1,3-dioxolan-4-yl]-1-(4-methoxyphenyl)-2-azetidinone (J. Org. Chem., 1988, 53, 4227) was used as a starting material to perform the same procedure as that of Reference Example 20, Step 2 and thereby to obtain the title compound as colorless oil.
$^1$H-NMR (400MHz, $CDCl_3$) δ : 1.10-1.16 (18H, m), 1.18-1.31 (3H, m), 1.54 (3H, s), 1.58 (3H, s), 3.79 (3H, s), 3.83 (1H, dd, J=8.8, 6.1Hz), 4.16 (1H, dd, J=8.8, 6.1Hz), 4.28 (1H, dd, J=8.8, 6.8Hz), 4.37-4.47 (1H, m), 5.05 (1H, d, J=5.4Hz), 6.83-6.86 (2H, m), 7.62-7.69 (2H, m).

Step 2: (3R,4S)-4-[(1S)-1,2-Dihydroxyethyl]-1-(4-methoxyphenyl)-3-[(triisopropylsilyl)oxy]-2-azetidinone

The compound obtained in Step 1 mentioned above (25 g) was dissolved in tetrahydrofuran:water (3.5:1, 380 ml), and the solution was added with p-toluenesulfonic acid monohydrate (3.17 g, 16.7 mmol), stirred at 60°C for 24 hours, and neutralized with sodium hydrogencarbonate under ice cooling. The solvent was evaporated under reduced pressure, and the resulting residue was purified by silica gel chromatography [developing solvent; ethyl acetate:n-hexane=1:8 to 1:2 (v/v)] to obtain the title compound (13.5 g) as pale yellow oil.
$^1$H-NMR (400MHz, $CDCl_3$) δ: 1.14 (18H, t, J=7.1Hz), 1.19-1.33 (3H, m), 2.07 (1H, dd, J=7.8, 3.9Hz), 3.04 (1H, d, J=2.9Hz), 3.63-3.75 (2H, m), 3.78 (3H, s), 4.16-4.23 (1H, m), 4.37 (1H, dd, J=5.2, 2.8Hz), 5.13 (1H, d, J=5.2Hz), 6.86 (2H, dt, J=9.8, 2.8Hz), 7.38 (2H, dt, J=9.8, 2.8Hz).

Step 3: (3R,4R)-4-[(1R)-1,2-Difluoroethyl]-1-(4-methoxyphenyl)-3-[(triisopropylsilyl)oxy]-2-azetidinone

The compound obtained in Step 2 mentioned above was used as a starting material to perform the same procedure as that of Reference Example 18, Step 1 using bis(methoxyethyl)aminosulfur trifluoride instead of diethylaminosulfur trifluoride and thereby to obtain the title compound as pale yellow oil.
$^1$H-NMR (400MHz, $CDCl_3$) δ : 0.97-1.29 (21H, m), 3.79 (3H, s), 4.38-4.60 (2H, m), 4.72-4.95 (1H, m), 5.00-5.29 (1H, m), 5.21 (1H, d, J=5.1Hz), 6.88 (2H, dt, J=9.8, 2.8Hz), 7.46 (2H, dt, J=9.8, 2.8Hz).
ESI-MS; m/z: 414 $(M+H)^+$.

Step 4: (3R,4R)-4-[(1R)-1,2-Difluoroethyl]-3-[(triisopropylsilyl)oxy]-2-azetidinone

The compound obtained in Step 3 mentioned above was used as a starting material to perform the same procedure as that of Reference Example 18, Step 2 and thereby to obtain the title compound as colorless solid.
$^1$H-NMR (400MHz, $CDCl_3$) δ : 1.10 (18H, t, J=6.6Hz), 1.13-1.32 (3H, m), 4.03-4.15 (1H, m), 4.53-5.04 (3H, m), 5.16 (1H, dd, J=4.9, 3.4Hz), 5.95 (1H, brs).

Step 5: (3R,4R)-1-(tert-Butoxycarbonyl)-4-[(1R)-1,2-difluoroethyl]-3-[(triisopropylsilyl)oxy]-2-azetidinone

The compound obtained in Step 4 mentioned above was used as a starting material to perform the same

procedure as that of Reference Example 18, Step 4 and thereby to obtain the title compound as pale yellow solid. $^1$H-NMR (400MHz, $CDCl_3$) δ : 1.09 (18H, t, J=6.2Hz), 1.14-1.29 (3H, m), 1.52 (9H, s), 4.25-4.34 (1H, m), 4.61-4.82 (2H, m), 4.94-5.13 (1H, m), 5.16 (1H, d, J=5.9Hz).

[Reference Example 51]

[Formula 59]

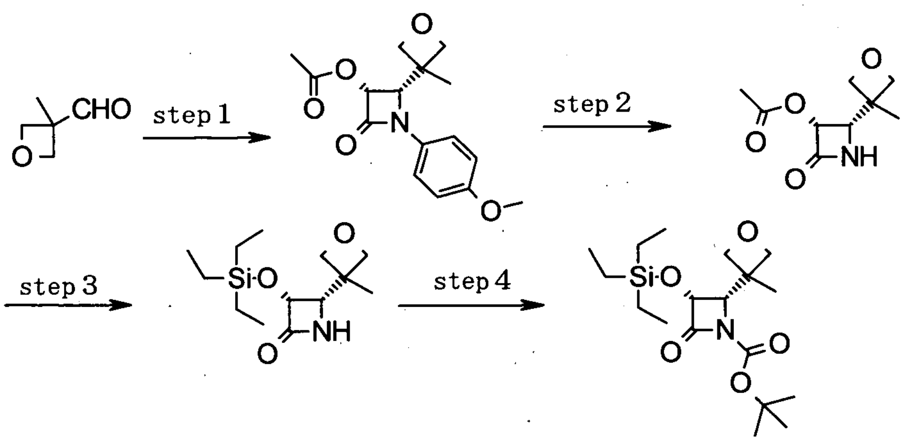


Step 1: cis-3-Acetoxy-1-(4-methoxyphenyl)-4-(3-methyl-3-oxetanyl)-2-azetidinone

3-Methyloxetane-3-carboxyaldehyde (U.S. Patent No. 5,354,865) was used as a starting material to perform the same procedure as that of Reference Example 20, Step 1 and thereby to obtain the title compound as colorless solid. $^1$H-NMR (400MHz, $CDCl_3$) δ : 1.46 (3H, s), 2.13 (3H, s), 3.80 (3H, s), 4.25 (1H, d, J=6.4Hz), 4.36 (1H, d, J=6.4Hz), 4.58 (1H, d, J=6.4Hz), 4.69 (1H, d, J=5.2Hz), 4.92 (1H, d, J=6.4Hz), 6.15 (1H, d, J=5.2Hz), 6.87-6.91 (2H, m), 7.18-7.20 (2H, m). ESI-MS; m/z: 306 $(M+H)^+$.

Step 2: cis-3-Acetoxy-4-(3-methyl-3-oxetanyl)-2-azetidinone

The compound obtained in Step 1 mentioned above was used as a starting material to perform the same procedure as that of Reference Example 18, Step 2 and thereby to obtain the title compound as pale yellow solid. $^1$H-NMR (400MHz, $d_6$-DMSO) δ : 1.21 (3H, s), 2.05 (3H, s), 3.78 (1H, d, J=4.9Hz), 4.17 (2H, dd, J=5.9,1.6Hz), 4.43 (1H, d, J=5.9Hz), 4.58 (1H, d, J=6.3Hz), 5.83 (1H, dd, J=2.7, 4.9Hz), 8.94 (1H, brs).
ESI-MS; m/z: 200 $(M+H)^+$.

Step 3: cis-4-(3-Methyl-3-oxetanyl)-3-[(triethylsilyl)oxy]-2-azetidinone

The compound obtained in Step 2 mentioned above was used as a starting material and reacted with triethylsilyl chloride instead of triisopropylsilyl chloride to perform the same procedure as that of Reference Example 20, Step 2 and thereby to obtain the title compound as colorless oil.
$^1$H-NMR (400MHz, $CDCl_3$) δ : 0.62-0.74 (6H, m), 0.96 (9H, t, J=7.9Hz), 1.41 (3H, s), 3.69 (1H, d, J=4.9Hz), 4.31 (1H, d, J=6.6Hz), 4.38 (1H, d, J=6.2Hz), 4.45 (1H, d, J=6.2Hz), 4.82 (1H, d, J=6.6Hz), 4.87 (1H, dd, J=2.8, 4.9Hz), 6.25 (1H, brs).

Step 4: cis-1-(tert-Butoxycarbonyl)-4-(3-methyl-3-oxetanyl)-3-[(triethylsilyl)oxy]-2-azetidinone

The compound obtained in Step 3 mentioned above was used as a starting material to perform the same procedure as that of Reference Example 18, Step 4 and thereby to obtain the title compound as colorless oil.
$^1$H-NMR (400MHz, $CDCl_3$) δ : 0.59-0.69 (6H, m), 0.96 (9H, t, J=7.8Hz), 1.44 (3H, s), 1.51 (9H, s), 4.38 (1H, d, J=6.3Hz), 4.40 (1H, d, J=6.3Hz), 4.47 (1H, d, J=6.1Hz), 4.64 (1H, d, J=6.3Hz), 4.81 (1H, d, J=6.3Hz), 4.93 (1H, d, J=6.1Hz).

ESI-MS; m/z: 372 $(M+H)^+$.

[Reference Example 52]

(S)-1-Azetidin-2-ylmethanol oxalate

[Formula 60]

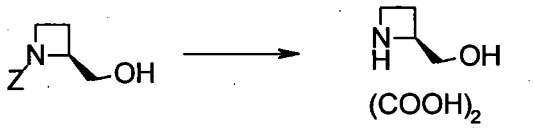

(S)-2-(Hydroxymethyl)-1-azetidinecarboxylic acid benzyl ester (Tetrahedron Lett., 2004, 45, 3607, 1.0 g) was dissolved in ethanol (10 ml), the solution was added with palladium/carbon (200 mg), and catalytic reduction was performed overnight under a hydrogen atmosphere. The catalyst was removed by filtration, and the solution was added with oxalic acid (244 mg), concentrated, and then added with ethanol and isopropanol. The deposited solid was collected by filtration, and dried to obtain the title compound (324 mg) as colorless solid.
$^1$H-NMR (400MHz, $D_2O$) δ : 2.40-2.58 (2H, m), 3.85-3.86 (2H, d, J=4.8Hz), 3.90-3.96 (1H, m), 4.04-4.11 (1H, m), 4.59-4.65 (1H, m).
FAB-MS; m/z: 88 $(M+H)^+$.

[Reference Example 53]

(S)-1-Azetidin-2-ylmethanol fumarate

[Formula 61]

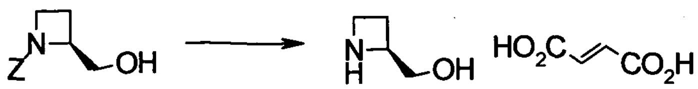

(S)-2-(Hydroxymethyl)-1-azetidinecarboxylic acid benzyl ester (500 mg) was dissolved in ethanol (5 ml), and the solution was added with palladium/carbon (100 mg), and catalytic reduction was performed for 2 hours under a hydrogen atmosphere. The catalyst was removed by filtration, and the solution was added with fumaric acid (262 mg), concentrated, and then added with ethyl acetate and isopropanol. The deposited solid was collected by filtration, and dried to obtain the title compound (389 mg) as colorless solid.
$^1$H-NMR (400MHz, $D_2O$) δ : 2.40-2.58 (2H, m), 3.85-3.86 (2H, d, J=4.8Hz), 3.90-3.96 (1H, m), 4.04-4.11 (1H, m), 4.59-4.65 (1H, m), 6.71 (2H, s).
FAB-MS; m/z: 88 $(M+H)^+$.

[Reference Example 54]

[Formula 62]

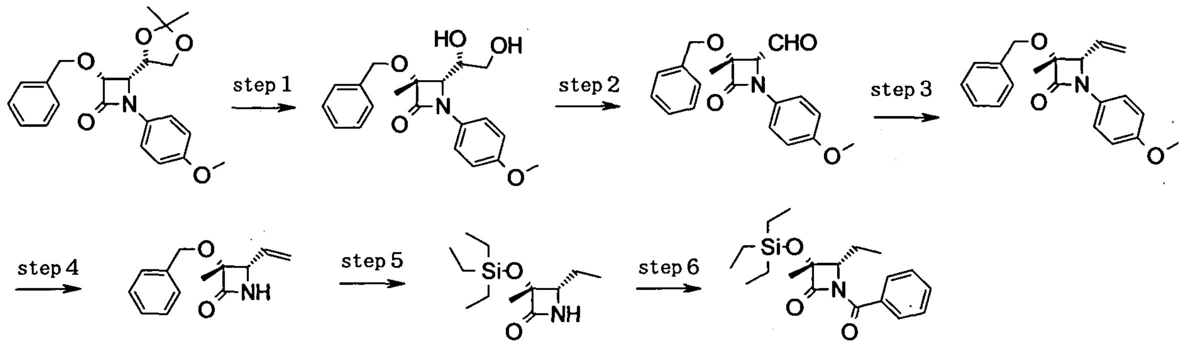


Step 1: (3R,4S)-3-(Benzyloxy)-4-[(1S)-1,2-dihydroxyethyl]-1-(4-methoxyphenyl)-3-methyl-2-azetidinone

(3R,4S)-3-Benzyloxy-4-[(4S)-2,2-dimethyl-1,3-dioxolan-4-yl]-1-(4-methoxyphen yl)-2-azetidinone (J. Org. Chem., 1998, 53, 4227, 3.75 g) was dissolved in tetrahydrofuran (70 ml), and the solution was added with lithium bis (trimethylsilyl)amide (1 mole solution in tetrahydrofuran, 24.5 ml) at -78°C, and stirred at the same temperature for 2.5 hours. The reaction mixture was added with methyl iodide (2.44 ml), and stirred at room temperature for 2 hours. The reaction mixture was added with saturated aqueous ammonium chloride, and extracted with ethyl acetate, and the organic layer was washed with saturated brine, and then dried over anhydrous sodium sulfate. The solvent was evaporated under reduced pressure, and the resulting residue was dissolved in tetrahydrofuran (80 ml). The solution was added with water (40 ml) and p-toluenesulfonic acid monohydrate (0.37 g), and refluxed for 2 days by heating. The reaction mixture was concentrated under reduced pressure, and the residue was added with saturated aqueous sodium hydrogencarbonate, and extracted with ethyl acetate. The organic layer was washed with saturated brine, and then dried over anhydrous sodium sulfate, and the solvent was evaporated under reduced pressure. The resulting residue was purified by silica gel column chromatography [developing solvent; ethyl acetate:n-hexane=1:3 (v/v)] to obtain the title compound (3.0 g) as brown oil.
$^1$H-NMR (400MHz, $CDCl_3$) δ : 1.72 (3H, s), 2.82 (1H, d, J=4.1Hz), 3.66-3.71 (2H, m), 3.80 (3H, s), 4.09 (1H, d, J=4.9Hz), 4.18-4.23 (1H, m), 4.86 (1H, d, J=11.2Hz), 5.02 (1H, d, J=11.0Hz), 6.88 (2H, d, J=9.0Hz), 7.31-7.39 (5H, m), 7.44 (2H, d, J=9.0Hz).

Step 2: (3R,4R)-3-(Benzyloxy)-4-formyl-1-(4-methoxyphenyl)-3-methyl-2-azetidinone

The compound obtained in Step 1 mentioned above (3.0 g) was dissolved in ethyl acetate (30 ml), and the solution was added with water (15 ml) and sodium metaperiodate (3.6 g), and stirred at 50°C for 3 hours. Two of the layers were separated, and then the aqueous layer was extracted with ethyl acetate. The organic layers were combined, washed with saturated aqueous sodium hydrogencarbonate and saturated brine, and then dried over anhydrous sodium sulfate, and the solvent was evaporated under reduced pressure to obtain the title compound (2.4 g) as pale brown solid.
$^1$H-NMR (400MHz, $CDCl_3$) δ : 1.80 (3H, s), 3.80 (3H, s), 4.28 (1H, d, J=3.4Hz), 4.77 (1H, d, J=11.0Hz), 4.85 (1H, d, J=11.0Hz), 6.88-6.91 (2H, m), 7.25-7.34 (7H, m), 9.77 (1H, d, J=3.3Hz).

Step 3: (3R,4S)-3-(Benzyloxy)-1-(4-methoxyphenyl)-3-methyl-4-vinyl-2-azetidinone

Methyltriphenylphosphonium bromide (5.4 g) was suspended in tetrahydrofuran (50 ml), and the suspension was added with n-butyl lithium (1.59 mole solution in n-hexane, 9.4 ml) at -78°C, and stirred at room temperature for 20 minutes. The reaction mixture was cooled to -78°C, added with a solution of the compound obtained in Step 2 mentioned above (3.25 g) in tetrahydrofuran (30 ml), and stirred overnight at room temperature. The reaction mixture was added with saturated aqueous ammonium chloride, and extracted with ethyl acetate. The organic layer was washed with saturated brine, and then dried over anhydrous sodium sulfate, and the solvent was evaporated under reduced pressure. The resulting residue was purified by silica gel column chromatography [developing solvent; ethyl acetate:n-hexane=1:5 (v/v)] to obtain the title compound (2.2 g) as yellow solid.
$^1$H-NMR (400MHz, $CDCl_3$) δ : 1.70 (3H, s), 3.79 (3H, s), 4.33 (1H, d, J=8.0Hz), 4.75 (1H, d, J=11.2Hz), 4.87 (1H, d, J=11.2Hz), 5.46 (1H, d, J=11.0Hz), 5.54 (1H, d, J=17.3Hz), 6.06 (1H, ddd, J=17.9, 9.8, 7.5Hz), 6.86 (2H, d, J=9.8Hz), 7.27-7.42 (7H, m).

Step 4: (3R,4S)-3-(Benzyloxy)-3-methyl-4-vinyl-2-azetidinone

The compound obtained in Step 3 mentioned above was used as a starting material to perform the same procedure as that of Reference Example 18, Step 2 and thereby to obtain the title compound as brown oil.
$^1$H-NMR (400MHz, $CDCl_3$) δ : 1.66 (3H, s), 3.99 (1H, d, J=8.3Hz), 4.69 (1H, d, J=11.2Hz), 4.82 (1H, d, J=11.2Hz), 5.30-5.39 (2H, m), 5.97-6.10 (2H, m), 7.28-7.36 (5H, m).

Step 5: (3R,4S)-4-Ethyl-3-methyl-3-[(triethylsilyl)oxy]-2-azetidinone

The compound obtained in Step 4 mentioned above was used as a starting material to perform the same procedure as that of Reference Example 18, Step 3 and thereby to obtain the title compound as colorless oil.
$^1$H-NMR (400MHz, $CDCl_3$) δ : 0.63-0.79 (6H, m), 0.90-1.00 (12H, m), 1.48-1.73 (2H, m), 1.60 (3H, s), 3.26 (1H, t, J=7.1Hz), 5.90 (1H, brs).

Step 6: (3R,4S)-1-Benzoyl-4-ethyl-3-methyl-3-[(triethylsilyl)oxy]-2-azetidinone

The compound obtained in Step 5 mentioned above was used as a starting material to perform the same procedure as that of Reference Example 33 and thereby to obtain the title compound as colorless oil.
$^1$H-NMR (400MHz, $CDCl_3$) δ : 0.68-0.81 (6H, m), 0.97-1.05 (12H, m), 1.57 (3H, s), 1.74-1.86 (1H, m), 1.95-2.07 (1H, m), 3.94 (1H, dd, J=9.3, 4.1Hz), 7.46 (2H, t, J=7.8Hz), 7.57 (1H, t, J=7.3Hz), 7.91 (2H, d, J=9.0Hz).

[Reference Example 55]

[Formula 63]

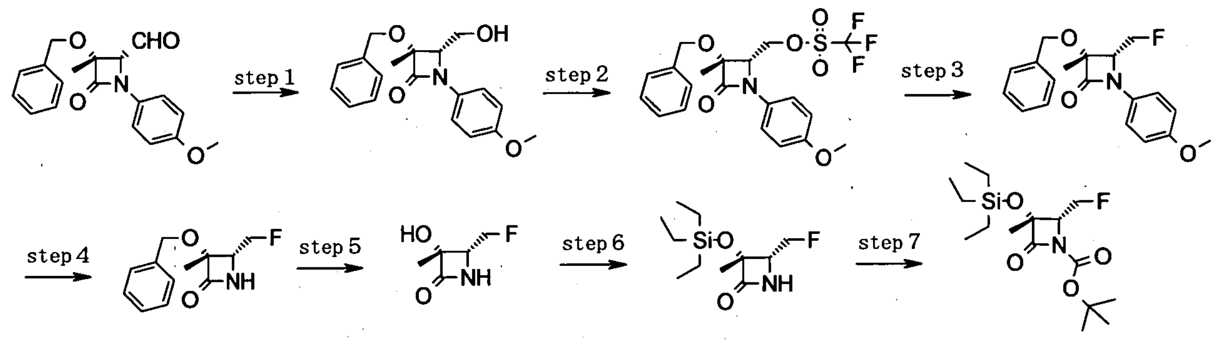


Step 1: (3R,4S)-3-(Benzyloxy)-4-(hydroxymethyl)-1-(4-methoxyphenyl)-3-methyl-2-azetidinone

The compound obtained in Reference Example 54, Step 2 (3.00 g) was dissolved in a mixed solvent of methanol (30 ml) and tetrahydrofuran (30 ml), and the solution was added with sodium borohydride (291 mg), and stirred at room temperature for 20 minutes. The reaction mixture was added with water and ethyl acetate, and the layers were separated. Organic substances were extracted from the aqueous layer with ethyl acetate. The organic layers were combined, washed with saturated brine, and dried over anhydrous sodium sulfate, and the solvent was evaporated under reduced pressure. The resulting residue was purified by silica gel column chromatography [developing solvent; ethyl acetate:n-hexane=1:2 (v/v)] to obtain the title compound (3.01 g) as colorless solid.
$^1$H-NMR (400MHz, $CDCl_3$) δ : 1.74 (3H, s), 2.52 (1H, t, J=6.9Hz), 3.79 (3H, s), 3.97-4.09 (3H, m), 4.86 (1H, d, J=10.8Hz), 5.01 (1H, d, J=11.0Hz), 6.87-6.91 (2H, m), 7.28-7.41 (7H, m).

Step 2: (3R,4S)-3-(Benzyloxy)-1-(4-methoxyphenyl)-3-methyl-4-(trifluoromethanesulfonyloxymethyl)-2-azetidinone

The compound obtained in Step 1 mentioned above (100 mg) was dissolved in dichloromethane (5 ml), and the solution was added with trifluoromethanesulfonic acid anhydride (103 μ 1) and triethylamine (106 μ 1) at 0°C, returned to room temperature, stirred for 1 hour, further added with trifluoromethanesulfonic acid anhydride (103 μ 1) and triethylamine (106 μl), and stirred at room temperature for 15 minutes. The reaction mixture was added with water, and the

layers were separated. Organic substances were extracted from the aqueous layer with dichloromethane. The organic layers were combined, washed with saturated brine, and dried over anhydrous sodium sulfate, and the solvent was evaporated under reduced pressure. The resulting residue was purified by silica gel column chromatography [developing solvent; ethyl acetate:n-hexane=1:17 (v/v)] to obtain the title compound (126 mg) as pale yellow oil.
$^1$H-NMR (400MHz, $CDCl_3$) δ : 1.74 (3H, s), 3.80 (3H, s), 4.24 (1H, t, J=5.4Hz), 4.72 (1H, dd, J=11.0, 5.9Hz), 4.84 (1H, d, J=11.3Hz), 4.92-4.97 (1H, m), 4.92 (1H, d, J=11.5Hz), 6.88-6.93 (2H, m), 7.29-7.41 (7H, m).
ESI-MS; m/z: 460 $(M+H)^+$.

Step 3: (3R,4R)-3-(Benzyloxy)-4-fluoromethyl-1-(4-methoxyphenyl)-3-methyl-2-azetidinone

The compound obtained in Step 2 mentioned above (126 mg) was dissolved in acetonitrile (5 ml), and the solution was added with tetra-n-butylammonium fluoride (1 mole solution in tetrahydrofuran, 1.26 ml), and stirred at room temperature for 45 minutes. The reaction mixture was added with water and ethyl acetate, and the layers were separated. Organic substances were extracted from the aqueous layer with ethyl acetate. The organic layers were combined, washed with saturated brine, and dried over anhydrous sodium sulfate, and the solvent was evaporated under reduced pressure. The resulting residue was purified by silica gel column chromatography [developing solvent; ethyl acetate:n-hexane=1:17 (v/v)] to obtain the title compound (73 mg) as colorless amorphous solid.
$^1$H-NMR (400MHz, $CDCl_3$) δ : 1.72 (3H, s), 3.79 (3H, s), 4.18 (1H, ddd, J=14.5, 6.9, 2.9Hz), 4.65-4.99 (4H, m), 6.87-6.91 (2H, m), 7.26-7.37 (5H, m), 7.46-7.51 (2H, m).
ESI-MS; m/z: 330 $(M+H)^+$.

Step 4: (3R,4R)-3-(Benzyloxy)-4-fluoromethyl-3-methyl-2-azetidinone

The compound obtained in Step 3 mentioned above was used as a starting material to perform the same procedure as that of Reference Example 18, Step 2 and thereby to obtain the title compound as brown oil.
$^1$H-NMR (400MHz, $CDCl_3$) δ : 1.68 (3H, s), 3.76-3.82 (1H, m), 4.49-4.80 (2H, m), 4.71 (1H, d, J=11.3Hz), 4.84 (1H, d, J=11.3Hz), 6.35 (1H, brs), 7.26-7.36 (5H, m).
ESI-MS; m/z: 224 $(M+H)^+$.

Step 5: (3R,4R)-4-Fluoromethyl-3-hydroxy-3-methyl-2-azetidinone

The compound obtained in Step 4 mentioned above was used as a starting material to perform the same procedure as that of Reference Example 19, Step 1 and thereby to obtain the title compound as colorless solid.
$^1$H-NMR (400MHz, $CDCl_3$) δ : 1.61 (3H, s), 3.39 (1H, d, J=2.4Hz), 3.76 (1H, ddd, J=15.9, 6.4, 3.6Hz), 4.56-4.83 (2H, m), 6.29 (1H, brs).
ESI-MS; m/z: 134 $(M+H)^+$.

Step 6: (3R,4R)-4-Fluoromethyl-3-methyl-3-[(triethylsilyl)oxy]-2-azetidinone

The compound obtained in Step 5 mentioned above was used as a starting material to perform the same procedure as that of Reference Example 19, Step 2 and thereby to obtain the title compound as pale yellow oil.
$^1$H-NMR (400MHz, $CDCl_3$) δ : 0.61-0.76 (6H, m), 0.96 (9H, t, J=8.1Hz), 1.56 (3H, s), 3.66-3.73 (1H, m), 4.47 (1H, ddd, J=48.4, 9.7, 8.5Hz), 4.67 (1H, ddd, J=46.6, 10.0, 3.7Hz), 6.21 (1H, brs).
ESI-MS; m/z: 248 $(M+H)^+$.

Step 7: (3R,4R)-1-(tert-Butoxycarbonyl)-4-fluoromethyl-3-methyl-3-[(triethylsilyl)oxy]-2-azetidinone

The compound obtained in Step 6 mentioned above was used as a starting material to perform the same procedure as that of Reference Example 18, Step 4 and thereby to obtain the title compound as colorless oil.
$^1$H-NMR (400MHz, $CDCl_3$) δ : 0.64-0.74 (6H, m), 0.96 (9H, t, J=7.8Hz), 1.53 (9H, s), 1.59 (3H, s), 3.91 (1H, ddd, J=15.1, 5.5, 4.2Hz), 4.59-4.80 (2H, m).
ESI-MS; m/z: 348 $(M+H)^+$.

[Reference Example 56]

(3R,4R)-3-[(tert-Butyldimethylsilyl)oxy]-4-(1,1-difluoroethyl)-1-(isopropoxycarbonyl)-3-methyl-2-azetidinone

[Formula 64]

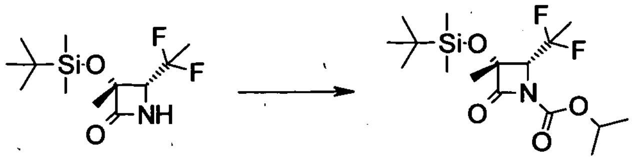

The compound obtained in Reference Example 43, Step 3 was used as a starting material to perform the same procedure as that of Reference Example 21 and thereby to obtain the title compound as pale yellow oil.
$^1$H-NMR (400MHz, $CDCl_3$) δ : 0.17 (3H, s), 0.26 (3H, s), 0.89 (9H, s), 1.32-1.36 (6H, m), 1.61 (3H, s), 1.74 (3H, t, J=18.7Hz), 3.96 (1H, dd, J=15.0, 4.9Hz), 5.06 (1H, septet, J=6.2Hz).

[Reference Example 57]

(3R,4R)-1-(Isopropoxycarbonyl)-3-methyl-3-[(triethylsilyl)oxy]-4-trifluoromethyl-2-azetidinone

[Formula 65]

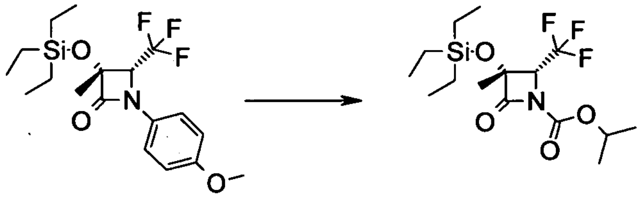

The compound obtained in Reference Example 46, Step 2 was used as a starting material, subjected to the same procedure as that of Reference Example 18, Step 2, and then the same procedure as that of Reference Example 21 and thereby to obtain the title compound as yellow oil.
$^1$H-NMR ($CDCl_3$) δ : 0.64-0.77 (6H, m), 0.96 (9H, t, J=8.1Hz), 1.32-1.36 (6H, m), 1.65 (3H, s), 4.14 (1H, q , J=5.9Hz), 5.07 (1H, septet, J=6.3Hz).

[Reference Example 58]

[Formula 66]

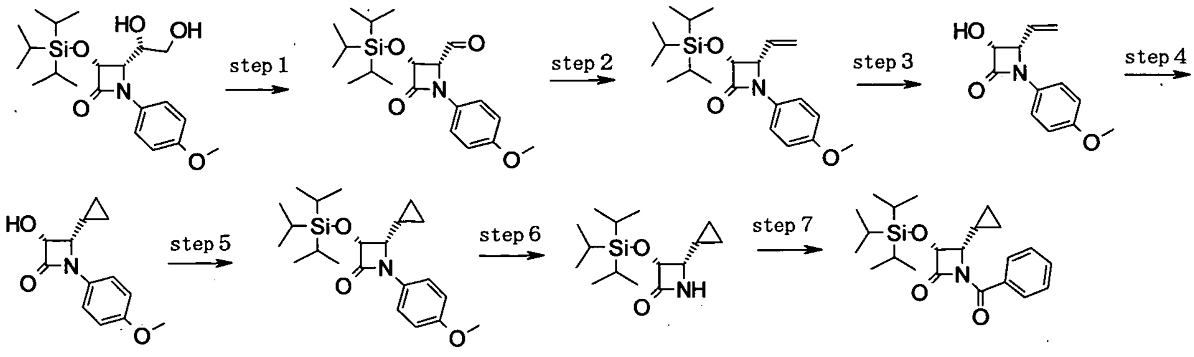


Step 1: (3R,4R)-4-Formyl-(4-methoxyphenyl)-3-[(triisopropylsilyl)oxy]-2-azetidinone

The compound obtained in Reference Example 50, Step 2 was used as a starting material to perform the same procedure as that of Reference Example 54, Step 2 and thereby to obtain the title compound as pale yellow solid.
$^1$H-NMR (400MHz, $CDCl_3$) δ : 1.07-1.27 (21H, m), 3.78 (3H, s), 4.46 (1H, dd, J=4.2, 5.4Hz), 5.30 (1H, d, J=5.4Hz), 6.84-6.88 (2H, m), 7.24-7.28 (2H, m), 9.77 (1H, d, J=4.2Hz).

Step 2: (3R,4S)-(4-Methoxyphenyl)-3-[(triisopropylsilyl)oxy]-4-vinyl-2-azetidinone

The compound obtained in Step 1 mentioned above was used as a starting material to perform the same procedure as that of Reference Example 54, Step 3 and thereby to obtain the title compound as colorless solid.
$^1$H-NMR (400MHz, $CDCl_3$) δ : 1.05-1.29 (21H, m), 3.78 (3H, s), 4.57 (1H, dd, J=8.3, 5.2Hz), 5.10 (1H, d, J=5.2Hz), 5.44-5.53 (2H, m), 5.93-6.02 (1H, m), 6.84 (2H, d, J=9.1Hz), 7.38 (2H, d, J=9.1Hz).
ESI-MS; m/z: 376 $(M+H)^+$.

Step 3: (3R,4S)-3-Hydroxy-(4-methoxyphenyl)-4-vinyl-2-azetidinone

The compound obtained in Step 2 mentioned above (4.80 g) was dissolved in tetrahydrofuran (100 ml), and the solution was added with tetra-n-butylammonium fluoride (1 mole solution in tetrahydrofuran, 19.2 ml) at 0°C, and stirred at 0°C for 1 hour. The reaction mixture was diluted with water, and extracted with ethyl acetate. The organic layer was washed with saturated brine, and then dried over anhydrous sodium sulfate, and the solvent was evaporated under reduced pressure. The resulting residue was purified by silica gel column chromatography [developing solvent; ethyl acetate:n-hexane=5:9 to 1:1 (v/v)] to obtain the title compound (2.31 g) as colorless solid.
$^1$H-NMR (400MHz, $CDCl_3$) δ : 2.90 (1H, d, J=8.3Hz), 3.78 (3H, s), 4.70 (1H, td, J=6.1, 0.6Hz), 5.07 (1H, dd, J=8.2, 5.3Hz), 5.47-5.58 (2H, m), 5.94-6.03 (1H, m), 6.85 (2H, d, J=8.8Hz), 7.36 (2H, d, J=8.8Hz).
ESI-MS; m/z: 220 $(M+H)^+$.

Step 4: (3R,4S)-4-Cyclopropyl-3-hydroxy-(4-methoxyphenyl)-2-azetidinone

The compound obtained in Step 3 mentioned above (1.16 g) was dissolved in 1,2-dichloroethane (50 ml), and the solution was added with diethylzinc (1 mole solution in n-hexane, 62.9 ml) at room temperature under an argon atmosphere, and stirred for 10 minutes. Then, this reaction solution was added dropwise with diiodomethane (10.1 ml), and stirred at room temperature for 7 hours. The reaction mixture was added with saturated aqueous ammonium chloride, and extracted with ethyl acetate. The organic layer was washed with saturated brine, and then dried over anhydrous sodium sulfate, and the solvent was evaporated under reduced pressure. The resulting residue was purified by silica gel column chromatography [developing solvent; ethyl acetate:n-hexane=1:4 to 1:1 (v/v)] to obtain the title compound (0.62 g) as colorless solid.
$^1$H-NMR (400MHz, $CDCl_3$) δ : 0.33-0.39 (1H, m), 0.51-0.57 (1H, m), 0.75-0.88 (2H, m), 1.02-1.11 (1H, m), 3.45 (1H, d, J=7.1Hz), 3.52 (1H, dd, J=9.1, 5.2Hz), 3.79 (3H, s), 4.99 (1H, dd, J=6.9, 4.9Hz), 6.87 (2H, d, J=8.8Hz), 7.44 (2H, d, J=8.8Hz).
ESI-MS; m/z: 234 $(M+H)^+$.

Step 5: (3R,4S)-4-Cyclopropyl-(4-methoxyphenyl)-3-[(triisopropylsilyl)oxy]-2-azetidinone

The compound obtained in Step 4 mentioned above was used as a starting material and reacted with triisopropylsilyl chloride instead of triethylsilyl chloride to perform the same procedure as that of Reference Example 19, Step 2 and thereby to obtain the title compound as colorless oil.
$^1$H-NMR (400MHz, $CDCl_3$) δ : 0.27-0.32 (1H, m), 0.49-0.54 (1H, m), 0.67-0.73 (1H, m), 0.76-0.82 (1H, m), 1.11-1.28 (22H, m), 3.45 (1H, dd, *J*=9.3, 4.9Hz), 3.79 (3H, s), 5.03 (1H, d, *J*=4.9Hz), 6.87 (2H, d, *J*=9.1Hz), 7.46 (2H, d, *J*=9.1Hz).
ESI-MS; m/z: 390 $(M+H)^+$.

Step 6: (3R,4S)-4-Cyclopropyl-3-[(triisopropylsilyl)oxy]-2-azetidinone

The compound obtained in Step 5 mentioned above was used as a starting material to perform the same procedure as that of Reference Example 18, Step 2 and thereby to obtain the title compound as pale brown oil.
$^1$H-NMR (400MHz, $CDCl_3$) δ : 0.17-0.30 (2H, m), 0.55-0.65 (2H, m), 1.04-1.28 (22H, m), 3.06 (1H, dd, *J*=8.8, 4.7Hz), 4.95 (1H, dd, *J*=4.7, 2.5Hz), 5.85 (1H, brs).
ESI-MS; m/z: 284 $(M+H)^+$.

Step 7: (3R,4S)-1-Benzoyl-4-cyclopropyl-3-[(triisopropylsilyl)oxy]-2-azetidinone

The compound obtained in Step 6 mentioned above was used as a starting material to perform the same procedure as that of Reference Example 33 and thereby to obtain the title compound as colorless oil.
$^1$H-NMR (400MHz, $CDCl_3$) δ : 0.32-0.37 (1H, m), 0.64-0.73 (2H, m), 0.79-0.84 (1H, m), 1.07-1.28 (22H, m), 3.83 (1H, dd, *J*=9.3, 6.4Hz), 5.04 (1H, d, *J*=6.4Hz), 7.46 (2H, t, *J*=7.7Hz), 7.57 (1H, tt, *J*=7.5, 1.5Hz), 7.94-7.96 (2H, m).
ESI-MS; m/z: 410 $(M+Na)^+$.

[Reference Example 59]

[Formula 67]

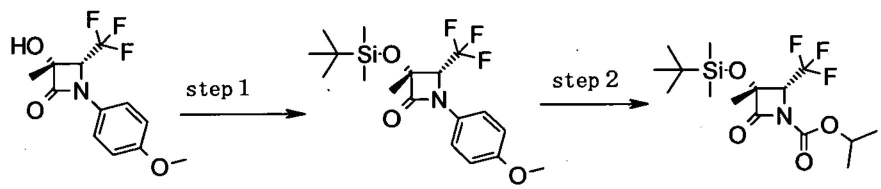


Step 1: (3R,4R)-3-[(tert-Butyldimethylsilyl)oxy]-1-(4-methoxyphenyl)-3-methyl-4-trifluoromethyl-2-azetidinone

The compound obtained in Reference Example 46, Step 1 was used as a starting material to perform the same procedure as that of Reference Example 43, Step 2 and thereby to obtain the title compound as brown oil.
$^1$H-NMR ($CDCl_3$) δ : 0.20 (3H, s), 0.29 (3H, s), 0.90 (9H, s), 1.64 (3H, s), 3.80 (3H, s), 4.21 (1H, q , J=5.9Hz), 6.87-6.92 (2H, m), 7.38-7.42 (2H, m).

Step 2: (3R,4R)-3-[(tert-Butyldimethylsilyl)oxy]-1-(isopropoxycarbonyl)-3-methyl-4-trifluoromethyl-2-azetidinone

The compound obtained in Step 1 mentioned above was used as a starting material to perform the same procedure as that of Reference Example 18, Step 2, and then the same procedure as that of Reference Example 21 and thereby to obtain the title compound as pale yellow oil.
$^1$H-NMR ($CDCl_3$) δ : 0.16 (3H, s), 0.24 (3H, s), 0.88 (9H, s), 1.33-1.37 (6H, m), 1.64 (3H, s), 4.15 (1H, q , J=5.9Hz), 5.07 (1H, septet, J=6.3Hz).

[Reference Example 60]

[Formula 68]

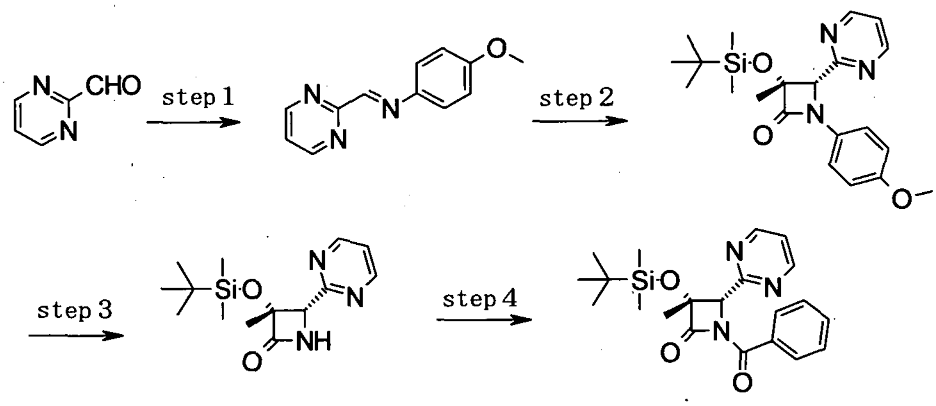


Step 1:4-Methoxy-N-[(E)-2-pyrimidinylmethylidene]aniline

The compound obtained in Reference Example 28, Step 2 (15 g) was dissolved in benzene (300 ml), and the solution was added with p-anisidine (13.7 g) and anhydrous sodium sulfate (20 g), and stirred at room temperature for 3 hours. The insoluble matters were removed by filtration, and the filtrate was concentrated. Then, the resulting residue was added with ether, and the deposited solid was collected by filtration, and dried to obtain the title compound (22.5 g) as pale yellow solid.
$^1$H-NMR (400MHz, $CDCl_3$) δ : 3.85 (3H, s), 6.95-6.99 (2H, m), 7.34 (1H, t, J=4.9Hz), 7.46-7.49 (2H, m), 8.70 (1H, s), 8.92 (2H, d, J=4.9Hz).

Step 2: cis-3-[(tert-Butyldimethylsilyl)oxy]-1-(4-methoxyphenyl)-3-methyl-4-(2-pyrimidinyl)-2-azetidinone

A solution of diisopropylamine (18 ml) in tetrahydrofuran (200 ml) was cooled on ice, added dropwise with n-butyl lithium (1.58 mole solution in n-hexane, 80 ml), and stirred for 15 minutes. Then, the reaction mixture was cooled to -78°C, added dropwise with a solution of methyl (S)-(-)-2-[(tert-butyldimethylsilyl)oxy]propionate (J. Org. Chem., 2002, 67, 772, 25.8 g) in tetrahydrofuran (100 ml), and stirred for 30 minutes. The reaction mixture was added dropwise with a solution of the compound obtained in Step 1 mentioned above (24 g) dissolved in tetrahydrofuran (200 ml), and warmed to room temperature overnight. The reaction mixture was added with saturated aqueous ammonium chloride, and extracted with ethyl acetate. The organic layer was successively washed with saturated aqueous sodium hydrogencarbonate, and saturated brine, and then dried over anhydrous sodium sulfate, and the solvent was evaporated under reduced pressure. The resulting residue was purified by silica gel column chromatography [developing solvent; ethyl acetate:n-hexane=1:5 (v/v)], and added with ether/n-hexane, and the deposited solid was collected by filtration, and dried to obtain the title compound (15.2 g) as colorless solid.
$^1$H-NMR (400MHz, $CDCl_3$) δ : 0.05 (3H, s), 0.18 (3H, s), 0.52 (9H, s), 1.55 (3H, s), 1.76 (3H, s), 3.76 (3H, s), 5.06 (1H, s), 6.80-6.83 (2H, m), 7.19-7.29 (3H, m), 8.73 (2H, d, J=4.9Hz).

Step 3: (3R,4S)-3-[(tert-Butyldimethylsilyl)oxy]-3-methyl-4-(2-pyrimidinyl)-2-azetidinone

The compound obtained in Step 2 mentioned above was used as a starting material to perform the same procedure as that of Reference Example 18, Step 2, and then the resulting racemate was subjected to optical resolution using an optically active column (CHIRALCEL OD-H, Daicel Chemical Industries) to obtain the title compound as pale yellow solid.
$^1$H-NMR (400MHz, $CDCl_3$) δ : -0.01 (3H, s), 0.14 (3H, s), 0.50 (9H, s), 1.74 (3H, s), 4.74 (1H, s), 6.31 (1H, s), 7.22 (1H, t, J=4.9Hz), 8.74 (2H, d, J=4.9Hz).

Step 4: (3R,4S)-1-Benzoyl-3-[(tert-butyldimethylsilyl)oxy]-3-methyl-4-(2-pyrimidinyl)-2-azetidinone

The compound obtained in Step 3 mentioned above was used as a starting material to perform the same procedure as that of Reference Example 33 and thereby to obtain the title compound as colorless amorphous solid.
$^1$H-NMR (400MHz, $CDCl_3$) δ : 0.06 (3H, s), 0.15 (3H, s), 0.53 (9H, s), 1.79 (3H, s), 5.31 (1H, s), 7.20 (1H, t, J=4.9Hz), 7.45-7.53 (2H, m), 7.58-7.63 (1H, m), 8.06-8.10 (2H, m), 8.72 (2H, d, J=4.9Hz).

[Reference Example 61]

[Formula 69]

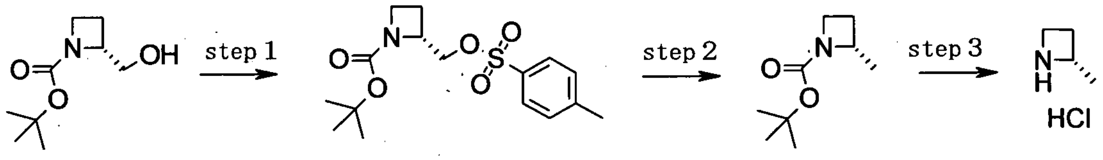


Step 1: (R)-2-(Toluene-4-sulfonyloxymethyl)azetidinecarboxylic acid tert-butyl ester

(R)-2-(Hydroxymethyl)-1-azetidinecarboxylic acid tert-butyl ester (1.40 g) and pyridine (5.6 ml) were dissolved in dichloromethane (14 ml), and the solution was added with p-toluenesulfonyl chloride (2.14 g) at 0°C, and stirred at room temperature for 24 hours. The reaction mixture was added with water, and extracted with ethyl acetate. The organic layer was successively washed with 1 N aqueous hydrochloric acid and saturated brine, and then dried over anhydrous sodium sulfate, and the solvent was evaporated under reduced pressure. The resulting residue was purified by silica gel column chromatography [developing solvent; ethyl acetate:n-hexane=1:19 to 2:3 (v/v)] to obtain the title compound (2.36 g) as colorless oil.
$^1$H-NMR (400MHz, $CDCl_3$) δ : 1.37 (9H, s), 2.10-2.20 (1H, br), 2.20-2.30 (1H, br), 2.44 (3H, s), 3.72-3.83 (2H, m), 4.13 (1H, dd, J=10.2, 2.8Hz), 4.22-4.29 (1H, br), 4.30-4.36 (1H, br), 7.34 (2H, d, J=8.1Hz), 7.80 (2H, d, J=8.1Hz).

Step 2: (S)-2-Methylazetidinecarboxylic acid tert-butyl ester

The compound obtained in Step 1 mentioned above was used as a starting material to perform the same procedure as that of Reference Example 49, Step 1 and thereby to obtain the title compound as colorless oil.
$^1$H-NMR (400MHz, $CDCl_3$) δ : 1.37 (3H, d, J=6.1Hz), 1.44 (9H, d, J=1.0Hz), 1.71-1.81 (1H, m), 2.23-2.32 (1H, m), 3.81 (2H, t, J=7.6Hz), 4.23-4.32 (1H, m).

Step 3: (S)-2-Methylazetidine hydrochloride

The compound obtained in Step 2 mentioned above was used as a starting material to perform the same procedure as that of Reference Example 25 and thereby to obtain the title compound as colorless oil.
$^1$H-NMR (400MHz, $CDCl_3$) δ : 1.67 (3H, d, J=6.9Hz), 2.26-2.37 (1H, m), 2.54-2.64 (1H, m), 3.91-4.08 (2H, m), 4.58-4.69 (1H, m), 9.59 (2H, brs).

[Reference Example 62]

[Formula 70]

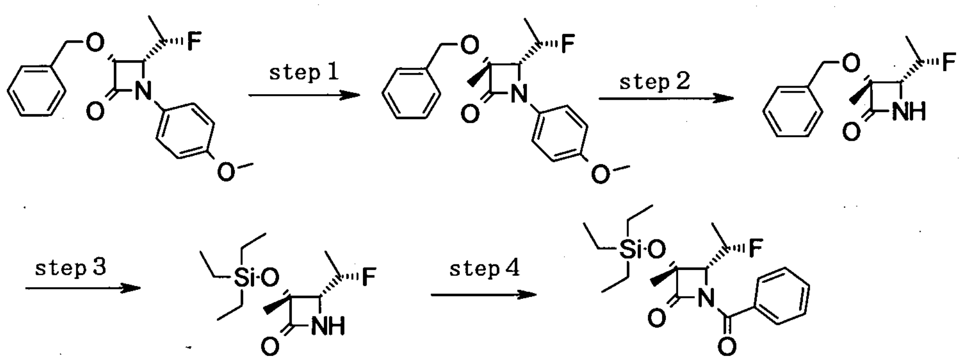


Step 1: (3R,4R)-3-(Benzyloxy)-4-[(1S)-1-fluoroethyl]-1-(4-methoxyphenyl)-3-methyl-2-azetidinone

The compound obtained in Reference Example 31, Step 1 (1.6 g) was dissolved in tetrahydrofuran (40 ml), and the solution was added with lithium bis(trimethylsilyl)amide (1 mole solution in tetrahydrofuran, 12.2 ml) at -78°C, and stirred at the same temperature for 2.5 hours. The reaction mixture was added with methyl iodide (1.2 ml), and stirred at 0°C for 1 hour. The reaction mixture was added with saturated aqueous ammonium chloride, and extracted with ethyl acetate. The organic layer was washed with saturated brine, and then dried over anhydrous sodium sulfate, and the solvent was evaporated under reduced pressure. The resulting residue was purified by silica gel column chromatography [developing solvent; ethyl acetate:n-hexane=1:8 (v/v)] to obtain the title compound (1.2 g) as yellow solid.
$^1$H-NMR (400MHz, $CDCl_3$) δ : 1.35 (3H, dd, J=25.6, 6.8Hz), 1.72 (3H, s), 3.80 (3H, s), 4.23 (1H, d, J=17.6Hz), 4.81 (1H, d, J=12.0Hz), 4.91 (1H, d, J=11.5Hz), 5.00-5.19 (1H, m), 6.89 (2H, d, J=8.8Hz), 7.29-7.38 (5H, m), 7.51 (2H, d, J=8.5Hz).

Step 2: (3R,4R)-3-(Benzyloxy)-4-[(1S)-1-fluoroethyl]-3-methyl-2-azetidinone

The compound obtained in Step 1 mentioned above was used as a starting material to perform the same procedure as that of Reference Example 18, Step 2 and thereby to obtain the title compound as orange oil.
$^1$H-NMR (400MHz, $CDCl_3$) δ : 1.38 (3H, dd, J=24.9, 6.3Hz), 1.69 (3H, s), 3.64 (1H, dd, J=12.4, 5.9Hz), 4.74 (1H, d, J=11.2Hz), 4.84 (1H, d, J=11.5Hz), 4.85-5.04 (1H, m), 5.83 (1H, brs), 7.27-7.36 (5H, m).

Step 3: (3R,4R)-4-[(1S)-1-Fluoroethyl]-3-methyl-3-[(triethylsilyl)oxy]-2-azetidinone

The compound obtained in Step 2 mentioned above was used as a starting material to perform the same procedure as that of Reference Example 18, Step 3 using triethylsilyl chloride instead of triisopropylsilyl chloride and thereby to obtain the title compound as colorless oil.
$^1$H-NMR (400MHz, $CDCl_3$) δ : 0.65-0.74 (6H, m), 0.97 (9H, t, J=7.8Hz), 1.37 (3H, dd, J=24.6, 6.6Hz), 1.57 (3H, s), 3.51 (1H, dd, J=12.7, 5.9Hz), 4.74-4.92 (1H, m), 5.93 (1H, brs).

Step 4: (3R,4R)-1-Benzoyl-4-[(1S)-1-fluoroethyl]-3-methyl-3-[(triethylsilyl)oxy]-2-azetidinone

The compound obtained in Step 3 mentioned above was used as a starting material to perform the same procedure as that of Reference Example 33 and thereby to obtain the title compound as colorless oil.
$^1$H-NMR (400MHz, $CDCl_3$) δ : 0.69-0.80 (6H, m), 1.00 (9H, t, J=7.8Hz), 1.48 (3H, dd, J=23.9, 7.6Hz), 1.60 (3H, s), 4.15 (1H, dd, J=16.8, 4.6Hz), 5.03-5.21 (1H, m), 7.48 (2H, t, J=7.6Hz), 7.60 (1H, t, J=7.8Hz), 7.93 (2H, d, J=8.3Hz).

[Reference Example 63]

(3R,4R)-4-Fluoromethyl-1-(isopropoxycarbonyl)-3-methyl-3-[(triethylsilyl)oxy]-2-azetidinone

[Formula 71]

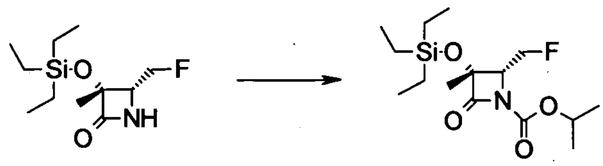

The compound obtained in Reference Example 55, Step 6 was used as a starting material to perform the same procedure as that of Reference Example 21 and thereby to obtain the title compound as pale yellow oil.
$^1$H-NMR (400MHz, $CDCl_3$) δ : 0.64-0.73 (6H, m), 0.96 (9H, t, J=7.8Hz), 1.33 (3H, d, J=4.4Hz), 1.34 (3H, d, J=4.4Hz), 1.59 (3H, s), 3.94 (1H, ddd, J=15.0, 5.0, 4.4Hz), 4.61-4.82 (2H, m), 5.05 (1H, septet, J=6.1Hz).

[Reference Example 64]

[Formula 72]

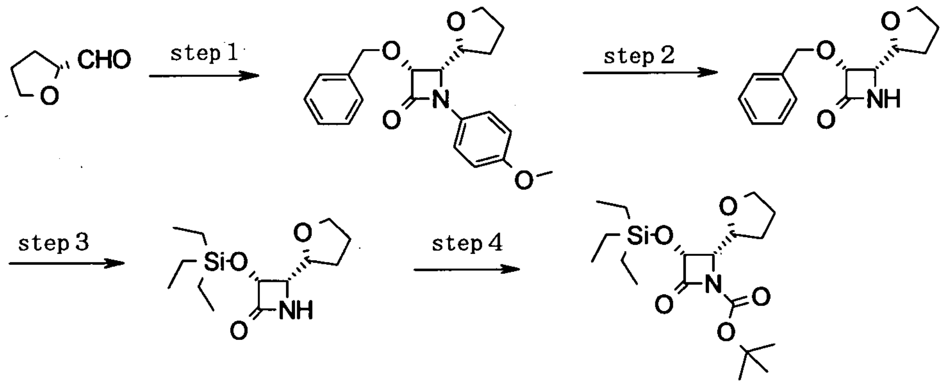


Step 1: (3R,4S)-3-(Benzyloxy)-1-(4-methoxyphenyl)-4-[(2R)-tetrahydrofuran-2-yl]-2-azetidinone

(2R)-Tetrahydrofuran-2-carboxyaldehyde (Can. J. Chem., 1983, 61, 1383) was used as a starting material to perform the same procedure as that of Reference Example 20, Step 1 using benzyloxyacetyl chloride instead of acetoxyacetyl chloride and thereby to obtain the title compound as colorless solid.
$^1$H-NMR (400MHz, $CDCl_3$) δ : 1.58-1.68 (1H, m), 1.85-1.94 (2H, m), 2.22-2.33 (1H, m), 3.72-3.79 (1H, m), 3.78 (3H, s), 3.89-3.96 (1H, m), 4.09-4.24 (2H, m), 4.74 (1H, d, J=5.4Hz), 4.77 (1H, d, J=12.0Hz), 4.97 (1H, d, J=11.7Hz), 6.85 (2H, d, J=9.0Hz), 7.29-7.40 (5H, m), 7.64 (2H, d, J=9.8Hz).

Step 2: (3R,4S)-3-(Benzyloxy)-4-[(2R)-tetrahydrofuran-2-yl]-2-azetidinone

The compound obtained in Step 1 mentioned above was used as a starting material to perform the same procedure as that of Reference Example 18, Step 2 and thereby to obtain the title compound as brown solid.
$^1$H-NMR (400MHz, $CDCl_3$) δ : 1.46-1.56 (1H, m), 1.87-1.97 (2H, m), 2.09-2.20 (1H, m), 3.58 (1H, dd, J=9.0, 5.1Hz), 3.71-3.87 (2H, m), 4.03-4.11 (1H, m), 4.66-4.72 (2H, m), 4.92 (1H, d, J=11.7Hz), 6.23 (1H, brs), 7.27-7.38 (5H, m).

Step 3: (3R,4S)-4-[(2R)-Tetrahydrofuran-2-yl]-3-[(triethylsilyl)oxy]-2-azetidinone

The compound obtained in Step 2 mentioned above was used as a starting material to perform the same

procedure as that of Reference Example 18, Step 3 using triethylsilyl chloride instead of triisopropylsilyl chloride and thereby to obtain the title compound as colorless oil.
$^1$H-NMR (400MHz, $CDCl_3$) δ : 0.64-0.73 (6H, m), 0.98 (9H, t, J=8.1Hz), 1.43-1.56 (1H, m), 1.87-1.97 (2H, m), 2.03-2.15 (1H, m), 3.51 (1H, dd, J=9.2, 5.0Hz), 3.70-3.78 (1H, m), 3.79-3.86 (1H, m), 3.94-4.02 (1H, m), 4.85 (1H, dd, J=5.1, 2.0Hz), 6.16 (1H, brs).

Step 4: (3R,4S)-1-(tert-Butoxycarbonyl)-4-[(2R)-tetrahydrofuran-2-yl]-3-[(triethylsilyl)oxy]-2-azetidinone

The compound obtained in Step 3 mentioned above was used as a starting material to perform the same procedure as that of Reference Example 18, Step 4 and thereby to obtain the title compound as colorless oil.
$^1$H-NMR (400MHz, $CDCl_3$) δ : 0.65-0.73 (6H, m), 0.98 (9H, t, J=7.6Hz), 1.52 (9H, s), 1.71-1.82 (1H, m), 1.84-1.95 (2H, m), 2.04-2.13 (1H, m), 3.75-3.81 (1H, m), 3.85-3.91 (1H, m), 4.02-4.07 (1H, m), 4.15 (1H, dd, J=13.9, 6.6Hz), 4.85 (1H, d, J=6.1Hz).

[Example 1]

(1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-hydroxy-3-(2-pyridyl)propionate

[Formula 73]

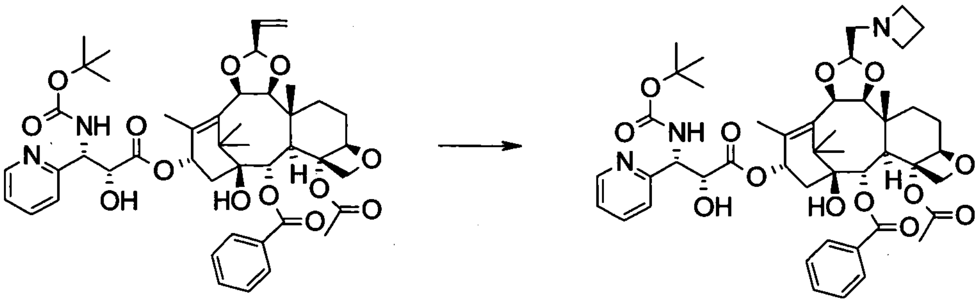

(1S,2S,3R,4S,5R,8R,9S,10R,13S)-4-(Acetoxy)-2-(benzoyloxy)-5,20-epoxy-1-hyd roxy-9,10-[(1S)-2-propenylidenedioxy]tax-11-en-13-yl (2R,3S)-3-(tert-butoxycarbonylamino)-2-hydroxy-3-(2-pyridyl)propionate (Bioorg. Med. Chem. Lett., 2003, 13, 185, 50 mg) was dissolved in tetrahydrofuran (1 ml), acetone (1 ml) and water (1 ml), and the solution was added with osmium tetroxide (0.76 mg) and N-methylmorpholine N-oxide (36.3 mg) at room temperature, and stirred for 6 hours. The reaction mixture was added with 10% aqueous sodium thiosulfate, and extracted with ethyl acetate. The organic layer was successively washed with saturated aqueous sodium hydrogencarbonate and saturated brine, and then dried over anhydrous sodium sulfate. The solvent was evaporated under reduced pressure, and then the residue was dissolved in tetrahydrofuran (1 ml), methanol (1 ml) and water (1 ml), and the solution was added with sodium metaperiodate (154 mg) at room temperature, and stirred for 2.5 hours. The reaction mixture was added with water and saturated brine , and extracted with ethyl acetate. The organic layer was washed with saturated aqueous sodium hydrogencarbonate, and then dried over anhydrous sodium sulfate. The solvent was evaporated under reduced pressure, and the resulting residue was dissolved in methanol (2 ml). The solution was added with triethylamine (0.0418 ml) and azetidine hydrochloride (29 mg) at room temperature, and stirred for 24 hours. The reaction mixture was concentrated, and the residue was dissolved in methanol (2 ml). The solution was added with sodium triacetoxyborohydride (67 mg), and stirred at room temperature for 2 hours. The reaction mixture was added with saturated aqueous sodium hydrogencarbonate, and extracted with ethyl acetate. The organic layer was washed with saturated brine, and then dried over anhydrous sodium sulfate. The solvent was evaporated under reduced pressure, and then the residue was purified by thin layer silica gel column chromatography [developing solvent; chloroform: methanol=20:1 (v/v)] to obtain the title compound (21.4 mg) as white amorphous solid.

$^1$H-NMR (400MHz, $CDCl_3$) δ : 1.22-2.24 (9H, m), 1.25 (3H, s), 1.44 (9H, s), 1.49 (3H, s), 1.60 (3H, s), 1.70 (3H, s), 2.31-2.37 (1H, m), 2.34 (3H, s), 2.75 (1H, dd, J=12.7, 5.9Hz), 2.84 (1H, dd, J=12.7, 4.2Hz), 2.92 (1H, d, J=5.1Hz), 3.36-3.40 (4H, m), 4.12 (1H, d, J=7.1Hz), 4.22 (1H, d, J=8.5Hz), 4.33 (1H, d, J=8.5Hz), 4.86-4.92 (3H, m), 5.20 (1H, d, J=7.3Hz), 5.35 (1H, d, J=8.3Hz), 5.94-5.99 (2H, m), 6.08 (1H, t, J=8.2Hz), 7.21-7.25 (1H, m), 7.41-7.49 (3H, m), 7.60 (1H, t, J=7.4Hz), 7.72 (1H, dt, J=7.7, 1.7Hz), 8.13 (2H, d, J=7.7Hz), 8.54 (1H, d, J=4.4Hz).
MS (FAB)m/z: 876 $(M+H)^+$.

[Example 2]

(1S,2S,3R,4S,5R,8R,9S,10R,13S)-4-(Acetoxy)-9,10-[(1S)-2-(azetidino)ethylidenedioxyl-2-(benzoyloxy)-5,20-epoxy-1-hydroxytax-11-en-13-yl

(2R,3S)-3-(tert-butoxycarbonylamino)-3-(3-fluoro-2-pyridyl)-2-hydroxypropionate

[Formula 74]

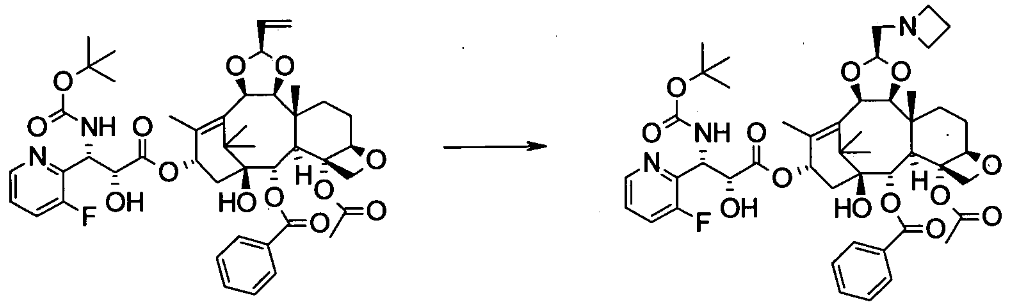

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate (Bioorg. Med. Chem. Lett., 2004, 14, 3209) was used as a starting material to perform the same procedure as that of Example 1 and thereby to obtain the title compound as white amorphous solid.
$^1$H-NMR (400MHz, $CDCl_3$) δ : 1.25-2.18 (9H, m), 1.28 (3H, s), 1.40 (9H, s), 1.50 (3H, s), 1.62 (3H, s), 1.78 (3H, s), 2.34 (3H, s), 2.42-2.48 (1H, m), 2.76 (1H, dd, J=12.5, 5.7Hz), 2.84 (1H, dd, J=12.5, 4.4Hz), 2.93 (1H, d, J=5.1Hz), 3.36-3.41 (4H, m), 4.13 (1H, d, J=7.1Hz), 4.23 (1H, d, J=8.3Hz), 4.33 (1H, d, J=8.3Hz), 4.68 (1H, d, J=2.7Hz), 4.88 (1H, t, J=5.1Hz), 4.91 (1H, s), 5.22 (1H, d, J=6.8Hz), 5.65 (1H, d, J=8.3Hz), 5.99 (1H, d, J=5.1Hz), 6.08 (1H, t, J=8.5Hz), 6.21 (1H, d, J=8.8Hz), 7.28-7.33 (1H, m), 7.42-7.49 (3H, m), 7.60 (1H, t, J=7.4Hz), 8.12-8.14 (2H, m), 8.40 (1H, d, J=4.6Hz).
MS (FAB)m/z: 894 $(M+H)^+$.

[Example 3]

(1S,2S,3R,4S,5R,8R,9S,10R,13S)-4-Acetoxy-2-(benzoyloxy)-5,20-epoxy-9,10-[(1S)-2-(3-fluoroazetidino)ethylidenedioxy]-1-hydroxytax-11-en-13-yl

(2R,3S)-3-(tert-butoxycarbonylamino)-2-hydroxy-3-(2-pyridyl)propionate

[Formula 75]

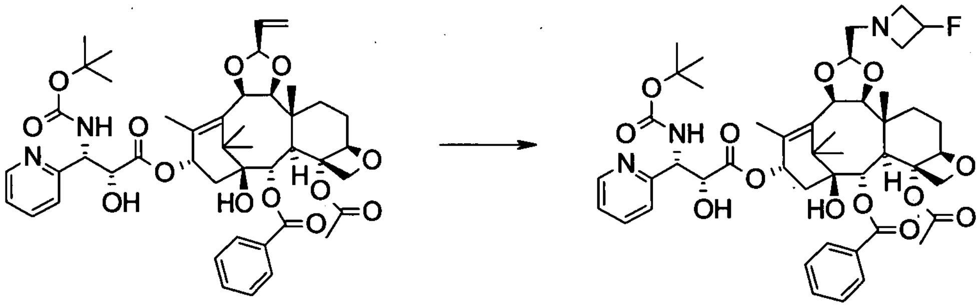

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 24, Step 2 were used as starting materials to perform the same procedure as that of Example 1 and thereby to obtain the title compound as white amorphous solid.
$^1$H-NMR (400MHz, $CDCl_3$) δ : 1.25-2.13 (7H, m), 1.26 (3H, s), 1.44 (9H, s), 1.49 (3H, s), 1.60 (3H, s), 1.71 (3H, s), 2.31-2.38 (1H, m), 2.35 (3H, s), 2.82 (1H, dd, J=12.3, 5.5Hz), 2.89-2.93 (2H, m), 3.27-3.39 (2H, m), 3.77-3.82 (2H, m), 4.13 (1H, d, J-7.1Hz), 4.21 (1H, d, J=8.3Hz), 4.33 (1H, d, J=8.3Hz), 4.86-4.92 (3H, m), 5.07-5.24 (2H, m), 5.35 (1H, d, J=9.3Hz), 5.94-5.98 (2H, m), 6.09 (1H, t, J=7.8Hz), 7.22-7.25 (1H, m), 7.41-7.49 (3H, m), 7.60 (1H, t, J=7.6Hz), 7.72 (1H, t, J=7.6Hz), 8.13 (2H, d, J=7.6Hz), 8.54 (1H, d, J=3.9Hz).
MS (FAB)m/z: 894 $(M+H)^+$.

[Example 4]

(1S,2S,3R,4S,5R,8R,9S,10R,13S)-4-(Acetoxy)-2-(benzoyloxy)-5,20-epoxy-9,10-[(1S)-2-(3-fluoroazetidino)ethylidenedioxy]-1-hydroxytax-11-en-13-yl

(2R,3S)-3-(tert-butoxycarbonylamino)-3-(3-fluoro-2-pyridyl)-2-hydroxypropionate

[Formula 76]

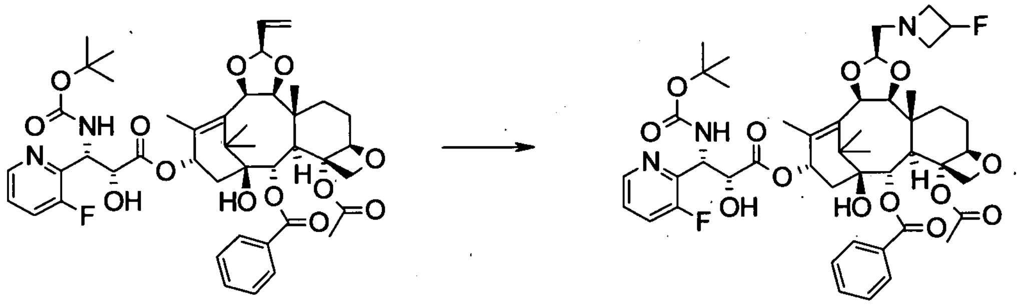

(1S,2S,3R,4S,5R,8R,9S, 10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 24, Step 2 were used as starting materials to perform the same procedure as that of Example 1 and thereby to obtain the title compound as white amorphous solid.
$^1$ H-NMR (400MHz, $CDCl_3$) δ : 1.26-2.16 (7H, m), 1.29 (3H, s), 1.40 (9H, s), 1.50 (3H, s), 1.61 (3H, s), 1.78 (3H, s), 2.34

(3H, s), 2.42-2.48 (1H, m), 2.82 (1H, dd, J=12.3, 5.7Hz), 2.92 (2H, dd, J=12.3, 4.4Hz), 3.27-3.39 (2H, m), 3.76-3.84 (2H, m), 4.14 (1H, d, J=7.1Hz), 4.23 (1H, d, J=8.3Hz), 4.33 (1H, d, J=8.3Hz), 4.67 (1H, d, J=2.2Hz), 4.87-4.92 (2H, m), 5.07-5.23 (2H, m), 5.64 (1H, d, J=8.8Hz), 5.99 (1H, d, J=4.9Hz), 6.09 (1H, t, J=8.3Hz), 6.21 (1H, d, J=7.8Hz), 7.28-7.33 (1H, m), 7.43-7.49 (3H, m), 7.60 (1H, t, J=7.6Hz), 8.14 (2H, d, J=7.6Hz), 8.40 (1H, d, J=4.2Hz).
MS (FAB)m/z: 912 $(M+H)^+$.

[Example 5]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxyazetidino)ethylidenedioxy]tax-11-en-13-yl

(2R,3S)-3-(tert-butoxycarbonylamino)-2-hydroxy-3-(2-pyridyl)propionate

[Formula 77]

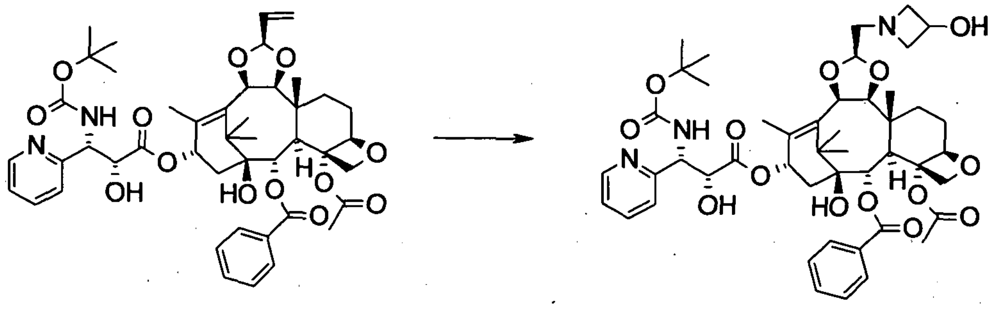

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and 3-azetidinol hydrochloride was used as a starting material to perform the same procedure as that of Example 1 and thereby to obtain the title compound as white amorphous solid.
$^1$ H-NMR (400MHz, $CDCl_3$) δ : 1.24-2.13 (8H, m), 1.26 (3H, s), 1.44 (9H, s), 1.49 (3H, s), 1.60 (3H, s), 1.71 (3H, s), 2.31-2.37 (1H, m), 2.35 (3H, s), 2.76-2.82 (1H, m), 2.86-2.93 (2H, m), 3.07-3.14 (2H, m), 3.74-3.80 (2H, m), 4.12 (1H, d, J=7.1Hz), 4.21 (1H, d, J=8.3Hz), 4.32 (1H, d, J=8.3Hz), 4.46-4.50 (1H, m), 4.86-4.92 (3H, m), 5.20 (1H, d, J=6.1Hz), 5.35 (1H, d, J=9.0Hz), 5.98 (2H, s), 6.09 (1H, s), 7.21-7.25 (1H, m), 7.41-7.50 (3H, m), 7.60 (1H, t, J=7.6Hz), 7.72 (1H, t, J=7.6Hz), 8.13 (2H, d, J=7.6Hz), 8.54 (1H, s).
FAB-MS; m/z: 892 $(M+H)^+$.

[Example 6]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxyazetidino)ethylidenedioxy]tax-11-en-13-yl

(2R,3S)-3-(tert-butoxycarbonylamino)-3-(3-fluoro-2-pyridyl)-2-hydroxypropionate

[Formula 78]

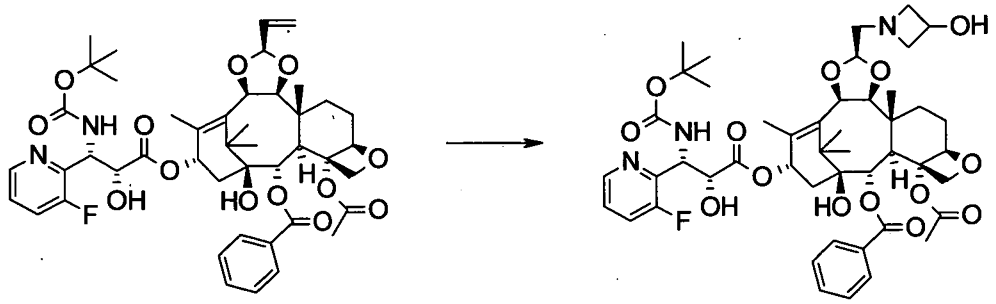

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and 3-azetidinol hydrochloride were used as starting materials to perform the same procedure as that of Example 1 and thereby to obtain the title compound as white amorphous solid.
$^1$H-NMR (400MHz, $CDCl_3$) δ : 1.24-2.16 (8H, m), 1.29 (3H, s), 1.40 (9H, s), 1.49 (3H, s), 1.61 (3H, s), 1.78 (3H, s), 2.35 (3H, s), 2.42-2.48 (1H, m), 2.79 (1H, dd, J=12.3, 5.6Hz), 2.88 (1H, dd, J=12.3, 4.2Hz), 2.93 (1H, d, J=5.1Hz), 3.11 (2H, dd, J=13.3, 7.2Hz), 3.74-3.80 (2H, m), 4.14 (1H, d, J=7.3Hz), 4.23 (1H, d, J=8.3Hz), 4.32 (1H, d, J=8.3Hz), 4.47 (1H, t, J=5.7Hz), 4.68 (1H, d, J=2.4Hz), 4.88 (1H, t, J=4.9Hz), 4.91 (1H, s), 5.22 (1H, d, J=7.1Hz), 5.64 (1H, d, J=8.5Hz), 5.98 (1H, d, J=4.9Hz), 6.08 (1H, t, J=8.5Hz), 6.24 (1H, d, J=8.5Hz), 7.28-7.33 (1H, m), 7.43-7.49 (3H, m), 7.60 (1H, t, J=7.6Hz), 8.13 (2H, d, J=7.6Hz), 8.40 (1H, d, J=4.6Hz).
FAB-MS; m/z: 910 $(M+H)^+$.

[Example 7]

(1S,2S,3R,4S,5R,8R,9S,10R,13S)-4-(Acetoxy)-2-(benzoyloxy)-5,20-epoxy-1-hydroxy-9,10-[(1S)-2-(3-methoxyazetidino)ethylidenedioxy]tax-11-en-13-yl

(2R,3S)-3-(tert-butoxycarbonylamino)-2-hydroxy-3-(2-pyridyl)propionate

[Formula 79]

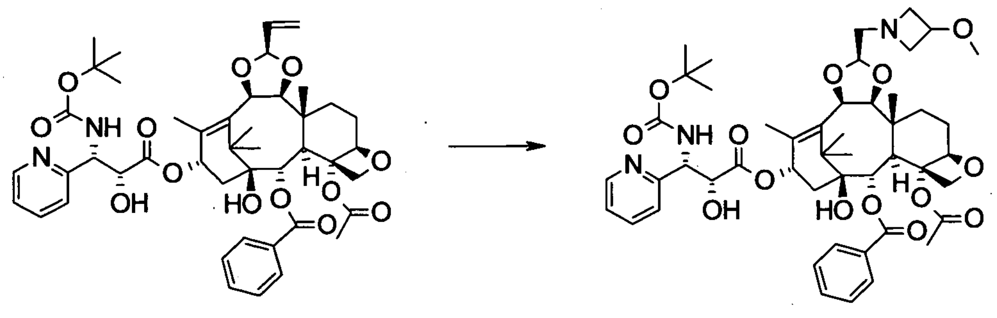

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 1 were used as starting materials to perform the same procedure as that of Example 1 and thereby to obtain the title compound as white amorphous solid.

$^1$H-NMR (400MHz, $CDCl_3$) δ : 1.18-2.13 (7H, m), 1.25 (3H, s), 1.44 (9H, s), 1.48 (3H, s), 1.60 (3H, s), 1.71 (3H, s), 2.31-2.37 (1H, m), 2.35 (3H, s), 2.80 (1H, dd, J=12.5, 5.5Hz), 2.88 (1H, dd, J=12.5, 4.4Hz), 2.92 (1H, d, J=5.1Hz), 3.06-3.12 (2H, m), 3.27 (3H, s), 3.72-3.77 (2H, m), 4.07 (1H, t, J=5.9Hz), 4.12 (1H, d, J=7.3Hz), 4.21 (1H, d, J=8.3Hz), 4.32 (1H, d, J=8.3Hz), 4.86-4.91 (3H, m), 5.20 (1H, d, J=7.1Hz), 5.35 (1H, d, J=9.8Hz), 5.95-5.98 (2H, m), 6.08 (1H, t, J=8.3Hz), 7.21-7.24 (1H, m), 7.41-7.49 (3H, m), 7.60 (1H, t, J=7.6Hz), 7.72 (1H, dt, J=7.6, 1.5Hz), 8.13 (2H, d, J=7.6Hz), 8.53 (1H, d, J=4.4Hz). FAB-MS; m/z: 906 $(M+H)^+$.

[Example 8]

(1S,2S,3R,4S,5R,8R,9S,10R,13S)-4-(Acetoxy)-2-(benzoyloxy)-5,20-epoxy-1-hydroxy-9,10-[(1S)-2-(3-methoxyazetidino)ethylidenedioxy]tax-11-en-13-yl

(2R,3S)-3-(tert-butoxycarbonylamino)-3-(3-fluoro-2-pyridyl)-2-hydroxypropionate

[Formula 80]

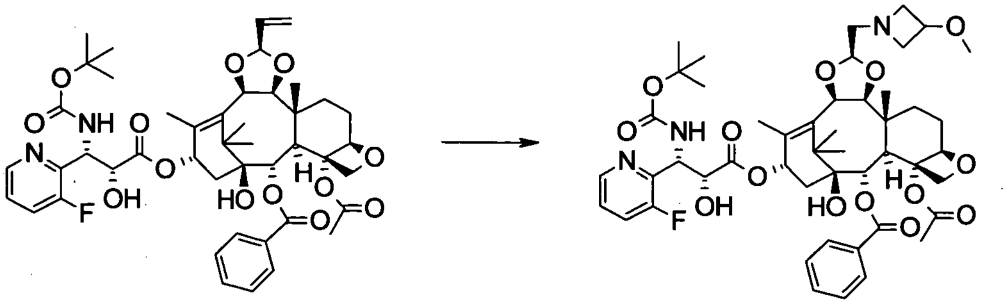

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 1 were used as starting materials to perform the same procedure as that of Example 1 and thereby to obtain the title compound as white amorphous solid.
$^1$ H-NMR (400MHz, $CDCl_3$) δ : 1.26-2.15 (7H, m), 1.28 (3H, s), 1.40 (9H, s), 1.49 (3H, s), 1.61 (3H, s), 1.78 (3H, s), 2.34 (3H, s), 2.42-2.48 (1H, m), 2.80 (1H, dd, J=12.5, 5.6Hz), 2.89 (1H, dd, J=12.5, 4.4Hz), 2.93 (1H, d, J=5.1Hz), 3.06-3.12 (2H, m), 3.28 (3H, s), 3.73-3.77 (2H, m), 4.08 (1H, t, J=5.7Hz), 4.14 (1H, d, J=7.3Hz), 4.23 (1H, d, J=8.3Hz), 4.33 (1H, d, J=8.3Hz), 4.67 (1H, d, J=2.4Hz), 4.87-4.91 (2H, m), 5.22 (1H, d, J=7.1Hz), 5.65 (1H, d, J=8.5Hz), 5.99 (1H, d, J=5.1Hz), 6.08 (1H, t, J=8.5Hz), 6.22 (1H, d, J=8.5Hz), 7.33-7.33 (1H, m), 7.43-7.49 (3H, m), 7.60 (1H, t, J=7.6Hz), 8.14 (2H, d, J=7.6Hz), 8.40 (1H, d, J=4.6Hz).
FAB-MS; m/z: 924 $(M+H)^+$.

[Example 9]

(1S,2S,3R,4S,5R,8R,9S,10R,13S)-4-(Acetoxy)-2-(benzoyloxy)-9,10-{(1S)-[2-(3-dimethylcarbamoyl)azetidino]ethylidenedioxy}-5,20-epoxy-1-hydroxytax-11-en-13-yl

(2R,3S)-3-(tert-butoxycarbonylamino)-2-hydroxy-3-(2-pyridyl)propionate

[Formula 81]

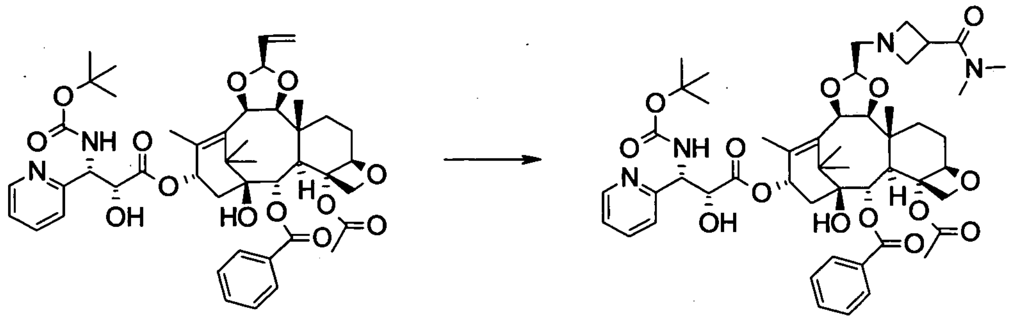

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 2, Step 2 were used as starting materials to perform the same procedure as that of Example 1 and thereby to obtain the title compound as white amorphous solid.
$^1$H-NMR (400MHz, $CDCl_3$) δ : 1.22-2.12 (6H, m), 1.25 (3H, s), 1.44 (9H, s), 1.48 (3H, s), 1.60 (3H, s), 1.71 (3H, s), 2.31-2.37 (1H, m), 2.35 (3H, s), 2.76-2.92 (4H, m), 2.91 (3H, s), 2.95 (3H, s), 3.39-3.58 (3H, m), 3.71 (2H, dd, J=13.7, 6.6Hz), 4.12 (1H, d, J=7.3Hz), 4.21 (1H, d, J=8.3Hz), 4.32 (1H, d, J=8.3Hz), 4.85-4.91 (3H, m), 5.20 (1H, d, J=6.6Hz), 5.35 (1H, d, J=9.0Hz), 5.95-5.97 (2H, m); 6.08 (1H, t, J=8.5Hz), 7.21-7.25 (1H, m), 7.41-7.49 (3H, m), 7.60 (1H, t, J=7.3Hz), 7.72 (1H, t, J=7.3Hz), 8.13 (2H, d, J=7.3Hz), 8.54 (1H, d, J=4.2Hz).
FAB-MS; m/z: 947 $(M+H)^+$.

[Example 10]

(1S,2S,3R,4S,5R,8R,9S,10R,13S)-4-(Acetoxy)-2-(benzoyloxy)-9,10-{(1S)-[2-(3-dimethylcarbamoyl)azetidino]ethylidenedioxy}-5,20-epoxy-1-hydroxytax-11-en-13-yl

(2R,3S)-3-(tert-butoxycarbonylamino)-3-(3-fluoro-2-pyridyl)-2-hydroxypropionate

[Formula 82]

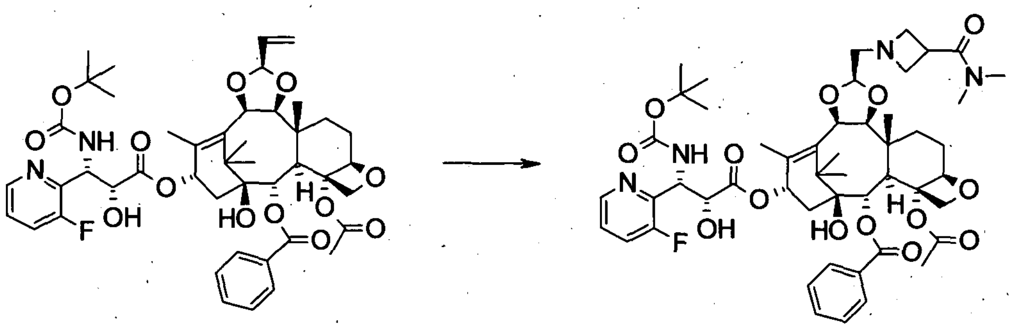

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,35)-3-(tert-butoxycarbonylamino)-3-(3-fluoro-2-pyridyl)-2-hydroxypropionate and the compound obtained in Reference Example 2, Step 2 were used as starting materials to perform the same procedure as that of Example 1 and thereby to obtain the title compound as white amorphous solid.
$^1$H-NMR (400MHz, $CDCl_3$) δ : 1.24-2.14 (7H, m), 1.28 (3H, s), 1.40 (9H, s), 1.49 (3H, s), 1.61 (3H, s), 1.78 (3H, s), 2.34 (3H, s), 2.45 (1H, dd, J=14.8, 9.6Hz), 2.79 (1H, dd, J=12.6, 5.9Hz), 2.86 (1H, dd, J=12.6, 4.5Hz), 2.91 (3H, s), 2.93 (1H, d, J=5.4Hz), 2.96 (3H, s), 3.39-3.47 (2H, m), 3.50-3.58 (1H, m), 3.69-3.74 (2H, m), 4.13 (1H, d, J=7.1Hz), 4.23 (1H, d, J=8.3Hz), 4.32 (1H, d, J=8.3Hz), 4.67 (1H, d, J=2.4Hz), 4.87 (1H, t, J=4.9Hz), 4.91 (1H, brs), 5.21 (1H, d, J=7.1Hz), 5.65 (1H, d, J=8.5Hz), 5.98 (1H, d, J=5.1Hz), 6.08 (1H, t, J=8.5Hz), 6.21 (1H, d, J=8.3Hz), 7.28-7.32 (1H, m), 7.42-7.49 (3H, m), 7.60 (1H, t, J=7.4Hz), 8.14 (2H, d, J=7.4Hz), 8.40 (1H, d, J=4.6Hz).
FAB-MS; m/z: 965 $(M+H)^+$.

[Example 11]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3S)-3-(tert-butoxycarbonylamino)-2-hydroxy-3-(2-pyridyl)propionate

[Formula 83]

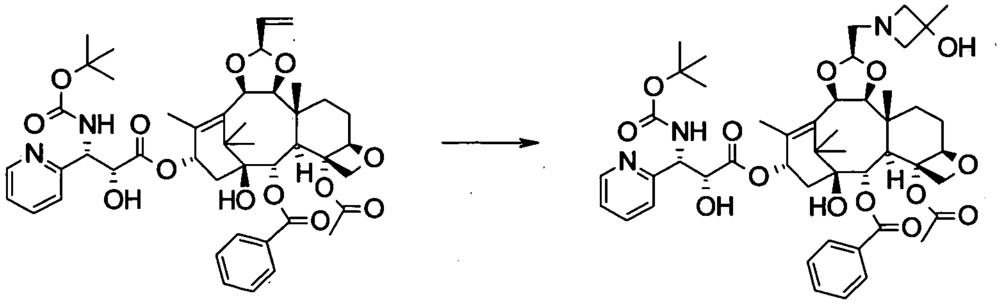

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 3 were used as starting materials to perform the same procedure as that of Example 1 and thereby to obtain the title compound as white amorphous solid.
$^1$H-NMR (400MHz, $CDCl_3$ ) δ : 1.21-2.12 (8H, m), 1.26 (3H, s), 1.43 (9H, s), 1.48 (3H, s), 1.51 (3H, s), 1.60 (3H, s), 1.71 (3H, s), 2.31-2.37 (1H, m), 2.35 (3H, s), 2.78 (1H, dd, J=12.5, 5.6Hz), 2.88-2.92 (2H, m), 3.21 (2H, t, J=8.1Hz), 3.42 (2H, t, J=8.1Hz), 4.12 (1H, d, J=7.1Hz), 4.21 (1H, d, J=8.5Hz), 4.32 (1H, d, J=8.5Hz), 4.88-4.92 (3H, m), 5.20 (1H, d, J=7.1Hz), 5.35 (1H, d, J=8.5Hz), 5.97 (2H, d, J=5.9Hz), 6.08 (1H, t, J=8.3Hz), 7.21-7.24 (1H, m), 7.41-7.49 (3H, m), 7.60 (1H, t, J=7.2Hz), 7.71 (1H, t, J=7.8Hz), 8.12 (2H, d, J=7.8Hz), 8.53 (1H, d, J=4.4Hz).
FAB-MS; m/z: 906 $(M+H)^+$.

[Example 12]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3S)-3-(tert-butoxycarbonylamino)-3-(3-fluoro-2-pyridyl)-2-hydroxypropionate

[Formula 84]

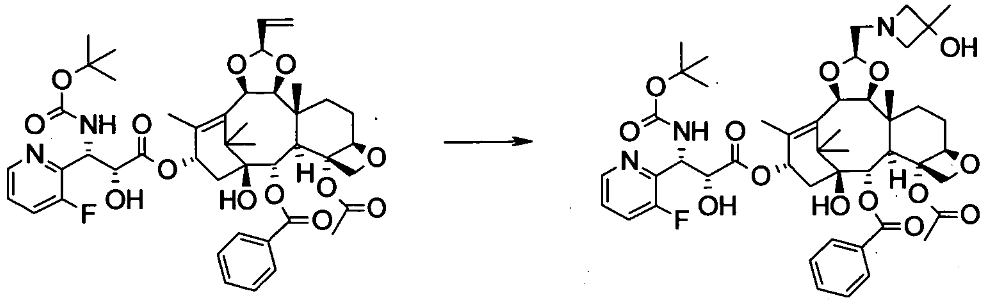

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 3 were used as starting materials to perform the same procedure as that of Example 1 and thereby to obtain the title compound as white amorphous solid.
$^1$H-NMR (400MHz, $CDCl_3$) δ : 1.24-2.13 (8H, m), 1.29 (3H, s), 1.40 (9H, s), 1.50 (3H, s), 1.52 (3H, s), 1.61 (3H, s), 1.78 (3H, s), 2.34 (3H, s), 2.45 (1H, dd, J=14.9, 9.3Hz), 2.80 (1H, dd, J=12.5, 5.9Hz), 2.88-2.94 (2H, m), 3.21 (2H, t, J=8.2Hz), 3.44 (2H, t, J=7.8Hz), 4.14 (1H, d, J=7.1Hz), 4.23 (1H, d, J=8.5Hz), 4.32 (1H, d, J=8.5Hz), 4.67 (1H, d, J=2.7Hz), 4.89-4.92 (2H, m), 5.22 (1H, d, J=6.8Hz), 5.65 (1H, d, J=8.3Hz), 5.99 (1H, d, J=4.9Hz), 6.09 (1H, t, J=8.4Hz), 6.21 (1H, d, J=8.1Hz), 7.28-7.32 (1H, m), 7.42-7.49 (3H, m), 7.60 (1H, t, J=7.4Hz), 8.12-8.15 (2H, m), 8.39-8.41 (1H, m).
FAB-MS; m/z: 924 $(M+H)^+$.

[Example 13]

(1S,2S,3R,4S,5R,8R,9S,10R,13S)-4-(Acetoxy)-2-(benzoyloxy)-5,20-epoxy-9,10-[(1S)-2-(3-ethyl-3-hydroxyazetidino)ethylidenedioxy]-1-hydroxytax-11-en-13-yl

(2R,3S)-3-(tert-butoxycarbonylamino)-3-(3-fluoro-2-pyridyl)-2-hydroxypropionate

[Formula 85]

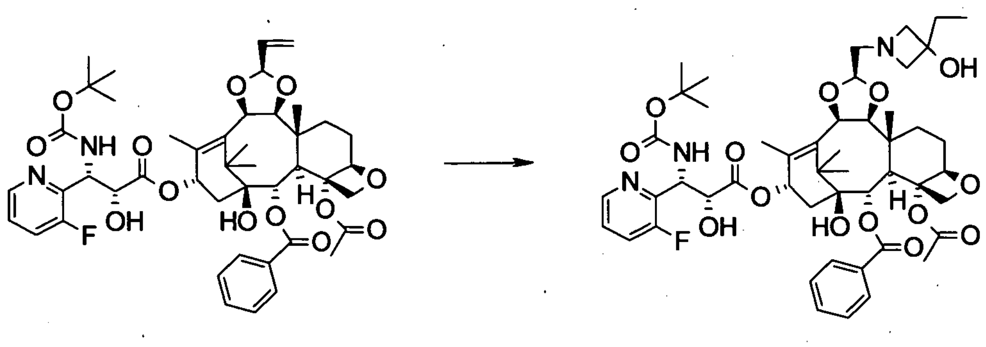

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarboriylamino)-3-(3-fluoro-2-pyridyl)-2-hydroxypropionate and the compound obtained in Reference Example 4, Step 2 were used as starting materials to perform the same procedure as that of Example 1 and thereby to obtain the title compound as white amorphous solid.
$^1$ H-NMR (400MHz, $CDCl_3$) δ : 0.97 (3H, t, J=7.3Hz), 1.21-2.48 (11H, m), 1.29 (3H, s), 1.40 (9H, s), 1.50 (3H, s), 1.62 (3H, s), 1.78 (3H, s), 2.34 (3H, s), 2.82 (1H, dd, J=12.5, 5.9Hz), 2.88-2.95 (2H, m), 3.12-3.22 (2H, m), 3.46 (2H, t, J=6.8Hz), 4.14 (1H, d, J=7.1Hz), 4.23 (1H, d, J=8.5Hz), 4.33 (1H, d, J=8.5Hz), 4.68 (1H, d, J=2.4Hz), 4.88-4.92 (2H, m), 5.22 (1H, d, J=6.3Hz), 5.65 (1H, d, J=7.6Hz), 5.99 (1H, d, J=5.1Hz), 6.09 (1H, t, J=8.8Hz), 6.17-6.23 (1H, m), 7.28-7.33 (1H, m), 7.41-7.50 (3H, m), 7.57-7.62 (1H, m), 8.10-8.15 (2H, m), 8.38-8.41 (1H, m).
FAB-MS; m/z: 938 $(M+H)^+$.

[Example 14]

(1S,2S,3R,4S,5R,8R,9S,10R,13S)-4-(Acetoxy)-2-(benzoyloxy)-9,10-[(1S)-2-(3-cyclopropyl-3-hydroxyazetidino)ethylidenedioxy]-5,20-epoxy-1-hydroxytax-11-en-13-yl

(2R,3S)-3-(tert-butoxycarbonylamino)-3-(3-fluoro-2-pyridyl)-2-hydroxypropionate

[Formula 86]

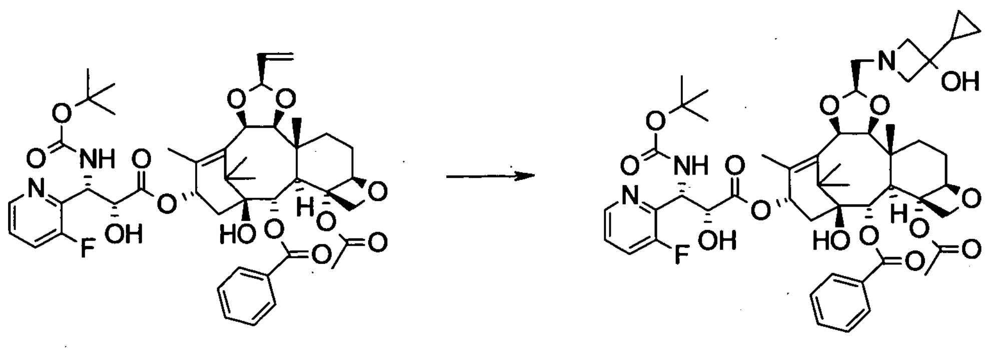

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 5, Step 2 were used as starting materials to perform the same procedure as that of Example 1 and thereby to obtain the title compound as white amorphous solid.

$^1$H-NMR (400MHz, $CDCl_3$) δ : 0.43-0.48 (2H, m), 0.51-0.58 (2H, m), 1.16-2.50 (10H, m), 1.29 (3H, s), 1.41 (9H, s), 1.50 (3H, s), 1.61 (3H, s), 1.78 (3H, s), 2.34 (3H, s), 2.79 (1H, dd, J=12.5, 5.9Hz), 2.86-2.95 (2H, m), 3.17-3.24 (2H, m), 3.33-3.39 (2H, m), 4.13 (1H, d, J=7.1Hz), 4.23 (1H, d, J=8.3Hz), 4.33 (1H, d, J=8.3Hz), 4.68 (1H, d, J=2.7Hz), 4.87-4.93 (2H, m), 5.21 (1H, d, J=6.8Hz), 5.65 (1H, d, J=8.3Hz), 5.99 (1H, d, J=5.1Hz), 6.09 (1H, t, J=8.7Hz), 6.21 (1H, d, J=9.0Hz), 7.28-7.33 (1H, m), 7.42-7.50 (3H, m), 7.60 (1H, t, J=7.4Hz), 8.14 (2H, d, J=7.3Hz), 8.40 (1H, d, J=4.6Hz).
FAB-MS; m/z: 950 $(M+H)^+$.

[Example 15]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-trifluoromethylazetidino)ethylidenedioxy]tax-11-en-13-yl

(2R,3S)-3-(tert-butoxycarbonylamino)-3-(3-fluoro-2-pyridyl)-2-hydroxypropionate

[Formula 87]

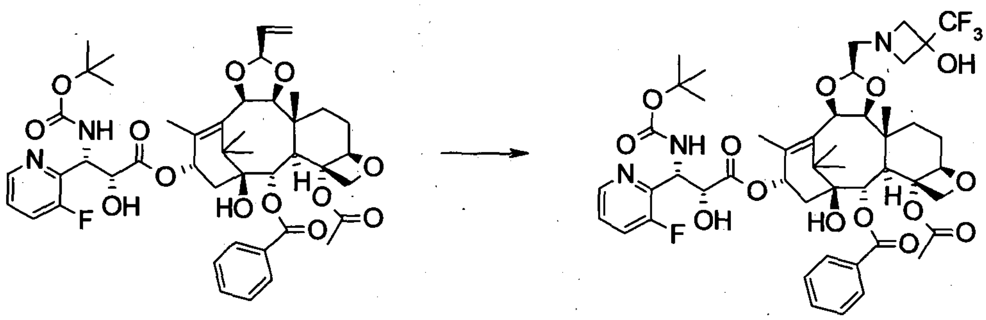

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 6, Step 2 were used as starting materials to perform the same procedure as that of Example 1 and thereby to obtain the title compound as white amorphous solid.
$^1$H-NMR (400MHz, $CDCl_3$) δ : 0.96-2.53 (7H, m), 1.29 (3H, s), 1.40 (9H, s), 1.50 (3H, s), 1.61 (3H, s), 1.78 (3H, s), 2.35 (3H, s), 2.86 (1H, dd, J=12.5, 5.6Hz), 2.92-2.97 (2H, m), 3.34-3.54 (4H, m), 3.69-3.78 (2H, m), 4.09-4.16 (1H, m), 4.23 (1H, d, J=8.3Hz), 4.33 (1H, d, J=8.3Hz), 4.68 (1H, d, J=2.7Hz), 4.89-4.93 (2H, m), 5.21 (1H, d, J=6.8Hz), 5.64 (1H, d, J=9.0Hz), 5.98 (1H, d, J=4.9Hz), 6.09 (1H, t, J=8.5Hz), 6.25 (1H, d, J=8.5Hz), 7.28-7.33 (1H, m), 7.43-7.50 (3H, m), 7.61 (1H; t, J=7.4Hz), 8.14 (2H, d, J=7.3Hz), 8.40 (1H, d, J=4.6Hz).
FAB-MS; m/z: 978 $(M+H)^+$.

[Example 16]

(1S,2S,3R,4S,5R,8R,9S,10R,13S)-4-(Acetoxy)-2-(benzoyloxy)-9,10-[(1S)-2-(3-cyanoazetidino)ethylidenedioxy]-5,20-epoxy-1-hydroxytax-11-en-13-yl

(2R,3S)-3-(tert-butoxycarbonylamino)-2-hydroxy-3-(2-pyridyl)propionate

[Formula 88]

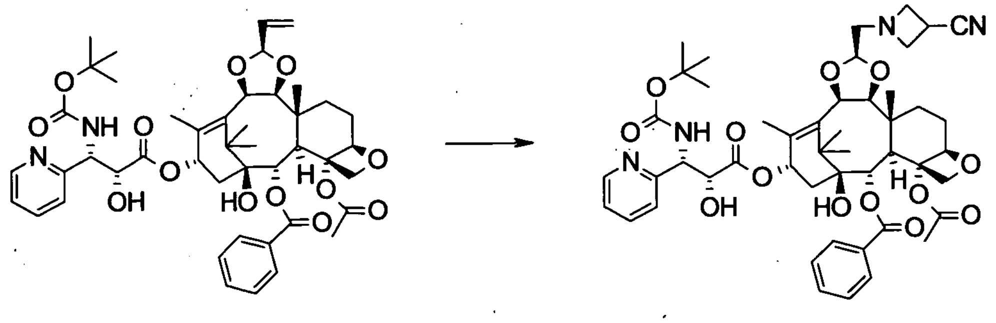

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 7 were used as starting materials to perform the same procedure as that of Example 1 and thereby to obtain the title compound as white amorphous solid.

$^1$H-NMR (400MHz, $CDCl_3$) δ : 1.24-2.14 (7H, m), 1.26 (3H, s), 1.44 (9H, s), 1.48 (3H, s), 1.58 (3H, s), 1.71 (3H, s), 2.31-2.37 (1H, m), 2.35 (3H, s), 2.77 (1H, dd, J=12.5, 5.6Hz), 2.85 (1H, dd, J=12.5, 4.6Hz), 2.92 (1H, d, J=5.1Hz), 3.30-3.35 (1H, m), 3.45-3.55 (2H, m), 3.70-3.75 (2H, m), 4.12 (1H, dd, J=7.3, 1.7Hz), 4.21 (1H, d, J=8.5Hz), 4.33 (1H, d, J=8.5Hz), 4.83-4.92 (3H, m), 5.20 (1H, d, J=7.1Hz), 5.35 (1H, d, J=7.8Hz), 5.92-5.98 (2H, m), 6.09 (1H, t, J=7.6Hz), 7.22-7.26 (1H, m), 7.41-7.49 (3H, m), 7.60 (1H, t, J=7.4Hz), 7.72 (1H, t, J=7.4Hz), 8.13 (2H, d, J=7.8Hz), 8.53 (1H, d, J=4.6Hz).

FAB-MS; m/z: 901 $(M+H)^+$, 923 $(M+Na)^+$.

[Example 17]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxymethylazetidino)ethylidenedioxy]tax-11-en-13-yl

(2R,3S)-3-(tert-butoxycarbonylamino)-2-hydroxy-3-(2-pyridyl)propionate

[Formula 89]

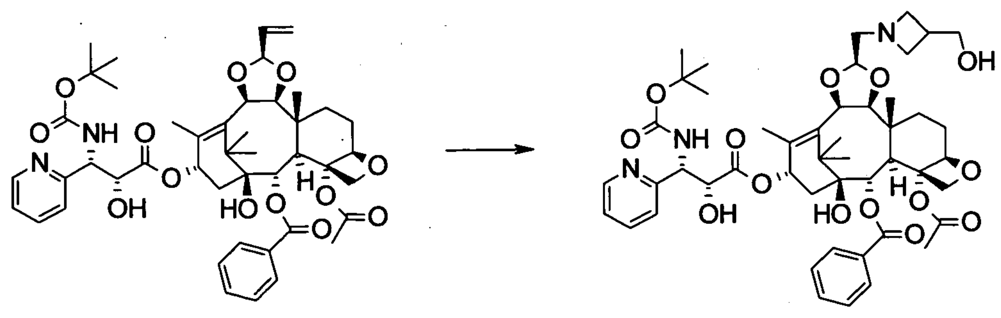

(1S,2S,3R,4S,5R,8R,9S,10R,13S)-4-(Acetoxy)-2-(benzoyloxy)-5,20-epoxy-1-hyd roxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 8 were used as starting materials to perform the same procedure as that of Example 1 and thereby to

obtain the title compound as white amorphous solid.
$^1$ H-NMR (400MHz, $CDCl_3$) δ : 1.23-2.12 (8H, m), 1.25 (3H, s), 1.44 (9H, s), 1.49 (3H, s), 1.60 (3H, s), 1.70 (3H, s), 2.31-2.37 (1H, m), 2.34 (3H, s), 2.65-2.71 (1H, m), 2.76 (1H, dd, J=12.5, 5.9Hz), 2.85 (1H, dd, J=12.5, 4.2Hz), 2.92 (1H, d, J=5.1Hz), 3.21-3.26 (2H, m), 3.47-3.53 (2H, m), 3.80 (2H, d, J=5.6Hz), 4.11 (1H, d, J=7.1Hz), 4.21 (1H, d, J=8.3Hz), 4.33 (1H, d, J=8.3Hz), 4.86-4.92 (3H, m), 5.19 (1H, d, J=6.8Hz), 5.35 (1H, d, J=9.3Hz), 5.94-5.99 (2H, m), 6.08 (1H, t, J=7.9Hz), 7.21-7.25 (1H, m), 7.41-7.49 (3H, m), 7.60 (1H, t, J=7.3Hz), 7.72 (1H, dt, J=7.6, 1.7Hz), 8.13 (2H, d, J=7.6Hz), 8.54 (1H, d, J=4.6Hz). FAB-MS; m/z: 906 $(M+H)^+$.

[Example 18]

(1S,2S,3R,4S,5R,8R,9S,10R,13S)-4-(Acetoxy)-9,10-{(1S)-2-[3-(N-acetyl-N-methylamino)azetidino]ethylidenedioxy}-2-(benzoyloxy)-5,20-epoxy-1-hydroxytax-11-en-13-yl

(2R,3S)-3-(tert-butoxycarbonylamino)-3-(3-fluoro-2-pyridyl)-2-hydroxypropionate

[Formula 90]

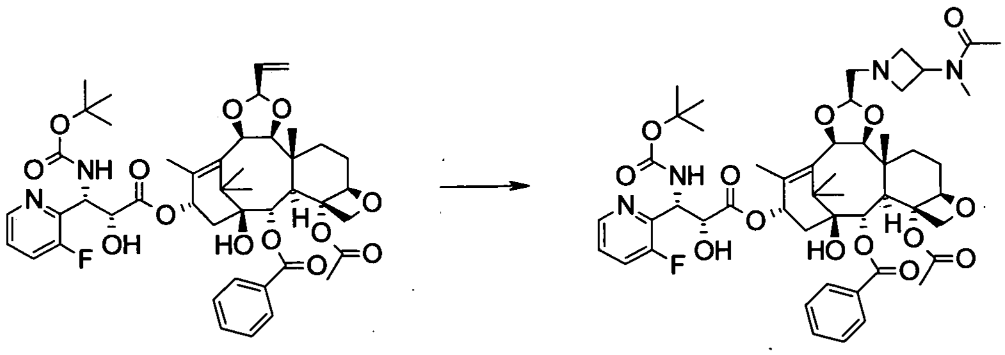

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 9, Step 2 were used as starting materials to perform the same procedure as that of Example 1 and thereby to obtain the title compound as white amorphous solid.
$^1$ H-NMR (400MHz, $CDCl_3$) δ : 1.26-2.13 (7H, m), 1.29 (3H, s), 1.40 (9H, s), 1.50 (3H, s), 1.61 (3H, s), 1.78 (3H, s), 2.08, 2.10 (total 3H, each s), 2.35 (3H, s), 2.42-2.49 (1H, m), 2.76-2.99 (3H, m), 3.03, 3.08 (total 3H, each s), 3.22-3.36 (2H, m), 3.73 (2H, dd, J=13.9, 7.1Hz), 4.13-4.18 (1H, m), 4.23 (1H, d, J=8.3Hz), 4.33 (1H, d, J=8.3Hz), 4.55-4.98 (3H, m), 4.68 (1H, d, J=2.7Hz), 5.21-5.28 (1H, m), 5.64 (1H, d, J=8.8Hz), 5.99 (1H, d, J=5.1Hz), 6.09 (1H, t, J=8.3Hz), 6.21 (1H, d, J=8.3Hz), 7.28-7.33 (1H, m), 7.43-7.49 (3H, m), 7.60 (1H, t, J=7.4Hz), 8.13 (2H, d, J=7.4Hz), 8.40 (1H, d, J=4.4Hz). FAB-MS; m/z: 965 $(M+H)^+$.

[Example 19]

(1S,2S,3R,4S,5R,8R,9S,10R,13S)-4-(Acetoxy)-2-(benzoyloxy)-5,20-epoxy-1-hydroxy-9,10-{(1S)-2-[3-(N-methanesulfonyl-N-methylamino)azetidino]ethylidenedioxy}tax-11-en-13-yl (2R,3S)-3-(tert-butoxycarbonylamino)-3-(3-fluoro-2-pyridyl)-2-hydroxypropionate

[Formula 91]

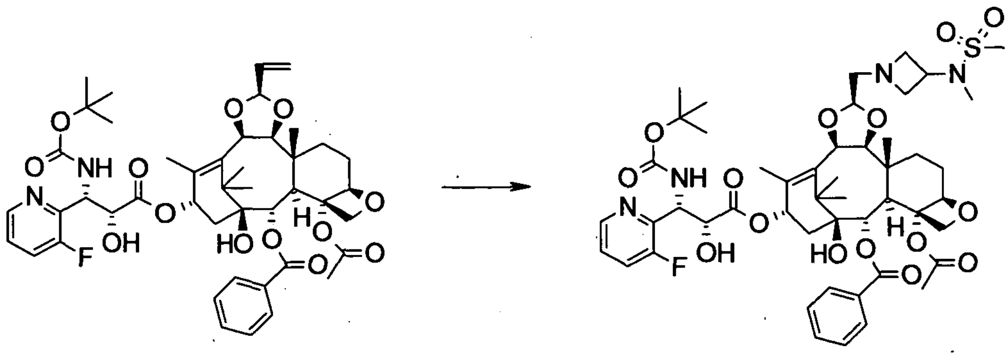

(1S,2S,3R,4S,6R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 10, Step 2 were used as starting materials to perform the same procedure as that of Example 1 and thereby to obtain the title compound as white amorphous solid.
$^1$H-NMR (400MHz, $CDCl_3$) δ : 1.26-2.16 (7H, m), 1.29 (3H, s), 1.40 (9H, s), 1.50 (3H, s), 1.61 (3H, s), 1.78 (3H, s), 2.34 (3H, s), 2.42-2.48 (1H, m), 2.76-2.94 (3H, m), 2.78 (3H, s), 2.91 (3H, s), 3.32 (2H, dd, J=17.6, 7.6Hz), 3.70 (2H, dd, J=13.1, 6.0Hz), 4.14 (1H, d, J=7.3Hz), 4.23 (1H, d, J=8.5Hz), 4.27-4.34 (2H, m), 4.67 (1H, d, J=2.0Hz), 4.86 (1H, t, J=5.0Hz), 4.91 (1H, s), 5.22 (1H, d, J=6.8Hz), 5.64 (1H, d, J=8.1Hz), 5.99 (1H, d, J=4.9Hz), 6.09 (1H, t, J=8.3Hz), 6.20 (1H, d, J=8.3Hz), 7.28-7.33 (1H, m), 7.43-7.50 (3H, m), 7.60 (1H, t, J=7.4Hz), 8.12-8.14 (2H, m), 8.40 (1H, d, J=4.6Hz).
FAB-MS; m/z: 1001 $(M+H)^+$.

[Example 20]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-methoxymethylazetidino]ethylidenedioxy}tax-11-en-13-yl

(2R,3S)-3-(tert-butoxycarbonylamino)-3-(3-fluoro-2-pyridyl)-2-hydroxypropionate

[Formula 92]

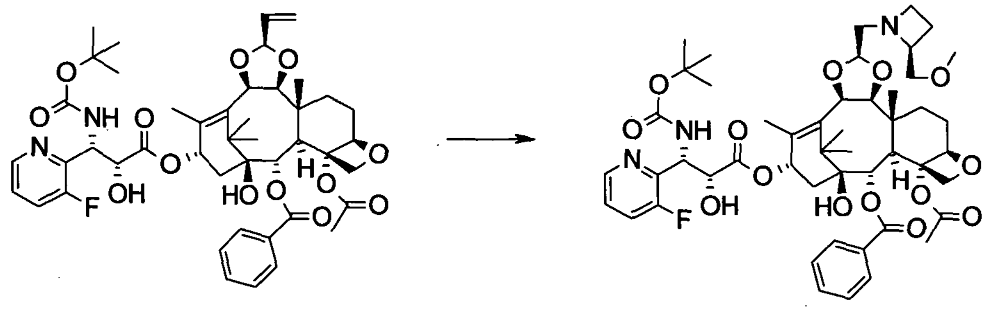

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 11, Step 2 were used as starting materials to perform the same procedure as that of Example 1 and thereby to obtain the title compound as white amorphous solid.
$^1$ H-NMR (400MHz, $CDCl_3$) δ : 1.24-2.10 (8H, m), 1.28 (3H, s), 1.40 (9H, s), 1.49 (3H, s), 1.61 (3H, s), 1.78 (3H, s), 2.34

(3H, s), 2.42-2.48 (1H, m), 2.77 (1H, dd, J=12.8, 4.8Hz), 2.93-3.04 (3H, m), 3.38 (3H, s), 3.43-3.55 (4H, m), 4.11-4.14 (2H, m), 4.23 (1H, d, J=8.3Hz), 4.33 (1H, d, J=8.3Hz), 4.67 (1H, d, J=2.2Hz), 4.88-4.92 (2H, m), 5.22 (1H, d, J=7.6Hz), 5.64 (1H, d, J=8.3Hz), 5.99 (1H, d, J=5.1Hz), 6.08 (1H, t, J=8.5Hz), 6.20 (1H, d, J=8.5Hz), 7.28-7.32 (1H, m), 7.42-7.49 (3H, m), 7.60 (1H, t, J=7.4Hz), 8.12-8.15 (2H, m), 8.40 (1H, d, J=4.6Hz).
FAB-MS; m/z: 938 $(M+H)^+$.

[Example 21]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-2-hydroxy-3-(2-pyridyl)propionate

[Formula 93]

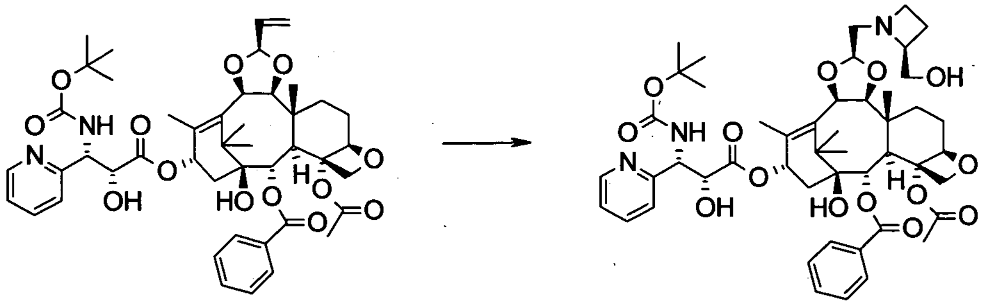

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 25 were used as starting materials to perform the same procedure as that of Example 1 and thereby to obtain the title compound as white amorphous solid.
$^1$H-NMR (400MHz, $CDCl_3$) δ: 1.23-2.12 (9H, m), 1.25 (3H, s), 1.44 (9H, s), 1.49 (3H, s), 1.58 (3H, s), 1.70 (3H, s), 2.20-2.37 (2H, m), 2.35 (3H, s), 2.83 (1H, dd, J=12.9, 4.4Hz), 2.88-2.93 (2H, m), 3.11 (1H, dd, J=15.7, 8.7Hz), 3.41-3.50 (3H, m), 3.64-3.67 (1H, m), 4.13 (1H, d, J=7.6Hz), 4.21 (1H, d, J=8.5Hz), 4.33 (1H, d, J=8.5Hz), 4.86-4.92 (3H, m), 5.22 (1H, d, J=7.1Hz), 5.35 (1H, d, J=9.3Hz), 5.93-5.97 (2H, m), 6.08 (1H, t, J=8.1Hz), 7.22-7.25 (1H, m), 7.41-7.49 (3H, m), 7.60 (1H, t, J=7.6Hz), 7.72 (1H, dt, J=7.6, 1.7Hz), 8.11-8.14 (2H, m), 8.54 (1H, d, J=4.6Hz).
FAB-MS; m/z: 906 $(M+H)^+$.

[Example 22]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-3-(3-fluoro-2-pyridyl)-2-hydroxypropionate

[Formula 94]

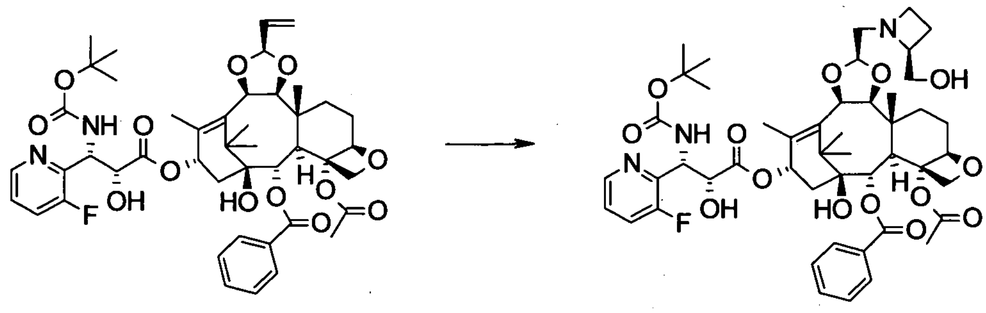

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 25 were used as starting materials to perform the same procedure as that of Example 1 and thereby to obtain the title compound as white amorphous solid.
$^{1}$H-NMR (400MHz, $CDCl_3$) $\delta$ : 1.25-2.48 (11H, m), 1.28 (3H, s), 1.41 (9H, s), 1.50 (3H, s), 1.59 (3H, s), 1.78 (3H, s), 2.34 (3H, s), 2.82-2.94 (3H, m), 3.09-3.15 (1H, m), 3.44 (1H, d, J=11.0Hz), 3.52 (2H, brs), 3.67 (1H, d, J=11.0Hz), 4.15 (1H, d, J=7.3Hz), 4.23 (1H, d, J=8.5Hz), 4.33 (1H, d, J=8.5Hz), 4.67 (1H, s), 4.88-4.91 (2H, m), 5.23 (1H, d, J=7.6Hz), 5.64 (1H, d, J=9.8Hz), 5.98 (1H, d, J=5.1Hz), 6.08 (1H, t, J=8.8Hz), 6.20 (1H, d, J=7.8Hz), 7.28-7.33 (1H, m), 7.42-7.49 (3H, m), 7.60 (1H, t, J=7.4Hz), 8.13 (2H, d, J=8.3Hz), 8.40 (1H, d, J=4.6Hz).

[Example 23]

(1S,2S,3R,4S,5R,8R,9S,10R,13S)-4-(Acetoxy)-2-(benzoyloxy)-9,10-{(1S)-2-[(2S)-2-(dimethylcarbamoyl)azetidino]ethylidenedioxy}-5,20-epoxy-1-hydroxytax-11-en-13-yl

(2R,3S)-3-(tert-butoxycarbonylamino)-2-hydroxy-3-(2-pyridyl)propionate

[Formula 95]

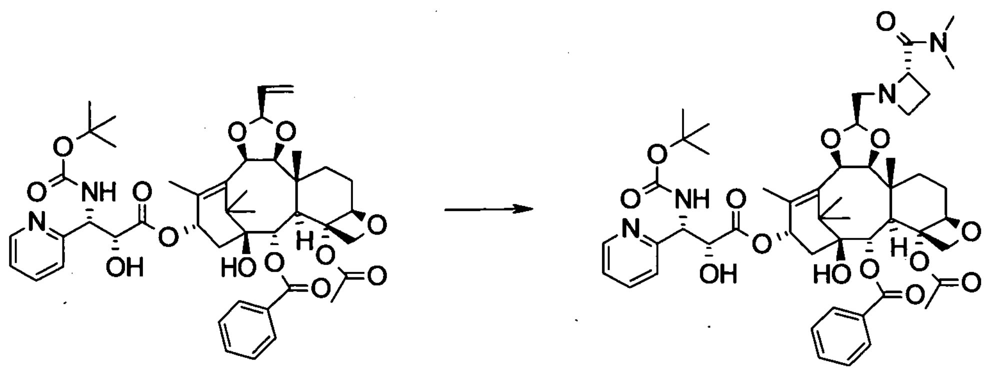

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyridyl)propionate and the compound obtained in Reference Example 12, Step 2 were used as starting materials to perform the same procedure as that of Example 1 and thereby to obtain the title compound as white amorphous solid.
$^1$ H-NMR (400MHz, $CDCl_3$) δ : 1.19-2.09 (7H, m), 1.20 (3H, s), 1.45 (9H, s), 1.48 (3H, s), 1.58 (3H, s), 1.65 (3H, s), 2.28-2.36 (3H, m), 2.33 (3H, s), 2.84 (1H, dd, J=12.5, 5.6Hz), 2.89-2.96 (2H, m), 2.91 (3H, s), 2.94 (3H, s), 3.06-3.12 (1H, m), 3.54-3.59 (1H, m), 3.95 (1H, t, J=8.4Hz), 4.08 (1H, d, J=7.1Hz), 4.21 (1H, d, J=8.3Hz), 4.32 (1H, d, J=8.3Hz), 4.89-4.92 (2H, m), 5.04 (1H, dd, J=5.4, 4.2Hz), 5.16 (1H, d, J=6.6Hz), 5.35 (1H, d, J=9.3Hz), 5.95-5.98 (2H, m), 6.04 (1H, t, J=8.5Hz), 7.22-7.25 (1H, m), 7.41-7.49 (3H, m), 7.60 (1H, t, J=7.4Hz), 7.72 (1H, dt, J=7.7, 1.6Hz), 8.12 (2H, d, J=7.7Hz), 8.54 (1H, d, J=4.4Hz).
FAB-MS; m/z: 947 $(M+H)^+$.

[Example 24]

(1S,2S,3R,4S,5R,8R,9S,10R,13S)-4-(Acetoxy)-2-(benzoyloxy)-9,10-{(1S)-2-[(2S)-2-(dimethylcarbamoyl)azetidino] ethylidenedioxy}-5,20-epoxy-1-hydroxytax-11-en-13-yl

(2R,3S)-3-(tert-butoxycarbonylamino)-3-(3-fluoro-2-pyridyl)-2-hydroxypropionate

[Formula 96]

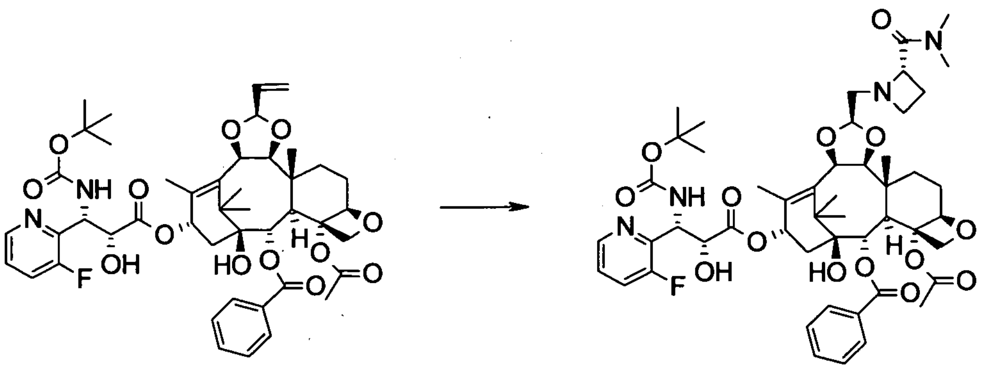

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 12, Step 2 were used as starting materials to perform the same procedure as that of Example 1 and thereby to obtain the title compound as white amorphous solid.
$^1$H-NMR (400MHz, $CDCl_3$) δ : 1.20-2.09 (7H, m), 1.23 (3H, s), 1.41 (9H, s), 1.48 (3H, s), 1.59 (3H, s), 1.73 (3H, s), 2.28-2.46 (3H, m), 2.32 (3H, s), 2.85 (1H, dd, J=12.2, 5.6Hz), 2.90-2.96 (2H, m), 2.92 (3H, s), 2.95 (3H, s), 3.07-3.12 (1H, m), 3.55-3.59 (1H, m), 3.96 (1H, t, J=8.5Hz), 4.10 (1H, d, J=7.1Hz), 4.22 (1H, d, J=8.3Hz), 4.32 (1H, d, J=8.3Hz), 4.68 (1H, s), 4.91 (1H, s), 5.05 (1H, dd, J=5.6, 4.2Hz), 5.18 (1H, d, J=7.1Hz), 5.66 (1H, d, J=8.8Hz), 5.99 (1H; d, J=5.1Hz), 6.05 (1H, t, J=8.5Hz), 6.20 (1H, d, J=9.0Hz), 7.28-7.33 (1H, m), 7.42-7.49 (3H, m), 7.60 (1H, t, J=7.4Hz), 8.12-8.14 (2H, m), 8.40 (1H, d, J=4.6Hz).
FAB-MS; m/z: 965 $(M+H)^+$.

[Example 25]

(1S,2S,3R,4S,5R,8R,9S,10R,13S)-4-(Acetoxy)-2-(benzoyloxy)-9,10-{(1S)-2-[3-(dimethylcarbamoyloxy)azetidino] ethylidenedioxy}-5,20-epoxy-1-hydroxytax-11-en-13-yl

(2R,3S)-3-(tert-butoxycarbonylamino)-3-(3-fluoro-2-pyridyl)-2-hydroxypropionate

[Formula 97]

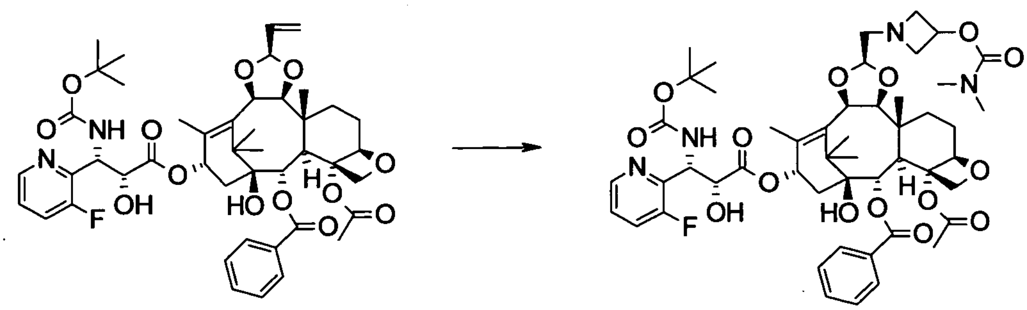

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 13, Step 2 were used as starting materials to perform the same procedure as that of Example 1 and thereby to obtain the title compound as white amorphous solid.
$^1$H-NMR (400MHz, $CDCl_3$) δ : 1.24-2.50 (13H, m), 1.29 (3H, s), 1.40 (9H, s), 1.50 (3H, s), 1.62 (3H, s), 1.78 (3H, s), 1.81 (3H, s), 2.77-2.98 (4H, m), 3.18-3.25 (1H, m), 3.55 (1H, d, J=2.0Hz), 3.80-3.87 (1H, m), 4.14 (1H, d, J=6.8Hz), 4.20-4.26 (1H, m), 4.33 (1H, d, J=8.5Hz), 4.65-4.71 (1H, m), 4.74-4.79 (1H, m), 4.87-4.93 (1H, m), 5.02-5.38 (3H, m), 5.64 (1H, d, J=8.3Hz), 5.98-6.00 (1H, m), 6.05-6.13 (1H, m), 6.17-6.24 (1H, m), 7.28-7.34 (1H, m), 7.42-7.50 (3H, m), 7.60 (1H, t, J=7.3Hz), 8.13 (2H, d, J=7.3Hz), 8.41 (1H, d, J=4.6Hz).
FAB-MS; m/z: 981 $(M+H)^+$.

[Example 26]

(1S,2S,3R,4S,5R,8R,9S,10R,13S)-4-(Acetoxy)-2-(benzoyloxy)-9,10-[(1S)-2-(3-carbamoyloxy)azetidino]ethylidenedioxy]-5,20-epoxy-1-hydroxytax-11-en-13-yl

(2R,3S)-3-(tert-butoxycarbonylamino)-3-(3-fluoro-2-pyridyl)-2-hydroxypropionate

[Formula 98]

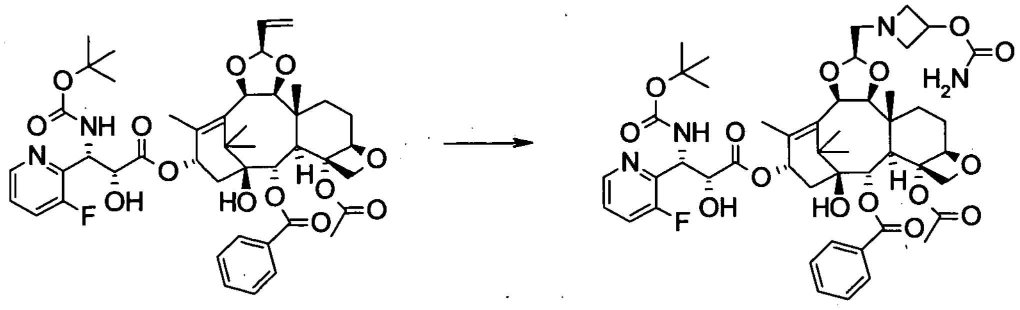

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 14, Step 2 were used as starting materials to perform the same procedure as that of Example 1 and thereby to obtain the title compound as white amorphous solid.

$^1$H-NMR (400MHz, $CDCl_3$) δ : 1.20-2.49 (8H, m), 1.28 (3H, s), 1.40 (9H, s), 1.50 (3H, s), 1.61 (3H, s), 1.78 (3H, s), 2.34 (3H, s), 2.80 (1H, dd, J=12.5, 5.9Hz), 2.88-2.95 (2H, m), 3.24-3.28 (2H, m), 3.80 (2H, q , J=6.8Hz), 4.09-4.15 (1H, m), 4.23 (1H, d, J=8.5Hz), 4.33 (1H, d, J=8.1Hz), 4.68 (1H, d, J=2.7Hz), 4.70 (2H, brs), 4.87-4.93 (2H, m), 5.04-5.11 (1H, m), 5.22 (1H, d, J=7.1Hz), 5.64 (1H, d, J=8.5Hz), 5.99 (1H, d, J=5.1Hz), 6.08 (1H, t, J=8.3Hz), 6.21 (1H, d, J=9.0Hz), 7.28-7.33 (1H, m), 7.42-7.50 (3H, m), 7.60 (1H, t, J=7.4Hz), 8.13 (2H, d, J=7.3Hz), 8.40 (1H, d, J=4.6Hz).

FAB-MS; m/z: 953 $(M+H)^+$.

[Example 27]

(1S,2S,3R,4S,5R,8R,9S,10R,13S)-4-(Acetoxy)-2-(benzoyloxy)-5,20-epoxy-1-hydroxy-9,10-{(1S)-2-[3-(N-methoxycarbonyl-N-methylamino)azetidino]ethylidenedioxy}tax-11-en-13-yl (2R,3S)-3-(tert-butoxycarbonylamino)-3-(3-fluoro-2-pyridyl)-2-hydroxypropionate

[Formula 99]

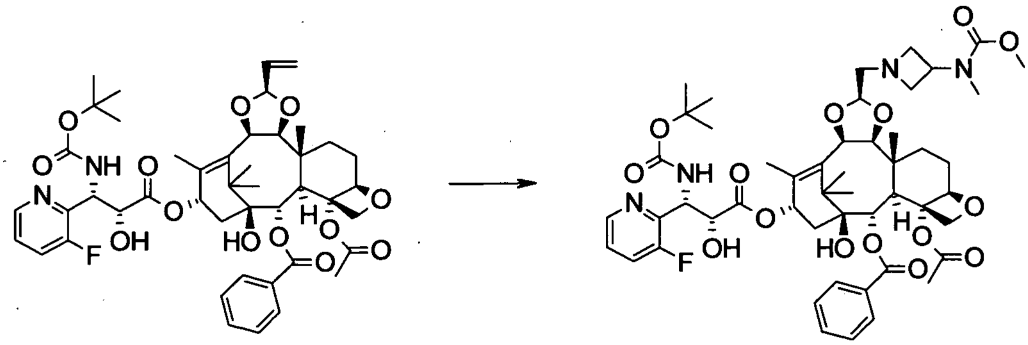

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 15, Step 2 were used as starting materials to perform the same procedure as that of Example 1 and thereby to obtain the title compound as white amorphous solid.

$^1$ H-NMR (400MHz, $CDCl_3$) δ : 1.23-2.49 (12H, m), 1.29 (3H, s), 1.40 (9H, s), 1.50 (3H, s), 1.62 (3H, s), 1.78 (3H, s), 1.81 (3H, s), 2.75-2.95 (1H, m), 2.96 (3H, s), 3.24 (1H, brs), 3.55 (1H, d, J=1.7Hz), 3.70 (3H, s), 4.14 (1H, d, J=7.3Hz), 4.20-4.26 (1H, m), 4.33 (1H, d, J=8.5Hz), 4.66-4.93 (4H, m), 5.20-5.37 (1H, m), 5.64 (1H, d, J=8.8Hz), 5.99 (1H, d, J=4.9Hz), 6.06-6.13 (1H, m), 6.18-6.25 (1H, m), 7.28-7.33 (1H, m), 7.42-7.50 (3H, m), 7.60 (1H, t, J=7.3Hz), 8.13 (2H, d, J=7.6Hz), 8.41 (1H, d, J=4.6Hz).

[Example 28]

(1S,2S,3R,4S,5R,8R,9S,10R,13S)-4-(Acetoxy)-2-(benzoyloxy)-5,20-epoxy-1-hydroxy-9,10-{(1S)-2-[3-(methanesulfonyl)azetidino]ethylidenedioxy}tax-11-en-13-yl

(2R,3S)-3-(tert-butoxycarbonylamino)-3-(3-fluoro-2-pyridyl)-2-hydroxypropionate

[Formula 100]

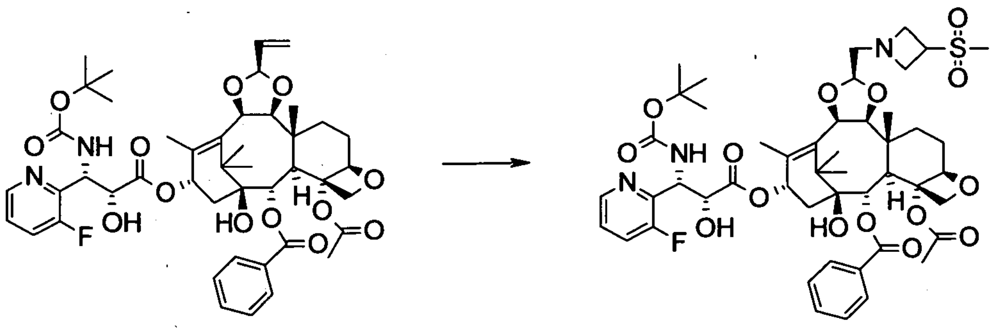

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 16, Step 3 were used as starting materials to perform the same procedure as that of Example 1 and thereby to obtain the title compound as white amorphous solid.
$^1$H-NMR (400MHz, $CDCl_3$) δ : 1.26-2.14 (7H, m), 1.29 (3H, s), 1.40 (9H, s), 1.50 (3H, s), 1.60 (3H, s), 1.78 (3H, s), 2.35 (3H, s), 2.45 (1H, dd, J=14.4, 9.5Hz), 2.83 (1H, dd, J=12.5, 5.6Hz), 2.88-2.93 (2H, m), 2.95 (3H, s), 3.68-3.79 (4H, m), 3.88-3.95 (1H, m), 4.14 (1H, d, J=7.3Hz), 4.23 (1H, d, J=8.5Hz), 4.33 (1H, d, J=8.5Hz), 4.67 (1H, d, J=2.2Hz), 4.86 (1H, t, J=4.9Hz), 4.91 (1H, s), 5.21 (1H, d, J=6.8Hz), 5.64 (1H, d, J=8.5Hz), 5.98 (1H, d, J=4.9Hz), 6.09 (1H, t, J=8.2Hz), 6.21 (1H, d, J=8.8Hz), 7.28-7.33 (1H, m), 7.43-7.49 (3H, m), 7.60 (1H, t, J=7.4Hz), 8.14 (2H, d, J=7.4Hz), 8.40 (1H, d, J=4.6Hz).
FAB-MS; m/z: 972 $(M+H)^+$.

[Example 29]

(1S,2S,3R,4S,5R,8R,9S,10R,13S)-4-(Acetoxy)-2-(benzoyloxy)-9,10-{(1S)-2-[3-(dimethylamino)azetidino]ethylidenedioxy}-5,20-epoxy-1-hydroxytax-11-en-13-yl

(2R,3S)-3-(tert-butoxycarbonylamino)-2-hydroxy-3-(3-fluoro-2-pyridyl)propionate

[Formula 101]

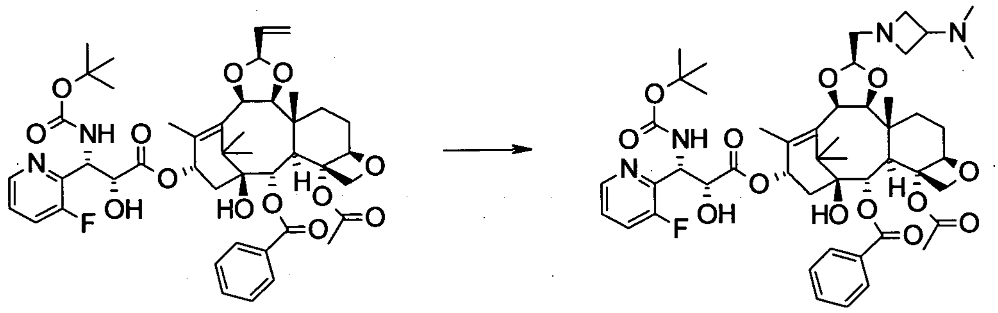

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and 3-(N,N-dimethylamino) azetidine hydrochloride (J. Med. Chem., 1993, 7, 801) were used as starting materials to perform the same procedure as that of Example 1 and thereby to obtain the title compound as white amorphous solid.

$^1$H-NMR (400MHz, $CDCl_3$) δ : 1.24-2.16 (7H, m), 1.28 (3H, s), 1.40 (9H, s), 1.48 (3H, s), 1.61 (3H, s), 1.78 (3H, s), 2.13 (6H, s), 2.34 (3H, s), 2.41-2.48 (1H, m), 2.81 (1H, dd, J=12.5, 5.6Hz), 2.86-2.96 (3H, m), 3.04 (2H, q , J=7.3Hz), 3.62-3.66 (2H, m), 4.13 (1H, d, J=7.3Hz), 4.23 (1H, d, J=8.3Hz), 4.32 (1H, d, J=8.3Hz), 4.67 (1H, d, J=2.7Hz), 4.87-4.91 (2H, m), 5.21 (1H, d, J=7.1Hz), 5.65 (1H, d, J=8.8Hz), 5.98 (1H, d, J=5.1Hz), 6.08 (1H, t, J=8.8Hz), 6.21 (1H, d, J=9.0Hz), 7.28-7.32 (1H, m), 7.42-7.49 (3H, m), 7.60 (1H, t, J=7.4Hz), 8.12-8.15 (2H, m), 8.40 (1H, d, J=4.6Hz).

MS (FAB)m/z: 937 $(M+H)^+$.

[Example 30]

(1S,2S,3R,4S,5R,8R,9S,10R,13S)-4-(Acetoxy)-2-(benzoyloxy)-5,20-epoxy-1-hydroxy-9,10-[(1S)-2-(methoxyamino-3-ylideneazetidino)ethylidenedioxy]tax-11-en-13-yl

(2R,3S)-3-(tert-butoxycarbonylamino)-3-(3-fluoro-2-pyridyl)-2-hydroxypropionate

[Formula 102]

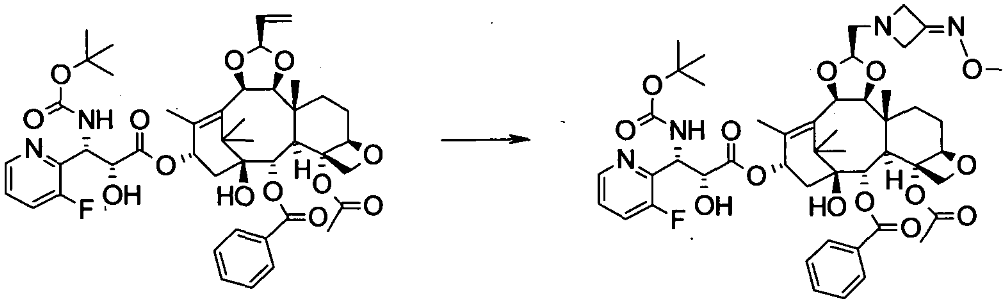

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 17, Step 2 were used as starting materials to perform the same procedure as that of Example 1 and thereby to obtain the title compound as white amorphous solid.

$^1$H-NMR (400MHz, $CDCl_3$) δ : 0.98-2.53 (7H, m), 1.29 (3H, s), 1.40 (9H, s), 1.50 (3H, s), 1.61 (3H, s), 1.79 (3H, s), 2.35

(3H, s), 2.91-2.97 (2H, m), 3.01 (1H, dd, J=15.4, 4.2Hz), 3.84 (1H, d, J=5.1Hz), 3.86 (3H, s), 4.15-4.17 (1H, m), 4.15 (4H, s), 4.23 (1H, d, J=8.3Hz), 4.33 (1H, d, J=8.3Hz), 4.68 (1H, d, J=2.4Hz), 4.89-4.93 (2H, m), 5.24 (1H, d, J=7.3Hz), 5.64 (1H, d, J=9.0Hz), 5.98 (1H, d, J=5.1Hz), 6.09 (1H, t, J=8.5Hz), 6.21 (1H, d, J=8.5Hz), 7.28-7.33 (1H, m), 7.42-7.50 (3H, m), 7.60 (1H, t, J=7.4Hz), 8.14 (2H, d, J=7.3Hz), 8.40 (1H, d, J=4.6Hz).
FAB-MS; m/z: 937 $(M+H)^+$.

[Example 31]

[Formula 103]

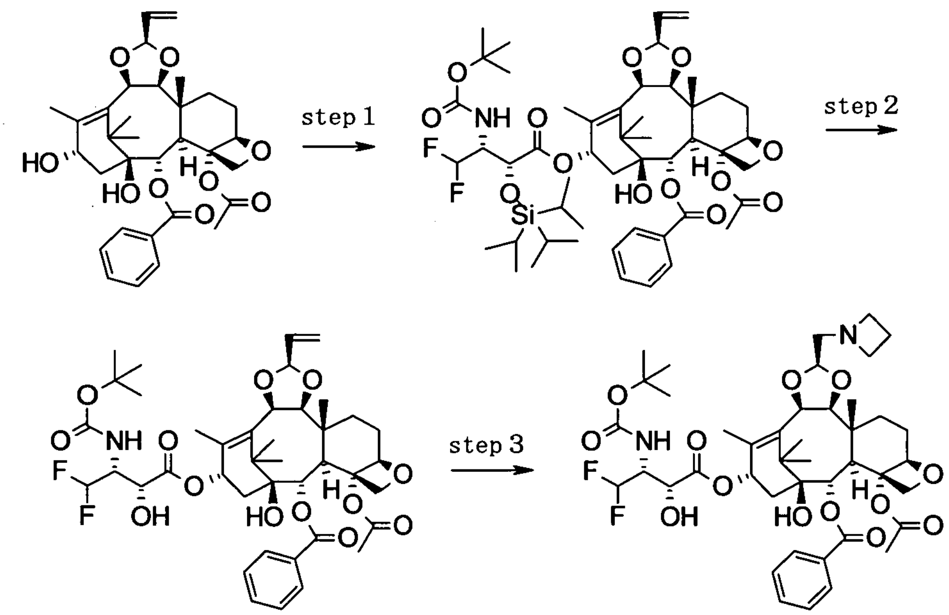


Step 1: (1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4,4-difluoro-2-[(triisopropylsilyl)oxy]butyrate

(1S,2S,3R,4S,5R,8R,9S, 10R, 13S)-4-(Acetoxy)-2-(benzoyloxy)-1,13-dihydroxy-5,20-epoxy-9,10-[(1S)-2-propenylidenedioxy]tax-11-ene (Bioorg. Med. Chem. Lett., 2003, 13, 185, 1.00 g) and (3R,4R)-1-(tert-butoxycarbonyl)-4-(difluoromethyl)-3-[(triisopropylsilyl)oxy]-2-azetidinone (Bioorg. Med. Chem., 2000, 8, 1619, 900 mg) were dissolved in tetrahydrofuran (35.0 ml) under an argon flow, and the solution was added with lithium bis(trimethylsilyl)amide (1 mole solution in tetrahydrofuran, 2.32 ml) at -78°C, and stirred at 0°C for 30 minutes. The reaction mixture was added with saturated aqueous ammonium chloride, and extracted with ethyl acetate. The organic layer was washed with saturated brine, and dried, and then the solvent was evaporated under reduced pressure. The resulting residue was purified by silica gel chromatography [developing solvent; ethyl acetate:n-hexane=1:9 to 1:4 (v/v)] to obtain the title compound (1.22 g) as white amorphous solid.
$^1$H-NMR (400MHz, $CDCl_3$) δ : 1.08-1.20 (21H, m), 1.24-2.04 (4H, m), 1.29 (3H, s), 1.38 (9H, s), 1.51 (3H, s), 1.64 (3H, s), 1.78 (3H, s), 2.23-2.30 (3H, m), 2.35 (3H, s), 2.93 (1H, d, J=4.9Hz), 4.19-4.21 (2H, m), 4.33-4.39 (2H, m), 4.87 (1H, s), 4.92 (1H, s), 5.03 (1H, d, J=10.7Hz), 5.20 (1H, d, J=6.3Hz), 5.26 (1H, d, J=6.6Hz), 5.45 (1H, d, J=10.5Hz), 5.57 (1H, d, J=17.3Hz), 5.78 (1H, dt, J=5.9, 56.2Hz), 5.95-6.04 (3H, m), 7.50 (2H, t, J=7.6Hz), 7.61 (1H, t, J=7.6Hz), 8.15 (2H, d, J=7.6Hz).
FAB-MS; m/z: 962 $(M+H)^+$, 984 $(M+Na)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4,4-difluoro-2-hydroxybutyrate

The compound obtained in Step 1 mentioned above (1.12 g) was dissolved in tetrahydrofuran (23 ml), and the solution was added with tetra-n-butylammonium fluoride (1 mole solution in tetrahydrofuran, 2.33 ml) at 0°C, and stirred for 10 minutes. The reaction mixture was added with saturated brine, and extracted with ethyl acetate. The organic layer was dried, and then the solvent was evaporated under reduced pressure. The resulting residue was purified by silica gel chromatography [developing solvent; ethyl acetate:n-hexane=1:2 (v/v)] to obtain the title compound (893 mg) as white amorphous solid.
$^1$ H-NMR (400MHz, $CDCl_3$) δ : 1.24-2.11 (6H, m), 1.25 (3H, s), 1.42 (9H, s), 1.50 (3H, s), 1.64 (3H, s), 1.79 (3H, s), 2.24-2.39 (1H, m), 2.34 (3H, s), 2.94 (1H, d, J=4.9Hz), 4.18 (1H, d, J=7.1Hz), 4.25 (1H, d, J=8.5Hz), 4.34 (1H, d, J=8.5Hz), 4.51-4.75 (3H, m), 4.94 (1H, s), 5.22-5.26 (2H, m), 5.32 (1H, d, J=6.8Hz), 5.47 (1H, d, J=10.5Hz), 5.58 (1H, d, J=17.3Hz), 5.76-6.05 (3H, m), 6.13 (1H, t, J=8.3Hz), 7.48 (2H, t, J=7.7Hz), 7.61 (1H, t, J=7.7Hz), 8.13 (2H, d, J=7.7Hz).
FAB-MS; m/z: 806 $(M+H)^+$ , 828 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4,4-difluoro-2-hydroxybutyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.23-2.18 (9H, m), 1.23 (3H, s), 1.42 (9H, s), 1.49 (3H, s), 1.61 (3H, s), 1.77 (3H, s), 2.32-2.38 (1H, m), 2.33 (3H, s), 2.76 (1H, dd, J=12.6, 5.9Hz), 2.84 (1H, dd, J=12.6, 4.3Hz), 2.92 (1H, d, J=4.9Hz), 3.36-3.40 (4H, m), 4.09-4.13 (1H, m), 4.25 (1H, d, J=8.5Hz), 4.33 (1H, d, J=8.5Hz), 4.49-4.58 (1H, m), 4.64 (1H, s), 4.87-4.90 (1H, m), 4.93 (1H, s), 5.21-5.26 (2H, m), 5.75-6.04 (2H, m), 6.11 (1H, t, J=8.5Hz), 7.48 (2H, t, J=7.6Hz), 7.61 (1H, t, J=7.6Hz), 8.13 (2H, d, J=7.6Hz).
FAB-MS; m/z: 849 $(M+H)^+$.

[Example 32]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxyazetidino)ethylidenedioxy]tax-11-en-13-yl

(2R,3R)-3-(tert-butoxycarbonylamino)-4,4-difluoro-2-hydroxybutyrate

[Formula 104]

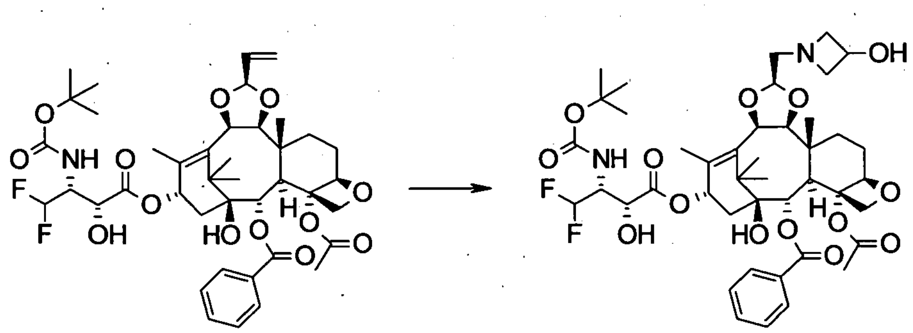

The compound obtained in Example 31, Step 2 and 3-azetidinol hydrochloride were used as starting materials to perform the same procedure as that of Example 1 and thereby to obtain the title compound as white amorphous solid.
$^1$ H-NMR (400MHz, $CDCl_3$) δ : 1.23-2.38 (10H, m), 1.24 (3H, s), 1.42 (9H, s), 1.49 (3H, s), 1.60 (3H, s), 1.77 (3H, s),

2.34 (3H, s), 2.82 (1H, dd, J=12.5, 5.4Hz), 2.90-2.94 (2H, m), 3.16-3.21 (2H, m), 3.77-3.82 (2H, m), 4.12 (1H, d, J=6.8Hz), 4.24 (1H, d, J=8.5Hz), 4.34 (1H, d, J=8.5Hz), 4.45-4.60 (2H, m), 4.65 (1H, s), 4.89-4.93 (2H, m), 5.22-5.24 (1H, m), 5.90 (1H, dt, J=55.9, 4.6Hz), 6.00 (1H, d, J=5.6Hz), 6.11 (1H, t, J=8.1Hz), 7.49 (2H, t, J=7.6Hz), 7.62 (1H, t, J=7.6Hz), 8.12-8.14 (2H, m).
FAB-MS; m/z: 865 $(M+H)^+$.

[Example 33]

(1S,2S,3R,4S,5R,8R,9S, 10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3R)-3-(tert-butoxycarbonylamino)-4,4-difluoro-2-hydroxybutyrate

[Formula 105]

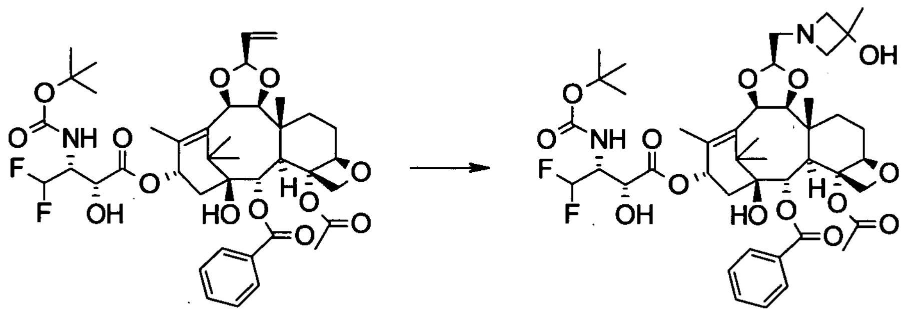

The compound obtained in Example 31, Step 2 and the compound obtained in Reference Example 3 were used as starting materials to perform the same procedure as that of Example 1 and thereby to obtain the title compound as white amorphous solid. $^1$H-NMR (400MHz, $CDCl_3$) $\delta$ : 1.21-2.06 (9H, m), 1.24 (3H, s), 1.42 (9H, s), 1.49 (3H, s), 1.52 (3H, s), 1.61 (3H, s), 1.76 (3H, s), 2.32-2.38 (1H, m), 2.34 (3H, s), 2.80 (1H, dd, J=12.0, 5.6Hz), 2.90-2.94 (2H, m), 3.20-3.24 (2H, m), 3.42-3.46 (2H, m), 4.12 (1H, d, J=7.1Hz), 4.24 (1H, d, J=8.3Hz), 4.34 (1H, d, J=8.3Hz), 4.49-4.58 (1H, m), 4.65 (1H, s), 4.90-4.93 (2H, m), 5.21-5.26 (1H, m), 5.90 (1H, dt, J=55.7, 5.6Hz), 6.00 (1H, d, J=4.9Hz), 6.11 (1H, t, J=8.8Hz), 7.49 (2H, t, J=7.6Hz), 7.62 (1H, t, J=7.6Hz), 8.12-8.14 (2H, m). FAB-MS; m/z: 879 $(M+H)^+$.

[Example 34]

[Formula 106]

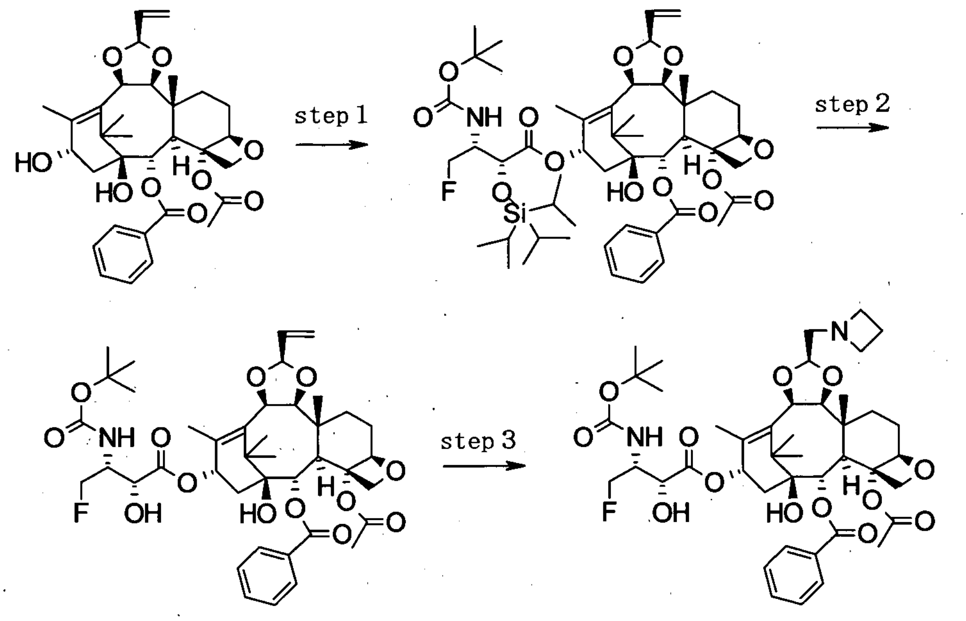


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4-fluoro-2-[(triisopropylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 18, Step 4 were used as starting materials to perform the same procedure as that of Example 31, Step 1 and thereby to obtain the title compound as white amorphous solid.
$^1$H-NMR (400MHz, $CDCl_3$) δ : 1.08-2.05 (26H, m), 1.29 (3H, s), 1.37 (9H, s), 1.51 (3H, s), 1.64 (3H, s), 1.79 (3H, s), 2.24-2.28 (2H, m), 2.33 (3H, s), 2.94 (1H, d, J=5.4Hz), 4.18-4.21 (2H, m), 4.33-4.48 (4H, m), 4.77-4.79 (2H, m), 4.92 (1H, s), 5.20 (1H, d, J=6.1Hz), 5.27 (1H, d, J=7.1Hz), 5.46 (1H, d, J=10.5Hz), 5.58 (1H, d, J=17.3Hz), 5.96-6.04 (3H, m), 7.49 (2H, t, J=7.6Hz), 7.61 (1H, t, J=7.4Hz), 8.14-8.16 (2H, m).
FAB-MS; m/z: 944 $(M+H)^+$, 966 $(M+Na)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.20-2.25 (7H, m), 1.25 (3H, s), 1.42 (9H, s), 1.50 (3H, s), 1.64 (3H, s), 1.81 (3H, s), 2.33 (3H, s), 2.35-2.42 (1H, m), 2.94 (1H, d, J=4.6Hz), 4.18 (1H, d, J=7.1Hz), 4.25 (1H, d, J=8.5Hz), 4.34 (1H, d, J=8.5Hz), 4.42-4.56 (4H, m), 4.94 (1H, s), 5.12 (1H, d, J=10.0Hz), 5.25 (1H, d, J=6.3Hz), 5.32 (1H, d, J=7.3Hz), 5.47 (1H, d, J=10.5Hz), 5.58 (1H, d, J=17.1Hz), 5.97-6.11 (3H, m), 7.48 (2H, t, J=7.6Hz), 7.59-7.63 (1H, m), 8.12-8.14 (2H, m).
FAB-MS; m/z: 788 $(M+H)^+$, 810 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4-fluoro-2-hydroxybutyrate

The compound obtained in Step 2 mentioned above was used as a starting material to perform the same

procedure as that of Example 1 and thereby to obtain the title compound as white amorphous solid.
$^1$ H-NMR (400MHz, $CDCl_3$) δ : 1.18-2.40 (10H, m), 1.24 (3H, s), 1.41 (9H, s), 1.49 (3H, s), 1.61 (3H, s), 1.78 (3H, s), 2.33 (3H, s), 2.74 (1H, dd, J=12.5, 5.6Hz), 2.83 (1H, dd, J=12.5, 4.4Hz), 2.92 (1H, d, J=4.9Hz), 3.32-3.40 (4H, m), 4.12 (1H, d, J=7.1Hz), 4.24 (1H, d, J=8.5Hz), 4.33 (1H, d, J=8.5Hz), 4.41-4.61 (4H, m), 4.86-4.89 (1H, m), 4.93 (1H, s), 5.17 (1H, d, J=9.0Hz), 5.22 (1H, d, J=6.8Hz), 6.00 (1H, d, J=5.1Hz), 6.06 (1H, t, J=8.5Hz), 7.48 (2H, t, J=7.6Hz), 7.61 (1H, t, J=7.6Hz), 8.13 (2H, d, J=7.6Hz).
FAB-MS; m/z: 831 $(M+H)^+$.

[Example 35]

(1S,2S,3R,4S,5R,8R,9S, 10R, 13S)-4-(Acetoxy)-2-(benzoyloxy)-5,20-epoxy-1-hydroxy-9,10-[(1S)-2-(3-hydroxyazetidino)ethylidenedioxy]tax-11-en-13-yl

(2R,3R)-3-(tert-butoxycarbonylamino)-4-fluoro-2-hydroxybutyrate

[Formula 107]

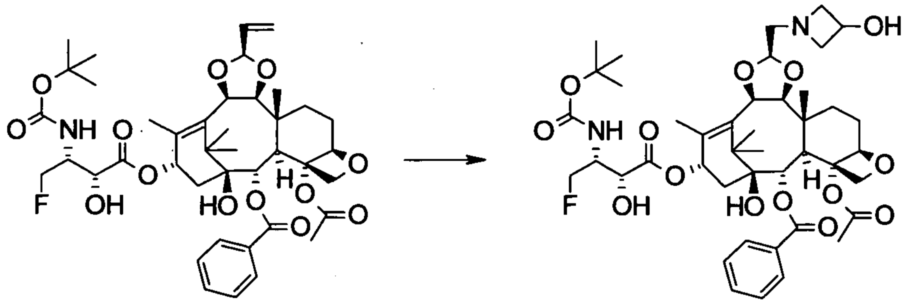

The compound obtained in Example 34, Step 2 and 3-azetidinol hydrochloride were used as starting materials to perform the same procedure as that of Example 1 and thereby to obtain the title compound as white amorphous solid.
$^1$H-NMR (400MHz, $CDCl_3$) δ : 1.23-2.40 (9H, m), 1.24 (3H, s), 1.41 (9H, s), 1.49 (3H, s), 1.61 (3H, s), 1.79 (3H, s), 2.33 (3H, s), 2.79 (1H, dd, J=12.5, 5.6Hz), 2.87-2.93 (2H, m), 3.08-3.14 (2H, m), 3.75-3.79 (2H, m), 4.12 (1H, d, J=7.1Hz), 4.24 (1H, d, J=8.5Hz), 4.32 (1H, d, J=8.5Hz), 4.41-4.55 (5H, m), 4.89 (1H, dd, J=5.4, 4.6Hz), 4.93 (1H, s), 5.14 (1H, d, J=9.0Hz), 5.23 (1H, d, J=7.1Hz), 6.00 (1H, d, J=5.1Hz), 6.07 (1H, t, J=8.3Hz), 7.48 (2H, t, J=7.7Hz), 7.59-7.63 (1H, m), 8.11-8.14 (2H, m).
FAB-MS; m/z: 847 $(M+H)^+$.

[Example 36]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3R)-3-(tert-butoxycarbonylamino)-4-fluoro-2-hydroxybutyrate

[Formula 108]

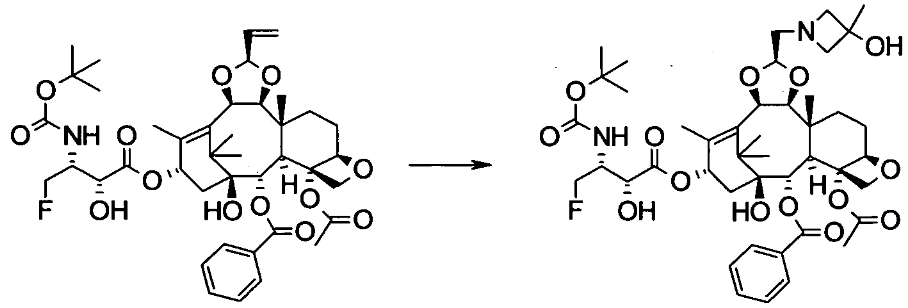

The compound obtained in Example 34, Step 2 and the compound obtained in Reference Example 3 were used as starting materials to perform the same procedure as that of Example 1 and thereby to obtain the title compound as white amorphous solid. $^1$H-NMR (400MHz, $CDCl_3$) δ : 1.23-2.05 (11H, m), 1.24 (3H, s), 1.41 (9H, s), 1.49 (3H, s), 1.60 (3H, s), 1.78 (3H, s), 2.32-2.40 (1H, m), 2.33 (3H, s), 2.79 (1H, dd, J=12.5, 5.6Hz), 2.89-2.93 (2H, m), 3.22 (2H, t, J=8.1Hz), 3.43 (2H, t, J=8.1Hz), 4.12 (1H, d, J=7.1Hz), 4.24 (1H, d, J=8.5Hz), 4.33 (1H, d, J=8.5Hz), 4.41-4.64 (4H, m), 4.91 (1H, dd, J=5.6, 4.6Hz), 4.93 (1H, s), 5.15 (1H, d, J=9.3Hz), 5.23 (1H, d, J=6.8Hz), 6.00 (1H, d, J=5.1Hz), 6.07 (1H, t, J=8.1Hz), 7.48 (2H, t, J=7.8Hz), 7.59-7.63 (1H, m), 8.12-8.14 (2H, m).
FAB-MS; m/z: 861 $(M+H)^+$.

[Example 37]

[Formula 109]

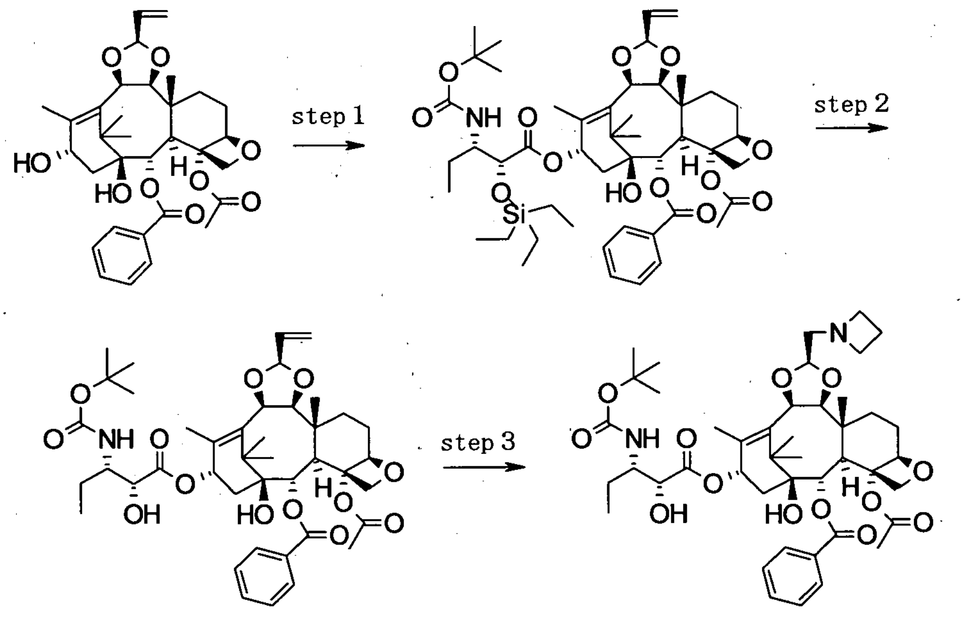


Step 1: (1S,2S,3R,4S,5R,8R,9S,10R,13S)-4-(Acetoxy)-2-(benzoyloxy)-5,20-epoxy-1-hydroxy-9,10-[(1S)-(2-propenylidenedioxy)]tax-11-en-13-yl

(2R,3S)-3-(tert-butoxycarbonylamino)-2-[(triethylsilyl)oxy]pentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 19, Step 3 were used as starting materials

to perform the same procedure as that of Example 31, Step 1 and thereby to obtain the title compound as white amorphous solid.
$^1$H-NMR (400MHz, $CDCl_3$) δ : 0.59-0.72 (6H, m), 0.95-2.38 (21H, m), 1.30 (3H, s), 1.37 (9H, s), 1.51 (3H, s), 1.64 (3H, s), 1.78 (3H, s), 2.36 (3H, s), 2.93 (1H, d, J=5.1Hz), 3.91-3.99 (1H, m), 4.19-4.22 (2H, m), 4.29-4.36 (2H, m), 4.78 (1H, d, J=10.3Hz), 4.94 (1H, s), 5.21 (1H, d, J=6.1Hz), 5.27 (1H, d, J=6.8Hz), 5.45 (1H, d, J=10.0Hz), 5.58 (1H, d, J=17.3Hz), 5.95-6.06 (2H, m), 7.49 (2H, t, J=7.7Hz), 7.61 (1H, t, J=7.3Hz), 8.13-8.16 (2H, m).

Step 2: (1S,2S,3R,4S,5R,8R,9S,10R,13S)-4-(Acetoxy)-2-(benzoyloxy)-5,20-epoxy-1-hydroxy-9,10-[(1S)-(2-propenylidenedioxy)]tax-11-en-13-yl

(2R,3S)-3-(tert-butoxycarbonylamino)-2-hydroxypentanoate

The compound obtained in Step 1 mentioned above was used as a starting material to perform the same procedure as that of Example 31, Step 2 and thereby to obtain the title compound as white amorphous solid. $^1$H-NMR (400MHz, $CDCl_3$) δ : 0.90-2.42 (12H, m), 1.26 (3H, s), 1.40 (9H, s), 1.50 (3H, s), 1.64 (3H, s), 1.81 (3H, s), 2.33 (3H, s), 2.94 (1H, d, J=5.1Hz), 3.98-4.07 (2H, m), 4.19 (1H, d, J=6.8Hz), 4.24 (1H, d, J=8.3Hz), 4.32-4.35 (2H, m), 4.83 (1H, d, J=9.8Hz), 4.93-5.00 (1H, m), 5.25 (1H, d, J=6.1Hz), 5.31 (1H, d, J=7.3Hz), 5.47 (1H, d, J=10.3Hz), 5.58 (1H, d, J=17.3Hz), 5.96-6.10 (3H, m), 7.48 (2H, t, J=7.7Hz), 7.61 (1H, t, J=7.4Hz), 8.12-8.15 (2H, m).
FAB-MS; m/z: 944 $(M+H)^+$ , 966 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxy-tax-11-en-13-yl

(2R,3S)-3-(tert-butoxycarbonylamino)-2-hydroxypentanoate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 0.97-2.42 (16H, m), 1.00 (3H, t, J=7.3Hz), 1.24 (3H, s), 1.39 (9H, s), 1.49 (3H, s), 1.62 (3H, d, J=9.3Hz), 1.78 (3H, s), 2.33 (3H, s), 2.75 (1H, dd, J=12.5, 5.6Hz), 2.83 (1H, dd, J=12.5, 4.4Hz), 2.92 (1H, d, J=4.9Hz), 3.32-3.41 (1H, m), 3.98-4.05 (1H, m), 4.12 (1H, d, J=7.1Hz), 4.24 (1H, d, J=8.3Hz), 4.31-4.35 (2H, m), 4.83-4.89 (1H, m), 4.93 (1H, s), 5.22 (1H, d, J=6.8Hz), 6.00 (1H, d, J=5.1Hz), 6.05 (1H, t, J=8.3Hz), 7.48 (2H, t, J=7.8Hz), 7.61 (1H, t, J=7.4Hz), 8.11-8.15 (2H, m).
FAB-MS; m/z: 827 $(M+H)^+$.

[Example 38]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxyazetidino)ethylidenedioxy]tax-11-en-13-yl

(2R,3S)-3-(tert-butoxycarbonylamino)-2-hydroxypentanoate

[Formula 110]

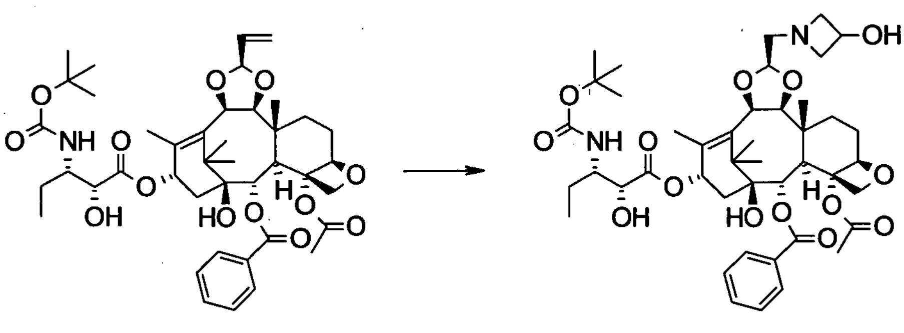

The compound obtained in Example 37, Step 2 and 3-azetidinol hydrochloride were used as starting materials

to perform the same procedure as that of Example 1 and thereby to obtain the title compound as white amorphous solid.
$^1$H-NMR (400MHz, $CDCl_3$) δ : 1.01 (3H, t, J=7.3Hz), 1.17-2.41 (10H, m), 1.25 (3H, s), 1.39 (9H, s), 1.49 (3H, s), 1.61 (3H, s), 1.78 (3H, s), 2.33 (3H, s), 2.79 (1H, dd, J=12.6, 5.2Hz), 2.86-2.93 (2H, m), 3.07-3.13 (2H, m), 3.75-3.82 (2H, m), 3.97-4.05 (1H, m), 4.13 (1H, d, J=7.1Hz), 4.24 (1H, d, J=8.3Hz), 4.31-4.35 (2H, m), 4.45-4.52 (2H, m), 4.83-4.90 (2H, m), 4.93 (1H, s), 5.22 (1H, d, J=6.8Hz), 5.99 (1H, d, J=5.1Hz), 6.06 (1H, t, J=8.5Hz), 7.48 (2H, t, J=7.7Hz), 7.61 (1H, t, J=7.4Hz), 8.11-8.15 (2H, m).
FAB-MS; m/z: 843 $(M+H)^+$.

[Example 39]

(1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3S)-3-(tert-butoxycarbonylamino)-2-hydroxypentanoate

[Formula 111]

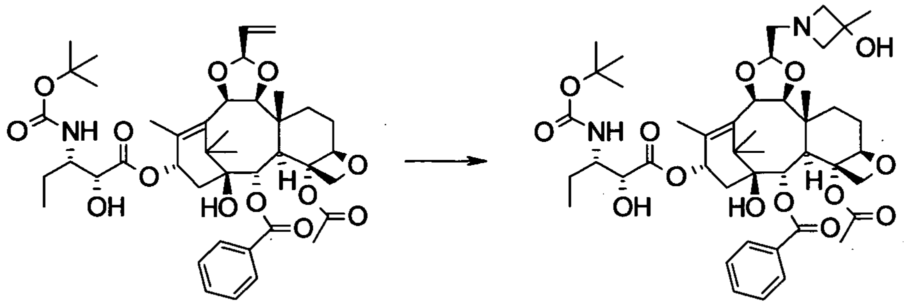

The compound obtained in Example 37, Step 2 and the compound obtained in Reference Example 3 were used as starting materials to perform the same procedure as that of Example 1 and thereby to obtain the title compound as white amorphous solid.
$^1$H-NMR (400MHz, $CDCl_3$) δ : 1.01 (3H, t, J=7.4Hz), 1.17-2.42 (10H, m), 1.25 (3H, s), 1.39 (9H, s), 1.49 (3H, s), 1.51 (3H, s), 1.60 (3H, s), 1.78 (3H, s), 2.33 (3H, s), 2.80 (1H, dd, J=12.5, 5.9Hz), 2.90-2.95 (2H, m), 3.25 (2H, t, J=8.5Hz), 3.45 (2H, t, J=8.3Hz), 3.98-4.05 (1H, m), 4.09-4.15 (2H, m), 4.24 (1H, d, J=8.3Hz), 4.31-4.35 (2H, m), 4.83-4.95 (3H, m), 5.22 (1H, d, J=6.8Hz), 5.99 (1H, d, J=5.1Hz), 6.06 (1H, t, J=8.3Hz), 7.48 (2H, t, J=7.7Hz), 7.61 (1H, t, J=7.4Hz), 8.11-8.15 (2H, m).
FAB-MS; m/z: 857 $(M+H)^+$ .

[Example 40]

(1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-2-hydroxy-3-(2-thiazolyl)propionate

[Formula 112]

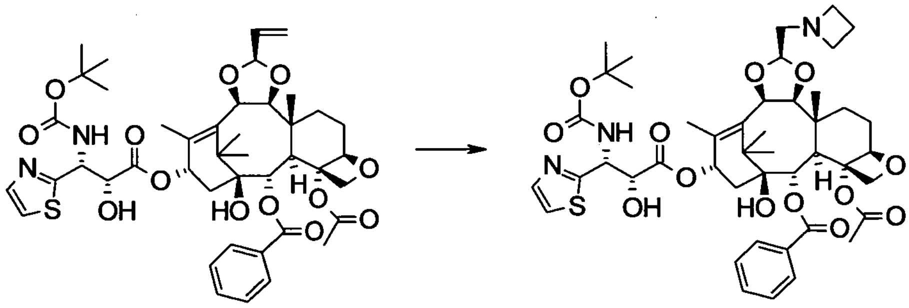

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-2-hydroxy-3-(2-thiazolyl)propionate (Bioorg. Med. Chem. Lett., 2004, 14, 3209) was used as a starting material to perform the same procedure as that of Example 1 and thereby to obtain the title compound as white amorphous solid.
$^1$H-NMR (400MHz, $CDCl_3$) δ: 1.24-2.16 (9H, m), 1.24 (3H, s), 1.45 (9H, s), 1.49 (3H, s), 1.61 (3H, s), 1.73 (3H, s), 2.30-2.36 (1H, m), 2.35 (3H, s), 2.74 (1H, dd, J=12.5, 5.9Hz), 2.82 (1H, dd, J=12.5, 4.4Hz), 2.92 (1H, d, J=5.1Hz), 3.31-3.40 (4H, m), 4.11 (1H, d, J=7.3Hz), 4.22 (1H, d, J=8.3Hz), 4.33 (1H, d, J=8.3Hz), 4.87 (1H, dd, J=5.9, 4.4Hz), 4.92 (1H, s), 5.00 (1H, d, J=2.2Hz), 5.21 (1H, d, J=7.1Hz), 5.60 (1H, d, J=9.8Hz), 5.95 (1H, d, J=9.8Hz), 5.99 (1H, d, J=5.1Hz), 6.12 (1H, t, J=8.2Hz), 7.31 (1H, d, J=3.2Hz), 7.47 (2H, t, J=7.5Hz), 7.60 (1H, t, J=7.5Hz), 7.76 (1H, d, J=3.2Hz), 8.12-8.14 (2H, m). FAB-MS; m/z: 882 $(M+H)^+$.

[Example 41]

[Formula 113]

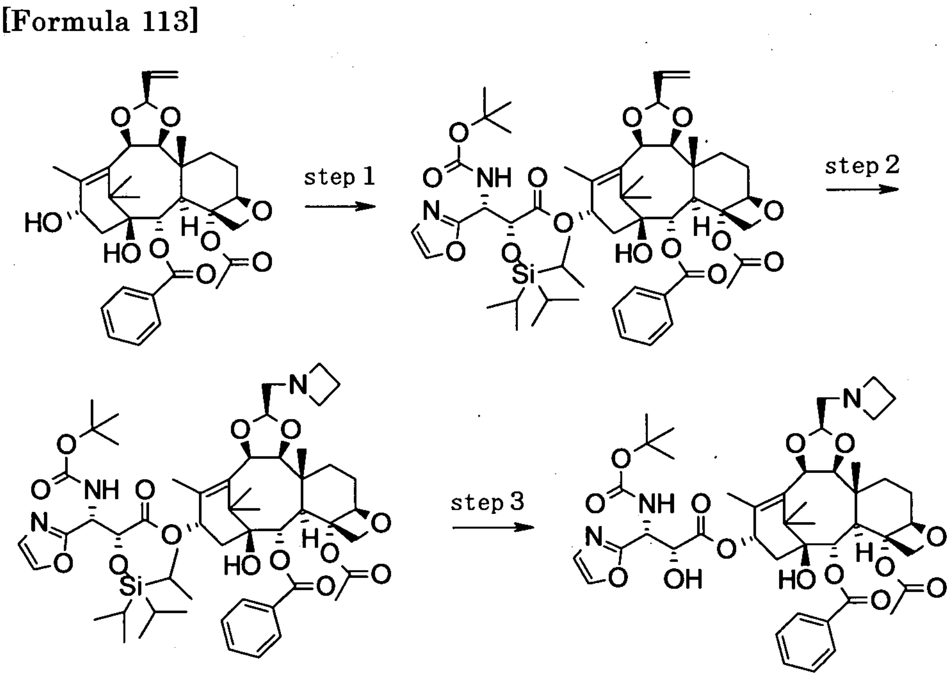

Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-3-(2-oxazolyl)-2-[(triisopropylsilyl)oxy]propionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 20, Step 4 were used as starting materials to perform the same procedure as that of Example 31, Step 1 and thereby to obtain the title compound as white amorphous solid.
$^1$ H-NMR (400MHz, $CDCl_3$) δ : 0.91-1.06 (21H, m), 1.31 (3H, s), 1.35-1.96 (5H, m), 1.40 (9H, s), 1.52 (3H, s), 1.65 (3H, s), 1.81 (3H, s), 2.02-2.39 (2H, m), 2.47 (3H, s), 2.97 (1H, d, J=5.9Hz), 4.21 (2H, d, J=7.6Hz), 4.34 (1H, d, J=8.5Hz), 4.95 (1H, brs), 5.09 (1H, d, J=1.7Hz), 5.21 (1H, d, J=6.6Hz), 5.28 (1H, d, J=7.1Hz), 5.46 (1H, d, J=10.3Hz), 5.47 (1H, s), 5.53 (1H, s), 5.58 (1H, d, J=17.8Hz), 5.96-6.05 (2H, m), 6.08 (1H, t, J=8.3Hz), 7.11 (1H, s), 7.47 (2H, t, J=7.3Hz), 7.59 (1H, t, J=7.8Hz), 7.65 (1H, s), 8.15 (1H, d, J=7.8Hz).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-3-(2-oxazolyl)-2-[(triisopropylsilyl)oxy]propionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.91-1.06 (21H, m), 1.29 (3H, s), 1.40 (9H, s), 1.51 (3H, s), 1.58-2.19 (7H, m), 1.62 (3H, s), 1.78 (3H, s), 2.24-2.37 (2H, m), 2.46 (3H, s), 2.74 (1H, dd, J=12.5, 5.9Hz), 2.81 (1H, dd, J=12.5, 4.4Hz), 2.95 (1H, d, J=5.4Hz), 3.31-3.40 (4H, m), 4.14 (1H, d, J=7.1Hz), 4.21 (1H, d, J=8.5Hz), 4.34 (1H, d, J=8.5Hz), 4.84 (1H, t, J=5.4Hz), 4.95 (1H, s), 5.09 (1H, s), 5.19 (1H, d, J=7.3Hz), 5.45 (1H, d, J=10.3Hz), 5.54 (1H, d, J=10.5Hz), 5.98 (1H, d, J=4.9Hz), 6.07 (1H, t, J=9.0Hz), 7.11 (1H, s), 7.47 (2H, t, J=7.8Hz), 7.59 (1H, t, J=7.8Hz), 7.65 (1H, s), 8.15 (2H, d, J=8.1Hz).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-2-hydroxy-3-(2-oxazolyl)propionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.25 (3H, s), 1.43 (9H, s), 1.49 (3H, s), 1.61 (3H, s), 1.79 (3H, s), 1.82-2.21 (8H, m), 2.26-2.43 (2H, m), 2.35 (3H, s), 2.75 (1H, dd, J=12.2, 5.6Hz), 2.83 (1H, dd, J=12.5, 4.4Hz), 2.94 (1H, d, J=4.6Hz), 3.32-3.41 (4H, m), 4.12 (1H, d, J=7.1Hz), 4.24 (1H, d, J=8.5Hz), 4.33 (1H, d, J=8.3Hz), 4.84 (1H, d, J=2.0Hz), 4.88 (1H, t, J=4.9Hz), 4.92 (1H, br s), 5.22 (1H, d, J=7.1Hz), 5.50 (1H, d, J=9.0Hz), 5.71 (1H, d, J=9.5Hz), 6.00 (1H, d, J=4.9Hz), 6.13 (1H, t, J=7.8Hz), 7.11 (1H, s), 7.48 (2H, t, J=7.7Hz), 7.61 (1H, t, J=7.6Hz), 7.67 (1H, s), 8.13 (2H, d, J=8.1Hz).
FAB-MS; m/z: 866 $(M+H)^+$.

[Example 42]

[Formula 114]

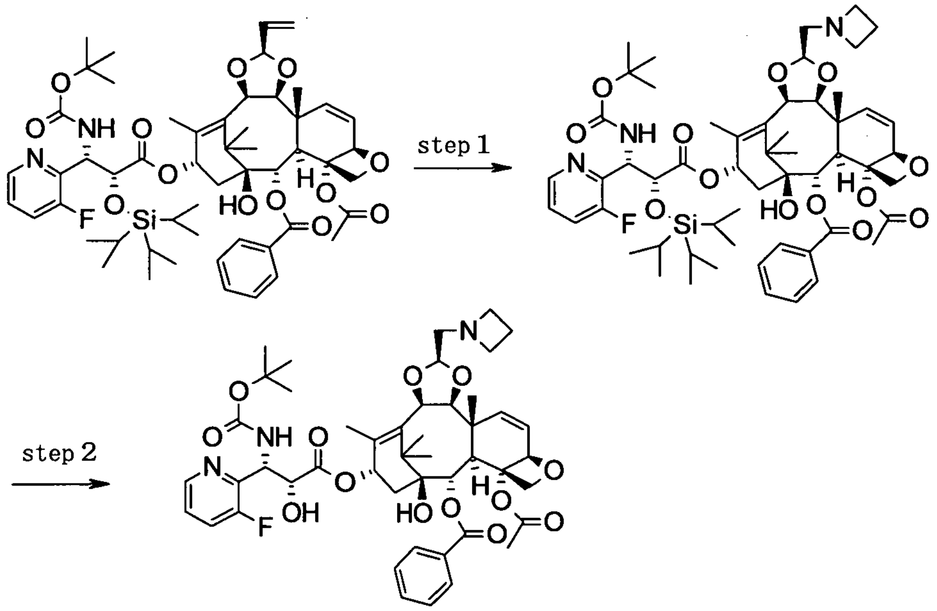


Step 1: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-6,11-dien-13-yl

(2R,3S)-3-(tert-butoxycarbonylamino)-3-(3-fluoro-2-pyridyl)-2-[(triisopropylsilyl)oxy]propionate

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-6,11-dien-13-yl

(2R, 3S)- 3-(tert- butoxycarbonylamino)- 3-(3- fluoro- 2- pyridyl)- 2-[(triisopropylsilyl) oxy] propionate (WO01/027115) was used as a starting material to perform the same procedure as that of Example 1 and thereby to obtain the title compound as white amorphous solid.
$^1$H-NMR (400MHz, $CDCl_3$) δ : 0.85-0.92 (21H, m), 1.26-2.17 (3H, m), 1.31 (3H, s), 1.38 (9H, s), 1.55 (3H, s), 1.56 (3H, s), 1.74 (3H, s), 2.39-2.48 (2H, m), 2.51 (3H, s), 2.77 (1H, dd, J=12.5, 5.4Hz), 2.84 (1H, dd, J=12.5, 4.6Hz), 3.13 (1H, d, J=5.9Hz), 3.37 (4H, t, J=7.1Hz), 4.00 (1H, d, J=7.8Hz), 4.32 (2H, s), 4.83 (1H, t, J=5.0Hz), 4.90 (1H, d, J=4.4Hz), 4.94 (1H, s), 5.11 (1H, d, J=7.8Hz), 5.62 (1H, d, J=10.0Hz), 5.68 (1H, dd, J=10.5, 4.4Hz), 5.95 (1H, d, J=5.9Hz), 6.08-6.11 (3H, m), 7.26-7.29 (1H, m), 7.41 (1H, t, J=9.3Hz), 7.49 (2H, t, J=7.6Hz), 7.57-7.61 (1H, m), 8.17-8.19 (2H, m), 8.40 (1H, d, J=4.6Hz).
FAB-MS; m/z: 1048 $(M+H)^+$.

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-6,11-dien-13-yl

(2R,3S)-3-(tert-butoxycarbonylamino)-3-(3-fluoro-2-pyridyl)-2-hydroxypropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.26-1.85 (2H, m), 1.27 (3H, s), 1.39 (9H, s), 1.53 (3H, s), 1.57 (3H, s), 1.72 (3H, s), 2.10-2.17 (2H, m), 2.28-2.50 (2H, m), 2.41 (3H, s), 2.78 (1H, dd, J=12.3, 5.5Hz), 2.85 (1H, dd, J=12.5, 4.6Hz), 3.10 (1H, d, J=5.9Hz), 3.38 (4H, t, J=7.1Hz), 3.97 (1H, d, J=7.8Hz), 4.27 (1H, d, J=8.3Hz), 4.33 (1H, d, J=8.3Hz), 4.66 (1H, d, J=2.4Hz), 4.85-4.88 (2H, m), 5.13 (1H, d, J=7.6Hz), 5.63 (1H, d, J=8.8Hz), 5.68 (1H, dd, J=10.3, 4.2Hz), 5.96 (1H, d, J=5.9Hz), 6.07-6.12 (2H, m), 6.21 (1H, d, J=8.3Hz), 7.29-7.33 (1H, m), 7.43-7.51 (3H, m), 7.60 (1H, t, J=7.4Hz), 8.14-8.17 (2H, m), 8.40 (1H, d, J=4.6Hz).
FAB-MS; m/z: 892 $(M+H)^+$.

[Example 43]

[Formula 115]

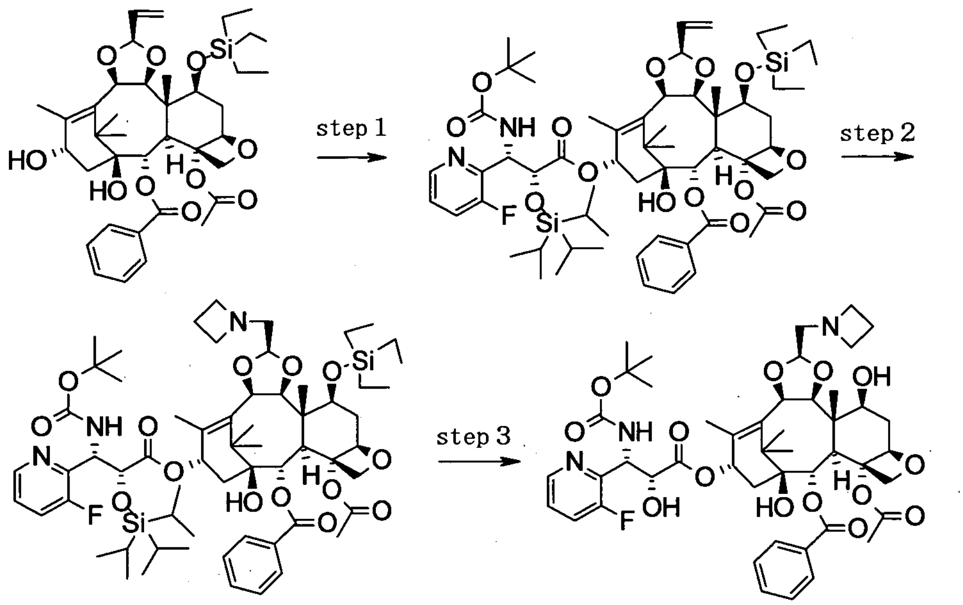


Step 1: (1S,2S,3R,4S,5R,7S,8R,9S,10R,13S)-4-(Acetoxy)-2-(benzoyloxy)-5,20-epoxy-1-hydroxy-9,10-[(1S)-2-propenylidenedioxy]-7-[(triethylsilyl)oxy]tax-11-en-13-yl

(2R,3S)-3-(tert-butoxycarbonylamino)-3-(3-fluoro-2-pyridyl)-2-[(triisopropylsilyl)oxy]propionate

(1S,2S,3R,4S,5R,7S,8R,9S,10R,13S)-4-(Acetoxy)-2-(benzoyloxy)-1,13-dihydroxy-5,20-epoxy-9,10-[(1S)-2-propenylidenedioxy]-7-[(triethylsilyl)oxy]tax-11-ene (Bioorg. Med. Chem. Lett., 2002, 12, 1083) and (3R,4S)-1-(tert-butoxycarbonyl)-4-(3-fluoro-2-pyridyl)-3-[(triisopropylsilyl)oxy]-2-azetidinone (Bioorg. Med. Chem. Lett., 2004, 14, 3209) were used as starting materials to perform the same procedure as that of Example 31, Step 1 and thereby to obtain the title compound as white amorphous solid.
$^1$H-NMR (400MHz, $CDCl_3$) δ : = 0.60-0.68 (6H, m), 0.86-0.98 (21H, m), 0.99 (9H, t, J=7.8Hz), 1.26 (3H, s), 1.37 (9H, s), 1.56 (3H, s), 1.57 (3H, s), 1.77 (1H, s), 1.78 (3H, s), 2.04-2.16 (2H, m), 2.30-2.44 (2H, m), 2.48 (3H, s), 3.24 (1H, d, J=5.6Hz), 3.94 (1H, dd, J=10.7, 5.4Hz), 4.35 (1H, d, J=8.3Hz), 4.43 (1H, d, J=8.1Hz), 4.60 (1H, d, J=8.8Hz), 4.85 (1H, dd, J=9.0, 4.9Hz), 4.94 (1H, d, J=2.0Hz), 5.06 (1H, d, J=5.9Hz), 5.29 (1H, d, J=9.0Hz), 5.45 (1H, d, J=10.5Hz), 5.56 (1H, d, J=17.6Hz), 5.61 (1H, d, J=10.3Hz), 5.92 (1H, d, J=5.6Hz), 6.04 (1H, d, J=9.8Hz), 6.07-6.18 (2H, m), 7.25-7.30 (1H, m), 7.40 (1H, t, J=9.8Hz), 7.47 (2H, t, J=7.6Hz), 7.57 (1H, t, J=7.3Hz), 8.12 (2H, d, J=7.1Hz), 8.39 (1H; d, J=4.6Hz).
FAB-MS; m/z: 1137 $(M+H)^+$.

Step 2: (1S,2S,3R,4S,5R,7S,8R,9S,10R,13S)-4-(Acetoxy)-9,10-[(1S)-2-(azetidino)ethylidenedioxy]-2-(benzoyloxy)-5,20-epoxy-1-hydroxy-7-[(triethylsilyl)oxy]tax-11-en-13-yl

(2R,3S)-3-(tert-butoxycarbonylamino)-3-(3-fluoro-2-pyridyl)-2-[(triisopropylsilyl)oxy]propionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.63 (6H, q , J=8.5Hz), 0.88-0.93 (21H, m), 0.98 (9H, t, J=7.8Hz), 1.25 (3H, s), 1.37 (9H, s), 1.51 (3H, s), 1.54 (3H, s), 1.76 (3H, s), 1.77 (1H, s), 2.03-2.19 (4H, m), 2.28-2.40 (2H, m), 2.48 (3H, s), 2.85 (2H, d, J=4.9Hz), 3.22 (1H, d, J=5.6Hz), 3.33 (4H, t, J=7.0Hz), 3.91 (1H, dd, J=10.3, 4.9Hz), 4.34 (1H, d, J=8.3Hz), 4.45 (2H,

d, J=7.8Hz), 4.68 (1H, t, J=5.1Hz), 4.84 (1H, dd, J=9.0, 5.1Hz), 4.93 (1H, d, J=2.0Hz), 5.19 (1H, d, J=8.8Hz), 5.61 (1H, d, J=10.3Hz), 5.89 (1H, d, J=5.6Hz), 6.05 (1H, d, J=10.0Hz), 6.11 (1H, t, J=9.3Hz), 7.25-7.30 (1H, m), 7.40 (1H, t, J=8.3Hz), 7.47 (2H, t, J=7.7Hz), 7.57 (1H, t, J=7.6Hz), 8.12 (2H, d, J=7.1Hz), 8.40 (1H, d, J=4.4Hz).
FAB-MS; m/z: 1180 $(M+H)^+$.

Step 3: (1S,2S,3R,4S,5R,7S,8S,9S,10R,13S)-4-(Acetoxy)-9,10-[(1S)-2-(azetidino)ethylidenedioxy]-2-(benzoyloxy)-1,7-dihydroxy-5,20-epoxytax-11-en-13-yl

(2R,3S)-3-(tert-butoxycarbonylamino)-3-(3-fluoro-2-pyridyl)-2-hydroxypropionate

The compound obtained in Step 2 mentioned above (0.55 g) was dissolved in pyridine (2 ml), and the solution was added with hydrogen fluoride/pyridine (1 ml) at 0°C, and stirred at room temperature for 22.5 hours. The reaction mixture was poured into ice water, neutralized with sodium hydrogencarbonate, and then extracted with ethyl acetate. The organic layer was dried, and then the solvent was evaporated under reduced pressure. The resulting residue was purified by silica gel chromatography [developing solvent; chloroform:methanol=50:1 (v/v)] to obtain the title compound (0.24 g) as white amorphous solid.
$^1$H-NMR (400MHz, $CDCl_3$) δ : 1.30 (3H, s), 1.40 (9H, s), 1.63 (3H, s), 1.67 (3H, s), 1.74 (3H, s), 1.90 (1H, s), 2.06-2.28 (6H, m), 2.38 (3H, s), 2.40-2.50 (1H, m), 2.77 (1H, dd, J=12.7, 5.6Hz), 2.83 (1H, dd, J=12.7, 4.4Hz), 2.94 (1H, d, J=4.9Hz), 3.31-3.41 (4H, m), 3.83 (1H, d, J=7.3Hz), 4.07 (1H, br s), 4.32 (1H, d, J=9.0Hz), 4.39 (1H, d, J=8.5Hz), 4.61-4.72 (2H, m), 4.85 (1H, t, J=4.6Hz), 5.10 (1H, s), 5.18 (1H, d, J=7.3Hz), 5.62 (1H, d, J=8.5Hz), 6.04 (1H, d, J=4.4Hz), 6.10 (1H, t, J=8.5Hz), 6.21 (1H, d, J=8.5Hz), 7.29-7.34 (1H, m), 7.42-7.51 (3H, m), 7.60 (1H, t, J=7.6Hz), 8.13 (2H, d, J=8.3Hz), 8.40 (1H, d, J=3.4Hz).
FAB-MS; m/z: 910 $(M+H)^+$.

[Example 44]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-4,4-difluoro-2-hydroxybutyrate

[Formula 116]

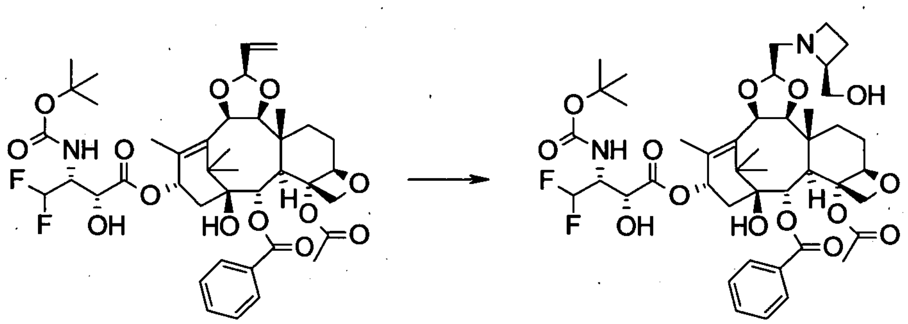

The compound obtained in Example 31, Step 2 and the compound obtained in Reference Example 25 were used as starting materials to perform the same procedure as that of Example 1 and thereby to obtain the title compound as white amorphous solid.
$^1$ H-NMR (400MHz, $CDCl_3$) δ : 1.22-2.05 (8H, m), 1.23 (3H, s), 1.42 (9H, s), 1.49 (3H, s), 1.58 (3H, s), 1.77 (3H, s), 2.21-2.38 (3H, m), 2.34 (3H, s), 2.84 (1H, dd, J=12.7, 4.6Hz), 2.89-2.94 (2H, m), 3.12 (1H, q , J=8.2Hz), 3.44 (1H, dd, J=11.5, 2.7Hz), 3.48-3.54 (2H, m), 3.66 (1H, dd, J=11.5, 2.9Hz), 4.13 (1H, d, J=7.1Hz), 4.24 (1H, d, J=8.3Hz), 4.33 (1H, d, J=8.3Hz), 4.48-4.58 (1H, m), 4.64 (1H, s), 4.90 (1H, t, J=4.9Hz), 4.93 (1H, s), 5.23-5.27 (2H, m), 5.89 (1H, td, J=55.7, 5.4Hz), 5.99 (1H, d, J=5.4Hz), 6.10 (1H, t, J=8.3Hz), 7.48 (2H, t, J=7.7Hz), 7.59-7.64 (1H, m), 8.11-8.14 (2H, m).
FAB-MS; m/z: 879 $(M+H)^+$.

[Example 45]

[Formula 117]

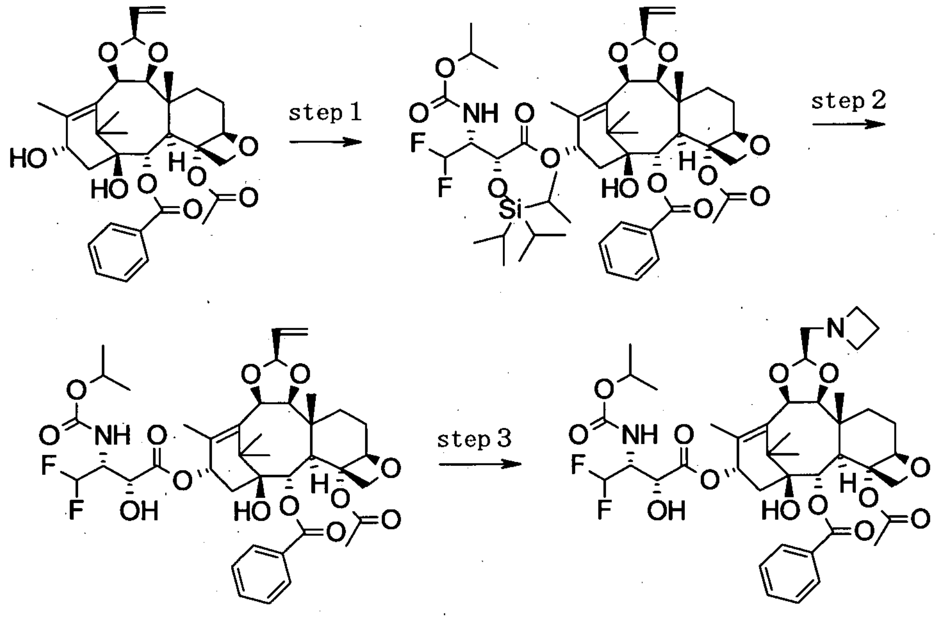


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-4,4-difluoro-3-(isopropoxycarbonylamino)-2-[(triisopropylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 21 were used as starting materials to perform the same procedure as that of Example 31, Step 1 and thereby to obtain the title compound as white amorphous solid.
$^1$H-NMR (400MHz, $CDCl_3$) δ : 1.08-2.10 (32H, m), 1.30 (3H, s), 1.51 (3H, s), 1.64 (3H, s), 1.78 (3H, s), 2.23 (2H, d, J=8.8Hz), 2.36 (3H, s), 2.93 (1H, d, J=5.1Hz), 4.20 (1H, d, J=7.1Hz), 4.21 (1H, d, J=8.3Hz), 4.33-4.43 (2H, m), 4.79-4.92 (3H, m), 5.10 (1H, d, J=10.5Hz), 5.20 (1H, d, J=6.1Hz), 5.26 (1H, d, J=6.8Hz), 5.45 (1H, d, J=10.5Hz), 5.57 (1H, d, J=17.3Hz), 5.66-6.07 (4H, m), 7.50 (2H, t, J=7.7Hz), 7.59-7.63 (1H, m), 8.14-8.16 (2H, m).
FAB-MS; m/z: 948 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-4,4-difluoro-2-hydroxy-3-(isopropoxycarbonylamino)butyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.19-1.25 (6H, m), 1.25 (3H, s), 1.47-2.11 (6H, m), 1.49 (3H, s), 1.64 (3H, s), 1.77 (3H, s), 2.32-2.38 (1H, m), 2.34 (3H, s), 2.93 (1H, d, J=4.9Hz), 4.17 (1H, d, J=7.1Hz), 4.26 (1H, d, J=8.5Hz), 4.34 (1H, d, J=8.5Hz), 4.60-4.66 (2H, m), 4.85-4.94 (3H, m), 5.25 (1H, d, J=6.3Hz), 5.30-5.34 (2H, m), 5.47 (1H, d, J=10.5Hz), 5.58 (1H, d, J=17.3Hz), 5.77-6.05 (3H, m), 6.15 (1H, t, J=8.1Hz), 7.48 (2H, t, J=7.7Hz), 7.59-7.64 (1H, m), 8.12-8.14 (2H, m).
FAB-MS; m/z: 792 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-4,4-difluoro-2-hydroxy-3-(isopropoxycarbonylamino)butyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.21 (6H, dd, J=16.8, 6.3Hz), 1.23 (3H, s), 1.47-2.16 (9H, m), 1.49 (3H, s), 1.61 (3H, s), 1.75 (3H, s), 2.30-2.36 (1H, m), 2.33 (3H, s), 2.74 (1H, dd, J=12.6, 5.6Hz), 2.83 (1H, dd, J=12.6, 4.4Hz), 2.91 (1H, d, J=4.9Hz), 3.32-3.40 (4H, m), 4.10 (1H, d, J=7.1Hz), 4.25 (1H, d, J=8.3Hz), 4.33 (1H, d, J=8.3Hz), 4.55-4.65 (2H, m), 4.86-4.93 (3H, m), 5.21 (1H, d, J=6.8Hz), 5.40 (1H, d, J=10.0Hz), 5.91 (1H, td, J=55.7, 5.1Hz), 6.01 (1H, d, J=5.1Hz), 6.13 (1H, t, J=8.4Hz), 7.48 (2H, t, J=7.7Hz), 7.59-7.63 (1H, m), 8.12-8.14 (2H, m).
FAB-MS; m/z: 835 $(M+H)^+$.

[Example 46]

[Formula 118]

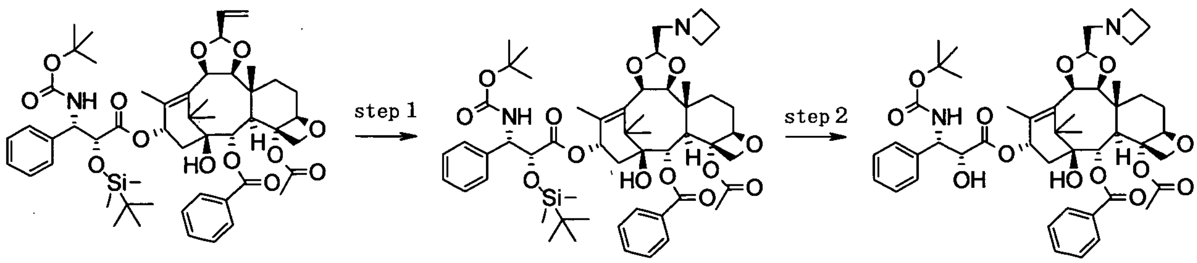


Step 1: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-[(tert-butyldimethylsilyl)oxy]-3-phenylpropionate

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-[(tert-butyldimethylsilyl)oxy]-3-phenylpropionate (WO96/33998) was used as a starting material to perform the same procedure as that of Example 1 and thereby to obtain the title compound as white amorphous solid.
$^1$H-NMR (400MHz, $CDCl_3$) δ : -0.34 (3H, s), -0.11 (3H, s), 0.74 (9H, s), 1.33 (3H, s), 1.39 (9H, s), 1.46-2.33 (9H, m), 1.52 (3H, s), 1.62 (3H, s), 1.76 (3H, s), 2.51 (3H, s), 2.74 (1H, dd, J=12.2, 5.6Hz), 2.81 (1H, dd, J=12.2, 4.6Hz), 2.94 (1H, d, J=5.4Hz), 3.32-3.39 (4H, m), 4.14 (1H, d, J=7.3Hz), 4.24 (1H, d, J=8.5Hz), 4.34 (1H, d, J=8.3Hz), 4.57 (1H, s), 4.84 (1H, t, J=5.4Hz), 4.97 (1H, brs), 5.18 (1H, d, J=7.1Hz), 5.32 (1H, d, J=9.3Hz), 5.48 (1H, d, J=9.0Hz), 5.98 (1H, d, J=5.4Hz), 6.20 (1H, t, J=8.8Hz), 7.27-7.39 (5H, m), 7.48 (2H, t, J=7.6Hz), 7.60 (1H, t, J=7.3Hz), 8.15 (2H, d, J=7.6Hz).
FAB-MS; m/z: 989 $(M+H)^+$.

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-hydroxy-3-phenylpropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.26 (3H, s), 1.41 (9H, s), 1.48 (3H, s), 1.60 (3H, s), 1.67 (3H, s), 1.77-2.43 (10H, m), 2.27 (3H, s), 2.73 (1H, dd, J=12.7, 5.6Hz), 2.82 (1H, dd, J=12.9, 4.4Hz), 2.90 (1H, d, J=5.4Hz), 3.32-3.38 (4H, m), 4.08 (1H, d, J=7.1Hz), 4.24 (1H, d, J=8.8Hz), 4.31 (1H, d, J=8.3Hz), 4.63 (1H, brs), 4.86 (1H, t, J=4.9Hz), 4.91 (1H, s), 5.19 (1H, d, J=7.1Hz), 5.31 (1H, d, J=9.3Hz), 5.70 (1H, d, J=9.3Hz), 6.00 (1H, d, J=4.9Hz), 6.06 (1H, t, J=8.1Hz), 7.29 (1H, t, J=6.8Hz), 7.36 (2H, t, J=7.6Hz), 7.42 (2H, d, J=7.3Hz), 7.47 (2H, t, J=7.7Hz), 7.60 (1H, t, J=7.6Hz), 8.12 (2H, d, J=7.6Hz).
FAB-MS; m/z: 875 $(M+H)^+$.

[Example 47]

[Formula 119]

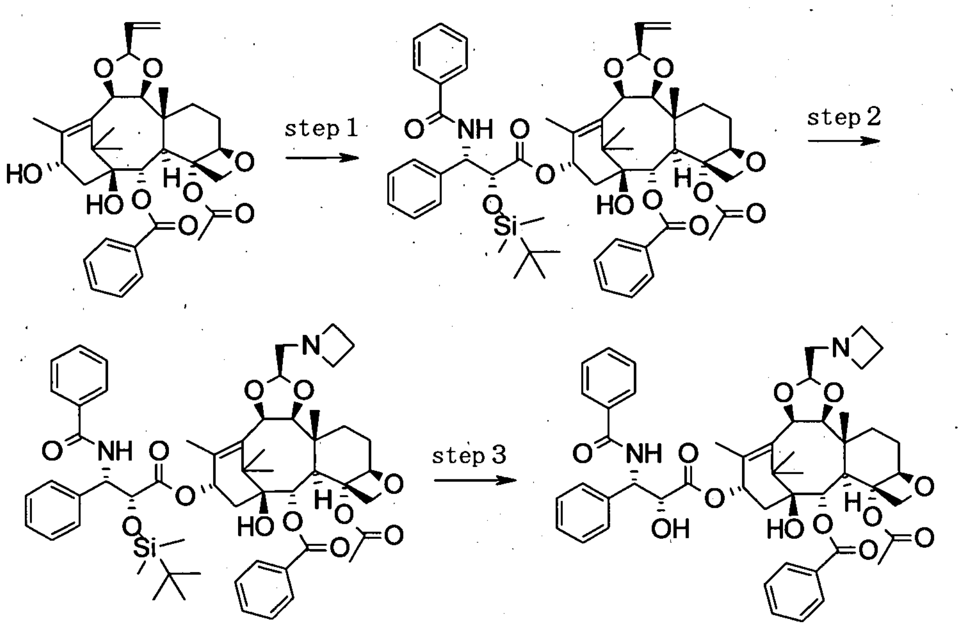


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(benzoylamino)-2-[(tert-butyldimethylsilyl)oxy]-3-phenylpropionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and

(3R,4S)-1-benzoyl-3-[(tert-butyldimethylsilyl)oxy]-2-azetidinone (Bioorg. Med. Chem. Lett., 1993, 3, 2467) were used as starting materials to perform the same procedure as that of Example 31, Step 1 and thereby to obtain the title compound as white amorphous solid.
$^1$ H-NMR (400MHz, $CDCl_3$) δ : -0.33 (3H, s), -0.03 (3H, s), 0.80 (9H, s), 1.30 (3H, s), 1.52 (3H, s), 1.64 (3H, s), 1.76-2.33 (7H, m), 1.79 (3H, s), 2.55 (3H, s), 2.92 (1H, d, J=4.9Hz), 4.20 (1H, d, J=6.8Hz), 4.25 (1H, d, J=8.5Hz), 4.35 (1H, d, J=8.8Hz), 4.71 (1H, d, J=2.2Hz), 4.98 (1H, br s), 5.22 (1H, d, J=5.9Hz), 5.27 (1H, d, J=7.3Hz), 5.46 (1H, d, J=10.5Hz), 5.58 (1H, d, J=17.3Hz), 5.78 (1H, d, J=9.3Hz), 5.96-6.07 (2H, m), 6.23 (1H, t, J=8.3Hz), 7.14 (1H, d, J=9.5Hz), 7.24-7.56 (9H, m), 7.60 (1H, t, J=7.1Hz), 7.78 (2H, d, J=7.8Hz), 8.15 (2H, d, J=7.8Hz).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(benzoylamino)-2-[(tert-butyldimethylsilyl)oxy]-3-phenylpropionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : -0.34 (3H, s), -0.03 (3H, s), 0.79 (9H, s), 1.26 (3H, s), 1.28 (3H, s), 1.51 (3H, s), 1.55-2.32 (9H, m), 1.77 (3H, s), 2.54 (3H, s), 2.73 (1H, dd, J=12.5, 5.6Hz), 2.81 (1H, dd, J=12.5, 4.2Hz), 2.91 (1H, d, J=4.6Hz), 3.31-3.39 (4H, m), 4.13 (1H, d, J=7.3Hz), 4.25 (1H, d, J=8.3Hz), 4.35 (1H, d, J=8.1Hz), 4.71 (1H, d, J=1.7Hz), 4.84 (1H, t, J=4.4Hz), 4.97 (1H, br s), 5.17 (1H, d, J=7.1Hz), 5.77 (1H, dd, J=9.0, 1.7Hz), 5.99 (1H, d, J=5.4Hz), 6.22 (1H, t, J=8.8Hz), 7.13 (1H, d, J=8.8Hz), 7.23-7.55 (9H, m), 7.60 (1H, t, J=7.6Hz), 7.78 (2H, d, J=7.1Hz), 8.15 (2H, d, J=7.3Hz).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(benzoylamino)-2-hydroxy-3-phenylpropionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.21 (3H, s), 1.42 (3H, s), 1.58 (3H, s), 1.65 (3H, s), 1.75-2.41 (10H, m), 2.31 (3H, s), 2.72 (1H, dd, J=12.2, 5.9Hz), 2.80 (1H, dd, J=12.5, 4.4Hz), 2.87 (1H, d, J=4.9Hz), 3.30-3.38 (4H, m), 4.03 (1H, d, J=7.1Hz), 4.24 (1H, d, J=8.8Hz), 4.32 (1H, d, J=8.5Hz), 4.76 (1H, d, J=2.4Hz), 4.83 (1H, t, J=5.4Hz), 4.92 (1H, brs), 5.11 (1H, d, J=7.3Hz), 5.86 (1H, dd, J=9.3, 2.2Hz), 5.99 (1H, d, J=5.1Hz), 6.07 (1H, t, J=8.1Hz), 7.25-7.55 (10H, m), 7.60 (1H, t, J=7.6Hz), 7.83 (2H, d, J=8.1Hz), 8.12 (2H, d, J=8.5Hz).
FAB-MS; m/z: 879 $(M+H)^+$.

[Example 48]

[Formula 120]

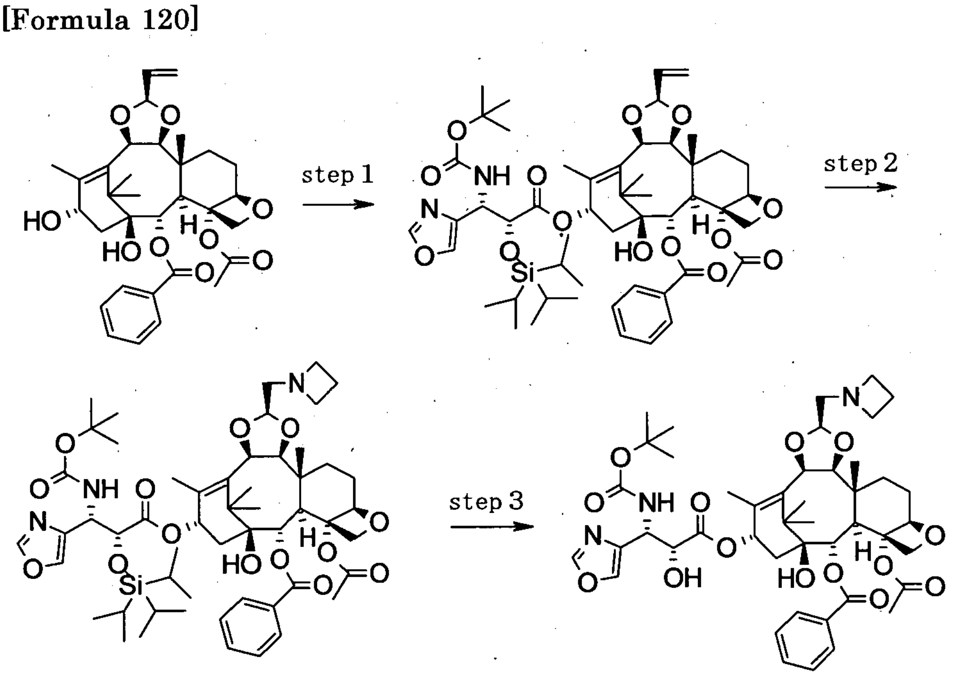

Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(4-oxazolyl)-2-[(triisopropylsilyl)oxy]propionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 22, Step 4 were used as starting materials to perform the same procedure as that of Example 31, Step 1 and thereby to obtain the title compound as white amorphous solid.
$^1$H-NMR (400MHz, $CDCl_3$) δ : 0.93-1.03 (21H, m), 1.30 (3H, s), 1.41 (9H, s), 1.52 (3H, s), 1.52-2.36 (7H, m), 1.65 (3H, s), 1.81 (3H, s), 2.47 (3H, s), 2.96 (1H, d, J=5.4Hz), 4.20 (1H, d, J=8.1Hz), 4.21 (1H, d, J=7.3Hz), 4.34 (1H, d, J=8.3Hz), 4.96 (1H, s), 5.15 (1H, s), 5.21 (1H, d, J=6.1Hz), 5.24-5.31 (2H, m), 5.46 (1H, d, J=10.3Hz), 5.58 (1H, d, J=17.3Hz), 5.95-6.05 (2H, m), 6.08 (1H, t, J=8.8Hz), 7.46 (1H, t, J=7.8Hz), 7.55-7.61 (2H, m), 7.85 (1H, s), 8.14 (1H, d, J=7.6Hz).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-3-(4-oxazolyl)-2-[(triisopropylsilyl)oxy]propionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.92-1.03 (21H, m), 1.28 (3H, s), 1.40 (9H, s), 1.51 (3H, s), 1.55-2.33 (9H, m), 1.61 (3H, s), 1.79 (3H, s), 2.47 (3H, s), 2.73 (1H, dd, J=12.5, 5.6Hz), 2.81 (1H, dd, J=12.2, 4.2Hz), 2.95 (1H, d, J=5.4Hz), 3.31-3.38 (4H, m), 4.14 (1H, d, J=7.6Hz), 4.20 (1H, d, J=8.1Hz), 4.34 (1H, d, J=8.3Hz), 4.83 (1H, t, J=5.1Hz), 4.95 (1H, s), 5.14 (1H, s), 5.19 (1H, d, J=7.8Hz), 5.23 (1H, d, J=10.3Hz), 5.27 (1H, d, J=10.5Hz), 5.97 (1H, d, J=5.4Hz), 6.07 (1H, t, J=9.0Hz), 7.46 (2H, t, J=7.6Hz), 7.55-7.61 (2H, m), 7.85 (1H, s), 8.14 (2H, d, J=8.1Hz).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-hydroxy-3-(4-oxazolyl)propionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.25 (3H, s), 1.42 (9H, s), 1.49 (3H, s), 1.61 (3H, s), 1.73 (3H, s), 1.70-2.47 (10H, m), 2.34 (3H, s), 2.74 (1H, dd, J=12.2, 5.9Hz), 2.82 (1H, dd, J=12.5, 4.6Hz), 2.92 (1H, d, J=5.1Hz), 3.30-3.40 (4H, m), 4.11 (1H, d, J=7.1Hz), 4.23 (1H, d, J=8.3Hz), 4.32 (1H, d, J=8.3Hz), 4.80 (1H, d, J=2.2Hz), 4.86 (1H, t, J=4.6Hz), 4.92 (1H, brs), 5.21 (1H, d, J=7.3Hz), 5.32 (1H, d, J=9.8Hz), 5.55 (1H, d, J=9.5Hz), 6.00 (1H, d, J=4.9Hz), 6.10 (1H, t, J=8.1Hz), 7.47 (2H, t, J=7.7Hz), 7.60 (1H, t, J=7.6Hz), 7.67 (1H, s), 7.87 (1H, s), 8.13 (2H, d, J=8.3Hz).
FAB-MS; m/z: 866 $(M+H)^+$.

[Example 49]

[Formula 121]

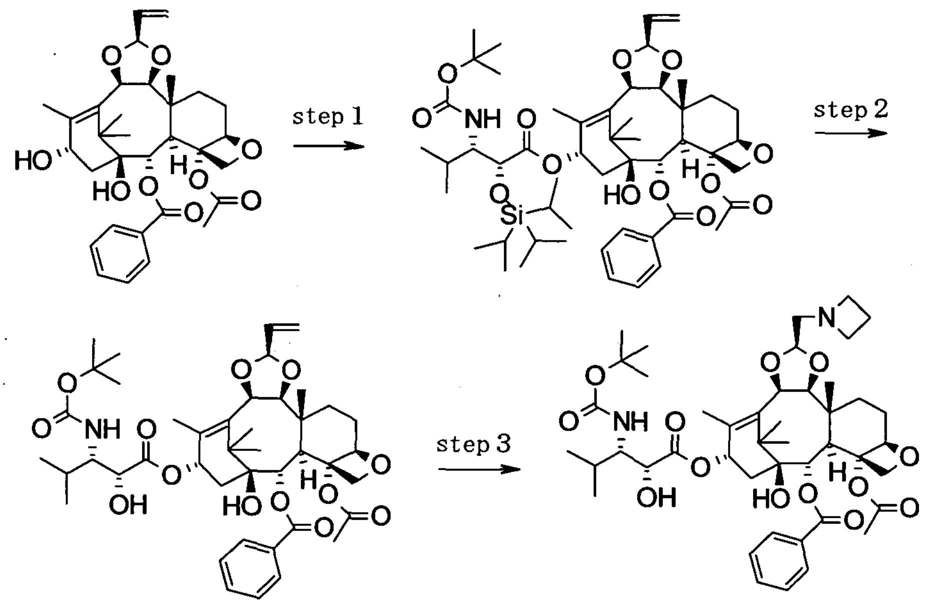


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-4-methyl-2-[(triisopropylsilyl)oxy]pentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 23, Step 3 were used as starting materials to perform the same procedure as that of Example 31, Step 1 and thereby to obtain the title compound as white amorphous solid.
$^1$H-NMR (400MHz, $CDCl_3$) δ : 1.01-2.05 (33H, m), 1.30 (3H, s), 1.36 (9H, s), 1.52 (3H, s), 1.64 (3H, s), 1.79 (3H, s), 2.20-2.33 (2H, m), 2.36 (3H, s), 2.92 (1H, d, J=5.1Hz), 3.64 (1H, t, J=10.3Hz), 4.19-4.22 (2H, m), 4.34 (1H, d, J=8.5Hz), 4.72 (1H, s), 4.78 (1H, d, J=10.3Hz), 4.93 (1H, s), 5.20 (1H, d, J=6.1Hz), 5.26 (1H, d, J=7.1Hz), 5.45 (1H, d, J=10.3Hz), 5.57 (1H, d, J=17.3Hz), 5.95-6.04 (3H, m), 7.49 (2H, t, J=7.6Hz), 7.61 (1H, t, J=7.6Hz), 8.15 (2H, d, J=7.6Hz).
FAB-MS; m/z: 954 $(M+H)^+$, 976 $(M+Na)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-4-methylpentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.02 (3H, d, J=6.8Hz), 1.06 (3H, d, J=6.8Hz), 1.24-2.09 (7H, m), 1.26 (3H, s), 1.38 (9H, s), 1.50 (3H, s), 1.64 (3H, s), 1.81 (3H, s), 2.31-2.41 (1H, m), 2.35 (3H, s), 2.93 (1H, d, J=5.1Hz), 3.80 (1H, t, J=9.6Hz), 3.98 (1H, br s), 4.19 (1H, d, J=7.1Hz), 4.24 (1H, d, J=8.5Hz), 4.33 (1H, d, J=8.5Hz), 4.50 (1H, s), 4.88 (1H, d, J=10.3Hz), 4.94 (1H, s), 5.25 (1H, d, J=6.3Hz), 5.31 (1H, d, J=6.8Hz), 5.46 (1H, d, J=10.7Hz), 5.58 (1H, d, J=17.3Hz), 5.97-6.08 (3H, m), 7.48 (2H, t, J=7.6Hz), 7.61 (1H, t, J=7.6Hz), 8.13 (2H, d, J=7.6Hz).
FAB-MS; m/z: 798 $(M+H)^+$, 820 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-hydroxy-4-methylpentanoate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.02 (3H, d, J=6.6Hz), 1.06 (3H, d, J=6.8Hz), 1.24-2.40 (11H, m), 1.25 (3H, s), 1.38 (9H, s), 1.50 (3H, s), 1.61 (3H, s), 1.79 (3H, s), 2.34 (3H, s), 2.74 (1H, dd, J=12.6, 5.9Hz), 2.82 (1H, dd, J=12.6, 4.3Hz), 2.92 (1H, d, J=5.1Hz), 3.31-3.40 (4H, m), 3.79 (1H, t, J=9.6Hz), 4.12 (1H, d, J=7.1Hz), 4.24 (1H, d, J=8.5Hz), 4.33 (1H, d, J=8.5Hz), 4.50 (1H, d, J=1.2Hz), 4.85-4.88 (2H, m), 4.93 (1H, s), 5.22 (1H, d, J=6.8Hz), 5.99-6.06 (2H, m), 7.48 (2H, t, J=7.7Hz), 7.59-7.63 (1H, m), 8.12-8.14 (2H, m).
FAB-MS; m/z: 841 $(M+H)^+$.

[Example 50]

[Formula 122]

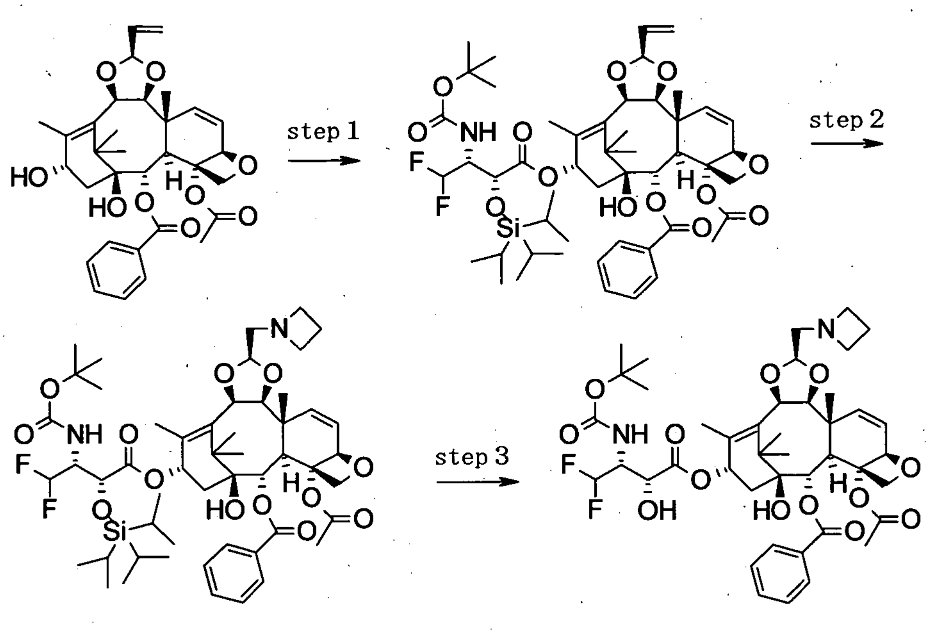


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-6,11-dien-13-yl

(2R,3R)-3-(tert-butoxycarbonylamino)-4,4-difluoro-2-[(triisopropylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-6,11-dien-13-yl (WO01/027115) and (3R,4R)-1-(tert-butoxycarbonylamino)-4-(difluoromethyl)-3-[(triisopropylsilyl)oxy]-2-azetidinone were used as starting materials to perform the same procedure as that of Example 31, Step 1 and thereby to obtain the title compound as white amorphous solid. $^1$H-NMR (400MHz, $CDCl_3$) δ : 1.02-1.60 (27H, m), 1.26 (3H, s), 1.38 (3H, s), 1.54 (3H, s), 1.58 (3H, s), 1.72 (3H, s), 1.84 (1H, s), 2.28-2.36 (2H, m), 2.39 (3H, s), 3.09 (1H, d, J=5.9Hz), 4.03 (1H, d, J=7.8Hz), 4.27-4.44 (3H, m), 4.86 (2H, d, J=4.2Hz), 5.04 (1H, d, J=10.3Hz), 5.19 (1H, d, J=7.8Hz), 5.21 (1H, d, J=6.3Hz), 5.48 (1H, d, J=10.5Hz), 5.60 (1H, d, J=17.3Hz), 5.64-5.71 (1H, m), 5.79 (1H, d, J=6.3Hz), 5.91-6.07 (3H, m), 6.11 (1H, d, J=10.3Hz), 7.52 (2H, t, J=7.4Hz), 7.62 (1H, t, J=7.4Hz), 8.17 (2H, d, J=7.3Hz).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-6,11-dien-13-yl

(2R,3R)-3-(tert-butoxycarbonylamino)-4,4-difluoro-2-[(triisopropylsilyl)oxy]butyrate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 1.04-2.36 (26H, m), 1.24 (3H, s), 1.37 (9H, s), 1.53 (3H, s), 1.55 (3H, s), 1.70 (3H, s), 2.38 (3H, s), 2.78 (1H, dd, J=12.5, 5.6Hz), 2.85 (1H, dd, J=12.4, 4.5Hz), 3.08 (1H, d, J=5.9Hz), 3.36-3.43 (4H, m), 3.96 (1H, d, J=7.8Hz), 4.27-4.44 (3H, m), 4.83-4.87 (3H, m), 5.04 (1H, d, J=10.3Hz), 5.10 (1H, d, J=7.6Hz), 5.68 (1H, dd, J=10.3, 4.2Hz), 5.79 (1H, d, J=6.1Hz), 5.94 (1H, d, J=5.9Hz), 6.03 (1H, t, J=9.1Hz), 6.08 (1H, d, J=10.3Hz), 7.52 (2H, t, J=7.6Hz), 7.62 (1H, t, J=7.6Hz), 8.17 (2H, d, J=7.6Hz).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-6,11-dien-13-yl

(2R,3R)-3-(tert-butoxycarbonylamino)-4,4-difluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.22 (3H, s), 1.23-2.39 (6H, m), 1.40 (9H, s), 1.52 (3H, s), 1.57 (3H, s), 1.69 (3H, s), 2.38 (3H, s), 2.78 (1H, dd, J=12.5, 5.6Hz), 2.86 (1H, dd, J=12.4, 4.3Hz), 3.08 (1H, d, J=5.6Hz), 3.38 (4H, t, J=7.1Hz), 3.93 (1H, d, J=7.4Hz), 4.26 (1H, d, J=8.1Hz), 4.35 (1H, d, J=8.3Hz), 4.46-4.56 (1H, m), 4.64 (1H, s), 4.84-4.90 (2H, m), 5.14 (1H, d, J=7.6Hz), 5.22 (1H, d, J=9.8Hz), 5.70 (1H, dd, J=10.3, 4.0Hz), 5.89 (1H, d, J=4.9Hz), 5.97 (1H, d, J=5.6Hz), 6.06-6.13 (1H, m), 6.09 (1H, d, J=10.3Hz), 7.50 (2H, t, J=7.6Hz), 7.62 (1H, t, J=7.6Hz), 8.15 (2H, d, J=7.6Hz).
FAB-MS; m/z: 847 $(M+H)^+$.

[Example 51]

(1S,2S,3R,4S,5R,8R,9S,10R,13S)-4-(Acetoxy)-2-(benzoyloxy)-5,20-epoxy-1-hydroxy-9,10-{(1S)-2-[(2R)-2-hydroxymethylazetidino]ethylidenedioxy}tax-11-en-13-yl

(2R,3S)-3-(tert-butoxycarbonylamino)-3-(3-fluoro-2-pyridyl)-2-hydroxypropionate

[Formula 123]

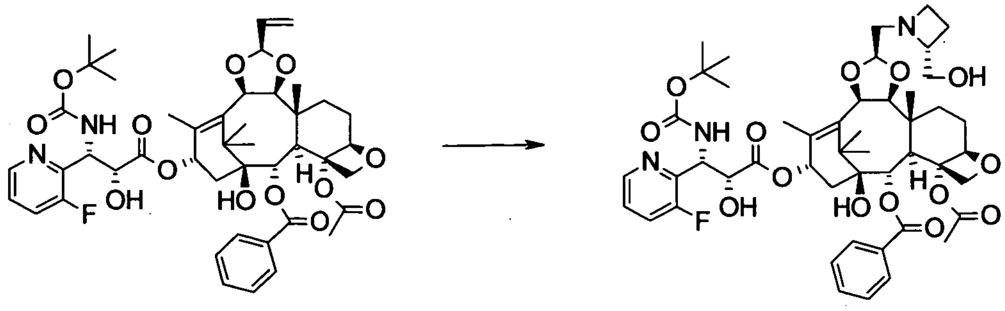

(1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 26 were used as starting materials to perform the same procedure as that of Example 1 and

thereby to obtain the title compound as white amorphous solid.
$^1$H-NMR (400MHz, $CDCl_3$) δ : 1.25-2.48 (11H, m), 1.29 (3H, s), 1.40 (9H, s), 1.49 (3H, s), 1.61 (3H, s), 1.78 (3H, s), 2.34 (3H, s), 2.79 (1H, dd, J=12.7, 5.6Hz), 2.92-2.96 (2H, m), 3.08 (1H, dd, J=16.4, 8.5Hz), 3.43-3.52 (3H, m), 3.65 (1H, dd, J=11.6, 3.1Hz), 4.15 (1H, d, J=7.1Hz), 4.22 (1H, d, J=8.5Hz), 4.32 (1H, d, J=8.5Hz), 4.68 (1H, d, J=2.7Hz), 4.88 (1H, dd, J=5.6, 4.4Hz), 4.91 (1H, s), 5.22 (1H, d, J=7.1Hz), 5.66 (1H, d, J=7.8Hz), 5.99 (1H, d, J=5.1Hz), 6.09 (1H, t, J=8.3Hz), 6.22 (1H, d, J=8.5Hz), 7.28-7.33 (1H, m), 7.43-7.49 (3H, m), 7.58-7.62 (1H, m), 8.12-8.15 (2H, m), 8.40 (1H, d, J=4.6Hz).
FAB-MS; m/z: 924 $(M+H)^+$.

[Example 52]

(1S,2S,3R,4S,5R,8R,9S,10R,13S)-4-(Acetoxy)-2-(benzoyloxy)-5,20-epoxy-1-hydroxy-9,10-{(1S)-2-[(2R)-2-methoxymethylazetidino]ethylidenedioxy}tax-11-en-13-yl

(2R,3S)-3-(tert-butoxycarbonylamino)-3-(3-fluoro-2-pyridyl)-2-hydroxypropionate

[Formula 124]

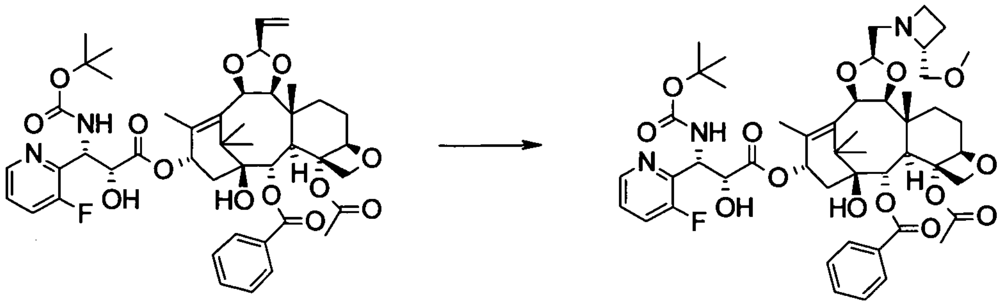

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 27, Step 2 were used as starting materials to perform the same procedure as that of Example 1 and thereby to obtain the title compound as white amorphous solid.
$^1$H-NMR (400MHz, $CDCl_3$) δ : 1.26-2.48 (9H, m), 1.28 (3H, s), 1.40 (9H, s), 1.49 (3H, s), 1.62 (3H, s), 1.78 (3H, s), 2.34 (3H, s), 2.70 (1H, dd, J=12.7, 6.6Hz), 2.93 (1H, d, J=5.1Hz), 2.97-3.08 (2H, m), 3.37 (3H, s), 3.43-3.56 (4H, m), 4.12 (1H, d, J=7.3Hz), 4.16 (1H, brs), 4.23 (1H, d, J=8.3Hz), 4.33 (1H, d, J=8.3Hz), 4.68 (1H, s), 4.89-4.91 (2H, m), 5.21 (1H, d, J=7.1Hz), 5.65 (1H, d, J=8.5Hz), 6.00 (1H, d, J=5.1Hz), 6.08 (1H, t, J=8.8Hz), 6.21 (1H, d, J=9.3Hz), 7.28-7.33 (1H, m), 7.42-7.49 (3H, m), 7.60 (1H, t, J=7.4Hz), 8.12-8.15 (2H, m), 8.40 (1H, d, J=4.9Hz).
FAB-MS; m/z: 938 $(M+H)^+$.

[Example 53]

[Formula 125]

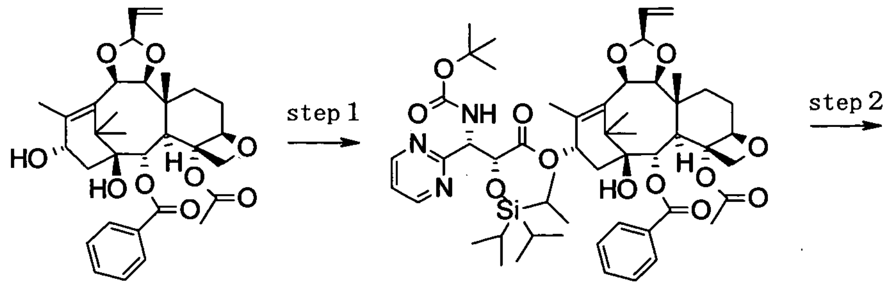


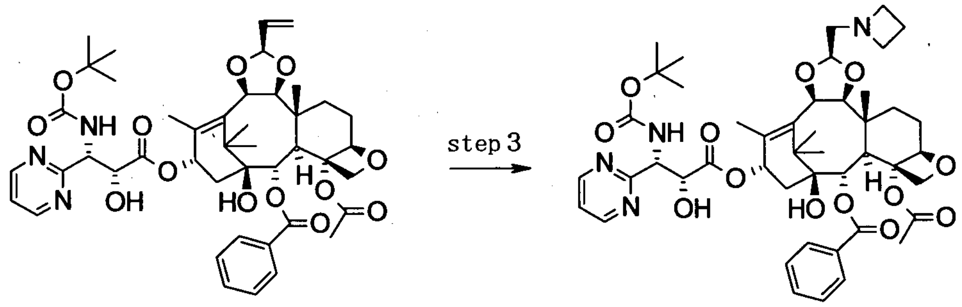


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(2-pyrimidinyl)-2-[(triisopropylsilyl)oxy]propionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 28, Step 6 were used as starting materials to perform the same procedure as that of Example 31, Step 1 and thereby to obtain the title compound as white amorphous solid.
$^1$H-NMR (400MHz, $CDCl_3$) δ : 0.85-0.92 (21H, m), 1.24-2.08 (5H, m), 1.34 (3H, s), 1.40 (9H, s), 1.53 (3H, s), 1.66 (3H, s), 1.84 (3H, s), 2.32-2.45 (2H, m), 2.58 (3H, s), 2.99 (1H, d, J=5.1Hz), 4.21-4.24 (2H, m), 4.35 (1H, d, J=8.3Hz), 4.98 (1H, s), 5.22 (1H, d, J=6.3Hz), 5.29 (1H, d, J=7.1Hz), 5.34 (1H, d, J=1.7Hz), 5.44-5.49 (2H, m), 5.58 (1H, d, J=17.3Hz), 5.85 (1H, d, J=10.0Hz), 5.97-6.05 (2H, m), 6.12 (1H, t, J=8.8Hz), 7.22 (1H, t, J=4.9Hz), 7.45 (2H, t, J=7.6Hz), 7.55-7.59 (1H, m), 8.14-8.16 (2H, m), 8.73 (2H, d, J=4.9Hz).
FAB-MS; m/z: 990 $(M+H)^+$, 1012 $(M+Na)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-hydroxy-3-(2-pyrimidinyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.24-2.25 (6H, m), 1.30 (3H, s), 1.42 (9H, s), 1.51 (3H, s), 1.65 (3H, s), 1.84 (3H, s), 2.43-2.49 (1H, m), 2.46 (3H, s), 2.97 (1H, d, J=5.1Hz), 3.91 (1H, s), 4.21-4.24 (2H, m), 4.34 (1H, d, J=8.3Hz), 4.95 (1H, s), 5.00 (1H, d, J=4.2Hz), 5.24 (1H, d, J=6.1Hz), 5.32 (1H, d, J=7.1Hz), 5.44-5.48 (2H, m), 5.58 (1H, d, J=17.3Hz), 5.97-6.14 (4H, m), 7.24-7.26 (1H, m), 7.47 (2H, t, J=7.7Hz), 7.57-7.61 (1H, m), 8.13-8.15 (2H, m), 8.76 (2H, d, J=4.9Hz).
FAB-MS; m/z: 834 $(M+H)^+$, 856 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-hydroxy-3-(2-pyrimidinyl)propionate

The compound obtained in Step 2 mentioned above was used as a starting material to perform the same

procedure as that of Example 1 and thereby to obtain the title compound as white amorphous solid.
$^1$H-NMR (400MHz, $CDCl_3$) δ : 1.26-2.24 (9H, m), 1.28 (3H, s), 1.42 (9H, s), 1.50 (3H, s), 1.62 (3H, s), 1.81 (3H, s), 2.42-2.48 (1H, m), 2.45 (3H, s), 2.74 (1H, dd, J=12.5, 5.8Hz), 2.82 (1H, dd, J=12.5, 4.4Hz), 2.95 (1H, d, J=5.1Hz), 3.32-3.40 (4H, m), 4.14 (1H, d, J=7.1Hz), 4.22 (1H, d, J=8.5Hz), 4.34 (1H, d, J=8.5Hz), 4.87 (1H, dd, J=5.8, 4.4Hz), 4.94 (1H, s), 4.99 (1H, d, J=2.0Hz), 5.22 (1H, d, J=6.8Hz), 5.44 (1H, dd, J=8.8, 1.5Hz), 6.00 (1H, d, J=5.1Hz), 6.06-6.13 (2H, m), 7.25 (1H, t, J=4.9Hz), 7.46 (2H, t, J=7.7Hz), 7.57-7.61 (1H, m), 8.13-8.15 (2H, m), 8.75 (2H, d, J=4.9Hz).
FAB-MS; m/z: 877 $(M+H)^+$.

[Example 54]

(1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-4,4-dimethyl-2-hydroxypentanoate

[Formula 126]

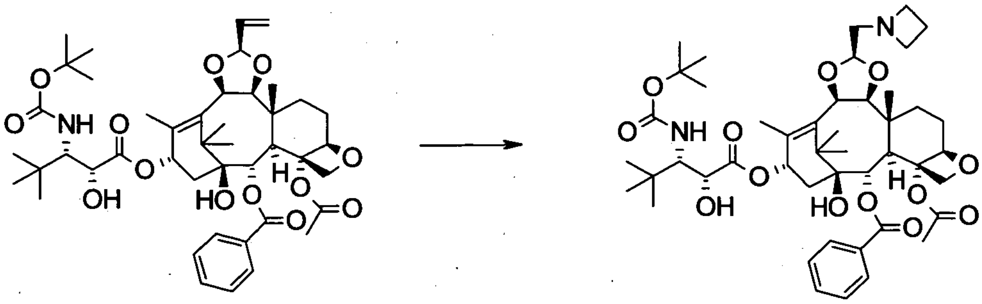

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-4,4-dimethyl-2-hydroxypentanoate (Bioorg. Med. Chem. Lett., 2004, 14, 3209) was used as a starting material to perform the same procedure as that of Example 1 and thereby to obtain the title compound as white amorphous solid.
$^1$H-NMR (400MHz, $CDCl_3$) δ : 1.04 (9H, s), 1.24 (3H, s), 1.34-2.40 (10H, m), 1.38 (9H, s), 1.49 (3H, s), 1.61 (3H, s), 1.78 (3H, s), 2.34 (3H, s), 2.74 (1H, dd, J=12.5, 5.9Hz), 2.83 (1H, dd, J=12.5, 4.4Hz), 2.92 (1H, d, J=5.1Hz), 3.31-3.39 (4H, m), 3.93 (1H, d, J=10.5Hz), 4.12 (1H, d, J=7.3Hz), 4.25 (1H, d, J=8.5Hz), 4.33 (1H, d, J=8.5Hz), 4.60 (1H, s), 4.87 (1H, dd, J=5.9, 4.4Hz), 4.93 (1H, s), 5.10 (1H, d, J=10.5Hz), 5.22 (1H, d, J=6.8Hz), 5.99-6.06 (2H, m), 7.48 (2H, t, J=7.7Hz), 7.59-7.63 (1H, m), 8.12-8.14 (2H, m).
FAB-MS; m/z: 855 $(M+H)^+$.

[Example 55]

[Formula 127]

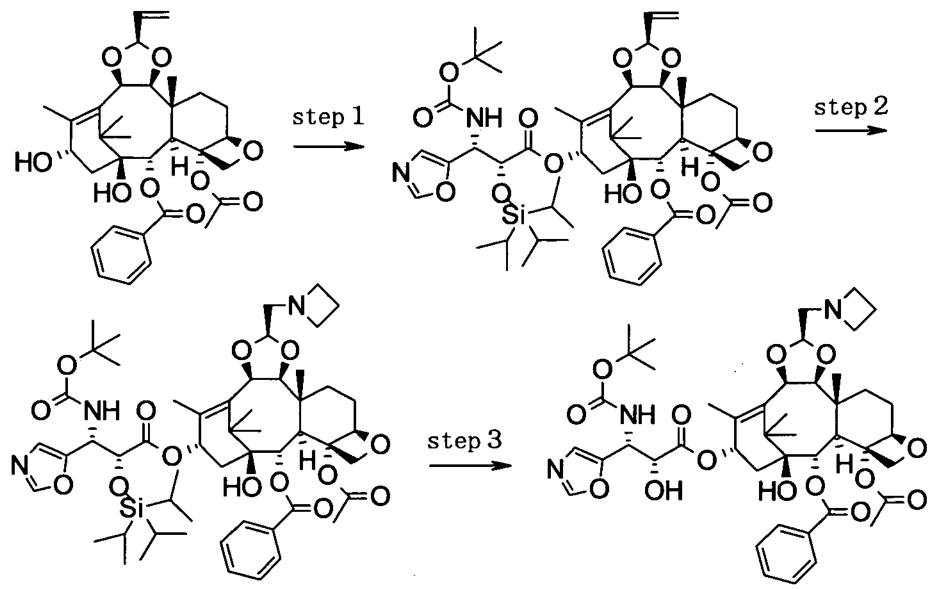


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-3-(5-oxazolyl)-2-[(triisopropylsilyl)oxy]propionate

(1S,25,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 29; Step 6 were used as starting materials to perform the same procedure as that of Example 31, Step 1 and thereby to obtain the title compound as white amorphous solid.
$^1$H-NMR (400MHz, $CDCl_3$) δ : 0.95-1.04 (21H, m), 1.30 (3H, s), 1.41 (9H, s), 1.52 (3H, s), 1.65 (3H, s), 1.80 (3H, s), 1.86-2.36 (7H, m), 2.41 (3H, s), 2.96 (1H, d, J=4.9Hz), 4.21 (2H, d, J=7.8Hz), 4.35 (1H, d, J=8.5Hz), 4.93-4.97 (2H, m), 5.21 (1H, d, J=6.6Hz), 5.26-5.33 (2H, m), 5.40 (1H, d, J=10.7Hz), 5.46 (1H, d, J=10.7Hz), 5.58 (1H, d, J=17.3Hz), 5.96-6.13 (3H, m), 7.02 (1H, s), 7.47 (2H, t, J=7.3Hz), 7.59 (1H, t, J=7.3Hz), 7.87 (1H, s), 8.14 (2H, d, J=8.5Hz).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-3-(5-oxazolyl)-2-[(triisopropylsilyl)oxy]propionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.95-1.01 (21H, m), 1.28 (3H, s), 1.40 (9H, s), 1.51 (3H, s), 1.54-2.18 (9H, m), 1.61 (3H, s), 1.77 (3H, s), 2.40 (3H, s), 2.73 (1H, dd, J=12.7, 5.9Hz), 2.81 (1H, dd, J=12.0, 4.4Hz), 2.94 (1H, d, J=4.9Hz), 3.32-3.39 (4H, m), 4.14 (1H, d, J=7.1Hz), 4.21 (1H, d, J=8.3Hz), 4.34 (1H, d, J=8.8Hz), 4.84 (1H, t, J=5.6Hz), 4.94 (2H, brs), 5.18 (1H, d, J=7.6Hz), 5.29 (1H, d, J=10.5Hz), 5.39 (1H, d, J=10.0Hz), 5.97 (1H, d, J=5.1Hz), 6.07 (1H, t, J=8.3Hz), 7.01 (1H, s), 7.47 (2H, t, J=7.7Hz), 7.59 (1H, t, J=7.6Hz), 7.86 (1H, s), 8.14 (2H, d, J=7.8Hz).
FAB-MS; m/z: 1022 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-2-hydroxy-3-(5-oxazolyl)propionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.26 (3H, s), 1.43 (9H, s), 1.49 (3H, s), 1.58-2.17 (10H, m), 1.61 (3H, s), 1.73 (3H, s), 2.32 (3H, s), 2.74 (1H, dd, J=12.5, 5.6Hz), 2.83 (1H, dd, J=12.5, 4.6Hz), 2.92 (1H, d, J=4.9Hz), 3.31-3.40 (4H, m), 4.10

(1H, d, J=7.1Hz), 4.25 (1H, d, J=8.3Hz), 4.33 (1H, d, J=8.5Hz), 4.69 (1H, s), 4.87 (1H, dd, J=5.6, 4.4Hz), 4.92 (1H, brs), 5.22 (1H, d, J=7.6Hz), 5.46-5.55 (2H, m), 6.01 (1H, d, J=5.1Hz), 6.13 (1H, t, J=8.3Hz), 7.07 (1H, s), 7.48 (2H, t, J=7.7Hz), 7.61 (1H, t, J=7.6Hz), 7.85 (1H, s), 8.13 (2H, d, J=8.1Hz).
FAB-MS; m/z: 866 $(M+H)^+$.

[Example 56]

[Formula 128]

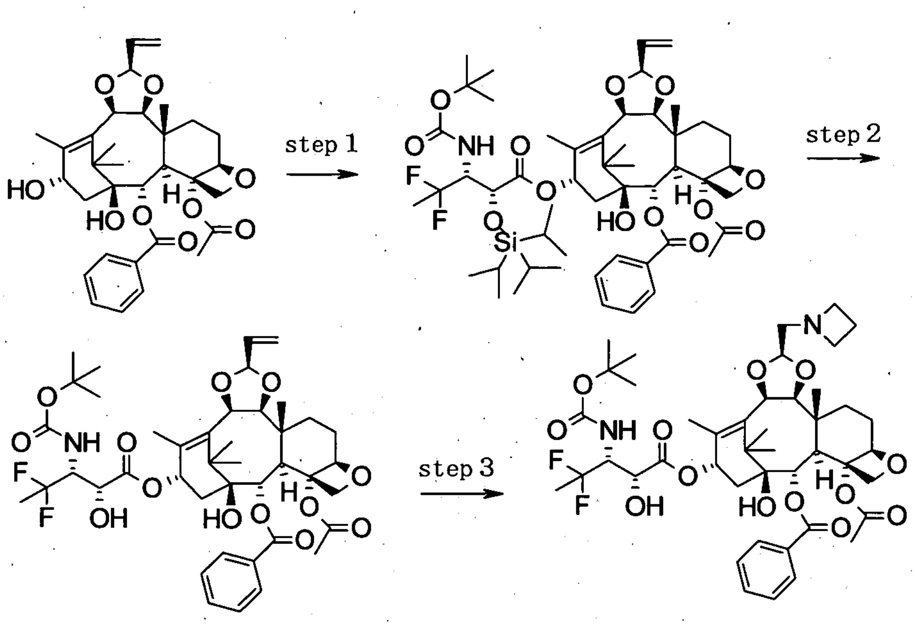


Step 1: (1S,2S,3R,4S,5R,8R,9S, 10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4,4-difluoro-2-[(triisopropylsilyl)oxy]pentanoate

(1S,2S,3R,4S,5R,8R,9S, 10R, 13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 30, Step 6 were used as starting materials to perform the same procedure as that of Example 31, Step 1 and thereby to obtain the title compound as white amorphous solid.
$^1$H-NMR (400MHz, $CDCl_3$) δ : 1.05-1.17 (21H, m), 1.24-2.31 (8H, m), 1.29 (3H, s), 1.39 (9H, s), 1.51 (3H, s), 1.64 (3H, s), 1.68 (3H, t, J=19.0Hz), 1.79 (3H, s), 1.85 (3H, s), 2.93 (1H, d, J=5.4Hz), 4.20 (2H, t, J=5.0Hz), 4.35 (1H, d, J=7.8Hz), 4.93 (1H, s), 5.03 (1H, s), 5.14-5.22 (2H, m), 5.26 (1H, d, J=7.3Hz), 5.45 (1H, d, J=10.5Hz), 5.57 (1H, d, J=17.1Hz), 5.95-6.04 (3H, m), 7.49 (2H, t, J=7.8Hz), 7.61 (1H, t, J=7.2Hz), 8.15 (2H, d, J=7.3Hz).

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxyhax-11-en-13-yl

(2R,3R)-3-(tert-butoxycarbonylamino)-4,4-difluoro-2-hydroxypentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.22-2.41 (10H, m), 1.25 (3H, s), 1.42 (9H, s), 1.64 (3H, s), 1.72 (3H, t, J=19.0Hz), 1.78 (3H, s), 2.35 (3H, s), 2.94 (1H, d, J=4.6Hz), 4.18 (1H, d, J=7.3Hz), 4.25 (1H, d, J=8.3Hz), 4.34 (1H, d, J=8.5Hz), 4.46-4.49

(1H, m), 4.50-4.61 (1H, m), 4.76-4.78 (1H, m), 4.93 (1H, s), 5.25 (1H, d, J=6.6Hz), 5.31 (2H, d, J=7.8Hz), 5.47 (1H, d, J=10.7Hz), 5.58 (1H, d, J=17.3Hz), 5.96-6.06 (2H, m), 6.10-6.16 (1H, m), 7.49 (2H, t, J=7.7Hz), 7.59-7.64 (1H, m), 8.12-8.15 (2H, m).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4,4-difluoro-2-hydroxypentanoate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.22-2.38 (10H, m), 1.24 (3H, s), 1.41 (9H, s), 1.49 (3H, s), 1.61 (3H, s), 1.72 (3H, t, J=19.3Hz), 1.76 (3H, s), 2.34 (3H, s), 2.75 (1H, dd, J=12.2, 5.6Hz), 2.84 (1H, dd, J=12.5, 4.2Hz), 2.92 (1H, d, J=5.1Hz), 3.34-3.41 (4H, m), 4.11 (1H, d, J=7.1Hz), 4.24 (1H, d, J=8.3Hz), 4.34 (1H, d, J=8.3Hz), 4.49-4.60 (1H, m), 4.76 (1H, s), 4.89 (1H, t, J=4.9Hz), 4.93 (1H, s), 5.22 (1H, d, J=6.8Hz), 5.31 (1H, d, J=10.5Hz), 6.00 (1H, d, J-5.1Hz), 6.08-6.14 (1H, m), 7.49 (2H, t, J=7.3Hz), 7.59-7.64 (1H, m), 8.11-8.15 (2H, m).
FAB-MS; m/z: 863 $(M+H)^+$.

[Example 57]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-4,4-difluoro-2-hydroxypentanoate

[Formula 129]

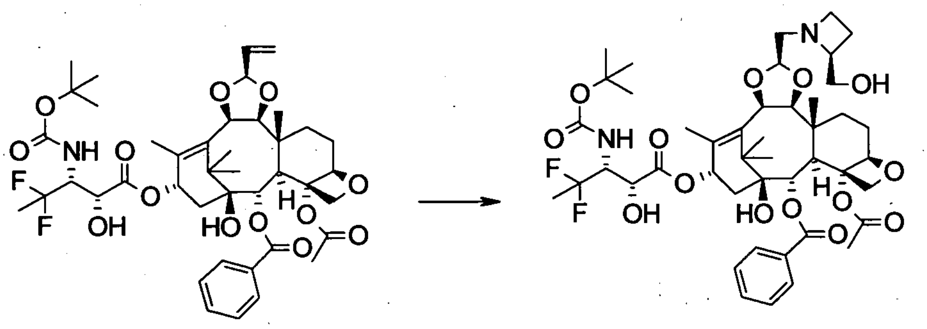

The compound obtained in Example 56, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.21-2.38 (11H, m), 1.23 (3H, s), 1.42 (9H, s), 1.49 (3H, s), 1.59 (3H, s), 1.72 (3H, t, J=19.0Hz), 1.76 (3H, s), 2.34 (3H, s), 2.84 (1H, dd, J=12.8, 4.5Hz), 2.88-2.94 (2H, m), 3.12 (1H, q , J=8.2Hz), 3.43 (1H, dd, J=11.7, 2.4Hz), 3.47-3.55 (2H, m), 3.67 (1H, dd, J=11.5, 2.9Hz), 4.13 (1H, d, J=7.1Hz), 4.24 (1H, d, J=8.3Hz), 4.33 (1H, d, J=8.3Hz), 4.49-4.60 (1H, m), 4.77 (1H, s), 4.87-4.94 (2H, m), 5.23 (1H, d, J=7.1Hz), 5.31 (1H, d, J=10.3Hz), 5.99 (1H, d, J=4.9Hz), 6.08-6.14 (1H, m), 7.49 (2H, t, J=7.7Hz), 7.59-7.64 (1H, m), 8.11-8.15 (2H, m).
FAB-MS; m/z: 893 $(M+H)^+$.

[Example 58]

[Formula 130]

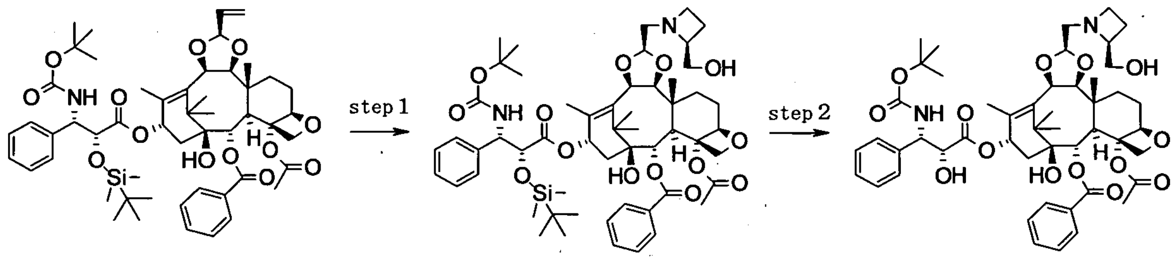


Step 1: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-2-[(tert-butyldimethylsilyl)oxy]-3-phenylpropionate

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-2-[(tert-butyldimethylsilyl)oxy]-3-phenylpropionate and the compound obtained in Reference Example 25 were used as starting materials to perform the same procedure as that of Example 1 and thereby to obtain the title compound as white amorphous solid.
$^1$H-NMR (400MHz, $CDCl_3$) δ : -0.34 (3H, s), -0.11 (3H, s), 0.74 (9H, s), 1.32 (3H, s), 1.39 (9H, s), 1.52 (3H, s), 1.60 (3H, s), 1.66-2.33 (10H, m), 1.76 (3H, s), 2.51 (3H, s), 2.81 (1H, dd, J=12.7, 4.6Hz), 2.90 (1H, dd, J=12.7, 5.1Hz), 2.94 (1H, d, J=4.9Hz), 3.06-3.15 (1H, m), 3.39-3.53 (3H, m), 3.64 (1H, dd, J=12.0, 2.9Hz), 4.16 (1H, d, J=7.3Hz), 4.24 (1H, d, J=8.5Hz), 4.34 (1H, d, J=7.8Hz), 4.56 (1H, s), 4.86 (1H, t, J=4.9Hz), 4.96 (1H, s), 5.20 (1H, d, J=6.8Hz), 5.32 (1H, d, J=9.0Hz), 5.48 (1H, d, J=9.0Hz), 5.96 (1H, d, J=4.9Hz), 6.20 (1H, t, J=9.3Hz), 7.25-7.39 (5H, m), 7.48 (2H, t, J=7.1Hz), 7.60 (1H, t, J=7.6Hz), 8.15 (2H, d, J=7.1Hz).
FAB-MS; m/z: 1019 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-2-hydroxy-3-phenylpropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.26 (3H, s), 1.41 (9H, s), 1.47 (3H, s), 1.58 (3H, s), 1.64 (3H, s), 1.76-2.43 (11H, m), 2.27 (3H, s), 2.83 (1H, dd, J=12.7, 4.6Hz), 2.86-2.92 (2H, m), 3.06-3.14 (1H, m), 3.39-3.53 (3H, m), 3.66 (1H, dd, J=12.0, 2.9Hz), 4.10 (1H, d, J=7.3Hz), 4.24 (1H, d, J=8.5Hz), 4.31 (1H, d, J=7.8Hz), 4.63 (1H, s), 4.88 (1H, t, J=4.9Hz), 4.91 (1H, s), 5.21 (1H, d, J=6.8Hz), 5.31 (1H, d, J=9.0Hz), 5.70 (1H, d, J=9.0Hz), 5.98 (1H, d, J=4.9Hz), 6.06 (1H, t, J=9.3Hz), 7.25-7.51 (7H, m), 7.60 (1H, t, J=7.6Hz), 8.12 (2H, d, J=7.1Hz).
FAB-MS; m/z: 905 $(M+H)^+$.

[Example 59]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-4-fluoro-2-hydroxybutyrate

[Formula 131]

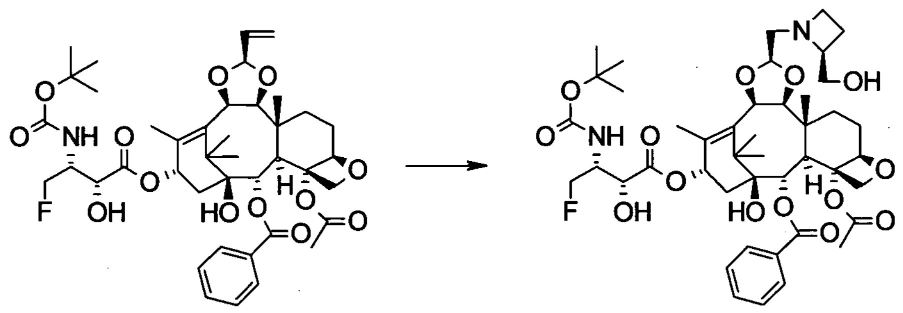

The compound obtained in Example 34, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.22-2.05 (9H, m), 1.23 (3H, s), 1.41 (9H, s), 1.49 (3H, s), 1.59 (3H, s), 1.79 (3H, s), 2.22-2.40 (2H, m), 2.33 (3H, s), 2.83 (1H, dd, J=12.7, 4.6Hz), 2.88-2.93 (2H, m), 3.07-3.13 (1H, m), 3.41-3.52 (3H, m), 3.65 (1H, dd, J=11.4, 3.1Hz), 4.13 (1H, d, J=7.3Hz), 4.24 (1H, d, J=8.5Hz), 4.33 (1H, d, J=8.5Hz), 4.41-4.64 (4H, m), 4.89 (1H, t, J=4.9Hz), 4.93 (1H, s), 5.13 (1H, d, J=9.0Hz), 5.24 (1H, d, J=7.1Hz), 5.99 (1H, d, J=5.1Hz), 6.06 (1H, t, J=8.3Hz), 7.48 (2H, t, J=7.7Hz), 7.59-7.63 (1H, m), 8.12-8.14 (2H, m).
FAB-MS; m/z: 861 $(M+H)^+$.

[Example 60]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-2-hydroxy-3-(2-thiazolyl)propionate

[Formula 132]

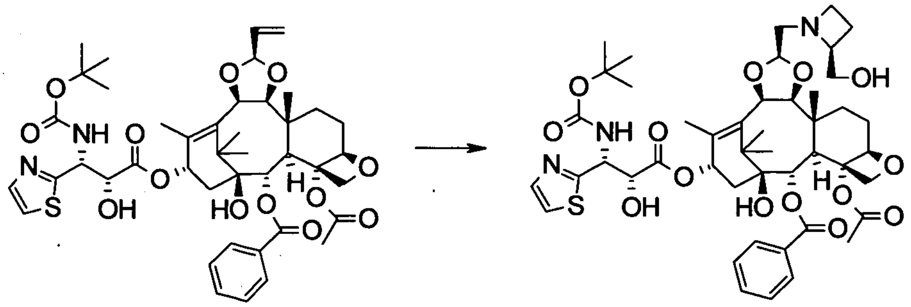

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl
(2R,3R)-3-(tert-butoxycarbonylamino)-2-hydroxy-3-(2-thiazolyl)propionate and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.23-2.37 (11H, m), 1.24 (3H, s), 1.45 (9H, s), 1.49 (3H, s), 1.58 (3H, s), 1.73 (3H, s), 2.34 (3H, s), 2.82 (1H, dd, J=12.9, 4.6Hz), 2.87-2.93 (2H, m), 3.07-3.13 (1H, m), 3.42 (1H, dd, J=11.5, 2.4Hz), 3.46-3.52

(2H, m), 3.65 (1H, dd, J=11.5, 3.1Hz), 4.12 (1H, d, J=7.1Hz), 4.22 (1H, d, J=8.3Hz), 4.32 (1H, d, J=8.3Hz), 4.87 (1H, t, J=5.0Hz), 4.92 (1H, s), 5.00 (1H, d, J=2.4Hz), 5.22 (1H, d, J=6.8Hz), 5.60 (1H, d, J=9.5Hz), 5.91-5.98 (2H, m), 6.12 (1H, t, J=8.3Hz), 7.31 (1H, d, J=3.2Hz), 7.47 (2H, t, J=7.7Hz), 7.58-7.62 (1H, m), 7.76 (1H, d, J=3.2Hz), 8.11-8.14 (2H, m). FAB-MS; m/z: 912 $(M+H)^+$.

[Example 61]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-4,4-dimethyl-2-hydroxypentanoate

[Formula 133]

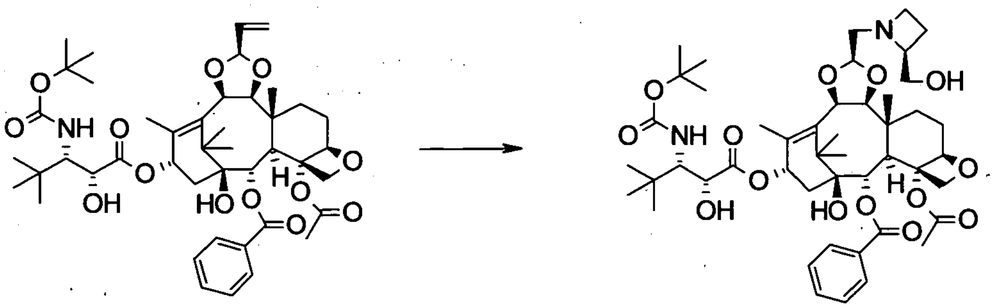

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-4,4-dimethyl-2-hydroxypentanoate and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.02-2.04 (9H, m), 1.05 (9H, s), 1.23 (3H, s), 1.39 (9H, s), 1.49 (3H, s), 1.58 (3H, s), 1.78 (3H, s), 2.22-2.40 (2H, m), 2.34 (3H, s), 2.83 (1H, dd, J=12.7, 4.6Hz), 2.87-2.92 (2H, m), 3.07-3.13 (1H, m), 3.42 (1H, dd, J=11.5, 2.4Hz), 3.46-3.52 (2H, m), 3.66 (1H, dd, J=11.5, 2.9Hz), 3.93 (1H, d, J=10.5Hz), 4.13 (1H, d, J=7.1Hz), 4.24 (1H, d, J=8.5Hz), 4.32 (1H, d, J=8.5Hz), 4.60 (1H, s), 4.89 (1H, t, J=4.9Hz), 4.93 (1H, s), 5.10 (1H, d, J=10.5Hz), 5.23 (1H, d, J=6.8Hz), 5.98 (1H, d, J=5.1Hz), 6.04 (1H, t, J=8.3Hz), 7.48 (2H, t, J=7.7Hz), 7.59-7.63 (1H, m), 8.11-8.14 (2H, m). FAB-MS; m/z: 885 $(M+H)^+$.

[Example 62]

(1S,2S,3R,4S,5R,8R,9S, 10R, 13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-2-hydroxy-3-(2-pyrimidinyl)propionate

[Formula 134]

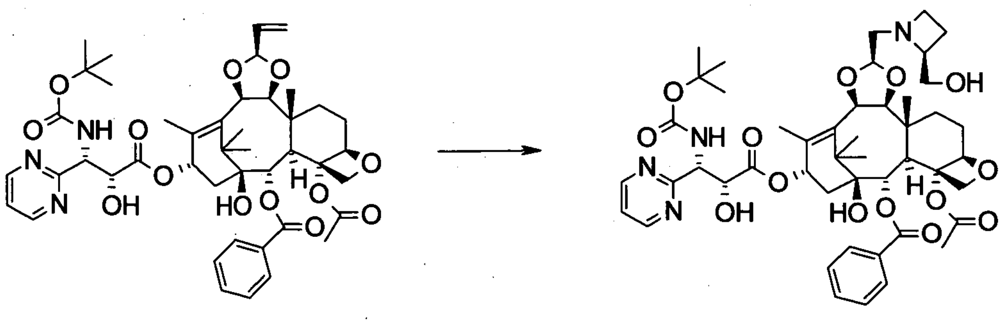

The compound obtained in Example 53, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.27-2.28 (10H, m), 1.28 (3H, s), 1.42 (9H, s), 1.50 (3H, s), 1.60 (3H, s), 1.81 (3H, s), 2.42-2.48 (1H, m), 2.45 (3H, s), 2.83 (1H, dd, J=12.7, 4.6Hz), 2.90 (1H, dd, J=12.7, 5.1Hz), 2.96 (1H, d, J=5.1Hz), 3.11 (1H, dd, J=16.1, 8.5Hz), 3.43 (1H, dd, J=11.5, 2.7Hz), 3.46-3.52 (2H, m), 3.66 (1H, dd, J=11.5, 3.1Hz), 4.16 (1H, d, J=7.3Hz), 4.22 (1H, d, J=8.5Hz), 4.34 (1H, d, J=8.5Hz), 4.88 (1H, t, J=4.9Hz), 4.94 (1H, s), 4.99 (1H, d, J=2.0Hz), 5.24 (1H, d, J=7.1Hz), 5.44 (1H, dd, J=8.8, 1.5Hz), 5.99 (1H, d, J=5.1Hz), 6.07-6.13 (2H, m), 7.24-7.26 (1H, m), 7.47 (2H, t, J=7.7Hz), 7.57-7.61 (1H, m), 8.13-8.15 (2H, m), 8.75 (2H, d, J=4.9Hz).
FAB-MS; m/z: 907 $(M+H)^+$.

[Example 63]

(1S,2S,3R,4S,5R,8R,9S, 10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-2-hydroxypentanoate

[Formula 135]

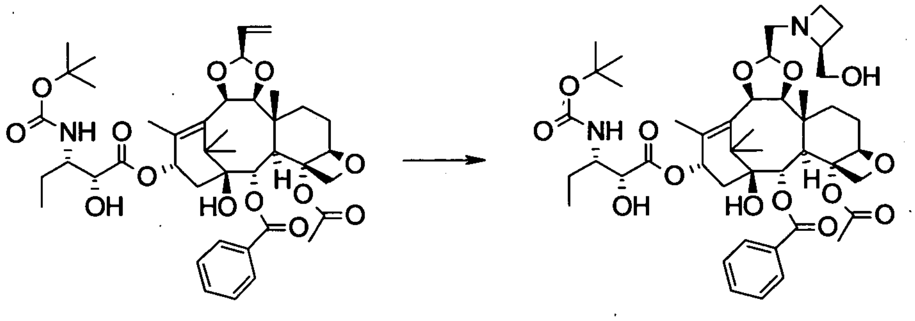

The compound obtained in Example 37, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.99-2.41 (10H, m), 1.01 (3H, t, J=7.4Hz), 1.24 (3H, s), 1.40 (9H, s), 1.49 (3H, s), 1.59 (3H, s), 1.78 (3H, s), 2.33 (3H, s), 2.83 (1H, dd, J=12.8, 4.5Hz), 2.88-2.93 (2H, m), 3.07-3.15 (1H, m), 3.43 (1H, dd, J=11.5, 2.4Hz), 3.47-3.54 (2H, m), 3.66 (1H, dd, J=11.5, 2.9Hz), 3.97-4.05 (1H, m), 4.14 (1H, d, J=7.1Hz), 4.24 (1H, d, J=8.3Hz), 4.31-4.35 (2H, m), 4.83-4.91 (2H, m), 4.93 (1H, s), 5.24 (1H, d, J=6.8Hz), 5.99 (1H, d, J=5.1Hz), 6.02-6.08 (1H, m), 7.48 (2H, t, J=7.7Hz), 7.59-7.63 (1H, m), 8.11-8.15 (2H, m).
FAB-MS; m/z: 857 $(M+H)^+$.

[Example 64]

(1S,2S,3R,4S,5R,8R,9S, 10R, 13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-2-hydroxy-4-methylpentanoate

[Formula 136]

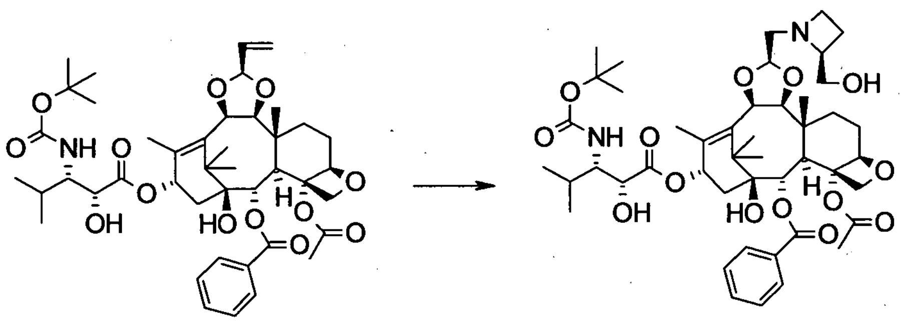

The compound obtained in Example 49, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.00-2.09 (10H, m), 1.02 (3H, d, J=6.8Hz), 1.06 (3H, d, J=6.8Hz), 1.24 (3H, s), 1.38 (9H, s), 1.49 (3H, s), 1.58 (3H, s), 1.79 (3H, s), 2.22-2.40 (2H, m), 2.34 (3H, s), 2.83 (1H, dd, J=12.9, 4.6Hz), 2.87-2.92 (2H, m), 3.10 (1H, dd, J=16.1, 8.3Hz), 3.42 (1H, dd, J=11.5, 2.4Hz), 3.46-3.52 (2H, m), 3.66 (1H, dd, J=11.5, 2.9Hz), 3.76-3.81 (1H, m), 4.14 (1H, d, J=7.1Hz), 4.24 (1H, d, J=8.5Hz), 4.33 (1H, d, J=8.5Hz), 4.49 (1H, d, J=1.5Hz), 4.86-4.93 (3H, m), 5.23 (1H, d, J=7.1Hz), 5.98 (1H, d, J=5.1Hz), 6.04 (1H, t, J=8.8Hz), 7.48 (2H, t, J=7.7Hz), 7.59-7.63 (1H, m), 8.12-8.14 (2H, m).
FAB-MS; m/z: 871 $(M+H)^+$.

[Example 65]

(1S,2S,3R,4S,5R,8R,9S, 10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-4,4-difluoro-2-hydroxy-3-(isopropoxycarbonylamino)butyrate

[Formula 137]

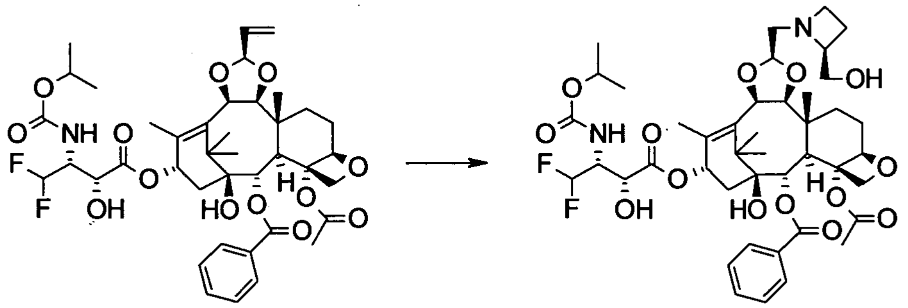

The compound obtained in Example 45, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.19-2.06 (15H, m), 1.23 (3H, s), 1.48 (3H, s), 1.58 (3H, s), 1.75 (3H, s), 2.23-2.37 (2H, m), 2.33 (3H, s), 2.83 (1H, dd, J=12.9, 4.4Hz), 2.88-2.93 (2H, m), 3.11 (1H, dd, J=16.1, 8.3Hz), 3.43 (1H, dd, J=11.4, 2.7Hz), 3.47-3.53 (2H, m), 3.65 (1H, dd, J=11.4, 3.1Hz), 4.12 (1H, d, J=7.1Hz), 4.25 (1H, d, J=8.5Hz), 4.33 (1H, d, J=8.5Hz), 4.56-4.65 (2H, m), 4.88-4.93 (3H, m), 5.23 (1H, d, J=6.8Hz), 5.35 (1H, d, J=10.0Hz), 5.91 (1H, dt, J=56.2, 4.4Hz), 6.00 (1H, d, J=5.1Hz), 6.13 (1H, t, J=8.5Hz), 7.48 (2H, t, J=7.7Hz), 7.59-7.64 (1H, m), 8.11-8.14 (2H, m).
FAB-MS; m/z: 865 $(M+H)^+$.

[Example 66]

[Formula 138]

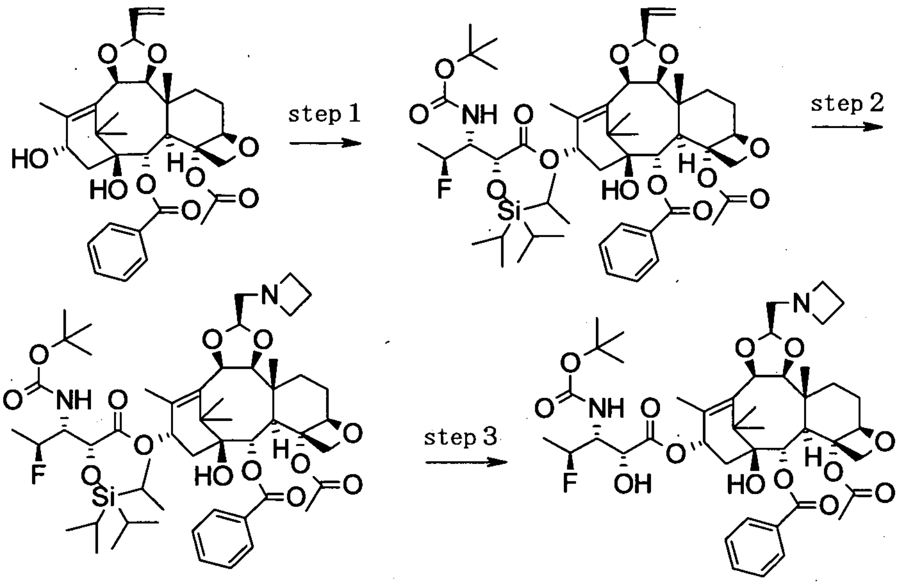


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R,4S)-3-(tert-butoxycarbonylamino)-4-fluoro-2-[(triisopropylsilyl)oxy]pentanoate

(1S,2S,3R,4S,5R,8R,9S,10R,13S)-4-(Acetoxy)-2-(benzoyloxy)-1,13-dihydroxy-5,220-epoxy-9,10-[(1S)-2-propenylidenedioxy]tax-11-ene and the compound obtained in Reference Example 31, Step 4 were used as starting materials to perform the same procedure as that of Example 31, Step 1 and thereby to obtain the title compound as white amorphous solid.
$^1$H-NMR (400MHz, $CDCl_3$) δ : 1.10-1.16 (21H, m), 1.28 (3H, s), 1.37 (9H, s), 1.44 (3H, dd, J=24.4, 6.1Hz), 1.51 (3H, s), 1.64 (3H, s), 1.79 (3H, s), 1.83-2.30 (7H, m), 2.34 (3H, s), 2.93 (1H, d, J=5.1Hz), 4.00-4.09 (1H, m), 4.17-4.22 (2H, m), 4.35 (1H, d, J=8.3Hz), 4.52-4.74 (1H, m), 4.84 (1H, d, J=10.5Hz), 4.90 (1H, s), 4.93 (1H, brs), 5.20 (1H, d, J=6.3Hz), 5.27 (1H, d, J=7.3Hz), 5.45 (1H, d, J=10.7Hz), 5.57 (1H, d, J=17.3Hz), 5.95-6.04 (3H, m), 7.50 (2H, t, J=7.7Hz), 7.61 (1H, t, J=7.6Hz), 8.16 (2H, d, J=7.1Hz). FAB-MS; m/z: 958 $(M+H)^+$.

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R,4S)-3-(tert-butoxycarbonylamino)-4-fluoro-2-[(triisopropylsilyl)oxy]pentanoate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 1.09-1.15 (21H, m), 1.26 (3H, s), 1.37 (9H, s), 1.43 (3H, dd, J=24.6, 6.3Hz), 1.50 (3H, s), 1.61 (3H, s), 1.76 (3H, s), 1.82-2.30 (9H, m), 2.33 (3H, s), 2.73 (1H, dd, J=12.2, 5.6Hz), 2.80 (1H, dd, J=12.2, 4.1Hz), 2.91 (1H, d, J=5.9Hz), 3.32-3.38 (4H, m), 4.00-4.08 (1H, m), 4.13 (1H, d, J=7.3Hz), 4.19 (1H, d, J=8.0Hz), 4.35 (1H, d, J=8.3Hz), 4.52-4.73 (1H, m), 4.81-4.86 (2H, m), 4.89 (1H, s), 4.92 (1H, brs), 5.17 (1H, d, J=7.6Hz), 5.94-6.02 (2H, m), 7.50 (2H, t, J=7.8Hz), 7.61 (1H, t, J=7:3Hz), 8.16 (2H, d, J=8.0Hz).
FAB-MS; m/z: 1001 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R,4S)-3-(tert-butoxycarbonylamino)-4-fluoro-2-hydroxypentanoate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.26 (3H, s), 1.39 (9H, s), 1.45 (3H, dd, J=24.6, 6.3Hz), 1.49 (3H, s), 1.61 (3H, s), 1.77 (3H, s), 1.80-2.16 (10H, m), 2.34 (3H, s), 2.74 (1H, dd, J=12.2, 5.6Hz), 2.83 (1H, dd, J=12.2, 4.4Hz), 2.92 (1H, d, J=4.9Hz), 3.32-3.38 (4H, m), 4.12 (1H, d, J=7.3Hz), 4.17-4.27 (1H, m), 4.24 (1H, d, J=8.0Hz), 4.34 (1H, d, J=8.3Hz), 4.58-4.80 (1H, m), 4.69 (1H, s), 4.87 (1H, t, J=5.1Hz), 4.93 (1H, brs), 4.97 (1H, d, J=10.2Hz), 5.22 (1H, d, J=7.6Hz), 6.00 (1H, d, J=5.4Hz), 6.08 (1H, t, J=8.3Hz), 7.49 (2H, t, J=7.8Hz), 7.61 (1H, t, J=7.3Hz), 8.14 (2H, d, J=8.0Hz).
FAB-MS; m/z: 845 $(M+H)^+$.

[Example 67]

[Formula 139]

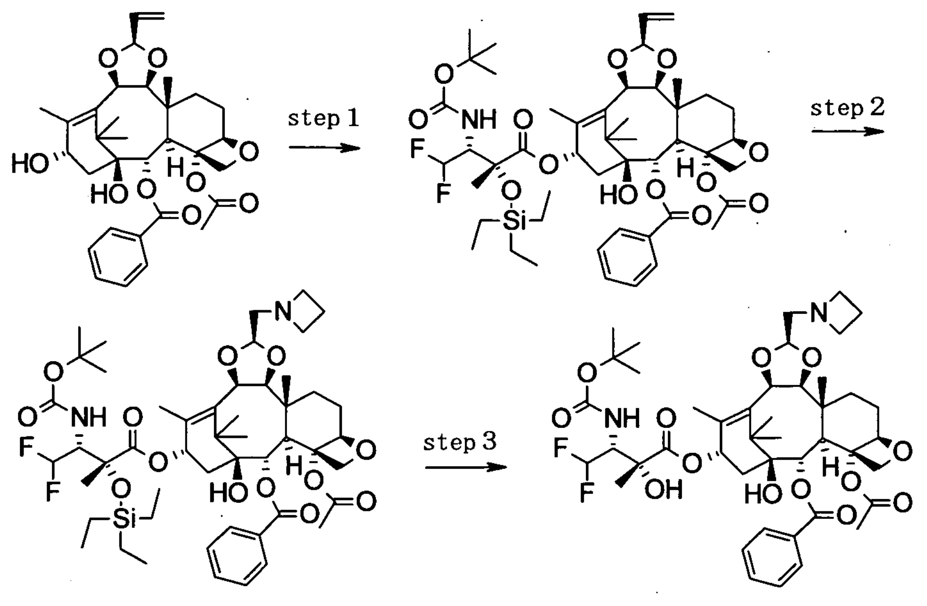

Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4,4-difluoro-2-methyl-2-[(triethylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 32, Step 4 were used as starting materials to perform the same procedure as that of Example 31, Step 1 and thereby to obtain the title compound as white amorphous solid.
$^1$H-NMR (400MHz, $CDCl_3$) δ : 0.62-0.80 (6H, m), 0.98 (9H, t, J=7.8Hz), 1.34 (3H, s), 1.36 (9H, s), 1.51 (3H, s), 1.59 (3H, s), 1.64 (3H, s), 1.72-2.20 (7H, m), 1.75 (3H, s), 2.50 (3H, s), 2.89 (1H, d, J=5.1Hz), 4.15-4.37 (1H, m), 4.18 (1H, d, J=7.3Hz), 4.25 (1H, d, J=8.3Hz), 4.30 (1H, d, J=8.3Hz), 4.90 (1H, s), 4.99 (1H, d, J=10.5Hz), 5.21 (1H, d, J=6.3Hz), 5.25 (1H, d, J=7.1Hz), 5.46 (1H, d, J=10.5Hz), 5.58 (1H, d, J=17.3Hz), 5.86 (1H, dt, J=55.9, 5.1Hz), 5.95-6.05 (2H, m), 6.12 (1H, t, J=9.3Hz), 7.49 (2H, t, J=7.8Hz), 7.60 (1H, t, J=7.6Hz), 8.16 (2H, d, J=7.1Hz).
FAB-MS; m/z: 934 $(M+H)^+$.

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4,4-difluoro-2-methyl-2-[(triethylsilyl)oxy]butyrate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.60-0.80 (6H, m), 0.98 (9H, t, J=7.9Hz), 1.33 (3H, s), 1.37 (9H, s), 1.51 (3H, s), 1.62 (6H, s), 1.70-2.21 (9H, m), 1.73 (3H, s), 2.63 (3H, s), 2.73 (1H, dd, J=12.4, 5.9Hz), 2.81 (1H, dd, J=12.2, 4.4Hz), 2.87 (1H, d, J=5.4Hz), 3.31-3.37 (4H, m), 4.11 (1H, d, J=7.1Hz), 4.24 (1H, d, J=8.5Hz), 4.27-4.36 (2H, m), 4.84 (1H, t, J=5.1Hz), 4.90 (1H, s), 4.98 (1H, d, J=10.7Hz), 5.16 (1H, d, J=7.3Hz), 5.86 (1H, dt, J=55.9, 5.9Hz), 5.94 (1H, d, J=5.9Hz), 6.11 (1H, t, J=8.3Hz), 7.49 (2H, t, J=7.7Hz), 7.61 (1H, t, J=7.3Hz), 8.16 (2H, d, J=7.1Hz).
FAB-MS; m/z: 977 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4,4-difluoro-2-hydroxy-2-methylbutyrate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.28 (3H, s), 1.41 (9H, s), 1.49 (3H, s), 1.62 (3H, s), 1.63 (3H, s), 1.76 (3H, s), 1.83-2.34 (10H, m), 2.63 (3H, s), 2.74 (1H, dd, J=12.4, 5.9Hz), 2.82 (1H, dd, J=12.2, 4.4Hz), 2.94 (1H, d, J=4.9Hz), 3.31-3.40 (4H, m), 4.11 (1H, d, J=7.1Hz), 4.27 (1H, d, J=8.5Hz), 4.30-4.44 (2H, m), 4.87 (1H, t, J=4.6Hz), 4.91 (1H, brs), 5.38 (1H, d, J=10.7Hz), 5.20 (1H, d, J=7.3Hz), 5.99 (1H, d, J=5.1Hz), 6.08 (1H, dt, J=55.9, 3.9Hz), 6.19 (1H, t, J=8.3Hz), 7.49 (2H, t, J=7.7Hz), 7.61 (1H, t, J=7.3Hz), 8.15 (2H, d, J=8.1Hz)
FAB-MS; m/z: 863 $(M+H)^+$.

[Example 68]

[Formula 140]

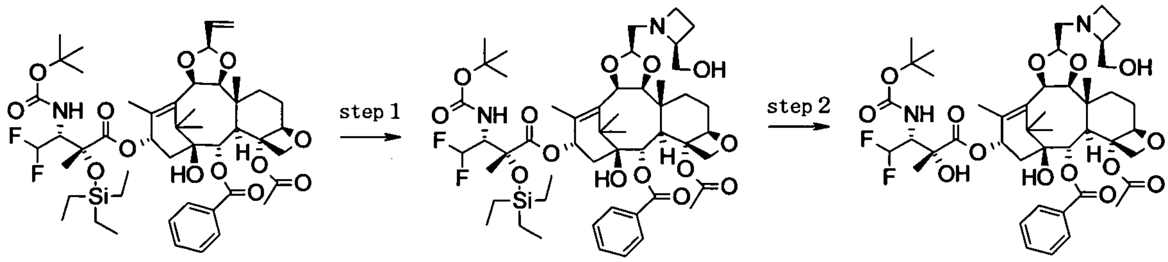


Step 1: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-4,4-difluoro-2-methyl-2-[(triethylsilyl)oxy]butyrate

The compound obtained in Example 67, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.60-0.79 (6H, m), 0.98 (9H, t, J=7.9Hz), 1.32 (3H, s), 1.36 (9H, s), 1.51 (3H, s), 1.53-2.27 (10H, m), 1.59 (3H, s), 1.63 (3H, s), 1.73 (3H, s), 2.63 (3H, s), 2.81 (1H, dd, J=12.7, 4.6Hz), 2.85-2.91 (2H, m), 3.06-3.13 (1H, m), 3.38-3.53 (3H, m), 3.63 (1H, dd, J=11.2, 3.2Hz), 4.12 (1H, d, J=7.1Hz), 4.24 (1H, d, J=8.5Hz), 4.27-4.36 (2H, m), 4.85 (1H, t, J=5.1Hz), 4.90 (1H, s), 4.98 (1H, d, J=10.7Hz), 5.18 (1H, d, J=7.3Hz), 5.86 (1H, dt, J=55.9, 5.9Hz), 5.93 (1H, d, J=5.9Hz), 6.12 (1H, t, J=8.8Hz), 7.49 (2H, t, J=7.7Hz), 7.61 (1H, t, J=7.3Hz), 8.16 (2H, d, J=7.1Hz).
FAB-MS; m/z: 1007 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-4,4-difluoro-2-hydroxy-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.27 (3H, s), 1.42 (9H, s), 1.49 (3H, s), 1.60 (3H, s), 1.61-2.28 (11H, m), 1.63 (3H, s), 1.76 (3H, s), 2.63 (3H, s), 2.82 (1H, dd, J=12.7, 4.6Hz), 2.89(1H, dd, J=12.9, 5.4Hz), 2.94 (1H, d, J=5.1Hz), 3.06-3.13 (1H, m), 3.39-3.52 (3H, m), 3.65 (1H, dd, J=11.2, 3.2Hz), 4.13 (1H, d, J=7.3Hz), 4.26 (1H, d, J=8.5Hz), 4.29-4.44 (2H, m), 4.88 (1H, t, J=5.1Hz), 4.91 (1H, s), 5.34 (1H, d, J=10.7Hz), 5.22 (1H, d, J=7.3Hz), 5.98 (1H, d, J=5.9Hz), 6.07 (1H, dt, J=55.9, 5.9Hz), 6.19 (1H, t, J=8.8Hz), 7.49 (2H, t, J=7.7Hz), 7.61 (1H, t, J=7.3Hz), 8.14 (2H, d, J=7.8Hz).
FAB-MS; m/z: 893 $(M+H)^+$.

[Example 69]

[Formula 141]

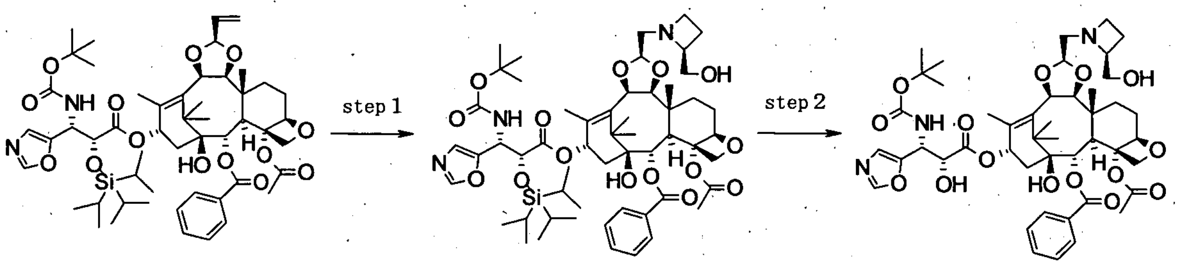

Step 1: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioXy}tax-11-en-13-yl

(2R,3R)-3-(tert-butoxycarbonylamino)-3-(5-oxazolyl)-2-[(triisopropylsilyl)oxy]propionate

The compound obtained in Example 55, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.94-1.07 (21H, m), 1.28 (3H, s), 1.40 (9H, s), 1.51 (3H, s), 1.59 (3H, s), 1.71-2.37 (9H, m), 1.78 (3H, s), 2.41 (3H, s), 2.81 (1H, dd, J=12.5, 4.6Hz), 2.90 (1H, dd, J=12.9, 4.9Hz), 2.94 (1H, d, J=5.9Hz), 3.06-3.14 (1H, m), 3.39-3.53 (3H, m), 3.64 (1H, dd, J=12.0, 3.2Hz), 4.16 (1H, d, J=7.3Hz), 4.21 (1H, d, J=8.3Hz), 4.34 (1H, d, J=8.5Hz), 4.85 (1H, t, J=4.9Hz), 4.94 (2H, brs), 5.21 (1H, d, J=7.8Hz), 5.30 (1H, d, J=10.3Hz), 5.39 (1H, d, J=10.0Hz), 5.96 (1H, d, J=5.4Hz), 6.07 (1H, t, J=8.5Hz), 7.02 (1H, s), 7.48 (2H, t, J=7.6Hz), 7.60 (1H, t, J=7.8Hz), 7.87 (1H, s), 8.14 (2H, d, J=8.1Hz). FAB-MS; m/z: 1052 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-2-hydroxy-3-(5-oxazolyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.26 (3H, s), 1.41 (9H, s), 1.47 (3H, s), 1.58 (3H, s), 1.64 (3H, s), 1.76-2.43 (11H, m), 2.27 (3H, s), 2.83 (1H, dd, J=12.7, 4.6Hz), 2.86-2.92 (2H, m), 3.06-3.14 (1H, m), 3.39-3.53 (3H, m), 3.66 (1H, dd, J=12.0, 2.9Hz), 4.10 (1H, d, J=7.3Hz), 4.24 (1H, d, J=8.5Hz), 4.31 (1H, d, J=7.8Hz), 4.63 (1H, s), 4.88 (1H, t, J=4.9Hz), 4.91 (1H, s), 5.21 (1H, d, J=6.8Hz), 5.31 (1H, d, J=9.0Hz), 5.70 (1H, d, J=9.0Hz), 5.98 (1H, d, J=4.9Hz), 6.06 (1H, t, J=9.3Hz), 7.07 (1H, s), 7.48 (2H, t, J=7.7Hz), 7.61 (1H, t, J=7.6Hz), 7.85 (1H, s), 8.13 (2H, d, J=8.1Hz).
FAB-MS; m/z: 896 $(M+H)^+$.

[Example 70]

[Formula 142]

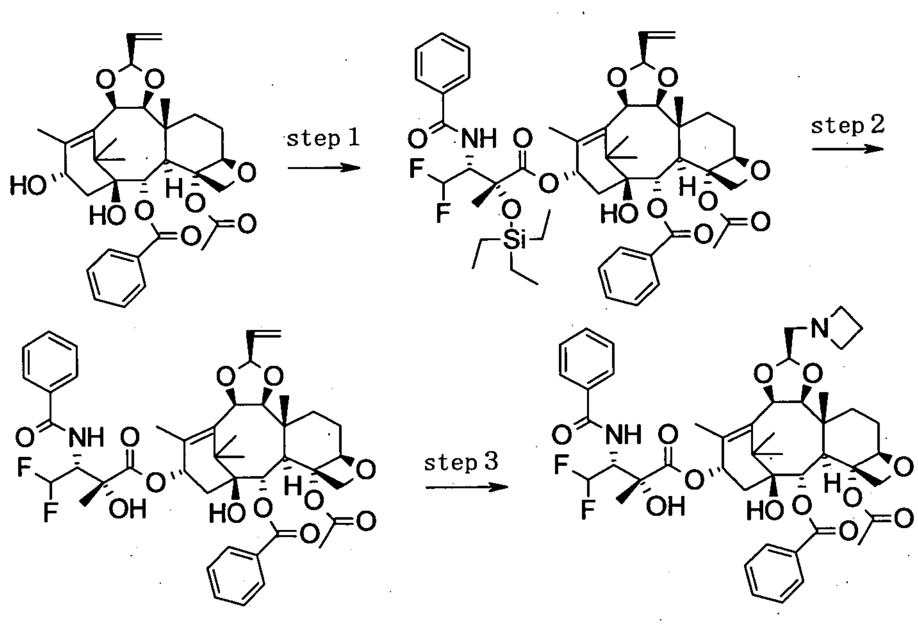

Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(benzoylamino)-4,4-difluoro-2-methyl-2-[(triethylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 33 were used as starting materials to perform the same procedure as that of Example 31, Step 1 and thereby to obtain the title compound as white amorphous solid, and used as it was for the following reaction.

Step 2: (1S,2S,3R,4S,5R,8R,9S, 10R, 13S)-4-(Acetoxy)-2-(benzoyloxy)-5,20-epoxy-1-hydroxy-9,10-[(1S)-2-propenylidenedioxy]tax-11-en-13-yl

(2R,3R)-3-(benzoylamino)-4,4-difluoro-2-hydroxy-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.27 (3H, s), 1.49 (3H, s), 1.64 (3H, s), 1.67 (3H, s), 1.75 (3H, s), 1.81-2.30 (8H, m), 2.45 (3H, s), 2.97 (1H, d, J=4.9Hz), 4.17 (1H, d, J=7.1Hz), 4.28 (1H, d, J=8.8Hz), 4.34 (1H, d, J=8.5Hz), 4.86 (1H, s), 4.93 (1H, s), 4.95-5.05 (1H, m), 5.24 (1H, d, J=6.3Hz), 5.27 (1H, d, J=7.3Hz), 5.47 (1H, d, J=10.5Hz), 5.58 (1H, d, J=17.3Hz), 5.95-6.06 (2H, m), 6.23 (1H, t, J=8.8Hz), 6.30 (1H, dt, J=55.9, 2.7Hz), 7.43-7.65 (6H, m), 7.84 (2H, d, J=8.1Hz), 8.15 (2H, d, J=8.1Hz).
FAB-MS; m/z: 824 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(benzoylamino)-4,4-difluoro-2-hydroxy-2-methylbutyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.26 (3H, s), 1.48 (3H, s), 1.61 (3H, s), 1.67 (3H, s), 1.73 (3H, s), 1.79-2.27 (10H, m), 2.44 (3H, s), 2.74 (1H, dd, J=12.4, 5.9Hz), 2.82 (1H, dd, J=12.2, 4.4Hz), 2.95 (1H, d, J=4.9Hz), 3.31-3.40 (4H, m), 4.10 (1H, d, J=7.1Hz), 4.28 (1H, d, J=8.5Hz), 4.34 (1H, d, J=8.5Hz), 4.79-4.89 (1H, m), 4.86 (1H, t, J=4.6Hz), 4.93 (1H, brs), 4.95-5.06 (1H, m), 5.18 (1H, d, J=7.3Hz), 6.01 (1H, d, J=5.1Hz), 6.22 (1H, t, J=8.3Hz), 6.29 (1H, dt, J=55.9, 3.9Hz), 7.42-7.57 (7H, m), 7.62 (1H, t, J=7.3Hz), 8.15 (2H, d, J=8.1Hz).
FAB-MS; m/z: 867 $(M+H)^+$.

[Example 71]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(benzoylamino)-4,4-difluoro-2-hydroxy-2-methylbutyrate

[Formula 143]

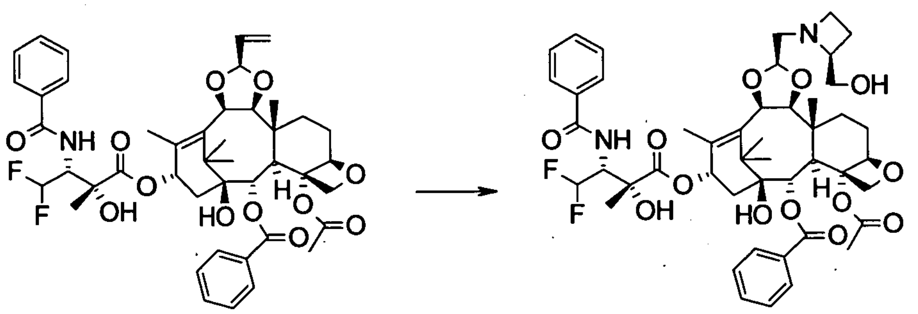

The compound obtained in Example 70, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) $\delta$ : 1.25 (3H, s), 1.48 (3H, s), 1.59 (3H, s), 1.67 (3H, s), 1.73 (3H, s), 1.78-2.31 (11H, m), 2.45 (3H, s), 2.82 (1H, dd, J=12.9, 4.9Hz), 2.90 (1H, dd, J=12.9, 5.1Hz), 2.95 (1H, d, J=4.9Hz), 3.06-3.14 (1H, m), 3.39-3.54 (3H, m), 3.64 (1H, dd, J=11.5, 2.7Hz), 4.11 (1H, d, J=7.1Hz), 4.27 (1H, d, J=8.5Hz), 4.33 (1H, d, J=8.5Hz), 4.73-4.85 (1H, m), 4.88 (1H, t, J=4.6Hz), 4.93 (1H, brs), 4.95-5.05 (1H, m), 5.20 (1H, d, J=7.3Hz), 5.99 (1H, d, J=5.1Hz), 6.21 (1H, t, J=8.3Hz), 6.29 (1H, dt, J=55.9, 2.4Hz), 7.43-7.57 (7H, m), 7.62 (1H, t, J=7.3Hz), 8.15 (2H, d, J=8.1Hz).
FAB-MS; m/z: 897 $(M+H)^+$.

[Example 72]

[Formula 144]

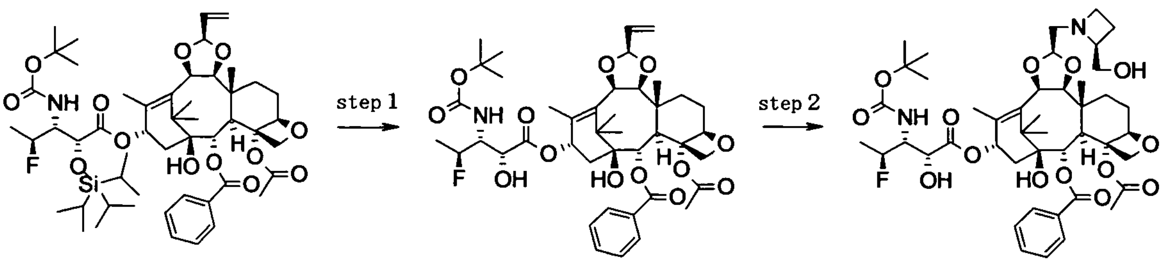


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R,4S)-3-(tert-butoxycarbonylamino)-4-fluoro-2-hydroxypentanoate

The compound obtained in Example 66, Step 1 was used as a starting material to perform the same procedure as that of Example 31, Step 2 and thereby to obtain the title compound as white amorphous solid.
$^1$H-NMR (400MHz, $CDCl_3$) $\delta$ : 1.24-2.10 (10H, m), 1.26 (3H, s), 1.40 (9H, s), 1.56 (3H, s), 1.64 (3H, s), 1.80 (3H, s), 2.33-2.39 (1H, m), 2.35 (3H, s), 2.94 (1H, d, J=4.9Hz), 4.18-4.25 (3H, m), 4.34 (1H, d, J=8.3Hz), 4.60-4.79 (2H, m), 4.94-4.97 (2H, m), 5.25 (1H, d, J=6.1Hz), 5.31 (1H, d, J=7.1Hz), 5.46 (1H, d, J=10.5Hz), 5.58 (1H, d, J=17.1Hz), 5.97-6.05 (2H, m), 6.10 (1H, t, J=8.2Hz), 7.48 (2H, t, J=7.8Hz), 7.61 (1H, t, J=7.3Hz), 8.12-8.15 (2H, m).
FAB-MS; m/z: 802 $(M+H)^+$, 824 $(M+Na)^+$.

Step 2: (1S,2S,3R,4S,5R,8R,9S, 10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R,4S)-3-(tert-butoxycarbonylamino)-4-fluoro-2-hydroxypentanoate

The compound obtained in Step 1 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.23-2.38 (14H, m), 1.24 (3H, s), 1.40 (9H, s), 1.49 (3H, s), 1.59 (3H, s), 1.77 (3H, s), 2.34 (3H, s), 2.83 (1H, dd, J=12.7, 4.6Hz), 2.88-2.93 (2H, m), 3.11 (1H, dd, J=16.4, 8.5Hz), 3.43 (1H, dd, J=11.5, 2.4Hz), 3.47-3.53 (2H, m), 3.66 (1H, dd, J=11.5, 2.7Hz), 4.13 (1H, d, J=7.1Hz), 4.18-4.24 (2H, m), 4.33 (1H, d, J=8.5Hz), 4.59-4.78 (2H, m), 4.89 (1H, t, J=4.6Hz), 4.93 (1H, s), 4.99 (1H, d, J=10.3Hz), 5.24 (1H, d, J=7.1Hz), 5.99 (1H, d, J=5.1Hz), 6.08 (1H, t, J=8.5Hz), 7.49 (2H, t, J=7.7Hz), 7.61 (1H, t, J=7.3Hz), 8.13 (2H, d, J=7.3Hz).
FAB-MS; m/z: 875 $(M+H)^+$.

[Example 73]

[Formula 145]

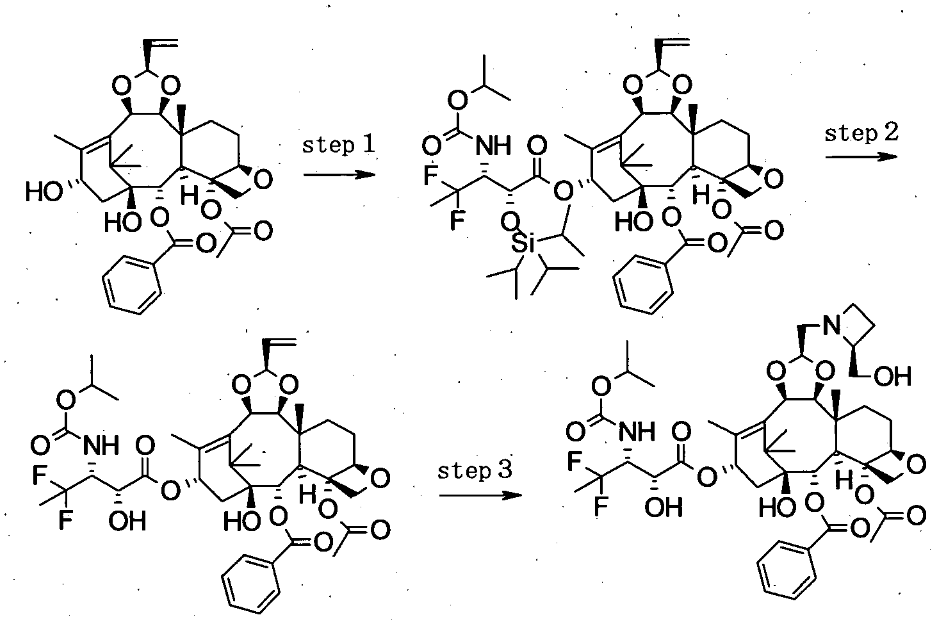


Step 1: (1S,2S,3R,4S,5R,8R,9S,10R,13S)-4-(Acetoxy)-2-(benzoyloxy)-5,20-epoxy-1-hydroxy-9,20-[(1S)-2-propenylidenedioxy]tax-11-en-13-yl

(2R,3R)-4,4-difluoro-3-(isopropoxycarbonylamino)-2-[(triisopropylsilyl)oxy]pentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 34 were used as starting materials to perform the same procedure as that of Example 31, Step 1 and thereby to obtain the title compound as white amorphous solid.
$^1$H-NMR (400MHz, $CDCl_3$) δ : 0.96-2.32 (13H, m), 1.07-1.15 (21H, m), 1.31 (3H, s), 1.51 (3H, s), 1.64 (3H, s), 1.69 (3H, t, J=18.6Hz), 1.79 (3H, s), 2.31 (3H, s), 2.93 (1H, d, J=4.9Hz), 4.20 (2H, d, J=7.1Hz), 4.26-4.37 (2H, m), 4.77-4.85 (1H, m), 4.93 (1H, s), 5.03 (1H, s), 5.18-5.28 (3H, m), 5.45 (1H, d, J=10.5Hz), 5.57 (1H, d, J=17.2Hz), 5.94-6.05 (3H, m), 7.49 (2H, t, J=7.7Hz), 7.58-7.63 (1H, m), 8.12-8.17 (2H, m).

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-4,4-difluoro-2-hydroxy-3-(isopropoxycarbonylamino)pentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.13-2.42 (11H, m), 1.25 (3H, s), 1.49 (3H, s), 1.64 (3H, s), 1.72 (3H, t, J=18.8Hz), 1.76 (3H, s), 2.35 (3H, s), 2.94 (1H, d, J=4.9Hz), 4.17 (1H, d, J=6.8Hz), 4.25 (1H, d, J=8.5Hz), 4.34 (1H, d, J=8.5Hz), 4.59-4.72 (2H, m), 4.78 (1H, s), 4.88-4.98 (2H, m), 5.25 (1H, d, J=6.1Hz), 5.30 (1H, d, J=6.8Hz), 5.42 (1H, d, J=10.2Hz), 5.47 (1H, d, J=10.5Hz), 5.58 (1H, d, J=17.1Hz), 5.96-6.06 (2H, m), 6.13-6.20 (3H, m), 7.48 (2H, t, J=7.7Hz), 7.59-7.64 (1H; m), 8.11-8.16 (2H, m).

Step 3: (1S,2S,3R,4S,5R,8R,9S,10R,13S)-4-(Acetoxy)-2-(benzoyloxy-5,20-epoxy-1-hydroxy-9,10-{(1S)-2-[(2S)-2-hydroxymethylazetidino]ethylidenedioxy}tax-11-en-13-yl

(2R,3R)-4,4-difluoro-2-hydroxy-3-(isopropoxycarbonylamino)pentanoate

The compound obtained in Step 2 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.12-2.44 (17H, m), 1.23 (3H, s), 1.48 (3H, s), 1.58 (3H, s), 1.72 (3H, t, J=18.8Hz), 1.74 (3H, s), 2.34 (3H, s), 2.84 (1H, dd, J=12.7, 4.4Hz), 2.87-2.94 (2H, m), 3.11 (1H, q , J=8.2Hz), 3.40-3.46 (1H, m), 3.46-3.55 (2H, m), 3.62-3.69 (1H, m), 4.12 (1H, d, J=7.3Hz), 4.24 (1H, d, J=8.3Hz), 4.33 (1H, d, J=8.3Hz), 4.58-4.67 (1H, m), 4.77 (1H, s), 4.86-4.95 (3H, m), 5.22 (1H, d, J=7.1Hz), 5.43 (1H, d, J=10.5Hz), 5.99 (1H, d, J=5.1Hz), 6.14 (1H, t, J=8.3Hz), 7.48 (2H, t, J=7.7Hz), 7.58-7.64 (1H, m), 8.11-8.15 (2H, m).
FAB-MS; m/z: 879 (M+H)+.

[Example 74]

[Formula 146]

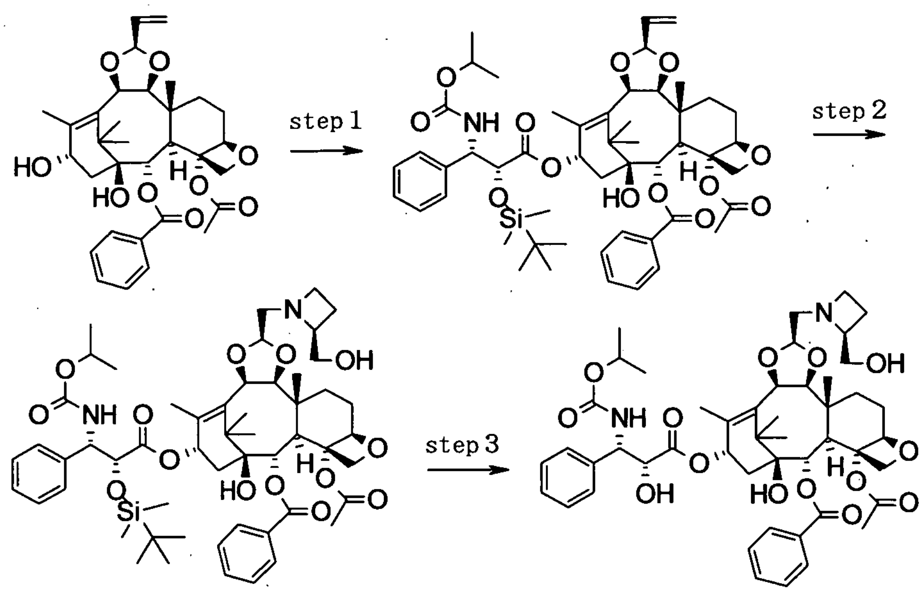

Step 1: (1S,2S,3R,4S,5R,8R,9S,10R, 13S)-4-(Acetoxy)-2-(benzoyloxy)-5,20-epoxy-1-hydroxy-9,10-[(1S)-2-propenylidenedioxy]tax-11-en-13-yl

(2R,3S)-2-[(tert-butyldimethylsilyl)oxy]-3-(isopropoxycarbonylamino)-3-phenylpropionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 35 were used as starting materials to perform the same procedure as that of Example 31, Step 1 and thereby to obtain the title compound as white amorphous solid.
$^1$H-NMR (400MHz, $CDCl_3$) δ : -0.35 (3H, s), -0.09 (3H, s), 0.74 (9H, s), 1.16-1.23 (6H, m), 1.35 (3H, s), 1.52 (3H, s), 1.66 (3H, s), 1.77 (3H, s), 1.83-2.31 (7H, m), 2.50 (3H, s), 2.94 (1H, d, J=5.1Hz), 4.21 (1H, d, J=7.6Hz), 4.24 (1H, d, J=8.8Hz), 4.35 (1H, d, J=8.8Hz), 4.58 (1H, s), 4.79-4.88 (1H, m), 4.98 (1H, s), 5.22 (1H, d, J=6.1Hz), 5.27 (1H, d, J=7.1Hz), 5.37 (1H, brs), 5.46 (1H, d, J=10.2Hz), 5.52-5.58 (1H, m), 5.58 (1H, d, J=17.3Hz), 5.96-6.05 (2H, m), 6.24 (1H, t, J=9.0Hz), 7.22-7.52 (7H, m), 7.60 (1H, t, J=7.6Hz), 8.15 (2H, d, J=7.3Hz).
FAB-MS; m/z: 932 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-2-[(tert-butyldimethylsilyl)oxy]-3-(isopropoxycarbonylamino)-3-phenylpropionate

The compound obtained in Step 1 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : -0.35 (3H, s), -0.09 (3H, s), 0.74 (9H, s), 1.16-1.22 (6H, m), 1.33 (3H, s), 1.51 (3H, s), 1.60 (3H, s), 1.75 (3H, s), 1.83-2.32 (10H, m), 2.50 (3H, s), 2.81 (1H, dd, J=12.9, 4.9Hz), 2.90 (1H, dd, J=12.7, 5.1Hz), 2.93 (1H, d, J=5.1Hz), 3.06-3.14 (1H, m), 3.39-3.53 (3H, m), 3.63 (1H, dd, J=11.5, 3.2Hz), 4.16 (1H, d, J=7.3Hz), 4.24 (1H, d, J=8.5Hz), 4.34 (1H, d, J=8.8Hz), 4.57 (1H, s), 4.79-4.88 (2H, m), 4.98 (1H, s), 5.19 (1H, d, J=7.3Hz), 5.35 (1H, d, J=8.5Hz), 5.55 (1H, d, J=9.5Hz), 5.97 (1H, d, J=5.4Hz), 6.23 (1H, t, J=9.0Hz), 7.25-7.41 (5H, m), 7.48 (2H, t, J=7.6Hz), 7.60 (1H, t, J=7.6Hz), 8.15 (2H, d, J=7.3Hz).
FAB-MS; m/z: 1005 $(M+H)^+$.

Step 3: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-2-hydroxy-3-(isopropoxycarbonylamino)-3-phenylpropionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.18-1.21 (6H, m), 1.23 (3H, s), 1.47 (3H, s), 1.58 (3H, s), 1.63 (3H, s), 1.70-2.40 (11H, m), 2.27 (3H, s), 2.82 (1H, dd, J=12.9, 4.9Hz), 2.85-2.91 (2H, m), 3.05-3.13 (1H, m), 3.39-3.53 (3H, m), 3.64 (1H, dd, J=11.5, 3.2Hz), 4.09 (1H, d, J=7.3Hz), 4.23 (1H, d, J=8.5Hz), 4.31 (1H, d, J=8.8Hz), 4.63 (1H, s), 4.82-4.90 (2H, m), 4.91 (1H, s), 5.20 (1H, d, J=7.3Hz), 5.36 (1H, d, J=8.5Hz), 5.81 (1H, d, J=9.5Hz), 5.98 (1H, d, J=5.1Hz), 6.08 (1H, t, J=9.0Hz), 7.25-7.49 (7H, m), 7.60 (1H, t, J=7.6Hz), 8.11 (2H, d, J=7.3Hz).
FAB-MS; m/z: 891 $(M+H)^+$.

[Example 75]

[Formula 147]

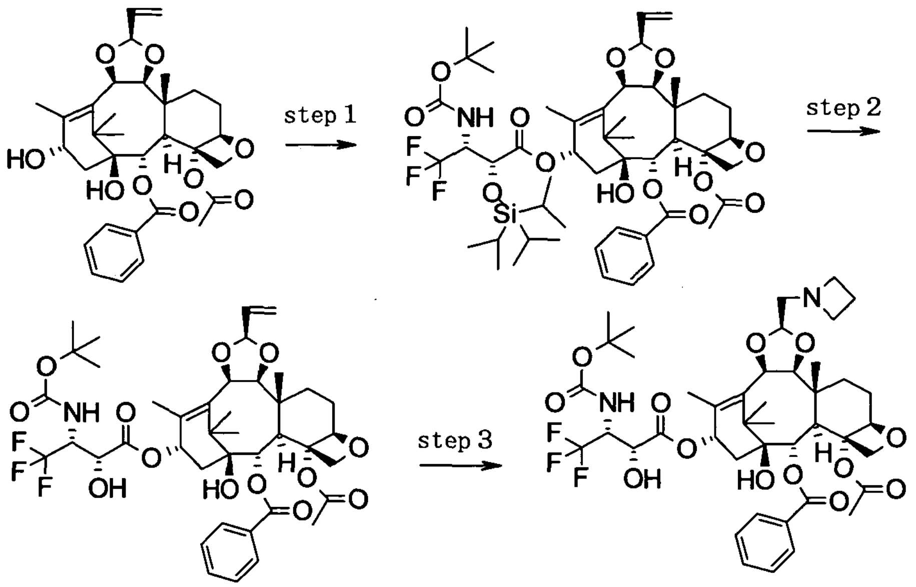


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-4,4,4-trifluoro-2-[(triisopropylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and (3R,4R)-1-(tert-butoxycarbonyl)-4-(trifluoromethyl)-3-[(triisopropylsilyl)oxy]-2-azetidinone (J. Fluorine. Chem., 2004, 125, 487) were used as starting materials to perform the same procedure as that of Example 31, Step 1 and thereby to obtain the title compound as white amorphous solid.
$^1$H-NMR (400MHz, $CDCl_3$) δ : 1.05-2.33 (8H, m), 1.07-1.15 (21H, m), 1.29 (3H, s), 1.39 (3H, s), 1.53 (9H, s), 1.64 (3H, s), 1.78 (3H, s), 2.32 (3H, s), 2.93 (1H, d, J=5.1Hz), 4.18-4.23 (2H, m), 4.34 (1H, d, J=9.0Hz), 4.61-4.69 (1H, m), 4.88-4.94 (1H, m), 4.96 (1H, s), 5.14-5.22 (2H, m), 5.26 (1H, d, J=7.1Hz), 5.45 (1H, d, J=10.5Hz), 5.57 (1H, d, J=17.3Hz), 5.95-6.05 (2H, m), 7.50 (2H, t, J=7.8Hz), 7.58-7.64 (1H, m), 8.13-8.17 (2H, m).

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(tert-butoxycarbonylamino)-2-hydroxy-4,4,4-trifluorobutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.23-2.39 (7H, m), 1.25 (3H, s), 1.43 (9H, s), 1.55 (3H, s), 1.64 (3H, s), 1.77 (3H, s), 2.34 (3H, s), 2.94 (1H, d, J=4.9Hz), 4.17 (1H, d, J=7.1Hz), 4.26 (1H, d, J=8.5Hz), 4.34 (1H, d, J=8.3Hz), 4.72 (1H, s), 4.82 (1H, s), 4.87-4.95 (2H, m), 5.26 (1H, d, J=6.1Hz), 5.31 (1H, d, J=6.6Hz), 5.36 (1H, d, J=10.5Hz), 5.47 (1H, d, J=10.5Hz), 5.58 (1H, d, J=17.3Hz), 5.95-6.06 (2H, m), 6.13-6.20 (1H, m), 7.48 (2H, t, J=7.8Hz), 7.59-7.64 (1H, m), 8.11-8.15 (2H, m).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-2-hydroxy-4,4,4-trifluorobutyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.22-2.37 (10H, m), 1.23 (3H, s), 1.42 (9H, s), 1.49 (3H, s), 1.61 (3H, s), 1.75 (3H, s),

2.34 (3H, s), 2.75 (1H, dd, J=12.6, 6.0Hz), 2.84 (1H, dd, J=12.4, 4.1Hz), 2.92 (1H, d, J=4.9Hz), 3.33-3.41 (4H, m), 4.11 (1H, d, J=7.1Hz), 4.25 (1H, d, J=8.5Hz), 4.34 (1H, d, J=8.0Hz), 4.72 (1H, s), 4.84-4.94 (3H, m), 5.22 (1H, d, J=7.1Hz), 5.39 (1H, d, J=10.5Hz), 6.01 (1H, d, J=4.9Hz), 6.10-6.18 (1H, m), 7.49 (2H, t, J=7.7Hz), 7.59-7.64 (1H, m), 8.11-8.15 (2H, m).
FAB-MS; m/z: 867 $(M+H)^+$.

[Example 76]

(1S,2S,3R,4S,5R,8R,9S,10R, 13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-2-hydroxy-4,4,4-trifluorobutyrate

[Formula 148]

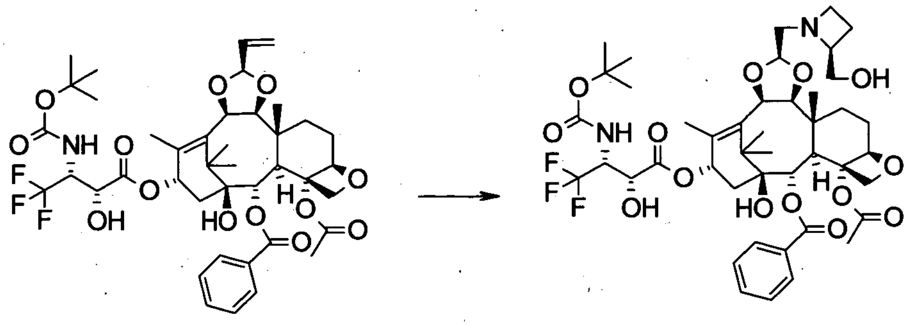

The compound obtained in Example 75, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.22 (3H, s), 1.22-2.37 (11H, m), 1.43 (9H, s), 1.48 (3H, s), 1.59 (3H, s), 1.75 (3H, s), 2.34 (3H, s), 2.84 (1H, dd, J=12.9, 4.4Hz), 2.88-2.94 (2H, m), 3.06-3.16 (1H, m), 3.43 (1H, dd, J=11.5, 2.4Hz), 3.46-3.54 (2H, m), 3.63-3.69 (1H, m), 4.12 (1H, d, J=6.8Hz), 4.25 (1H, d, J=8.5Hz), 4.34 (1H, d, J=8.3Hz), 4.72 (1H, s), 4.85-4.95 (3H, m), 5.23 (1H, d, J=7.1Hz), 5.38 (1H, d, J=10.7Hz), 6.00 (1H, d, J=4.9Hz), 6.09-6.18 (1H, m), 7.49 (2H, t, J=7.7Hz), 7.58-7.64 (1H, m), 8.11-8.15 (2H, m).
FAB-MS; m/z: 897 $(M+H)^+$.

[Example 77]

[Formula 149]

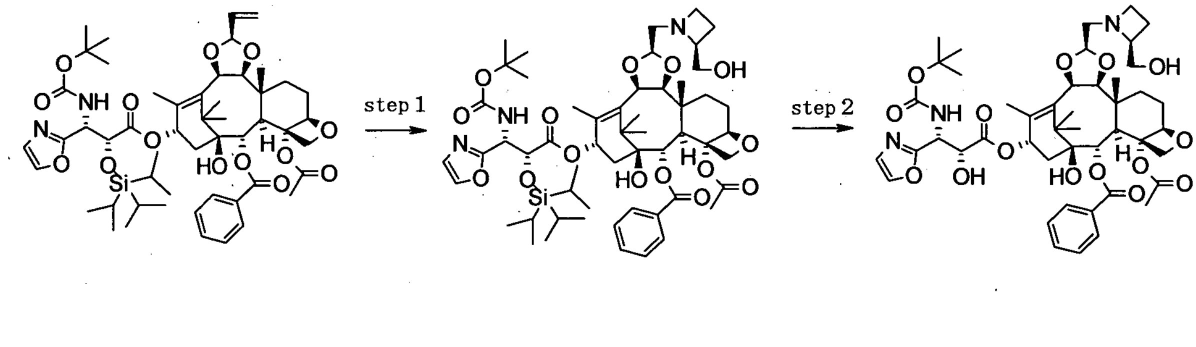

Step 1: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-3-(2-oxazolyl)-2-[(triisopropylsilyl)oxy]propionate

The compound obtained in Example 41, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.94-1.01 (21H, m), 1.29 (3H, s), 1.39 (9H, s), 1.51 (3H, s), 1.59 (3H, s), 1.74-2.39 (10H, m), 1.78 (3H, s), 2.46 (3H, s), 2.81 (1H, dd, J=12.5, 4.6Hz), 2.89 (1H, dd, J=12.9, 4.9Hz), 2.95 (1H, d, J=5.9Hz), 3.06-3.14 (1H, m), 3.39-3.53 (3H, m), 3.63 (1H, dd, J=11.2, 3.2Hz), 4.16 (1H, d, J=7.3Hz), 4.21 (1H, d, J=8.3Hz), 4.34 (1H, d, J=8.5Hz), 4.85 (1H, t, J=4.9Hz), 4.94 (2H, brs), 5.20 (1H, d, J=6.8Hz), 5.44 (1H, d, J=10.3Hz), 5.53 (1H, d, J=10.0Hz), 5.96 (1H, d, J=5.4Hz), 6.08 (1H, t, J=8.5Hz), 7.11 (1H, s), 7.47 (2H, t, J=7.6Hz), 7.59 (1H, t, J=7.8Hz), 7.64 (1H, s), 8.14 (2H, d, J=8.1Hz). FAB-MS; m/z: 1052 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonylamino)-2-hydroxy-3-(2-oxazolyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.26 (3H, s), 1.43 (9H, s), 1.49 (3H, s), 1.59 (3H, s), 1.74-2.43 (11H, m), 1.79 (3H, s), 2.34 (3H, s), 2.82 (1H, dd, J=12.5, 4.6Hz), 2.89 (1H, dd, J=12.9, 4.9Hz), 2.93 (1H, d, J=5.9Hz), 3.06-3.14 (1H, m), 3.39-3.53 (3H, m), 3.65 (1H, dd, J=11.0, 3.2Hz), 4.13 (1H, d, J=7.3Hz), 4.23 (1H, d, J=8.3Hz), 4.33 (1H, d, J=8.5Hz), 4.84 (1H, d, J=2.4Hz), 4.88 (1H, t, J=4.9Hz), 4.92 (1H, brs), 5.24 (1H, d, J=6.8Hz), 5.49 (1H, d, J=10.3Hz), 5.71 (1H, d, J=10.0Hz), 5.99 (1H, d, J=5.4Hz), 6.12 (1H, t, J=8.5Hz), 7.11 (1H, s), 7.47 (2H, t, J=7.6Hz), 7.60 (1H, t, J=7.3Hz), 7.67 (1H, s), 8.13 (2H, d, J=8.1Hz).
FAB-MS; m/z: 896 $(M+H)^+$.

[Example 78]

[Formula 150]

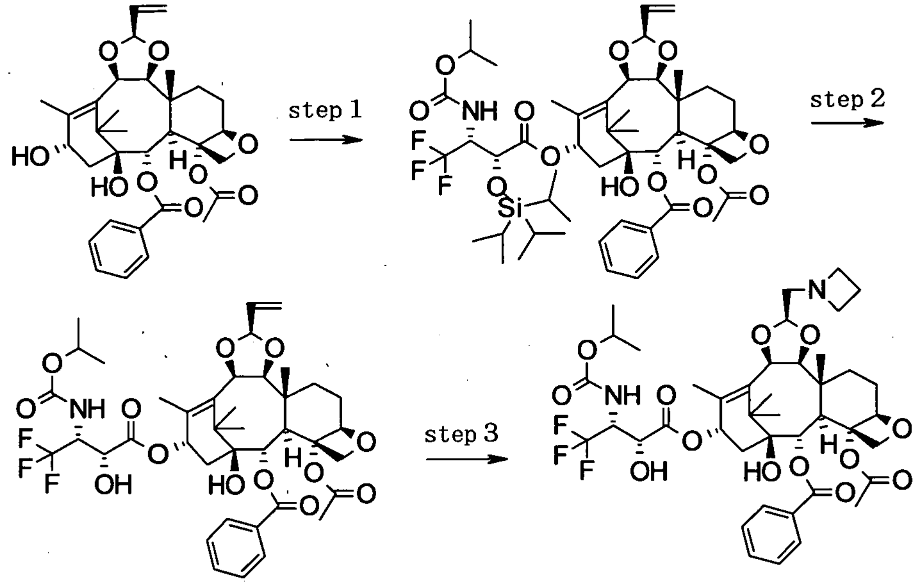


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-3-(isopropoxycarbonylamino)-4,4,4-trifluoro-2-[(triisopropylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 36 were used as starting materials to perform the same procedure as that of Example 31, Step 1 and thereby to obtain the title compound as white amorphous solid.
$^1$H-NMR (400MHz, $CDCl_3$) δ : 1.04-1.16 (21H, m), 1.05-2.33 (7H, m), 1.18 (3H, d, J=6.1Hz), 1.22 (3H, d, J=6.4Hz), 1.31 (3H, s), 1.51 (3H, s), 1.65 (3H, s), 1.78 (3H, s), 2.32 (3H, s), 2.92 (1H, d, J=5.1Hz), 4.18-4.23 (2H, m), 4.34 (1H, d, J=8.3Hz), 4.65-4.75 (1H, m), 4.79-4.87 (1H, m), 4.92 (1H, s), 4.96-4.98 (1H, m), 5.20 (1H, d, J=6.1Hz), 5.23-5.28 (2H, m), 5.46 (1H, d, J=10.8Hz), 5.57 (1H, d, J=17.4Hz), 5.95-6.09 (3H, m), 7.50 (2H, t, J=7.8Hz), 7.58-7.64 (1H, m), 8.13-8.17 (2H, m).

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-2-hydroxy-3-(isopropoxycarbonylamino)-4,4,4-trifluorobutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.16-2.37 (12H, m), 1.24 (3H, s), 1.55 (3H, s), 1.64 (3H, s), 1.75 (3H, s), 2.35 (3H, s), 2.94 (1H, d, J=5.4Hz), 4.16 (1H, d, J=6.9Hz), 4.26 (1H, d, J=8.6Hz), 4.34 (1H, d, J=8.3Hz), 4.74 (1H, s), 4.90-5.05 (3H, m), 5.25 (1H, d, J=6.1Hz), 5.30 (1H, d, J=6.9Hz), 5.43-5.49 (2H, m), 5.58 (1H, d, J=17.4Hz), 5.96-6.06 (2H, m), 6.14-6.22 (3H, m), 7.48 (2H, t, J=7.7Hz), 7.59-7.64 (1H, m), 8.11-8.15 (2H, m).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-2-hydroxy-3-(isopropoxycarbonylamino)-4,4,4-trifluorobutyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.17-2.37 (10H, m), 1.19 (3H, d, J=6.4Hz), 1.23 (3H, s), 1.25 (3H, d, J=6.1Hz), 1.48 (3H, s), 1.61 (3H, s), 1.72 (3H, s), 2.34 (3H, s), 2.75 (1H, dd, J=12.5, 5.9Hz), 2.84 (1H, dd, J=12.5, 4.2Hz), 2.92 (1H, d,

J=4.9Hz), 3.32-3.41 (4H, m), 4.10 (1H, d, J=7.1Hz), 4.25 (1H, d, J=8.3Hz), 4.34 (1H, d, J=8.6Hz), 4.73 (1H, s), 4.85-5.00 (4H, m), 5.21 (1H, d, J=7.1Hz), 5.50 (1H, d, J=10.3Hz), 6.01 (1H, d, J=4.9Hz), 6.13-6.20 (1H, m), 7.48 (2H, t, J=7.7Hz), 7.59-7.65 (1H, m), 8.11-8.15 (2H, m).
FAB-MS; m/z: 853 $(M+H)^+$.

[Example 79]

(1S,2S,3R,4S,5R,8R,9S, 10R, 13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-2-hydroxy-3-(isopropoxycarbonylamino)-4,4,4-trifluorobutyrate

[Formula 151]

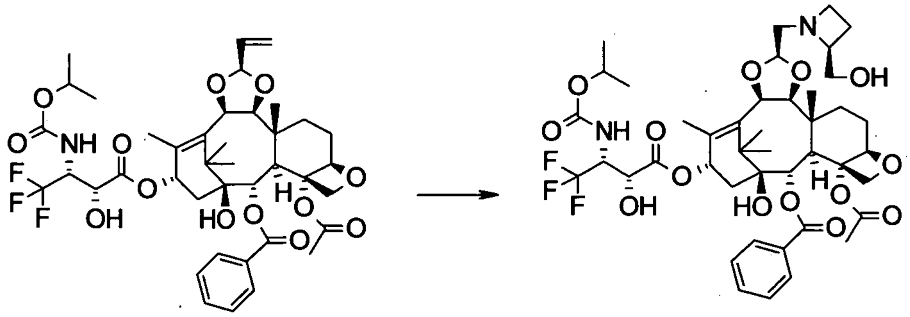

The compound obtained in Example 78, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.18-2.37 (11H, m), 1.20 (3H, d, J=6.4Hz), 1.22 (3H, s), 1.25 (3H, d, J=6.4Hz), 1.48 (3H, s), 1.58 (3H, s), 1.73 (3H, s), 2.34 (3H, s), 2.83 (1H, dd, J=12.7, 4.4Hz), 2.87-2.94 (2H, m), 3.06-3.15 (1H, m), 3.42 (1H, dd, J=11.5, 2.5Hz), 3.46-3.54 (2H, m), 3.66 (1H, dd, J=11.3, 2.7Hz), 4.11 (1H, d, J=7.1Hz), 4.25 (1H, d, J=8.6Hz), 4.34 (1H, d, J=8.3Hz), 4.73 (1H, s), 4.86-5.01 (4H, m), 5.22 (1H, d, J=7.1Hz), 5.48 (1H, d, J=10.3Hz), 6.00 (1H, d, J=5.1Hz), 6.13-6.20 (1H, m), 7.48 (2H, t, J=7.7Hz), 7.59-7.64 (1H, m), 8.11-8.15 (2H, m).
FAB-MS; m/z: 883 $(M+H)^+$.

[Example 80]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-(1-hydroxy-1-methylethyl)azetidino]ethylidenedioxy}tax-11-en-13-yl

(2R,3S)-3-(tert-butoxycarbonylamino)-3-(3-fluoro-2-pyridyl)-2-hydroxypropionate

[Formula 152]

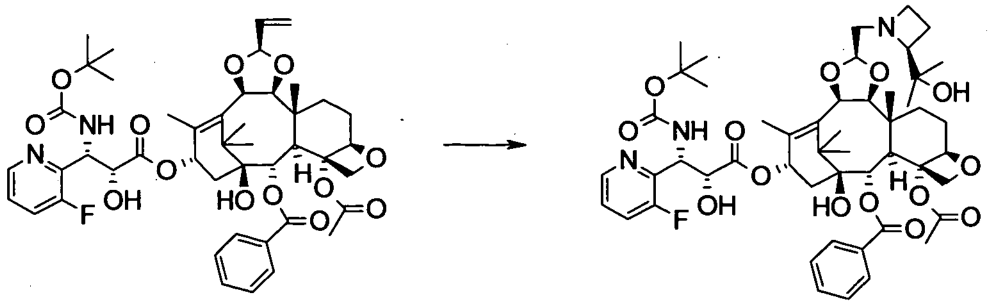

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-hydroxypropionate and the compound obtained in Reference Example 37 were used as starting materials to perform the same procedure as that of Example 1 and thereby to obtain the title compound as white amorphous solid.
$^1$H-NMR (400MHz, $CDCl_3$) δ: 1.03 (3H, s), 1.15 (3H, s), 1.24-2.48 (11H, m), 1.29 (3H, s), 1.40 (9H, s), 1.50 (3H, s), 1.60 (3H, s), 1.79 (3H, s), 2.35 (3H, s), 2.83 (1H, dd, J=12.6, 5.5Hz), 2.94 (1H, d, J=5.1Hz), 3.00-3.07 (2H, m), 3.22 (1H, t, J=7.7Hz), 3.51 (1H, t, J=6.8Hz), 4.17 (1H, d, J=7.1Hz), 4.23 (1H, d, J=8.5Hz), 4.33 (1H, d, J=8.5Hz), 4.67 (1H, s), 4.88-4.91 (2H, m), 5.24 (1H, d, J=6.8Hz), 5.65 (1H, d, J=8.3Hz), 5.98 (1H, d, J=5.1Hz), 6.09 (1H, t, J=8.8Hz), 6.20 (1H, d, J=8.5Hz), 7.28-7.32 (1H, m), 7.43-7.49 (3H, m), 7.58-7.62 (1H, m), 8.13 (2H, d, J=8.3Hz), 8.40-8.41 (1H, m).
FAB-MS; m/z: 952 $(M+H)^+$.

[Example 81]

[Formula 153]

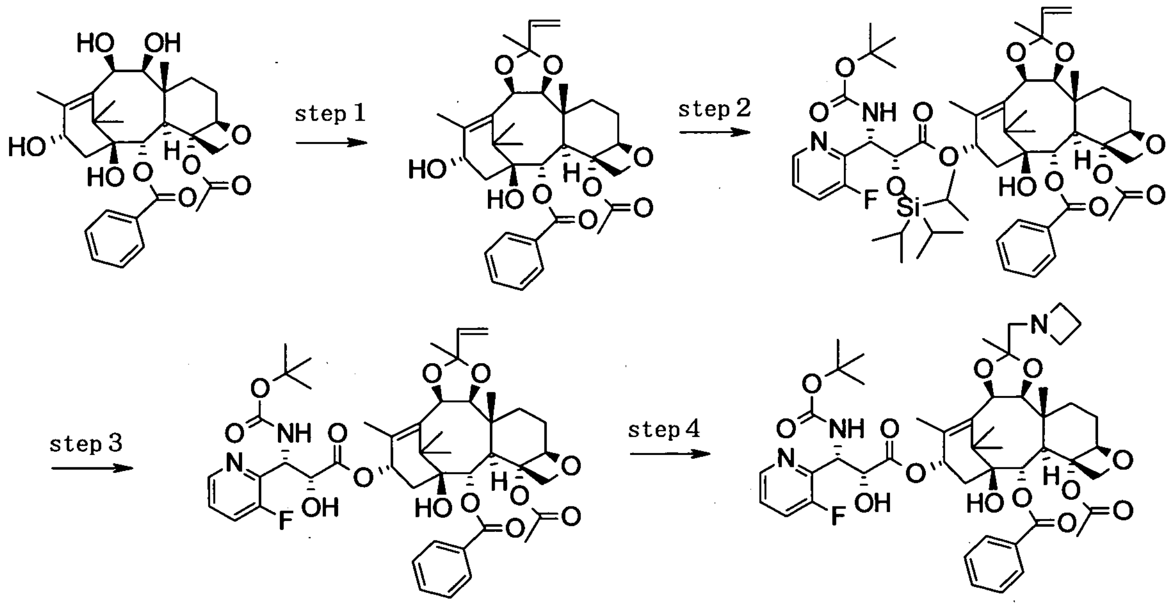

Step 1: (1S,2S,3R,4S,5R,8R,9S,10R,13S)-4-(Acetoxy)-2-(benzoyloxy)-1,13-dihydroxy-5,20-epoxy-9,10-[(1-methyl)-2-propenylidenedioxy]tax-11-ene

(1S,2S,3R,4S,5R,8R,9S,10R,13S)-4-(Acetoxy)-2-(benzoyloxy)-5,20-epoxy-1,9,10,13-tetrahydroxytax-11-ene (Bioorg. Med. Chem. Lett., 2002, 12, 2815, 350 mg) and 3,3-dimethoxy-1-butene (J. Am. Chem. Soc., 1935, 57, 2255, 383 mg) were dissolved in tetrahydrofuran (7 ml), and the solution was added with (±)-camphor-10-sulfonic acid (77 mg), and stirred at room temperature for 1 hour. The reaction mixture was added with saturated aqueous sodium hydrogencarbonate, and extracted with ethyl acetate. The organic layer was successively washed with water and saturated brine, and then dried over anhydrous sodium sulfate. The solvent was evaporated under reduced pressure, and then the resulting residue was purified by using thin layer silica gel chromatography [developing solvent; ethyl acetate: n-hexane=1:1 (v/v)] to obtain the title compound (115 mg) as white amorphous solid.
$^1$H-NMR (400MHz, $CDCl_3$) δ : 1.18 (3H, s), 1.40 (3H, s), 1.50 (3H, s), 1.54-2.17 (7H, m), 1.62 (3H, s), 2.00 (3H, s), 2.21-2.39 (1H, m), 2.34 (3H, s), 2.47 (1H, d, J=10.6Hz), 3.06 (1H, d, J=4.5Hz), 4.12 (1H, d, J=7.4Hz), 4.23 (1H, d, J=8.3Hz), 4.32 (1H, d, J=8.3Hz), 4.80 (1H, br), 4.85 (1H, s), 5.20 (1H, dd, J=11.0, 1.2Hz), 5.44 (1H, dd, J=17.5, 1.2Hz), 5.66 (1H, d, J=7.1Hz), 5.96 (1H, d, J=5.1Hz), 6.05 (1H, dd, J=17.5, 11.0Hz), 7.47 (2H, t, J=7.8Hz), 7.58 (1H, t, J=7.8Hz), 8.13 (2H, d, J=7.8Hz).
FAB-MS; m/z: 583 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-[(1-methyl)-2-propenylidenedioxy]tax-11-en-13-yl

(2R,3S)-3-(tert-butoxycarbonylamino)-3-(3-fluoro-2-pyridyl)-2-[(triisopropylsilyl)oxy]propionate

The compound obtained in Step 1 mentioned above and (3R,4S)-1-(tert-butoxycarbonyl)-4-(3-fluoro-2-pyridyl)-3-[(triisopropylsilyl)oxy]-2-azeti dinone (Bioorg. Med. Chem. Lett., 2004, 14, 3209) were used as starting materials to perform the same procedure as that of Example 31, Step 1 and thereby to obtain the title compound as white amorphous solid.
$^1$H-NMR (400MHz, $CDCl_3$) δ 0.87-0.93 (21H, m), 1.24-2.04 (6H, m), 1.38 (3H, s), 1.39 (9H, s), 1.46 (3H, s), 1.48 (3H, s), 1.67 (3H, s), 1.84 (3H, s), 2.30-2.41 (2H, m), 2.49 (3H, s), 2.96 (1H, d, J=5.1Hz), 4.21 (1H, d, J=8.3Hz), 4.27 (1H, d, J=7.3Hz), 4.35 (1H, d, J=8.3Hz), 4.95-4.97 (2H, m), 5.20 (1H, dd, J=11.0, 1.0Hz), 5.45 (1H, dd, J=17.6, 1.0Hz), 5.59-5.62 (2H, m), 5.96-6.11 (3H, m), 7.26-7.28 (1H, m), 7.37-7.49 (3H, m), 7.56-7.60 (1H, m), 8.15-8.17 (2H, m), 8.40 (1H, d, J=4.4Hz).
FAB-MS; m/z: 1021 $(M+H)^+$, 1043 $(M+Na)^+$.

Step 3: (1S,2S,3R,4S,5R,8R,9S,10R,13S)-4-(Acetoxy)-2-(benzoyloxy)-5,20-epoxy-1-hydroxy-9,10-[(1-methyl)-2-propenylidenedioxy]tax-11-en-13-yl

(2R,3S)-3-(tert-butoxycarbonylamino)-3-(3-fluoro-2-pyridyl)-2-hydroxypropionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.24-2.16 (7H, m), 1.31 (3H, s), 1.41 (9H, s), 1.42 (3H, s), 1.50 (3H, s), 1.66 (3H, s), 1.80 (3H, s), 2.35 (3H, s), 2.45 (1H, dd, J=14.6, 9.5Hz), 2.93 (1H, d, J=5.1Hz), 4.21-4.24 (2H, m), 4.32 (1H, d, J=8.3Hz), 4.68 (1H, dd, J=4.9, 2.7Hz), 4.92 (1H, s), 5.21 (1H, dd, J=11.0, 1.2Hz), 5.45 (1H, dd, J=17.6, 1.2Hz), 5.62-5.66 (2H, m), 5.98 (1H, d, J=5.1Hz), 6.04-6.20 (3H, m), 7.28-7.32 (1H, m), 7.42-7.49 (3H, m), 7.57-7.61 (1H, m), 8.12-8.14 (2H, m), 8.40-8.41 (1H, m).
FAB-MS; m/z: 865 $(M+H)^+$, 887 $(M+Na)^+$.

Step 4: (1S,2S,3R,4S,5R,8R,9S,10R,13S)-4-(Acetoxy)-9,10-[(1-methyl)-2-(azetidino)ethylidenedioxy]-2-(benzoyloxy)-5,20-epoxy-1-hydroxytax-11-en-13-yl

(2R,3S)-3-(tert-butoxycarbonylamino)-3-(3-fluoro-2-pyridyl)-2-hydroxypropionate

The compound obtained in Step 3 mentioned above was used as a starting material to perform the same procedure as that of Example 1 and thereby to obtain the title compound as white amorphous solid.
$^1$H-NMR (400MHz, $CDCl_3$) δ : 1.24-2.14 (9H, m), 1.29 (3H, s), 1.40 (9H, s), 1.44 (3H, s), 1.55 (3H, s), 1.62 (3H, s), 1.78 (3H, s), 2.35 (3H, s), 2.44 (1H, dd, J=14.6, 9.5Hz), 2.74 (2H, s), 2.95 (1H, d, J=4.9Hz), 3.35 (4H, t, J=6.7Hz), 4.19 (1H, d, J=7.6Hz), 4.25 (1H, d, J=8.3Hz), 4.33 (1H, d, J=8.3Hz), 4.67 (1H, d, J=2.7Hz), 4.92 (1H, s), 5.58 (1H, d, J=7.6Hz),

5.64 (1H, d, J=8.8Hz), 5.98 (1H, d, J=5.1Hz), 6.09 (1H, t, J=8.7Hz), 6.20 (1H, d, J=8.5Hz), 7.28-7.32 (1H, m), 7.42-7.49 (3H, m), 7.58-7.62 (1H, m), 8.13-8.15 (2H, m), 8.40 (1H, dt, J=4.6, 1.2Hz).
FAB-MS; m/z: 908 $(M+H)^+$.

[Example 82]

[Formula 154]

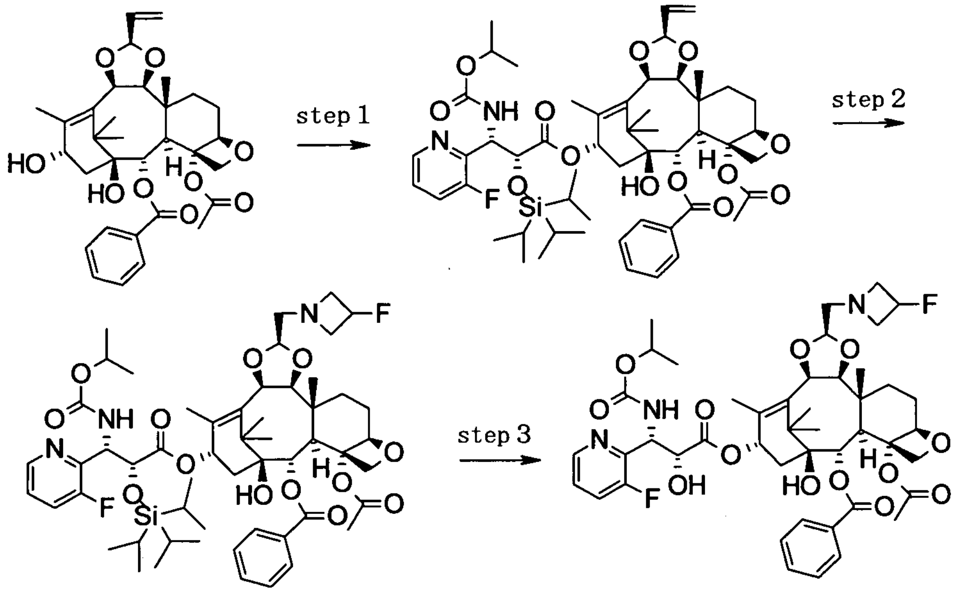


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(3-fluoro-2-pyridyl)-3-(isopropoxycarbonylamino)-2-[(triisopropylsilyl)oxy]propionate

(1S,2S,3R,4S,5R,8R,9S,10R,13S)-4-(Acetoxy)-2-(benzoyloxy)-1,13-dihydroxy-5, 20-epoxy-9,10-[(1S)-2-propenylidenedioxyhax-11-ene and the compound obtained in Reference Example 38 were used as starting materials to perform the same procedure as that of Example 31, Step 1 and thereby to obtain the title compound as white amorphous solid.
$^1$H-NMR (400MHz, $CDCl_3$) δ : 0.87-0.96 (21H, m), 1.16-1.23 (6H, m), 1.37 (3H, s), 1.53 (3H, s), 1.66 (3H, s), 1.80 (3H, s), 1.89-2.39 (7H, m), 2.48 (3H, s), 2.98 (1H, d, J=5.1Hz), 4.20-4.25 (2H, m), 4.36 (1H, d, J=8.8Hz), 4.77-4.85 (1H, m), 4.96 (1H, brs), 4.98 (1H, d, J=2.7Hz), 5.20 (1H, d, J=6.1Hz), 5.27 (1H, d, J=7.6Hz), 5.46 (1H, d, J=10.5Hz), 5.58 (1H, d, J=17.6Hz), 5.63 (1H, d, J=9.8Hz), 5.95-6.06 (2H, m), 6.06-6.16 (2H, m), 7.23-7.29 (1H, m), 7.36-7.51 (3H, m), 7.59 (1H, t, J=7.8Hz), 8.16 (2H, d, J=7.1Hz), 8.38 (1H, d, J=4.9Hz).
FAB-MS; m/z: 993 $(M+H)^+$.

Step 2: (1S,2S,3R,4S,5R,8R,9S,10R,13S)-4-(Acetoxy)-2-(benzoyloxy)-5,20-epoxy-9,10-[(1S)-2-(3-fluoroazetidino) ethylidenedioxy]-1-hydroxytax-11-en-13-yl

(2R,3S)-3-(3-fluoro-2-pyridyl)-3-(isopropoxycarbonylamino)-2-[(triisopropylsilyl)oxy]propionate

The compound obtained in Step 1 mentioned above and the compound obtained in Reference Example 24, Step 2 were used as starting materials to perform the same procedure as that of Example 1 and thereby to obtain the title compound as white amorphous solid.
$^1$H-NMR (400MHz, $CDCl_3$) δ : 0.87-0.98 (21H, m), 1.14-1.23 (6H, m), 1.36 (3H, s), 1.52 (3H, s), 1.62 (3H, s), 1.71-2.39 (7H, m), 1.78 (3H, s), 2.47 (3H, s), 2.82 (1H, dd, J=11.2, 5.6Hz), 2.90 (1H, dd, J=12.7, 4.6Hz), 2.96 (1H, d, J=5.9Hz),

3.25-3.40 (2H, m), 3.79 (2H, brs), 4.17 (1H, d, J=7.6Hz), 4.21 (1H, d, J=8.8Hz), 4.36 (1H, d, J=8.3Hz), 4.76-4.87 (2H, m), 4.95 (1H, brs), 4.97 (1H, brs), 5.04-5.24 (2H, m), 5.62 (1H, d, J=8.8Hz), 5.97 (1H, d, J=4.9Hz), 6.04-6.15 (2H, m), 7.24-7.27 (1H, m), 7.40 (1H, t, J=8.8Hz), 7.47 (2H, t, J=7.8Hz), 7.59 (1H, t, J=7.3Hz), 8.16 (2H, d, J=7.8Hz), 8.38 (1H, d, J=3.9Hz).
FAB-MS; m/z: 1054 $(M+H)^+$.

Step 3: (1S,2S,3R,4S,5R,8R,9S,10R,13S)-4-(Acetoxy)-2-(benzoyloxy)-5,20-epoxy-9,10-[(1S)-2-(3-fluoroazetidino) ethylidenedioxy]-1-hydroxytax-11-en-13-yl

(2R,3S)-3-(3-fluoro-2-pyridyl)-2-hydroxy-3-(isopropoxycarbonylamino)propionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) $\delta$ : 1.14-1.23 (6H, m), 1.28 (3H, s), 1.49 (3H, s), 1.61 (3H, s), 1.76 (3H, s), 1.82-2.47 (8H, m), 2.33 (3H, s), 2.82 (1H, dd, J=11.2, 5.6Hz), 2.87-2.95 (2H, m), 3.25-3.40 (2H, m), 3.72-3.85 (2H, m), 4.13 (1H, d, J=7.6Hz), 4.23 (1H, d, J=8.8Hz), 4.32 (1H, d, J=8.3Hz), 4.70 (1H, d, J=3.2Hz), 4.80-4.93 (3H, m), 5.04-5.24 (2H, m), 5.70 (1H, d, J=8.8Hz), 5.99 (1H, d, J=4.9Hz), 6.11 (1H, t, J=7.3Hz), 6.26 (1H, d, J=8.3Hz), 7.27-7.33 (1H, m), 7.40-7.50 (3H, m), 7.60 (1H, t, J=7.3Hz), 8.13 (2H, d, J=7.8Hz), 8.39 (1H, d, J=3.9Hz).
FAB-MS; m/z: 898 $(M+H)^+$.

[Example 83]

[Formula 155]

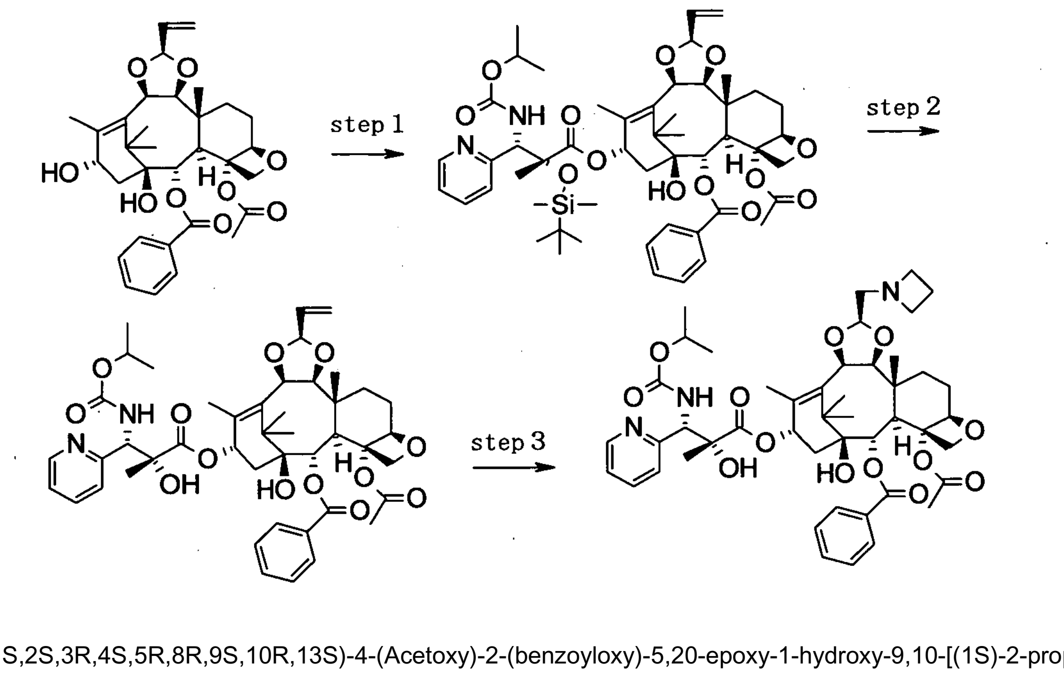


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-2-[(tert-butyldimethylsilyl)oxy]-3-(isopropoxycarbonylamino)-2-methyl-3-(2-pyridyl)propionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 39 were used as starting materials to perform the same procedure as that of Example 31, Step 1 and thereby to obtain the title compound as white amorphous solid, which was used as it was for the following reaction.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-2-hydroxy-3-(isopropoxycarbonylamino)-2-methyl-3-(2-pyridyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.14-2.23 (7H, m), 1.21 (6H, dd, J=7.8, 6.3Hz), 1.30 (3H, s), 1.50 (3H, s), 1.53 (3H, s), 1.58 (3H, s), 1.64 (3H, s), 2.50 (3H, s), 2.95 (1H, d, J=4.9Hz), 4.18 (1H, d, J=7.1Hz), 4.21 (1H, d, J=8.3Hz), 4.35 (1H, d, J=8.3Hz), 4.87-4.95 (2H, m), 5.16 (1H, d, J=10.0Hz), 5.20-5.23 (2H, m), 5.45 (1H, d, J=10.5Hz), 5.57 (1H, d, J=17.1Hz), 5.94-6.04 (3H, m), 6.14-6.20 (2H, m), 7.22-7.27 (1H, m), 7.43-7.50 (3H, m), 7.60 (1H, t, J=7.3Hz), 7.72 (1H, td, J=7.7, 1.6Hz), 8.14-8.16 (2H, m), 8.51 (1H, dd, J=4.2, 0.7Hz).
FAB-MS; m/z: 833 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-2-hydroxy-3-(isopropoxycarbonylamino)-2-methyl-3-(2-pyridyl)propionate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.14-2.22 (9H, m), 1.21 (6H, t, J=6.6Hz), 1.28 (3H, s), 1.49 (3H, s), 1.53 (3H, s), 1.55 (3H, s), 1.60 (3H, s), 2.50 (3H, s), 2.73 (1H, dd, J=12.5, 5.6Hz), 2.81 (1H, dd, J=12.5, 4.4Hz), 2.93 (1H, d, J=5.1Hz), 3.30-3.37 (4H, m), 4.11 (1H, d, J=7.3Hz), 4.21 (1H, d, J=8.3Hz), 4.34 (1H, d, J=8.3Hz), 4.82-4.94 (3H, m), 5.12-5.16 (2H, m), 5.96 (2H, d, J=5.1Hz), 6.13-6.19 (2H, m), 7.22-7.25 (1H, m), 7.42-7.50 (3H, m), 7.58-7.62 (1H, m), 7.72 (1H, td, J=7.8, 1.7Hz), 8.14-8.16 (2H, m), 8.49-8.51 (1H, m). FAB-MS; m/z: 876 $(M+H)^+$.

[Example 84]

[Formula 156]

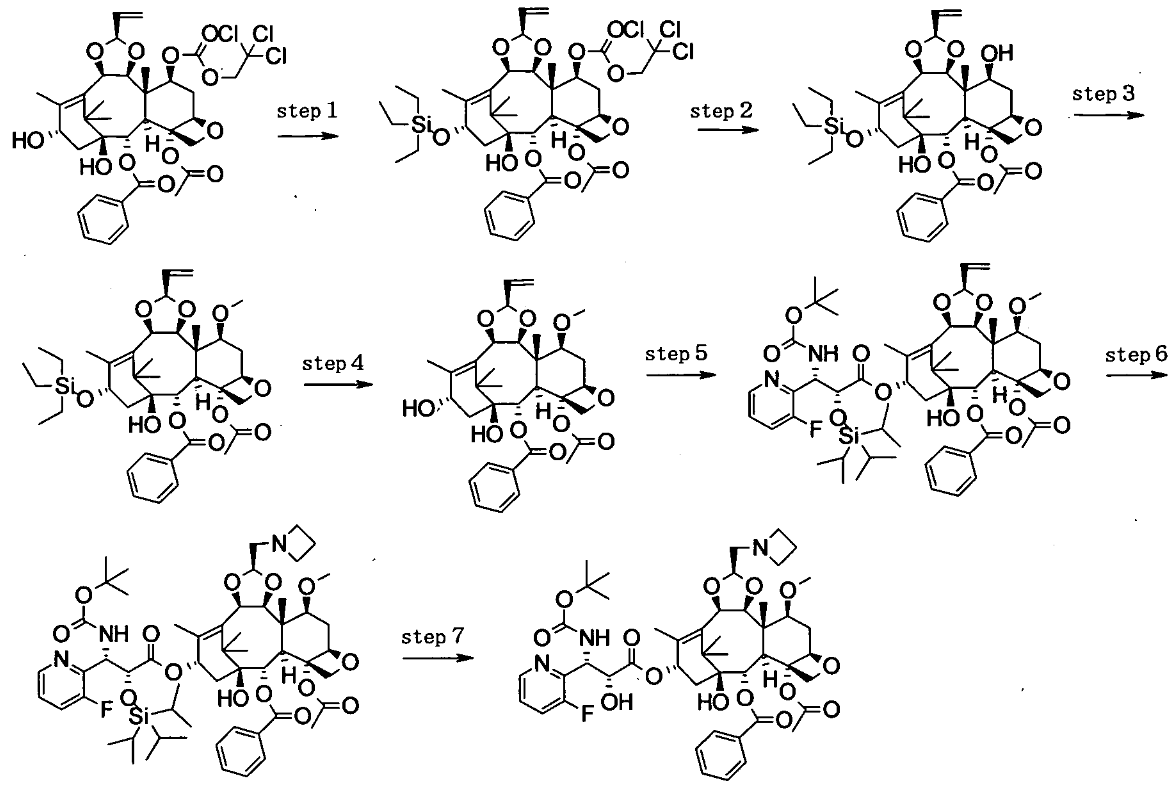


Step 1: (1S,2S,3R,4S,5R,7S,8S,9S,10R,13S)-4-(Acetoxy)-2-(benzoyloxy)-5,20-epoxy-1-hydroxy-9,10-[(1S)-2-propenylidenedioxy]-7-[(2,2,2-trichloroethoxycarbonyl)oxy]-13-[(triethylsilyl)oxy]tax-11-ene

(1S,2S,3R,4S,5R,7S,8S,9S,10R,13S)-4-(Acetoxy)-2-(benzoyloxy)-1,13-dihydroxy-5,20-epoxy-9,10-[(1S)-2-propenylidenedioxy]-7-[(2,2,2-trichloroethoxycarbonyl)oxy]tax-11-ene (WO96/33998, 2.60 g) and 2,6-lutidine (0.68 ml) were dissolved in dichloromethane (52 ml), and cooled to -45°C. Then, the solution was added dropwise with triethylsilyl trifluoromethanesulfonate (1.16 ml), and stirred at the same temperature for 1 hour. The reaction mixture was added with saturated aqueous sodium hydrogencarbonate, and the organic layer was washed with saturated brine, and then dried over anhydrous sodium sulfate. The solvent was evaporated under reduced pressure, and then the resulting residue was added with n-hexane/ether. The deposited solid was collected by filtration, and dried to obtain the title compound (2.60 g) as colorless solid.
$^1$H-NMR (400MHz, $CDCl_3$) δ : 0.56-0.75 (6H, m), 1.01 (9H, t, J=7.8Hz), 1.22 (3H, s), 1.58 (3H, s), 1.61 (3H, s), 1.76 (3H, s), 1.89 (3H, s), 2.05-2.40 (3H, m), 2.28 (3H, s), 3.17 (1H, d, J=5.3Hz), 4.02 (1H, d, J=8.3Hz), 4.31 (1H, d, J=8.3Hz), 4.38 (1H, d, J=8.3Hz), 4.66 (1H, d, J=11.7Hz), 4.86 (1H, d, J=11.7Hz), 4.91 (1H, t, J=4.9Hz), 4.97 (1H, t, J=8.8Hz), 5.05-5.15 (2H, m), 5.30 (1H, d, J=8.3Hz), 5.45 (1H, d, J=10.7Hz), 5.56 (1H, d, J=17.6Hz), 5.90 (1H, d, J=5.3Hz), 6.03 (1H, ddd, J=17.1, 10.7, 5.9Hz), 7.47 (2H, t, J=7.8Hz), 7.58 (1H, t, J=7.8Hz), 8.12 (2H, d, J=7.8Hz).

Step 2: (1S,2S,3R,4S,5R,7S,8S,9S,10R,13S)-4-(Acetoxy)-2-(benzoyloxy)-1,7-dihydroxy-5,20-epoxy-9,10-[(1S)-2-propenylidenedioxy]-13-[(triethylsilyl)oxy]tax-11-ene

The compound obtained in Step 1 mentioned above (2.60 g) was dissolved in ethyl acetate, and the solution was added with acetic acid (8.48 ml) and active zinc (12.8 g), and stirred at room temperature for 2 hours. The insoluble matters were removed by filtration through Celite, and the filtrate was successively washed with saturated aqueous sodium hydrogencarbonate and saturated brine, and then dried over anhydrous sodium sulfate. The solvent was evaporated under reduced pressure, and then the resulting residue was purified by silica gel chromatography [developing solvent; chloroform:acetone=15:1 (v/v)] to obtain the title compound (1.05 g) as colorless solid.
$^1$H-NMR (400MHz, $CDCl_3$) δ : 0.59-0.73 (6H, m), 1.01 (9H, t, J=7.8Hz), 1.25 (3H, s), 1.61 (3H, s), 1.65 (3H, s), 1.85 (3H, s), 1.86 (1H, s), 2.03-2.34 (4H, m), 2.27 (3H, s), 2.95 (1H, d, J=4.9Hz), 3.97 (1H, d, J=7.4Hz), 4.07 (1H, br), 4.25 (1H, d, J=8.3Hz), 4.39 (1H, d, J=8.3Hz), 4.55 (1H, brd, J=6.8Hz), 4.98 (1H, t, J=8.3Hz), 5.10 (1H, s), 5.19 (1H, d, J=6.3Hz),

5.25 (1H, d, J=6.8Hz), 5.43 (1H, d, J=10.8Hz), 5.56 (1H, d, J=17.1Hz), 5.91-6.10 (2H, m), 7.47 (2H, t, J=7.8Hz), 7.59 (1H, t, J=7.8Hz), 8.12 (2H, d, J=7.8Hz).

Step 3: (1S,2S,3R,4S,5R,7S,8S,9S,10R,13S)-4-(Acetoxy)-2-(benzoyloxy)-5,20-epoxy-1-hydroxy-7-methoxy-9,10-[(1S)-2-propenylidenedioxy]-13-[(triethylsilyl)oxy]tax-11-ene

The compound obtained in Step 2 mentioned above (1.00 g) was dissolved in 2,6-di-tert-butylpyridine (3.5 ml), and the solution was added with methyl trifluoromethanesulfonate (0.80 ml), and stirred at room temperature for 4 hours. The reaction mixture was diluted with ethyl acetate, and the organic layer was washed with saturated brine, and then dried over anhydrous sodium sulfate. The solvent was evaporated under reduced pressure, and then the resulting residue was purified by silica gel chromatography [developing solvent; ethyl acetate:n-hexane=1:5 to 1:2 (v/v)] to obtain the title compound (180 mg) as colorless solid.
$^1$H-NMR (400MHz, $CDCl_3$) δ : 0.55-0.75 (6H, m), 1.01 (9H, t, J=7.8Hz), 1.16 (3H, s), 1.56 (3H, s), 1.71 (3H, s), 1.89 (3H, s), 1.95-2.05 (2H, m), 2.10 (1H, dd, J=15.2, 8.8Hz), 2.24 (1H, dd, J=15.2, 8.8Hz), 2.27 (3H, s), 2.48-2.55 (1H, m), 3.25 (1H, d, J=5.9Hz), 3.31 (1H, dd, J=10.5, 4.6Hz), 3.39 (3H, s), 4.31 (1H, d, J=8.3Hz), 4.40 (1H, d, J=8.3Hz), 4.42 (1H, d, J=9.1Hz), 4.86 (1H, dd, J=8.5, 5.6Hz), 4.95 (1H, dd, J=8.8, 8.3Hz), 5.14 (1H, d, J=6.1Hz), 5.34 (1H, d, J=9.1Hz), 5.46 (1H, d, J=10.7Hz), 5.58 (1H, d, J=17.6Hz), 5.82 (1H, d, J=5.9Hz), 6.12 (1H, ddd, J=17.6, 10.7, 6.1Hz), 7.47 (2H, t, J=7.3Hz), 7.58 (1H, t, J=7.3Hz), 8.08 (2H, d, J=7.3Hz).

Step 4: (1S,2S,3R,4S,5R,7S,8S,9S,10R,13S)-4-(Acetoxy)-2-(benzoyloxy)-1,13-dihydroxy-5,20-epoxy-7-methoxy-9,10-[(1S)-2-propenylidenedioxy]tax-11-ene

The compound obtained in Step 3 mentioned above (180 mg) was dissolved in pyridine (4 ml), and the solution was added with hydrogen fluoride/pyridine (1 ml) under ice cooling, returned to room temperature, and stirred overnight. The reaction mixture was cooled in ice, diluted with ethyl acetate, and neutralized with saturated aqueous sodium hydrogencarbonate. The organic layer was successively washed with 1 N aqueous hydrochloric acid and saturated brine, and then dried over anhydrous sodium sulfate. The solvent was evaporated under reduced pressure, and then the resulting residue was purified by thin layer silica gel column chromatography [developing solvent; ethyl acetate:n-hexane=1:1 (v/v)] to obtain the title compound (110 mg) as colorless solid.
$^1$H-NMR (400MHz, $CDCl_3$) δ : 1.12 (3H, s), 1.56 (3H, s), 1.61 (3H, s), 1.77 (1H, s), 1.96 (3H, s), 1.85-2.40 (5H, m), 2.32 (3H, s), 3.22 (1H, d, J=5.1Hz), 3.37 (3H, s), 3.41 (1H, dd, J=7.8, 4.9Hz), 4.29 (1H, dd, J=7.8Hz), 4.36 (1H, d, J=8.1Hz), 4.43 (1H, d, J=7.8Hz), 4.82-4.90 (2H, m), 5.17 (1H, d, J=5.9Hz), 5.36 (1H, d, J=8.1Hz), 5.48 (1H, d, J=10.3Hz), 5.59 (1H, d, J=17.1Hz), 5.92 (1H, d, J=5.1Hz), 6.14 (1H, ddd, J=17.1, 10.3, 5.9Hz), 7.47 (2H, t, J=7.3Hz), 7.59 (1H, t, J=7.3Hz), 8.11 (2H, d, J=7.6Hz).

Step 5: (1S,2S,3R,4S,5R,7S,8S,9S,10R,13S)-4-(Acetoxy)-2-(benzoyloxy)-5,20-epoxy-1-hydroxy-7-methoxy-9,10-[(1S)-2-propenylidenedioxy]tax-11-en-13-yl

(2R,3S)-3-(tert-butoxycarbonylamino)-3-(3-fluoro-2-pyridyl)-2-[(triisopropylsilyl)oxy]propionate

The compound obtained in Step 4 mentioned above and (3R,4S)-1-(tert-butoxycarbonyl)-4-(3-fluoro-2-pyridyl)-3-[(triisopropylsilyl)oxy]-2-azetidinone were used as starting materials to perform the same procedure as that of Example 31, Step 1 and thereby to obtain the title compound as white amorphous solid.
$^1$H-NMR (400MHz, $CDCl_3$) δ : 0.87-0.96 (21H, m), 1.28 (3H, s), 1.38 (9H, s), 1.56 (3H, s), 1.59 (3H, s), 1.80 (3H, s), 1.97-2.46 (5H, m), 2.49 (3H, s), 3.27 (1H, d, J=6.3Hz), 3.29-3.35 (1H, m), 3.39 (3H, s), 4.32 (1H, d, J=8.8Hz), 4.36 (1H, d, J=9.0Hz), 4.48 (1H, d, J=8.3Hz), 4.83-4.90 (1H, m), 4.93 (1H, brs), 5.14 (1H, d, J=5.9Hz), 5.32 (1H, d, J=8.8Hz), 5.46 (1H, d, J=10.5Hz), 5.55-5.66 (2H, m), 5.92 (1H, d, J=5.4Hz), 6.02-6.18 (3H, m), 7.25-7.29 (1H, m), 7.40 (1H, t, J=9.8Hz), 7.47 (2H, t, J=7.6Hz), 7.57 (1H, t, J=7.6Hz), 8.12 (2H, d, J=7.8Hz), 8.40 (1H, d, J=4.4Hz).
FAB-MS; m/z: 1037 $(M+H)^+$.

Step 6: (1S,2S,3R,4S,5R,7S,8S,9S,10R,13S)-4-(Acetoxy)-9,10-[(1S)-2-(azetidino)ethylidenedioxy]-2-(benzoyloxy)-5,20-epoxy-1-hydroxy-7-methoxytax-11-en-13-yl

(2R,3S)-3-(tert-butoxycarbonylamino)-3-(3-fluoro-2-pyridyl)-2-[(triisopropylsilyl)oxy]propionate

The compound obtained in Step 5 mentioned above was used as a starting material to perform the same procedure as that of Example 1 and thereby to obtain the title compound as white amorphous solid.
$^1$H-NMR (400MHz, $CDCl_3$) δ : 0.83-0.94 (21H, m), 1.27 (3H, s), 1.38 (9H, s), 1.51 (3H, s), 1.55 (3H, s), 1.59-2.52 (7H,

m), 1.78 (3H, s), 2.48 (3H, s), 2.81-2.86 (2H, m), 3.23 (1H, d, J=5.9Hz), 3.26-3.38 (5H, m), 3.35 (3H, s), 4.18 (1H, d, J=8.8Hz), 4.30 (1H, d, J=8.1Hz), 4.49 (1H, d, J=8.3Hz), 4.74 (1H, t, J=5.1Hz), 4.85 (1H, t, J=6.6Hz), 4.93 (1H, brs), 5.21 (1H, d, J=9.0Hz), 5.62 (1H, d, J=10.0Hz), 5.89 (1H, d, J=5.4Hz), 6.05 (1H, d, J=10.7Hz), 6.11 (1H, t, J=9.0Hz), 7.24-7.28 (1H, m), 7.40 (1H, t, J=10.5Hz), 7.47 (2H, t, J=7.8Hz), 7.57 (1H, t, J=8.5Hz), 8.12 (2H, d, J=9.0Hz), 8.40 (1H, d, J=4.2Hz).
FAB-MS; m/z: 1080 $(M+H)^+$.

Step 7: (1S,2S,3R,4S,5R,7S,8S,9S,10R,13S)-4-(Acetoxy)-9,10-[(1S)-2-(azetidino)ethylidenedioxy]-2-(benzoyloxy)-5,20-epoxy-1-hydroxy-7-methoxytax-11-en-13-yl

(2R,3S)-3-(tert-butoxycarbonylamino)-3-(3-fluoro-2-pyridyl)-2-hydroxypropionate

The compound obtained in Step 6 mentioned above was used as a starting material to perform the same procedure as that of Example 31, Step 2 and thereby to obtain the title compound as white amorphous solid.
$^1$H-NMR (400MHz, $CDCl_3$) δ : 1.24 (3H, s), 1.39 (9H, s), 1.54 (3H, s), 1.60 (3H, s), 1.75-2.51 (8H, m), 1.78 (3H, s), 2.35 (3H, s), 2.87 (2H, d, J=5.1Hz), 3.12 (1H, d, J=4.6Hz), 3.33-3.39 (5H, m), 3.35 (3H, s), 4.27 (2H, d, J=9.0Hz), 4.42 (1H, d, J=8.5Hz), 4.65 (1H, d, J=1.5Hz), 4.76 (1H, t, J=5.1Hz), 4.86 (1H, dd, J=7.1, 3.9Hz), 5.26 (1H, d, J=8.5Hz), 5.65 (1H, d, J=8.3Hz), 5.92 (1H, d, J=5.1Hz), 6.10 (1H, t, J=7.8Hz), 6.19 (1H, d, J=9.0Hz), 7.28-7.33 (1H, m), 7.42-7.50 (3H, m), 7.59 (1H, t, J=8.5Hz), 8.10 (2H, d, J=8.5Hz), 8.40 (1H, d, J=5.6Hz).
FAB-MS; m/z: 924 $(M+H)^+$.

[Example 85]

[Formula 157]

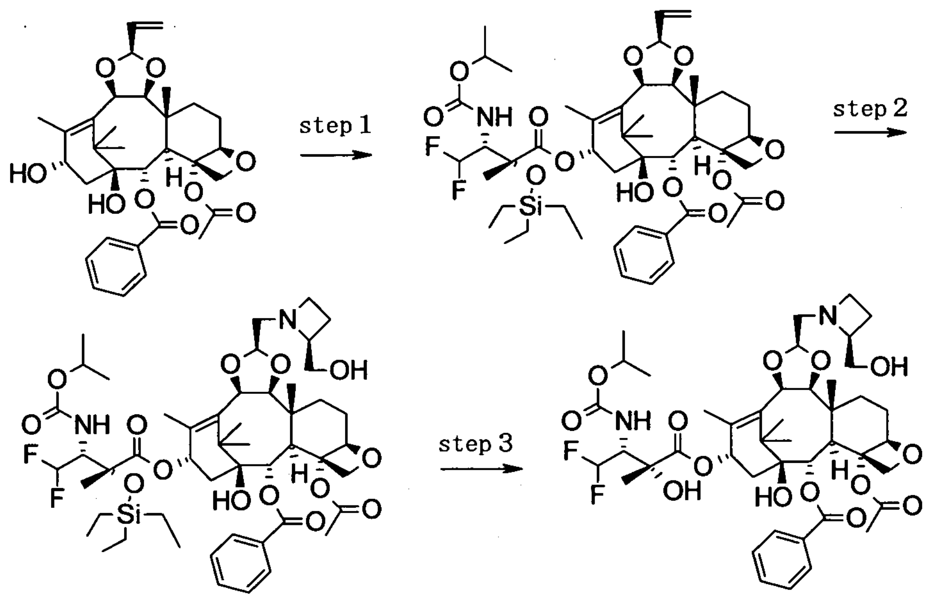


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-4,4-difluoro-3-(isopropoxycarbonylamino)-2-methyl-2-[(triethylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 40 were used as starting materials to perform the same procedure as that of Example 31, Step 1 and thereby to obtain the title compound as white amorphous solid, and used as it was for the following reaction.

Step 2: (1S,2S,3R,4S,5R,8R,9S, 10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-4,4-difluoro-3-(isopropoxycarbonylamino)-2-methyl-2-[(triethylsilyl)oxy]butyrate

The compound obtained in Step 1 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.61-0.80 (6H, m), 0.98 (9H, t, J=8.0Hz), 1.14 (3H, d, J=6.1Hz), 1.18 (3H, d, J=6.1Hz), 1.26 (3H, s), 1.33 (3H, s), 1.50 (3H, s), 1.60 (3H, s), 1.73 (3H, s), 1.80-2.32 (10H, m), 2.49 (3H, s), 2.81 (1H, dd, J=12.9, 5.1Hz), 2.85-2.92 (2H, m), 3.10 (1H, q , J=8.1Hz), 3.42 (1H, d, J=11.7Hz), 3.44-3.52 (2H, m), 3.63 (1H, dd, J=11.3, 3.2Hz), 4.12 (1H, d, J=7.1Hz), 4.24 (1H, d, J=8.3Hz), 4.31 (1H, d, J=8.5Hz), 4.33-4.38 (1H, m), 4.77-4.87 (2H, m), 4.91 (1H, brs), 5.03 (1H, d, J=10.2Hz), 5.18 (1H, d, J=7.6Hz), 5.95 (1H, d, J=4.9Hz), 5.88 (1H, dt, J=54.1, 4.9Hz), 6.12 (1H, t, J=8.5Hz), 7.49 (2H, t, J=7.6Hz), 7.61 (1H, t, J=8.3Hz), 8.16 (2H, d, J=8.0Hz).
FAB-MS; m/z: 993 $(M+H)^+$.

Step 3: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-4,4-difluoro-2-hydroxy-3-(isopropoxycarbonylamino)-2-methylbutyrate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.20 (3H, d, J=7.1Hz), 1.23 (3H, d, J=6.1Hz), 1.27 (3H, s), 1.48 (3H, s), 1.60 (3H, s), 1.63 (3H, s), 1.75 (3H, s), 1.79-2.35 (11H, m), 2.42 (3H, s), 2.83 (1H, dd, J=12.9, 5.1Hz), 2.90 (1H, dd, J=12.4, 5.4Hz), 2.94 (1H, d, J=5.1Hz), 3.10 (1H, q , J=8.1Hz), 3.43 (1H, d, J=11.7Hz), 3.45-3.53 (2H, m), 3.65 (1H, dd, J=11.3, 2.3Hz), 4.12 (1H, d, J=7.1Hz), 4.26 (1H, d, J=8.3Hz), 4.32 (1H, d, J=8.5Hz), 4.35-4.50 (1H, m), 4.85-4.95 (3H, m), 5.21 (1H, d, J=7.6Hz), 5.46 (1H, d, J=8.8Hz), 5.99 (1H, d, J=4.9Hz), 6.09 (1H, dt, J=54.1, 2.7Hz), 6.20 (1H, t, J=8.5Hz), 7.49 (2H, t, J=7.6Hz), 7.61 (1H, t, J=8.3Hz), 8.15 (2H, d, J=8.0Hz).
FAB-MS; m/z: 879 $(M+H)^+$.

[Example 86]

[Formula 158]

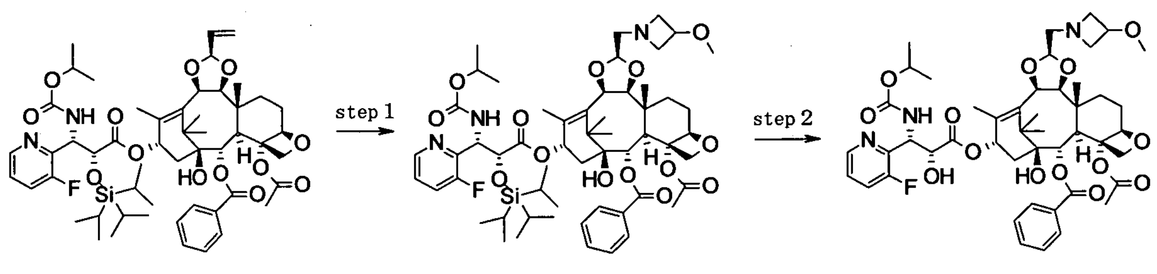


Step 1: (1S,2S,3R,4S,5R,8R,9S,10R,13S)-4-(Acetoxy)-2-(benzoyloxy)-5,20-epoxy-1-hydroxy-9,10-[(1S)-2-(3-methoxyazetidino)ethylidenedioxy]tax-11-en-13-yl

(2R,3S)-3-(3-fluoro-2-pyridyl)-3-(isopropoxycarbonylamino)-2-[(triisopropylsilyl)oxy]propionate

The compound obtained in Example 82, Step 1 and the compound obtained in Reference Example 1 were used as starting materials to perform the same procedure as that of Example 1 and thereby to obtain the title compound as white amorphous solid. $^1$H-NMR (400MHz, $CDCl_3$) δ : 0.87-0.98 (21H, m), 1.16-1.23 (6H, m), 1.35 (3H, s), 1.51 (3H, s), 1.55-2.38 (7H, m), 1.62 (3H, s), 1.78 (3H, s), 2.47 (3H, s), 2.79 (1H, dd, J=12.4, 6.1Hz), 2.87 (1H, dd, J=12.9, 4.6Hz), 2.96 (1H, d, J=5.4Hz), 3.04-3.12 (2H, m), 3.27 (3H, s), 3.70-3.78 (2H, m), 4.04-4.12 (1H, m), 4.16 (1H, d, J=7.8Hz), 4.21 (1H, d, J=8.5Hz), 4.36 (1H, d, J=7.8Hz), 4.77-4.86 (2H, m), 4.95 (1H, brs), 4.97 (1H, d, J=3.4Hz), 5.18 (1H, d, J=7.6Hz),

5.62 (1H, d, J=9.5Hz), 5.97 (1H, d, J=5.1Hz), 6.05-6.15 (2H, m), 7.24-7.29 (1H, m), 7.40 (1H, t, J=9.1Hz), 7.47 (2H, t, J=7.8Hz), 7.59 (1H, t, J=7.4Hz), 8.16 (2H, d, J=8.5Hz), 8.38 (1H, d, J=4.6Hz).
FAB-MS; m/z: 1066 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-(3-methoxyazetidino)ethylidenedioxy]tax-11-en-13-yl

(2R,3S)-3-(3-fluoro-2-pyridyl)-2-hydroxy-3-(isopropoxycarbonylamino)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.15-1.24 (6H, m), 1.28 (3H, s), 1.49 (3H, s), 1.54-2.46 (8H, m), 1:61 (3H, s), 1.76 (3H, s), 2.33 (3H, s), 2.79 (1H, dd, J=12.4, 5.6Hz), 2.88 (1H, dd, J=12.4, 4.6Hz), 2.92 (1H, d, J=5.6Hz), 3.06-3.11 (2H, m), 3.27 (3H, s), 3.69-3.78 (2H, m), 4.03-4.15 (2H, m), 4.23 (1H, d, J=9.0Hz), 4.32 (1H, d, J=9.0Hz), 4.70 (1H, d, J=2.7Hz), 4.81-4.93 (3H, m), 5.21 (1H, d, J=7.1Hz), 5.70 (1H, d, J=8.3Hz), 5.99 (1H, d, J=5.1Hz), 6.11 (1H, t, J=8.7Hz), 6.25 (1H, d, J=7.8Hz), 7.27-7.32 (1H, m), 7.41-7.51 (3H, m), 7.60 (1H, t, J=7.2Hz), 8.13 (2H, d, J=8.5Hz), 8.39 (1H, d, J=4.6Hz).
FAB-MS; m/z: 910 $(M+H)^+$.

[Example 87]

[Formula 159]

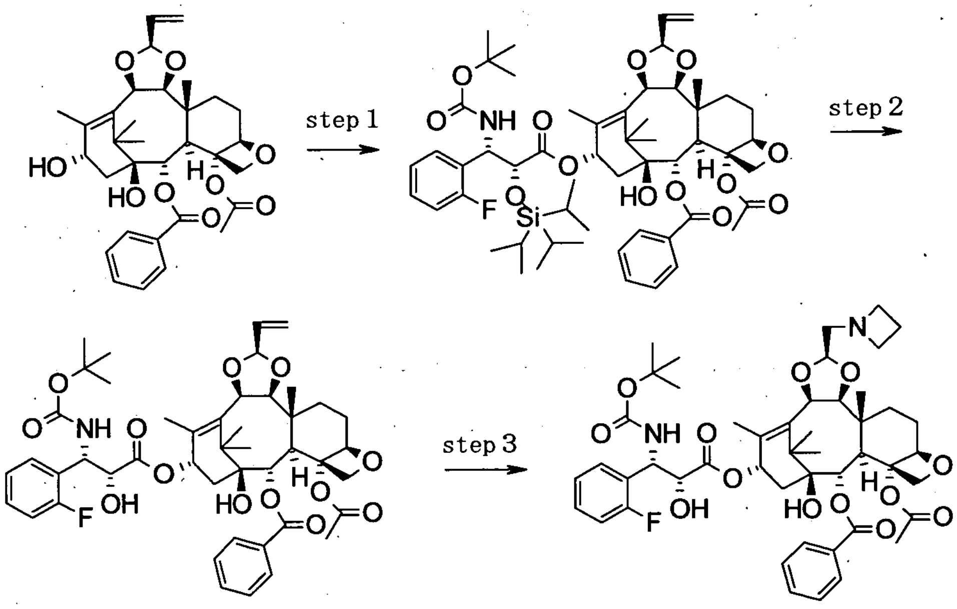


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(2-fluorophenyl)-2-[(triisopropylsilyl)oxy]propionate

(1S,2S,3R,4S,5R,8R,9S, 10R, 13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 41, Step 4 were used as starting materials to perform the same procedure as that of Example 31, Step 1 and thereby to obtain the title compound as white amorphous solid.
$^1$H-NMR (400MHz, $CDCl_3$) δ : 0.72-1.06 (21H, m), 1.08-2.37 (7H, m), 1.35 (3H, s), 1.38 (9H, s), 1.53 (3H, s), 1.65 (3H, s), 1.81 (3H, s), 2.47 (3H, s), 2.97 (1H, d, J=5.1Hz), 4.21-4.23 (2H, m), 4.34 (1H, d, J=8.3Hz), 4.93 (2H, s), 5.20 (1H, d, J=6.1Hz), 5.27 (1H, d, J=6.8Hz), 5.45 (2H, d, J=10.2Hz), 5.54-5.60 (2H, m), 5.96-6.04 (2H, m), 6.15 (1H, t, J=8.3Hz),

7.04-7.09 (1H, m), 7.14 (1H, t, J=7.6Hz), 7.25-7.34 (2H, m), 7.47 (2H, t, J=7.3Hz), 7.56-7.60 (1H, m), 8.14-8.16 (2H, m). FAB-MS; m/z: 1006 $(M+H)^+$, 1028 $(M+Na)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(2-fluorophenyl)-2-hydroxypropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.24-2.10 (6H, m), 1.28 (3H, s), 1.41 (9H, s), 1.49 (3H, s), 1.64 (3H, s), 1.79 (3H, s), 2.29 (3H, s), 2.38 (1H, dd, J=15.1, 9.5Hz), 2.94 (1H, d, J=4.9Hz), 4.18 (1H, d, J=6.8Hz), 4.25 (1H, d, J=8.3Hz), 4.32 (1H, d, J=8.3Hz), 4.46 (1H, brs), 4.62 (1H, brs), 4.92 (1H, s), 5.25 (1H, d, J=6.1Hz), 5.32 (1H, d, J=6.8Hz), 5.47 (1H, d, J=10.5Hz), 5.56-5.67 (3H, m), 5.97-6.06 (2H, m), 6.15 (1H, t, J=8.4Hz), 7.05-7.10 (1H, m), 7.14-7.18 (1H, m), 7.27-7.33 (1H, m), 7.40-7.52 (3H, m), 7.58-7.62 (1H, m), 8.11-8.13 (2H, m).
FAB-MS; m/z: 850 $(M+H)^+$, 872 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-3-(2-fluorophenyl)-2-hydroxypropionate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.24-2.17 (9H, m), 1.26 (3H, s), 1.40 (9H, s), 1.49 (3H, s), 1.61 (3H, s), 1.77 (3H, s), 2.29 (3H, s), 2.34-2.40 (1H, m), 2.75 (1H, dd, J=12.5, 5.9Hz), 2.83 (1H, dd, J=12.5, 4.2Hz), 2.92 (1H, d, J=4.9Hz), 3.32-3.40 (4H, m), 4.09-4.13 (1H, m), 4.24 (1H, d, J=8.5Hz), 4.31 (1H, d, J=8.5Hz), 4.62 (1H, s), 4.88 (1H, dd, J=5.9, 4.2Hz), 4.91 (1H, s), 5.22 (1H, d, J=7.1Hz), 5.64 (2H, s), 6.00 (1H, d, J=4.9Hz), 6.13 (1H, t, J=8.5Hz), 7.05-7.10 (1H, m), 7.16 (1H, td, J=7.5, 1.1Hz), 7.26-7.32 (1H, m), 7.37-7.49 (3H, m), 7.58-7.62 (1H, m), 8.11-8.13 (2H, m).
FAB-MS; m/z: 893 $(M+H)^+$.

[Example 88]

(1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(tert-butoxycarbonylamino)-3-(2-fluorophenyl)-2-hydroxypropionate

[Formula 160]

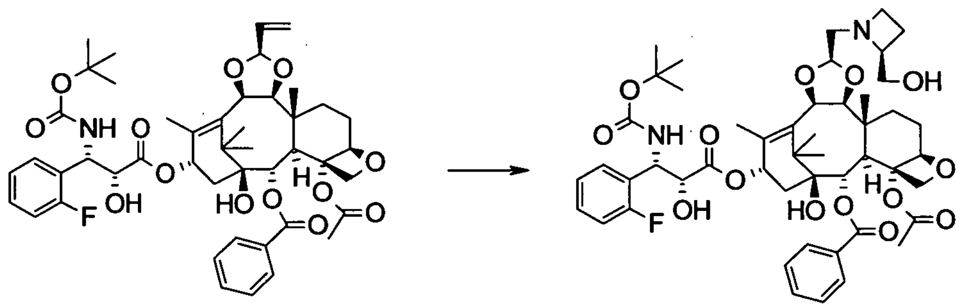

The compound obtained in Example 87, Step 2 and the compound obtained in Reference Example 25 were used as starting materials to perform the same procedure as that of Example 1 and thereby to obtain the title compound as white amorphous solid.

$^1$H-NMR (400MHz, $CDCl_3$) δ : 1.24-2.13 (9H, m), 1.25 (3H, s), 1.41 (9H, s), 1.49 (3H, s), 1.59 (3H, s), 1.77 (3H, s), 2.20-2.40 (2H, m), 2.29 (3H, s), 2.83 (1H, dd, J=12.8, 4.5Hz), 2.88-2.92 (2H, m), 3.11 (1H, dd, J=16.2, 8.7Hz), 3.43 (1H, dd, J=11.5, 2.4Hz), 3.46-3.52 (2H, m), 3.66 (1H, dd, J=11.5, 2.9Hz), 4.12 (1H, d, J=7.3Hz), 4.24 (1H, d, J=8.5Hz), 4.31 (1H, d, J=8.5Hz), 4.62 (1H, s), 4.88-4.91 (2H, m), 5.24 (1H, d, J=6.8Hz), 5.60-5.67 (2H, m), 5.99 (1H, d, J=4.9Hz), 6.13 (1H, t, J=8.1Hz), 7.05-7.10 (1H, m), 7.14-7.18 (1H, m), 7.26-7.32 (1H, m), 7.37-7.49 (3H, m), 7.58-7.62 (1H, m), 8.11-8.13 (2H, m).
FAB-MS; m/z: 923 $(M+H)^+$.

[Example 89]

[Formula 161]

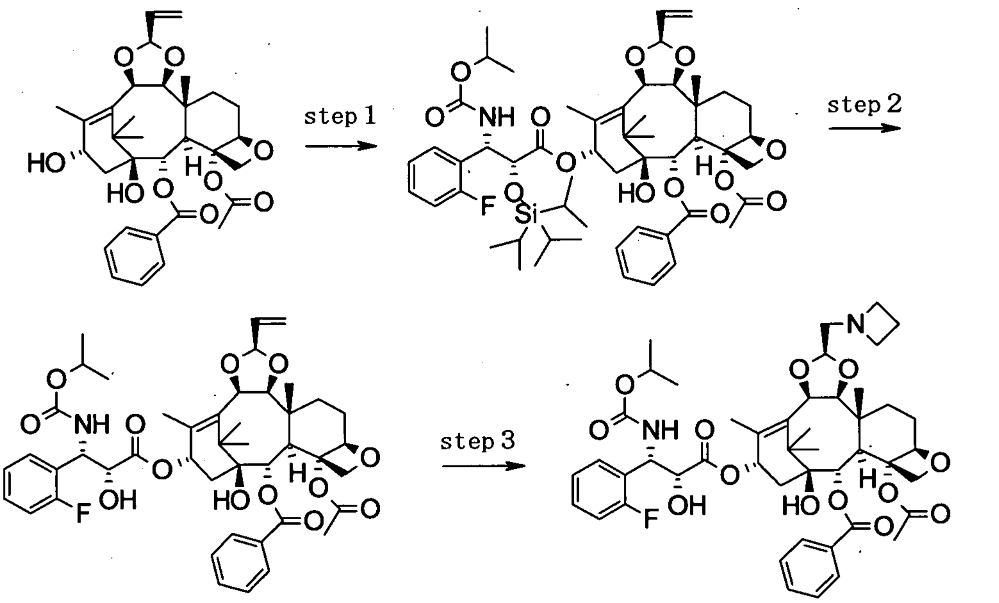


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(2-fluorophenyl)-3-(isopropoxycarbonylamino)-2-[(triisopropylsilyl)oxy]propionate

(1S,2S,3R,4S,5R,8R,9S, 10R,13S)-4-(Acetoxy)-2-(benzoyloxy)-1,13-dihydroxy-5, 20-epoxy-9,10-[(1S)-2-propenylidenedioxy]tax-11-ene and the compound obtained in Reference Example 42 were used as starting materials to perform the same procedure as that of Example 31, Step 1 and thereby to obtain the title compound as white amorphous solid.
$^1$H-NMR (400MHz, $CDCl_3$) δ : 0.72-1.05 (21H, m), 1.06-2.33 (13H, m), 1.36 (3H, s), 1.52 (3H, s), 1.65 (3H, s), 1.80 (3H, s), 2.47 (3H, s), 2.96 (1H, d, J=5.1Hz), 4.21-4.23 (2H, m), 4.35 (1H, d, J=8.3Hz), 4.78-4.85 (2H, m), 4.94-4.95 (2H, m), 5.20 (1H, d, J=6.3Hz), 5.26 (1H, d, J=7.1Hz), 5.44-5.60 (4H, m), 5.96-6.04 (2H, m), 6.15 (1H, t, J=8.8Hz), 7.05-7.09 (1H, m), 7.12-7.16 (1H, m), 7.21-7.33 (2H, m), 7.48 (2H, t, J=7.3Hz), 7.57-7.61 (1H, m), 8.14-8.16 (2H, m).
FAB-MS; m/z: 992 $(M+H)^+$, 1041 $(M+Na)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxyltax-11-en-13-yl

(2R,3S)-3-(2-fluorophenyl)-2-hydroxy-3-(isopropoxycarbonylamino)propionate

The compound obtained in Step 1 mentioned above was used as a starting material to perform the same procedure as that of Example 31, Step 2 and thereby to obtain the title compound as white amorphous solid.

$^1$H-NMR (400MHz, $CDCl_3$) δ: 0.91-2.10 (7H, m), 1.20 (6H, d, J=6.1Hz), 1.27 (3H, s), 1.49 (3H, s), 1.64 (3H, s), 1.77 (3H, s), 2.28 (3H, s), 2.36 (1H, dd, J=15.0, 9.9Hz), 2.93 (1H, d, J=4.6Hz), 4.17 (1H, d, J=6.8Hz), 4.25 (1H, d, J=8.3Hz), 4.32 (1H, d, J=8.3Hz), 4.63 (1H, s), 4.86-4.98 (2H, m), 5.25 (1H, d, J=6.1Hz), 5.32 (1H, d, J=6.3Hz), 5.46 (1H, d, J=10.3Hz), 5.58 (1H, d, J=17.1Hz), 5.71 (2H, brs), 5.97-6.05 (2H, m), 6.17 (1H, t, J=8.2Hz), 7.08 (1H, t, J=9.4Hz), 7.16 (1H, t, J=7.6Hz), 7.26-7.32 (1H, m), 7.40-7.52 (3H, m), 7.60 (1H, t, J=7.4Hz), 8.12 (2H, d, J=8.3Hz).
FAB-MS; m/z: 836 $(M+H)^+$, 858 $(M+Na)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(2-fluorophenyl)-2-hydroxy-3-(isopropoxycarbonylamino)propionate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.00-2.16 (9H, m), 1.20 (6H, d, J=6.3Hz), 1.25 (3H, s), 1.48 (3H, s), 1.61 (3H, s), 1.75 (3H, s), 2.28 (3H, s), 2.35 (1H, dd, J=15.1, 9.8Hz), 2.74 (1H, dd, J=12.4, 5.7Hz), 2.83 (1H, dd, J=12.4, 4.4Hz), 2.92 (1H, d, J=4.9Hz), 3.32-3.40 (4H, m), 4.10 (1H, d, J=7.1Hz), 4.24 (1H, d, J=8.5Hz), 4.31 (1H, d, J=8.5Hz), 4.63 (1H, s), 4.87 (1H, dd, J=5.6, 4.6Hz), 4.91 (2H, s), 5.22 (1H, d, J=6.8Hz), 5.61-5.75 (2H, m), 6.00 (1H, d, J=4.9Hz), 6.15 (1H, t, J=8.0Hz), 7.05-7.10 (1H, m), 7.16 (1H, t, J=7.6Hz), 7.27-7.32 (1H, m), 7.37-7.48 (3H, m), 7.60 (1H, t, J=7.3Hz), 8.11-8.13 (2H, m).
FAB-MS; m/z: 879 $(M+H)^+$.

[Example 90]

(1S,2S,3R,4S,5R,8R,9S,10R, 13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(2-fluorophenyl)-2-hydroxy-3-(isopropoxycarbonylamino)propionate

[Formula 162]

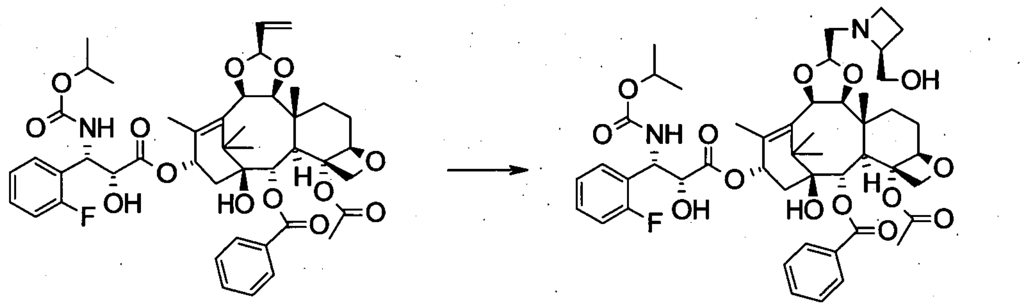

The compound obtained in Example 89, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.88-2.05 (9H, m), 1.20 (6H, d, J=6.1Hz), 1.25 (3H, s), 1.48 (3H, s), 1.59 (3H, s), 1.75 (3H, s), 2.20-2.38 (2H, m), 2.28 (3H, s), 2.83 (1H, dd, J=12.9, 4.6Hz), 2.88-2.93 (2H, m), 3.11 (1H, dd, J=16.2, 8.7Hz), 3.43 (1H, dd, J=11.5, 2.6Hz), 3.46-3.52 (2H, m), 3.66 (1H, dd, J=11.5, 2.9Hz), 4.12 (1H, d, J=7.3Hz), 4.24 (1H, d, J=8.3Hz), 4.31 (1H, d, J=8.3Hz), 4.63 (1H, s), 4.86-4.91 (3H, m), 5.24 (1H, d, J=7.1Hz), 5.63-5.76 (2H, m), 5.99 (1H, d, J=5.1Hz), 6.14 (1H, t, J=8.3Hz), 7.05-7.10 (1H, m), 7.16 (1H, td, J=7.6, 1.0Hz), 7.27-7.32 (1H, m), 7.38-7.48 (3H, m), 7.58-7.62 (1H, m), 8.10-8.13 (2H, m).
FAB-MS; m/z: 909 $(M+H)^+$.

[Example 91]

[Formula 163]

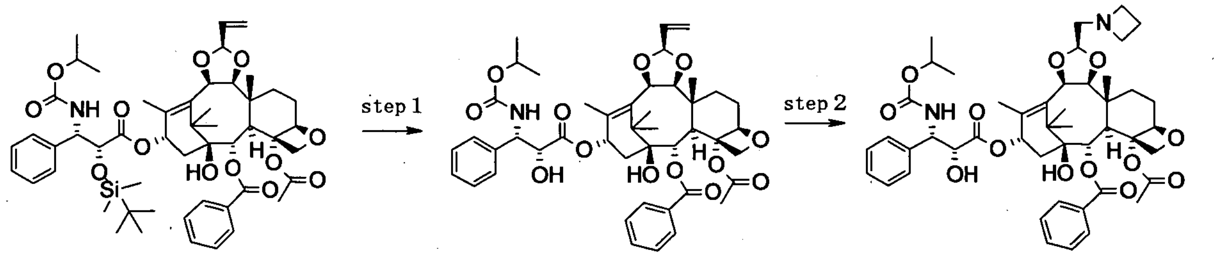


Step 1: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3S)-2-hydroxy-3-(isopropoxycarbonylamino)-3-phenylpropionate

The compound obtained in Example 74, Step 1 was used as a starting material to perform the same procedure as that of Example 31, Step 2 and thereby to obtain the title compound as white amorphous solid.
$^1$H-NMR (400MHz, $CDCl_3$) δ : 0.86-2.10 (6H, m), 1.20 (6H, d, J=6.9Hz), 1.25 (3H, s), 1.48 (3H, s), 1.60 (3H, s), 1.63 (3H, s), 2.27 (3H, s), 2.33-2.40 (1H, m), 2.91 (1H, d, J=5.2Hz), 4.15 (1H, d, J=6.9Hz), 4.24 (1H, d, J=8.6Hz), 4.32 (1H, d, J=8.6Hz), 4.62 (2H, d, J=9.6Hz), 4.85-4.92 (2H, m), 5.24 (1H, d, J=6.1Hz), 5.27-5.30 (1H, m), 5.37 (1H, d, J=9.3Hz), 5.46 (1H, d, J=10.8Hz), 5.62 (1H, t, J=25.6Hz), 5.81 (1H, d, J=9.8Hz), 5.96-6.05 (2H, m), 6.10 (1H, t, J=8.6Hz), 7.27-7.32 (1H, m), 7.34-7.38 (2H, m), 7.41-7.49 (4H, m), 7.55-7.62 (1H, m), 8.12 (2H, dd, J=8.1, 1.2Hz).

Step 2: (1S,2S,3R,4S,5R,8R,9S,10R,13S)-4-Acetoxy-9,10-[(1S)-2-(azetidino)ethylidenedioxyl-2-(benzoyloxy)-5,20-epoxy-1-hydroxytax-11-en-13-yl

(2R,3S)-2-hydroxy-3-(isopropoxycarbonylamino)-3-phenylpropionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 1.20 (6H, d, J=6.1Hz), 1.24 (3H, s), 1.47 (3H, s), 1.59-2.17 (15H, m), 2.27 (3H, s), 2.35 (1H, d, J=15.2, 10.1Hz), 2.61-2.90 (3H, m), 3.30-3.43 (4H, m), 4.08 (1H, d, J=7.1Hz), 4.24 (1H, d, J=8.6Hz), 4.31 (1H, d, J=8.6Hz), 4.63 (1H, s), 4.84-4.92 (3H, m), 5.19 (1H, d, J=6.9Hz), 5.36 (1H, d, J=9.1H), 5.81 (1H, d, J=9.1Hz), 6.00 (1H, d, J=4.9Hz), 6.08 (1H, t, J=8.3Hz), 7.27-7.37 (3H, m), 7.41-7.49 (4H, m), 7.58-7.62 (1H, m), 8.12 (2H, dd, J=8.3, 1.2Hz).
FAB-MS; m/z: 861 $(M+H)^+$.

[Example 92]

[Formula 164]

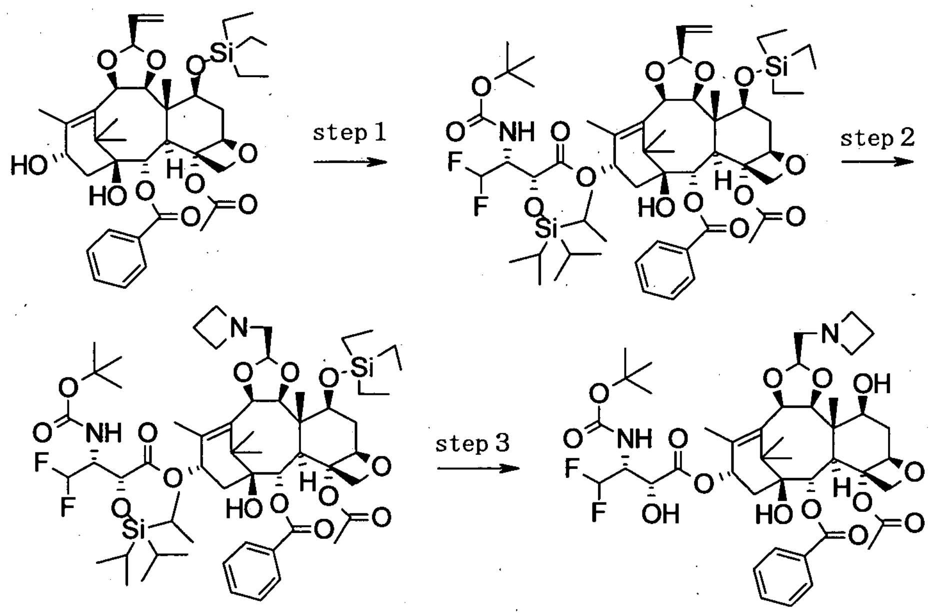


Step 1: (1S,2S,3R,4S,5R,7S,8R,9S,10R,13S)-4-Acetoxy-2-(benzoyloxy)-5,20-epoxy-1-hydroxy-9,10-[(1S)-2-propenylidenedioxy]-7-[(triethylsilyl)oxy]tax-11-en-13-yl

(2R,3R)-3-(tert-butoxycarbonylamino)-4,4-difluoro-2-[(triisopropylsilyl)oxy]butyrate

(1S,2S,3R,4S,5R,7S,8R,9S,10R,13S)-4-(Acetoxy)-2-(benzoyloxy)-1,13-dihydroxy-5,20-epoxy-9,10-[(1S)-2-propenylidenedioxy]-7-[(triethylsilyl)oxy]tax-11-ene and (3R,4R)-1-(tert-butoxycarbonyl)-4-(difluoromethyl)-3-[(triisopropylsilyl)oxy]-2-azetidinone were used as starting materials to perform the same procedure as that of Example 31, Step 1 and thereby to obtain the title compound as white amorphous solid.
$^1$H-NMR (400MHz, $CDCl_3$) δ : 0.60-0.67 (6H, m), 0.87-1.76 (4H, m), 0.99 (9H, t, J=8.1Hz), 1.10-1.12 (21H, m), 1.19 (3H, s), 1.37 (9H, s), 1.59 (3H, s), 1.75 (3H, s), 2.03-2.10 (1H, m), 2.20-2.26 (1H, m), 2.32-2.42 (2H, m), 2.34 (3H, s), 3.17 (1H, d, J=5.4Hz), 3.92 (1H, dd, J=10.2, 5.5Hz), 4.32-4.40 (3H, m), 4.64 (1H, d, J=8.8Hz), 4.80-4.85 (2H, m), 5.02 (1H, d, J=10.3Hz), 5.06 (1H, d, J=5.9Hz), 5.28 (1H, d, J=8.8Hz), 5.46 (1H, d, J=10.8Hz), 5.56 (1H, d, J=17.7Hz), 5.78 (1H, td, Jt=56.1, Jd=6.1Hz), 5.90 (1H, d, J=5.6Hz), 6.05 (1H, t, J=9.3Hz), 6.10-6.18 (1H, m), 7.50 (2H, t, J=7.6Hz), 7.59 (1H, tt, J=7.5, 1.6Hz), 8.11 (2H, d, J=7.1Hz).

Step 2: (1S,2S,3R,4S,5R,7S,8R,9S,10R,13S)-4-Acetoxy-9,10-[(1S)-2-(azetidino)ethylidenedioxy]-2-(benzoyloxy)-5,20-epoxy-1-hydroxy-7-[(triethylsilyl)oxy]tax-11-en-13-yl

(2R,3R)-3-(tert-butoxycarbonylamino)-4,4-difluoro-2-[(triisopropylsilyl)oxy]butyrate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.58-0.66 (6H, m), 0.93-2.25 (7H, m), 0.97 (9H, t, J=7.8Hz), 1.09-1.12 (21H, m), 1.19 (3H, s), 1.36 (9H, s), 1.51 (3H, s), 1.73 (3H, s), 1.88 (1H, brs), 2.30-2.39 (2H, m), 2.34 (3H, s), 2.85 (2H, d, J=4.9Hz), 3.15 (1H, d, J=5.4Hz), 3.33 (4H, t, J=7.0Hz), 3.89 (1H, dd, J=10.3, 5.4Hz), 4.30-4.40 (3H, m), 4.49 (1H, d, J=8.8Hz), 4.67 (1H, t, J=5.0Hz), 4.79-4.84 (2H, m), 5.02 (1H, d, J=10.3Hz), 5.18 (1H, d, J=9.1Hz), 5.78 (1H, td, Jt=55.9, Jd=6.1Hz), 5.88 (1H, d, J=5.6Hz), 6.05 (1H, t, J=8.8Hz), 7.50 (2H, t, J=7.6Hz), 7.59 (1H, t, J=7.4Hz), 8.11 (2H, d, J=7.1Hz).

Step 3: (1S,2S,3R,4S,5R,7S,8S,9S,10R,13S)-4-Acetoxy-9,10-[(1S)-2-(azetidino)ethylidenedioxy]-2-(benzoyloxy)-1,7-dihydroxy-5,20-epoxytax-11-en-13-yl

(2R,3R)-3-(tert-butoxycarbonylamino)-4,4-difluoro-2-hydroxybutyrate

The compound obtained in Step 2 mentioned above was used as a starting material to perform the same

procedure as that of Example 43, Step 3 and thereby to obtain the title compound as white amorphous solid.
$^1$H-NMR (400MHz, $CDCl_3$) δ : 1.25 (3H, s), 1.39-1.46 (1H, m), 1.41 (9H, s), 1.62 (3H, s), 1.66 (3H, s), 1.72 (3H, s), 1.89 (1H, s), 2.04-2.39 (7H, m), 2.36 (3H, s), 2.79-2.95 (3H, m), 3.33-3.46 (4H, m), 3.81 (1H, d, J=7.11Hz), 4.08 (1H, brs), 4.33 (1H, d, J=8.8Hz), 4.40 (1H, d, J=8.6Hz), 4.45-4.56 (1H, m), 4.63 (1H, s), 4.87-4.90 (1H, m), 5.11 (1H, s), 5.17-5.23 (2H, m), 5.89 (1H, td, Jt=56.1, Jd=5.2Hz), 6.06 (1H, d, J=4.9Hz), 6.11 (1H, t, J=8.8Hz), 7.49 (2H, t, J=7.7Hz), 7.59-7.64 (1H, m), 8.11-8.13 (2H, m).

FAB-MS; m/z: 865 $(M+H)^+$

[Example 93]

(1S,2S,3R,4S,5R,8R,9S,10R,13S)-4-(Acetoxy)-9,10-{(1S)-2-[(2S)-2-(dimethylcarbamoyl) azetidino]ethylidenedioxy}-2-(benzoyloxy)-5,20-epoxy-1-hydroxytax-11-en-13-yl

(2R,3R)-3-(tert-butoxycarbonylamino)-4,4-difluoro-2-hydroxybutyrate

[Formula 165]

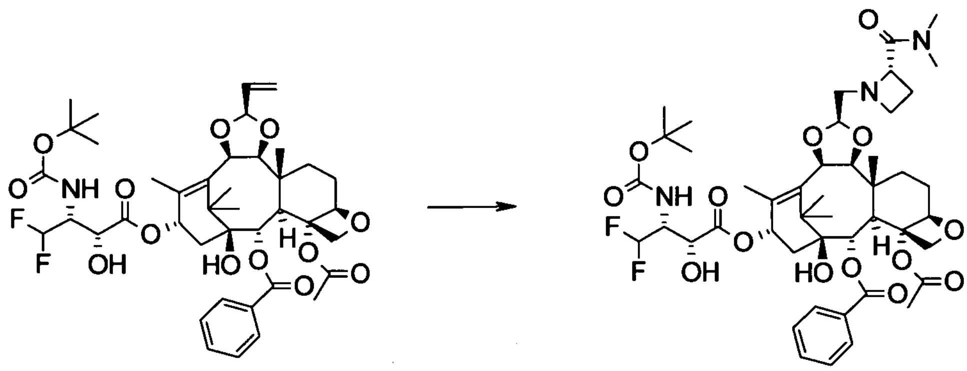

The compound obtained in Example 31, Step 2 and the compound obtained in Reference Example 12, Step 2 were used as starting materials to perform the same procedure as that of Example 1 and thereby to obtain the title compound as white amorphous solid.
$^1$H-NMR (400MHz, $CDCl_3$) δ : 1.16-2.37 (9H, m), 1.18 (3H, s), 1.44 (9H, s), 1.48 (3H, s), 1.58 (3H, s), 1.75 (3H, s), 2.33 (3H, s), 2.82-3.01 (3H, m), 2.92 (3H, s), 2.94 (3H, s), 3.09 (1H, q , J=8.6Hz), 3.54-3.60 (1H, m), 3.95 (1H, t, J=8.5Hz), 4.09 (1H, d, J=7.1Hz), 4.24 (1H, d, J=8.3Hz), 4.32 (1H, d, J=8.6Hz), 4.48-4.59 (1H, brm), 4.65 (1H, s), 4.72-4.80 (1H, brm), 4.93 (1H, s), 5.07 (1H, t, J=3.9Hz), 5.19 (1H, d, J=6.9Hz), 5.28 (1H, d, J=10.0Hz), 5.89 (1H, td, J=56.1, 4.7Hz), 6.00 (1H, d, J=5.1Hz), 6.08 (1H, t, J=7.8Hz), 7.48 (2H, t, J=7.7Hz), 7.61 (1H, t, J=7.5Hz), 8.13 (2H, d, J=7.4Hz).
FAB-MS; m/z: 921 $(M+H)^+$.

[Example 94]

(1S,2S,3R,4S,5R,8R,9S,10R,13S)-4-(Acetoxy)-2-(benzoyloxy)-9,10-{(1S)-2-[3-(dimethylcarbamoyl)azetidino]ethylidenedioxy}-5,20-epoxy-1-hydroxytax-11-en-13-yl

(2R,3R)-3-(tert-butoxycarbonylamino)-4,4-difluoro-2-hydroxybutyrate

[Formula 166]

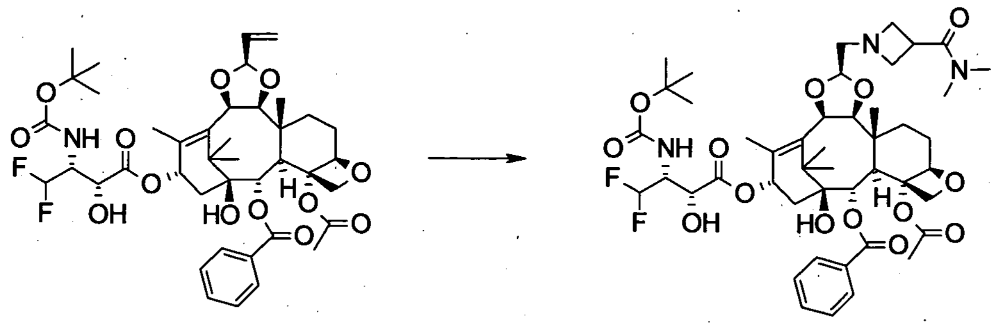

The compound obtained in Example 31, Step 2 and the compound obtained in Reference Example 2, Step 2 were used as starting materials to perform the same procedure as that of Example 1 and thereby to obtain the title compound as white amorphous solid.

$^1$H-NMR (400MHz, $CDCl_3$) δ : 1.15-2.39 (9H, m), 1.23 (3H, s), 1.42 (9H, s), 1.48 (3H, s), 1.60 (3H, s), 1.77 (3H, s), 2.33 (3H, s), 2.79 (1H, dd, J=12.5, 5.6Hz), 2.86 (1H, dd, J=12.5, 4.4Hz), 2.91 (3H, s), 2.96 (3H, s), 3.39-3.49 (2H, m), 3.50-3.58 (1H, m), 3.68-3.75 (2H, m), 4.11 (1H, d, J=7.1Hz), 4.24 (1H, d, J=8.6Hz), 4.33 (1H, d, J=8.6Hz), 4.45-4.60 (1H, brm), 4.64 (1H, s), 4.88 (1H, t, J=4.9Hz), 4.92 (1H, s), 5.22 (1H, d, J=6.9Hz), 5.26 (1H, d, J=9.8Hz), 5.90 (1H, td, J=55.9, 4.9Hz), 5.99 (1H, d, J=4.9Hz), 6.11 (1H, t, J=7.8Hz), 7.48 (2H, t, J=7.7Hz), 7.61 (1H, t, J=7.5Hz), 8.13 (2H, d, J=7.4Hz).

FAB-MS; m/z: 921 $(M+H)^+$.

[Example 95]

[Formula 167]

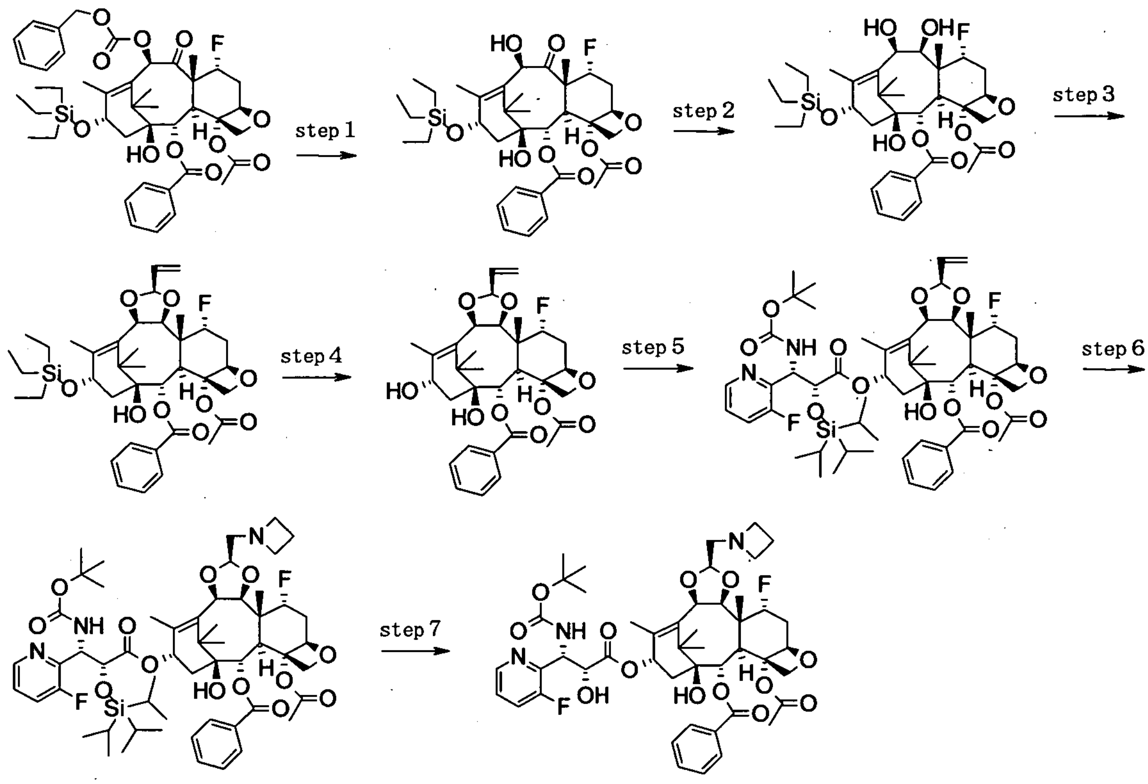

Step 1: (1S,2S,3R,4S,5R,7R,8R,10R,13S)-4-(Acetoxy)-2-(benzoyloxy)-1,10-dihydroxy-5,20-epoxy-7-fluoro-9-oxo-13-[(triethylsilyl)oxy]tax-11-ene

(1S,2S,3R,4S,5R,7R,8R,10R,13S)-4-(Acetoxy)-2-(benzoyloxy)-10-[(benzyloxycar bonyl)oxy]-5,20-epoxy-7-fluoro-1-hydroxy-9-oxo-13-[(triethylsilyl)oxy]tax-11-ene (Bioorg. Med. Chem. Lett., 2002, 12, 2815, 1.25 g) was dissolved in a mixed solvent of ethanol (20 ml) and tetrahydrofuran (10 ml), and the solution was added with 10% palladium/carbon (0.3 g), and stirred for 1 hour under a hydrogen atmosphere. The catalyst was removed by filtration, and then the filtrate was concentrated under reduced pressure. The resulting residue was purified by silica gel chromatography [developing solvent; ethyl acetate:n-hexane=1:2 (v/v)] to obtain the title compound (1.02 g) as colorless solid.
$^1$H-NMR (400MHz, $CDCl_3$) δ : 0.60-0.72 (6H, m), 1.01 (9H, t, J=7.8Hz), 1.09 (3H, s), 1.17 (3H, s), 1.77 (3H, s), 1.96 (3H, s), 2.11-2.36 (3H, m), 2.30 (3H, s), 2.52-2.65 (1H, m), 4.05 (1H, d, J=7.2Hz), 4.06 (1H, s), 4.28 (1H, d, J=8.3Hz), 4.37 (1H, d, J=8.3Hz), 4.55 (1H, dd, J=47.4, 4.2Hz), 4.95 (1H, t, J=8.8Hz), 5.05 (1H, d, J=8.5Hz), 5.27 (1H, s), 5.71 (1H, d, J=7.2Hz), 6.41 (1H, s), 7.49 (2H, t, J=7.8Hz), 7.60 (1H, t, J=7.8Hz), 8.12 (2H, d, J=7.8Hz).

Step 2: (1S,2S,3R,4S,5R,7R,8R,9S,10R,13S)-4-(Acetoxy)-2-(benzoyloxy)-5,20-epoxy-7-fluoro-13-[(triethylsilyl)oxy]-1,9,10-trihydroxytax-11-ene

The compound obtained in Step 1 mentioned above (1.00 g) was dissolved in tetrahydrofuran (20 ml), and the solution was cooled on ice. Then, the solution was added dropwise with borane/tetrahydrofuran complex (1 mole solution in tetrahydrofuran, 45 ml), and stirred at the same temperature for 4 hours. The reaction mixture was added with saturated aqueous sodium hydrogencarbonate, and extracted with ethyl acetate, and the organic layer was dried over anhydrous sodium sulfate. The solvent was evaporated under reduced pressure, and then the resulting residue was purified by silica gel chromatography [developing solvent; ethyl acetate:n-hexane=1:2 (v/v)] to obtain the title compound (270 mg) as colorless solid. $^1$H-NMR (400MHz, $CDCl_3$) δ : 0.59-0.72 (6H, m), 1.02 (9H, t, J=7.8Hz), 1.26 (3H, s), 1.62 (3H, s), 1.73 (3H, s), 1.84 (3H, d, J=3.4Hz), 2.19-2.28 (3H, m), 2.26 (3H, s), 2.47-2.55 (1H, m), 3.23 (1H, d, J=2.2Hz), 3.45 (1H, d, J=5.1Hz), 4.11-4.14 (1H, m), 4.15 (1H, d, J=8.3Hz), 4.38 (1H, d, J=8.3Hz), 4.74 (1H, dd, J=47.6, 4.4Hz), 5.01 (1H, t, J=8.3Hz), 5.03 (1H, d, J=8.3Hz), 5.33 (1H, br), 5.99 (1H, d, J=7.3Hz), 7.47 (2H, t, J=8.3Hz), 7.56 (1H, t, J=8.3Hz), 8.11 (2H, d, J=8.3Hz).

Step 3: (1S,2S,3R,4S,5R,7R,8R,9S, 10R, 13S)-4-(Acetoxy)-2-(benzoyloxy)-5,20-epoxy-7-fluoro-1-hydroxy-9,10-[(1S)-2-propenylidenedioxy]-13-[(triethylsilyl)oxy]tax-11-ene

The compound obtained in Step 2 mentioned above and 3,3-diethoxy-1-propene were used as starting materials to perform the same procedure as that of Example 81, Step 1 and thereby to obtain the title compound as colorless solid.
$^1$H-NMR (400MHz, $CDCl_3$) δ : 0.59-0.73 (6H, m), 1.01 (9H, t, J=7.8Hz), 1.17 (3H, s), 1.53 (3H, s), 1.62 (3H, s), 1.74 (1H, s), 1.88 (3H, s), 2.06-2.26 (3H, m), 2.27 (3H, s), 2.47-2.58 (1H, m), 3.47 (1H, d, J=5.6Hz), 4.27 (1H, d, J=8.5Hz), 4.37 (1H, d, J=8.8Hz), 4.38 (1H, d, J=8.5Hz), 4.79 (1H, dt, J=45.5, 5.0Hz), 4.96 (1H, brt, J=8.8Hz), 5.00 (1H, dd, J=7.0, 3.2Hz), 5.12 (1H, d, J=5.9Hz), 5.43 (1H, d, J=8.8Hz), 5.47 (1H, d, J=10.5Hz), 5.59 (1H, d, J=17.1Hz), 5.85 (1H, d, J=5.6Hz), 6.11 (1H, ddd, J=17.1, 10.5, 5.9Hz), 7.48 (2H, t, J=8.3Hz), 7.59 (1H, t, J=8.3Hz), 8.11 (2H, d, J=8.3Hz).

Step 4: (1S,2S,3R,4S,5R,7R,8R,9S,10R,13S)-4-(Acetoxy)-2-(benzoyloxy)-1,13-dihydroxy-5,20-epoxy-7-fluoro-9,10-[(1S)-2-propenylidenedioxy]tax-11-ene

The compound obtained in Step 3 mentioned above was used as a starting material to perform the same procedure as that of Example 84, Step 4 and thereby to obtain the title compound as colorless solid.
$^1$H-NMR (400MHz, $CDCl_3$) δ : 1.08 (3H, s), 1.57 (3H, s), 1.64 (3H, s), 1.73 (1H, s), 1.93 (3H, s), 2.08-2.36 (3H, m), 2.32 (3H, s), 2.43-2.55 (2H, m), 3.58 (1H, d, J=5.3Hz), 4.27 (1H, d, J=7.8Hz), 4.28 (1H, d, J=8.5Hz), 4.40 (1H, d, J=7.8Hz), 4.78 (1H, dt, J=45.8, 4.9Hz), 4.74-4.79 (1H, m), 4.94 (1H, d, J=4.9Hz), 5.13 (1H, d, J=6.1Hz), 5.45 (1H, d, J=8.5Hz), 5.49 (1H, d, J=10.5Hz), 5.59 (1H, d, J=17.2Hz), 5.90 (1H, d, J=5.3Hz), 6.14 (1H, ddd, J=17.2, 10.5, 6.1Hz), 7.49 (2H, t, J=7.8Hz), 7.61(1H, t, J=7.8Hz), 8.12 (2H, d, J=7.8Hz).

Step 5: (1S,2S,3R,4S,5R,7R,8R,9S,10R,13S)-4-(Acetoxy)-2-(benzoyloxy)-1-hydroxy-5,20-epoxy-7-fluoro-9,10-[(1S)-2-propenylidenedioxy]tax-11-en-13-yl

(2R,3S)-3-(tert-butoxycarbonylamino)-3-(3-fluoro-2-pyridyl)-2-[(triisopropylsilyl)oxy]propionate

The compound obtained in Step 4 mentioned above and (3R,4S)-1-(tert-butoxycarbonyl)-4-(3-fluoro-2-pyridyl)-3-[(triisopropylsilyl)oxy]-2-azetidinone were used as starting materials to perform the same procedure as that of Example

31, Step 1 and thereby to obtain the title compound as white amorphous solid.
$^1$H-NMR (400MHz, $CDCl_3$) δ : 0.82-0.95 (21H, m), 1.28 (3H, s), 1.38 (9H, s), 1.51-2.54 (5H, m), 1.57 (3H, s), 1.65 (3H, s), 1.80 (3H, s), 2.50 (3H, s), 3.52 (1H, d, J=5.6Hz), 4.34 (1H, d, J=8.3Hz), 4.36-4.42 (2H, m), 4.73-4.90 (1H, m), 4.95 (1H, brs), 4.97-5.02 (1H, m), 5.12 (1H, d, J=5.9Hz), 5.41 (1H, d, J=8.1Hz), 5.47 (1H, d, J=11.0Hz), 5.56-5.64 (2H, m), 5.93 (1H, d, J=5.4Hz), 6.01-6.18 (3H, m), 7.24-7.29 (1H, m), 7.40 (1H, t, J=9.2Hz), 7.48 (2H, t, J=7.1Hz), 7.58 (1H, t, J=7.1Hz), 8.14 (2H, d, J=8.3Hz), 8.40 (1H, d, J=3.9Hz). FAB-MS; m/z: 1025 $(M+H)^+$.

Step 6: (1S,2S,3R,4S,5R,7R,8R,9S,10R,13S)-4-(Acetoxy)-9,10-[(1S)-2-(azetidino)ethylidenedioxy]-2-(benzoyloxy)-5,20-epoxy-7-fluoro-1-hydroxytax-11-en-13-yl

(2R,3S)-3-(tert-butoxycarbonylamino)-3-(3-fluoro-2-pyridyl)-2-[(triisopropylsilyl)oxy]propionate

The compound obtained in Step 5 mentioned above was used as a starting material to perform the same procedure as that of Example 1 and thereby to obtain the title compound as white amorphous solid.
$^1$H-NMR (400MHz, $CDCl_3$) δ : 0.82-0.93 (21H, m), 1.27 (3H, s), 1.37 (9H, s), 1.54 (3H, s), 1.62 (3H, s), 1.64-2.48 (7H, m), 1.78 (3H, s), 2.50 (3H, s), 2.81-2.91 (2H, m), 3.35 (4H, t, J=7.0Hz), 3.49 (1H, d, J=5.6Hz), 4.29-4.36 (2H, m), 4.39 (1H, d, J=9.0Hz), 4.70-4.88 (1H, m), 4.73 (1H, t, J=5.1Hz), 4.94 (1H, d, J=2.4Hz), 4.98 (1H, brs), 5.31 (1H, d, J=8.8Hz), 5.61 (1H, d, J=9.8Hz), 5.90 (1H, d, J=5.6Hz), 6.06 (1H, d, J=10.5Hz), 6.12 (1H, t, J=9.0Hz), 7.24-7.29 (1H, m), 7.40 (1H, t, J=9.1Hz), 7.48 (2H, t, J=7.6Hz), 7.59 (1H, t, J=7.6Hz), 8.14 (2H, d, J=8.5Hz), 8.40 (1H, d, J=4.4Hz).
FAB-MS; m/z: 1068 $(M+H)^+$.

Step 7: (1S,2S,3R,4S,5R,7R,8R,9S,10R,13S)-4-(Acetoxy)-9,10-[(1S)-2-(azetidino)ethylidenedioxy]-2-(benzoyloxy)-5,20-epoxy-7-fluoro-1-hydroxytax-11-en-13-yl

(2R,3S)-3-(tert-butoxycarbonylamino)-3-(3-fluoro-2-pyridyl)-2-hydroxypropionate

The compound obtained in Step 6 mentioned above was used as a starting material to perform the same procedure as that of Example 31, Step 2 and thereby to obtain the title compound as white amorphous solid.
$^1$H-NMR (400MHz, $CDCl_3$) δ : 1.22 (3H, s), 1.39 (9H, s), 1.53-2.55 (8H, m), 1.57 (3H, s), 1.62 (3H, s), 1.77 (3H, s), 2.36 (3H, s), 2.83-2.93 (2H, m), 3.35 (4H, t, J=7.1Hz), 3.48 (1H, d, J=5.1Hz), 4.24 (1H, d, J=9.0Hz), 4.28 (1H, d, J=8.8Hz), 4.38 (1H, d, J=8.5Hz), 4.65 (1H, d, J=2.7Hz), 4.68-4.84 (1H, m), 4.73 (1H, t, J=4.6Hz), 4.95 (1H, d, J=6.3Hz), 5.36 (1H, d, J=8.8Hz), 5.64 (1H, d, J=8.5Hz), 5.91 (1H, d, J=5.4Hz), 6.09 (1H, t, J=8.5Hz), 6.18 (1H, d, J=8.0Hz), 7.27-7.33 (1H, m), 7.42-7.52 (3H, m), 7.60 (1H, t, J=6.8Hz), 8.11 (2H, d, J=9.0Hz), 8.40 (1H, d, J=4.6Hz).
FAB-MS; m/z: 912 $(M+H)^+$.

[Example 96]

(1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R)-4,4-difluoro-2-hydroxy-3-(isopropoxycarbonylamino)pentanoate

[Formula 168]

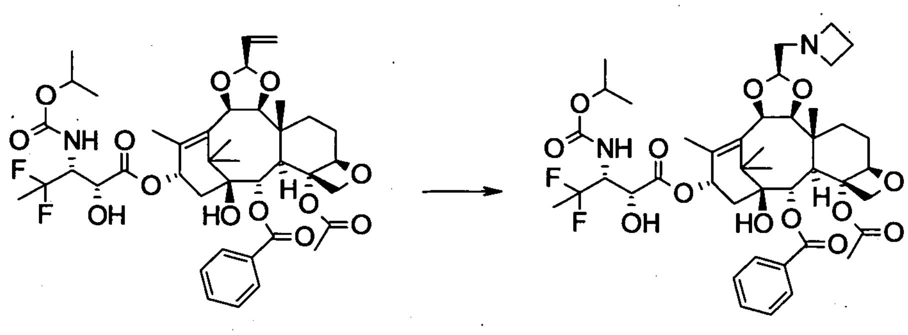

The compound obtained in Example 73, Step 2 was used as a starting material to perform the same procedure as that of Example 1 and thereby to obtain the title compound as white amorphous solid.
$^1$H-NMR (400MHz, $CDCl_3$) δ : 1.16-1.27 (6H, m), 1.17-2.37 (8H, m), 1.23 (3H, s), 1.48 (3H, s), 1.61 (3H, s), 1.72 (3H, t, J=19.1Hz), 1.73 (3H, s), 2.34 (3H, s), 2.75 (1H, dd, J=12.5, 5.9Hz), 2.83 (1H, dd, J=12.5, 4.2Hz), 2.92 (1H, d, J=4.9Hz), 3.36 (4H, dd, J=11.0, 6.9Hz), 4.10 (1H, d, J=6.9Hz), 4.25 (1H, d, J=8.6Hz), 4.34 (1H, d, J=8.6Hz), 4.57-4.68 (1H, m), 4.77 (1H, s), 4.88 (1H, t, J=5.1Hz), 4.90-4.97 (3H, m), 4.93 (1H, s), 5.21 (1H, d, J=6.9Hz), 5.44 (1H, d, J=10.3Hz), 6.01 (1H, d, J=4.9Hz), 6.15 (1H, t, J=7.8Hz), 7.48 (2H, t, J=7.7Hz), 7.58-7.64 (1H, m), 8.11-8.15 (2H, m).
FAB-MS; m/z: 849 $(M+H)^+$.

[Example 97]

[Formula 169]

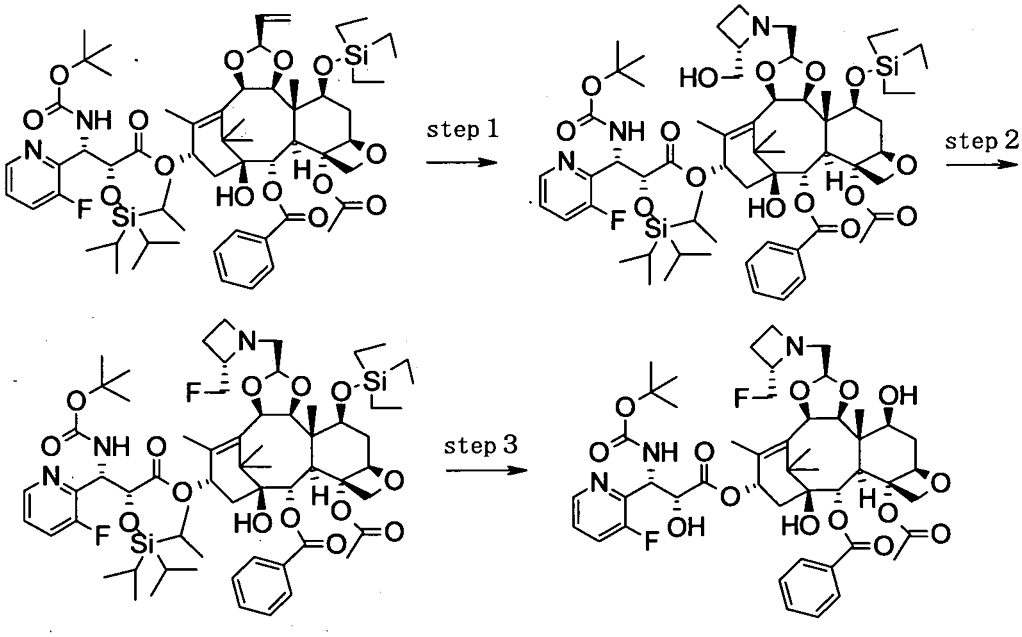


Step 1: (1S,2S,3R,4S,5R,7S,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}-7-[(triethylsilyl)oxyhax-11-en-13-yl

(2R,3S)-3-(tert-butoxycarbonylamino)-3-(3-fluoro-2-pyridyl)-2-[(triisopropylsilyl)oxy]propionate

The compounds obtained in Example 43, Step 1 and Reference Example 25 were used as starting materials to perform the same procedure as that of Example 1 and thereby to obtain the title compound as white amorphous solid. The resultant was used for the subsequent reaction as it was as a crude product.

Step 2: (1S,2S,3R,4S,5R,7S,8R,9S,10R,13S)-4-Acetoxy-2-(benzoyloxy)-5,20-epoxy-9,10-{(1S)-2-[(2S)-fluoromethylazetidino]ethylidenedioxy}-1-hydroxy-7-[(triethylsilyl)oxy]tax-11-en-13-yl

(2R,3S)-3-(tert-butoxycarbonylamino)-3-(3-fluoro-2-pyridyl)-2-[(triisopropylsilyl)oxy]propionate

The compound obtained in Step 1 mentioned above (396 mg) was dissolved in dichloromethane (10 ml), and the solution was added with diethylaminosulfur trifluoride (56 μl) at -78°C under an argon atmosphere, stirred at -78°C for 1 hour and at 0°C for 1 hour, further added with diethylaminosulfur trifluoride (56 μl), further stirred at room temperature for 30 minutes, and then added with water under ice cooling, and the layers were separated. The organic layer was washed with saturated brine, and dried over anhydrous sodium sulfate, and the solvent was evaporated under reduced pressure. The resulting residue was purified by silica gel chromatography [ethyl acetate:n-hexane=1:2 (v/v)] to obtain the title compound as white solid (181 mg).

$^1$H-NMR (400MHz, $CDCl_3$) δ : 0.60-0.68 (6H, m), 0.85-2.63 (14H, m), 0.86-0.94 (21H, m), 0.99 (9H, t, J=7.8Hz), 1.26 (3H, s), 1.37 (9H, s), 1.52 (3H, s), 1.77 (3H, s), 2.48 (3H, s), 2.82-3.06 (3H, m), 3.24 (1H, d, J=5.6Hz), 3.93 (1H, dd, J=10.8, 4.7Hz), 4.34 (1H, d, J=8.1Hz), 4.43-4.51 (2H, m), 4.81-4.88 (2H, m), 4.93 (1H, s), 5.20-5.27 (1H, m), 5.62 (1H, d, J=9.8Hz), 5.89 (1H, d, J=5.4Hz), 6.04 (1H, d, J=10.1Hz), 6.12 (1H, t, J=9.3Hz), 7.24-7.29 (1H, m), 7.40 (1H, t, J=9.3Hz), 7.47 (2H, t, J=7.4Hz), 7.54-7.59 (1H, m), 8.12 (2H, d, J=7.1Hz), 8.40 (1H, d, J=4.2Hz).

Step 3: (1S,2S,3R,4S,5R,7S,8S,9S,10R,13S)-4-Acetoxy-2-(benzoyloxy)-1,7-dihydroxy-5,20-epoxy-9,10-{(1S)-2-[(2S)-fluoromethylazetidino]ethylidenedioxy}tax-11-en-13-yl

(2R,3S)-3-(tert-butoxycarbonylamino)-3-(3-fluoro-2-pyridyl)-2-hydroxypropionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.32 (3H, s), 1.40 (9H, s), 1.62 (3H, s), 1.68 (3H, s), 1.76 (3H, s), 1.88-2.27 (6H, m), 2.39 (3H, s), 2.42-2.50 (1H, m), 2.60 (1H, q, J=7.5Hz), 2.75-2.88 (2H, m), 2.94-3.15 (4H, m), 3.85 (1H, d, J=7.4Hz), 3.91 (1H, brs), 4.05-4.11 (1H, m), 4.32 (1H, d, J=8.6Hz), 4.40 (1H, d, J=8.6Hz), 4.64-4.70 (2H, m), 5.03 (1H, dd, J=5.4, 3.9Hz), 5.10-5.28 (3H, m), 5.63 (1H, d, J=8.6Hz), 6.05 (1H, d, J=4.9Hz), 6.11 (1H, t, J=8.6Hz), 6.21 (1H, d, J=8.6Hz), 7.29-7.34 (1H, m), 7.44-7.52 (3H, m), 7.60 (1H, t, J=7.4Hz), 8.13 (2H, d, J=7.6Hz), 8.40 (1H, d, J=4.4Hz).
FAB-MS; m/z: 942 $(M+H)^+$

[Example 98]

[Formula 170]

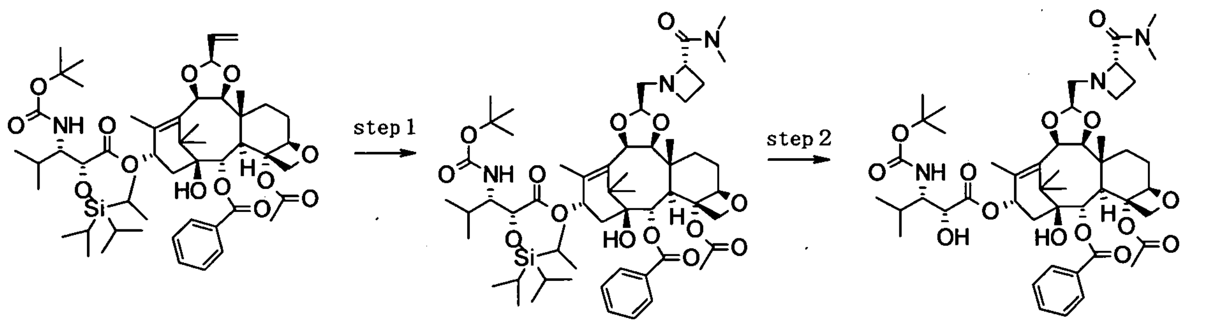


Step 1: (1S,2S,3R,4S,5R,8R,9S,10R,13S)-4-(Acetoxy)-2-(benzoyloxy)-9,10-{(1S)-2-[(2S)-2-(dimethylcarbamoyl)azetidino] ethylidenedioxy}-5, 20-epoxy-1-hydroxytax-11-en-13-yl

(2R,3S)-3-(tert-butoxycarbonylamino)-4-methyl-2-[(triisopropylsilyl)oxy]pentanoate

The compound obtained in Example 49, Step 1 and the compound obtained in Reference Example 12, Step 2 were used as starting materials to perform the same procedure as that of Example 1 and thereby to obtain the title compound as white amorphous solid.
$^1$H-NMR (400MHz, $CDCl_3$) δ : 1.01 (3H, d, J=6.7Hz), 1.03 (3H, d, J=6.7Hz), 1.07-2.33 (10H, m), 1.08-1.14 (21H, m), 1.25 (3H, s), 1.35 (9H, s), 1.49 (3H, s), 1.58 (3H, s), 1.74 (3H, s), 2.35 (3H, s), 2.83-2.92 (3H, m), 2.91 (3H, s), 2.93 (3H, s), 3.08 (1H, dd, J=15.4, 8.5Hz), 3.55 (1H, dd, J=10.7, 5.9Hz), 3.62 (1H, t, J=10.0Hz), 3.95 (1H, t, J=8.5Hz), 4.11 (1H, d, J=7.3Hz), 4.20 (1H, d, J=8.0Hz), 4.33 (1H, d, J=8.0Hz), 4.70 (1H, s), 4.78 (1H, d, J=10.5Hz), 4.92 (1H, brs), 5.01 (1H, t, J=4.6Hz), 5.13 (1H, d, J=6.8Hz), 5.92-6.00 (2H, m), 7.49 (2H, t, J=7.8Hz), 7.61 (1H, t, J=7.3Hz), 8.11-8.17 (2H, m).

Step 2: (1S,2S,3R,4S,5R,8R,9S, 10R,13S)-4-(Acetoxy)-2-(benzoyloxy)-9,10-{(1S)-2-[(2S)-2-(dimethylcarbamoyl)azetidino]ethylidenedioxy}-5,20-epoxy-1-hydroxytax-11-en-13-yl

(2R,3S)-3-(tert-butoxycarbonylamino)-2-hydroxy-4-methylpentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.02 (3H, d, J=6.7Hz), 1.05 (3H, d, J=6.7Hz), 1.19-2.40 (13H, m), 1.20 (3H, s), 1.40 (9H, s), 1.49 (3H, s), 1.77 (3H, s), 2.33 (3H, s), 2.84 (1H, dd, J=12.5, 5.9Hz), 2.90-2.97 (2H, m), 2.92 (3H, s), 2.94 (3H, s), 3.09 (1H, dd, J=15.4, 8.1Hz), 3.53-3.60 (1H, m), 3.81 (1H, t, J=9.5Hz), 3.95 (1H, t, J=8.7Hz), 4.05 (1H, s), 4.08-4.12 (1H, m), 4.24 (1H, d, J=8.5Hz), 4.32 (1H, d, J=8.5Hz), 4.49 (1H, s), 4.87 (1H, d, J=10.5Hz), 4.93 (1H, s), 5.06 (1H, t, J=4.8Hz), 5.19 (1H, d, J=6.3Hz), 5.97-6.06 (2H, m), 7.48 (2H, t, J=7.7Hz), 7.61 (1H, t, J=7.4Hz), 8.10-8.15 (2H, m).
FAB-MS; m/z: 912 $(M+H)^+$

[Example 99]

[Formula 171]

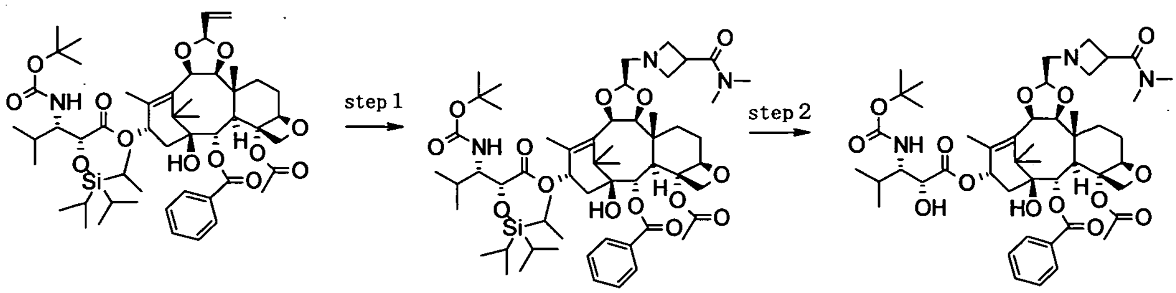


Step 1: (1S,2S,3R,4S,5R,8R,9S, 10R,13S)-4-(Acetoxy)-2-(benzoyloxy)-9,10-{(1S)-2-[3-(dimethylearbamoyl)azetidino]ethylidenedioxy}-5,20-epoxy-1-hydroxytax-11-en-13-yl

(2R,3S)-3-(tert-butoxycarbonylamino)-4-methyl-2-[(triisopropylsilyl)oxy]pentanoate

The compound obtained in Example 49, Step 1 and the compound obtained in Reference Example 2, Step 2 were used as starting materials to perform the same procedure as that of Example 1 and thereby to obtain the title compound as white amorphous solid.
$^1$H-NMR (400MHz, $CDCl_3$) δ : 1.01 (3H, d, J=6.7Hz), 1.03 (3H, d, J=6.7Hz), 1.07-2.34 (11H, m), 1.08-1.14 (21H, m), 1.28 (3H, s), 1.35 (9H, s), 1.49 (3H, s), 1.76 (3H, s), 2.35 (3H, s), 2.75-2.91 (3H, m), 2.90 (3H, s), 2.95 (3H, s), 3.38-3.48 (2H, m), 3.54 (1H, t, J=7.1Hz), 3.63 (1H, t, J=10.1Hz), 3.68-3.76 (2H, m), 4.13 (1H, d, J=7.3Hz), 4.20 (1H, d, J=8.5Hz), 4.34 (1H, d, J=8.0Hz), 4.71 (1H, s), 4.77 (1H, d, J=10.7Hz), 4.84 (1H, t, J=3.9Hz), 4.92 (1H, s), 5.16 (1H, d, J=7.3Hz), 5.92-6.00 (2H, m), 7.49 (2H, t, J=7.8Hz), 7.61 (1H, t, J=7.1Hz), 8.15 (2H, d, J=7.6Hz).

Step 2: (1S,2S,3R,4S,5R,8R,9S,10R,13S)-4-(Acetoxy)-2-(benzoyloxy)-9,10-{(1S)-2-[3-(dimethylcarbamoyl)azetidinolethylidenedioxy}-5,20-epoxy-1-hydroxytax-11-en-13-yl

(2R,3S)-3-(tert-butoxycarbonylamino)-2-hydroxy-4-methylpentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.02 (3H, d, J=6.7Hz), 1.05 (3H, d, J=6.7Hz), 1.18-2.42 (12H, m), 1.24 (3H, s), 1.38 (9H, s), 1.48 (3H, s), 1.78 (3H, s), 2.34 (3H, s), 2.76-2.90 (3H, m), 2.91 (3H, s), 2.96 (3H, s), 3.39-3.49 (2H, m), 3.50-3.59 (1H, m), 3.68-3.83 (3H, m), 4.13 (1H, d, J=7.6Hz), 4.23 (1H, d, J=8.3Hz), 4.32 (1H, d, J=8.5Hz), 4.49 (1H, s), 4.84 (1H, d, J=10.0Hz), 4.88 (1H, t, J=5.1Hz), 4.92 (1H, s), 5.22 (1H, d, J=7.3Hz), 5.98 (1H, d, J=4.9Hz), 6.04 (1H, t, J=7.8Hz), 7.48 (2H, t, J=7.8Hz), 7.60 (1H, t, J=7.4Hz), 8.16-8.11 (2H, m).

FAB-MS; m/z: 912 $(M+H)^+$

[Example 100]

(1S,2S,3R,4S,5R,8R,9S,10R,13S)-4-(Acetoxy)-2-(benzoyloxy)-5,20-epoxy-9,10-[(1S)-2-(3-fluoroazetidino)ethylidenedioxy]-1-hydroxytax-11-en-13-yl

(2R,3R)-3-(tert-butoxycarbonylamino)-4-fluoro-2-hydroxybutyrate

[Formula 172]

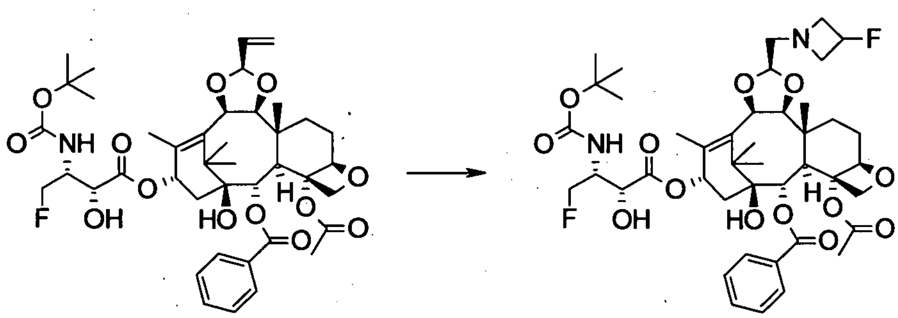

The compound obtained in Example 34, Step 2 and the compound obtained in Reference Example 24, Step 2 were used as starting materials to perform the same procedure as that of Example 1 and thereby to obtain the title compound as white amorphous solid.
$^1$H-NMR (400MHz, $CDCl_3$) δ : 1.21-2.41 (7H, m), 1.24 (3H, s), 1.41 (9H, s), 1.49 (3H, s), 1.60 (3H, s), 1.78 (3H, s), 2.32 (3H, s), 2.82 (1H, dd, J=12.3, 5.6Hz), 2.88-2.95 (2H, m), 3.26-3.40 (2H, m), 3.75-3.84 (1H, m), 3.79 (2H, d, J=7.6Hz), 4.13 (1H, d, J=7.1Hz), 4.24 (1H, d, J=8.6Hz), 4.32 (1H, d, J=8.6Hz), 4.37-4.55 (3H, m), 4.52 (1H, s), 4.58-4.65 (1H, m), 4.88 (1H, t, J=4.9Hz), 4.93 (1H, s), 5.04-5.15 (1H, m), 5.19-5.25 (1H, m), 5.99 (1H, d, J=4.9Hz), 6.06 (1H, t, J=7.6Hz), 7.48 (2H, t, J=7.7Hz), 7.58-7.63 (1H, m), 8.10-8.14 (2H, m).
FAB-MS; m/z: 849 $(M+H)^+$.

[Example 101]

(1S,2S,3R,4S,5R,8R,9S, 10R,13S)-4-(Acetoxy)-2-(benzoyloxy)-5,20-epoxy-1-hydroxy-9,10-[(1S)-2-(3-methoxyazetidino)ethylidenedioxy]tax-11-en-13-yl

(2R,3R)-3-(tert-butoxycarbonylamino)-4-fluoro-2-hydroxybutyrate

[Formula 173]

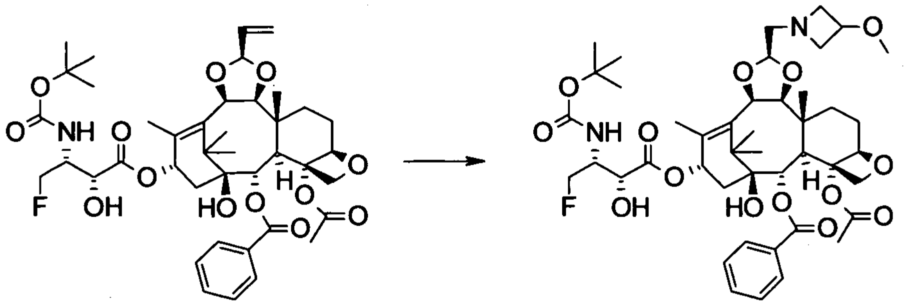

The compound obtained in Example 34, Step 2 and the compound obtained in Reference Example 1 were used as starting materials to perform the same procedure as that of Example 1 and thereby to obtain the title compound as white amorphous solid. $^{1}$H-NMR (400MHz, $CDCl_3$) δ : 1.20-2.41 (6H, m), 1.24 (3H, s), 1.41 (9H, s), 1.48 (3H, s), 1.60 (3H, s), 1.78 (3H, s), 2.33 (3H, s), 2.80 (1H, dd, J=12.5, 5.6Hz), 2.86-2.94 (2H, m), 3.06-3.12 (2H, m), 3.27 (3H, s), 3.71-3.78 (2H, m), 4.04-4.14 (2H, m), 4.24 (1H, d, J=8.3Hz), 4.33 (1H, d, J=8.3Hz), 4.39-4.65 (4H, m), 4.89 (1H, t, J=4.9Hz), 4.93 (1H, s), 5.14 (1H, d, J=8.6Hz), 5.23 (1H, d, J=6.9Hz), 6.00 (2H, d, J=4.9Hz), 6.07 (2H, t, J=7.8Hz), 7.48 (2H, t, J=7.6Hz), 7.58-7.64 (1H, m), 8.11-8.15 (2H, m).
FAB-MS; m/z: 861 $(M+H)^+$.

[Example 102]

[Formula 174]

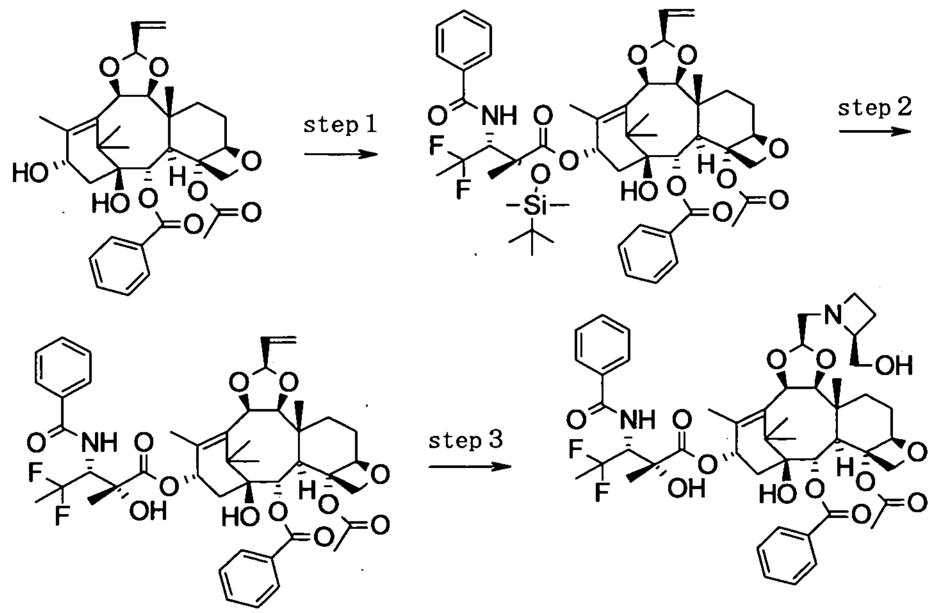


Step 1: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3R)-3-(benzoylamino)-2-[(tert-butyldimethylsilyl)oxy]-4,4-difluoro-2-methylpentanoate

(1S,2S,3R,4S,5R,8R,9S,10R,13S)-4-(Acetoxy)-2-(benzoyloxy)-1,13-dihydroxy-5, 20-epoxy-9,10-[(1S)-2-propenylidenedioxy]tax-11-ene and the compound obtained in Reference Example 43, Step 4 were used as starting materials to perform the same procedure as that of Example 31, Step 1 and thereby to obtain the title compound as white amorphous solid. The resultant was used for the subsequent reaction as it was as a crude product.

FAB-MS; m/z: 911 $(M+H)^+$

Step 2: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3R)-3-(benzoylamino)-4,4-difluoro-2-hydroxy-2-methylpentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.27 (3H, s), 1.49 (3H, s), 1.61-2.24 (10H, m), 1.64 (3H, s), 1.716 (3H, s), 1.719 (3H, s), 2.48 (3H, s), 2.95 (1H, d, J=5.2Hz), 4.14-4.17 (1H, m), 4.26 (1H, d, J=8.3Hz), 4.33 (1H, d, J=8.3Hz), 4.37 (1H, s), 4.94 (1H, s), 4.98-5.08 (1H, m), 5.21-5.25 (2H, m), 5.46 (1H, d, J=10.5Hz), 5.57 (1H, d, J=17.2Hz), 5.96-6.04 (2H, m), 6.19 (1H, t, J=8.8Hz), 7.16 (1H, d, J=10:1Hz), 7.44-7.63 (6H, m), 7.78-7.82 (2H, m), 8.14-8.18 (2H, m).

Step 3: (1S,2S,3R,4S,5R,8R,9S, 10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-3-(benzoylamino)-4,4-difluoro-2-hydroxy-2-methylpentanoate

The compound obtained in Step 2 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.25 (3H, s), 1.48 (3H, s), 1.54-2.12 (12H, m), 1.58 (3H, s), 1.70 (3H, s), 1.72 (3H, s), 2.17-2.28 (2H, m), 2.48 (3H, s), 2.80-2.94 (3H, m), 3.11 (1H, q, J=8.1Hz), 3.40-3.53 (3H, m), 3.65 (1H, dd, J=11.4, 2.8Hz), 4.11 (1H, d, J=7.4Hz), 4.25 (1H, d, J=8.3Hz), 4.30-4.35 (2H, m), 4.86 (1H, t, J=4.9Hz), 4.93 (1H, s), 4.98-5.07 (1H, m), 5.16 (1H, d, J=7.4Hz), 5.98 (1H, d, J=5.2Hz), 6.17 (1H, t, J=8.8Hz), 7.13 (1H, d, J=10.1Hz), 7.44-7.63 (6H, m), 7.79 (2H, d, J=7.6Hz), 8.16 (2H, d, J=7.6Hz).
FAB-MS; m/z: 911 $(M+H)^+$

[Example 103]

[Formula 175]

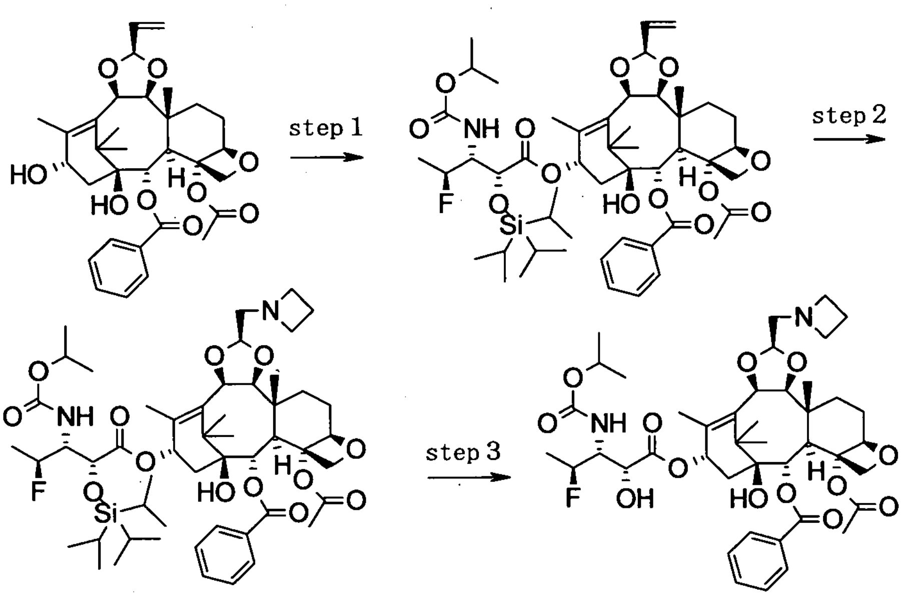

Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R,4S)-4-fluoro-3-(isopropoxycarbonylamino)-2-[(triisopropylsilyl)oxy]pentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 44 were used as starting materials to perform the same procedure as that of Example 31, Step 1 and thereby to obtain the title compound as white amorphous solid.
$^1$H-NMR (400MHz, $CDCl_3$) δ : 1.09-1.22 (27H, m), 1.30 (3H, s), 1.44 (3H, dd, J=24.2, 6.3Hz), 1.51 (3H, s), 1.56-2.28 (7H, m), 1.64 (3H, s), 1.79 (3H, brs), 2.34 (3H, s), 2.93 (1H, d, J=4.9Hz), 4.05-4.16 (1H, m), 4.20 (2H, d, J=7.3Hz), 4.35 (1H, d, J=8.3Hz), 4.54-4.84 (2H, m), 4.88-4.96 (3H, m), 5.20 (1H, d, J=6.1Hz), 5.26 (1H, d, J=7.3Hz), 5.45 (1H, d, J=10.3Hz), 5.57 (1H, d, J=17.3Hz), 5.95-6.07 (3H, m), 7.49 (2H, t, J=7.7Hz), 7.61 (1H, t, J=7.3Hz), 8.15 (2H, d, J=8.8Hz).
FAB-MS; m/z: 966 $(M+Na)^+$.

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R,4S)-4-fluoro-3-(isopropoxycarbonylamino)-2-[(triisopropylsilyl)oxy]pentanoate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 1.08-1.20 (27H, m), 1.28 (3H, s), 1.43 (3H, dd, J=24.6, 6.6Hz), 1.50 (3H, s), 1.58-2.26 (9H, m), 1.61 (3H, s), 1.76 (3H, s), 2.34 (3H, s), 2.73 (1H, dd, J=12.4, 5.9Hz), 2.80 (1H, dd, J=12.7, 4.9Hz), 2.91 (1H, d, J=4.9Hz), 3.31-3.38 (4H, m), 4.04-4.11 (1H, m), 4.13 (1H, d, J=7.3Hz), 4.19 (1H, d, J=8.3Hz), 4.35 (1H, d, J=8.3Hz), 4.53-4.73 (1H, m), 4.74-4.80 (1H, m), 4.83 (1H, t, J=5.4Hz), 4.88-4.94 (3H, m), 5.17 (1H, d, J=7.6Hz), 5.94-6.05 (2H, m), 7.49 (2H, t, J=7.7Hz), 7.61 (1H, t, J=7.4Hz), 8.15 (2H, d, J=8.0Hz).
FAB-MS; m/z: 987 $(M+H)^+$.

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3R,4S)-4-fluoro-2-hydroxy-3-(isopropoxycarbonylamino)pentanoate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.16 (3H, d, J=6.6Hz), 1.21 (3H, d, J=7.1Hz), 1.24 (3H, s), 1.45 (3H, dd, J=24.9, 6.3Hz), 1.49 (3H, s), 1.59-2.18 (10H, m), 1.61 (3H, s), 1.75 (3H, s), 2.34 (3H, s), 2.74 (1H, dd, J=12.5, 5.9Hz), 2.82 (1H, dd, J=12.5, 4.2Hz), 2.92 (1H, d, J=5.1Hz), 3.30-3.40 (4H, m), 4.11 (1H, d, J=7.1Hz), 4.24-4.36 (1H, m), 4.24 (1H, d, J=8.3Hz), 4.33 (1H, d, J=8.5Hz), 4.61-4.80 (1H, m), 4.70 (1H, s), 4.82-4.97 (3H, m), 5.09 (1H, d, J=10.0Hz), 5.21 (1H, d, J=6.8Hz), 6.01 (1H, d, J=5.1Hz), 6.12 (1H, t, J=8.3Hz), 7.48 (2H, t, J=7.7Hz), 7.61 (1H, t, J=7.3Hz), 8.13 (2H, d, J=8.8Hz).
FAB-MS; m/z: 831 $(M+H)^+$.

[Example 104]

[Formula 176]

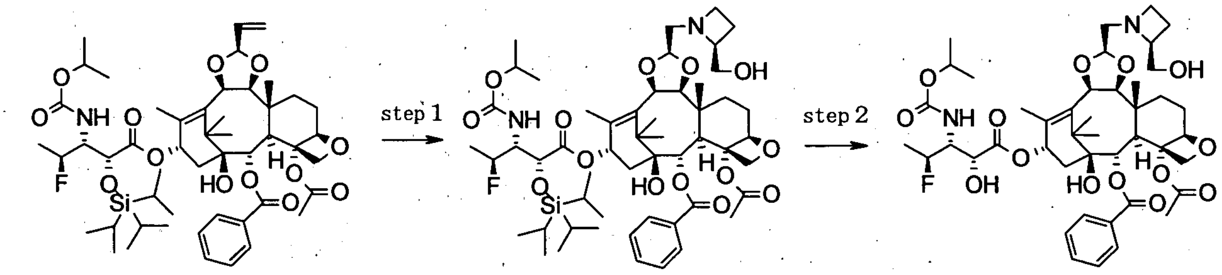


Step 1: (1S,2S,3R,4S,5R,8R,9S, 10R,13S)-4-(Acetoxy)-2-(benzoyloxy)-5,20-epoxy-1-hydroxy-9,10-{(1S)-2- [(2S)-2-hydroxymethylazetidino]ethylidenedioxy}tax-11-en-13-yl

(2R,3R,4S)-4-fluoro-3-(isopropoxycarbonylamino)-2-[(triisopropylsilyl)oxy]pentanoate

The compound obtained in Example 103, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.07-1.21 (27H, m), 1.28 (3H, s), 1.43 (3H, dd, J=24.6, 5.9Hz), 1.50 (3H, s), 1.55-2.28 (10H, m), 1.59 (3H, s), 1.76 (3H, s), 2.34 (3H, s), 2.80 (1H, dd, J=12.7, 5.4Hz), 2.89 (1H, dd, J=12.9, 5.1Hz), 2.91 (1H, d, J=5.9Hz), 3.09 (1H, dd, J=16.1, 9.0Hz), 3.39-3.52 (3H, m), 3.63 (1H, dd, J=11.2, 2.9Hz), 4.04-4.12 (1H, m), 4.14 (1H, d, J=7.3Hz), 4.19 (1H, d, J=8.8Hz), 4.35 (1H, d, J=7.8Hz), 4.54-4.73 (1H, m), 4.73-4.81 (1H, m), 4.84 (1H, t, J=5.1Hz), 4.88-4.95 (3H, m), 5.19 (1H, d, J=7.3Hz), 5.93-6.05 (2H, m), 7.50 (2H, t, J=7.6Hz), 7.61 (1H, t, J=7.4Hz), 8.15 (2H, d, J=7.3Hz).
FAB-MS; m/z: 1017 $(M+H)^+$.

Step 2: (1S,2S,3R,4S,5R,8R,9S, 10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R,4S)-4-fluoro-2-hydroxy-3-(isopropoxycarbonylamino)pentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.17 (3H, d, J=7.1Hz), 1.21 (3H, d, J=7.3Hz), 1.23 (3H, s), 1.45 (3H, dd, J=23.9, 5.6Hz), 1.49 (3H, s), 1.54-2.32 (11H, m), 1.58 (3H, s), 1.75 (3H, s), 2.34 (3H, s), 2.83 (1H, dd, J=12.7, 4.6Hz), 2.86-2.94 (2H, m), 3.10 (1H, dd, J=16.1, 8.5Hz), 3.39-3.52 (3H, m), 3.65 (1H, dd, J=11.4, 2.8Hz), 4.12 (1H, d, J=7.1Hz), 4.24-4.34 (1H, m), 4.24 (1H, d, J=8.5Hz), 4.33 (1H, d, J=8.1Hz), 4.61-4.80 (1H, m), 4.70 (1H, brs), 4.83-4.98 (3H, m), 5.11 (1H, d, J=10.3Hz), 5.23 (1H, d, J=7.1Hz), 5.99 (1H, d, J=5.1Hz), 6.11 (1H, t, J=8.2Hz), 7.48 (2H, t, J=8.3Hz), 7.61 (1H, t, J=7.3Hz), 8.13 (2H, d, J=8.1Hz).
FAB-MS; m/z: 861 $(M+H)^+$.

[Example 105]

[Formula 177]

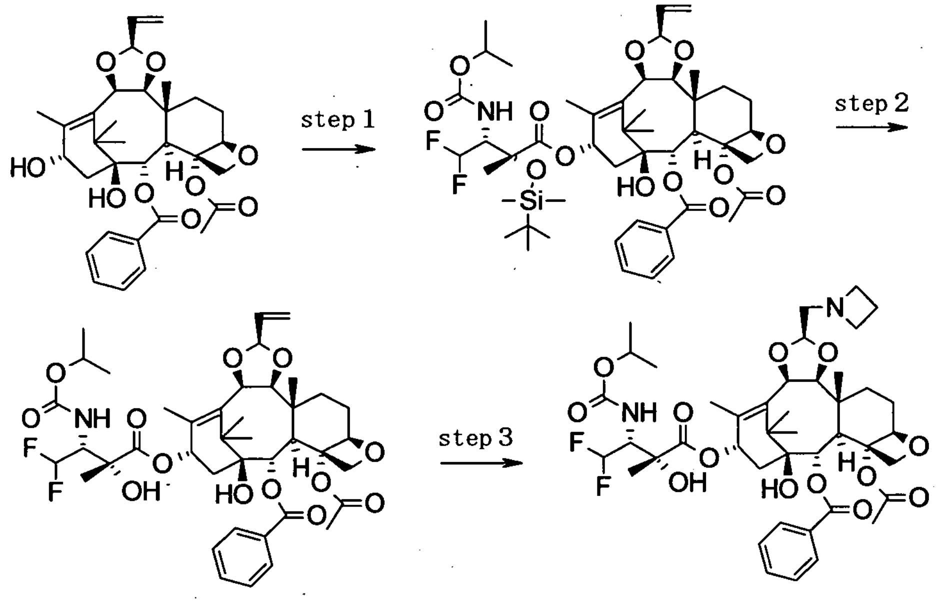


Step 1: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3R)-2-[(tert-butyldimethylsilyl)oxy]-4,4-difluoro-3-(isopropoxycarbonylamino)-2-methylbutyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 45, Step 3 were used as starting materials to perform the same procedure as that of Example 31, Step 1 and thereby to obtain the title compound as white amorphous solid. The resultant was used for the subsequent reaction as it was as a crude product.

Step 2: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3R)-4,4-difluoro-2-hydroxy-3-(isopropoxycarbonylamino)-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.18-1.30 (9H, m), 1.49 (3H, s), 1.60-2.31 (7H, m), 1.63 (3H, s), 1.65 (3H, s), 1.77 (3H, s), 2.43 (3H, s), 2.96 (1H, d, J=5.2Hz), 4.18 (1H, d, J=7.1Hz), 4.27 (1H, d, J=8.3Hz), 4.33 (1H, d, J=8.6Hz), 4.38-4.49 (1H, m), 4.66 (1H, s), 4.82-4.99 (2H, m), 5.23-5.31 (2H, m), 5.48 (2H, t, J=11.0Hz), 5.58 (1H, d, J=17.7Hz), 5.95-6.25 (4H, m), 7.48 (2H, t, J=7.6Hz), 7.61 (1H, t, J=7.4Hz), 8.15 (2H, d, J=7.4Hz).

Step 3: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3R)-4,4-difluoro-2-hydroxy-3-(isopropoxycarbonylamino)-2-methylbutyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.20 (3H, d, J=6.4Hz), 1.23 (3H, d, J=6.4Hz), 1.27 (3H, s), 1.48 (3H, s), 1.60-2.24 (9H, m), 1.62 (3H, s), 1.63 (3H, s), 1.75 (3H, s), 2.42 (3H, s), 2.75 (1H, dd, J=12.5, 5.9Hz), 2.83 (1H, dd, J=12.5, 4.4Hz), 2.94 (1H, d, J=5.2Hz), 3.32-3.40 (4H, m), 4.11 (1H, d, J=6.9Hz), 4.27 (1H, d, J=8.3Hz), 4.33 (1H, d, J=8.3Hz), 4.37-4.48 (1H, m), 4.64 (1H, brs), 4.85-4.94 (3H, m), 5.20 (1H, d, J=6.9Hz), 5.47 (1H, d, J=10.5Hz), 5.95-6.24 (3H, m), 7.49 (2H, t, J=7.8Hz), 7.61 (1H, t, J=7.4Hz), 8.15 (2H, d, J=7.4Hz).

FAB-MS; m/z: 849 $(M+H)^+$.

[Example 106]

(1S,2S,3R,4S,5R,8R,9S,10R,13S)-4-(Acetoxy)-2-(benzoyloxy)-5,20-epoxy-9,10-[(1S)-2-(3-fluoroazetidino)ethylidenedioxyl-1-hydroxytax-11-en-13-yl

(2R,3S)-3-(tert-butoxycarbonylamino)-2-hydroxy-3-(2-pyrimidinyl)propionate

[Formula 178]

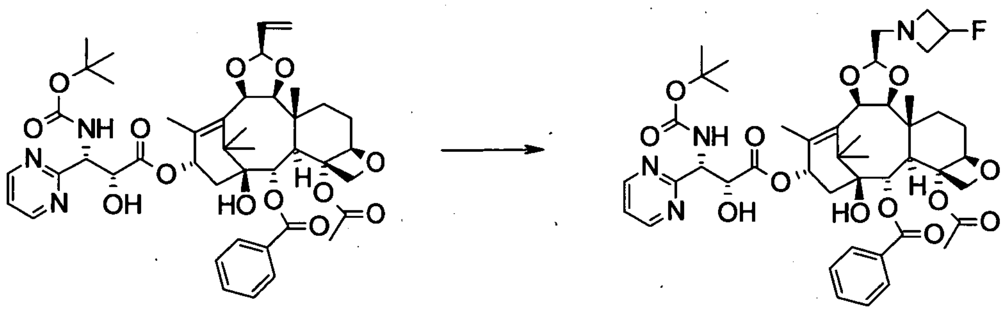

The compound obtained in Example 53, Step 2 and the compound obtained in Reference Example 24, Step 2 were used as starting materials to perform the same procedure as that of Example 1 and thereby to obtain the title compound as white amorphous solid.
$^1$H-NMR (400MHz, $CDCl_3$) δ : 1.21-2.49 (8H, m), 1.29 (3H, s), 1.42 (9H, s), 1.50 (3H, s), 1.62 (3H, s), 1.81 (3H, s), 2.46 (3H, s), 2.83 (1H, dd, J=12.5, 5.6Hz), 2.92 (1H, dd, J=12.4, 4.5Hz), 2.96 (1H, d, J=5.1Hz), 3.27-3.40 (2H, m), 3.75-3.85 (2H, m), 4.16 (1H, d, J=7.1Hz), 4.22 (1H, d, J=8.3Hz), 4.34 (1H, d, J=8.3Hz), 4.88 (1H, t, J=5.0Hz), 4.94 (1H, s), 4.99 (1H, s), 5.05-5.26 (2H, m), 5.44 (1H, dd, J=8.8, 1.5Hz), 5.99 (1H, d, J=5.1Hz), 6.05-6.14 (2H, m), 7.25 (2H, t, J=4.9Hz), 7.47 (2H, t, J=7.7Hz), 7.56-7.62 (2H, m), 8.75 (2H, d, J=4.9Hz).
FAB-MS; m/z: 895 $(M+H)^+$.

[Example 107]

[Formula 179]

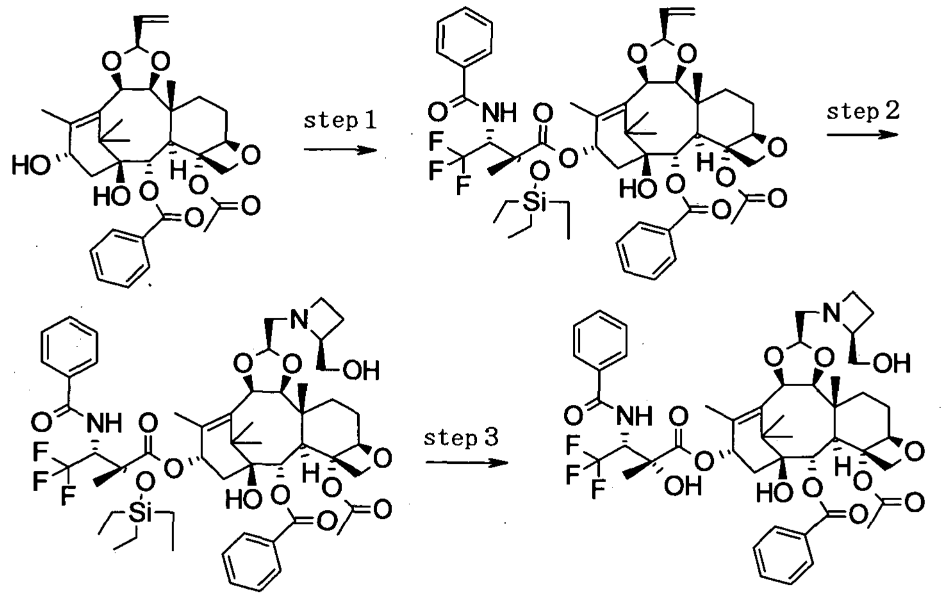


Step 1: (1S,2S,3R,4S,5R,8R,9S, 10R,13S)-4-Acetoxy-2-(benzoyloxy)-5,20-epoxy-1-hydroxy-9,10-[(1S)-2-propenylidenedioxy]tax-11-en-13-yl

(2R,3R)-3-(benzoylamino)-2-methyl-2- [(triethylsilyl)oxy]-4,4,4-trifluorobutyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 46, Step 3 were used as starting materials to perform the same procedure as that of Example 31, Step 1 and thereby to obtain the title compound as white amorphous solid.
$^1$H-NMR (400MHz, $CDCl_3$) δ : 0.68-0.83 (6H, m), 1.01 (9H, t, J=7.8Hz), 1.24 (3H, s), 1.51 (3H, s), 1.60-2.08 (9H, m), 1.63 (3H, s), 1.75 (3H, s), 2.15-2.22 (1H, m), 2.57 (3H, s), 2.87 (1H, d, J=4.9Hz), 4.17 (1H, d, J=7.1Hz), 4.26 (1H, d, J=8.3Hz), 4.33 (1H, d, J=8.6Hz), 4.94 (1H, s), 5.17-5.26 (3H, m), 5.45 (1H, d, J=10.3Hz), 5.57 (1H, d, J=17.4Hz), 5.95-6.10 (3H, m), 6.76 (1H, d, J=9.3Hz), 7.40-7.45 (2H, m), 7.49-7.54 (3H, m), 7.60-7.64 (1H, m), 7.68-7.70 (2H, m), 8.17-8.21 (2H, m).

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-3-(benzoylamino)-2-methyl-2-[(triethylsilyl)oxy]-4,4,4-trifluorobutyrate

The compound obtained in Step 1 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.66-0.82 (6H, m), 1.01 (9H, t, J=8.2Hz), 1.22 (3H, s), 1.50 (3H, s), 1.57 (3H, s), 1.60-2.03 (8H, m), 1.72 (3H, s), 1.92 (3H, s), 2.15-2.27 (2H, m), 2.56 (3H, s), 2.78-2.90 (3H, m), 3.09 (1H, q, J=8.3Hz), 3.39-3.50 (3H, m), 3.62 (1H, dd, J=11.5, 2.9Hz), 4.10-4.12 (1H, m), 4.25 (1H, d, J=8.3Hz), 4.33 (1H, d, J=8.3Hz), 4.84 (1H, t, J=4.8Hz), 4.93 (1H, s), 5.15-5.22 (2H, m), 5.95 (1H, d, J=4.9Hz), 6.05 (1H, t, J=9.0Hz), 6.76 (1H, d, J=9.8Hz), 7.42 (2H, t, J=7.8Hz), 7.51 (3H, t, J=7.6Hz), 7.62 (1H, t, J=7.4Hz), 7.68 (2H, d, J=7.6Hz), 8.18 (2H, d, J=7.6Hz).

Step 3: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-3-(benzoylamino)-2-hydroxy-2-methyl-4,4,4-trifluorobutyrate

The compound obtained in Step 2 mentioned above was used as a starting material to perform the same

procedure as that of Example 31, Step 2 and thereby to obtain the title compound as white amorphous solid.
$^1$H-NMR (400MHz, $CDCl_3$) δ : 1.22 (3H, s), 1.47 (3H, s), 1.57 (3H, s), 1.61 (3H, s), 1.62-2.28 (10H, m), 1.75 (3H, s), 2.48 (3H, s), 2.79-2.92 (3H, m), 3.09 (1H, q, J=8.1Hz), 3.39-3.51 (3H, m), 3.63 (1H, dd, J=11.4, 3.1Hz), 4.07 (1H, d, J=7.1Hz), 4.26 (1H, d, J=8.5Hz), 4.32 (1H, d, J=8.3Hz), 4.72 (1H, brs), 4.84 (1H, t, J=5.1Hz), 4.92 (1H, s), 5.11 (1H, d, J=7.1Hz), 5.32-5.40 (1H, m), 5.97 (1H, d, J=5.1Hz), 6.17 (1H, t,J=8.7Hz), 7.11 (1H, d, J=10.0Hz), 7.43-7.56 (5H, m), 7.61 (1H, t, J=7.3Hz), 7.78 (2H, d, J=7.6Hz), 8.15 (2H, d, J=7.1Hz).
FAB-MS; m/z: 915 $(M+H)^+$.

[Example 108]

[Formula 180]

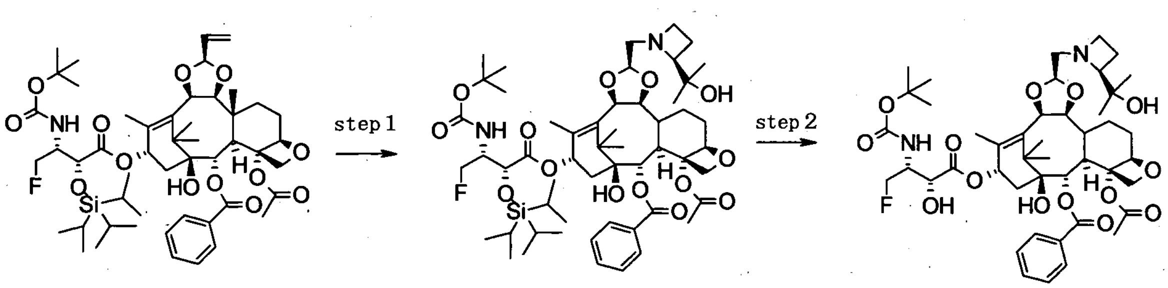


Step 1: (1S,2S,3R,4S,5R,8R,9S,10R,13S)-4-(Acetoxy)-2-(benzoyloxy)-5,20-epoxy-9,10-{(1S)-2-[(2S)-2-(1-hydroxy-1-methylethyl)azetidino]ethylidenedioxy}-1-hydroxytax-11-en-13-yl

(2R,3R)-3-(tert-butoxycarbonylamino)-4-fluoro-2-[(triisopropylsilyl)oxy]butyrate

The compound obtained in Example 34, Step 1 and the compound obtained in Reference Example 37 were used as starting materials to perform the same procedure as that of Example 1 and thereby to obtain the title compound as white amorphous solid.
$^1$H-NMR (400MHz, $CDCl_3$) δ : 1.02 (3H, s), 1.11-1.21 (21H, m), 1.27 (3H, s), 1.37 (9H, s), 1.50 (3H, s), 1.59-2.02 (13H, m), 1.77 (3H, s), 2.17-2.27 (3H, m), 2.33 (3H, s), 2.80 (1H, dd, J=12.8, 5.4Hz), 2.93 (1H, d, J=5.4Hz), 3.01-3.06 (2H, m), 3.21 (1H, t, J=8.0Hz), 3.48-3.53 (1H, m), 4.17 (2H, dd, J=12.3, 7.8Hz), 4.32-4.48 (4H, m), 4.78-4.80 (2H, m), 4.86 (1H, t, J=5.0Hz), 4.91 (1H, s), 5.19 (1H, d, J=7.4Hz), 5.95 (1H, d, J=5.2Hz), 6.02 (1H, t, J=9.0Hz), 7.49 (2H, t, J=7.6Hz), 7.61 (1H, t, J=7.4Hz), 8.15 (2H, d, J=7.1Hz).

Step 2: (1S,2S,3R,4S,5R,8R,9S,10R,13S)-4-(Acetoxy)-2-(benzoyloxy)-5,20-epoxy-9,10-{(1S)-2-[(25)-2-(1-hydroxy-1-methylethyl)azetidino]ethylidenedioxy}-1-hydroxytax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.03 (3H, s), 1.15 (3H, s), 1.24 (3H, s), 1.41 (9H, s), 1.49 (3H, s), 1.60-2.03 (11H, m), 1.79 (3H, m), 2.18-2.41 (2H, m), 2.33 (3H, s), 2.83 (1H, dd, J=12.8, 5.2Hz), 2.93 (1H, d, J=5.1Hz), 3.00-3.07 (2H, m), 3.22 (1H, t, J=7.9Hz), 3.49-3.53 (1H, m), 4.15 (1H, d, J=7.1Hz), 4.24 (1H, d, J=8.5Hz), 4.33 (1H, d, J=8.3Hz), 4.41-4.53 (5H, m), 4.90 (1H, t, J=4.8Hz), 4.94 (1H, s), 5.10 (1H, d, J=8.3Hz), 5.25 (1H, d, J=7.3Hz), 5.99 (1H, d, J=4.9Hz), 6.05-6.11 (1H, m), 7.48 (2H, t, J=7.7Hz), 7.61 (1H, t, J=7.4Hz), 8.13 (2H, d, J=7.3Hz).
FAB-MS; m/z: 889 $(M+H)^+$.

[Example 109]

[Formula 181]

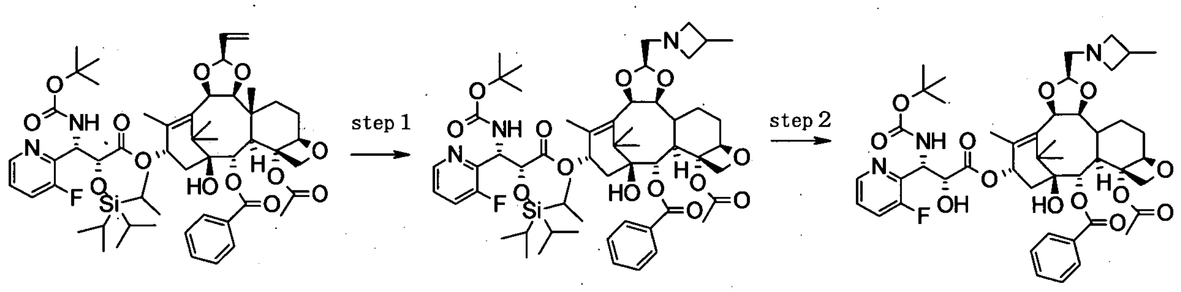


Step 1: (1S,2S,3R,4S,5R,8R,9S,10R,13S)-4-(Acetoxy)-2-(benzoyloxy)-5,20-epoxy-1-hydroxy-9,10-[(1S)-2-(3-methylazetidino)ethylidenedioxy]tax-11-en-13-yl

(2R,3S)-3-(tert-butoxycarbonylamino)-3-(3-fluoro-2-pyridyl)-2-[(triisopropylsilyl)oxy]propionate

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-[(triisopropylsilyl)oxy]propionate (Bioorg. Med. Chem. Lett., 2004, 14, 3209) and the compound obtained in Reference Example 47 were used as starting materials to perform the same procedure as that of Example 1 and thereby to obtain the title compound as white amorphous solid.
$^1$H-NMR (400MHz, $CDCl_3$) $\delta$ : 0.80-1.00 (21H, m), 1.16 (3H, d, J=6.6Hz), 1.21-2.11 (4H, m), 1.35 (3H, s), 1.38 (9H, s), 1.53 (3H, s), 1.63 (3H, s), 1.79 (3H, s), 2.27-2.42 (2H, m), 2.48 (3H, s), 2.58-2.88 (6H, m), 2.97 (1H, d, J=5.6Hz), 3.61 (2H, dd, J=12.8, 5.7Hz), 4.15 (1H, d, J=7.3Hz), 4.22 (1H, d, J=8.3Hz), 4.35 (1H, d, J=8.3Hz), 4.84 (1H, t, J=5.0Hz), 4.92-4.99 (2H, m), 5.18 (1H, d, J=7.1Hz), 5.60 (1H, d, J=9.5Hz), 5.97 (1H, d, J=5.1Hz), 6.03-6.15 (2H, m), 7.23-7.30 (1H, m), 7.37-7.42 (1H, m), 7.47 (2H, t, J=7.6Hz), 7.55-7.63 (1H, m), 8.13-8.20 (2H, m), 8.40 (1H, d, J=4.6Hz).
ESI-MS; m/z: 1065 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-(3-methylazetidino)ethylidenedioxy]tax-11-en-13-yl

(2R,3S)-3-(tert-butoxycarbonylamino)-3-(3-fluoro-2-pyridyl)-2-hydroxypropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) $\delta$ : 1.00-2.25 (7H, m), 1.18 (3H, d, J=6.8Hz), 1.28 (3H, s), 1.39 (9H; d, J=12.9Hz), 1.50 (3H, s), 1.62 (3H, s), 1.78 (3H, s), 2.34 (3H, s), 2.45 (1H, t, J=12.2Hz), 2.59-3.03 (6H, m), 3.58-3.75 (2H, m), 4.13 (1H, d, J=7.1Hz), 4.23 (1H, d, J=8.5Hz), 4.33 (1H, d, J=8.5Hz), 4.67 (1H, d, J=2.4Hz), 4.91 (2H, s), 5.22 (1H, d, J=7.3Hz), 5.65 (1H, d, J=8.8Hz), 5.99 (1H, d, J=5.1Hz), 6.08 (1H, t, J=8.4Hz), 6.20 (1H, d, J=8.8Hz), 7.25-7.35 (1H, m), 7.42-7.49 (3H, m), 7.60 (1H, t, J=7.4Hz), 8.09-8.18 (2H, m), 8.40 (1H, d, J=4.6Hz).
ESI-MS; m/z: 908 $(M+H)^+$.

[Example 110]

[Formula 182]

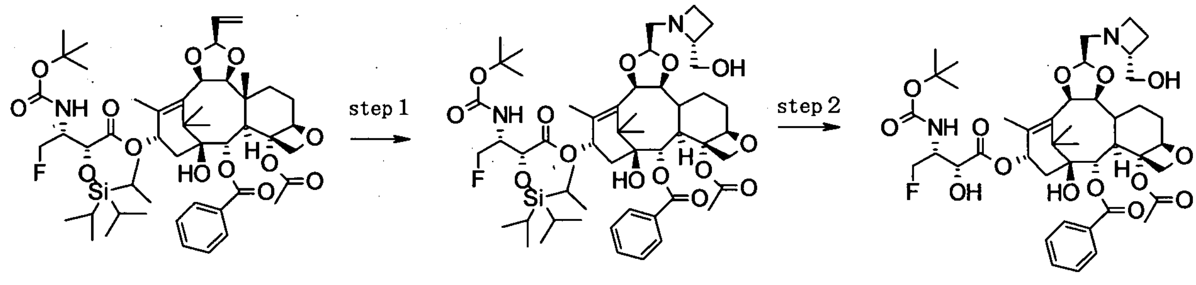


Step 1: (1S,2S,3R,4S,5R,8R,9S,10R,13S)-4-(Acetoxy)-2-(benzoyloxy)-5,20-epoxy-1-hydroxy-9,10-{(1S)-2-[(2R)-2-hydroxymethylazetidino]ethylidenedioxy}tax-11-en-13-yl

(2R,3R)-3-(tert-butoxycarbonylamino)-4-fluoro-2-[(triisopropylsilyl)oxy]butyrate

The compound obtained in Example 34, Step 1 and the compound obtained in Reference Example 26 were used as starting materials to perform the same procedure as that of Example 1 and thereby to obtain the title compound as white amorphous solid.
$^1$H-NMR (400MHz, $CDCl_3$) δ : 1.04-1.20 (21H, m), 1.27 (3H, s), 1.37 (9H, s), 1.50 (3H, s), 1.60 (3H, s), 1.77 (3H, s), 1.87-2.06 (7H, m), 2.20-2.27 (3H, m), 2.33 (3H, s), 2.77 (1H, dd, J=12.8, 5.5Hz), 2.91 (2H, dd, J=13.0, 4.4Hz), 3.07 (1H, q , J=8.3Hz), 3.41-3.51 (3H, m), 3.64 (1H, dd, J=11.3, 2.7Hz), 4.10-4.20 (2H, m), 4.32-4.48 (4H, m), 4.79-4.80 (2H, m), 4.84 (1H, t, J=5.0Hz), 4.91 (1H, s), 5.18 (1H, d, J=7.4Hz), 5.96 (1H, d, J=5.2Hz), 6.02 (1H, t, J=9.0Hz), 7.50 (2H, t, J=7.6Hz), 7.61 (1H, t, J=7.5Hz), 8.15 (2H, d, J=7.4Hz).

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2R)-2-hydroxymethylazetidino]ethylidenedioxy}tax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.18-2.07 (9H, m), 1.24 (3H, s), 1.42 (9H, s), 1.48 (3H, s), 1.61 (3H, s), 1.79 (3H, s), 2.20-2.41 (2H, m), 2.33 (3H, s), 2.79 (1H, dd, J=12.7, 5.6Hz), 2.88-2.95 (2H, m), 3.08 (1H, q, J=8.2Hz), 3.41-3.52 (3H, m), 3.65 (1H, dd, J=11.4, 2.8Hz), 4.13 (1H, d, J=6.9Hz), 4.24 (1H, d, J=8.3Hz), 4.33 (1H, d, J=8.5Hz), 4.38-4.63 (4H, m), 4.89 (1H, t, J=4.9Hz), 4.94 (1H, s), 5.11 (1H, d, J=8.8Hz), 5.23 (1H, d, J=6.9Hz), 6.01 (1H, d, J=5.1Hz), 6.07 (1H, t, J=8.2Hz), 7.49 (2H, t, J=7.6Hz), 7.60-7.64 (1H, m), 8.13 (2H, d, J=8.1Hz).
FAB-MS; m/z: 861 (M+H)$^+$.

[Example 111]

[Formula 183]

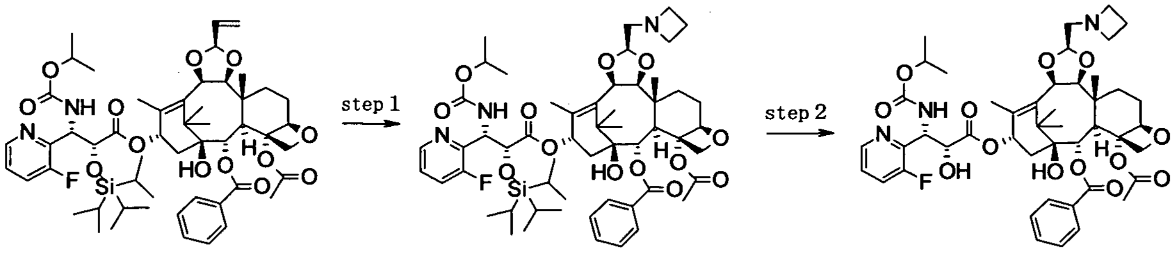

Step 1: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(3-fluoro-2-pyridyl)-3-(isopropoxycarbonylamino)-2-[(triisopropylsilyl)oxy]propionate

The compound obtained in Example 82, Step 1 was used as a starting material to perform the same procedure as that of Example 1 and thereby to obtain the title compound as white amorphous solid.
$^1$H-NMR (400MHz, $CDCl_3$) δ : 0.90-0.94 (21H, m), 1.17-1.21 (6H, m), 1.35 (3H, s), 1.52 (3H, s), 1.63 (3H, s), 1.78 (3H, s), 1.91-2.14 (7H, m), 2.33 (2H, d, J=9.3Hz), 2.47 (3H, s), 2.71-2.83 (2H, m), 2.96 (1H, d, J=5.1Hz), 3.33-3.37 (4H, m), 4.15 (1H, d, J=7.3Hz), 4.22 (1H, d, J=8.3Hz), 4.36 (1H, d, J=8.1Hz), 4.79-4.84 (2H, m), 4.95-4.98 (2H, m), 5.17 (1H, d, J=7.3Hz), 5.62 (1H, d, J=9.5Hz), 5.98 (1H, d, J=5.1Hz), 6.07-6.14 (2H, m), 7.24-7.28 (1H, m), 7.40 (1H, t, J=8.8Hz), 7.47 (2H, t, J=7.7Hz), 7.59 (1H, t, J=7.5Hz), 8.16 (2H, d, J=7.3Hz), 8.38 (1H, d, J=4.6Hz).

Step 2: (1S;2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(3-fluoro-2-pyridyl)-2-hydroxy-3-(isopropoxycarbonylamino)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.19-1.24 (6H, m), 1.28 (3H, s), 1.49 (3H, s), 1.62 (3H, s), 1.76 (3H, s), 1.81-1.93 (5H, m), 2.03-2.16 (4H, m), 2.33 (3H, s), 2.38-2.44 (1H, m), 2.72-2.85 (2H, m), 2.93 (1H, d, J=4.9Hz), 3.32-3.40 (4H, m), 4.12 (1H, d, J=7.1Hz), 4.23 (1H, d, J=8.3Hz), 4.32 (1H, d, J=7.8Hz), 4.70 (1H, d, J=2.9Hz), 4.85-4.91 (3H, m), 5.21 (1H, d, J=7.1Hz), 5.71 (1H, d, J=8.8Hz), 6.00 (1H, d, J=5.1Hz), 6.11 (1H, t, J=8.4Hz), 6.27 (1H, d, J=8.5Hz), 7.28-7.32 (1H, m), 7.42-7.49 (3H, m), 7.60 (1H, t, J=7.4Hz), 8.13 (2H, dd, J=8.3, 1.2Hz), 8.40 (1H, d, J=4.6Hz).
FAB-MS; m/z: 880 $(M+H)^+$.

[Example 112]

[Formula 184]

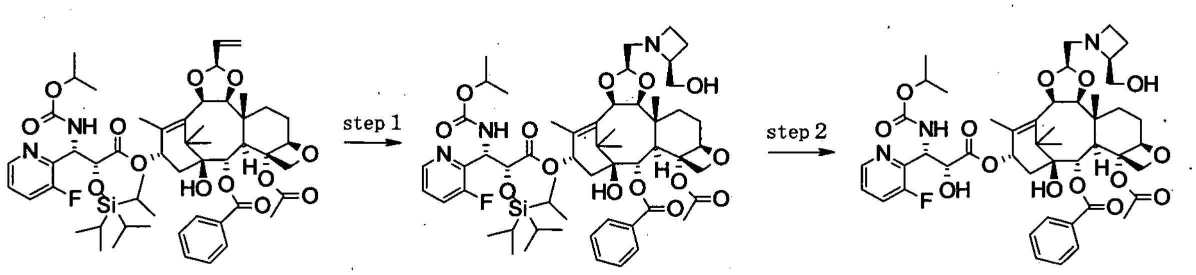


Step 1: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(3-fluoro-2-pyridyl)-3-(isopropoxycarbonylamino)-2-[(triisopropylsilyl)oxy]propionate

The compound obtained in Example 82, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.90-0.94 (21H, m), 1.18-1.21 (6H, m), 1.35 (3H, s), 1.52 (3H, s), 1.61 (3H, s), 1.79 (3H, s), 1.92-2.05 (7H, m), 2.21-2.34 (3H, m), 2.48 (3H, s), 2.79-2.97 (3H, m), 3.08-3.13 (1H, m), 3.41-3.51 (3H, m), 3.64 (1H, dd, J=11.6, 2.6Hz), 4.20 (2H, dd, J=18.1, 7.8Hz), 4.36 (1H, d, J=8.3Hz), 4.79-4.85 (2H, m), 4.95-4.97 (2H, m), 5.20 (1H, d, J=7.6Hz), 5.62 (1H, d, J=9.6Hz), 5.96 (1H, d, J=4.9Hz), 6.07-6.15 (2H, m), 7.25-7.29 (1H, m), 7.38-7.49 (3H,

m), 7.59 (1H, t, J=6.9Hz), 8.16 (2H, d, J=8.1Hz), 8.39 (1H, d, J=4.4Hz).

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(3-fluoro-2-pyridyl)-2-hydroxy-3-(isopropoxycarbonylamino)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.19-1.23 (6H, m), 1.28 (3H, s), 1.49 (3H, s), 1.59 (3H, s), 1.76 (3H, s), 1.81-2.09 (9H, m), 2.20-2.42 (2H, m)2.33 (3H, s), 2.80-2.93 (3H, m), 3.10 (1H, q , J=8.2Hz), 3.41-3.51 (3H, m), 3.66 (1H, dd, J=11.5, 3.2Hz), 4.13 (1H, d, J=7.3Hz), 4.23 (1H, d, J=8.3Hz), 4.32 (1H, d, J=7.6Hz), 4.70 (1H, s), 4.85-4.91 (3H, m), 5.23 (1H, d, J=7.1Hz), 5.70 (1H, d, J=8.5Hz), 5.98 (1H, d, J=5.1Hz), 6.11 (1H, t, J=8.5Hz), 6.27 (1H, d, J=8.1Hz), 7.28-7.33 (1H, m), 7.43-7.49 (3H, m), 7.60 (1H, t, J=7.3Hz), 8.13 (2H, d, J=7.1Hz), 8.40 (1H, d, J=4.6Hz).
FAB-MS; m/z: 910 $(M+H)^+$.

[Example 113]

[Formula 185]

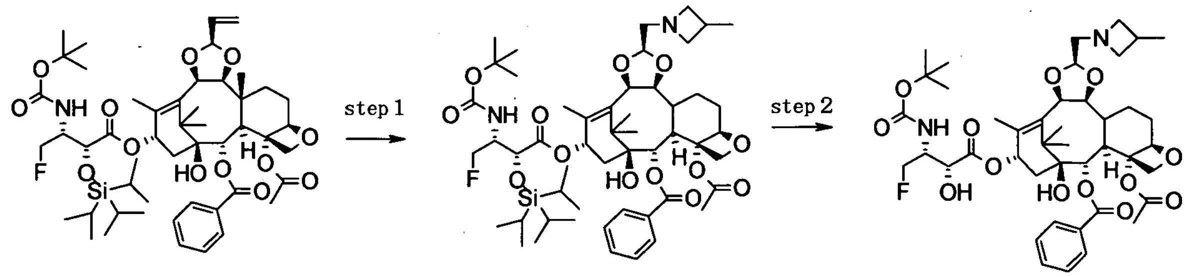


Step 1: (1S,2S,3R,4S,5R,8R,9S,10R,13S)-4-(Acetoxy)-2-(benzoyloxy)-5,20-epoxy-1-hydroxy-9,10-[(1S)-2-(3-methylazetidino)ethylidenedioxy]tax-11-en-13-yl

(2R,3R)-3-(tert-butoxycarbonylamino)-4-fluoro-2-[(triisopropylsilyl)oxy]butyrate

The compound obtained in Example 34, Step 1 and the compound obtained in Reference Example 47 were used as starting materials to perform the same procedure as that of Example 1 and thereby to obtain the title compound as white amorphous solid.
ESI-MS; m/z: 1001 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-(3-methylazetidino)ethylidenedioxy]tax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.17 (3H, d, J=6.8Hz), 1.24 (3H, s), 1.28-2.44 (7H, m), 1.41 (9H, s), 1.49 (3H, s), 1.61 (3H, s), 1.79 (3H, s), 2.33 (3H, s), 2.63-2.93 (7H, m), 3.57-3.71 (2H, m), 4.12 (1H, d, J=7.1Hz), 4.25 (1H, d, J=8.3Hz), 4.33 (1H, d, J=8.3Hz), 4.37-4.69 (4H, m), 4.86-4.98 (2H, m), 5.11 (1H, d, J=9.0Hz), 5.23 (1H, d, J=7.3Hz), 6.01 (1H, d, J=4.9Hz), 6.03-6.12 (1H, m), 7.44-7.54 (2H, m), 7.61 (1H, t, J=7.4Hz), 8.13 (2H, d, J=7.3Hz).
ESI-MS; m/z: 845 $(M+H)^+$.

[Example 114]

[Formula 186]

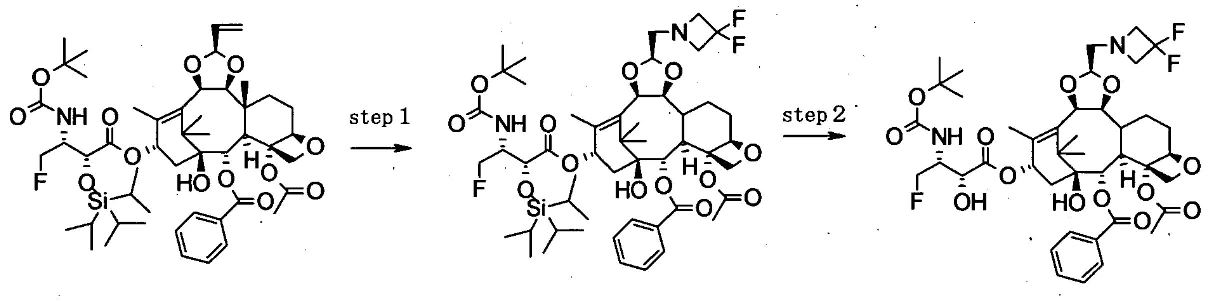


Step 1: (1S,2S,3R,4S,5R,8R,9S,10R,13S)-4-(Acetoxy)-2-(benzoyloxy)-9,10-[(1S)-2-(3,3-difluoroazetidino)ethylidenedioxy]-5,20-epoxy-1-hydroxytax-11-en-13-yl

(2R,3R)-3-(tert-butoxycarbonylamino)-4-fluoro-2-[(triisopropylsilyl)oxy]butyrate

The compound obtained in Example 34, Step 1 and the compound obtained in Reference Example 48, Step 2 were used as starting materials to perform the same procedure as that of Example 1 and thereby to obtain the title compound as white amorphous solid.
ESI-MS; m/z: 1023 $(M+H)^+$.

Step 2: (1S,2S,3R,4S,5R,8R,9S,10R,13S)-4-(Acetoxy)-2-(benzoyloxy)-9,10-[(1S)-2-(3,3-difluoroazetidino)ethylidenedioxy]-5,20-epoxy-1-hydroxytax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) $\delta$ : 1.25 (3H, s), 1.33-2.45 (7H, m), 1.41 (9H, s), 1.49 (3H, s), 1.60 (3H, s), 1.80 (3H, s), 2.33 (3H, s), 2.88 (1H, dd, J=12.2, 5.6Hz), 2.93 (1H, d, J=5.1Hz), 2.99 (1H, dd, J=12.2, 4.0Hz), 3.74 (4H, td, J=12.0, 2.0Hz), 4.14 (1H, d, J=6.8Hz), 4.24 (1H, d, J=8.5Hz), 4.29-4.67 (5H, m), 4.33 (1H, d, J=8.5Hz), 4.87-4.98 (2H, m), 5.10 (1H, d, J=8.8Hz), 5.24 (1H, d, J=6.8Hz), 6.00 (1H, d, J=4.9Hz), 6.07 (1H, t, J=8.3Hz), 7.49 (2H, t, J=7.8Hz), 7.62 (1H, t, J=7.8Hz), 8.13 (2H, d, J=7.8Hz).
ESI-MS; m/z: 867 $(M+H)^+$.

[Example 115]

[Formula 187]

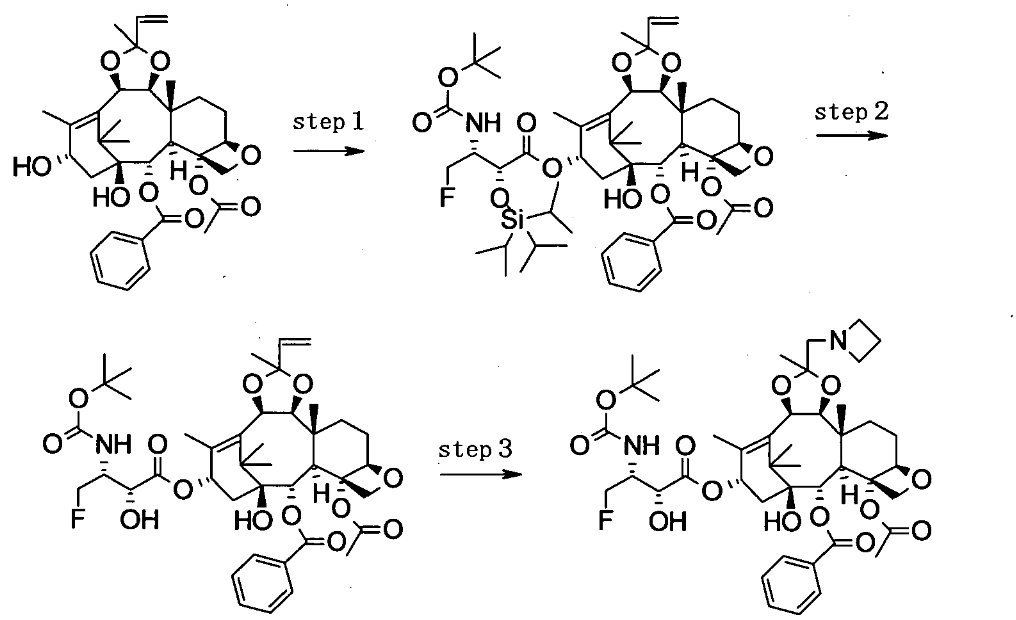


Step 1: (1S,2S,3R,4S,5R,8R,9S,10R,13S)-4-(Acetoxy)-2-(benzoyloxy)-5,20-epoxy-1-hydroxy-9,10-[(1-methyl)-2-propenylidenedioxy]tax-11-en-13-yl

(2R,3R)-3-(tert-butoxycarbonylamino)-4-fluoro-2-[(triisopropylsilyl)oxy]butyrate

The compound obtained in Example 81, Step 1 and the compound obtained in Reference Example 18, Step 4 were used as starting materials to perform the same procedure as that of Example 31, Step 1 and thereby to obtain the title compound as white amorphous solid.
$^1$H-NMR (400MHz, $CDCl_3$) δ : 1.08-1.19 (21H, m), 1.08-2.34 (6H, m), 1.30 (3H, s), 1.38 (9H, s), 1.43 (3H, s), 1.47 (3H, s), 1.65 (3H, s), 1.81 (3H, s), 2.33 (3H, s), 2.92 (1H, d, J=4.9Hz), 4.18 (1H, d, J=8.6Hz), 4.24 (1H, d, J=7.1Hz), 4.31-4.52 (5H, m), 4.76-4.81 (2H, m), 4.92 (1H, s), 5.20 (1H, dd, J=10.9, 1.1Hz), 5.44 (1H, dd, J=17.5, 1.1Hz), 5.60 (1H, d, J=7.1Hz), 5.95 (1H, d, J=5.1Hz), 6.01-6.11 (2H, m), 7.49 (2H, t, J=7.6Hz), 7.58-7.63 (1H, m), 8.13-8.17 (2H, m).

Step 2: (1S,2S,3R,4S,5R,8R,9S,10R,13S)-4-(Acetoxy)-2-(benzoyloxy)-5,20-epoxy-1-hydroxy-9,10-[(1-methyl)-2-propenylidenedioxy]tax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.24-2.41 (7H, m), 1.26 (3H, s), 1.42 (9H, s), 1.50 (3H, s), 1.59 (3H, s), 1.65 (3H, s), 1.82 (3H, s), 2.33 (3H, s), 2.92 (1H, d, J=4.9Hz), 4.19-4.26 (2H, m), 4.32 (1H, d, J=8.3Hz), 4.38-4.66 (5H, m), 4.94 (1H, s), 5.07-5.14 (1H, m), 5.21 (1H, d, J=11.0Hz), 5.44 (1H, d, J=17.4Hz), 5.64 (1H, d, J=7.1Hz), 6.00 (1H, d, J=5.1Hz), 6.03-6.12 (2H, m), 7.48 (2H, t, J=7.8Hz), 7.57-7.63 (1H, m), 8.11-8.15 (2H, m).

Step 3: (1S,2S,3R,4S,5R,8R,9S,10R,13S)-4-(Acetoxy)-2-(benzoyloxy)-5,20-epoxy-1-hydroxy-9,10-[(1-methyl)-2-(azetidino)ethylidenedioxy]tax-11-en-13-yl

(2R,3R)-3-(tert-butoxycarbonylamino)-4-fluoro-2-hydroxybutyrate

The compound obtained in Step 2 mentioned above was used as a starting material to perform the same procedure as that of Example 1 and thereby to obtain the title compound as white amorphous solid.
$^1$H-NMR (400MHz, $CDCl_3$) δ : 1.22-2.39 (9H, m), 1.24 (3H, s), 1.41 (9H, s), 1.44 (3H, s), 1.54 (3H, s), 1.61 (3H, s), 1.80 (3H, s), 2.33 (3H, s), 2.73 (1H, d, J=12.7Hz), 2.77 (1H, d, J=12.7Hz), 2.94 (1H, d, J=4.9Hz), 3.33-3.39 (4H, m), 4.17

(1H, d, J=7.4Hz), 4.26 (1H, d, J=8.6Hz), 4.33 (1H, d, J=8.6Hz), 4.40-4.65 (5H, m), 4.94 (1H, s), 5.09 (1H, d, J=9.1Hz), 5.60 (1H, d, J=7.4Hz), 5.99 (1H, d, J=4.9Hz), 6.08 (1H, t, J=7.8Hz), 7.48 (2H, t, J=7.7Hz), 7.61 (1H, t, J=7.5Hz), 8.13 (2H, d, J=7.6Hz).
FAB-MS; m/z: 845 $(M+H)^+$.

[Example 116]

(1S,2S,3R,4S,5R,8R,9S,10R,13S)-4-(Acetoxy)-2-(benzoyloxy)-5,20-epoxy-1-hydroxy-9,10-{1-methyl-2-[(2S)-2-hydroxymethylazetidino]ethylidenedioxy}tax-11-en-13-yl

(2R,3R)-3-(tert-butoxycarbonylamino)-4-fluoro-2-hydroxybutyrate

[Formula 188]

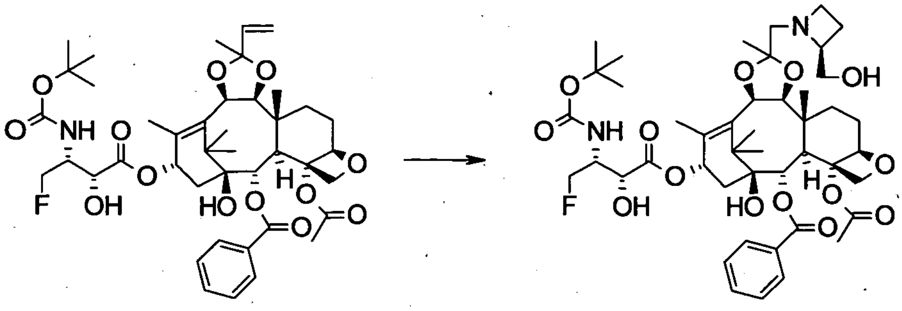

The compound obtained in Example 115, Step 2 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 1.22-2.39 (10H, m), 1.23 (3H, s), 1.41 (9H, s), 1.45 (3H, s), 1.54 (3H, s), 1.58 (3H, s), 1.80 (3H, s), 2.33 (3H, s), 2.77 (1H, d, J=12.7Hz), 2.83 (1H, d, J=12.7Hz), 2.94 (1H, d, J=5.1Hz), 3.11 (1H, q , J=7.8Hz), 3.41 (1H, d, J=11.5Hz), 3.45-3.53 (2H, m), 3.62 (1H, dd, J=11.5, 2.9Hz), 4.19 (1H, d, J=7.6Hz), 4.27 (1H, s), 4.33 (1H, d, J=8.3Hz), 4.39-4.65 (5H, m), 4.94 (1H, s), 5.08 (1H, d, J=9.1Hz), 5.61 (1H, d, J=7.6Hz), 5.97 (1H, d, J=5.1Hz), 6.07 (1H, t, J=7.6Hz), 7.48 (2H, t, J=7.6Hz), 7.61 (1H, t, J=7.4Hz), 8.13 (2H, d, J=7.6Hz).
FAB-MS; m/z: 875 $(M+H)^+$.

[Example 117]

(1S,2S,3R,4S,5R,8R,9S,10R,13S)-4-(Acetoxy)-2-(benzoyloxy)-5,20-epoxy-1-hydroxy-9,10-{(1S)-2-[(2R)-2-methylazetidino]ethylidenedioxy}tax-11-en-13-yl

(2R,3R)-3-(tert-butoxycarbonylamino)-4-fluoro-2-hydroxybutyrate

[Formula 189]

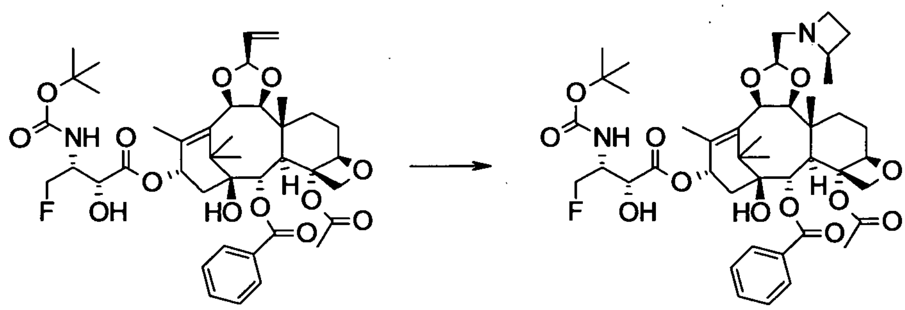

The compound obtained in Example 34, Step 2 and the compound obtained in Reference Example 49 were used as starting materials to perform the same procedure as that of Example 1 and thereby to obtain the title compound as white amorphous solid.
$^1$H-NMR (400MHz, $CDCl_3$) $\delta$ : 1.15-2.41 (8H, m), 1.23 (3H, d, J=6.1Hz), 1.24 (3H, s), 1.41 (9H, s), 1.49 (3H, s), 1.61 (3H, s), 1.79 (3H, s), 2.33 (3H, s), 2.74 (1H, dd, J=12.6, 4.3Hz), 2.87 (1H, dd, J=12.7, 5.4Hz), 2.91-3.01 (2H, m), 3.25-3.33 (1H, m), 3.50-3.54 (1H, m), 4.12 (1H, d, J=7.1Hz), 4.25 (1H, d, J=8.6Hz), 4.33 (1H, d, J=8.6Hz), 4.39-4.65 (4H, m), 4.91 (2H, t, J=5.4Hz), 5.12 (1H, d, J=8.6Hz), 5.24 (1H, d, J=7.1Hz), 6.01 (2H, d, J=4.9Hz), 6.07 (2H, t, J=7.6Hz), 7.48 (2H, t, J=7.7Hz), 7.61 (1H, t, J=7.4Hz), 8.13 (2H, d, J=7.6Hz).
FAB-MS; m/z: 845 $(M+H)^+$.

[Example 118]

[Formula 190]

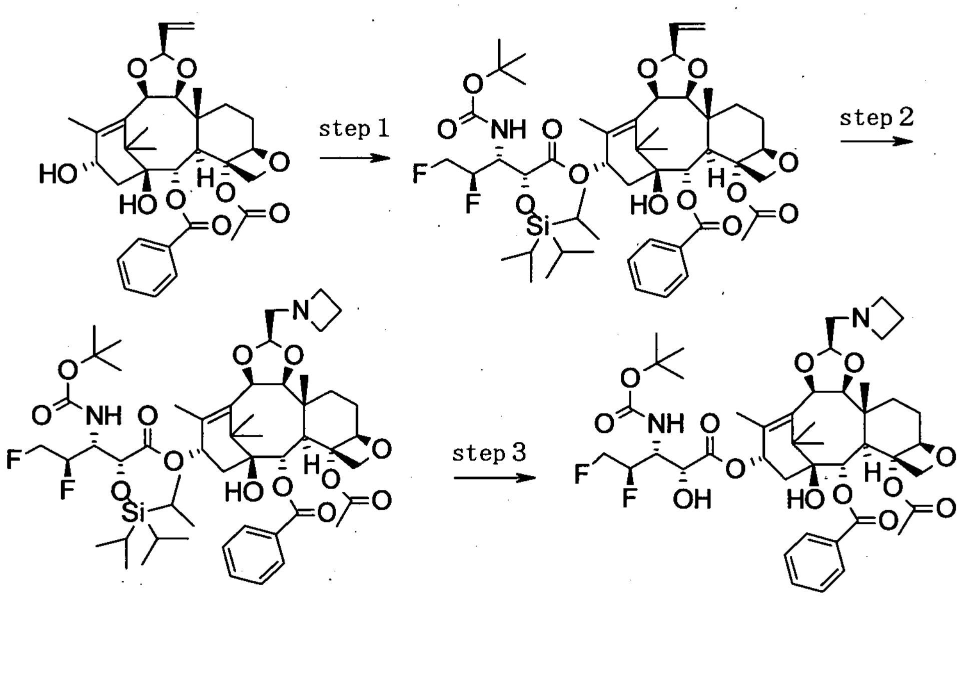

Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R,4R)-3-(tert-butoxycarbonylamino)-4,5-difluoro-2-[(triisopropylsilyl)oxylpentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 50, Step 5 were used as starting materials to perform the same procedure as that of Example 31, Step 1 and thereby to obtain the title compound as white amorphous solid.
$^1$H-NMR (400MHz, $CDCl_3$) δ : 1.05-1.22 (21H, m), 1.27 (3H, s), 1.32-2.44 (7H, m), 1.37 (9H, s), 1.51 (3H, s), 1.58 (3H, s), 1.79 (3H, s), 2.32 (3H, s), 2.92 (1H, d, J=4.9Hz), 4.16-4.22 (2H, m), 4.31-4.41 (2H, m), 4.51-4.99 (6H, m), 5.20 (1H, d, J=6.3Hz), 5.26 (1H, d, J=8.4Hz), 5.45 (1H, d, J=10.3Hz), 5.57 (1H, d, J=17.3Hz), 5.94-6.06 (3H, m), 7.44-7.54 (2H, m), 7.61 (1H, t, J=7.4Hz), 8.15 (2H, d, J=7.4Hz).

Step 2: (1S,2S,3R,4S,5R,8R,9S,10R,13S)-4-(Acetoxy)-2-(benzoyloxy)-5,20-epoxy-1-hydroxy-9,10-[(1S)-2-(azetidino) ethylidenedioxy]tax-11-en-13-yl

(2R,3R,4R)-3-(tert-butoxycarbonylamino)-4,5-difluoro-2-[(triisopropylsilyl)oxy]pentanoate

The compound obtained in Step 1 mentioned above was used as a starting material to perform the same procedure as that of Example 1 and thereby to obtain the title compound as white amorphous solid.
ESI-MS; m/z: 1019 $(M+H)^+$.

Step 3: (1S,2S,3R,4S,5R,8R,9S,10R,13S)-4-(Acetoxy)-2-(benzoyloxy)-5,20-epoxy-1-hydroxy-9,10-[(1S)-2-(azetidino) ethylidenedioxy]tax-11-en-13-yl

(2R,3R,4R)-3-(tert-butoxycarbonylamino)-4,5-difluoro-2-hydroxypentanoate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.24 (3H, s), 1.24-2.41 (10H, m), 1.40 (9H, s), 1.49 (3H, s), 1.61 (3H, s), 1.77 (3H, s), 2.34 (3H, s), 2.72-2.95 (3H, m), 3.42 (4H, brs), 4.12 (1H, d, J=7.1Hz), 4.24 (1H, d, J=8.3Hz), 4.34 (1H, d, J=8.3Hz), 4.45-4.94 (7H, m), 5.05 (1H, d, J=9.8Hz), 5.23 (1H, d, J=6.8Hz), 6.01 (1H, d, J=5.4Hz), 6.04-6.16 (1H, m), 7.43-7.53 (2H, m), 7.62 (1H, t, J=7.4Hz), 8.13 (2H, d, J=7.4Hz).
ESI-MS; m/z: 863 $(M+H)^+$.

[Example 119]

[Formula 191]

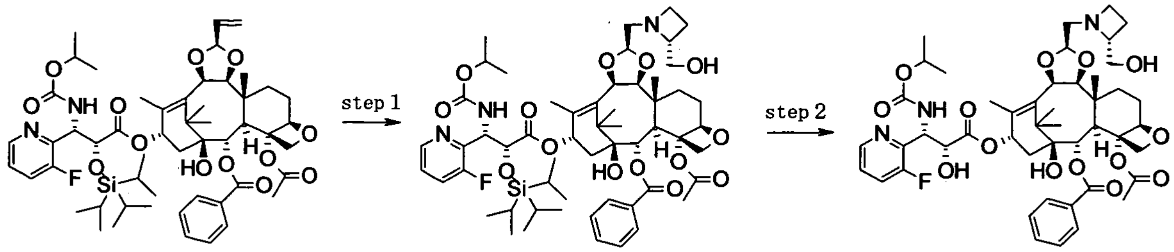


Step 1: (1S,2S,3R,4S,5R,8R,9S,10R,13S)-4-(Acetoxy)-2-(benzoyloxy)-5,20-epoxy-1-hydroxy-9,10-{(1S)-2-[(2R)-2-hydroxymethylazetidino]ethylidenedioxy}tax-11-en-13-yl

(2R,3S)-3-(3-fluoro-2-pyridyl)-3-(isopropoxycarbonylamino)-2-[(triisopropylsilyl)oxy]propionate

The compound obtained in Example 82, Step 1 and the compound obtained in Reference Example 26 were

used as starting materials to perform the same procedure as that of Example 1 and thereby to obtain the title compound as white amorphous solid.
$^1$H-NMR (400MHz, $CDCl_3$) δ : 0.90-0.94 (21H, m), 1.14-1.22 (8H, m), 1.35 (3H, s), 1.51 (3H, s), 1.62 (3H, s), 1.79 (3H, s), 1.91-2.00 (5H, m), 2.21-2.34 (3H, m), 2.47 (3H, s), 2.78 (1H, dd, J=12.9, 5.5Hz), 2.89-2.97 (2H, m), 3.08 (1H, q , J=8.3Hz), 3.42-3.51 (3H, m), 3.65 (1H, dd, J=11.4, 2.8Hz), 4.19 (2H, dd, J=15.2, 7.8Hz), 4.36 (1H, d, J=8.1Hz), 4.79-4.86 (2H, m), 4.95-4.98 (2H, m), 5.18 (1H, d, J=7.1Hz), 5.62 (1H, d, J=9.8Hz), 5.97 (1H, d, J=4.9Hz), 6.09-6.15 (2H, m), 7.25-7.29 (1H, m), 7.38-7.49 (3H, m), 7.59 (1H, t, J=7.4Hz), 8.16 (2H, d, J=7.4Hz), 8.38 (1H, d, J=4.7Hz).

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2R)-2-hydroxymethylazetidino]ethylidenedioxy}tax-11-en-13-yl

(2R,3S)-3-(3-fluoro-2-pyridyl)-2-hydroxy-3-(isopropoxycarbonylamino)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.14-1.26 (8H, m), 1.28 (3H, s), 1.49 (3H, s), 1.61 (3H, s), 1.76 (3H, s), 1.81-2.09 (7H, m), 2.22-2.44 (2H, m), 2.33 (3H, s), 2.79 (1H, dd, J=12.7, 5.6Hz), 2.91-2.95 (2H, m), 3.08 (1H, q , J=8.3Hz), 3.42-3.50 (3H, m), 3.65 (1H, dd, J=11.5, 2.9Hz), 4.09-4.14 (1H, m), 4.23 (1H, d, J=8.6Hz), 4.32 (1H, d, J=8.3Hz), 4.70 (1H, d, J=2.9Hz), 4.83-4.91 (3H, m), 5.22 (1H, d, J=7.1Hz), 5.70 (1H, d, J=8.8Hz), 5.99 (1H, d, J=4.9Hz), 6.11 (1H, t, J=8.3Hz), 6.27 (1H, d, J=8.6Hz), 7.28-7.32 (1H, m), 7.44-7.49 (3H, m), 7.60 (1H, t, J=7.4Hz), 8.13 (2H, d, J=7.4Hz), 8.40 (1H, d, J=4.7Hz). FAB-MS; m/z: 910 $(M+H)^+$.

[Example 120]

[Formula 192]

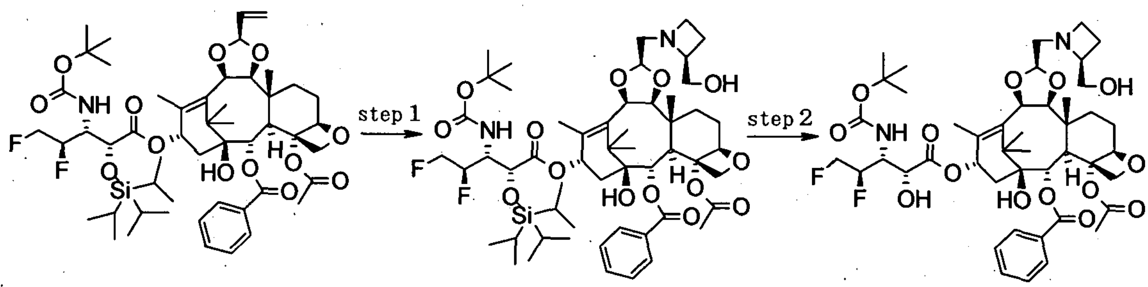


Step 1: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R,4R)-3-(tert-butoxycarbonylamino)-4,5-difluoro-2-[(triisopropylsilyl)oxy]pentanoate

The compound obtained in Example 118, Step 1 and the compound obtained in Reference Example 25 were used as starting materials to perform the same procedure as that of Example 1 and thereby to obtain the title compound as white amorphous solid.
ESI-MS; m/z: 1049 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R,4R)-3-(tert-butoxycarbonylamino)-4,5-difluoro-2-hydroxypentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.23 (3H, s), 1.29-2.41 (11H, m), 1.40 (9H, s), 1.49 (3H, s), 1.59 (3H, s), 1.77 (3H, s), 2.34 (3H, s), 2.80-2.98 (3H, m), 3.14 (1H, q , J=8.2Hz), 3.39-3.59 (3H, m), 3.62-3.72 (1H, m), 4.13 (1H, d, J=7.1Hz),

4.24 (1H, d, J=8.5Hz), 4.34 (1H, d, J=8.5Hz), 4.45-4.94 (7H, m), 5.07 (1H, d, J=10.0Hz), 5.24 (1H, d, J=7.3Hz), 5.99 (1H, d, J=5.1Hz), 6.03-6.16 (1H, m), 7.43-7.53 (2H, m), 7.62 (1H, t, J=7.3Hz), 8.13 (2H, d, J=7.3Hz).
ESI-MS; m/z: 893 $(M+H)^+$.

[Example 121]

[Formula 193]

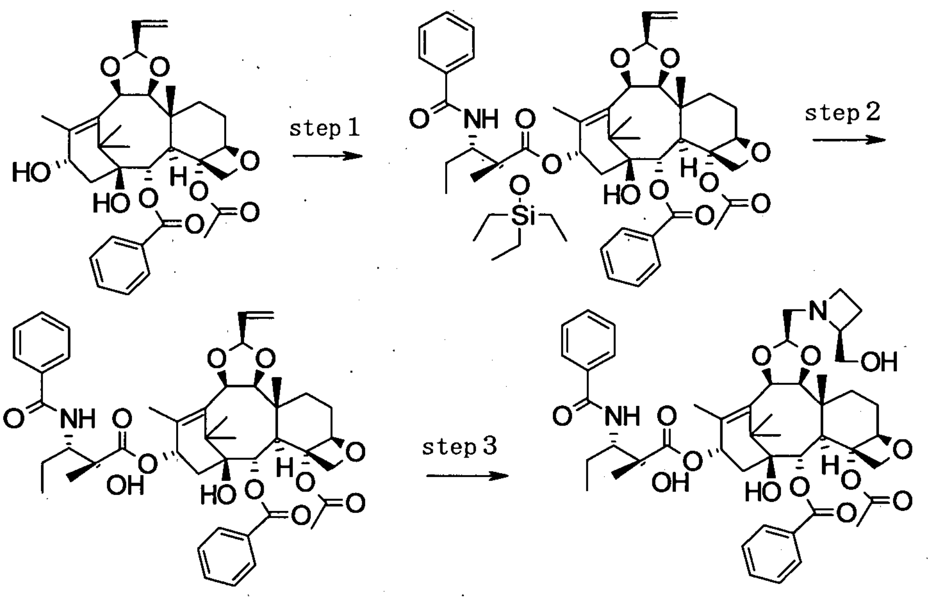


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(benzoylamino)-2-methyl-2-[(triethylsilyl)oxy]pentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 54, Step 6 were used as starting materials to perform the same procedure as that of Example 31, Step 1 and thereby to obtain the title compound as white amorphous solid, which was used as it was for the following reaction.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(benzoylamino)-2-hydroxy-2-methylpentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.01 (3H, t, J=7.1Hz), 1.26 (3H, s), 1.49 (3H, s), 1.52-2.24 (9H, m), 1.61 (3H, s), 1.63 (3H, s), 1.68 (3H, s), 2.50 (3H, s), 2.92 (1H, d, J=5.1Hz), 4.14 (1H, d, J=7.6Hz), 4.26 (1H, d, J=9.0Hz), 4.30-4.36 (2H, m), 4.54 (1H, dt, J=3.7, 10.2Hz), 4.95 (1H, s), 5.19-5.24 (2H, m), 5.46 (1H, d, J=10.5Hz), 5.57 (1H, d, J=17.6Hz), 5.94-6.05 (2H, m), 6.19 (1H, t, J=8.9Hz), 6.56 (1H, d, J=10.5Hz), 7.39-7.64 (6H, m), 7.75 (2H, d, J=8.5Hz), 8.16 (2H, d, J=9.0Hz).
ESI-MS; m/z: 802 $(M+H)^+$.

Step 3: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3S)-3-(benzoylamino)-2-hydroxy-2-methylpentanoate

The compound obtained in Step 2 mentioned above and the compound obtained in Reference Example 52 were used as starting materials to perform the same procedure as that of Example 1 and thereby to obtain the title compound as white amorphous solid.
$^1$H-NMR (400MHz, $CDCl_3$) δ : 1.01 (3H, t, J=7.4Hz), 1.24 (3H, s), 1.45-2.29 (12H, m), 1.48 (3H, s), 1.58 (3H, s), 1.61 (3H, s), 1.66 (3H, s), 2.50 (3H, s), 2.81 (1H, dd, J=12.7, 5.1Hz), 2.85-2.92 (2H, m), 3.05-3.13 (1H, m), 3.38-3.52 (3H, m), 3.64 (1H, dd, J=11.5, 2.9Hz), 4.09 (1H, d, J=7.1Hz), 4.23-4.36 (3H, m), 4.54 (1H, td, J=10.7, 2.8Hz), 4.85 (1H, t, J=4.9Hz), 4.94 (1H, s), 5.15 (1H, d, J=7.1Hz), 5.98 (1H, d, J=5.4Hz), 6.17 (1H, t, J=8.4Hz), 6.54 (1H, d, J=10.3Hz), 7.39-7.64 (6H, m), 7.74 (2H, d, J=8.5Hz), 8.16 (2H, d, J=8.5Hz).
ESI-MS; m/z: 875 $(M+H)^+$.

[Example 122]

[Formula 194]

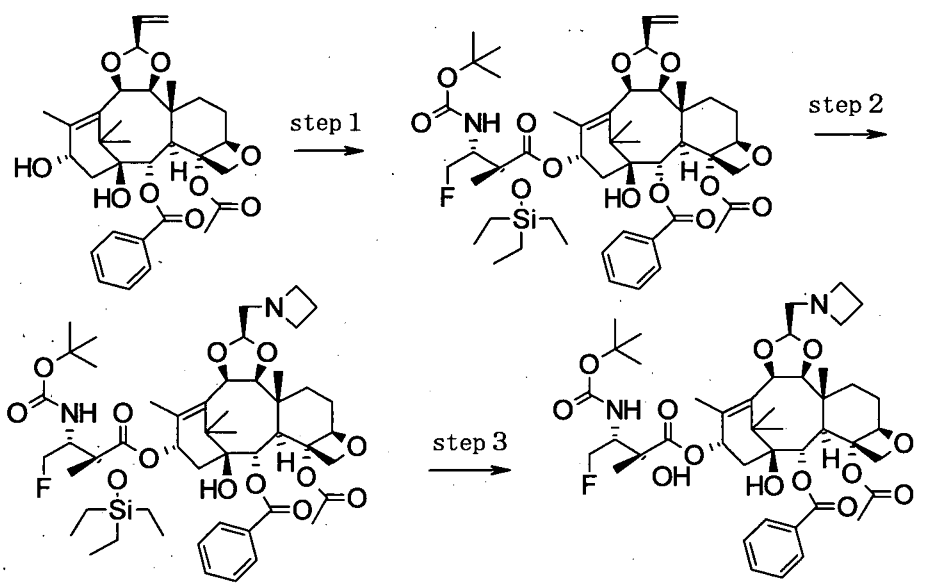


Step 1: (1S,2S,3R,4S,5R,8R,9S,10R,13S)-4-(Acetoxy)-2-(benzoyloxy)-5,20-epoxy-1-hydroxy-9,10-[(1S)-(2-propenylidenedioxy)]tax-11-en-13-yl

(2R,3R)-3-(tert-butoxycarbonylamino)-4-fluoro-2-methyl-2-[(triethylsilyl)oxy]butyrate

(1S,2S,3R,4S,5R,8R,9S, 10R, 13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 55, Step 7 were used as starting materials to perform the same procedure as that of Example 31, Step 1 and thereby to obtain the title compound as white amorphous solid.
$^1$H-NMR (400MHz, $CDCl_3$) δ : 0.61-0.79 (6H, m), 0.98 (9H, t, J=8.0Hz), 1.34 (3H, s), 1.35 (9H, s), 1.40-2.25 (7H, m), 1.51 (3H, s), 1.65 (3H, s), 1.74 (3H, s), 1.76 (3H, s), 2.49 (3H, s), 2.89 (1H, d, J=4.9Hz), 4.18 (1H, d, J=7.2Hz), 4.24 (1H, d, J=8.3Hz), 4.30-4.45 (3H, m), 4.57-4.74 (1H, m), 4.83 (1H, d, J=9.3Hz), 4.91, (1H, s), 5.21 (1H, d, J=6.4Hz), 5.26

(1H, d, J=7.4Hz), 5.46 (1H, d, J=10.3Hz), 5.58 (1H, d, J=17.4Hz), 5.95-6.04 (2H, m), 6.14 (1H, t, J=9.3Hz), 7.49 (2H, t, J=7.4Hz), 7.60 (1H, t, J=6.9Hz), 8.16 (2H, d, J=8.3Hz).

Step 2: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4-fluoro-2-methyl-2-[(triethylsilyl)oxy]butyrate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : 0.61-0.79 (6H, m), 0.98 (9H, t, J=7.8Hz), 1.33 (3H, s), 1.34-2.24 (10H, m), 1.35 (9H, s), 1.51 (3H, s), 1.61 (3H, s), 1.73 (3H, s), 1.74 (3H, s), 2.49 (3H, s), 2.82-2.95 (2H, m), 3.49-3.54 (3H, m), 4.22-4.36 (3H, m), 4.40-4.45 (1H, m), 4.57-4.62 (1H, m), 4.68-4.78 (2H, m), 4.84 (1H, d, J=10.0Hz), 4.89-4.95 (2H, m), 5.17 (1H, d, J=7.1Hz), 5.94 (1H, d, J=5.1Hz), 6.13 (1H, t, J=8.8Hz), 7.49 (2H, t, J=7.7Hz), 7.61 (1H, t, J=7.8Hz), 8.16 (2H, d, J=7.4Hz).

Step 3: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3R)-3-(tert-butoxycarbonylamino)-4-fluoro-2-hydroxy-2-methylbutyrate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.28 (3H, s), 1.41 (9H, s), 1.49 (3H, s), 1.60-2.17 (9H, m), 1.61 (3H, s), 1.62 (3H, s), 1.77 (3H, s), 2.24 (1H, dd, J=14.7, 9.3Hz), 2.43 (3H, s), 2.74 (1H, dd, J=12.5, 5.9Hz), 2.83 (1H, dd, J=12.5, 4.3Hz), 2.95 (1H, d, J=4.9Hz), 3.32-3.38 (4H, m), 4.12 (1H, d, J=7.1Hz), 4.17-4.29 (2H, m), 4.32 (1H, d, J=8.3Hz), 4.54-4.60 (1H, m), 4.66-4.72 (1H, m), 4.87 (1H, t, J=4.8Hz), 4.91 (1H, s), 5.20 (1H, d, J=6.9Hz), 5.28-5.30 (1H, m), 5.99 (1H, d, J=4.9Hz), 6.18 (1H, t, J=8.5Hz), 7.48 (2H, t, J=7.7Hz), 7.61 (1H, t, J=7.4Hz), 8.15 (2H, d, J=7.6Hz).
FAB-MS; m/z: 845 $(M+H)^+$.

[Example 123]

[Formula 195]

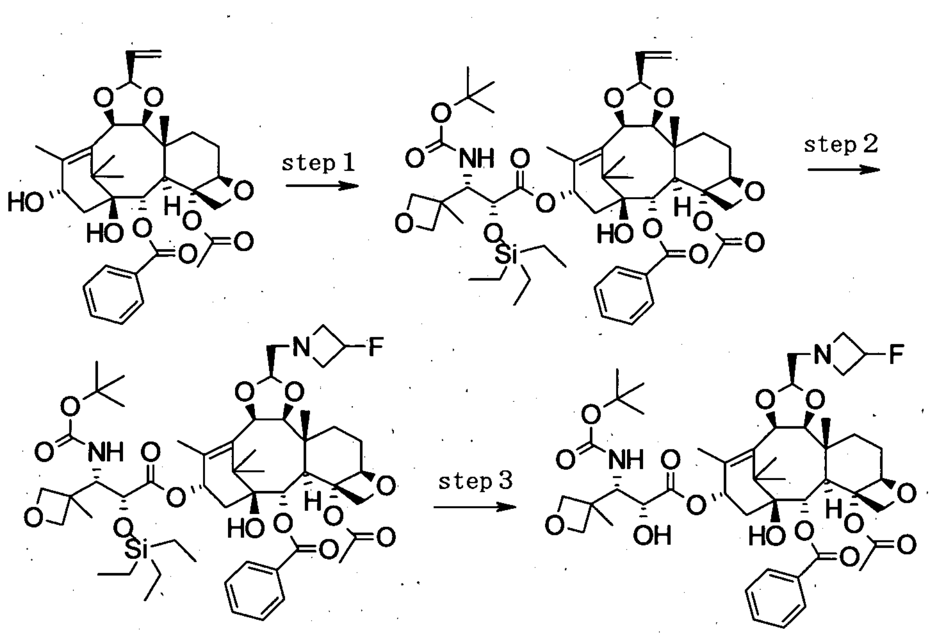

Step 1: (1S,2S,3R,4S,5R,8R,9S,10R,13S)-4-(Acetoxy)-2-(benzoyloxy)-5,20-epoxy-1-hydroxy-9,10-[(1S)-(2-propenylidenedioxy)]tax-11-en-13-yl

(2R,3S)-3-(tert-butoxycarbonylamino)-3-(3-methyl-3-oxetanyl)-2-[(triethylsilyl)oxy]pro pionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 51, Step 4 were used as starting materials to perform the same procedure as that of Example 31, Step 1 and thereby to obtain the title compound as white amorphous solid.
$^1$H-NMR (400MHz, $CDCl_3$) δ : 0.56-0.71 (6H, m), 0.98 (9H, t, J=7.8Hz), 1.19-2.29 (7H, m), 1.30 (3H, s), 1.39 (9H, s), 1.51 (3H, s), 1.54 (3H, s), 1.64 (3H, s), 1.78 (3H, s), 2.36 (3H, s), 2.91 (1H, d, J=4.6Hz), 3.96 (1H, dd, J=10.5, 2.0Hz), 4.18-4.28 (4H, m), 4.33 (1H, d, J=8.3Hz), 4.60 (1H, d, J=2.2Hz), 4.64 (1H, d, J=5.9Hz), 4.74 (1H, d, J=6.6Hz), 4.92 (1H, s), 5.20 (1H, d, J=6.3Hz), 5.26 (1H, d, J=6.8Hz), 5.33 (1H, d, J=10.5Hz), 5.45 (1H, d, J=10.7Hz), 5.57 (1H, d, J=17.3Hz), 5.94-6.06 (3H, m), 7.46-7.51 (2H, m), 7.57-7.64 (1H, m), 8.16-8.11 (2H, m).

Step 2: (1S,2S,3R,4S,5R,8R,9S,10R,13S)-4-(Acetoxy)-2-(benzoyloxy)-5,20-epoxy-9,10-[(1S)-2-(3-fluoroazetidino) ethylidenedioxy]-1-hydroxytax-11-en-13-yl

(2R,3S)-3-(tert-butoxycarbonylamino)-3-(3-methyl-3-oxetanyl)-2-[(triethylsilyl)oxy]propionate

The compound obtained in Step 1 mentioned above and the compound obtained in Reference Example 24, Step 2 were used as starting materials to perform the same procedure as that of Example 1 and thereby to obtain the title compound as white amorphous solid.
$^1$H-NMR (400MHz, $CDCl_3$) δ : 0.55-0.71 (6H, m), 0.98 (9H, t, J=8.0Hz), 1.22-2.24 (7H, m), 1.29 (3H, s), 1.39 (9H, s), 1.50 (3H, s), 1.53 (3H, s), 1.60 (3H, s), 1.76 (3H, s), 2.36 (3H, s), 2.81 (1H, dd, J=12.4, 5.6Hz), 2.87-2.94 (2H, m), 3.25-3.40 (2H, m), 3.73-3.88 (2H, m), 3.96 (1H, d, J=10.2Hz), 4.15 (1H, d, J=7.6Hz), 4.18-4.29 (3H, m), 4.33 (1H, d, J=8.5Hz), 4.60 (1H, s), 4.64 (1H, d, J=5.9Hz), 4.74 (1H, d, J=6.3Hz), 4.84 (1H, t, J=5.0Hz), 4.92 (1H, brs), 5.06-5.28 (2H, m), 5.34 (1H, d, J=10.7Hz), 5.95 (1H, d, J=4.9Hz), 6.02 (1H, t, J=8.5Hz), 7.49 (2H, t, J=7.6Hz), 7.61 (1H, t, J=7.3Hz), 8.16-8.11 (2H, m).

Step 3: (1S,2S,3R,4S,5R,8R,9S,10R,13S)-4-(Acetoxy)-2-(benzoyloxy)-5,20-epoxy-9,10-[(1S)-2-(3-fluoroazetidino) ethylidenedioxy]-1-hydroxytax-11-en-13-yl

(2R,3S)-3-(tert-butoxycarbonylamino)-2-hydroxy-3-(3-methyl-3-oxetanyl)propionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.24 (3H, s), 1.37-2.41 (7H, m), 1.41 (9H, s), 1.49 (3H, s), 1.54 (3H, s), 1.61 (3H, s), 1.81 (3H, s), 2.31 (3H, s), 2.82 (1H, dd, J=12.3, 5.9Hz), 2.88-2.95 (2H, m), 3.26-3.40 (2H, m), 3.75-3.86 (2H, m), 4.13 (1H, d, J=7.1Hz), 4.20-4.35 (6H, m), 4.48 (1H, s), 4.67 (1H, d, J=6.1Hz), 4.80 (1H, d, J=6.8Hz), 4.90 (1H, dd, J=5.4, 4.4Hz), 4.93 (1H, brs), 5.27-5.05 (2H, m), 5.36 (1H, d, J=10.3Hz), 6.00 (1H, d, J=4.4Hz), 6.07 (1H, t, J=8.3Hz), 7.48 (2H, t, J=7.8Hz), 7.61 (1H, tt, J=7.3, 1.5Hz), 8.15-8.10 (2H, m).
FAB-MS; m/z: 887 $(M+H)^+$.

[Example 124]

[Formula 196]

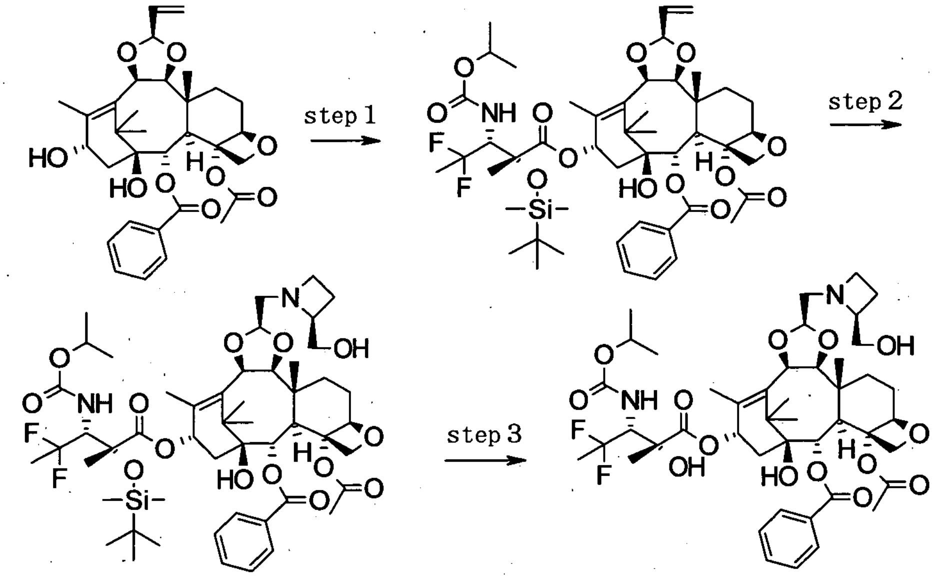


Step 1: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3R)-2-[(tert-butyldimethylsilyl)oxy]-4,4-difluoro-3-(isopropoxycarbonylamino)-2-methylpentanoate

(1S,2S,3R,4S,5R,8R,9S,10R,13S)-4-(Acetoxy)-2-(benzoyloxy)-1,13-dihydroxy-5, 20-epoxy-9,10-[(1S)-2-propenylidenedioxy]tax-11-ene and the compound obtained in Reference Example 56 were used as starting materials to perform the same procedure as that of Example 31, Step 1 and thereby to obtain the title compound as pale yellow amorphous solid, which was used as it was for the following reaction.

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-2-[(tert-butyldimethylsilyl)oxy]-4,4-difluoro-3-(isopropoxycarbonylamino)-2-methylpentanoate

The compound obtained in Step 1 mentioned above and the compound obtained in Reference Example 25 were used as starting materials to perform the same procedure as that of Example 1 and thereby to obtain the title compound as pale yellow amorphous solid.
$^1$H-NMR (400MHz, $CDCl_3$) δ : 0.13 (3H, s), 0.23 (3H, s), 0.94 (9H, s), 1.15 (3H, d, J=6.1Hz), 1.20 (3H, d, J=6.1Hz), 1.21-2.32 (11H, m), 1.32 (3H, s), 1.50 (3H, s), 1.60 (3H, s), 1.66 (3H, t, J=19.4Hz), 1.72 (3H, s), 1.85 (3H, s), 2.45 (3H, s), 2.80-2.93 (3H, m), 3.11 (1H, q , J=8.1Hz), 3.41-3.52 (3H, m), 3.64 (1H, dd, J=11.3, 3.2Hz), 4.24 (1H, d, J=8.1Hz), 4.30-4.43 (1H, m), 4.33 (1H, d, J=8.8Hz), 4.76-4.85 (1H, m), 4.86 (1H, t, J=4.7Hz), 4.93 (1H, s), 5.14 (1H, d, J=10.3Hz), 5.18 (1H, d, J=7.4Hz), 5.97 (1H, d, J=4.9Hz), 6.10 (1H, t, J=9.1Hz), 7.50 (2H, t, J=7.7Hz), 7.61 (1H, t, J=7.5Hz), 8.16 (2H, d, J=7.8Hz).

Step 3: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-4,4-difluoro-2-hydroxy-3-(isopropoxycarbonylamino)-2-methylpentanoate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.18 (3H, d, J=6.1Hz), 1.22 (3H, d, J=6.1Hz), 1.28 (3H, s), 1.49 (3H, s), 1.58-2.28 (14H, m), 1.60 (3H, s), 1.67 (3H, s), 1.75 (3H, s), 2.45 (3H, s), 2.81-2.93 (3H, m), 3.11 (1H, q , J=8.3Hz), 3.42-3.51 (3H, m), 3.66 (1H, d, J=11.5Hz), 4.13 (1H, d, J=7.1Hz), 4.25 (1H, d, J=8.6Hz), 4.33 (1H, d, J=8.3Hz), 4.47-4.56 (1H, m), 4.87-4.92 (3H, m), 5.20 (1H, d, J=6.6Hz), 5.43 (1H, d, J=10.3Hz), 5.98 (1H, d, J=3.7Hz), 6.18 (1H, t, J=8.6Hz), 7.49 (2H, t, J=7.2Hz),

7.62 (1H, t, J=7.4Hz), 8.15 (2H, d, J=8.3Hz).
FAB-MS; m/z: 893 $(M+H)^+$.

[Example 125]

[Formula 197]

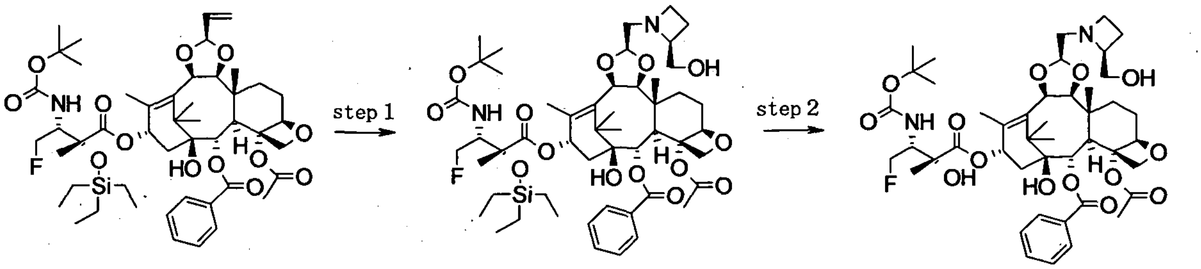


Step 1: (1S,2S,3R,4S,5R,8R,9S, 10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-3-(tert-butoxycarbonylamino)-4-fluoro-2-methyl-2-[(triethylsilyl)oxy]butyrate

The compound obtained in Example 122, Step 1 and the compound obtained in Reference Example 25 were used as starting materials to perform the same procedure as that of Example 1 and thereby to obtain the title compound as pale yellow amorphous solid.
$^1$H-NMR (400MHz, $CDCl_3$) δ : 0.61-0.78 (6H, m), 0.98 (9H, t, J=8.1Hz), 1.31-2.27 (11H, m), 1.33 (3H, s), 1.35 (9H, s), 1.51 (3H, s), 1.60 (3H, s), 1.74 (6H, s), 2.49 (3H, s), 2.78-2.96 (3H, m), 3.10 (1H, dd, J=15.9, 8.8Hz), 3.39-3.53 (3H, m), 3.63 (1H, dd, J=11.6, 2.9Hz), 4.09-4.45 (4H, m), 4.57-4.73 (1H, m), 4.82-5.00 (3H, m), 5.19 (1H, d, J=7.4Hz), 5.94 (1H, d, J=5.1Hz), 6.13 (1H, t, J=9.3Hz), 7.49 (2H, t, J=7.6Hz), 7.61 (1H, t, J=7.6Hz), 8.16 (2H, d, J=7.6Hz).

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-3-(tert-butoxycarbonylamino)-4-fluoro-2-hydroxy-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.28 (3H, s), 1.39-2.08 (8H, m), 1.41 (9H, s), 1.49 (3H, s), 1.60 (3H, s), 1.61 (3H, s), 1.77 (3H, s), 2.20-2.27 (3H, m), 2.43 (3H, s), 2.83 (1H, dd, J=12.7, 4.7Hz), 2.91 (1H, dd, J=13.0, 5.1Hz), 2.94 (1H, d, J=5.1Hz), 3.10 (1H, dd, J=16.2, 8.8Hz), 3.41-3.52 (3H, m), 3.65 (1H, dd, J=11.5, 2.9Hz), 4.13 (1H, d, J=7.1Hz), 4.19-4.28 (1H, m), 4.26 (1H, d, J=8.6Hz), 4.32 (1H, d, J=8.6Hz), 4.57 (1H, d, J=4.4Hz), 4.69 (1H, d, J=4.4Hz), 4.89 (1H, t, J=5.0Hz), 4.91 (1H, s), 5.22 (1H, d, J=7.1Hz), 5.31 (1H, d, J=10.5Hz), 5.97 (1H, d, J=4.9Hz), 6.17 (1H, t, J=8.6Hz), 7.48 (2H, t, J=7.8Hz), 7.61 (1H, t, J=7.6Hz), 8.15 (2H, d, J=7.1Hz).
FAB-MS; m/z: 875 $(M+H)^+$.

[Example 126]

[Formula 198]

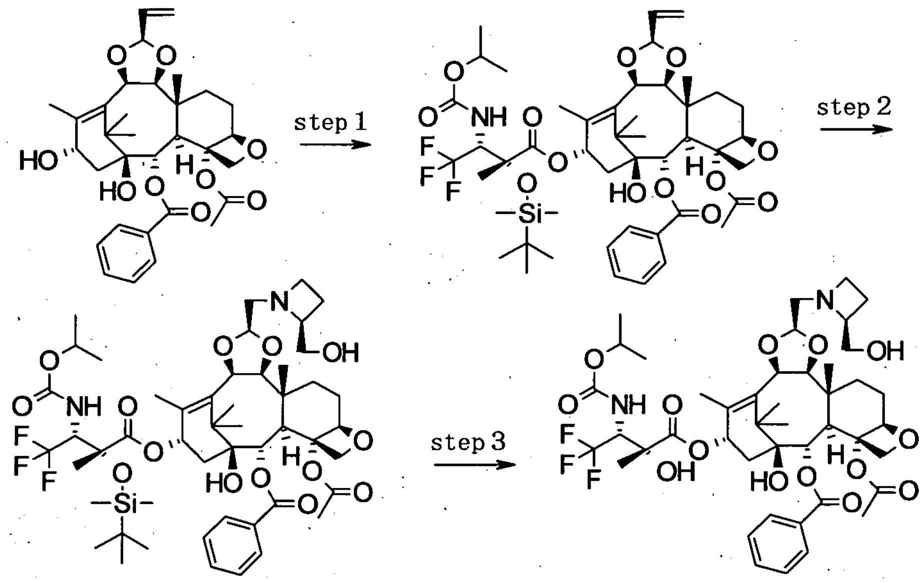


Step 1: (1S,2S,3R,4S,5R,8R,9S,10R,13S)-4-Acetoxy-2-(benzoyloxy)-5,20-epoxy-1-hydroxy-9,10-[(1S)-2-propenylidenedioxy]tax-11-en-13-yl

(2R,3R)-2-[(tert-butyldimethylsilyl)oxy]-3-(isopropoxycarbonylamino)-2-methyl-4,4,4-trifluorobutyrate

(1S,2S,3R,4S,5R,8R,9S,10R,13S)-4-(Acetoxy)-2-(benzoyloxy)-1,13-dihydroxy-5, 20-epoxy-9,10-[(1S)-2-propenylidenedioxy]tax-11-ene and the compound obtained in Reference Example 59, Step 2 were used as starting materials to perform the same procedure as that of Example 31, Step 1 and thereby to obtain the title compound as white amorphous solid, which was used as it was for the following reaction.

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-2-[(tert-butyldimethylsilyl)oxy]-3-(isopropoxycarbonylamino)-2-methyl-4,4,4-trifluorobutyrate

The compound obtained in Step 1 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) $\delta$ : 0.14 (3H, s), 0.24 (3H, s), 0.92 (9H, s), 1.15 (3H, d, J=6.4Hz), 1.20 (3H, d, J=6.4Hz), 1.32 (3H, s), 1.50 (3H, s), 1.56-2.29 (10H, m), 1.60 (3H, s), 1.72 (3H, s), 1.85 (3H, s), 2.46 (3H, s), 2.79-2.91 (3H, m), 3.10 (1H, q , J=8.3Hz), 3.40-3.51 (3H, m), 3.63 (1H, dd, J=11.5, 2.9Hz), 4.12 (1H, d, J=7.4Hz), 4.24 (1H, d, J=8.3Hz), 4.32 (1H, d, J=8.3Hz), 4.33-4.50 (1H, m), 4.62-4.70 (1H, m), 4.79-4.87 (2H, m), 4.92 (1H, s), 5.16-5.19 (1H, m), 5.96 (1H, d, J=5.1Hz), 6.08-6.18 (1H, m), 7.50 (2H, t, J=7.6Hz), 7.61 (1H, t, J=7.1Hz), 8.16 (2H, d, J=8.1Hz).

Step 3: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-2-hydroxy-3-(isopropoxycarbonylamino)-2-methyl-4,4,4-trifluorobutyrate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) $\delta$ : 1.19 (3H, d, J=6.4Hz), 1.23 (3H, d, J=6.4Hz), 1.27 (3H, s), 1.48 (3H, s), 1.60-2.09 (8H, m), 1.60 (3H, s), 1.69 (3H, s), 1.72 (3H, s), 2.17-2.28 (2H, m), 2.43 (3H, s), 2.81-2.94 (3H, m), 3.11 (1H, dd, J=15.9, 8.0Hz), 3.43 (1H, dd, J=11.7, 2.0Hz), 3.46-3.53 (2H, m), 3.66 (1H, dd, J=11.3, 2.9Hz), 4.12 (1H, d, J=7.1Hz), 4.26 (1H, d, J=8.6Hz), 4.33 (1H, d, J=8.6Hz), 4.66 (1H, s), 4.76-4.96 (4H, m), 5.20 (1H, d, J=6.9Hz), 5.50 (1H, d, J=10.3Hz), 5.99 (1H, d, J=4.9Hz), 6.21 (1H, t, J=8.5Hz), 7.49 (2H, t, J=7.7Hz), 7.62 (1H, t, J=7.2Hz), 8.15 (2H, d, J=7.8Hz).
FAB-MS; m/z: 897 $(M+H)^+$.

[Example 127]

[Formula 199]

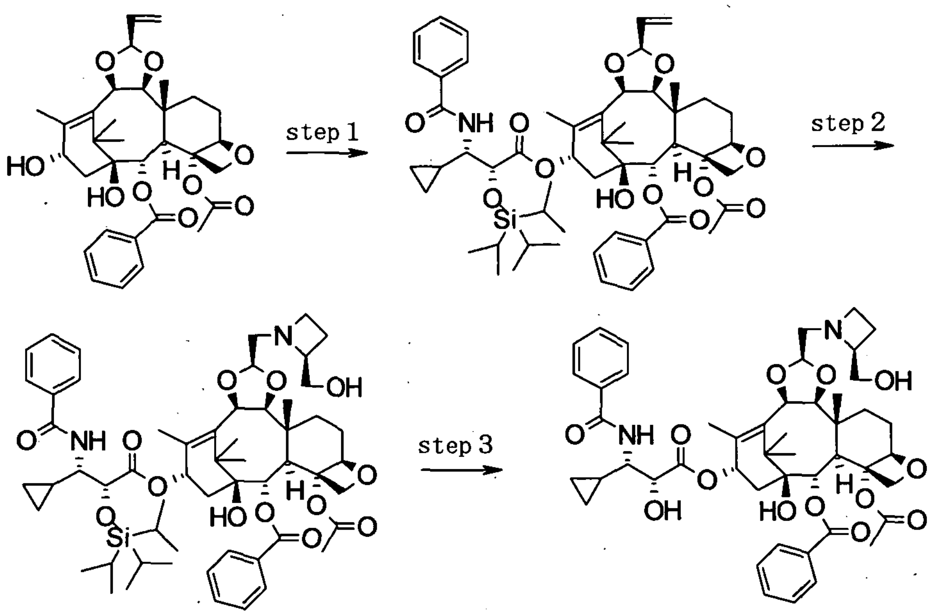


Step 1: (1S,2S,3R,4S,5R,8R,9S,10R,13S)-4-(Acetoxy)-2-(benzoyloxy)-5,20-epoxy-1-hydroxy-9,10-[(1S)-(2-propenylidenedioxy)]tax-11-en-13-yl

(2R,3S)-3-(benzoylamino)-3-cyclopropyl-2-[(triisopropylsilyl)oxy]butyrate

(1S,2S,3R,4S,5R,8R,9S,10R,13S)-4-(Acetoxy)-2-(benzoyloxy)-1,13-dihydroxy-5, 20-epoxy-9,10-[(1S)-2-propenylidenedioxy]tax-11-ene and the compound obtained in Reference Example 58, Step 7 were used as starting materials to perform the same procedure as that of Example 31, Step 1 and thereby to obtain the title compound as white amorphous solid.
$^1$H-NMR (400MHz, $CDCl_3$) δ : 0.39-0.42 (1H, m), 0.62-0.69 (3H, m), 1.10-1.34 (26H, m), 1.51 (3H, s), 1.62 (3H, s), 1.78 (3H, s), 1.87-1.93 (3H, m), 2.03-2.06 (1H, m), 2.19 (2H, d, J=8.8Hz), 2.39 (3H, s), 2.89 (1H, d, J=4.7Hz), 3.93 (1H, td, J=9.4, 1.1Hz 4.20 (2H, dd, J=12.1, 7.7Hz), 4.35 (1H, d, J=8.3Hz), 4.81 (1H, d, J=1.5Hz), 4.94 (1H, s), 5.19 (1H, dd, J=6.6, 2.5Hz), 5.24 (1H, d, J=6.9Hz), 5.45 (1H, d, J=10.3Hz), 5.56 (1H, d, J=17.2Hz), 5.95-6.06 (3H, m), 6.63 (1H, d, J=9.3Hz), 7.38-7.53 (5H, m), 7.59-7.63 (1H, m), 7.70 (2H, d, J=7.1Hz), 8.14 (2H, d, J=7.1Hz).
ESI-MS; m/z: 978 $(M+Na)^+$.

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3S)-3-(benzoylamino)-3-cyclopropyl-2-[(triisopropylsilyl)oxy]butyrate

The compound obtained in Step 1 mentioned above and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.38-0.41 (1H, m), 0.61-0.67 (3H, m), 1.09-1.31 (26H, m), 1.50 (3H, s), 1.56 (3H, s), 1.75 (3H, s), 1.88-2.04 (7H, m), 2.16-2.25 (2H, m), 2.38 (3H, s), 2.81-2.86 (3H, m), 3.09 (1H, dd, J=16.3, 8.2Hz), 3.39-3.50 (3H, m), 3.62 (1H, dd, J=11.6, 3.1Hz), 3.92 (1H, t, J=9.6Hz), 4.13 (1H, d, J=7.4Hz), 4.22 (1H, d, J=8.3Hz), 4.34 (1H, d, J=8.3Hz), 4.80 (1H, s), 4.83 (1H, t, J=4.9Hz), 4.94 (1H, s), 5.17 (1H, d, J=7.4Hz), 5.95 (1H, d, J=5.2Hz), 6.02 (1H, t,

J=8.7Hz), 6.63 (1H, d, J=9.6Hz), 7.38-7.52 (5H, m), 7.61 (1H, t, J=7.1Hz), 7.69 (2H, d, J=7.1Hz), 8.14 (2H, d, J=7.4Hz).

Step 3: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3S)-3-(benzoylamino)-3-cyclopropyl-2-hydroxybutyrate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 0.48-0.66 (4H, m), 1.20 (3H, s), 1.24-1.34 (1H, m), 1.48 (3H, s), 1.56 (3H, s), 1.65 (3H, s), 1.75-2.05 (8H, m), 2.20-2.35 (2H, m), 2.37 (3H, s), 2.79-2.90 (3H, m), 3.09 (1H, dd, J=16.2, 8.7Hz), 3.39-3.51 (4H, m), 3.63 (1H, dd, J=11.5, 2.9Hz), 4.07-4.14 (2H, m), 4.25 (1H, d, J=8.5Hz), 4.33 (1H, d, J=8.3Hz), 4.59 (1H, d, J=2.0Hz), 4.84 (1H, t, J=5.0Hz), 4.96 (1H, s), 5.14 (1H, d, J=7.3Hz), 5.98 (1H, d, J=5.1Hz), 6.09 (1H, t, J=8.5Hz), 6.79 (1H, d, J=9.3Hz), 7.41-7.53 (5H, m), 7.59-7.63 (1H, m), 7.77-7.80 (2H, m), 8.11-8.14 (2H, m).
FAB-MS; m/z: 873 $(M+H)^+$.

[Example 128]

[Formula 200]

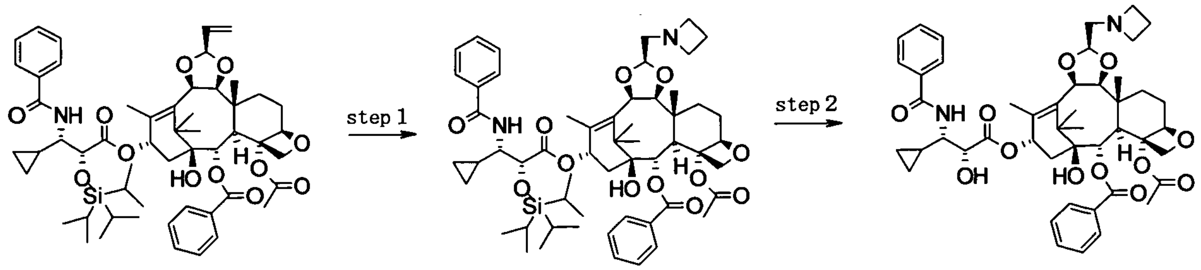


Step 1: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3S)-3-(benzoylamino)-3-cyclopropyl-2-[(triisopropylsilyl)oxy]butyrate

The compound obtained in Example 127, Step 1 was used as a starting material to perform the same procedure as that of Example 1 and thereby to obtain the title compound as white amorphous solid.
$^1$H-NMR (400MHz, $CDCl_3$) δ : 0.38-0.42 (1H, m), 0.61-0.68 (3H, m), 1.13-1.35 (26H, m), 1.50 (3H, s), 1.59 (3H, s), 1.75 (3H, s), 1.84-2.20 (8H, m), 2.38 (3H, s), 2.72 (1H, dd, J=12.5, 5.6Hz), 2.79 (1H, dd, J=12.5, 4.4Hz), 2.88 (1H, d, J=5.2Hz), 3.30-3.37 (4H, m), 3.92 (1H, td, J=9.3, 1.2Hz), 4.12 (1H, d, J=7.1Hz), 4.22 (1H, d, J=8.3Hz), 4.34 (1H, d, J=8.3Hz), 4.80-4.83 (2H, m), 4.94 (1H, s), 5.15 (1H, d, J=7.1Hz), 5.96 (1H, d, J=5.2Hz), 6.02 (1H, t, J=9.0Hz), 6.63 (1H, d, J=9.6Hz), 7.38-7.52 (5H, m), 7.59-7.63 (1H, m), 7.68-7.71 (2H, m), 8.13-8.15 (2H, m).
ESI-MS; m/z: 999 $(M+H)^+$.

Step 2: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3S)-3-(benzoylamino)-3-cyclopropyl-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 0.48-0.65 (4H, m), 1.21 (3H, s), 1.26-1.34 (1H, m), 1.48 (3H, s), 1.59 (3H, s), 1.65 (3H,

s), 1.84-2.15 (8H, m), 2.31-2.35 (1H, m), 2.37 (3H, s), 2.72 (1H, dd, J=12.5, 5.6Hz), 2.81 (1H, dd, J=12.4, 4.3Hz), 2.90 (1H, d, J=4.9Hz), 3.28-3.38 (4H, m), 4.06 (1H, d, J=7.1Hz), 4.12 (1H, td, J=9.3, 1.3Hz), 4.25 (1H, d, J=8.6Hz), 4.34 (1H, d, J=8.6Hz), 4.59 (1H, d, J=1.2Hz), 4.63 (1H, brs), 4.83 (1H, t, J=5.0Hz), 4.96 (1H, s), 5.13 (1H, d, J=6.6Hz), 6.00 (1H, d, J=4.9Hz), 6.09 (1H, t, J=8.2Hz), 6.79 (1H, d, J=9.3Hz), 7.39-7.52 (5H, m), 7.61 (1H, t, J=7.5Hz), 7.78 (2H, d, J=7.5Hz), 8.13 (2H, d, J=8.1Hz).
FAB-MS; m/z: 843 $(M+H)^+$.

[Example 129]

[Formula 201]

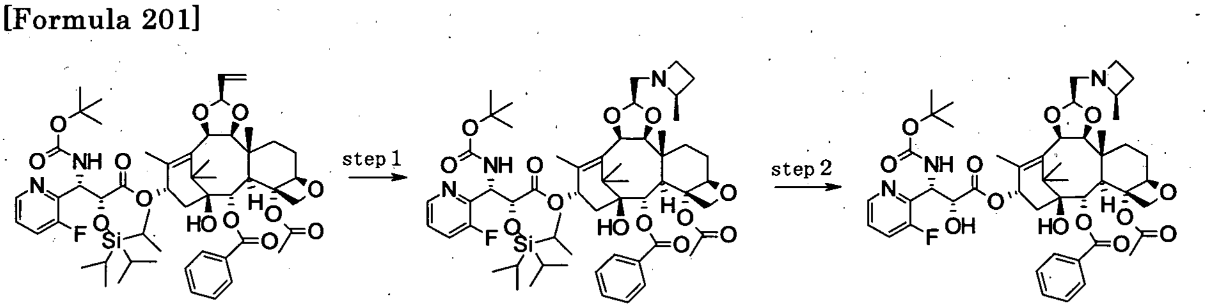


Step 1: (1S,2S,3R,4S,5R,8R,9S,10R,13S)-4-(Acetoxy)-2-(benzoyloxy)-5,20-epoxy-1-hydroxy-0-{(1S)-2-[(2R)-2-methylazetidino]ethylidenedioxy}tax-11-en-13-yl

(2R,3S)-3-(tert-butoxycarbonylamino)-3-(3-fluoro-2-pyridyl)-2-[(triisopropylsilyl)oxy]propionate

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl
(2R,3S)-3-(tert-butoxycarbonylamino)-3-(3-fluoro-2-pyridyl)-2-[(triisopropylsilyl)oxy]propionate and the compound obtained in Reference Example 49, Step 2 were used as starting materials to perform the same procedure as that of Example 1 and thereby to obtain the title compound as white amorphous solid.
$^1$H-NMR (400MHz, $CDCl_3$) δ : 0.86-0.93 (21H, m), 1.17-2.51 (9H, m), 1.23 (3H, d, J=6.1Hz), 1.34 (3H, s), 1.38 (9H, s), 1.52 (3H, s), 1.63 (3H, s), 1.80 (3H, s), 2.49 (3H, s), 2.72 (1H, dd, J=12.6, 4.5Hz), 2.86 (1H, dd, J=12.6, 5.3Hz), 2.93-3.00 (2H, m), 3.23-3.33 (1H, m), 3.51 (1H, t, J=6.9Hz), 4.16 (1H, d, J=8.3Hz), 4.22 (1H, d, J=8.3Hz), 4.35 (1H, d, J=8.3Hz), 4.86 (1H, t, J=4.9Hz), 4.94-4.96 (2H, m), 5.19 (1H, d, J=7.4Hz), 5.60 (1H, d, J=9.8Hz), 5.97 (1H, d, J=5.1Hz), 6.05-6.12 (2H, m), 7.24-7.29 (1H, m), 7.39 (1H, t, J=9.2Hz), 7.47 (2H, t, J=7.8Hz), 7.59 (1H, t, J=7.5Hz), 8.16 (2H, d, J=7.4Hz), 8.40 (1H, d, J=4.4Hz).

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2R)-2-methylazetidino]ethylidenedioxy}tax-11-en-13-yl

(2R,3S)-3-(tert-butoxycarbonylamino)-3-(3-fluoro-2-pyridyl)-2-hydroxypropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.22-2.49 (10H, m), 1.24 (3H, d, J=5.6Hz), 1.28 (3H, s), 1.40 (9H, s), 1.50 (3H, s), 1.62 (3H, s), 1.78 (3H, s), 2.34 (3H, s), 2.73 (1H, dd, J=12.7, 4.4Hz), 2.87 (1H, dd, J=12.6, 5.3Hz), 2.92-3.00 (2H, m), 3.23-3.33 (1H, m), 3.48-3.55 (1H, m), 4:14 (1H, d, J=7.1Hz), 4.23 (1H, d, J=8.3Hz), 4.33 (1H, d, J=8.3Hz), 4.67 (1H, d, J=2.2Hz), 4.88-4.93 (2H, m), 5.23 (1H, d, J=7.1Hz), 5.65 (1H, d, J=8.8Hz), 5.99 (1H, d, J=4.9Hz), 6.08 (1H, t, J=8.2Hz), 6.21 (1H, d, J=8.3Hz), 7.28-7.33 (1H, m), 7.42-7.50 (3H, m), 7.60 (1H, t, J=7.4Hz), 8.14 (2H, d, J=7.8Hz), 8.40 (1H, d, J=4.7Hz).
FAB-MS; m/z: 908 $(M+H)^+$.

[Example 130]

[Formula 202]

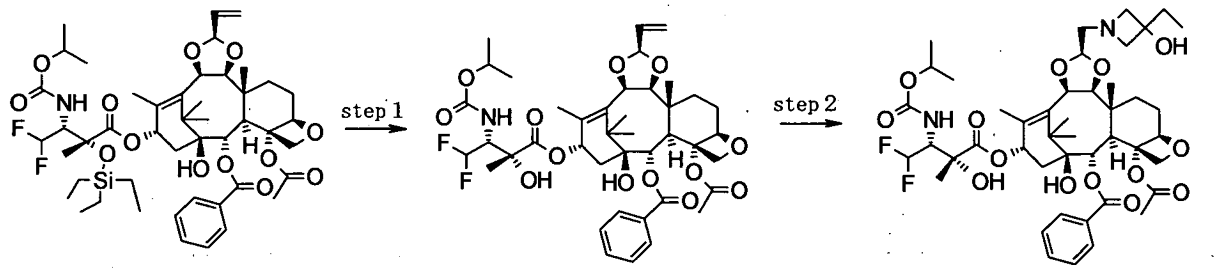


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R)-4,4-difluoro-2-hydroxy-3-(isopropoxycarbonylamino)-2-methylbutyrate

The compound obtained in Example 85, Step 1 was used as a starting material to perform the same procedure as that of Example 31, Step 2 and thereby to obtain the title compound as white amorphous solid.
$^1$H-NMR (400MHz, $CDCl_3$) δ : 1.20 (3H, d, J=7.1Hz), 1.23 (3H, d, J=6.3Hz), 1.29 (3H, s), 1.49 (3H, s), 1.58 (3H, s), 1.63 (3H, s), 1.77 (3H, s), 1.80-2.33 (7H, m), 2.43 (3H, s), 2.96 (1H, d, J=4.4Hz), 4.18 (1H, d, J=6.8Hz), 4.27 (1H, d, J=8.5Hz), 4.33 (1H, d, J=8.3Hz), 4.36-4.49 (1H, m), 4.66 (1H, s), 4.88-4.95 (2H, m), 5.25 (1H, d, J=6.3Hz), 5.29 (1H, d, J=7.6Hz), 5.44-5.49 (2H, m), 5.58 (1H, d, J=17.3Hz), 5.93-6.12 (3H, m), 6.22 (1H, t, J=9.6Hz), 7.48 (2H, t, J=7.7Hz), 7.61 (1H, t, J=7.4Hz), 8.15 (2H, d, J=8.5Hz).

Step 2: (1S,2S,3R,4S,5R,8R,9S,10R,13S)-4-(Acetoxy)-2-(benzoyloxy)-5,20-epoxy-9,10-[(1S)-2-(3-ethyl-3-hydroxyazetidino)ethylidenedioxy]-1-hydroxytax-11-en-13-yl

(2R,3R)-4,4-difluoro-2-hydroxy-3-(isopropoxycarbonylamino)-2-methylbutyrate

The compound obtained in Step 1 mentioned above and the compound obtained in Reference Example 4, Step 2 were used as starting materials to perform the same procedure as that of Example 1 and thereby to obtain the title compound as white amorphous solid.
$^1$H-NMR (400MHz, $CDCl_3$) δ : 0.97 (3H, t, J=7.3Hz), 1.20 (3H, d, J=6.3Hz), 1.23 (3H, d, J=6.3Hz), 1.28 (3H, s), 1.48 (3H, s), 1.52-2.26 (10H, m), 1.57 (3H, s), 1.62 (3H, s), 1.75 (3H, s), 2.42 (3H, s), 2.80 (1H, dd, J=12.8, 5.7Hz), 2.88-2.97 (2H, m), 3.15 (2H, t, J=8.4Hz), 3.46 (2H, t, J=6.8Hz), 4.12 (1H, d, J=6.8Hz), 4.27 (1H, d, J=8.1Hz), 4.33 (1H, d, J=8.5Hz), 4.37-4.50 (1H, m), 4.63 (1H, brs), 4.87-4.95 (3H, m), 5.20 (1H, d, J=6.8Hz), 5.46 (1H, d, J=10.5Hz), 6.00 (1H, d, J=4.6Hz), 6.10 (1H, td, J=55.7, 3.2Hz), 6.20 (1H, t, J=9.0Hz), 7.49 (2H, t, J=7.8Hz), 7.62 (1H, t, J=7.4Hz), 8.15 (2H, d, J=8.3Hz).
ESI-MS; m/z: 893 $(M+H)^+$.

[Example 131]

[Formula 203]

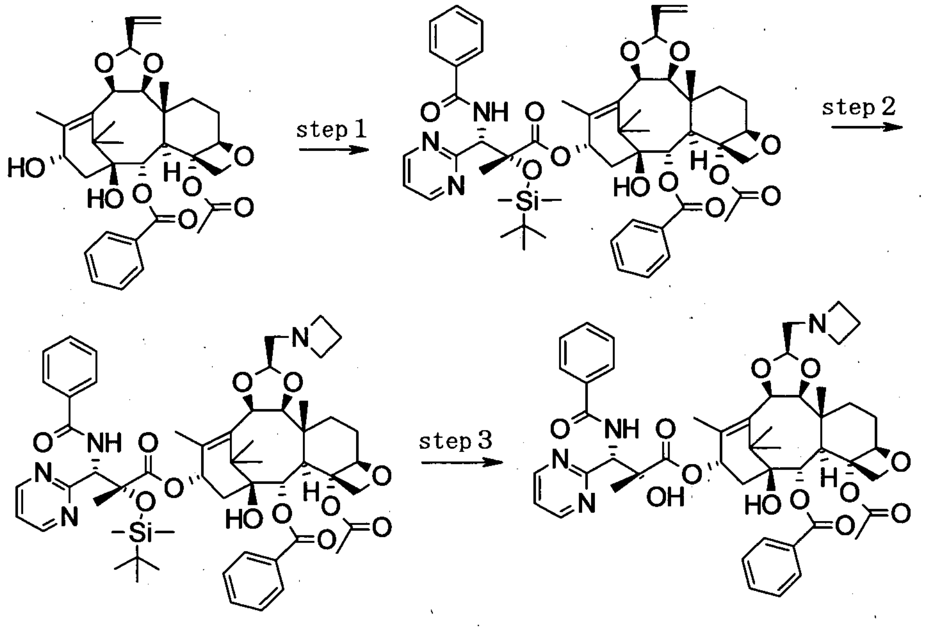


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(benzoylamino)-2-[(tert-butyldimethylsilyl)oxy]-2-methyl-3-(2-pyrimidinyl)propionate

(1S,2S,3R,4S,5R,8R,9S,10R,13S)-4-(Acetoxy)-2-(benzoyloxy)-1,13-dihydroxy-5, 20-epoxy-9,10-[(1S)-2-propenylidenedioxy]tax-11-ene and the compound obtained in Reference Example 60, Step 4 were used as starting materials to perform the same procedure as that of Example 31, Step 1 and thereby to obtain the title compound as white amorphous solid.
$^1$H-NMR (400MHz, $CDCl_3$) δ : -0.10 (3H, s), 0.25 (3H, s), 0.81 (9H, s), 0.87-2.35 (7H, m), 1.31 (3H, s), 1.53 (3H, s), 1.65 (3H, s), 1.76 (3H, s), 1.80 (3H, s), 2.83 (3H, s), 2.93 (1H, d, J=4.9Hz), 4.20 (1H, d, J=6.9Hz), 4.27 (1H, d, J=8.3Hz), 4.36 (1H, d, J=8.3Hz), 5.01 (1H, s), 5.22 (1H, d, J=6.4Hz), 5.27 (1H, d, J=6.9Hz), 5.45 (1H, d, J=10.3Hz), 5.58 (1H, d, J=17.2Hz), 5.90 (1H, d, J=10.0Hz), 5.96-6.06 (2H, m), 6.19 (1H, t, J=8.9Hz), 7.21-7.25 (1H, m), 7.41 (2H, t, J=7.7Hz), 7.45-7.51 (3H, m), 7.56-7.61 (1H, m), 7.77 (2H, d, J=8.1Hz), 8.19 (2H, d, J=8.3Hz), 8.72 (2H, dd, J=4.9, 1.0Hz).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(benzoylamino)-2-[(tert-butyldimethylsilyl)oxy]-2-methyl-3-(2-pyrimidinyl)propionate

The compound obtained in Step 1 mentioned above was used as a starting material to perform the same procedure as that of Example 1 and thereby to obtain the title compound as white amorphous solid.
$^1$H-NMR (400MHz, $CDCl_3$) δ : -0.11 (3H, s), 0.24 (3H, s), 0.80 (9H, s), 1.24-2.35 (9H, m), 1.29 (3H, s), 1.52 (3H, s), 1.61 (3H, s), 1.75 (3H, s), 1.78 (3H, s), 2.75-2.82 (1H, m), 2.82 (3H, s), 2.86 (1H, dd, J=12.5, 4.4Hz), 2.91 (1H, d, J=5.1Hz), 3.38-3.44 (4H, m), 4.13 (1H, d, J=7.1Hz), 4.27 (1H, d, J=8.3Hz), 4.36 (1H, d, J=8.1Hz), 4.87 (1H, t, J=4.9Hz), 5.00 (1H, s), 5.19 (1H, d, J=7.1Hz), 5.89 (1H, d, J=10.0Hz), 5.99 (1H, d, J=4.7Hz), 6.18 (1H, t, J=8.6Hz), 7.23 (1H, t, J=4.8Hz), 7.41 (2H, t, J=7.7Hz), 7.45-7.51 (3H, m), 7.57-7.62 (1H, m), 7.76 (2H, d, J=7.4Hz), 8.19 (2H, d, J=8.1Hz), 8.72 (2H, d, J=4.9Hz).

Step 3: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(benzoylamino)-2-hydroxy-2-methyl-3-(2-pyrimidinyl)propionate

The compound obtained in Step 2 mentioned above was used as a starting material to perform the same procedure as that of Example 31, Step 2 and thereby to obtain the title compound as white amorphous solid.

$^1$H-NMR (400MHz, $CDCl_3$) δ : 1.01-2.26 (9H, m), 1.29 (3H, s), 1.50 (3H, s), 1.61 (3H, s), 1.62 (3H, s), 1.64 (3H, s), 2.61 (3H, s), 2.74 (1H, dd, J=12.5, 5.9Hz), 2.82 (1H, dd, J=12.5, 4.4Hz), 2.93 (1H, d, J=5.1Hz), 3.32-3.40 (4H, m), 4.09-4.12 (1H, m), 4.24 (1H, d, J=8.3Hz), 4.35 (1H, d, J=8.3Hz), 4.84 (1H, t, J=5.0Hz), 4.96 (1H, s), 5.01 (1H, brs), 5.13 (1H, d, J=7.1Hz), 5.92 (1H, d, J=9.6Hz), 5.98 (1H, d, J=4.9Hz), 6.20 (1H, t, J=8.8Hz), 7.27-7.30 (1H, m), 7.41-7.53 (5H, m), 7.58-7.63 (1H, m), 7.72 (1H, d, J=9.6Hz), 7.86 (2H, d, J=7.4Hz), 8.15-8.19 (2H, m), 8.76 (2H, d, J=4.9Hz).
FAB-MS; m/z: 895 $(M+H)^+$.

[Example 132]

[Formula 204]

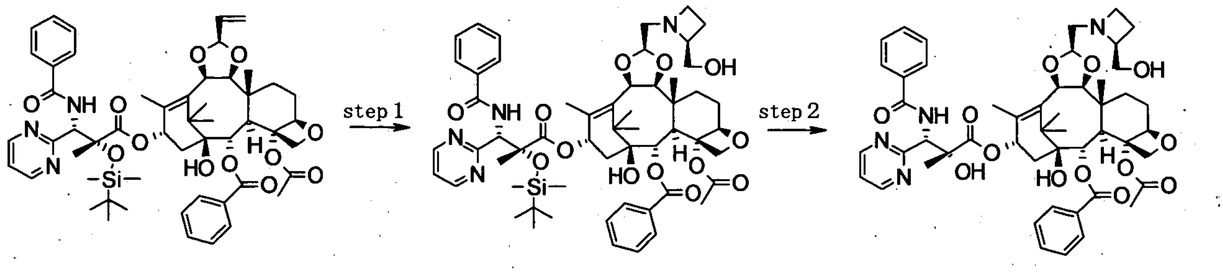


Step 1: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3S)-3-(benzoylamino)-2-[(tert-butyldimethylsilyl)oxy]-2-methyl-3-(2-pyrimidinyl)propionate

The compound obtained in Example 131, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : -0.11 (3H, s), 0.24 (3H, s), 0.80 (9H, s), 1.20-2.34 (10H, m), 1.28 (3H, s), 1.52 (3H, s), 1.59 (3H, s), 1.75 (3H, s), 1.78 (3H, s), 2.77-2.86 (1H, m), 2.82 (3H, s), 2.90 (1H, dd, J=11.8, 4.9Hz), 2.91 (1H, d, J=4.9Hz), 3.07-3.15 (1H, m), 3.39-3.54 (3H, m), 3.64 (1H, dd, J=11.5, 2.7Hz), 4.14 (1H, d, J=7.4Hz), 4.27 (1H, d, J=8.3Hz), 4.35 (1H, d, J=8.3Hz), 4.86 (1H, t, J=4.9Hz), 5.00 (1H, s), 5.20 (1H, d, J=7.1Hz), 5.89 (1H, d, J=10.0Hz), 5.98 (1H, d, J=4.9Hz), 6.18 (1H, t, J=9.1Hz), 7.23 (1H, t, J=4.9Hz), 7.41 (2H, t, J=7.5Hz), 7.48 (3H, t, J=6.9Hz), 7.59 (1H, t, J=7.2Hz), 7.76 (2H, d, J=7.4Hz), 8.19 (2H, d, J=7.4Hz), 8.72 (2H, d, J=4.9Hz).

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3S)-3-(benzoylamino)-2-hydroxy-2-methyl-3-(2-pyrimidinyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.22-2.27 (10H, m), 1.28 (3H, s), 1.50 (3H, s), 1.59 (3H, s), 1.63 (3H, s), 1.63 (3H, s), 2.62 (3H, s), 2.82 (1H, dd, J=13.0, 4.7Hz), 2.88 (1H, dd, J=12.7, 5.1Hz), 2.93 (1H, d, J=4.9Hz), 3.06-3.14 (1H, m), 3.42 (1H, dd, J=11.5, 2.7Hz), 3.45-3.52 (2H, m), 3.64 (1H, dd, J=11.4, 3.1Hz), 4.12 (1H, d, J=7.4Hz), 4.24 (1H, d, J=8.3Hz), 4.35 (1H, d, J=8.1Hz), 4.85 (1H, t, J=4.9Hz), 4.93-5.01 (2H, m), 5.15 (1H, d, J=7.1Hz), 5.92 (1H, d, J=9.6Hz), 5.97 (1H, d, J=5.1Hz), 6.19 (1H, t, J=8.8Hz), 7.28 (1H, t, J=4.9Hz), 7.41-7.54 (5H, m), 7.58-7.63 (1H, m), 7.72 (1H, d, J=9.8Hz), 7.84-7.87 (2H, m), 8.15-8.18 (2H, m), 8.76 (2H, d, J=4.9Hz).
FAB-MS; m/z: 925 $(M+H)^+$.

[Example 133]

[Formula 205]

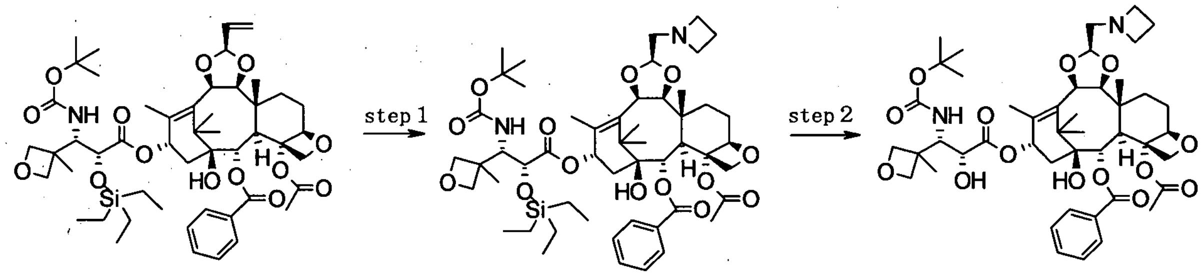


Step 1: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-3-(3-methyl-3-oxetanyl)-2-[(triethylsilyl)oxy]propionate

The compound obtained in Example 123, Step 1 was used as a starting material to perform the same procedure as that of Example 1 and thereby to obtain the title compound as white amorphous solid.
$^1$H-NMR (400MHz, $CDCl_3$) δ : 0.55-0.71 (6H, m), 0.98 (9H, t, J=8.1Hz), 1.10-2.37 (9H, m), 1.28 (3H, s), 1.38 (9H, s), 1.50 (3H, s), 1.53 (3H, s), 1.60 (3H, s), 1.75 (3H, s), 2.35 (3H, s), 2.80-2.99 (3H, m), 3.53 (4H, brs), 3.95 (1H, dd, J=10.4, 1.8Hz), 4.14 (1H, d, J=7.1Hz), 4.20 (1H, d, J=8.5Hz), 4.25 (2H, dd, J=8.2, 6.3Hz), 4.33 (1H, d, J=8.2Hz), 4.59 (1H, d, J=2.0Hz), 4.63 (1H, d, J=5.9Hz), 4.74 (1H, d, J=6.6Hz), 4.91 (2H, brs), 5.17 (1H, d, J=7.3Hz), 5.34 (1H, d, J=10.5Hz), 5.95 (1H, d, J=5.1Hz), 6.01 (1H, t, J=8.9Hz), 7.49 (2H, t, J=7.7Hz), 7.61 (1H, tt, J=7.4, 1.2Hz), 8.16-8.10 (2H, m).

Step 2: (1S,2S,3R,4S,5R,8R,9S,10R,13S)-4-(Acetoxy)-9,10-[(1S)-2-(azetidino)ethylidenedioxy]-2-(benzoyloxy)-5,20-epoxy-1-hydroxytax-11-en-13-yl

(2R,3S)-3-(tert-butoxycarbonylamino)-2-hydroxy-3-(3-methyl-3-oxetanyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.12-2.40 (9H, m), 1.23 (3H, s), 1.41 (9H, s), 1.49 (3H, s), 1.54 (3H, s), 1.60 (3H, s), 1.80 (3H, s), 2.31 (3H, s), 2.84 (3H, m), 3.37-3.46 (4H, m), 3.48-3.67 (1H, m), 4.12 (1H, d, J=7.1Hz), 4.19-4.34 (5H, m), 4.48 (1H, s), 4.67 (1H, d, J=6.1Hz), 4.80 (1H, d, J=6.8Hz), 4.89-4.95 (2H, m), 5.23 (1H, d, J=6.8Hz), 5.37 (1H, d, J=10.0Hz), 6.00 (1H, d, J=5.1Hz), 6.06 (1H, t, J=8.0Hz), 7.48 (2H, t, J=7.8Hz), 7.61 (1H, tt, J=7.4, 1.2Hz), 8.09-8.14 (2H, m).
FAB-MS; m/z: 869 $(M+H)^+$.

[Example 134]

[Formula 206]

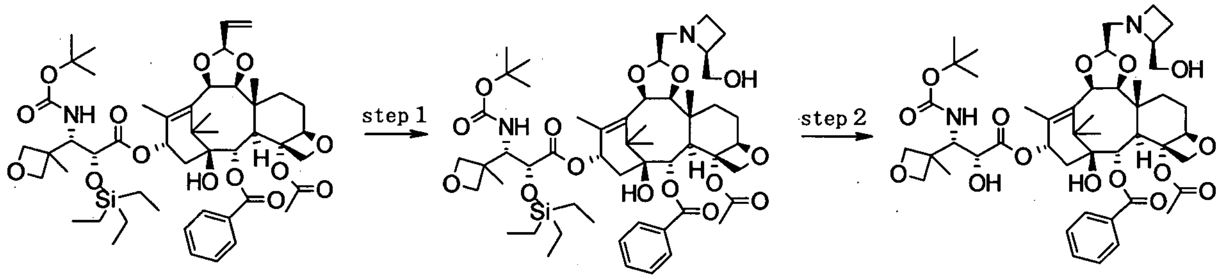


Step 1: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3S)-3-(tert-butoxycarbonylamino)-3-(3-methyl-3-oxetanyl)-2-[(triethylsilyl)oxy]propionate

The compound obtained in Example 123, Step 1 and the compound obtained in Reference Example 25 were used as starting materials to perform the same procedure as that of Example 1 and thereby to obtain the title compound as white amorphous solid.
$^1$H-NMR (400MHz, $CDCl_3$) δ : 0.55-0.71 (6H, m), 0.95-2.29 (10H, m), 0.98 (9H, t, J=7.8Hz), 1.28 (3H, s), 1.38 (9H, s), 1.50 (3H, s), 1.53 (3H, s), 1.58 (3H, s), 1.76 (3H, s), 2.35 (3H, s), 2.82 (1H, dd, J=12.7, 4.6Hz), 2.89 (1H, d, J=5.1Hz), 2.91 (1H, dd, J=12.7, 4.6Hz), 3.12 (1H, q , J=8.3Hz), 3.44 (1H, dd, J=11.7, 2.8Hz), 3.47-3.55 (2H, m), 3.64 (1H, dd, J=11.7, 2.8Hz), 3.95 (1H, dd, J=10.5, 1.7Hz), 4.15 (1H, d, J=7.3Hz), 4.20 (1H, d, J=8.3Hz), 4.25 (2H, dd, J=8.3, 6.3Hz), 4.32 (1H, d, J=8.1Hz), 4.59 (1H, d, J=2.2Hz), 4.63 (1H, d, J=5.9Hz), 4.74 (1H, d, J=6.8Hz), 4.85 (1H, t, J=4.9Hz), 4.91 (1H, brs), 5.18 (1H, d, J=7.1Hz), 5.33 (1H, d, J=10.3Hz), 5.94 (1H, d, J=5.1Hz), 6.01 (1H, t, J=8.9Hz), 7.49 (2H, t, J=7.7Hz), 7.60 (1H, tt, J=7.4, 1.5Hz), 8.15-8.11 (2H, m).

Step 2: (1S,2S,3R,4S,5R,8R,9S,10R,13S)-4-Acetoxy-2-(benzoyloxy)-5,20-epoxy-1-hydroxy-9,10-{(1S)-2-[(25)-hydroxymethylazetidino]ethylidenedioxy}tax-11-en-13-yl

(2R,3S)-3-(tert-butoxycarbonylamino)-2-hydroxy-3-(3-methyl-3-oxetanyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.22-2.40 (10H, m), 1.23 (3H, s), 1.41 (9H, s), 1.49 (3H, s), 1.54 (3H, s), 1.57 (3H, s), 1.80 (3H, s), 2.31 (3H, s), 2.86-2.93 (2H, m), 3.00 (1H, dd, J=12.8, 4.8Hz), 3.19 (1H, q , J=8.4Hz), 3.46-3.63 (4H, m), 3.66-3.72 (1H, m), 4.13 (1H, d, J=7.1Hz), 4.18-4.34 (5H, m), 4.48 (1H, s), 4.67 (1H, d, J=5.9Hz), 4.80 (1H, d, J=6.8Hz), 4.90-4.96 (2H, m), 5.24 (1H, d, J=7.1Hz), 5.38 (1H, d, J=10.2Hz), 5.98 (1H, d, J=4.9Hz), 6.06 (1H, t, J=8.3Hz), 7.47 (2H, t, J=7.7Hz), 7.60 (1H, tt, J=7.3, 1.2Hz), 8.13-8.09 (2H, m).
FAB-MS; m/z: 899 $(M+H)^+$.

[Example 135]

[Formula 207]

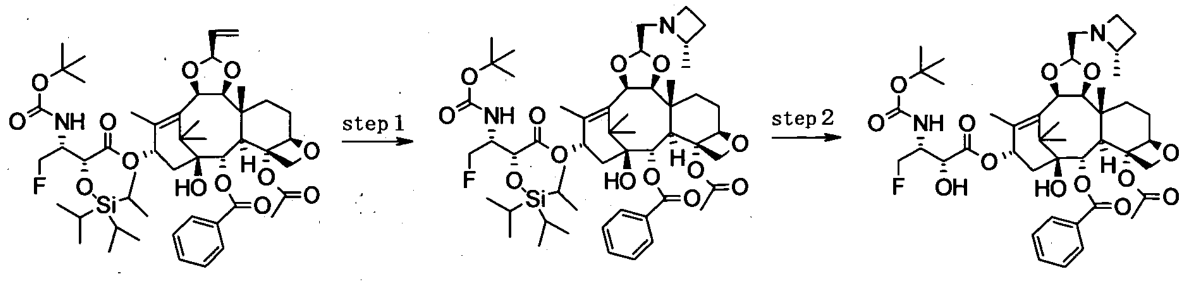


Step 1: (1S,2S,3R,4S,5R,8R,9S,10R,13S)-4-(Acetoxy)-2-(benzoyloxy)-5,20-epoxy-1-hydroxy-9,1 0-{(1S)-2-[(2S)-2-methylazetidino]ethylidenedioxy}tax-11-en-13-yl

(2R,3R)-3-(tert-butoxycarbonylamino)-4-fluoro-2-[(triisopropylsilyl)oxy]butyrate

The compound obtained in Example 34, Step 1 and the compound obtained in Reference Example 61, Step 3 were used as starting materials to perform the same procedure as that of Example 1 and thereby to obtain the title compound as white amorphous solid.
$^1$H-NMR (400MHz, $CDCl_3$) δ : 1.08-2.34 (9H, m), 1.09-1.14 (21H, m), 1.21 (3H, d, J=5.9Hz), 1.26 (3H, s), 1.37 (9H, s), 1.50 (3H, s), 1.61 (3H, s), 1.76 (3H, s), 2.32 (3H, s), 2.62 (1H, dd, J=12.6, 6.3Hz), 2.88-2.95 (3H, m), 3.21-3.30 (1H, m), 3.50 (1H, t, J=7.1Hz), 4.12 (1H, d, J=7.1Hz), 4.19 (1H, d, J=8.3Hz), 4.30-4.48 (4H, m), 4.76-4.80 (2H, m), 4.84-4.88 (1H, m), 4.91 (1H, s), 5.17 (1H, d, J=7.1Hz), 5.96 (1H, d, J=5.1Hz), 6.01 (1H, t, J=8.6Hz), 7.49 (2H, t, J=7.6Hz), 7.57-7.63 (1H, m), 8.14 (2H, d, J=8.3Hz).

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-methylazetidino]ethylidenedioxy}tax-11-en-13-yl

(2R,3R)-3-(tert-butoxycarbonylamino)-4-fluoro-2-hydroxybutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.20-2.41 (10H, m), 1.22 (3H, d, J=6.4Hz), 1.23 (3H, s), 1.41 (9H, s), 1.49 (3H, s), 1.61 (3H, s), 1.78 (3H, s), 2.33 (3H, s), 2.64 (1H, dd, J=12.5, 6.4Hz), 2.89-2.96 (3H, m), 3.22-3.31 (1H, m), 3.48-3.54 (1H, m), 4.11 (1H, d, J=7.1Hz), 4.24 (1H, d, J=8.6Hz), 4.33 (1H, d, J=8.3Hz), 4.39-4.65 (4H, m), 4.88-4.92 (1H, m), 4.94 (1H, s), 5.12 (1H, d, J=9.1Hz), 5.23 (1H, d, J=6.9Hz), 6.01 (1H, d, J=4.9Hz), 6.06 (1H, t, J=7.6Hz), 7.48 (2H, t, J=7.7Hz), 7.59-7.64 (1H, m), 8.11-8.15 (2H, m).
ESI-MS; m/z: 845 $(M+H)^+$.

[Example 136]

[Formula 208]

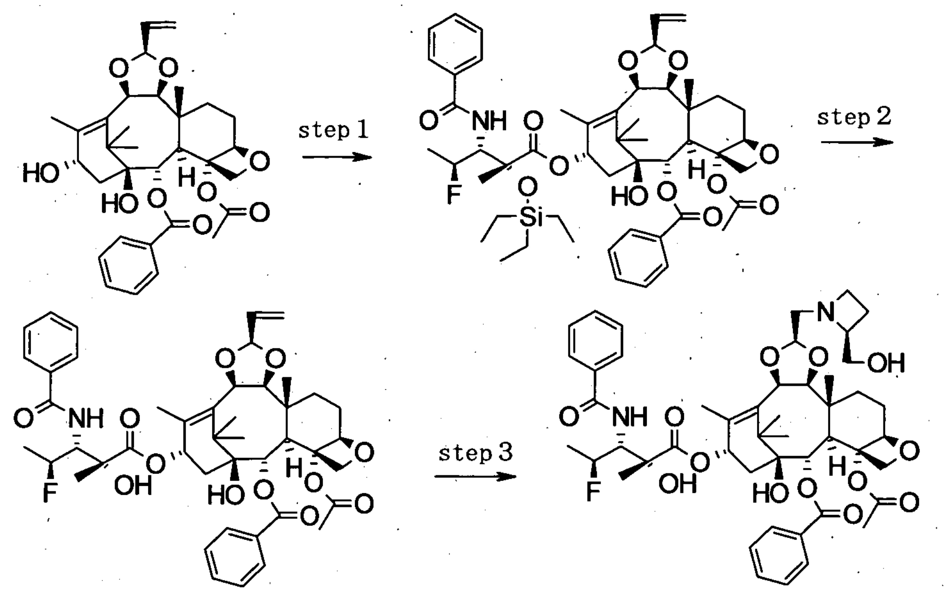


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R,4S)-3-(benzoylamino)-4-fluoro-2-methyl-2-[(triethylsilyl)oxy]pentanoate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 62, Step 4 were used as starting materials to perform the same procedure as that of Example 31, Step 1 and thereby to obtain the title compound as white amorphous solid, which was used as it was for the following reaction.

Step 2: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3R,4S)-3-(benzoylamino)-4-fluoro-2-hydroxy-2-methylpentanoate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) $\delta$ : 1.26 (3H, s), 1.48 (3H, dd, J=24.4, 7.3Hz), 1.49 (3H, s), 1.56-2.17 (7H, m), 1.59 (3H, s), 1.62 (3H, s), 1.67 (3H, s), 2.54 (3H, s), 2.91 (1H, d, J=4.9Hz), 4.14 (1H, d, J=7.6Hz), 4.21 (1H, brs), 4.25 (1H, d, J=8.8Hz), 4.34 (1H, d, J=8.8Hz), 4.82-5.01 (2H, m), 4.95 (1H, s), 5.21 (2H, d, J=6.3Hz), 5.45 (1H, d, J=10.7Hz), 5.56 (1H, d, J=17.6Hz), 5.94-6.05 (2H, m), 6.15 (1H, t, J=8.8Hz), 6.72 (1H, d, J=10.0Hz), 7.41-7.65 (6H, m), 7.72 (2H, d, J=8.5Hz), 8.17 (2H, d, J=7.8Hz).

Step 3: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R,4S)-3-(benzoylamino)-4-fluoro-2-hydroxy-2-methylpentanoate

The compound obtained in Step 2 mentioned above and the compound obtained in Reference Example 52 were used as starting materials to perform the same procedure as that of Example 1 and thereby to obtain the title compound as white amorphous solid.
$^1$H-NMR (400MHz, $CDCl_3$) $\delta$ : 1.22 (3H, s), 1.48 (3H, dd, J=24.6, 6.1Hz), 1.48 (3H, s), 1.57 (3H, s), 1.59-2.29 (10H, m), 1.66 (3H, s), 1.71 (3H, s), 2.54 (3H, s), 2.81 (1H, dd, J=13.4, 4.6Hz), 2.84-2.91 (2H, m), 3.09 (1H, dd, J=16.3, 8.3Hz), 3.38-3.52 (3H, m), 3.63 (1H, dd, J=11.2, 2.9Hz), 4.08-4.26 (1H, m), 4.09 (1H, d, J=7.8Hz), 4.25 (1H, d, J=8.5Hz), 4.33 (1H, d, J=8.0Hz), 4.81-5.00 (1H, m), 4.85 (1H, t, J=6.1Hz), 4.94 (1H, brs), 5.07-5.16 (2H, m), 5.96 (1H, d, J=5.9Hz), 6.13 (1H, t, J=8.7Hz), 6.70 (1H, d, J=10.0Hz), 7.42 (2H, t, J=7.3Hz), 7.47-7.54 (3H, m), 7.62 (1H, t, J=7.8Hz), 7.72 (2H,

d, J=7.3Hz), 8.17 (2H, d, J=7.8Hz).
ESI-MS; m/z: 893 $(M+H)^+$.

[Example 137]

[Formula 209]

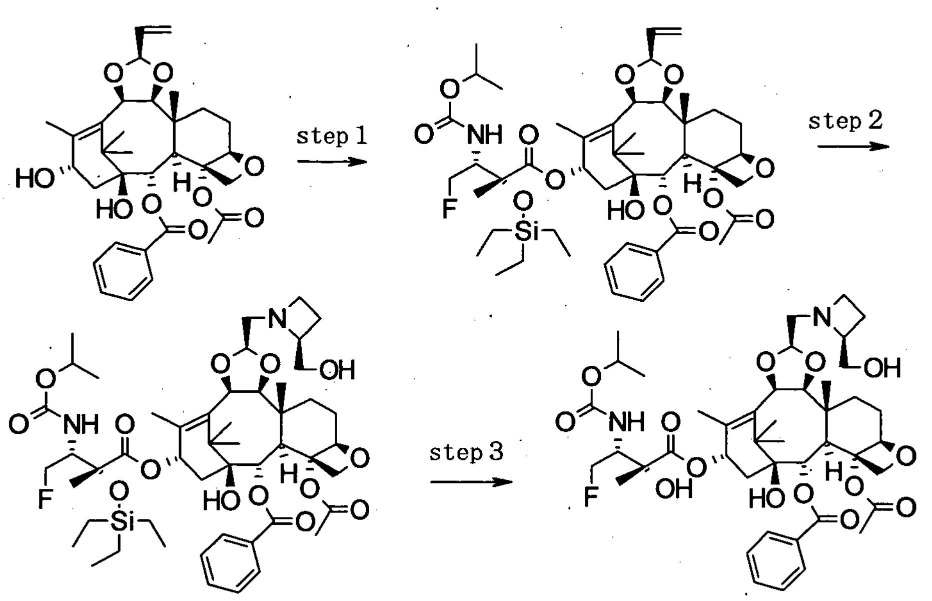


Step 1: (1S,2S,3R,4S,5R,8R,9S, 10R,13S)-4-(Acetoxy)-2-(benzoyloxy)-5,20-epoxy-1-hydroxy-9,10-[(1S)-(2-propenylidenedioxy)]tax-11-en-13-yl

(2R,3R)-4-fluoro-3-(isopropoxycarbonylamino)-2-methyl-2-[(triethylsilyl)oxy]butyrate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 63 were used as starting materials to perform the same procedure as that of Example 31, Step 1 and thereby to obtain the title compound as white amorphous solid.
$^1$H-NMR (400MHz, $CDCl_3$) δ : 0.62-0.80 (6H, m), 0.99 (9H, t, J=7.8Hz), 1.15 (6H, dd, J=15.9, 6.4Hz), 1.21-2.21 (7H, m), 1.35 (3H, s), 1.51 (3H, s), 1.66 (3H, s), 1.76 (6H, s), 2.49 (3H, s), 2.89 (1H, d, J=5.1Hz), 4.18 (1H, d, J=7.4Hz), 4.23 (1H, d, J=8.3Hz), 4.31-4.84 (5H, m), 4.89 (1H, d, J=9.3Hz), 4.92 (1H, s), 5.22 (1H, d, J=5.9Hz), 5.26 (1H, d, J=6.9Hz), 5.46 (1H, d, J=10.5Hz), 5.58 (1H, d, J=17.4Hz), 5.96-6.04 (2H, m), 6.14 (1H, t, J=8.8Hz), 7.49 (2H, t, J=7.7Hz), 7.61 (1H, t, J=7.4Hz), 8.17 (2H, d, J=7.8Hz).

Step 2: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-4-fluoro-3-(isopropoxycarbonylamino)-2-methyl-2-[(triethylsilyl)oxy]butyrate

The compound obtained in Step 1 mentioned above and the compound obtained in Reference Example 25 were used as starting materials to perform the same procedure as that of Example 1 and thereby to obtain the title compound as pale yellow amorphous solid, which was used as it was for the following reaction.

Step 3: (1S,2S,3R,4S,5R,8R,9S,10R,13S)-4-Acetoxy-2-(benzoyloxy)-5,20-epoxy-1-hydroxy-9,10-{(1S)-2-[(2S)-hydroxymethylazetidino]ethylidenedioxy}tax-11-en-13-yl

(2R,3R)-4-fluoro-2-hydroxy-3-(isopropoxycarbonylamino)-2-methylbutyrate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.19 (3H, d, J=6.6Hz), 1.22 (3H, d, J=6.6Hz), 1.27 (3H, s), 1.49 (3H, s), 1.57-2.06 (8H, m), 1.60 (6H, s), 1.76 (3H, s), 2.20-2.30 (2H, m), 2.42 (3H, s), 2.83 (1H, dd, J=12.7, 4.2Hz), 2.91 (1H, dd, J=12.7, 5.0Hz), 2.95 (1H, d, J=4.7Hz), 3.11 (1H, q , J=8.2Hz), 3.43 (1H, d, J=12.0Hz), 3.46-3.53 (2H, m), 3.65 (1H, d, J=9.8Hz), 4.13 (1H, d, J=6.9Hz), 4.23-4.35 (1H, m), 4.26 (1H, d, J=8.6Hz), 4.33 (1H, d, J=8.3Hz), 4.47 (1H, brs), 4.56-4.61 (1H, m), 4.67-4.73 (1H, m), 4.85-4.94 (3H, m), 5.22 (1H, d, J=7.1Hz), 5.40 (1H, d, J=9.8Hz), 5.99 (1H, d, J=4.4Hz), 6.18 (1H, t, J=8.5Hz), 7.48 (2H, t, J=7.4Hz), 7.61 (1H, t, J=7.4Hz), 8.15 (2H, d, J=7.8Hz).
FAB-MS; m/z: 861 $(M+H)^+$.

[Example 138]

[Formula 210]

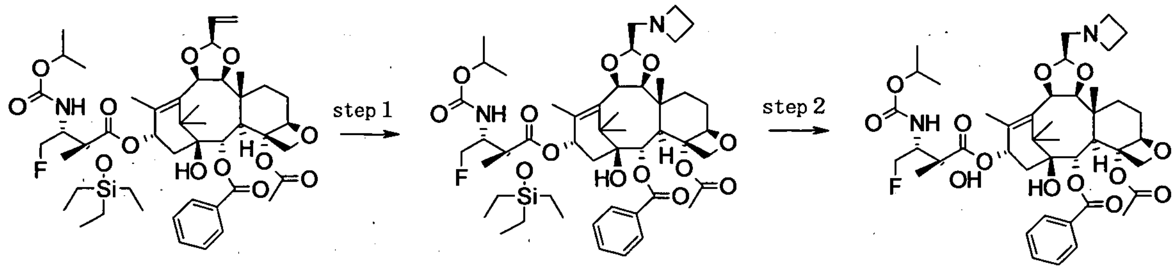


Step 1: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3R)-4-fluoro-3-(isopropoxycarbonylamino)-2-methyl-2-[(triethylsilyl)oxy]butyrate

The compound obtained in Example 137, Step 1 was used as a starting material to perform the same procedure as that of Example 1 and thereby to obtain the title compound as pale yellow amorphous solid.
$^1$H-NMR (400MHz, $CDCl_3$) δ : 0.61-0.78 (6H, m), 0.98 (9H, t, J=7.6Hz), 1.13 (3H, d, J=6.1Hz), 1.17 (3H, d, J=6.4Hz), 1.33 (3H, s), 1.50 (3H, s), 1.58-2.2 (9H, m), 1.62 (3H, s), 1.73 (3H, s), 1.75 (3H, s), 2.49 (3H, s), 2.74 (1H, dd, J=12.3, 5.9Hz), 2.81 (1H, dd, J=12.7, 5.0Hz), 2.87 (1H, d, J=4.9Hz), 3.31-3.39 (4H, m), 4.11 (1H, d, J=7.4Hz), 4.23 (1H, d, J=8.3Hz), 4.31-4.46 (3H, m), 4.59-4.91 (5H, m), 5.17 (1H, d, J=7.4Hz), 5.97 (1H, d, J=5.1Hz), 6.13 (1H, t, J=8.9Hz), 7.49 (2H, t, J=7.6Hz), 7.58-7.63 (1H, m), 8.17 (2H, d, J=7.8Hz).

Step 2: (1S,2S,3R,4S,5R,8R,9S,10R,13S)-4-Acetoxy-9,10-[(1S)-2-(azetidino)ethylidenedioxy]-2-(benzoyloxy)-5,20-epoxy-1-hydroxytax-11-en-13-yl

(2R,3R)-4-fluoro-2-hydroxy-3-(isopropoxycarbonylamino)-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.18 (3H, d, J=6.4Hz), 1.21 (3H, d, J=6.1Hz), 1.27 (3H, s), 1.48 (3H, s), 1.56-2.25 (10H, m), 1.60 (3H, s), 1.62 (3H, s), 1.76 (3H, s), 2.42 (3H, s), 2.74 (1H, dd, J=12.3, 5.9Hz), 2.83 (1H, dd, J=12.3, 4.4Hz), 2.95 (1H, d, J=4.9Hz), 3.33-3.40 (4H, m), 4.11 (1H, d, J=7.1Hz), 4.23-4.34 (1H, m), 4.26 (1H, d, J=8.3Hz), 4.32 (1H, d, J=8.6Hz), 4.55-4.60 (1H, m), 4.67-4.72 (1H, m), 4.84-4.93 (3H, m), 5.20 (1H, d, J=7.1Hz), 5.41 (1H, d, J=10.0Hz), 6.00 (1H, d, J=4.9Hz), 6.18 (1H, t, J=8.3Hz), 7.48 (2H, t, J=7.6Hz), 7.59-7.63 (1H, m), 8.15 (2H, d, J=7.8Hz).

FAB-MS; m/z: 831 $(M+H)^+$.

[Example 139]

[Formula 211]

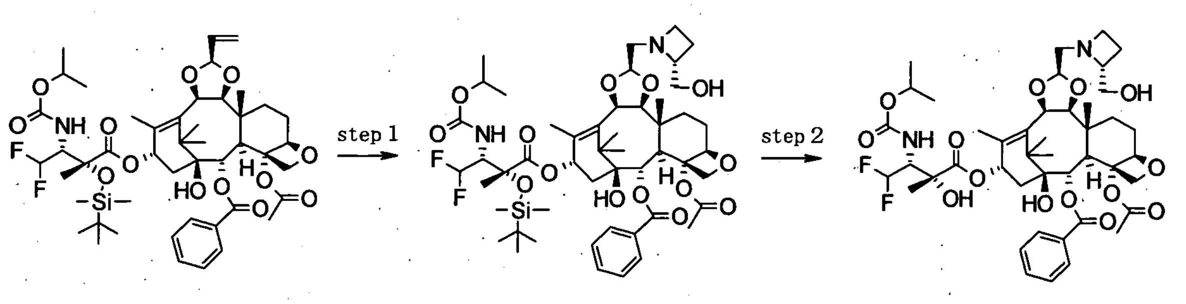


Step 1: (1S,2S,3R,45,5R,8R,9S,10R,13S)-4-Acetoxy-,2-(benzoyloxy)-5,20-epoxy-1-hydroxy-9,10-{(1S)-2-[(2R)-hydroxymethylazetidino]ethylidenedioxy}tax-11-en-13-yl

(2R,3R)-2-[(tert-butyldimethylsilyl)oxy]-4,4-difluoro-3-(isopropoxycarbonylamino)-2-methylbutyrate

The compound obtained in Example 105, Step 1 and the compound obtained in Reference Example 26 were used as starting materials to perform the same procedure as that of Example 1 and thereby to obtain the title compound as pale yellow amorphous solid.
$^1$H-NMR (400MHz, $CDCl_3$) δ : 0.15 (3H, s), 0.25 (3H, s), 0.92 (9H, s), 1.15 (3H, d, J=5.9Hz), 1.20 (3H, d, J=6.1Hz), 1.24-2.44 (10H, m), 1.33 (3H, s), 1.49 (3H, s), 1.62 (3H, s), 1.73 (3H, s), 1.80 (3H, s), 2.49 (3H, s), 2.79 (1H, dd, J=13.0, 5.4Hz), 2.86 (1H, d, J=4.9Hz), 2.95 (1H, dd, J=13.2, 3.9Hz), 3.10 (1H, q , J=8.3Hz), 3.44-3.54 (3H, m), 3.66 (1H, dd, J=11.8, 2.0Hz), 4.12 (1H, d, J=7.1Hz), 4.23 (1H, d, J=8.3Hz), 4.29-4.40 (2H, m), 4.72-5.00 (4H, m), 5.17 (1H, d, J=7.1Hz), 5.89 (1H, td, J=55.7, 4.7Hz), 5.96 (1H, d, J=4.9Hz), 6.12 (1H, t, J=8.8Hz), 7.50 (2H, t, J=7.8Hz), 7.61 (1H, t, J=7.4Hz), 8.17 (2H, d, J=8.1Hz).
FAB-MS; m/z: 993 $(M+H)^+$.

Step 2: (1S,2S,3R,4S,5R,8R,9S,10R,13S)-4-Acetoxy-2-(benzoyloxy)-5,20-epoxy-1-hydroxy-9,10-{(1S)-2-[(2R)-hydroxymethylazetidino]ethylidenedioxy}tax-11-en-13-yl

(2R,3R)-4,4-difluoro-2-hydroxy-3-(isopropoxycarbonylamino)-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.20 (3H, d, J=6.2Hz), 1.23 (3H, d, J=6.4Hz), 1.27 (3H, s), 1.47 (3H, s), 1.61 (3H, s), 1.61-2.06 (8H, m), 1.63 (3H, s), 1.75 (3H, s), 2.18-2.29 (2H, m), 2.42 (3H, s), 2.79 (1H, dd, J=12.7, 5.6Hz), 2.91-2.95 (2H, m), 3.08 (1H, q , J=8.3Hz), 3.42-3.53 (3H, m), 3.65 (1H, dd, J=11.4, 2.8Hz), 4.12 (1H, d, J=7.1Hz), 4.26 (1H, d, J=8.3Hz), 4.32 (1H, d, J=8.6Hz), 4.38-4.48 (1H, m), 4.64 (1H, brs), 4.86-4.95 (3H, m), 5.20 (1H, d, J=7.1Hz), 5.48 (1H, d, J=10.5Hz), 6.00 (1H, d, J=4.9Hz), 6.09 (1H, td, J=55.3, 3.2Hz), 6.20 (1H, t, J=8.6Hz), 7.49 (2H, t, J=7.7Hz), 7.59-7.64 (1H, m), 8.14 (2H, d, J=7.7Hz).
FAB-MS; m/z: 879 $(M+H)^+$.

[Example 140]

[Formula 212]

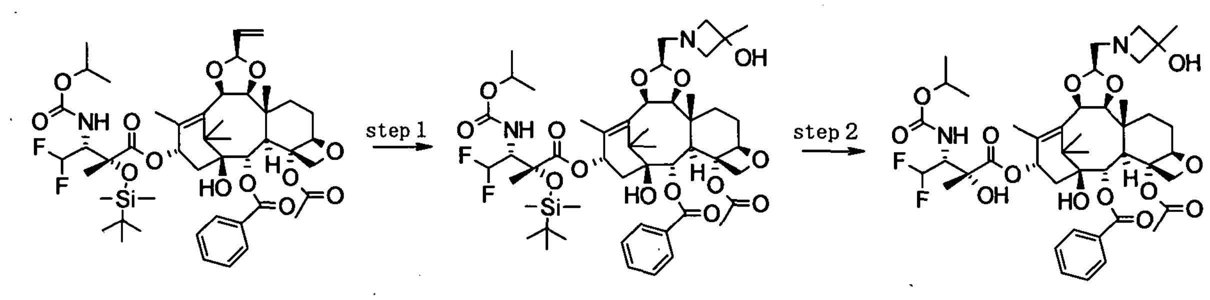


Step 1: (1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3R)-2-[(tert-butyldimethylsilyl)oxy]-4,4-difluoro-3-(isopropoxycarbonylamino)-2-methylbutyrate

The compound obtained in Example 105, Step 2 and the compound obtained in Reference Example 3 were used as starting materials to perform the same procedure as that of Example 1 and thereby to obtain the title compound as pale yellow amorphous solid.
$^1$H-NMR (400MHz, $CDCl_3$) δ : 0.15 (3H, s), 0.25 (3H, s), 0.92 (9H, s), 1.15 (3H, d, J=6.1Hz), 1.20 (3H, d, J=6.1Hz), 1.34 (3H, s), 1.50 (6H, s), 1.59-2.20 (6H, m), 1.62 (3H, s), 1.73 (3H, s), 1.80 (3H, s), 2.49 (3H, s), 2.83-2.88 (2H, m), 2.95 (1H, dd, J=12.3, 3.9Hz), 3.37-3.49 (4H, m), 3.84 (2H, brs), 4.12 (1H, d, J=7.1Hz), 4.24 (1H, d, J=8.3Hz), 4.29-4.40 (2H, m), 4.74-4.93 (3H, m), 4.97 (1H, d, J=10.3Hz), 5.17 (1H, d, J=7.1Hz), 5.89 (1H, td, J=55.4, 4.7Hz), 5.96 (1H, d, J=4.7Hz), 6.12 (1H, t, J=8.7Hz), 7.50 (2H, t, J=7.8Hz), 7.61 (1H, t, J=6.7Hz), 8.17 (2H, d, J=8.1Hz).
FAB-MS; m/z: 993 $(M+H)^+$.

Step 2: (1S,2S,3R,4S,5R,8R,9S,10R,13S)-4-(Acetoxy)-2-(benzoyloxy)-5,20-epoxy-1-hydroxy-9,10-[(1S)-2-(3-hydroxy-3-methylazetidino)ethylidenedioxy]tax-11-en-13-yl

(2R,3R)-4,4-difluoro-2-hydroxy-3-(isopropoxycarbonylamino)-2-methylbutyrate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.19 (3H, d, J=6.2Hz), 1.23 (3H, d, J=6.4Hz), 1.28 (3H, s), 1.48 (3H, s), 1.51 (3H, s), 1.60-2.07 (7H, m), 1.61 (3H, s), 1.63 (3H, s), 1.74 (3H, s), 2.22 (1H, dd, J=14.8, 9.7Hz), 2.42 (3H, s), 2.79 (1H, dd, J=12.5, 5.6Hz), 2.89-2.94 (2H, m), 3.19-3.29 (2H, m), 3.38-3.46 (2H, m), 4.11 (1H, d, J=7.1Hz), 4.27 (1H, d, J=8.3Hz), 4.33 (1H, d, J=8.3Hz), 4.37-4.48 (1H, m), 4.67 (1H, brs), 4.87-4.94 (3H, m), 5.20 (1H, d, J=6.9Hz), 5.50 (1H, d, J=10.5Hz), 5.99 (1H, d, J=4.9Hz), 6.09 (1H, td, J=55.4, 3.1Hz), 6.20 (1H, t, J=8.3Hz), 7.49 (2H, t, J=7.5Hz), 7.62 (1H, t, J=7.5Hz), 8.15 (2H, d, J=7.4Hz).
FAB-MS; m/z: 879 $(M+H)^+$.

[Example 141]

[Formula 213]

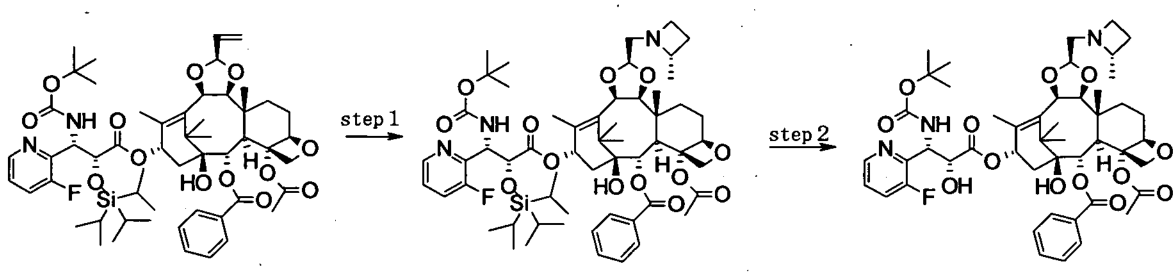


Step 1: (1S,2S,3R,4S,5R,8R,9S, 10R,13S)-4-(Acetoxy)-2-(benzoyloxy)-5,20-epoxy-1-hydroxy-9,10-{(1S)-2-[(2S)-2-methylazetidino]ethylidenedioxy}tax-11-en-13-yl

(2R,3S)-3-(tert-butoxycarbonylamino)-3-(3-fluoro-2-pyridyl)-2-[(triisopropylsilyl)oxy]propionate

(1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonylamino)-3-(3-fluoro-2-pyridyl)-2-[(triisopropylsilyl)oxy]propionate and the compound obtained in Reference Example 61, Step 3 were used as starting materials to perform the same procedure as that of Example 1 and thereby to obtain the title compound as white amorphous solid.
$^1$H-NMR (400MHz, $CDCl_3$) δ : 0.86-0.93 (21H, m), 0.86-2.37 (9H, m), 1.22 (3H, d, J=6.1Hz), 1.34 (3H, s), 1.38 (9H, s), 1.52 (3H, s), 1.63 (3H, s), 1.79 (3H, s), 2.49 (3H, s), 2.63 (1H, dd, J=12.5, 6.1Hz), 2.89-2.99 (3H, m), 3.21-3.31 (1H, m), 3.48-3.53 (1H, m), 4.16 (1H, d, J=7.6Hz), 4.22 (1H, d, J=8.3Hz), 4.35 (1H, d, J=8.1Hz), 4.85-4.88 (1H, m), 4.94-4.97 (2H, m), 5.18 (1H, d, J=7.1Hz), 5.60 (1H, d, J=10.0Hz), 5.98 (1H, d, J=5.4Hz), 6.05-6.11 (2H, m), 7.24-7.29 (1H, m), 7.37-7.43 (1H, m), 7.47 (2H, t, J=7.7Hz), 7.56-7.61 (1H, m), 8.15-8.18 (2H, m), 8.40 (1H, d, J=4.7Hz).

Step 2: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-methylazetidino]ethylidenedioxy}tax-11-en-13-yl

(2R,3S)-3-(tert-butoxycarbonylamino)-3-(3-fluoro-2-pyridyl)-2-hydroxypropionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 1.19-2.15 (9H, m), 1.22 (3H, d, J=6.1Hz), 1.28 (3H, s), 1.40 (9H, s), 1.50 (3H, s), 1.62 (3H, s), 1.78 (3H, s), 2.34 (3H, s), 2.40-2.49 (1H, m), 2.64 (1H, dd, J=12.5, 6.4Hz), 2.89-2.97 (3H, m), 3.23-3.32 (1H, m), 3.48-3.55 (1H, m), 4.13 (1H, d, J=7.1Hz), 4.23 (1H, d, J=8.3Hz), 4.33 (1H, d, J=8.3Hz), 4.68 (1H, d, J=2.7Hz), 4.87-4.93 (2H, m), 5.22 (1H, d, J=6.9Hz), 5.65 (1H, d, J=8.3Hz), 6.00 (1H, d, J=5.1Hz), 6.08 (1H, t, J=8.3Hz), 6.21 (1H, d, J=8.3Hz), 7.28-7.33 (1H, m), 7.42-7.50 (3H, m), 7.57-7.62 (1H, m), 8.11-8.15 (2H, m), 8.40 (1H, d, J=4.7Hz).
FAB-MS; m/z: 908 $(M+H)^+$.

[Example 142]

[Formula 214]

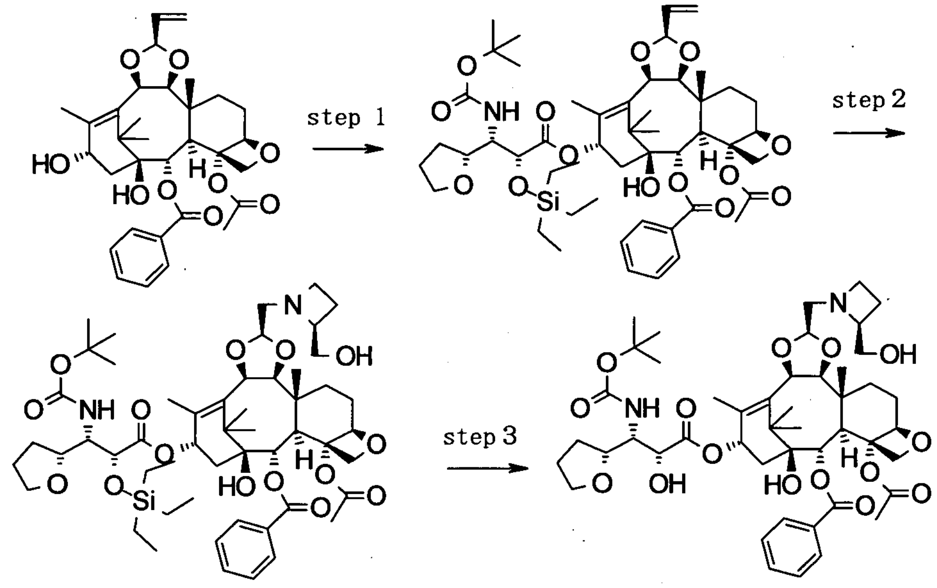


Step 1: (1S,2S,3R,4S,5R,8R,9S,10R,13S)-4-(Acetoxy)-2-(benzoyloxy)-5,20-epoxy-1-hydroxy-9,10-[(1S)-2-propenylidenedioxy]tax-11-en-13-yl

(2R,3S)-3-(tert-butoxycarbonyl)-3-[(2R)-tetrahydrofuran-2-yl]-2-[(triethylsilyl)oxy]propionate

(1S,2S,3R,4S,5R,8R,9S,10R,13S)-4-(Acetoxy)-2-(benzoyloxy)-1,13-dihydroxy-5,20-epoxy-9,10-[(1S)-2-propenylidenedioxy]tax-11-ene and the compound obtained in Reference Example 64, Step 4 were used as starting materials to perform the same procedure as that of Example 31, Step 1 and thereby to obtain the title compound as white amorphous solid.
$^1$H-NMR (400MHz, $CDCl_3$) δ : 0.62-0.70 (6H, m), 0.99 (9H, t, J=7.6Hz), 1.32 (3H, s), 1.41 (9H, s), 1.47-2.27 (11H, m), 1.51 (3H, s), 1.64 (3H, s), 1.79 (3H, s), 2.39 (3H, s), 2.96 (1H, d, J=5.4Hz), 3.70-3.77 (1H, m), 3.83-3.90 (1H, m), 3.92-3.98 (1H, m), 4.17-4.23 (2H, m), 4.34 (2H, d, J=8.3Hz), 4.42 (1H, d, J=3.7Hz), 4.91-4.96 (2H, m), 5.20 (1H, d, J=6.3Hz), 5.27 (1H, d, J=6.8Hz), 5.45 (1H, d, J=10.5Hz), 5.57 (1H, d, J=17.1Hz), 5.94-6.04 (2H, m), 6.07 (1H, t, J=8.5Hz), 7.48 (2H, t, J=7.7Hz), 7.60 (1H, t, J=7.4Hz), 8.15 (2H, d, J=8.5Hz).
ESI-MS; m/z: 940 $(M+H)^+$.

Step 2: (1S,2S,3R,4S,5R,8R,9S, 10R, 13S)-4-(Acetoxy)-2-(benzoyloxy)-5,20-epoxy-1-hydroxy-0-{(1S)-2-[(2S)-2-hydroxymethylazetidino]ethylidenedioxy}tax-11-en-13-yl

(2R,3S)-3-(tert-butoxycarbonyl)-3- [(2R)-tetrahydrofuran-2-yl]-2- [(triethylsilyl)oxy]propionate

The compound obtained in Step 1 mentioned above and the compound obtained in Reference Example 52 were used as starting materials to perform the same procedure as that of Example 1 and thereby to obtain the title compound as white amorphous solid.
$^1$H-NMR (400MHz, $CDCl_3$) δ : 0.60-0.7.0 (6H, m), 0.98 (9H, t, J=7.9Hz), 1.30 (3H, s), 1.41 (9H, s), 1.50 (3H, s), 1.55-2.31 (14H, m), 1.59 (3H, s), 1.77 (3H, s), 2.39 (3H, s), 2.80 (1H, dd, J=12.9, 4.6Hz), 2.88 (1H, dd, J=12.8, 5.0Hz), 2.94 (1H, d, J=4.9Hz), 3.09 (1H, q, J=8.2Hz), 3.39-3.52 (3H, m), 3.63 (1H, dd, J=11.2, 2.9Hz), 3.69-3.77 (1H, m), 3.83-3.98 (2H, m), 4.05-4.12 (1H, m), 4.16 (1H, d, J=7.1Hz), 4.19 (1H, d, J=8.3Hz), 4.34 (1H, d, J=8.5Hz), 4.42 (1H, d, J=3.7Hz), 4.84 (1H, t, J=4.9Hz), 4.91-4.96 (2H, m), 5.20 (1H, d, J=7.1Hz), 5.95 (1H, d, J=5.1Hz), 6.05 (1H, t, J=9.0Hz), 7.48 (2H, t, J=7.8Hz), 7.60 (1H, t, J=7.4Hz), 8.14 (2H, d, J=8.0Hz).
ESI-MS; m/z: 1013 $(M+H)^+$.

Step 3: (1S,2S,3R,4S,5R,8R,9S,10R,13S)-4-(Acetoxy)-2-(benzoyloxy)-5,20-epoxy-1-hydroxy-9,10-{(1S)-2-[(2S)-2-hydroxymethylazetidino]ethylidenedioxy}tax-11-en-13-yl

(2R,3R)-3-(tert-butoxycarbonyl)-2-hydroxy-3-[(2R)-tetrahydrofuran-2-yl]propionate

The compound obtained in Step 2 mentioned above was used as a starting material to perform the same procedure as that of Example 31, Step 2 and thereby to obtain the title compound as white amorphous solid.
$^1$H-NMR (400MHz, $CDCl_3$) δ : 1.25 (3H, s), 1.42 (9H, s), 1.49 (3H, s), 1.59 (3H, s), 1.71-2.44 (14H, m), 1.83 (3H, s), 2.31 (3H, s), 2.83 (1H, dd, J=12.7, 5.1Hz), 2.89 (1H, dd, J=12.9, 5.4Hz), 2.93 (1H, d, J=5.4Hz), 3.10 (1H, q , J=8.3Hz), 3.38-3.53 (3H, m), 3.65 (1H, dd, J=11.1, 3.1Hz), 3.70-3.78 (2H, m), 3.83-3.92 (1H, m), 4.10-4.25 (4H, m), 4.32 (1H, d, J=8.1Hz), 4.47 (1H, s), 4.88 (1H, t, J=5.0Hz), 4.93 (1H, s), 5.19-5.28 (2H, m), 5.93-6.02 (2H, m), 7.47 (2H, t, J=7.7Hz), 7.60 (1H, t, J=7.4Hz), 8.12 (2H, d, J=7.1Hz). ESI-MS; m/z: 899 $(M+H)^+$.

[Example 143]

[Formula 215]

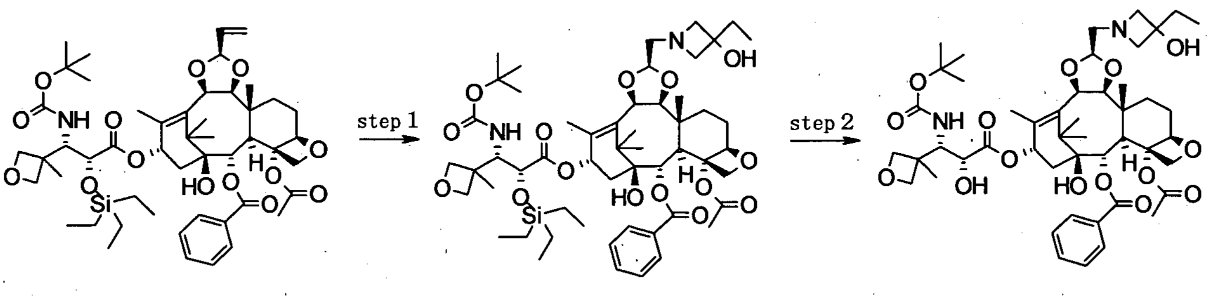


Step 1: (1S,2S,3R,4S,5R,8R,9S,10R,13S)-4-(Acetoxy)-2-(benzoyloxy)-5,20-epoxy-9,10-[(1S)-2-(3-ethyl-3-hydroxyazetidino)ethylidenedioxy] -hydroxytax-11-en-13-yl

(2R,3S)-3-(tert-butoxycarbonylamino)-3-(3-methyl-3-oxetanyl)-2-[(triethylsilyl)oxy]propionate

The compound obtained in Example 123, Step 1 and the compound obtained in Reference Example 4, Step 2 were used as starting materials to perform the same procedure as that of Example 1 and thereby to obtain the title compound as white amorphous solid.
$^1$H-NMR (400MHz, $CDCl_3$) δ : 0.55-0.71 (6H, m), 0.97 (3H, t, J=7.3Hz), 0.98 (9H, t, J=8.1Hz), 1.27-2.21 (10H, m), 1.29 (3H, s), 1.39 (9H, s), 1.50 (3H, s), 1.53 (3H, s), 1.60 (3H, s), 1.76 (3H, s), 2.35 (3H, s), 2.78-2.94 (3H, m), 3.17 (2H, t, J=7.7Hz), 3.43-3.51 (2H, m), 3.96 (1H, dd, J=10.4, 1.8Hz), 4.14 (1H, d, J=8.1Hz), 4.18-4.28 (3H, m), 4.33 (1H, d, J=8.1Hz), 4.59 (1H, d, J=2.0Hz), 4.64 (1H, d, J=5.9Hz), 4.74 (1H, d, J=6.8Hz), 4.86 (1H, t, J=4.9Hz), 4.91 (1H, brs), 5.17 (1H, d, J=6.8Hz), 5.33 (1H, d, J=10.5Hz), 5.95 (1H, d, J=5.1Hz), 6.02 (1H, t, J=8.9Hz), 7.49 (2H, t, J=7.6Hz), 7.61 (1H, tt, J=7.3, 1.5Hz), 8.11-8.16 (2H, m).

Step 2: (1S,2S,3R,4S,5R,8R,9S,10R,13S)-4-(Acetoxy)-2-(benzoyloxy)-5,20-epoxy-9,10-[(1S)-2-(3-ethyl-3-hydroxyazetidino)ethylidenedioxy]-1-hydroxytax-11-en-13-yl

(2R,3S)-3-(tert-butoxycarbonylamino)-2-hydroxy-3-(3-methyl-3-oxetanyl)propionate

The compound obtained in Step 1 mentioned above was used as a starting material to perform the same procedure as that of Example 31, Step 2 and thereby to obtain the title compound as white amorphous solid.
$^1$H-NMR (400MHz, $CDCl_3$) δ : 0.97 (3H, t, J=7.6Hz), 1.24 (3H, s), 1.37-2.41 (10H, m), 1.41 (9H, s), 1.49 (3H, s), 1.54 (3H, s), 1.61 (3H, s), 1.80 (3H, s), 2.31 (3H, s), 2.80 (1H, dd, J=12.3, 6.0Hz), 2.89-2.95 (2H, m), 3.15 (2H, t, J=8.8Hz), 3.46 (2H, t, J=7.4Hz), 4.12 (1H, d, J=7.1Hz), 4.20-4.34 (6H, m), 4.48 (1H, s), 4.67 (1H, d, J=6.1Hz), 4.80 (1H, d, J=7.1Hz), 4.89-4.95 (2H, m), 5.24 (1H, d, J=6.8Hz), 5.36 (1H, d, J=10.5Hz), 6.00 (1H, d, J=4.9Hz), 6.07 (1H, t, J=8.5Hz), 7.48

(2H, t, J=7.7Hz), 7.61 (1H, tt, J=7.3, 1.2Hz), 8.10-8.15 (2H, m).
FAB-MS; m/z: 913 $(M+H)^+$.

(Test Example 1)

Three types of human tumor cell lines (all of the cells were obtained from American Type Culture Collection) were seeded on 96-well microplates as follows.
NCI-H460 (human lung cancer cell line): 5.0 x $10^2$ cells/190 μL/well
HCT-15 (P glycoprotein (P-gp) expressing human colon cancer cell line, J. Natl. Cancer Inst., 2001, 93:1234-1245): 1.0 x $10^3$ cells/190 μL/well
HCT116 (human colon cancer cell line): 1.0 x $10^3$ cells/190 μL/well
On the next day, each test compound was dissolved in 100% dimethyl sulfoxide (DMSO) at a concentration of 10 mg/mL. This solution was diluted with 0.01% Cremophor EL-PBS(-) (Cremophor EL: Sigma Chemical Co., St. Louis, MO, USA, PBS: Invitrogen Corp., Carlsbad, CA, USA), and 10 μL of the diluted solution was added to each well (final concentration: 100 to 0.000381 ng/mL). The cells were cultured for three days, and then 20 μ/L of a 5 mg/mL solution of 3-(4,5-dimethylthiazol-2-yl)-2,5-diphenyl-2H-tetrazolium bromide (MTT, Sigma Chemical Co.) was added to each well. The cells were further cultured for 4 hours, and then the medium was removed by centrifugation. By adding 150 μ L of DMSO to each well, the produced formazan product was dissolved. The absorbance of the solution was measured at 540 nm. The drug concentration suppressing cell proliferation of the drug addition group to 50% of the cell proliferation of the drug free group (GI50 value, ng/mL) was calculated, and used as an index of anti-cell activity.
The results are shown in Table 1. The compounds of the present invention showed significant antitumor effect against the cancer cell lines including the drug resistant cells.
[Table 1]

| | NCI-H460 (GI50 value, ng/mL) | HCT-15 (GI50 value, ng/mL) | HCT116 (GI50 value, ng/mL) |
|---|---|---|---|
| Paclitaxel | 1.87 | > 100 | 1.71 |
| Compound of Example 1 | 0.19 | 1.14 | 0.244 |
| Compound of Example 2 | 0.606 | 2.31 | 0.669 |
| Compound of Example 5 | 0.162 | 8.08 | 0.229 |
| Compound of Example 6 | 0.172 | 7.94 | 0.183 |
| Compound of Example 8 | 0.128 | 0.236 | 0.155 |
| Compound of Example 11 | 0.109 | 4.21 | 0.173 |

(Test Example 2)

Mice (C57BL/6, Charles River Japan) were subcutaneously transplanted with B16 melanoma BL6 (Cancer Res., Poste, G., Fidler, I.J. et al., 1980, 40:1636; Bioorg. Med. Chem. Lett., Takeda, Y. et al., 2003, 13:185-190), or M5076 (obtained from Japanese Foundation for Cancer Research), which is a histiocytoma expressing P-gp. Four days after the transplantation in the case of B16 melanoma BL6, or 8 to 10 days after the transplantation in the case of M5076, each test compound suspended in a 0.5% aqueous solution of carboxymethylcellulose sodium, or dissolved in EtOH: Tween 80:5% glucose = 5:5:90 (v/v %) was orally (po) or intravenously (iv) administered to the mice. Ten days after the administration in the case of B16 melanoma BL6, or 8 to 10 days after the administration in the case of M5076, the mice were dissected, and tumor weights were measured. The antitumor effect was calculated in accordance with the following equation.

[1- (Average tumor weight of compound administration group/average tumor weight of solvent administration group)] x 100

An antitumor effect of 58% or more was considered effective (Int. J. Cancer, 1989, 43:637-44), and the maximum tolerance dose (MTD, maximum dose not causing death, mg/kg) and minimum effective dose (MED, mg/kg) showing

effectiveness were obtained, respectively. The therapeutic index was calculated in accordance with the equation: MTD/MED.
The results are shown in Table 2. ND means that the test was not performed.
The effective dose range means the range of from MED to MTD.

[Table 2]

| | B16 Melanoma BL6 | | | | M5076 | | | |
|---|---|---|---|---|---|---|---|---|
| | po | | iv | | po | | iv | |
| | Effective dose range (mg/kg) | Therapeutic index | Effective dose range (mg/kg) | Therapeutic index | Effective dose range (mg/kg) | Therapeutic index | Effective dose range (mg/kg) | Therapeutic index |
| Compound of WO01/27115, Example 7 | 5.88-12.0 | 2.0 | 4.12-12.0 | 2.9 | 8.40 | 1.0 | 8.40 | 1.0 |
| Compound of Example 1 | 8.61-25.0 | 2.9 | ND | ND | ND | ND | ND | ND |
| Compound of Example 2 | 6.71-19.6 | 2.9 | 7.21-21.0 | 2.9 | 13.7-28 | 2.0 | 14.7 | 1.0 |
| Compound of Example 5 | 14.7-87.6 | 6.0 | ND | ND | 30.0 | 1.0 | ND | ND |
| Compound of Example 6 | 9.10-38.0 | 4.2 | ND | ND | 18.6-38.0 | 2.0 | ND | ND |
| Compound of Example 8 | 10.3-30.0 | 2.9 | ND | ND | 21.0-30.0 | 1.4 | ND | ND |
| Compound of Example 9 | 10.3-61.3 | 6.0 | ND | ND | ND | ND | ND | ND |
| Compound of Example 11 | 7.07-42.0 | 5.9 | ND | ND | ND | ND | ND | ND |
| Compound of Example 12 | 11.6-48.0 | 4.1 | ND | ND | ND | ND | ND | ND |
| Compound of Example 21 | 7.63-45.0 | 5.9 | ND | ND | ND | ND | ND | ND |
| Compound of Example 22 | 8.40-35.0 | 4.2 | 5.88-35.0 | 6.0 | 17.2-35 | 2.0 | 12.0-24.5 | 2.0 |
| Compound of Example 23 | 20.6-60.0 | 2.9 | ND | ND | ND | ND | ND | ND |

(continued)

| | B16 Melanoma BL6 | | | | M5076 | | | |
|---|---|---|---|---|---|---|---|---|
| | po | | iv | | po | | iv | |
| | Effective dose range (mg/kg) | Therapeutic index | Effective dose range (mg/kg) | Therapeutic index | Effective dose range (mg/kg) | Therapeutic index | Effective dose range (mg/kg) | Therapeutic index |
| Compound of Example 28 | 13.2-55.0 | 4.2 | ND | ND | ND | ND | ND | ND |
| Compound of Example 31 | 8.82-18.0 | 2.0 | 3.43-10.0 | 2.9 | ND | ND | 10.0 | 1.0 |
| Compound of Example 34 | ND | ND | 10.1-42.0 | 4.2 | ND | ND | ND | ND |
| Compound of Example 37 | 14.7-30.0 | 2.0 | 7.21-30.0 | 4.2 | ND | ND | 14.7-21.0 | 1.4 |
| Compound of Example 40 | ND | ND | 5.52-23.0 | 4.2 | ND | ND | ND | ND |
| Compound of Example 41 | 8.58-25.0 | 2.9 | 5.29-22.0 | 4.2 | ND | ND | 17.5-25.0 | 1.4 |

(Test Example 3)

Solubility of each compound was determined in the first solution for disintegration test according to Japanese Pharmacopoeia (henceforth referred to as the first solution, solution obtained by adding 7 mL of hydrochloric acid and water to 2.00 g of sodium chloride to dissolve sodium chloride and fill up the solution to 1000 mL, pH 1.2) and the second solution for disintegration test according to Japanese Pharmacopoeia (henceforth referred to as the second solution, solution obtained by adding 118 mL of 0.2 mol/L solution of sodium hydroxide to 6.80 g of potassium dihydrogenphosphate, adding water to the mixture to dissolve potassium dihydrogenphosphate and filling up the solution to 1000 mL, pH 6.8).
Each test compound was dissolved in 100% dimethyl sulfoxide (DMSO) at a concentration of 10 mM, and 50 μL of the solution was lyophilized. The lyophilized test compound was added with 250 μL of the first solution or second solution, and the mixture was stored at room temperature for 12 hours or longer under light shielding, and filtered (0.7 μm). The filtrate was taken in a volume of 90 μL, and added with 50% DMSO (90 μL) to obtain a two-fold diluted solubility sample. The two-fold dilution solubility sample was taken in a volume of 20 μL, and added with 50% DMSO (180 μL) to obtain a 20-fold diluted solubility sample. Solubility of each test sample was determined by quantifying the test compound in the resulting diluted solubility test samples using high-performance liquid chromatography.
The results are shown in Table 3.
[Table 3]

| | Solubility (μg/mL) | |
|---|---|---|
| | pH 1.2 | pH 6.8 |
| Paclitaxel | < 4 | < 4 |
| Compound of WO01/27115, Example 7 | 760-950 | 36 |
| Compound of Example 1 | 1200 | 170 |
| Compound of Example 2 | 1100 | 170 |
| Compound of Example 5 | 1000 | 140 |
| Compound of Example 6 | 690 | 87 |
| Compound of Example 8 | 600 | 10 |
| Compound of Example 9 | 1400 | 130 |
| Compound of Example 11 | 1300 | 170 |
| Compound of Example 12 | 1100 | 92 |
| Compound of Example 21 | > 1000 | 150 |
| Compound of Example 22 | 1100 | 85 |
| Compound of Example 23 | 1400 | 360 |
| Compound of Example 28 | 1300 | 18 |
| Compound of Example 31 | 1000 | 160 |
| Compound of Example 34 | 830 | 310 |
| Compound of Example 37 | 670 | 210 |
| Compound of Example 40 | 1000 | 150 |
| Compound of Example 41 | 910 | 370 |

[Industrial Applicability]

The compounds of the present invention have high antitumor effect against cancer cells including drug resistant cells, and accordingly, they can be used as potent anticancer agents. Furthermore, the compounds of the present invention have a wide effective dose range, thereby an optimum dosage wide apart from MTD can be expected, and therefore, they can be used as safe anticancer agents less likely to cause side effects.

## Claims

1. A compound represented by the general formula (1) or a salt thereof, or a solvate thereof:

[Formula 1]

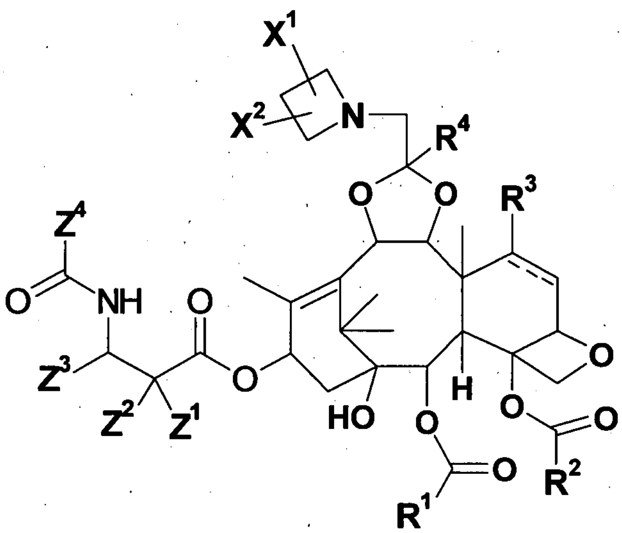

(1)

[wherein $X^1$ and $X^2$ independently represent hydrogen atom, a halogen atom, hydroxyl group, cyano group, carboxy group, an alkoxy group which may be substituted, an alkoxycarbonyl group, an alkyl group which may be substituted, an alkenyl group which may be substituted, an alkynyl group which may be substituted, a phenyl group which may be substituted, an alkanoyl group which may be substituted, a carbamoyl group which may be substituted, a carbamoyloxy group which may be substituted, an alkylsulfonyl group which may be substituted, an aminosulfonyl group which may be substituted, an amino group which may be substituted, or a 4- to 6-membered saturated heterocyclic group, or

$X^1$ and $X^2$ may together form oxo group or a =N-OY group (Y represents hydrogen atom, or an alkyl group which may be substituted), or $X^1$ and $X^2$ may form a 4- to 6-membered saturated hydrocarbon ring binding as a spiro ring system or a condensed ring system, or a 5- or 6-membered saturated heterocyclic ring binding as a spiro ring system or a condensed ring system together with the carbon atom or atoms to which $X^1$ and $X^2$ bind,

$R^1$ represents a phenyl group which may be substituted,

$R^2$ represents an alkyl group which may be substituted, an alkenyl group which may be substituted, or an alkoxy group which may be substituted,

$R^3$ represents hydrogen atom, a halogen atom, hydroxyl group, or an alkoxy group which may be substituted,

$R^4$ represents hydrogen atom, or an alkyl group which may be substituted,

$Z^1$ and $Z^2$ independently represent hydrogen atom, a halogen atom, hydroxyl group, an alkoxy group which may be substituted, or an alkyl group which may be substituted,

$Z^3$ represents cyano group, an alkyl group which may be substituted, an alkenyl group which may be substituted, an alkynyl group which may be substituted, a phenyl group which may be substituted, or a 4- to 6-membered saturated or unsaturated heterocyclic group which may be substituted,

$Z^4$ represents an alkyl group which may be substituted, an alkenyl group which may be substituted, an alkynyl group which may be substituted, a phenyl group which may be substituted, an aralkyl group which may be substituted, a 5- or 6-membered aromatic heterocyclic group which may be substituted, or an alkoxy group which may be substituted, and

the 6- and 7-positions of the partial structure represented by the following formula (2)

[Formula 2]

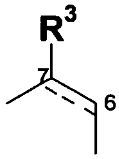

(2)

may be bound with a double bond (when the bond is a double bond, $R^3$ is hydrogen atom).

**2.** The compound or a salt thereof, or a solvate thereof according to claim 1, wherein $X^1$ and $X^2$ independently represent hydrogen atom, a halogen atom, hydroxyl group, cyano group, an alkoxy group which may be substituted, an alkyl group which may be substituted, a carbamoyl group which may be substituted, a carbamoyloxy group which may be substituted, an alkylsulfonyl group which may be substituted, or an amino group which may be substituted, or $X^1$ and $X^2$ together represent a =N-OY group (Y represents hydrogen atom, or an alkyl group which may be substituted).

**3.** The compound or a salt thereof, or a solvate thereof according to claim 1 or 2, wherein $R^2$ is an alkyl group which may be substituted.

**4.** The compound or a salt thereof, or a solvate thereof according to any one of claims 1 to 3, wherein $R^3$ is hydrogen atom.

**5.** The compound or a salt thereof, or a solvate thereof according to any one of claims 1 to 4, wherein $R^4$ is hydrogen atom.

**6.** The compound or a salt thereof, or a solvate thereof according to any one of claims 1 to 5, wherein $Z^1$ is hydroxyl group.

**7.** The compound or a salt thereof, or a solvate thereof according to any one of claims 1 to 6, wherein $Z^2$ is hydrogen atom, or an alkyl group which may be substituted

**8.** The compound or a salt thereof, or a solvate thereof according to any one of claims 1 to 7, wherein $Z^3$ is an alkyl group which may be substituted, or a 4- to 6-membered saturated or unsaturated heterocyclic group which may be substituted.

**9.** The compound or a salt thereof, or a solvate thereof according to any one of claims 1 to 8, wherein $Z^4$ is a phenyl group which may be substituted, or an alkoxy group which may be substituted.

**10.** The compound or a salt thereof, or a solvate thereof according to any one of claims 1 to 9, wherein the 6- and 7-positions of the partial structure represented by the formula (2) are bonded with a single bond.

**11.** The compound or a salt thereof, or a solvate thereof according to any one of claims 1 to 10, which has the absolute configuration represented by the general formula (3):

[Formula 3]

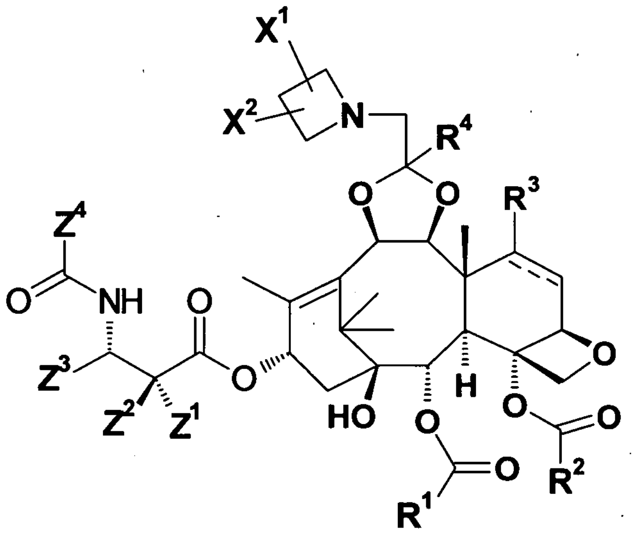

(3)

(wherein $X^1$, $X^2$, $R^1$ to $R^4$, and $Z^1$ to $Z^4$ have the same meanings as those defined above).

12. A medicament comprising the compound or a salt thereof, or a solvate thereof according to any one of claims 1 to 11 as an active ingredient.

13. An anticancer agent comprising the compound or a salt thereof, or a solvate thereof according to any one of claims 1 to 11 as an active ingredient.

14. A pharmaceutical composition comprising the compound or a salt thereof, or a solvate thereof according to any one of claims 1 to 11, and a pharmaceutically acceptable carrier.

15. Use of the compound or a salt thereof, or a solvate thereof according to any one of claims 1 to 11 for manufacture of a medicament.

16. Use of the compound or a salt thereof, or a solvate thereof according to any one of claims 1 to 11 for manufacture of an anticancer agent.

17. A method for therapeutic treatment of cancer, which comprises administering the compound or a salt thereof, or a solvate thereof according to any one of claims 1 to 11.

**INTERNATIONAL SEARCH REPORT**

International application No.
PCT/JP2006/321094

A. CLASSIFICATION OF SUBJECT MATTER
*C07D493/08*(2006.01)i, *A61K31/397*(2006.01)i, *A61K31/422*(2006.01)i, *A61K31/443*(2006.01)i, *A61P35/00*(2006.01)i

According to International Patent Classification (IPC) or to both national classification and IPC

B. FIELDS SEARCHED

Minimum documentation searched (classification system followed by classification symbols)
C07D493/08, A61K31/397, A61K31/422, A61K31/443, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 9-012578 A (Daiichi Pharmaceutical Co., Ltd.), 14 January, 1997 (14.01.97), & WO 96/033998 A1 & EP 826688 A1 & US 6075140 A & US 2001/0041796 A1 & US 2003/0162971 A1 | 1-16 |
| Y | WO 2001/027115 A1 (Daiichi Pharmaceutical Co., Ltd.), 19 April, 2001 (19.04.01), & US 2002/0143178 A1 & JP 2002-332287 A | 1-16 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 December, 2006 (06.12.06) | 19 December, 2006 (19.12.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/321094

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 17
because they relate to subject matter not required to be searched by this Authority, namely:
Claim 17 pertains to methods for treatment of the human body by therapy and thus relates to a subject matter which this International Searching Authority is not required, under the provisions of the PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 9012578 A
- WO 01027115 A
- JP 2002332287 A
- JP 2003342269 A
- WO 200127115 A
- US 5354865 A
- WO 9633998 A
- WO 0127115 A

### Non-patent literature cited in the description

- *Bioorg. Med. Chem. Lett.,* 2002, vol. 12, 2815
- *Tetrahedron,* 1992, vol. 48, 6985
- *Tetrahedron Lett.,* 1994, vol. 35, 8931
- *J. Med. Chem.,* 1995, vol. 38, 2263
- *Tetrahedron Lett.,* 1995, vol. 36, 1609
- *Chem. Pharm. Bull.,* 2002, vol. 50, 1398
- *J. Am. Chem. Soc.,* 1938, vol. 60, 1160
- *Tetrahedron Lett.,* 1976, vol. 17, 2935
- *Tetrahedron Lett.,* 1993, vol. 34, 4149
- *J. Org. Chem.,* 1972, vol. 37, 3953
- *Tetrahedron Lett.,* 1996, vol. 37, 1297
- *J. Heterocycl. Chem.,* 1994, vol. 31, 271
- *J. Med. Chem.,* 1993, vol. 36, 801
- *Tetrahedron,* 1993, vol. 49, 8211
- *J. Med. Chem.,* 2001, vol. 44, 94
- *J. Med. Chem.,* 1994, vol. 37, 2655
- *J. Org. Chem.,* 1991, vol. 56, 449
- *Bioorg. Med. Chem.,* 2000, vol. 8, 1619
- *Bioorg. Med. Chem.,* 2003, vol. 11, 4315
- *J. Org. Chem.,* 1995, vol. 60, 5174
- *J. Org. Chem.,* 1994, vol. 59, 3123
- *J. Am. Chem. Soc.,* 1995, vol. 117, 6394
- *J. Org. Chem.,* 1997, vol. 62, 8826
- *Tetrahedron,* 1998, vol. 54, 4991
- *Bioorg. Med. Chem. Lett.,* 2003, vol. 13, 185
- *Chem. Pharm. Bull.,* 1973, vol. 21, 228, 345
- *J. Med. Chem.,* 2005, vol. 24, 7637
- *J. Org. Chem.,* 1988, vol. 53, 4227
- *Tetrahedron Lett.,* 2004, vol. 45, 3607
- *J. Org. Chem.,* 1998, vol. 53, 4227
- *J. Org. Chem.,* 2002, vol. 67, 772
- *J. Med. Chem.,* 1993, vol. 7, 801
- *Bioorg. Med. Chem. Lett.,* 2002, vol. 12, 1083
- *Bioorg. Med. Chem. Lett.,* 1993, vol. 3, 2467
- P glycoprotein (P-gp) expressing human colon cancer cell line. *J. Natl. Cancer Inst.,* 2001, vol. 93, 1234-1245
- **POSTE, G. ; FIDLER, I.J. et al.** *Cancer Res.,* 1980, vol. 40, 1636
- **TAKEDA, Y. et al.** *Bioorg. Med. Chem. Lett.,* 2003, vol. 13, 185-190
- *Int. J. Cancer,* 1989, vol. 43, 637-44